(19) Europäisches Patentamt / European Patent Office / Office européen des brevets

(11) **EP 4 472 649 B1**

(12) **EUROPEAN PATENT SPECIFICATION**

(45) Date of publication and mention of the grant of the patent:
**24.06.2026 Bulletin 2026/26**

(21) Application number: **23702534.1**

(22) Date of filing: **24.01.2023**

(51) International Patent Classification (IPC):
***A61K 36/05*** *(2006.01)* ***A61K 9/51*** *(2006.01)*
***C12N 15/88*** *(2006.01)* ***C12N 15/00*** *(2006.01)*
***A61K 48/00*** *(2006.01)* ***A61P 1/00*** *(2006.01)*

(52) Cooperative Patent Classification (CPC):
**A61K 9/5176; A61K 9/0043; A61K 9/0073; A61K 31/713; A61K 36/05; A61K 39/0008; A61K 45/06; A61P 1/00; C12N 15/88;**
A61K 2039/53; A61K 2039/543; A61K 2039/55555; A61K 2039/55566; A61K 2039/55577

(86) International application number:
**PCT/EP2023/051650**

(87) International publication number:
**WO 2023/144127 (03.08.2023 Gazette 2023/31)**

(54) **EXTRACELLULAR VESICLES FROM MICROALGAE, THEIR BIODISTRIBUTION UPON ADMINISTRATION, AND USES**

EXTRAZELLULÄRE VESIKEL AUS MIKROALGEN, IHRE BIOVERTEILUNG BEI VERABREICHUNG UND VERWENDUNGEN

VÉSICULES EXTRACELLULAIRES PROVENANT DE MICROALGUES, LEUR BIODISTRIBUTION SUITE À LEUR ADMINISTRATION, ET LEURS UTILISATIONS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.01.2022 US 202263305230 P**
**20.07.2022 US 202263368975 P**
**24.10.2022 US 202263418959 P**

(43) Date of publication of application:
**11.12.2024 Bulletin 2024/50**

(60) Divisional application:
**26179984.5**

(73) Proprietor: **AGS Therapeutics SAS**
**75003 Paris (FR)**

(72) Inventors:
- **DRITTANTI, Lila**
**75011 Paris (FR)**
- **VEGA, Manuel**
**75011 Paris (FR)**

(74) Representative: **Boult Wade Tennant LLP**
**Salisbury Square House**
**8 Salisbury Square**
**London EC4Y 8AP (GB)**

(56) References cited:
**EP-A1- 3 967 745** **EP-A1- 3 967 746**
**WO-A1-2017/161010**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PICCIOTTO S. ET AL.: "Isolation of extracellular vesicles from microalgae: towards the production of sustainable and natural nanocarriers of bioactive compounds", vol. 9, no. 8, 20 April 2021 (2021-04-20), GB, pages 2917 - 2930, XP055979612, ISSN: 2047-4830, Retrieved from the Internet <URL:https://pubs.rsc.org/en/content/articlepdf/2021/bm/d0bm01696a> DOI: 10.1039/D0BM01696A**
- **KURUVINASHETTI K. ET AL.: "Algal Extracellular Vesicles for Therapeutic Applications", 2020 IEEE 20TH INTERNATIONAL CONFERENCE ON NANOTECHNOLOGY (IEEE-NANO), IEEE, 29 July 2020 (2020-07-29), pages 354 - 357, XP033817380, DOI: 10.1109/NANO47656.2020.9183452**


Remarks:

The complete document including Reference Table(s) and the Sequence Listing(s) can be downloaded from the EPO website

## Description

Provided are compositions containing extracellular vesicles from microalgae (MEVs) of the family Chlorellaceae that are exogenously loaded with bioactive cargo and uses thereof to deliver to various tissues and organs. The MEVs contain bioactive cargo, such as therapeutics, and the MEVs can serve to deliver cargo to particular organs and tissues, for treatment of diseases, disorders, and conditions.

## Background

Extracellular vesicles (EVs) are natural particles produced by most cells. EVs include exosomes (generally about 30-150 nm in size), which are released to the extracellular environment upon fusion of multivesicular endosomes with the plasma membrane, and include microvesicles (about 50-1000 nm), which are produced by the outward budding of membrane vesicles from the cell surface. Exosomes and microvesicles have similar properties, and in general are referred to as EVs.

EVs facilitate intercellular communication via cell-cell transfer of proteins and nucleic acids, such as microRNAs (miRNAs), long noncoding RNAs (lncRNAs), and mRNAs. By virtue of this, EVs derived from mammals and plants have been used as carriers for short interfering RNA (siRNA) delivery, microRNA (miRNA), and small molecule drugs. They are a promising delivery vehicle. There is a need for conveniently produced EVs that are readily delivered to cells and tissues. It is an object herein to provide such EVs.

## Summary

Provided are cargo-loaded extracellular vesicles (EVs) from microalgae of the family Chlorellaceae for use for administration to subjects *in vivo* and cells and cell lines *in vitro.* EVs are loaded with cargo that includes bioactive molecules, including biomolecules and small molecules, such as diagnostic and/or therapeutic molecules. The EVs herein are from microalgae. Microalgae are unicellular green algae, and include those that belong to the order *Chlorellales,* in particular the *Chlorellaceae* family, and in particular those that belong to the *Chlorella* genus, such as *Chlorella vulgaris*. Microalgae extracellular vesicles (MEVs) can be manufactured on a large scale. MEVs are exogenously loaded with the bioactive molecule cargo. The MEVs can be endogenously loaded (endo-loaded) by producing them in genetically-modified microalgae that encode or express proteins, polypeptides, small peptides, various RNA molecules and/or other biomolecules that the microalgae can be genetically programmed to express and thereby package in MEVs.

The MEVs can be exogenously loaded following isolation or partial purification/isolation of the MEVs from microalgae by contacting the MEVs with the cargo to produce the compositions in which substantially all of the MEVs have substantially the same exogenously-loaded heterologous cargo. The biodistribution pattern does not depend upon the manner in which the MEVs are loaded (see *e.g.,* Example 14, in which exogenously and endogenously loaded MEVs (as a control) deliver biologically active cargo).

The MEVs provided herein have unique biodistribution patterns, which are a function of the route of administration. Biodistribution of the MEVs is different from mammalian EVs and other EVs and/or nanoparticles. For example, systemically delivered mammalian EVs accumulate in the liver, kidneys and spleen. Some mammalian-derived secreted EVs have limited pharmaceutical acceptability (see, *e.g.,* International PCT Publication No. WO2021/122880). While others have shown that certain photosynthetic microalgae release EVs into growth medium, there is no description or understanding of the use of such EVs as drugs or as drug delivery vehicles; there is no description of or understanding of their fate upon administration. It is shown herein that MEVs upon administration via various routes are distributed to organs and tissues differently from mammalian EVs. As one example, while mammalian EVs, with the exception of bovine milk EVs, cannot be administered orally because they do not survive the harsh environment of the stomach, MEVs can be orally administered and delivered to the intestine, from where they traffic to the spleen, including the white spleen. It is shown herein, for example, that intranasally administered MEVs follow unique trafficking patterns and traffic to specific areas of the brain.

As shown and described herein, MEVs, upon intranasal (IN) administration, traverse unique pathways to the brain. Upon IN administration, the MEVs are internalized by olfactory sensory neurons (OSN) from where they travel to the glomeruli. MEVs arriving to the glomeruli from the olfactory sensory neurons (OSN) enter the mitral neurons and tufted neurons and travel intracellularly following a clear pathway with clear kinetics throughout the lateral olfactory tract (LOT). LOTs are composed of the long axons of mitral and tufted neurons that travel from the olfactory bulb (OB) to various anterior - posterior brain regions directly involved in the olfactory network of connections, which include the: anterior olfactory nucleus, olfactory tubercle, tenia tecta, piriform cortex, amygdala, and entorhinal cortex. Lateral ramifications of the main long axons of the mitral/tufted neurons enter and colonize each of the brain regions, the anterior olfactory nucleus, olfactory tubercle, tenia tecta, piriform cortex, amygdala, and entorhinal cortex. Inside these regions, the mitral/tufted axons are connected (via synapses) with neurons from other regions (having a more secondary olfactory role), including

the frontal cortex, the hypothalamus, the thalamus, and the hippocampus.

Regions reached by MEVs via IN administration reach all and each of the brain regions connected to the olfactory nerve and the lateral olfactory tract (LOT) in both hemispheres; ventral, lateral and dorsal regions; external and internal regions; along the antero-posterior axis. These regions are: the anterior olfactory nucleus, the olfactory tubercle, the tenia tecta, the piriform cortex, the amygdala, the entorhinal cortex, the primary motor cortex, the frontal cortex, the agranular insular cortex, the primary somatosensory cortex, the auditory cortex, the retrosplenial granular cortex, the temporal association cortex, the basolateral amygdaloid nucleus, the arcuate hypothalamic nucleus, the corpus callosum, the internal capsule, the thalamus, and the hippocampus (fimbria, dentata gyrus).

The MEVs are loaded with a variety of cargos (also referred to as "payloads"), including, but not limited to, RNA, such as inhibitory RNAs and other RNA products, oligonucleotides, plasmids, peptides, proteins, and/or small molecules. As shown herein, the MEVs can deliver the cargo to organs, tissues, and cells, and can be targeted by the route of delivery, where they can be delivered. It is shown herein that the MEVs, including the *Chlorella* MEVs, have a striking capacity to pass through stringent natural barriers, such as the digestive tract, and olfactory neurons, that are not shared by other extracellular vesicles (EVs) from other sources, including mammalian EVs.

As described herein, the MEVs can be exogenously loaded (exo-loaded) with a diversity of biologically active molecules, such as siRNA, mRNA, plasmids, ASO, peptides, proteins, and/or small molecules, which allows for a variety of therapeutic, diagnostic, and other uses. The MEVs also can be loaded endogenously by the microalgae in which they are produced (see, description herein, see, also, U.S. provisional application Serial No. 63/349,006, filed on June 03, 2022). As shown herein, MEV biodistribution is determined by the route of administration. Thus, MEVs can deliver their cargo to a variety of tissues and organs, including, for example, to the lungs, to the intestine, to the GALT, to the spleen, to the liver, and to the brain, depending on whether they are administered intratracheally, orally, intravenously or intranasally.

As demonstrated herein the MEVs have many uses, including therapeutic uses, including delivery of therapeutics for treatment and/or prevention (including reducing the risk or severity) of diseases, disorders, and conditions. These uses include therapeutic uses, including immunomodulation, immuno-oncology, treatment of genetic or metabolic disorders, neurologic disorders, psychiatric disorders, respiratory disorders, among others.

Cargos (also referred to as "payloads"), include, but are not limited to, RNA, such as inhibitory RNAs and other RNA products, oligonucleotides, plasmids, peptides, proteins, and small molecules. Exogenously-loaded MEVs can be loaded with almost any molecule of interest; endogenously-loaded MEVs, where the microalgae cells are genetically-modified to express or encode a product produce MEVs that contain cargo, such as RNA, DNA, peptides, small peptides, polypeptides, and proteins that are produced and packaged in EVs by the microalgae.

The MEVs can deliver the cargo to organs, tissues, and cells, and can be targeted by the route of delivery, where they can be delivered. It is shown herein that the MEVs, including the *Chlorella* MEVs, have a striking capacity to pass through stringent natural barriers, such as the digestive tract, and olfactory neurons, that is not shared by other extracellular vesicles (EVs) from other sources, including mammalian EVs.

Provided are compositions that contain MEVs, such as exogenously cargo-loaded MEVs, particularly those produced by the order *Chlorellales,* in particular the *Chlorellaceae* family, and in particular the *Chlorella* genus, such as *Chlorella vulgaris.* The compositions include pharmaceutical compositions that can be formulated for a particular route of delivery.

Methods for loading the MEVs are described and provided. The cargos are bioactive molecules or combinations thereof, including biomolecules and small molecules. The cargos include, for example, biomolecules, including biopolymers, such as DNA and RNA, proteins, protein complexes, protein-nucleic acid complexes, plasmids, and also include small molecules, such as small molecule drugs. The bioactive molecules include therapeutics, such as anti-cancer compounds and biomolecules, such as RNAi, oligonucleotides, and proteins, and complexes, and diagnostic molecules, such as detectable markers, molecules that are cosmetics, and molecules that act as anti-infectives for humans, animals and plants. Methods of treatment of diseases and disorders, including pathogen infections and cancers, and uses for the MEVs for treatment for the diseases and disorders are provided as are methods of diagnosis.

Cargo-loaded MEVs have applications in a variety of fields, including diagnosis, prophylaxis, treatment of human and other animal diseases, industrial uses, cosmetic uses, veterinary uses, and for use in the crop industry. The MEVs, with appropriate cargo for each application, can be used, for example, as vaccines, as gene therapy delivery vectors, for gene silencing, for gene editing, for transfection for industry and research, for analytical methods, for cell-based assays, and for other uses and applications. The cargo-loaded MEVs can be used for treatment of diseases, disorders, and conditions, and for industrial, and cosmetic uses. Diseases, disorders, and conditions, include, but are not limited to: genetic disorders, disorders of the digestive tract, disorders of the respiratory tract, disorders of the central nervous system (CNS), disorders of the skin, including natural disorders, and disorders induced by trauma, disorders of the urogenital tract, disorders of the naso-buccal cavity, disorders of the cardio-vascular system, immune and immunomodulatory disorders, cancers, ocular disorders, disorders of the liver, systemic disorders, and diseases, disorders, and conditions caused by or involving a pathogen, such as a bacterium, virus, or parasite.

Target tissues for treatment and/or delivery include, for example, epithelia and mucosa cells *(e.g.,* any kind of

either external or internal mucosa: mouth, gut, uterus, trachea, bladder, and others), endothelial cells, sensory cells (e.g., visual, auditory), cancer cells, tumor cells, blood cells, blood cell precursors, neural system cells *(e.g.,* neurons, glial cells and other CNS and peripheral nervous cells), cells of the immune system (e.g., lymphocytes, immuno-regulatory cells, effector cells), germ cells, secretory cells, gland cells, muscle cells, stem cells, including, for example, embryonic or tissue specific stem cells, liver cells, infected cells, such as cells infected with virus, bacteria, fungi, or other pathogens, native cells, and NS genetically engineered cells.

Provided are compositions that contain isolated microalgae extracellular vesicles (MEVs), where the microalgae is a species of the genus *Chlorella;* and the composition is formulated for administration to a subject. The *Chlorella* extracellular vesicles can contain a heterologous bioactive cargo molecule that has been introduced into the isolated extracellular vesicles, whereby the vesicles in the composition that contain heterologous bioactive molecule cargo contain the same bioactive molecule cargo, where: the cargo molecule is heterologous to *Chlorella;* and the bioactive cargo is a biomolecule or a small molecule.

For all embodiments, the *Chlorella* is any species of *Chlorella,* such as, but not limited to, *Chlorella* selected from among *Chlorella ellipsoidea, Chlorella pyrenoidosa, Chlorella sorokiniana, Chlorella vulgaris,* and *Chlorella variabilis.* In particular embodiments, the *Chlorella* is *Chlorella vulgaris.*

Provided are compositions that contain isolated microalgae extracellular vesicles (MEVs), where the microalgae is a species of *Chlorella;* the MEVs in the composition contain heterologous bioactive molecule cargo that has been introduced into the isolated MEVs, whereby the vesicles in the composition that contain the heterologous bioactive molecule cargo contain the same cargo. The cargo is heterologous, not endogenous, to *Chlorella;* and the cargo is a biomolecule or a small molecule drug. Each of the MEVs that contain cargo can comprise a plurality of different heterologous cargos.

The cargo includes any molecules that are intended for delivery to or on a plant or animal. In general, the cargo is bioactive. Bioactive cargo includes, for example, any molecules, such as biomolecules, including biopolymers, and small molecules, that can have an effect on a plant or animal when administered. Cargo includes, for example, proteins, peptides, and nucleic acids. The bioactive molecules can be synthetic, naturally-occurring, and/or modified to alter a property or activity. Included are any molecules that have been used as drugs or therapeutics or diagnostics or cosmetics or in industry. The cargo can be, but is not limited to, a therapeutic for treating or preventing a disease or disorder or condition, or treating or preventing a symptom thereof. The cargo can be a nucleic acid molecule, a polypeptide, a protein, a plasmid, an aptamer, or an antisense oligonucleotide.

The cargo in the MEVs in the compositions can comprise a biopolymer. Biopolymers include a naturally-occurring biopolymers, or synthetic biopolymers, or modified biopolymers. The biopolymer can be a nucleic acid or protein that includes modifications, where the modifications comprise insertions, deletions, replacements, and transpositions of nucleotides or amino acid residues, and/or, where the biopolymer is a protein, the modifications also can comprise post-translational modifications. Post-translational modifications include, but are not limited to, glycosylation, hyper-glycosylation, PEGylation, sialylation, albumination, other half-life extending moieties, and other modifications that improve or alter pharmacological dynamic or kinetic properties of the protein.

Nucleic acids, such as DNA and RNA, are among the molecules that can be cargo. If the cargo is RNA or protein, it can be provided as the cargo or it can be encoded by nucleic acid that then is expressed in the organism to whom it is administered. Exemplary of RNA is inhibitory RNA (RNAi) and mRNA, including modified mRNA. RNAi includes, for example, silencing RNA (siRNA) or short-hairpin RNA (shRNA), micro-RNA (miRNA), short activating RNA (saRNA), and long non-coding RNA (lncRNA). RNA products also include double stranded RNA and ribozymes. The cargo also can be an oligonucleotide, such as an anti-sense oligonucleotide or an allele-specific oligonucleotide. The cargo can comprise a gene editing system, such as a CRISPR-Cas system, and modified and improved gene editing systems, such as CRISPR-associated and CRISPR-like systems (see, *e.g.,* published US patent application Nos. 20200332273 and 20200332274 each to Applicant Metagenomi).

The cargo includes therapeutic or diagnostic or theragnostic proteins or peptides, protein complexes, such complexes that contain two or more proteins or a protein and nucleic acid, or a protein and aptamer, or combinations of proteins, nucleic acids, and other molecules. The cargo can be or can encode a protein that is an antibody or antigen-binding fragment thereof. Antibodies can be of any form, including single chain forms, nanobodies, camelids, and other forms, such as an scFv, a bi-specific antibody, or an antigen-binding fragment thereof. Antibodies and antigen-binding fragments thereof include a checkpoint inhibitor antibody or antigen-binding fragment thereof, or a tumor antigen-specific antibody or antigen-binding fragment thereof, or an anti-oncogene specific antibody or antigen-binding fragment thereof, or a tumor-specific receptor or signaling molecule antibody or antigen-binding fragment thereof. Exemplary antibodies and antigen-binding fragments thereof specifically bind to and inhibit one or more of CTLA-4, PD-1, PD-L1, PD-L2, the PD-1/PDL1 pathway, the PD-1/PDL2 pathway, HER2, EGFR, TIM-3, LAG-3, BTLA-4, HHLA-2, CD28, and other checkpoints or immune suppressors, or tumor antigens.

The cargo in the MEVs in the compositions can include immune stimulating products, or antigens, and can be used as a vaccine to induce an immunoprotective response upon administration. The cargo can be a DNA, RNA, protein, or

virus. The cargo can contain nucleic acid or protein or a nucleic acid encoding a protein that is a therapeutic product for treatment of cancer, or an infectious disease, or a neurodegenerative disease or other CNS disorder, or aging, or aging associated disease, or ophthalmic disorders, or immunological disorders. The cargo can be a cosmeceutical or a cosmetic or cosmetically active product. The cargo can comprise a small molecule bioactive molecule, such as a small molecule bioactive molecule, such as a small molecule drug. Exemplary drugs include chemotherapeutics and prodrugs. The cargo in the MEVs in the compositions can be or comprise a diagnostic marker or detectable product, such as, but not limited to, luciferase or nucleic acid encoding the luciferase, a fluorescent protein or nucleic acid encoding a fluorescent protein, or a luciferase operon.

The cargo can comprise DNA. The DNA can be a plasmid, such as one that encodes a product for expression in the animal or plant to which it is administered. Exemplary products include therapeutic products and diagnostic products. These include proteins and RNA products, including the RNA products listed above. Since the MEVs are intended for administration to animals and plants, the plasmids generally encode the product under control of eukaryotic regulatory signals and sequences, including eukaryotic promoters and translation sequences, such as RNA polymerase II and III promoters. Exemplary promoters include RNA polymerase II promoters, such as from animals, plants, and plant or animal viruses. Exemplary promoters, include, but are not limited to, a cytomegalovirus promoter, a simian virus 40 promoter, a herpes simplex promoter, an Epstein Barr virus promoter, an adenovirus promoter, a synthetic promoter, an actin promoter, and synthetic chimeric promoters. Other eukaryotic transcription sequences and eukaryotic translation sequences, include, but are not limited to, one or more of an enhancer, a poly A sequence, and/or an internal ribosome entry site (IRES) sequence.

The plasmids can encode one or two or more cargo products. For expression of the cargo product the encoding nucleic acid is operably linked to regulatory sequences recognized by a eukaryotic cell.

Methods of preparing the MEVs are provided. The methods include introducing the cargo into isolated MEVs. The cargo includes any molecule for whom delivery into or onto an animal or plant is desired. Generally, the cargo is or contains or provides a bioactive molecule product, including small molecules and biopolymers. The biopolymers are naturally-occurring, or synthetic, or modified, or combinations thereof. The cargo includes a protein, nucleic acid, or small molecule. The cargo can be loaded into the MEVs by any method known to those of skill in the art; these methods include, for example, one or more of electroporation, sonication, extrusion, and use of surfactants. In some embodiments the MEVs are from *Chlorella,* such as but not limited to a species of *Chlorella* selected from among *Chlorella ellipsoidea, Chlorella pyrenoidosa, Chlorella sorokiniana, Chlorella vulgaris,* and *Chlorella variabilis.* The MEVs produced by the methods and any of the MEVs provided herein, including the compositions containing the MEVs can be used as one or more of: a method of diagnosis, a vaccine, a therapy for treatment, a diagnostic of a disease, a treatment of a disease or disorder or condition, a cosmetic, an industrial application, and/or any use known to those of skill in the art.

The MEVs can be used in any such method, which include methods of treatment of a disease, disorder, or condition. Exemplary of diseases, disorders, and conditions is cancer, such as a cancer that comprises a solid tumor or a hematological malignancy, or metastases thereof. Other diseases, disorders, and conditions include those of or involving the respiratory system, of or involving the central nervous system or the nervous system, of or involving the skin and exposed epithelia or mucosa, of or involving the digestive tract, and of or involving an infectious agent. Infectious agents include bacteria, viruses, parasites, prions, oomycetes, and fungi.

The cargo can provide therapeutic molecules for treatment, or can induce an immune response to serve as a vaccine. The MEVs can contain a cargo that comprises an immunostimulatory protein or an antigen or encodes an immunostimulatory protein or antigen, whereby the MEVs, upon administration are immunostimulating and elicit an innate or adaptive immune response, or the MEVs and/or the cargo can elicit an immunoprotective response to prevent or treat a disease or disorder or condition. The MEVs can be used to treat a disease, disorder, or condition resulting from trauma. Trauma includes, but is not limited to, trauma from or involving wounds, burns, surgery, skin cuts, broken bones, hair loss, dermis exposure, mucosal exposure, fibrosis, lacerations, and ulcerations. The MEVs can be used to elicit an effect to treat a condition resulting from natural aging, or pathogenic or disease or otherwise induced aging. Other diseases, disorders, and conditions that can be treated by the MEVs, are diseases, disorders, and conditions of the skin or the eye. These include dermatitis, wrinkles and/or other age-related changes in the skin, macular degeneration, glaucoma, diabetic retinopathies, cataracts, or conditions resulting from diabetic retinopathy.

The compositions containing the MEVs can be formulated for administration by any route of administration. Routes include, but are not limited to, local, systemic, topical, parenteral, enteral, mucosal, inhalation into the lung or intranasal, vaginal, rectal, aural, oral, and other routes of administration. The MEVs can be formulated in any form, including as a tablet; as a liquid, such as an emulsion; as a powder; as a cream; as a gel; or as an aerosol; the form and formulation respective to the route of administration including for oral administration, for nebulization, or for inhalation.

The compositions or MEVs can be used in any of the methods and treatments described herein or known to those of skill in the art. Methods include, for example, any described herein, including, for example, for use for one or more of gene silencing, gene interference, gene therapy, gene/protein overexpression, gene editing, inhibition or stimulation of protein activity, and pathway signaling. The compositions and MEVs can be used for prophylaxis and/or vaccination. They

can be used in agro-veterinary applications, such as crop sciences, phytopathogens, and animal diseases, for dermatological applications, and for cosmetic applications. They can be used for industrial purposes, for example for manufacturing, characterization, and calibration.

Provided are methods for treating diseases, disorders, and conditions in which treatment can be effected by delivery of active agents to the brain. Provided are compositions prepared for intranasal delivery. The compositions contain microalgae extracellular vesicles that contain the active agent. The microalgae extracellular vesicles can be loaded by any suitable method (see, methods and MEVs described in International Patent Publication No. PCT/EP2022/070371, U.S. provisional application Serial No. 63/349,006), which include exogenous loading following production of the MEVs and also endogenous loading *in vivo* by microalgae genetically modified to package nucleic acids or encoded products into MEVs.

The EVs are from microalgae, which are unicellular green algae, and include those that belong to the order *Chlorellales,* in particular the *Chlorellaceae* family, and in particular those that belong to the *Chlorella* genus, such as *Chlorella vulgaris*. The MEVs are provided in compositions formulated for nasal administration. The MEVs can be loaded exogenously after isolation, or can be endogenously loaded by genetically modified microalgae that encode and package heterologous nucleic acid and/or proteins in the MEVs *in vivo.* An advantage of exogenously loading (exo-loading) cargo into MEVs is that the amount of cargo/MEV can be controlled, and distribution of the exogenous cargo in the MEVs is predictable, and substantially uniform, such that the average cargo molecule or amount of cargo/MEV can be known. A large variety of bioactive molecules, including biomolecules and small molecules, such as drugs and organic compounds, can be loaded into the MEVs. The MEVs also can be endogenously loaded by genetically modified microalgae to package heterologous nucleic acids and/or proteins.

The resulting MEVs, whether endo- or exo-loaded are not toxic; they can be administered into cells *in vitro,* or can be administered *in vivo* and have distribution patterns that depend upon the route of administration. For purposes herein, the MEVs are for delivery to the brain by intranasal administration.

It is shown herein that MEVs traffic to the brain via unique pathways and mechanisms following intranasal (IN) administration. These pathways and mechanisms are not shared by exosomes from other sources nor by nanoparticles. It is shown herein that following intranasal delivery, the MEVs traffic via the olfactory nerve and throughout the lateral olfactory tract (LOT) to a large number of interconnected brain regions. The MEVs traffic via intraneuronal axonal transport and also transport between neurons across synapses. The MEVs have the ability to cross-over synapses: at least over (i) the synapses between the olfactory sensory neurons (OSN) and the mitral/tufted neurons, (ii) the synapses between the mitral/tufted neurons and the local neurons in the various brain regions colonized by the lateral olfactory tract (LOT), and (iii) the synapses between the neurons in the brain regions colonized by the LOT and neurons from the frontal cortex, the hippocampus, the thalamus, and the hypothalamus.

The biodistribution of MEVs follows pathways and connections in the neural network of the olfactory nerve, and the mitral/tufted neurons throughout the entire brain. The trafficking and pathways provide access (biodistribution) to brain regions (within 1 and 16 hours after IN administration) that include: anterior olfactory nucleus, olfactory tubercle, tenia tecta, piriform cortex, amygdala, entorhinal cortex, primary motor cortex, frontal cortex, agranular insular cortex, primary somatosensory cortex, auditory cortex, retrosplenial granular cortex, temporal association cortex, basolateral amygdaloid nucleus, arcuate hypothalamic nucleus, corpus callosum, internal capsule, thalamus, hippocampus (fimbria, dentate gyrus). Thus, the MEVs can deliver active agents, particularly biologically active payloads, to specific regions of the brain. Payloads also can include, but are not limited to, proteins, mRNA, DNA, small molecules, any agent that can be exogenously loaded (exo-loaded) into MEVs, or that can be packaged in MEVs *in vivo* by microalgae, particularly genetically modified microalgae that encode or produce the agent. The MEVs, thus, provide for effective delivery of bioactive small molecules, including lipophilic small molecules, proteins, DNA and mRNA to neurons, astrocytes, glial cells, and neural stem cells. *In vivo* MEVs provide for therapeutic and diagnostic uses and for diagnostic and experimental uses. Delivery is exemplified in the Examples, which show effective delivery and expression of various exemplary cargo, such as catalase, GFP, luciferase, nerve growth factors (NGFs), TrkA (tropomyosin kinase A), neurotrophic factors (NT-3, NT-4, BDNF (brain derived neurotrophic factor, CNTF (ciliary neurotrophic factor), EPO, IGF-1, bFGF (basic fibroblast growth factor), hGH, psilocybin/psilocin, harmine, temozolomide, rivastigmine, and/or rhodamine, to neurons, astrocytes, glial cells and/or neural stem cells, *in vitro* and *in vivo*.

The MEV is a unique vehicle for delivery of cargo to specific tissues, which delivery depends upon the route of administration. Trafficking of MEVs *in vivo,* as shown, can be distinct from MEVs from other sources. For example, in contrast to mammalian EVs, MEVs can be orally administered and traffic through the GALT. MEVs that are administered intranasally traffic unique pathways to the brain, and, for example, can be used for: (i) the treatment and/or the prevention of brain disorders, including, but not limited to, cognitive, emotional, behavioral, psychiatric, neurologic, degenerative, and cancer; (ii) for the study, *in vitro* or *in vivo,* of brain disorders; (iii) for the diagnosis of brain disorders; and (iv) for recreational and therapeutic uses.

**MEVs and delivery to the brain**

For purposes herein, the MEVs are for delivery to the brain by intranasal administration. The MEVs can be used to deliver bioactive cargo for treatment, including the prevention or reducing the risk of diseases, disorders, and conditions of the brain or involving the brain, and/or for detection or diagnosis of a disease, disorder, or condition of or involving the brain, and/or to monitor treatment of such diseases, disorders, and conditions.

In general provided are compositions and drug delivery systems for delivery to the brain. As described above and herein, intranasal administration effects delivery to the brain. It also is shown herein that MEVs traffic to the brain via unique pathways and mechanisms following intranasal (IN) administration. These pathways and mechanisms are not shared by exosomes from other sources nor by nanoparticles. It is shown herein that following intranasal delivery, the MEVs traffic via the olfactory nerve and throughout the lateral olfactory tract (LOT) to a large number of interconnected brain regions. The MEVs traffic via neuronal axonal transport. The MEVs have the ability to cross-over synapses: at least over (i) the synapses between the olfactory sensory neurons (OSN) and the mitral/tufted neurons, (ii) the synapses between the mitral/tufted neurons and the local neurons in the various brain regions colonized by the lateral olfactory tract (LOT), and (iii) the synapses between the neurons in the brain regions colonized by the LOT and neurons from the frontal cortex, the hippocampus, the thalamus, and the hypothalamus.

Provided are compositions and uses thereof, where the compositions comprise microalgae extracellular vesicles (MEVs that deliver a bioactive molecule to the brain following intranasal administration or to cells *in vitro,* wherein the MEVs comprise the bioactive molecule as cargo. Methods of delivery of a bioactive molecule to the brain by intranasally administering a composition comprising microalgae extracellular vesicles (MEVs) that comprise cargo that is a bioactive molecule are provided.

Also provided are cell compositions and cell therapy compositions that contain MEVs in which the cargo is for treatment of diseases, disorders, and conditions of particular organs, tissues, and systems. The cell compositions can be prepared *in vitro* by introducing MEVs containing cargo for treating the particular disease, disorder, or condition, and then administering the cell therapy to cells. For example, the compositions containing MEVs can be administered *in vitro* to neurons, astrocytes, glial cells, and/or neural stem cells; the resulting cells can be used for cellular therapy, such as by administration to the brain.

For all embodiments of the methods and compositions described and contemplated herein, diseases, disorders, and conditions include any described herein and known to those of skill in the art that can be treated, detected, diagnosed, and/or monitored by delivery of a molecule to the brain by intranasal administration.

The biodistribution of MEVs follows pathways and connections in the neural network of the olfactory nerve, and the mitral/tufted neurons throughout the entire brain. The trafficking and pathways provide access (biodistribution) to brain regions (within 1 and 16 hours after IN administration) that include: anterior olfactory nucleus, olfactory tubercle, tenia tecta, piriform cortex, amygdala, entorhinal cortex, primary motor cortex, frontal cortex, agranular insular cortex, primary somatosensory cortex, auditory cortex, retrosplenial granular cortex, temporal association cortex, basolateral amygdaloid nucleus, arcuate hypothalamic nucleus, corpus callosum, internal capsule, thalamus, hippocampus (fimbria, dentate gyrus). Thus, the MEVs can deliver active agents, particularly biologically active payloads, to specific regions of the brain. Payloads may also include, but are not limited to, proteins, mRNA, DNA, small molecules, and any agent that can be exogenously loaded (exo-loaded) into MEVs, or that can be packaged in MEVs *in vivo* by microalgae, particularly genetically modified microalgae that encode or produce the agent. The MEVs, thus, provide for effective delivery of bioactive small molecules, including lipophilic small molecules, proteins, DNA and mRNA to neurons, astrocytes, glial cells, and neural stem cells. *In vivo* MEVs provide for therapeutic and diagnostic uses and for diagnostic and experimental uses. Delivery is exemplified in the Examples, which show effective delivery and expression of catalase, GFP, luciferase, nerve growth factors (NGFs), TrkA (tropomyosin kinase A), neurotrophic factors (NT-3, NT-4, BDNF (brain derived neurotrophic factor), CNTF (ciliary neurotrophic factor), EPO, IGF-1, bFGF (basic fibroblast growth factor), hGH), psilocybin/psilocin, harmine, tomozolonide, rivastigmine, and rhodamine, to neurons, astrocytes, glial cells, and/or neural stem cells, *in vitro* and *in vivo.*

The MEVs provide a unique vehicle for, among many uses and methods, i) the treatment and/or the prevention of brain disorders, including, but not limited to, cognitive, emotional, behavioral, psychiatric, neurologic, degenerative, and cancer; (ii) for the study, *in vitro* or *in vivo,* of brain disorders; (iii) for the diagnosis of brain disorders; and (iv) for recreational and therapeutic uses.

Provided are methods of delivery of a bioactive molecule to the brain, by intranasally administering a composition comprising microalgae extracellular vesicles (MEVs) containing cargo that comprises the bioactive molecule, whereby the MEVs travel to the brain via the olfactory nerve and throughout the lateral olfactory tract (LOT) to interconnected brain regions for delivery to one or more of the olfactory bulb, anterior olfactory nucleus, olfactory tubercle, tenia tecta, piriform cortex, amygdala, entorhinal cortex, primary motor cortex, frontal cortex, agranular insular cortex, primary somatosensory cortex, auditory cortex, retrosplenial granular cortex, temporal association cortex, basolateral amygdaloid nucleus, mammillary body, arcuate hypothalamic nucleus, corpus callosum, internal capsule, thalamus, and hippocampus, where:

the bioactive molecule is any molecule that can effect treatment of a disease, disorder, or condition, or that can be used to detect a disease, disorder, or condition, or that can be used to monitor treatment of a disease, disorder, or condition; and the bioactive molecule is heterologous to the microalgae and/or the MEVs.

The methods of delivery include methods of treatment. Provided are methods of treating or treatment of a disease, disorder, or condition of the brain or a disease, disorder, or condition involving the brain, by intranasally administering a composition comprising microalgae extracellular vesicles (MEVs) containing cargo that comprises a bioactive molecule, whereby the MEVs travel to the brain via the olfactory nerve and throughout the lateral olfactory tract (LOT) to interconnected brain regions for delivery to one or more of the olfactory bulb, anterior olfactory nucleus, olfactory tubercle, tenia tecta, piriform cortex, amygdala, entorhinal cortex, primary motor cortex, frontal cortex, agranular insular cortex, primary somatosensory cortex, auditory cortex, retrosplenial granular cortex, temporal association cortex, basolateral amygdaloid nucleus, mammillary body, arcuate hypothalamic nucleus, corpus callosum, internal capsule, thalamus, and hippocampus, where: the bioactive molecule is any molecule that can effect treatment of a disease, disorder, or condition of the brain or involving the brain; and the bioactive molecule is heterologous to the microalgae and/or the MEVs.

Also provided are methods of detecting a disease, disorder, or condition of the brain or a disease, disorder, or condition involving the brain, or of monitoring treatment of disease, disorder, or condition of the brain, by intranasally administering a composition comprising an microalgae extracellular vesicles (MEVs) containing a bioactive molecule, whereby the MEVs travel to the brain via the olfactory nerve and throughout the lateral olfactory tract (LOT) to interconnected brain regions for delivery to one or more of the olfactory bulb, anterior olfactory nucleus, olfactory tubercle, tenia tecta, piriform cortex, amygdala, entorhinal cortex, primary motor cortex, frontal cortex, agranular insular cortex, primary somatosensory cortex, auditory cortex, retrosplenial granular cortex, temporal association cortex, basolateral amygdaloid nucleus, mammillary body, arcuate hypothalamic nucleus, corpus callosum, internal capsule, thalamus, and hippocampus (fimbria, dentata gyrus), wherein the bioactive molecule comprises a reporter or detectable marker, where: the bioactive molecule is any molecule that can be used to detect or diagnose a disease, disorder, or condition, or that can be used to monitor treatment of a disease, disorder, or condition, or that can be used to detect or diagnose a disease, disorder, or condition and treat the disease, disorder, or condition; the disease, disorder, or condition is a disease, disorder, or condition of the brain or involving the brain; and the bioactive molecule is heterologous to the microalgae and/or the MEVs.

Provided are compositions that comprise microalgae extracellular vesicles (MEVs) containing cargo comprising a bioactive molecule, where: the composition is formulated for intranasal delivery to the brain via the olfactory nerve and throughout the lateral olfactory tract (LOT) to interconnected brain regions for delivery to one or more of the olfactory bulb, anterior olfactory nucleus, olfactory tubercle, tenia tecta, piriform cortex, amygdala, entorhinal cortex, primary motor cortex, frontal cortex, agranular insular cortex, primary somatosensory cortex, auditory cortex, retrosplenial granular cortex, temporal association cortex, basolateral amygdaloid nucleus, mammillary body, arcuate hypothalamic nucleus, corpus callosum, internal capsule, thalamus, and hippocampus; the bioactive molecule is any molecule that can effect treatment of a disease, disorder, or condition, or that can be used to detect or diagnose a disease, disorder, or condition, or that can be used to monitor treatment of a disease, disorder, or condition, or detect, diagnose, monitor, and/or treat a disease, disorder, or condition; the disease, disorder, or condition is a disease, disorder, or condition of the brain or involving the brain; and the bioactive molecule is heterologous to the microalgae and/or the MEVs.

Also provided are compositions, comprising microalgae extracellular vesicles (MEVs) for use for delivering cargo comprising a bioactive molecule to the brain to treat a disease, disorder, or condition of the brain or involving the brain, or for diagnosing or detecting or monitoring treatment of a disease, disorder, or condition of the brain or involving the brain, or for treating, diagnosing, detecting, and/or monitoring a disease, disorder, or condition of the brain or involving the brain, where: the MEVs comprise the bioactive molecule for delivery to the brain; the composition is formulated for intranasal delivery brain via the olfactory nerve and throughout the lateral olfactory tract (LOT) to interconnected brain regions for delivery to one or more of the olfactory bulb, anterior olfactory nucleus, olfactory tubercle, tenia tecta, piriform cortex, amygdala, entorhinal cortex, primary motor cortex, frontal cortex, agranular insular cortex, primary somatosensory cortex, auditory cortex, retrosplenial granular cortex, temporal association cortex, basolateral amygdaloid nucleus, mammillary body, arcuate hypothalamic nucleus, corpus callosum, internal capsule, thalamus, and hippocampus; the bioactive molecule is any molecule that can effect treatment of a disease, disorder, or condition, or that can be used to detect a disease, disorder, or condition, or that can be used to monitor treatment of a disease, disorder, or condition; and the bioactive molecule is heterologous to the microalgae and/or the MEVs.

In accord with these methods and compositions the MEVs travel in the brain via intraneuronal axonal transport, and transport between neurons across synapses. For example, the MEVs travel in the brain by virtue of neuronal axonal transport, and the MEVs are delivered to the fimbria or dentata gyrus of the hippocampus. The MEVs follow the pathways and connections in the neural network comprising the olfactory nerve, and the mitral/tufted neurons throughout the entire brain. Upon intranasal administration, the MEVs traverse one or more of: (i) the synapses between the olfactory sensory neurons (OSNs) and the mitral/tufted neurons; (ii) the synapses between the mitral/tufted neurons and the local neurons in

the brain regions colonized by the LOT; and (iii) the synapses between the neurons in the brain regions colonized by the LOT and neurons from or to the frontal cortex, the hippocampus, the thalamus, and the hypothalamus. For example, the MEVs traverse (i), (ii), and (iii), or (i) and (ii), such as the pathway traversed by the MEVs upon intranasal administration depicted in Figure 35.

The methods and compositions provided herein are for intranasal administration. Following intranasal administration, the MEVs are delivered, for example, to or are for delivery to one or more of the corpus callosum, the dorsal fornix, the dorsal hippocampal commissure, and the fimbria of the hippocampus.

The compositions provided herein and the compositions used in the methods can be formulated as a suspension or as an emulsion, such as a nanoemulsion or as a microemulsion. Those of skill in the art understand and are familiar with the properties of nanoemulsions and microemulsions and their formation. In the compositions, the MEVs contain the bioactive cargo. For example, the MEVs can be prepared so that on the average each MEV contains a pre-determined amount of bioactive molecule, such as, for example, 1 to 100 of the bioactive molecules per MEV. The selection of amount of cargo per MEV is within the level of skill in the art and depends upon factors known to the skilled artisan, such as the particular a disease, disorder, or condition treated or the use of the MEVs, the subject, the particular cargo, and other such parameters and factors. Similarly the concentration of MEVs depends upon the particular cargo and use. For example, the concentration of MEVs in the composition can be about or at 0.1 to 10 mg/mL, and lower or higher, and intermediate concentrations. The compositions can be formulated for single dosage administration (direct administration without dilution), or multiple dose administration for administration in aliquots and/or for dilution to a desired concentration. Exemplary amounts of compositions for administration are 0.1 to 100 mL, such as 1 to 10 mL, 1 to 5 mL, 0.1 to 1 mL, and any suitable amount. The compositions can be administered as a single dose or as a series of doses or other regimen. The compositions can be administrated as part of a combination therapy protocol.

The compositions can be formulated, for example, as a liquid, a powder, troche, granules, a liquid, an oil, a suspension, or an emulsion, suitable for intranasal administration or processing, such as by dilution or dissolution for intranasal administration.

The compositions and methods include those in which the MEVs were endogenously loaded by genetically-modified microalgae that encode the bioactive molecule or a pathway for its production. The MEVs also include those in which the cargo was exogenously loaded in purified or partially purified MEVs. The MEVS can contain a plurality of different heterologous cargos. For purposes herein the cargo includes therapeutics for treating or preventing a disease or condition of the brain or involving the brain, or treating or preventing a symptom thereof.

The microalgae used to produce the MEVs for use in the methods can be microalgae from a division of microalgae selected from among Euglenophyta (Euglenoids), Chrysophyta (Golden-brown algae and Diatoms), Pyrrophyta (Fire algae), Chlorophyta (Green algae), Rhodophyta (Red algae), Phaeophyta (Brown algae), and Xanthophyta (Yellow-green algae). For example, the microalgae is a species of Chlorophyceae or Trebouxiophyceae or Chlorophyta, such as *Chlorella* or *Chlamydomonas.*

*Chlorella* species include, but are not limited to, *Chlorella ellipsoidea, Chlorella pyrenoidosa, Chlorella sorokiniana, Chlorella vulgaris,* and *Chlorella variabilis,* such as *Chlorella vulgaris* and *Chlorella variabilis.* In particular embodiments the *Chlorella* is *Chlorella vulgaris.* For example, the methods and compositions include those in which the microalgae is a species of *Chlorella;* the MEVs in the composition contain heterologous bioactive molecule cargo that has been exogenously introduced into the isolated MEVs, whereby on the average the vesicles in the composition that contain the heterologous bioactive molecule cargo contain the same heterologous cargo, where: the cargo is heterologous to *Chlorella;* and the cargo is a biomolecule or a small molecule drug or any cargo for delivery to the brain as described herein and/or known to those of skill in the art. Also included are methods and compositions in which the MEVs are *Chlorella* extracellular vesicles; the *Chlorella* extracellular vesicles comprise a heterologous bioactive molecule cargo that is endogenously introduced into the extracellular vesicles by the microalgae, wherein the cargo molecule is heterologous to *Chlorella;* and the bioactive cargo is a biomolecule for treating a disease, disorder, or condition of the brain or involving the brain.

Provided are methods and compositions, where: the MEVs are *Chlorella* extracellular vesicles; the *Chlorella* extracellular vesicles comprise a heterologous bioactive molecule cargo that has been introduced into isolated extracellular vesicles, whereby the vesicles in the composition that contain the heterologous bioactive molecule cargo contain, on average, the same bioactive molecule cargo, where: the cargo molecule is heterologous to *Chlorella;* and the bioactive cargo is a therapeutic or detectable molecule for treating, monitoring, and/or diagnosing a disease, disorder, or condition of or involving the brain. In other embodiments, the MEVs are *Chlorella* extracellular vesicles; the *Chlorella* extracellular vesicles comprise a heterologous bioactive molecule cargo that is endogenously introduced into the extracellular vesicles by the microalgae, whereby the vesicles in the composition that contain the heterologous bioactive molecule cargo contain the same bioactive molecule cargo, where: the cargo molecule is heterologous to *Chlorella;* and the bioactive cargo is a biomolecule or a small molecule. In other embodiments, the MEVs in the composition contain heterologous bioactive molecule cargo that has been exogenously introduced into the isolated MEVs, whereby on the average the vesicles in the composition that contain the heterologous bioactive molecule cargo contain the same cargo, where: the cargo is

heterologous to *Chlorella;* and the cargo is a biomolecule or a small molecule. In other embodiments the cargo is endogenously introduced into the MEVs by modifying the microalgae to express or produce the cargo, such as a nucleic acid or protein, or biochemical pathway product. In exemplary embodiments, the *Chlorella* is *Chlorella vulgaris.*

Cargo includes, but is not limited to, a biomolecule, a biopolymer, such as a naturally-occurring biopolymer, or is a synthetic biopolymer, or is a modified biopolymer, such as, for example, a nucleic acid molecule, a polypeptide, a protein, a plasmid, an aptamer, or an antisense oligonucleotide. Cargo includes, but is not limited to, DNA or RNA, such as, for example, inhibitory RNA (RNAi), mRNA or modified mRNA, silencing RNA (siRNA), short-hairpin RNA (shRNA), micro-RNA (miRNA), self-amplifying RNA, short activating RNA (saRNA), long non-coding RNA (lncRNA), a ribozyme, or a double-stranded RNA. Cargo includes oligonucleotides, such as an anti-sense oligonucleotide or an allele-specific oligonucleotide or an anti-sense oligonucleotide (ASO), a gene editing system, such as for example a CRISPR-CAS system, a CRISPR-associated or CRISPR-like system(s). The cargo can comprise DNA, such as a plasmid, where the plasmid encodes the therapeutic and/or detectable or diagnostic product, or an RNA product, such as RNAi and the forms of RNA noted above, including an anti-sense oligonucleotide or a ribozyme or a double-stranded RNA. The plasmid can encode the cargo product under control of a eukaryotic promoter, such as an RNA polymerase II or III promoter, such as a eukaryotic virus promoter, such as, for example, a cytomegalovirus promoter, a simian virus 40 promoter, a herpes simplex promoter, an Epstein Barr virus promoter, an adenovirus promoter, a synthetic promoter, other promoters, such as an actin promoter, or a synthetic chimeric promoter. The plasmid can also comprise other regulatory sequences for expression, such as other eukaryotic transcription sequences and eukaryotic translation sequences. The MEV cargo can comprise a small molecule for effecting treatment or detection or diagnosis or monitoring of a disease, disorder, or condition of the brain or involving the brain.

Cargo includes any molecule of interest for delivery to the brain. This includes, for example, cargo that encodes or is an immune modulator, such as, for example, an immunomodulatory agent to increase or decrease production of one or more cytokines; up- or down-regulate self-antigen presentation; mask MHC antigens; or promote the proliferation, differentiation, migration, or an activation state of one or more types of immune cells. The cargo can comprise or encode a hormone or a cytokine or a chemokine. The cargo can comprise a prodrug or a vector encoding an enzyme that converts a prodrug into a drug for treating a disease, disorder, or condition of the brain or involving the brain. The cargo can comprise or encode an antibiotic, anti-viral, anti-fungal, anti-parasitic, or other anti-infectious agent for treatment of infections of or involving the brain. The cargo can comprise a therapeutic nucleic acid or protein or a nucleic acid encoding a protein that is a therapeutic product for treatment of cancer or a tumor in the brain, or an infectious disease in the brain, or a neurodegenerative disease or other central nervous system (CNS) disorder, or for treating dementia. The cargo can comprise a chemotherapeutic drug for treating a disease, disorder, or condition of the brain or involving the brain, and/or encodes or comprises an antibody or antigen-binding fragment thereof, such as, for example, an scFv, a bi-specific antibody, or an antigen-binding fragment thereof. The cargo can comprise nucleic acid for gene therapy.

The MEVs can comprise two more cargo products. Cargo can comprise a therapeutic product, or a diagnostic product, or a detectable product, or combinations thereof, for detecting, diagnosing and/or monitoring a disease, disorder, or condition of the brain or involving the brain, or combinations thereof. The diagnostic can comprise a luciferase or nucleic acid encoding the luciferase, a fluorescent protein or nucleic acid encoding a fluorescent protein, or a luciferase operon, or combinations thereof. The bioactive molecule cargo can comprise any molecule that has an effect on a cell or organism to which it is delivered, or that is detectable or serves as a detectable marker or a biomarker, to thereby effect treatment, detection, diagnosis, or monitoring or treatment of a disease, disorder, or condition of the brain or involving the brain.

The cargo can comprise one or more of bioactive small molecules, peptides (polypeptides, proteins), RNAs (mRNAs, siRNAs, miRNAs, lncRNAs), DNAs (anti-sense oligonucleotide (ASOs), plasmids, DNA fragments), and gene editing complexes. The bioactive molecules can be a diagnostic, or a therapeutic, or a theragnostic for treating, diagnosing, detecting, and/or monitoring treatment of a disease, disorder, or condition of the brain or involving the brain. Cargo can comprise, for example, one or more of a hormone, a growth factor, an enzyme, an immunomodulatory compound, a receptor, a receptor agonist, or a receptor antagonist for treating a disease, disorder, or condition of the brain or involving the brain.

The disease, disorder, or condition can comprise a tumor in the brain. The cargo can comprise, for example, an oncolytic virus that infects glial tumors, or can comprise a therapeutic for treatment of glial tumors. The disease, disorder or condition can be a neurodegenerative disease (such as Parkinson's, or Alzheimer's, or Huntington's, or Creutzfeldt-Jakob disease, or other neurodegenerative disease), or a cognitive disorder (such as dementia, or amnesia, or delirium, or other cognitive disorder), or a brain disorder (such as encephalitis, or seizures, or tumors, or other brain disorder), or a nervous system disorder (such as pain, or seizures, or infections, or other nervous system disorder), or a genetic disease (such as cystic fibrosis, thalassemia, sickle cell anemia, Huntington's Disease, Duchenne's muscular dystrophy, Tay-Sachs disease, or other genetic disease), or a brain tumor, or Niemann-Pick disease, or a prion disease, or Parkinson's disease, or multiple sclerosis, or amyotrophic lateral sclerosis (ALS), or muscular dystrophy, or other disease of the brain or involving the brain. The disease, disorder, or condition of or involving the brain can be a cancer or a disease, disorder, or condition treated or prevented by a vaccine, and/or can be a disease, disorder, or condition that is caused by or involves an

infectious agent. Infectious agents include, for example, one or more of a bacterium, a virus, an oomycete, a parasite, a prion, and a fungus.

For treatment or diagnosis or detection or monitoring, the MEVs, upon intranasal administration, can deliver the cargo to one or more of neurons, astrocytes, glial cells, and neural stem cells. The compositions containing the MEVs can be used to deliver cargo to neurons, astrocytes, glial cells and/or neural stem cells *in vivo.* As discussed below, the MEVs also can be used to deliver the cargo to cells *in vitro* for cell therapy. The resulting cells can be administered. Provided are compositions and methods for delivery of bioactive molecules into cells *in vitro* by introducing a composition comprising microalgae extracellular vesicles (MEVs) containing the bioactive cargo into a cell *in vitro* for cell therapy, diagnostics, and/or detection, where: the bioactive molecule is any molecule that can effect treatment of a disease, disorder, or condition, or that can be used to detect a disease, disorder, or condition, or that can be used to monitor treatment of a disease, disorder, or condition; and the bioactive molecule is heterologous to the microalgae and/or the MEVs. The resulting cells that contain the MEVs provided herein can be used for cell therapy for treating a disease, disorder, or condition of the brain or involving the brain. Cells for cell therapy include, but are not limited to, stem cells, immune cells, or a cell line, with the proviso that the stem cells are not one or more of embryonic or pluripotent or totipotent stem cells in jurisdictions that preclude such cells. The cells can be modified cells, such as CAR-T cells that are designed to target particular cells, tissues, and organs.

Diseases, disorders, and conditions include one or more of a cognitive, emotional, behavioral, psychiatric, neurologic, degenerative, genetic, malignant (cancer), and/or traumatic brain disease, disorder, or condition. The disease, disorder, or condition of the brain or involving the brain can be one that results from injury to the brain or central nervous system (CNS). The MEVs can comprise a therapeutic cargo that is psychoactive or treats a psychiatric disorder, or is an immunomodulatory product, or is a detectable product, or treats brain injury or trauma, or treats cancer, or treats neurological brain disorders, or treats CNS disorders, or treats genetic brain disorders, or treats brain cancer, or has anti-aging activity, or has brain regenerative activity.

The MEVs can, for example, comprise cargo for one or more of: (i) the treatment or the prevention or reducing the risk of brain diseases, disorders, and conditions; (ii) the study, *in vitro* and/or *in vivo* of brain diseases, disorders, and conditions; (iii) the diagnosis of brain diseases, disorders, and conditions; and (iv) recreational use. The diseases, disorders, and conditions include, but are not limited to, cognitive, emotional, behavioral, psychiatric, neurologic, and/or neurodegenerative diseases, disorders, and conditions or a disease, disorder, or condition resulting from injury to the brain or central nervous system (CNS). The diseases, disorders, and conditions are selected from among brain and/or CNS cancers or tumors, genetic disorders, brain injury or trauma, and infections.

Cargo can be selected from among anti-depressants, antipsychotics, anxiolytics, pain killers, psychedelics, hallucinogens, and memory enhancers. For example, the cargo can comprise a carboline, or lysergic acid, or psilocybin, or a derivative thereof. Because of the direct pathway to areas of the brain, the MEVs administered intranasally provide a vehicle for delivery of psychoactive agents. The MEVs can deliver agents for treatment of psychiatric and/or mental disorders.

The MEVs can be used to deliver cargo, such as, for example, hydrophilic compounds, that, when administered systemically or locally to a location other than the nose, is unable to reach the brain after hepatic first-pass metabolism, or that has poor intestinal absorption, and cargo that cannot cross the blood brain barrier. Intranasal administration in MEVs provides for delivery of such compounds, which cannot, in general, otherwise be administered such that they get to the brain.

Diseases, disorders, and conditions for treatment with intranasally administered MEVs, include, but are not limited to, borderline personality disorder, an eating disorder, schizophrenia, attention deficient/hyperactivity disorder (ADHD), autism, bipolar disorder, borderline personality disorder, anxiety, depression, obsessive-compulsive disorder (OCD), and post-traumatic stress disorder (OCD). In some examples the cargo comprises a bioactive molecule for treatment of conditions as follows:

| Bioactive molecule or Drug | Condition(s) |
|---|---|
| 3,4-methylenedioxymetham-phetamine (MDMA; "ecstasy") | Anxiety, Bipolar Disorder, Schizophrenia |
| 5-hydroxytryptamine (5-$HT_1$) receptor agonists | Schizophrenia |
| AGN-241751 | Depression |
| Agomelatine | Depression |
| ALKS-5461 | Depression |
| Amantadine | Autism or Alzheimer Disease |

(continued)

| Bioactive molecule or Drug | Condition(s) |
|---|---|
| Amitriptyline (Elavil®, Merck and Co.) | Anxiety, Depression, Eating Disorders |
| Amoxapine | Anxiety, Depression |
| Amphetamines: (Extended Release amphetamines XR-OS, EROS; Dextroamphetamine sulfate; Lisdexamfetamine; Methamphetamine; Mixed amphetamine salts; Racemic amphetamine sulfate; Triple-bead mixed amphetamine salts) | ADHD |
| Aripiprazole | Autism, Bipolar Disorder, Borderline Personality Disorder, Schizophrenia |
| Arketamine | Bipolar Disorder |
| Asenapine | Bipolar Disorder, Schizophrenia |
| Aspirin | Bipolar Disorder, Depression |
| Atomoxetine | ADHD |
| AV-101 | Depression |
| AVP-786 | Depression |
| AVP-923 | Depression |
| AXS-05 | Depression |
| Ayahuasca | Depression |
| Azapirones | Anxiety |
| AZD2327 | Depression |
| Benzodiazepines | Anxiety, Bipolar Disorder |
| Beta blockers/Azapirones | Anxiety |
| Biperiden | Depression |
| Brexanolone | Depression |
| BTRX-246040 (former LY2940094) | Depression |
| Buprenorphine | Depression |
| Bupropion (Wellbutrin®, Zyban®, Aplenzin®) | Anxiety, Depression |
| Buspirone (Buspar, Bristol Myers Squibb) | Depression, Eating disorders |
| Caffeine | OCD |
| Cannabidiol (CBD) | Anxiety |
| Carbamazepine | Bipolar Disorder |
| Cariprazine | Bipolar Disorder, Schizophrenia |
| Celecoxib | Bipolar Disorder |
| Chlorpromazine | Bipolar Disorder, Schizophrenia |
| Cilostazol | Depression |
| Citalopram (Celexa®) | Anxiety, Depression, OCD, PTSD |
| Clomipramine | OCD, Autism |
| Clonidine | Autism |

(continued)

| Bioactive molecule or Drug | Condition(s) |
|---|---|
| Clonidine-XR | ADHD |
| Clozapine | Bipolar Disorder, Schizophrenia, Autism |
| Coenzyme Q10 | Bipolar Disorder |
| Corticotropin Releasing Factor (CRF) antagonists | Anxiety |
| Cysteamine | Depression |
| D-cycloserine | Depression |
| Desipramine (Norpramin®) | Anxiety, Depression |
| Desipramine (Norpramin®, Aventis) | Eating disorders |
| Desvenlafaxine (Pristiq®) | Anxiety, Depression |
| Dexamethasone | PTSD |
| Dextromethorphan | Depression |
| Divalproex | Borderline Personality Disorder |
| Donepezil | Autism |
| Doxepin | Anxiety, Depression |
| Duloxetine (Cymbalta®) | Anxiety, Depression, Borderline Personality Disorder |
| Ebselen | Bipolar Disorder |
| Endoxifen | Bipolar Disorder |
| Escitalopram (Lexapro®) | Anxiety, Depression, OCD, PTSD, Autism |
| Esketamine | Bipolar Disorder, Depression |
| Eslicarbazepine | Bipolar Disorder |
| Fludrocortisone | Depression |
| Fluoxetine (Prozac®) | Anxiety, Depression, Borderline Personality |
| | Disorder, OCD, PTSD, Autism, Eating Disorders |
| Fluvoxamine | Depression, OCD, Autism |
| Gabapentin | Anxiety, PTSD |
| Galantamine | Autism |
| Glycine | OCD |
| Guanfacine-XR | ADHD |
| Guanfacine | Autism |
| Haloperidol | Bipolar Disorder, Borderline Personality Disorder |
| Hydrocortisone | PTSD |
| ibuprofen | Bipolar Disorder, Depression |
| Imipramine (Tofranil®, Ciba Geigy) | Anxiety, Depression, Eating disorders |
| Infliximab | Depression |

(continued)

| Bioactive molecule or Drug | Condition(s) |
|---|---|
| Isocarboxazid (Marplan®) | Anxiety, Depression |
| JNJ-67953964 | Depression |
| Ketamine | Anxiety, Depression, OCD, PTSD, Bipolar Disorder |
| Lamotrigine | Bipolar Disorder, OCD, Autism |
| Levetiracetam | Autism |
| Levomilnacipran (Fetzima®) | Anxiety, Depression |
| Licarbazepine | Bipolar Disorder |
| Lithium salts | Bipolar Disorder |
| lumateperone tosylate (Caplyta®) | Bipolar Disorder, Schizophrenia |
| Lysergic acid diethylamide (LSD) | Anxiety, Depression |
| Memantine | Bipolar Disorder, Autism, OCD |
| Methylfolate supplementation | Depression |
| Methylphenidate: Dexmethylphenidate | ADHD, Autism |
| Metyrapone | Depression |
| Mifepristone | Depression |
| Minocycline | Bipolar Disorder, Depression, OCD, Anxiety |
| MK0869 | Depression |
| Myo-inositol | OCD |
| N-Acetyl-Cysteine | Bipolar Disorder, OCD |
| Nalmefene | Borderline Personality Disorder |
| Naltrexone | Depression, Autism, Borderline Personality Disorder |
| Naproxen | Bipolar Disorder, Depression |
| Neurokinin-1 ($NK_1$) antagonists | Anxiety, Depression |
| Neuropeptide-Y (NPY) receptor agonists | Anxiety, Bipolar Disorder, Depression, PTSD |
| Nortriptyline (Pamelor®) | Anxiety, Autism, Depression |
| Olanzapine | Bipolar Disorder, Borderline Personality Disorder, PTSD, Schizophrenia |
| Omega-3 Polyunsaturated Fatty Acids; Fish oil | Borderline Personality Disorder, Depression, PTSD |
| Ondansetron (Zofran®, GlaxoSmithKline) | OCD, Eating Disorders |
| Oxcarbazepine | Bipolar Disorder |
| Oxytocin | Anxiety, Autism, Depression, PTSD, Schizophrenia |

(continued)

| Bioactive molecule or Drug | Condition(s) |
|---|---|
| Paliperidone | Bipolar Disorder, Borderline Personality Disorder, Schizophrenia |
| Paroxetine (Paxil®, Pexeva®) | Anxiety, Autism, Depression, OCD, PTSD |
| Phenelzine (Nardil®) | Anxiety, Depression |
| Pioglitazone | Bipolar Disorder |
| Prazosin | PTSD |
| Pregabalin | Anxiety |
| Probiotics | Anxiety, Bipolar Disorder, Depression |
| Propanolol | PTSD |
| Protriptyline | Anxiety, Depression |
| Psilocybin | Anxiety, Depression |
| Quetiapine | Anxiety, Bipolar Disorder, Borderline Personality Disorder |
| Rapastinel | Depression |
| Riluzole | Anxiety, OCD |
| Risperidone | Anxiety, Autism, Bipolar Disorder, OCD, PTSD, Schizophrenia |
| Rivastigmine | Autism |
| S-adenosylmethionine (SAMe) | Depression |
| SAGE-217 | Depression |
| Sarcosine | OCD |
| Scopolamine | Depression |
| Selegiline (Emsam®) | Anxiety, Depression |
| Sertraline (Zoloft®) | Anxiety, Autism, Borderline Personality Disorder, OCD, PTSD |
| Sildenafil | Depression |
| SSR149415 | Depression |
| Tamoxifen | Bipolar Disorder |
| TNF-$\alpha$ inhibitor | Bipolar Disorder |
| Topiramate (Topomax®, Ortho-McNeil Pharmaceutical) | Bipolar disorder, Eating Disorders, OCD, PTSD |
| Tranylcypromine (Parnate®) | Anxiety, Depression |
| Trimipramine. Tetracyclic: Maprotiline | Anxiety |
| Valproate | Bipolar Disorder |
| Valproic acid and derivatives | Autism, Bipolar Disorder, Borderline Personality Disorder |

(continued)

| Bioactive molecule or Drug | Condition(s) |
|---|---|
| Vasopressin (V1B) antagonists | Anxiety, Depression |
| Venlafaxine | Anxiety, Autism, Depression, OCD, PTSD |
| Verapamil | Bipolar Disorder |
| Vilazodone | Depression |
| Vildagliptin | Depression |
| Vortioxetine | Depression |
| Ziprasidonc | Bipolar Disorder, Schizophrenia |

Diseases, disorders, and conditions involving the brain or of the brain include, but are not limited to, genetic disorders, neurodegenerative diseases, and metabolic disorders affecting brain functions, other brain-related conditions, and/or metabolic diseases. Exemplary of diseases, disorders, and conditions, include, but are not limited to, human psychiatric disorders; non-human animal brain disorders, CNS disorders; anxiety disorders, such as panic disorders, social anxiety, phobia-related disorders, and generalized anxiety disorders; attention deficit hyperactivity disorders, such as inattentive type, hyperactive-impulsive type, combination type; autism spectrum disorders, such as Asperger's syndrome, Childhood Disintegrative Disorder (CDD), Kanner's syndrome, Pervasive Developmental Disorder (PDD-NOS); bipolar disorders, such as Bipolar I disorder, Bipolar II disorder, bipolar with mixed features, bipolar with seasonal pattern major depression, Cyclothymia, rapid cycling bipolar; eating disorders, such as anorexia nervosa, bulimia nervosa, muscle dysmorphia, binge eating disorder, other specified eating or feeding disorder (OSFED), compulsive over eating, Prader Willi syndrome, Diabulimia, orthorexia nervosa, selective eating, drunkorexia, pregorexia; personality disorders, including but not limited to, antisocial personality disorder, borderline personality disorder, histrionic personality disorder, narcissistic personality disorder, avoidant personality disorder, dependent personality disorder, obsessive-compulsive disorder (OCD); post-traumatic stress disorders (PTSD), such as acute stress disorder, uncomplicated PTSD, complex PTSD, comorbid PTSD; classic Rett Syndrome, CDKL5-related Atypical Rett Syndrome; schizophrenia disorders, such as catatonic schizophrenia, disorganized schizophrenia, paranoid schizophrenia, residual schizophrenia, and undifferentiated schizophrenia; and other such psychiatric and brain-related conditions. Other diseases, disorders, and conditions of the brain or involving the brain, include, for example, Alzheimer's disease, prion diseases, such as Creutzfeldt-Jakob disease, Niemann-Pick disease, amyotrophic lateral sclerosis (ALS), Friedreich ataxia, Huntington's disease, Lewy body disease, Parkinson's disease, spinal muscular atrophy, Tay-Sachs disease, Wilson's disease, leukodystrophy, epilepsy, multiple sclerosis, encephalitis, and migraines.

MEV cargo for delivery to the brain includes, for example, cargo comprising one or more of psychoactive agents, enzymes, growth factors, and detectable products for treatment or detection or monitoring a disease, disorder, or condition of the brain or involving the brain. Such cargo includes, for example, one or more of TrkA (tropomyosin kinase A), neurotropic factors selected from among NT-3, NT-4, BDNF (brain derived neurotrophic factor), CNTF (ciliary neurotrophic factor), psilocybin and/or psilocin, harmine, tomozolonide, rivastigmine, GABAB1A receptor, GABAB1A receptor siRNA, PTEN siRNA (SEQ ID NOs.: 136-138); miR-17 (miRNA; SEQ ID NOs:139-141), MALAT1 (SEQ ID NO:142); 5-hydroxytryptamine-1A (5-) and 5-hydroxytryptamine-3 (5-HT3) receptor agonists, for example, Azapirones, Methylphenidate, Dexmethylphenidate, Ondansetron (such as the product sold under the trademark Zofran®); Acetylcholinesterase inhibitors, for example, Donepezil, Galantamine, Rivastigmine; Alpha-1-receptor antagonists, for example, Prazosin; Anticonvulsants, for example, Gabapentin, Pregabalin, Topiramate (such as the product sold as Topomax®), Carbamazepine, Eslicarbazepine, Levetiracetam, Licarbazepine, Oxcarbazepine, Valproic acid and derivatives, Lamotrigine; Antipsychotics, for example, Apriprazone, Asenapine, Cariprazine, Chlorpromazine, Clozapine, Haloperidol, Lumateperone tosylate (such as the product sold as Caplyta®), Olanzapine, Paliperidone, Quetiapine, Risperidone, Ziprasidone; Beta blockers, for example, Azapirones, Propranolol; Drugs that modulate the cholinergic system, for example, Biperiden, scopolamine; Corticotropin Releasing Factor (CRF) antagonists; drugs that modulate the GABAergic system, for example, Benzodiazepine, Brexanolone, Sage-217; Glucocorticoid receptor agonists, for example, Hydrocortisone; drugs involved in glutamatergic modulation, for example, AGN-241751, AV-101, AVP-786, AVP-923, AXS-05, D-cycloserine, Dextromethorphan, Rapastinel; Glycine, and glycine reuptake inhibitors, for example, Sarcosine; drugs that modulate the hypothalamic-pituitary-adrenal (HPA) axis, for example, Fludrocortisone, Metyrapone, Mifepristone, and probiotics; drugs that modulate the Kynurenine Pathway (KP); drugs that modulate the limbic and paralimbic brain areas, for example, Cannabidiol (CBD); drugs that modulate the melatonergic system, for example, Agomelatine; Fatty

acids, peptides, nucleic acids and other precursor molecules, for example, alpha-omega fatty acids, Coenzyme Q10, Myo-inositol, Methylfolate, S-adenosylmethionine, Cysteamine, and Oxytocin; Monoamine oxidase inhibitors (MAOIs), for example, Isocarboxazid (Marplan®), Phenelzine (Nardil®), Selegiline (Emsam®), and Tranylcypromine (Parnate®); Mood stabilizers, for example, lithium salts, Valproate, Ebselen, and Divalproex; Multimodal antidepressants, for example Vilazodone and Vortioxetine; N-Nitrosodimethylamine (NDMA)-receptor antagonists, for example, Amantadine, Arketamine, Ketamine, Memantine, Riluzole, Esketamine; Neurokinin-1 (NK1) receptor antagonists; Neuropeptide Y (NPY) receptor agonists; drugs with Neurotrophic effects, Cilostazol, Sildenafil, and Vildagliptin; Norepinephrine-dopamine reuptake inhibitors (NDRIs), Bupropion (Wellbutrin®, Zyban®, Aplenzin®); drugs that act on the opiate system, for example, ALKS-5461, AZD2327, BTRX-246040 (LY2940094), Buprenorphine, JNJ-67953964, Nalmefene, and Naltrexone; Protein Kinase C inhibitors or anti-estrogen drugs, for example, Endoxifen, Tamoxifen, and Verapamil; Psychedelic drugs, for example, 3,4-methylenedioxymethamphetamine (MDMA), Ayahuasca, Lysergic acid diethylamide (LSD), Psilocybin; Selective serotonin reuptake inhibitors (SSRIs), for example, Citalopram (Celexa®), Escitalopram (Lexapro®), Fluvoxamine, Paroxetine (Paxil®, Pexeva®), and Sertraline (Zoloft®); Selective norepinephrine transporter inhibitors, for example, Atomoxetine; Serotonin-norepinephrine reuptake inhibitors (SNRIs), for example, Desvenlafaxine (Pristiq®), Duloxetine (Cymbalta®), Levomilaciran (Fetzima®), and Venlafaxine; Stimulants, including adenosine receptor antagonists, and alpha-2-adrenergic receptor agonists, for example, Caffeine, Clonidine, Guanfacine, Extended Release amphetamines XR-OS, Dextroamphetamine sulfate, Lisdexamfetamine, Methamphetamine, Mixed amphetamine salts, Racemic amphetamine sulfate, Triple-bead mixed amphetamine salts; Substance P-antagonists, for example, Aprepitant (MK0869) and Fosaprepitant (MK-0517); Tricyclic serotonin-norepinephrine reuptake inhibitors, for example, Amitriptyline (Elavil®), Amoxapine, Buspirone (Buspar), Clomipramine, Desipramine (Norpramin®), Doxepin, Imipramine (Tofranil®), Maprotiline, Nortriptyline (Pamelor), Protriptyline, and Trimipramine; and V asopressin 1B (V1B) receptor antagonists, for example, Nelivaptan (SSR149415). Exemplary cargo for delivery to the brain can comprise, for example, catalase, GFP, luciferase, nerve growth factors (NGFs), TrkA (tropomyosin kinase A), neurotrophic factors, including, but not limited to NT-3, NT-4, BDNF (brain derived neurotrophic factor), CNTF (ciliary neurotrophic factor), psilocybin/psilocin, harmine, tomozolonide, rivastigmine, and/or rhodamine. As noted above, compositions containing the MEVs can be delivered to neurons, astrocytes, glial cells, and/or neural stem cells, for administration via intranasal administration or administration to cells *in vitro* for gene therapy.

**Delivery of Agonists and Antagonists of Toll-like Receptors (TLRs)**

The drug delivery systems and compositions provided herein can be used to deliver agonists and/or antagonists of toll-like receptors (TLRs). Provided are drug delivery systems and compositions that comprise microalgae extracellular (MEVs) containing cargo that comprises an agonist or antagonist of a toll-like receptor (TLR), of an internalized receptor, and/or of an intracellular (endosomal) receptor. For example, the cargo in the MEVs can comprise an agonist or antagonist of a toll-like receptor (TLR), an internalized receptor, and/or an intracellular (endosomal) receptor. The TLR and agonist thereof can be one or more of:

| TLR Member | Ligand(s)/Agonists |
|---|---|
| TLR1 | Triacyl lipopeptides (Pam3CSK4) |
| TLR2 | Zymosan, Porin, Modulin, Lipoproteins, Lipotechoic acid, Diacyl lipopeptides, Atypical LPS, Peptidoglycan, Triacyl lipopeptides |
| TLR3 | dsRNA |
| TLR4 | Mannans, Taxol, LPS |
| TLR5 | bacterial flagellin, profilin, HMGB1, Small molecule agonists (CBLB502) |
| TLR6 | Zymosan, Porin, Modulin, Lipoproteins, Lipotechoic acid, Diacyl lipopeptides (Pam2CSK4), Atypical LPS, Peptidoglycan |
| TLR7 | imidazoquinoline, loxoribine, ssRNA, bropirimine, resiquimod |
| TLR8 | ssRNA, small synthetic compounds |
| TLR9 | CpG DNA |
| TLR10 | Diacyl and Triacyl lipopeptides |
| TLR11 | Profilin-like protein, unpathogenic bacteria |

The TLR and antagonist can be one or more of:

| TLR Member | Ligand(s)/Antagonists |
|---|---|
| TLR1 | Small molecule antagonists (CU-T12-9, MMG-11) |
| TLR2 | Small Molecule Antagonists(AT1-AT8, CU-CPT22, CU-T12-9, MMG-11, NPT1220-312), Phloretin, Sulfoglycolipids |
| TLR3 | Small Molecule Antagonists (CU-CPT4a), Monoclonal antibodies (CNTO4685, CNTO5429) |
| TLR4 | Small Molecule Antagonists (Norbinaltorphimine, T4Ics, T5342126, Simvastatin |
| TLR5 | Small Molecule Antagonist (TH1020) |
| TLR6 | Simvastatin |
| TLR7 | Chloroquine, hydroxychloroquine, quinacrine |
| TLR8 | Small Molecule Antagonist (CU-CPT8m, CU-CPT9a) |
| TLR9 | Small Molecule Antagonists(NPT1220-312), chloroquine, hydroxychloroquine, quinacrine; Suppressive or inhibitory oligonucleotides |

Thus, provided herein are MEVs that contain cargo and can be used to deliver cargo to organs, tissues, and/or cells involved in a particular disease, disorder, or condition. The cargo can be selected for treating, diagnosing, and/or detecting the disease, disorder, or condition, and/or for monitoring treatment. By virtue of the unique trafficking of the MEVs as described and demonstrated herein, the MEVs provide a unique delivery vehicle.

## Brief Description of Drawings

**Figure 1** provides an exemplary profile of light intensity used in HECTOR PBR cultures.

**Figure 2** depicts an exemplary elution profile for higher purity of MEV preparations, where MEVs previously concentrated by TFF and purified by ultracentrifugation and formulated in PBS at concentration of $10^{11}$ to $10^{13}$ per mL are seeded in a pre-packed column qEV1 from IZON. The MEVs are eluted using PBS solution. The elution fractions of 0.5 mL are collected. MEVs are recovered in the first fractions as shown in the figure. The most concentrated fractions (4-5) are pooled and stored at 4°C before use.

**Figure 3** provides exemplary images of MEVs obtained using Transmission Electron Microscopy (TEM).

**Figure 4** provides an electropherogram of the small RNA library.

**Figure 5** provides representative patterns of biodistribution according to the route of administration, for the Intravenous (IV), Intratracheal (IT) and Per os (PO) routes.

**Figure 6** depicts the *in vivo* full body imaging of a representative animal after intravenous administration as described in Example 5.

**Figure 7** depicts the *in vivo* full body imaging of a representative animal per os (oral) administration as described in Example 5.

**Figure 8** depicts the *in vivo* full body imaging of a representative animal after intranasal administration as described in Example 5.

**Figure 9** depicts the *in vivo* full body imaging of a representative animal after intratracheal administration as described in Example 5.

**Figure 10** depicts the kinetics of accumulation in liver, lungs and spleen (average of 6 animals) after intravenous administration, as described in Example 5.

**Figure 11** depicts the kinetics of accumulation in lungs, spleen and intestine (average of 6 animals) per os administration, as described in Example 5.

**Figure 12** depicts the kinetics of accumulation lungs and kidneys (average of 4 animals) after intranasal administration, as described in Example 5.

**Figure 13** depicts the kinetics of accumulation in lungs, spleen and intestine (average of 3 animals) after intratracheal administration, as described in Example 5.

**Figures 14A-D** depict *ex vivo* fluorescence analysis (total radiant efficiency) in organs **[A)** liver; **B)** spleen; **C)** lungs; and **D)** brain] isolated 3 days after intravenous (IV), intranasal (IN), per os (PO), and intratracheal (IT) administration.

**Figures 15A** and **15B** depict A) Hematoxylin & Eosin staining of intestine (G = GALT tissue) and B) DAPI (nuclei)

staining and MEV-PKH26 fluorescence.

**Figure 16** depicts the SPLEEN pulp stained with DAPI (for nuclei) and MEV-PKH26 (red fluorescence), shown as white puncta.

**Figure 17** displays a diagram showing the migration of MEV from the GALT to the spleen.

**Figures 18A-I** show results of assessment of toxicity of MEVs in a mouse model after oral (PO) or intratracheal (IT) administration at different doses in 4 groups of mice for each parameter. MEV toxicity was evaluated by chemistry parameters: ALAT, ASAT, urea and creatine (FIGS. 18A-D, respectively); and 2) by hematology parameters: red blood cells, hemoglobin, hematocrit, MCV and eosinophils (FIGS. 18E-I, respectively). The groups were: Group 1 mice administered 100 μl of PBS (white bars) by PO delivery; Group 2 mice were administered 100 μl of $4*10^{11}$ MEV/ mouse by PO delivery (white bar with black dots); Group 3 mice were administered 100 μl of $4*10^{12}$ MEV/ mouse by PO delivery (white bars with vertical lines); Group 4 mice were administered 100 μl of $4*10^{11}$ MEV/ mouse by IT delivery (squared bars). Data were measured for six mice per group for each parameter. **FIG. 18A** shows ALAT: Alanine Aminotransferase; **FIG. 18B** shows ASAT: Aspartate Aminotransferase; **FIG. 18C** shows urea; **FIG. 18D** shows creatine; **FIG. 18E** shows red blood cells; **FIG. 18F** shows hemoglobin; **FIG. 18G** shows hematocrit; **FIG. 18H** shows MCV (Mean Corpuscular Volume); and **FIG. 18I** shows eosinophils. PO designates per os (oral delivery) and IT designates Intratracheal administration.

**Figure 19** depicts *in vivo* delivery and expression of mRNA after topical instillation of MEVs into the eyes in rabbits.

**Figures 20A and 20B** depict *in vitro* delivery of GFP protein to human monocytes.

**Figures 21A and 21B** depict *in vitro* delivery of GFP protein to human keratinocytes.

**Figure 22** shows confocal microscopy of Hep-G2 cells including GFP protein expression in Hep-G2 cells after 24h incubation with MEVs loaded with GFP-protein (MEV-GFP) or MEVs loaded with mRNA-eGFP (MEV-mRNA).

**Figure 23** shows confocal microscopy Huh7 cells, including GFP protein expression in Hep-G2 cells.

**Figure 24** shows *in vitro* delivery of GFP-mRNA and GFP- encoding mRNA loaded MEV to human fibroblasts.

**Figures 25A-D** show results of the flow cytometry analysis in MEV penetration and delivery study using human fibroblasts.

**Figure 26** shows antibacterial activity of *Chlorella* MEVs exogenously loaded with siRNAs directed against *Pto* DC3000 *cfa6* and *hrpL* genes.

**Figure 27** shows delivery of the bioactive flg22 peptides exogenously loaded in Chlorella MEVs.

**Figure 28** is a schematic that depicts routes for passage through the olfactory epithelium.

**Figure 29** shows a positive control Dir-MEV on DAPI-stained brain slice: a drop of MEV suspension deposited on top of a brain tissue slide. Puncta are Dir-labeled MEV.

**Figure 30** is a schematic of the Insula and its connections (reproduced from Gogolla (2017) "The insular cortex," Current Biology:27(12): R580-R586.

**Figure 31** is a schematic diagram of brain neuronal pathway from the olfactory sensory neurons (OSN) through the olfactory bulb (OB) to the mitral and tufted neurons, to the olfactory tract (OT).

**Figure 32** is a schematic showing the pathways and approximate average distances from the olfactory and respiratory epithelium to CNS targets (reproduced from Lochhead et al. (2019). "Perivascular and Perineural Pathways Involved in Brain Delivery and Distribution of Drugs after Intranasal Administration" Pharmaceutics 11(11):598, doi.org/10.3390/pharmaceutics11110598).

**Figure 33** is a schematic of a cortical projection of mitral and tufted cells showing a ventrolateral view of the brain (reproduced from Imai (2014) "Construction of functional neuronal circuitry in the olfactory bulb," Seminars in Cell and Developmental Biology 35, DOI:10.1016/j.semcdb.2014.07.012).

**Figure 34** shows transport of MEVs via olfactory pathway. After IN administration, MEVs are taken by the olfactory epithelium transported by axonal transport by olfactory neurons to the olfactory bulb then by mitral and tufted neurons to the primary olfactory regions that process the olfactory signal (reproduced from Selvaraj et al. (2018) Artificial Cells, Nanomedicine, and Biotechnology An International Journal 46:2088-2095, doi.org/10.1080/21691401.2017.1420073).

**Figure 35** depicts the olfactive pathway used by MEVs after IN administration (schematic of the general pathway reproduced from "What-when-how in Depth tutorials and information, Olfaction and Taste, Sensory system, part 1" (what-when-how.com)).

**Figures 36(a) -(g):** (a) General overview of the experimental design of brain biodistribution studies. (b) Position of the 5 brain sections studied; (c) the regions analyzed to determine the PK and biodistribution of MEVs in each of the 5 brain sections studied.; (d)-(g) depict and identify regions of the brain for reference with the following figures that show MEVs in the brain following IN administration.

**Figures 37(a)-(d)** show the pharmacokinetic (PK) and biodistribution of MEVs in different regions of section 1 from Figures 36. Images of labelled-MEVs with DiR are the black dots.

**Figure 38** shows the PK and biodistribution of MEVs in different regions of section 1, providing a graphical representation of the total number of labelled MEVs with DiR spots per surface of regions of section 1, normalized

by total analyzed area.

**Figures 39 (a)-(d)** show the PK and Biodistribution of MEVs in different regions of section 2 (showing images of labelled-MEVs with DiR).

**Figures 40(a)-(d)** show the PK and biodistribution of MEVs in different regions of section 2 as a graphical representation of the total number of labelled MEVs with DiR spots per surface of regions of section 2, normalized by total analyzed area.

**Figures 41 (a)-(d)** show the PK and biodistribution of MEVs in different regions of section 3; images of DiR-labelled MEVs.

**Figures 42 (a)-(f)** show the PK and biodistribution of MEVs in different regions of section 3 in a graphical representation of total number of labelled MEVs with DiR spots per surface of regions of section 3, normalized by total analyzed area.

**Figures 43 (a)-(d)** show the PK and biodistribution of MEVs in different regions of section 4 as images of MEVs labelled with DiR.

**Figures 44 (a)-(d)** show the PK and biodistribution of MEVs in different regions of section 4, providing a graphical representation of total number of labelled MEVs with DiR spots per surface of regions of section 4, normalized by total analyzed area.

**Figures 45 (a)-(d)** show the PK and biodistribution of MEVs in different regions of section 5; images of DiR-labelled MEVs.

**Figures 46 (a) and (b)** show the kinetics of brain penetration by the MEVs, from the rostral to the distal parts of the brain.

**Figures 47 (a) and (b)** depict the blood-brain barrier (reproduced from Cecchelli et al. (2007) Nat Rev Drug Discov. 6(8):650-661).

**Figure 48** shows a microscopic image of mouse intestinal epithelium 8 hours after PKH26-labeled MEV administration.

**Figure 49** shows whole-body bioluminescence imaging of a representative animal treated with MEVs loaded with luciferase mRNA.

**Figure 50** depicts whole-body bioluminescence imaging of a representative animal treated with MEVs loaded with luciferase enzyme.

**Figure 51** depicts the timeline for image analysis using the Incucyte® live cell analyzer.

## DETAILED DESCRIPTION

### Outline

- **A. DEFINITIONS**
- **B. MICROALGAE AND OVERVIEW**
- **C. EXTRACELLULAR VESICLES**
  - **1. Types of Extracellular Vesicles (EVs)**
    - **a. Exosomes**
    - **b. Microvesicles**
    - **c. Apoptotic Bodies**
  - **2. Uptake of EVs**
  - **3. General Methods for Isolating EVs**
    - **a. Ultracentrifugation**
    - **b. Size-Based Techniques**
    - **c. Immunoaffinity Capture-Based Techniques**
    - **d. Exosome Precipitation**
    - **e. Microfluidic Based Isolation Techniques**
  - **4. Microalgae and Microalgae-Derived Extracellular Vesicles (MEVs)**
  - **5. Green algae - *Chlorella* species**

**a. Life Cycle**
**b. Genomic Analyses of *Chlorella* Species**
**c. Commercial and Biotechnological Uses of *Chlorella***
**d. *Chlorella* MEVs**

**D. EXOGENOUSLY LOADED MICROALGAE EXTRACELLULAR VESICLES (MEVS), CARGO, AND TARGETS**

**1. Isolation of MEVs**
**2. MEV Loading and Cargos**
**3. Generation of Payload-Loaded MEVs**

**a. Electroporation**
**b. Sonication**
**c. Extrusion**
**d. Surfactants**
**e. Other Methods**

4. Exemplary Cargo and Exemplary Uses of the Exogenously Loaded MEVs

a. Cargo

1) RNA Cargo
2) Antibody Cargo

b. Diseases and Methods of Treatment
c. Agro-Veterinary Applications
d. Cosmetic and Dermatological Applications

E. PHARMACEUTICAL COMPOSITIONS, FORMULATIONS, KITS, ARTICLES OF MANUFACTURE AND COMBINATIONS

1. Pharmaceutical Compositions and Formulations
2. Articles of Manufacture/Kits and Combinations
3. Administration of Exogenously Loaded MEVs and Routes of Administration
4. Combination Therapies

F. BIODISTRIBUTION OF MEVs FOLLOWING ADMINISTRATION VIA VARIOUS ROUTES

1. Biodistribution of mammalian EVs

2. Microalgae EVs Biodistribution

a. Oral Administration

1) Components of the Lymphatic System
2) Targeting GALT

3. Diseases and conditions treated by MEVs

G. FORMULATIONS, ROUTES OF ADMINISTRATION, AND DISEASE AND DISORDERS
H. BIODISTRIBUTION AND DELIVERY OF MEVs TO THE BRAIN VIA INTRANASAL (IN) ADMINISTRATION FOR TREATING DISEASES, DISORDERS, AND CONDITIONS OF THE BRAIN AND CNS

1. Brain structure

**a.** Anterior Olfactory Nucleus
**b.** Tenia Tecta
**c.** Olfactory Tubercle

**d.** Piriform Cortex
**e.** Amygdala
**f.** Entorhinal Cortex
**g.** Frontal Cortex
**h.** Striatum: caudate nucleus and putamen
i. Nucleus accumbens
j. Thalamus
k. Hypothalamus
l. Substantia nigra pars compacta
**m. Hippocampus**
**n. Colliculus**
**o. Pontine Raphe nuclei**

**2. The Brain Blood Barrier**
**3. Brain and target cells**
**4. Differences between Biodistribution of MEVs and other delivery vehicles**
**5. Intranasal administration**
**6. MEVs and delivery to the brain following intranasal administration**
**7. Trafficking and biodistribution of MEVs following intranasal (IN) administration**
**8. Primary and secondary circuitry of the olfactory system and regions reached by the MEVs upon IN administration**
**9. Delivery of MEVs via IN administration to the brain - exemplary bioactive cargo and uses thereof**

**I. MEV-MEDIATED INTRACELLULAR SIGNALING**
**J. EXAMPLES**

### A. Definitions

Unless defined otherwise, all technical and scientific terms used herein have the same meaning as is commonly understood by one of skill in the art to which the invention(s) belong. All patents, patent applications, published applications and publications, GenBank® sequences, databases, websites and other published materials referred to throughout the entire disclosure herein, unless noted otherwise, are incorporated by reference in their entirety. In the event that there are a plurality of definitions for terms herein, those in this section prevail. Where reference is made to a URL or other such identifier or address, it is understood that such identifiers can change and particular information on the internet can come and go, but equivalent information can be found by searching the internet. Reference thereto evidences the availability and public dissemination of such information.

As used herein, cargo refers exogenous molecules, such as bioactive molecules, including biomolecules, and small molecules, that are loaded into the microalgae extracellular vesicles (MEVs) provided herein after the MEVs have been isolated. This includes cargo that is heterologous to the MEVs.

As used herein, in general, heterologous with respect to cargo in an MEV refers to cargo in the MEVs that does not naturally-occur in the MEVs but is loaded exogenously, as discussed above. It also refers to cargo in MEVs that have been loaded endogenously in the MEVs by genetically-modified microalgae. MEVs with heterologous cargo, comprise cargo that does not occur naturally in the MEVs.

As used herein, a bioactive molecule or bioactive agent refers to any molecule or agent that can have a biological activity, such as therapeutic activity, or as a detectable marker, or that can act *in vivo* on a subject. Bioactive agents and molecules include biomolecules, such as DNA, RNA, proteins, other biopolymers, and small molecules, such as small molecule drugs and pharmaceuticals, immunogens, and any molecules that would be delivered to a subject, such as a human or other animal or a plant or a microorganism (bacteria or other), in connection with a therapy, a diagnostic application, or other such uses, such as a cosmetic. The bioactive agent or molecule can function as or have an activity as, for example, a therapeutic, an immunogen, a diagnostic, a detectable marker, or a cosmetic. The bioactive molecules for use herein are any that can be loaded into a microalgae extracellular vesicle (MEV).

As used herein, a biomolecule refers to any biologically active biopolymer or molecule that occurs, or can occur, in a living organism or virus or that is a modified form of such biopolymer or molecule. Biomolecules, thus, include modified naturally-occurring biomolecules, such as, for example proteins that include a modified primary sequence, such as by deletions, insertions, and/or replacements of amino acids to alter the primary sequence, and/or by modification, such as post-translational modifications of the protein.

As used herein, when it is stated that MEVs have the same or substantially the same loaded cargo or amount thereof, it is understood that this refers to an average among the population of MEVs in a composition. It is understood, that

when MEVs are loaded exogenously the ratio of cargo/MEV can be selected so that each MEV has, on average, a predetermined amount of cargo. As a simple example, to load an average of one molecule of cargo/MEV, the skilled person could calculate the amount of cargo to load into a composition of MEVs, and understands that in the composition of MEVs, some would have more than one molecule of cargo/MEV, and others would have none. On average, the MEVs would have one molecule of cargo/MEV. The skilled person understands, that, in general, the amount of cargo/MEV will be more than the one molecule/MEV, and that the amount of cargo depends upon a variety of parameters, including the cargo, the target tissues and/or cells, the disease, disorder, or condition treated, and the subject treated. Generally, more than one molecule of cargo per MEV, on the average, such as at least 10 or about 10 molecules/MEV are loaded. Substantially more cargo, 100, 500, 1000, $10^4$ molecules/MEV and more, also can be loaded. The amount loaded depends upon the target, disease, disorder, or condition, the subject, and the cargo, and the capacity of the MEV. It is within the skill in the art to select the amount.

As used herein, a subject is any organism, generally an animal or plant, into which or on which the composition containing the MEV is introduced. Subjects include, but are not limited to, humans, plants, particularly crop plants, and animals, including farm animals and pets, such as dogs and cats, and zoo animals.

As used herein, a drug delivery system refers to a composition that contains MEVs provided herein that contain cargo for delivery to tissues. As shown herein, by virtue of the formulation and route of administration of the composition containing the MEVs the trafficking route and/or ultimate destination of the MEVs, upon administration, can selected. For example, as demonstrated herein, intranasal administration of the MEVs results in trafficking of the MEVs to the brain. Orally administered MEVs can target gut-associated lymphoid tissue (GALT). Thus, GALT is a target (effective compartment) and/or a route through which MEVs and their therapeutic agent cargo can be used to deliver cargo. The delivery system refers to the combining of formulation for a particular route of administration to target particular tissues for treatment of diseases, disorders, and conditions of these tissues or involving these tissues.

As used herein, disease or disorder or condition refers to a pathological or undesirable or undesired condition in an organism resulting from a cause or condition including, but not limited to, infections, acquired conditions, and genetic conditions, and those characterized by identifiable symptoms or characteristics.

As used herein, treating a subject with a disease, disorder, or condition means that the subject’s symptoms or manifestations of the disease or conditions are partially or totally alleviated, or remain static following treatment.

As used herein, treatment refers to any effects that ameliorate symptoms of a disease or disorder. Treatment encompasses prophylaxis, therapy and/or cure. Treatment also encompasses any pharmaceutical use of any MEV or composition provided herein. Treatment refers to any effects that ameliorate or prevent or other reduce or eliminate any symptom or manifestation of a disease or disorder. Treatment also encompasses any pharmaceutical use of any MEV or composition provided herein.

As used herein, prophylaxis refers to prevention of a potential disease and/or a prevention of worsening of symptoms or progression of a disease. Prevention or prophylaxis, and grammatically equivalent forms thereof, refer to methods in which the risk or probability of developing a disease or condition is reduced or eliminated and products that reduce or eliminate the risk or probability of developing a disease or condition.

As used herein, a modification with reference to modification of a sequence of amino acids of a polypeptide or a sequence of nucleotides in a nucleic acid molecule refers to and includes deletions, insertions, and replacements of amino acids or nucleotides, respectively. These include modifications of the primary sequence of a polypeptide or protein. Methods of modifying a polypeptide and nucleic acid molecule are routine to those of skill in the art, such as by using recombinant DNA methodologies. Modifications, when referring to polypeptide or protein, not to a sequence, refer to post-translational or post-purification changes, such as conjugation or linkage of moieties that alter properties of polypeptide or protein, such as half-life extending moieties, glycosylation, purification tags, detectable reporters, and other such moieties.

As used herein, a modification of a genome or a plasmid or gene includes deletions, replacements, insertions, and translocations of nucleic acid. These include any changes to the native or naturally-occurring nucleic acid sequence.

As used herein, RNA interference (RNAi) is a biological process in which RNA molecules inhibit gene expression or translation, by neutralizing targeted mRNA molecules to inhibit translation and thereby expression of a targeted gene.

As used herein, RNA molecules that act via RNAi are referred to as inhibitory by virtue of their silencing of expression of a targeted gene. Silencing expression means that expression of the targeted gene is reduced or suppressed or inhibited.

As used herein, gene silencing via RNAi is said to inhibit, suppress, disrupt or silence expression of a targeted gene. A targeted gene contains sequences of nucleotides that correspond to the sequences in the inhibitory RNA, whereby the inhibitory RNA silences expression of mRNA. Small interfering RNAs (siRNAs) are small pieces of double-stranded (ds) RNA, usually about 21 nucleotides long, with 3’ overhangs (2 nucleotides) at each end that can be used to interfere with the translation of proteins by binding to and promoting the degradation of messenger RNA (mRNA) at specific sequences. In doing so, siRNAs prevent the production of specific proteins based on the nucleotide sequences of their corresponding mRNAs. The process is called RNA interference (RNAi), and also is referred to as siRNA silencing or siRNA

knockdown. A short-hairpin RNA or small-hairpin RNA (shRNA) is an artificial RNA molecule with a tight hairpin turn that can be used to silence target gene expression via RNA interference (RNAi). Expression of shRNA in cells is typically accomplished by delivery of plasmids or through viral or bacterial vectors.

As used herein, non-coding RNAs are RNAs that do not encode a protein. Classes of non-coding RNA, include, but are not limited to, small interfering RNAs (siRNAs) and microRNAs (miRNAs). As used herein, inhibiting, suppressing, disrupting or silencing a targeted gene refers to processes that alter expression, such as translation, of the targeted gene, whereby activity or expression of the product encoded by the targeted gene is reduced. Reduction includes a complete knock-out or a partial knockout, whereby, with reference to the MEVs provided herein and administration herein, treatment is effected.

As used herein, a tumor microenvironment (TME) is the cellular environment in which the tumor exists, including surrounding blood vessels, immune cells, fibroblasts, bone marrow-derived inflammatory cells, lymphocytes, signaling molecules and the extracellular matrix (ECM). Conditions that exist include, but are not limited to, increased vascularization, hypoxia, low pH, increased lactate concentration, increased pyruvate concentration, increased interstitial fluid pressure and altered metabolites or metabolism, such as higher levels of adenosine, indicative of a tumor.

As used herein, recitation that a nucleic acid or encoded RNA targets a gene means that it inhibits or suppresses or silences expression of the gene by any mechanism. Generally, such nucleic acid includes at least a portion complementary to the targeted gene, where the portion is sufficient to form a hybrid with the complementary portion.

As used herein, deletion, when referring to a nucleic acid or polypeptide sequence, refers to the deletion of one or more nucleotides or amino acids compared to a sequence, such as a target polynucleotide or polypeptide or a native or wild-type sequence.

As used herein, insertion, when referring to a nucleic acid or amino acid sequence, describes the inclusion of one or more additional nucleotides or amino acids, within a target, native, wild-type or other related sequence. Thus, a nucleic acid molecule that contains one or more insertions compared to a wild-type sequence, contains one or more additional nucleotides within the linear length of the sequence.

As used herein, additions to nucleic acid and amino acid sequences describe addition of nucleotides or amino acids onto either termini compared to another sequence.

As used herein, substitution or replacement refers to the replacing of one or more nucleotides or amino acids in a native, target, wild-type or other nucleic acid or polypeptide sequence with an alternative nucleotide or amino acid, without changing the length (as described in numbers of residues) of the molecule. Thus, one or more substitutions in a molecule does not change the number of amino acid residues or nucleotides of the molecule. Amino acid replacements compared to a particular polypeptide can be expressed in terms of the number of the amino acid residue along the length of the polypeptide sequence.

As used herein, at a position corresponding to, or a recitation that nucleotides or amino acid positions correspond to nucleotides or amino acid positions in a disclosed sequence, such as set forth in the Sequence Listing, refers to nucleotides or amino acid positions identified upon alignment with the disclosed sequence to maximize identity using a standard alignment algorithm, such as the GAP algorithm. By aligning the sequences, one skilled in the art can identify corresponding residues, for example, using conserved and identical amino acid residues as guides. In general, to identify corresponding positions, the sequences of amino acids are aligned so that the highest order match is obtained (see, *e.g.,* Computational Molecular Biology, Lesk, A.M., ed., Oxford University Press, New York, 1988; Biocomputing: Informatics and Genome Projects, Smith, D. W., ed., Academic Press, New York, 1993; Computer Analysis of Sequence Data, Part I, Griffin, A.M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M Stockton Press, New York, 1991; and Carrillo et al. (1988) SIAM J Applied Math 48:1073).

As used herein, alignment of a sequence refers to the use of homology to align two or more sequences of nucleotides or amino acids. Typically, two or more sequences that are related by 50% or more identity are aligned. An aligned set of sequences refers to 2 or more sequences that are aligned at corresponding positions and can include aligning sequences derived from RNAs, such as ESTs and other cDNAs, aligned with genomic DNA sequence. Related or variant polypeptides or nucleic acid molecules can be aligned by any method known to those of skill in the art. Such methods typically maximize matches, and include methods, such as using manual alignments and by using the numerous alignment programs available (e.g., BLASTP) and others known to those of skill in the art. By aligning the sequences of polypeptides or nucleic acids, one skilled in the art can identify analogous portions or positions, using conserved and identical amino acid residues as guides. Further, one skilled in the art also can employ conserved amino acid or nucleotide residues as guides to find corresponding amino acid or nucleotide residues between and among human and non-human sequences. Corresponding positions also can be based on structural alignments, for example by using computer simulated alignments of protein structure. In other instances, corresponding regions can be identified. One skilled in the art also can employ conserved amino acid residues as guides to find corresponding amino acid residues between and among human and non-human sequences.

As used herein, a property of a polypeptide, such as an antibody, refers to any property exhibited by a

polypeptide, including, but not limited to, binding specificity, structural configuration or conformation, protein stability, resistance to proteolysis, conformational stability, thermal tolerance, and tolerance to pH conditions. Changes in properties can alter an activity of the polypeptide. For example, a change in the binding specificity of the antibody polypeptide can alter the ability to bind an antigen, and/or various binding activities, such as affinity or avidity, or *in vivo* activities of the polypeptide.

As used herein, an activity or a functional activity of a polypeptide, such as an antibody, refers to any activity exhibited by the polypeptide. Such activities can be empirically determined. Exemplary activities include, but are not limited to, ability to interact with a biomolecule, for example, through antigen-binding, DNA binding, ligand binding, or dimerization, or enzymatic activity, for example, kinase activity or proteolytic activity. For an antibody (including antibody fragments), activities include, but are not limited to, the ability to specifically bind a particular antigen, affinity of antigen-binding *(e.g.,* high or low affinity), avidity of antigen-binding *(e.g.,* high or low avidity), on-rate, off-rate, effector functions, such as the ability to promote antigen neutralization or clearance, virus neutralization, and *in vivo* activities, such as the ability to prevent infection or invasion of a pathogen, or to promote clearance, or to penetrate a particular tissue or fluid or cell in the body. Activity can be assessed *in vitro* or *in vivo* using recognized assays, such as ELISA, flow cytometry, surface plasmon resonance or equivalent assays to measure on- or off-rate, immunohistochemistry and immunofluorescence histology and microscopy, cell-based assays, flow cytometry and binding assays (e.g., panning assays).

As used herein, bind, bound, and grammatical variations thereof refer to the participation of a molecule in any interaction with another molecule or among molecules, resulting in a stable association in which the molecules are in close proximity to one another. Binding includes, but is not limited to, non-covalent bonds, covalent bonds (such as reversible and irreversible covalent bonds), and includes interactions between molecules such as, but not limited to, proteins, nucleic acids, carbohydrates, lipids, and small molecules, such as chemical compounds including drugs.

As used herein, antibody refers to immunoglobulins and immunoglobulin fragments, whether natural or partially or wholly synthetically, such as recombinantly produced, including any fragment thereof containing at least a portion of the variable heavy chain and light region of the immunoglobulin molecule that is sufficient to form an antigen binding site and, when assembled, to specifically bind an antigen. Hence, an antibody includes any protein having a binding domain that is homologous or substantially homologous to an immunoglobulin antigen-binding domain (antibody combining site). For example, an antibody refers to an antibody that contains two heavy chains (which can be denoted H and H') and two light chains (which can be denoted L and L'), where each heavy chain can be a full-length immunoglobulin heavy chain or a portion thereof sufficient to form an antigen binding site (e.g., heavy chains include, but are not limited to, VH chains, VH-CH1 chains and VH-CH1-CH2-CH3 chains), and each light chain can be a full-length light chain or a portion thereof sufficient to form an antigen binding site *(e.g.,* light chains include, but are not limited to, VL chains and VL-CL chains). Each heavy chain (H and H') pairs with one light chain (L and L', respectively). Typically, antibodies minimally include all or at least a portion of the variable heavy (VH) chain and/or the variable light (VL) chain. The antibody also can include all or a portion of the constant region.

For purposes herein, the term antibody includes full-length antibodies and portions thereof including antibody fragments, such as anti-tumor antibody or anti-pathogen or gene silencing fragments. Antibody fragments, include, but are not limited to, Fab fragments, Fab' fragments, $F(ab')_2$ fragments, Fv fragments, disulfide-linked Fvs (dsFv), Fd fragments, Fd' fragments, single-chain Fvs (scFv), single-chain Fabs (scFab), diabodies, anti-idiotypic (anti-Id) antibodies, or antigen-binding fragments of any of the above. Antibody also includes synthetic antibodies, recombinantly produced antibodies, multispecific antibodies (e.g., bispecific antibodies), human antibodies, non-human antibodies, humanized antibodies, chimeric antibodies, and intrabodies. Antibodies provided herein include members of any immunoglobulin class *(e.g.,* IgG, IgM, IgD, IgE, IgA and IgY), any subclass *(e.g.,* IgG1, IgG2, IgG3, IgG4, IgA1 and IgA2) or sub-subclass (e.g., IgG2a and IgG2b).

As used herein, nucleic acid refers to at least two linked nucleotides or nucleotide derivatives, including a deoxyribonucleic acid (DNA) and a ribonucleic acid (RNA), joined together, typically by phosphodiester linkages. Also included in the term nucleic acid are analogs of nucleic acids such as peptide nucleic acid (PNA), phosphorothioate DNA, and other such analogs and derivatives or combinations thereof. Nucleic acids also include DNA and RNA derivatives containing, for example, a nucleotide analog or a backbone bond other than a phosphodiester bond, for example, a phosphotriester bond, a phosphoramidate bond, a phosphorothioate bond, a thioester bond, or a peptide bond (peptide nucleic acid). The term also includes, as equivalents, derivatives, variants and analogs of either RNA or DNA made from nucleotide analogs, single (sense or antisense) and double-stranded nucleic acids. Deoxyribonucleotides include deoxyadenosine, deoxycytidine, deoxyguanosine and deoxythymidine. For RNA, the uracil base is uridine.

As used herein, an isolated nucleic acid molecule is one which is separated from other nucleic acid molecules which are present in the natural source of the nucleic acid molecule. An isolated nucleic acid molecule, such as a cDNA molecule, can be substantially free of other cellular material, or culture medium when produced by recombinant techniques, or substantially free of chemical precursors or other chemicals when chemically synthesized. Exemplary isolated nucleic acid molecules provided herein include isolated nucleic acid molecules encoding RNAi or a therapeutic protein.

As used herein, operably linked with reference to nucleic acid sequences, regions, elements or domains means that the nucleic acid regions are functionally related to each other. For example, a nucleic acid encoding a leader peptide can be operably linked to a nucleic acid encoding a polypeptide, whereby the nucleic acids can be transcribed and translated to express a functional fusion protein, wherein the leader peptide effects secretion of the fusion polypeptide. In some instances, the nucleic acid encoding a first polypeptide (*e.g.,* a leader peptide) is operably linked to a nucleic acid encoding a second polypeptide and the nucleic acids are transcribed as a single mRNA transcript, but translation of the mRNA transcript can result in one of two polypeptides being expressed. For example, an amber stop codon can be located between the nucleic acid encoding the first polypeptide and the nucleic acid encoding the second polypeptide, such that, when introduced into a partial amber suppressor cell, the resulting single mRNA transcript can be translated to produce either a fusion protein containing the first and second polypeptides, or can be translated to produce only the first polypeptide. In another example, a promoter can be operably linked to nucleic acid encoding a polypeptide, whereby the promoter regulates or mediates the transcription of the nucleic acid.

As used herein, synthetic, with reference to, for example, a synthetic nucleic acid molecule or a synthetic gene or a synthetic peptide refers to a nucleic acid molecule or polypeptide molecule that is produced by recombinant methods and/or by chemical synthesis methods.

As used herein, the residues of naturally occurring $\alpha$-amino acids are the residues of those 20 $\alpha$-amino acids found in nature which are incorporated into protein by the specific recognition of the charged tRNA molecule with its cognate mRNA codon in humans.

As used herein, polypeptide refers to two or more amino acids covalently joined. The terms polypeptide and protein are used interchangeably herein.

As used herein, a peptide refers to a polypeptide that is from 2 to about or 40 amino acids in length.

As used herein, reference to proteins, unless otherwise specified, includes all forms of peptides, polypeptides, small peptides, and proteins.

As used herein, an amino acid is an organic compound containing an amino group and a carboxylic acid group. A polypeptide contains two or more amino acids. For purposes herein, amino acids contained in the antibodies provided include the twenty naturally-occurring amino acids (see Table below), non-natural amino acids, and amino acid analogs (*e.g.,* amino acids wherein the $\alpha$-carbon has a side chain). As used herein, the amino acids, which occur in the various amino acid sequences of polypeptides appearing herein, are identified according to their well-known, three-letter or one-letter abbreviations (see Table below). The nucleotides, which occur in the various nucleic acid molecules and fragments, are designated with the standard single-letter designations used routinely in the art.

As used herein, amino acid residue refers to an amino acid formed upon chemical digestion (hydrolysis) of a polypeptide at its peptide linkages. The amino acid residues described herein are generally in the L isomeric form. Residues in the D isomeric form can be substituted for any L-amino acid residue, as long as the desired functional property is retained by the polypeptide. $NH_2$ refers to the free amino group present at the amino terminus of a polypeptide. COOH refers to the free carboxy group present at the carboxyl terminus of a polypeptide. In keeping with standard polypeptide nomenclature described in J. Biol. Chem., 243:3557-59 (1968) and adopted at 37 C.F.R. §§ 1.821 - 1.822, abbreviations for amino acid residues are shown in the following Table:

**Table of Correspondence**

| SYMBOL | | |
|---|---|---|
| **1-Letter** | **3-Letter** | **AMINO ACID** |
| Y | Tyr | Tyrosine |
| G | Gly | Glycine |
| F | Phe | Phenylalanine |
| M | Met | Methionine |
| A | Ala | Alanine |
| S | Ser | Serine |
| I | Ile | Isoleucine |
| L | Leu | Leucine |
| T | Thr | Threonine |
| V | Val | Valine |
| P | Pro | Proline |

(continued)

| SYMBOL | | |
|---|---|---|
| 1-Letter | 3-Letter | AMINO ACID |
| K | Lys | Lysine |
| H | His | Histidine |
| Q | Gln | Glutamine |
| E | Glu | Glutamic acid |
| z | Glx | Glutamic Acid and/or Glutamine |
| W | Trp | Tryptophan |
| R | Arg | Arginine |
| D | Asp | Aspartic acid |
| N | Asn | Asparagine |
| B | Asx | Aspartic Acid and/or Asparagine |
| C | Cys | Cysteine |
| X | Xaa | Unknown or other |

All sequences of amino acid residues represented herein by a formula have a left to right orientation in the conventional direction of amino-terminus to carboxyl-terminus. The phrase amino acid residue is defined to include the amino acids listed in the above Table of Correspondence, modified, non-natural and unusual amino acids. A dash at the beginning or end of an amino acid residue sequence indicates a peptide bond to a further sequence of one or more amino acid residues or to an amino-terminal group such as $NH_2$ or to a carboxyl-terminal group such as COOH.

In a peptide or protein, suitable conservative substitutions of amino acids are known to those of skill in the art and generally can be made without altering a biological activity of a resulting molecule. Those of skill in the art recognize that, in general, single amino acid substitutions in non-essential regions of a polypeptide do not substantially alter biological activity (see, *e.g.,* Watson et al., Molecular Biology of the Gene, 4th Edition, 1987, The Benjamin/Cummings Pub. Co., p. 224).

Such substitutions can be made in accordance with the exemplary substitutions set forth in the following Table:

**Exemplary conservative amino acid substitutions**

| Original residue | Exemplary Conservative substitution(s) |
|---|---|
| Ala (A) | Gly; Ser |
| Arg (R) | Lys |
| Asn (N) | Gln; His |
| Cvs (C) | Ser |
| Gln (Q) | Asn |
| Glu (E) | Asp |
| Glv (G) | Ala; Pro |
| His (H) | Asn; Gln |
| Ile (I) | Leu; Val |
| Leu (L) | Ile; Val |
| Lys (K) | Arg; Gln; Glu |
| Met (M) | Leu; Tyr; Ile |
| Phe (F) | Met; Leu; Tyr |
| Ser (S) | Thr |
| Thr (T) | Ser |

(continued)

| Original residue | Exemplary Conservative substitution(s) |
|---|---|
| Trp (W) | Tvr |
| Tyr (Y) | Trp; Phe |
| Val (V) | Ile; Leu |

Other substitutions also are permissible and can be determined empirically or in accord with other known conservative or non-conservative substitutions.

As used herein, naturally occurring amino acids refer to the 20 L-amino acids that occur in polypeptides.

As used herein, the term non-natural amino acid refers to an organic compound that has a structure similar to a natural amino acid but has been modified structurally to mimic the structure and reactivity of a natural amino acid. Non-naturally occurring amino acids thus include, for example, amino acids or analogs of amino acids other than the 20 naturally occurring amino acids and include, but are not limited to, the D-stereoisomers of amino acids. Exemplary non-natural amino acids are known to those of skill in the art, and include, but are not limited to, 2-Aminoadipic acid (Aad), 3-Aminoadipic acid (bAad), β-alanine/β-Amino-propionic acid (Bala), 2-Aminobutyric acid (Abu), 4-Aminobutyric acid/piperidinic acid (4Abu), 6-Aminocaproic acid (Acp), 2-Aminoheptanoic acid (Ahe), 2-Aminoisobutyric acid (Aib), 3-Aminoisobutyric acid (Baib), 2-Aminopimelic acid (Apm), 2,4-Diaminobutyric acid (Dbu), Desmosine (Des), 2,2'-Diaminopimelic acid (Dpm), 2,3-Diaminopropionic acid (Dpr), N-Ethylglycine (EtGly), N-Ethylasparagine (EtAsn), Hydroxylysine (Hyl), allo-Hydroxylysine (Ahyl), 3-Hydroxyproline (3Hyp), 4-Hydroxyproline (4Hyp), Isodesmosine (Ide), allo-Isoleucine (Aile), N-Methylglycine, sarcosine (MeGly), N-Methylisoleucine (Melle), 6-N-Methyllysine (MeLys), N-Methylvaline (MeVal), Norvaline (Nva), Norleucine (Nle), and Ornithine (Orn).

As used herein, a DNA construct is a single or double stranded, linear or circular DNA molecule that contain segments of DNA combined and juxtaposed in a manner not found in nature. DNA constructs exist as a result of human manipulation, and include clones and other copies of manipulated molecules.

As used herein, a DNA segment is a portion of a larger DNA molecule having specified attributes. For example, a DNA segment encoding a specified polypeptide is a portion of a longer DNA molecule, such as a plasmid or plasmid fragment, which, when read from the 5' to 3' direction, encodes the sequence of amino acids of the specified polypeptide.

As used herein, the term polynucleotide means a single- or double-stranded polymer of deoxyribonucleotides or ribonucleotide bases read from the 5' to the 3' end. Polynucleotides include RNA and DNA, and can be isolated from natural sources, synthesized *in vitro,* or prepared from a combination of natural and synthetic molecules. The length of a polynucleotide molecule is given herein in terms of nucleotides (abbreviated nt) or base pairs (abbreviated bp). The term nucleotides is used for single- and double-stranded molecules where the context permits. When the term is applied to double-stranded molecules it is used to denote overall length and will be understood to be equivalent to the term base pairs. It will be recognized by those skilled in the art that the two strands of a double-stranded polynucleotide can differ slightly in length and that the ends thereof can be staggered; thus, all nucleotides within a double-stranded polynucleotide molecule cannot be paired. Such unpaired ends will, in general, not exceed 20 nucleotides in length.

As used herein, production by recombinant methods refers means the use of the well-known methods of molecular biology for expressing proteins encoded by cloned DNA.

As used herein, heterologous nucleic acid is nucleic acid that encodes products (*i.e.,* RNA and/or proteins) that are not normally produced *in vivo* by the cell in which it is expressed, or nucleic acid that is in a locus in which it does not normally occur, or that mediates or encodes mediators that alter expression of endogenous nucleic acid, such as DNA, by affecting transcription, translation, or other regulatable biochemical processes. Heterologous nucleic acid, such as DNA, also is referred to as foreign nucleic acid. Any nucleic acid, such as DNA, that one of skill in the art would recognize or consider as heterologous or foreign to the cell in which it is expressed, is herein encompassed by heterologous nucleic acid; heterologous nucleic acid includes exogenously added nucleic acid that is also expressed endogenously. Heterologous nucleic acid is generally not endogenous to the cell into which it is introduced, but has been obtained from another cell or prepared synthetically or is introduced into a genomic locus in which it does not occur naturally, or its expression is under the control of regulatory sequences or a sequence that differs from the natural regulatory sequence or sequences.

Examples of heterologous nucleic acid herein include, but are not limited to, a DNA molecule, an RNA molecule, a plasmid, and an antisense oligonucleotide. In the MEV, the heterologous nucleic acid can be encoded on a plasmid. Heterologous nucleic acid, such as DNA, includes nucleic acid that can, in some manner, mediate expression of DNA that encodes a therapeutic product, or it can encode a product, such as a peptide or RNA, that in some manner mediates, directly or indirectly, expression of a therapeutic product.

As used herein, cell therapy involves the delivery of MEVs to a subject to treat a disease or condition. The MEVs are exogenously loaded with cargo, so that they deliver or express products when introduced to a subject. The MEVs also can be endogenously loaded with cargo (see, *e.g.*, copending U.S. provisional application Serial No. 63/349,006, filed on

June 03, 2022, which details preparation of endogenously-loaded MEVs and producer cell lines thereof), and used as described herein. The trafficking of MEVs generally is independent of manner in which they are loaded with cargo. The microalgae can be modified to alter properties of the resulting MEVs. Endogenously-loaded MEVs can be used in the methods and compositions described herein.

As used herein, genetic therapy involves the transfer of heterologous nucleic acid, such as DNA, into certain cells, such as target cells, of a mammal, particularly a human, with a disorder or condition for which such therapy is sought. The nucleic acid, such as DNA, is introduced into the selected target cells in a manner such that the heterologous nucleic acid, such as DNA, is expressed and a therapeutic product(s) encoded thereby is produced. Genetic therapy can also be used to deliver nucleic acid encoding a gene product that replaces a defective gene or supplements a gene product produced by the mammal or the cell in which it is introduced. The introduced nucleic acid can encode a therapeutic compound, such as a growth factor or inhibitor thereof, or a tumor necrosis factor or inhibitor thereof, such as a receptor thereof, that is not normally produced in the mammalian host or that is not produced in therapeutically effective amounts or at a therapeutically useful time. The heterologous nucleic acid, such as DNA, encoding the therapeutic product, can be modified prior to introduction into the cells of the afflicted host in order to enhance or otherwise alter the product or expression thereof. Genetic therapy can also involve delivery of an inhibitor or repressor or other modulator of gene expression.

As used herein, expression refers to the process by which polypeptides are produced by transcription and translation of polynucleotides. The level of expression of a polypeptide can be assessed using any method known in art, including, for example, methods of determining the amount of the polypeptide produced from the host cell. Such methods can include, but are not limited to, quantitation of the polypeptide in the cell lysate by ELISA, Coomassie blue staining following gel electrophoresis, Lowry protein assay and Bradford protein assay.

As used herein, a host cell is a cell that is used to receive, maintain, reproduce and/or amplify a vector. A host cell also can be used to express the polypeptide encoded by the vector. The nucleic acid contained in the vector is replicated when the host cell divides, thereby amplifying the nucleic acids.

As used herein, a vector is a replicable nucleic acid from which one or more heterologous proteins can be expressed when the vector is transformed into an appropriate host cell. Reference to a vector includes those vectors into which a nucleic acid encoding a polypeptide or fragment thereof can be introduced, typically by restriction digestion and ligation. Reference to a vector also includes those vectors that contain nucleic acid encoding a polypeptide or RNA. The vector is used to introduce the nucleic acid encoding the polypeptide into the host cell for amplification of the nucleic acid or for expression/display of the polypeptide encoded by the nucleic acid. The vectors typically remain episomal, but can be designed to effect integration of a gene or portion thereof into a chromosome of the genome. Also contemplated are vectors that are artificial chromosomes, such as yeast artificial chromosomes and mammalian artificial chromosomes. Selection and use of such vehicles are well-known to those of skill in the art. A vector also includes virus vectors or viral vectors. Viral vectors are engineered viruses that are operatively linked to exogenous genes to transfer (as vehicles or shuttles) the exogenous genes into cells.

As used herein, an expression vector includes vectors capable of expressing DNA that is operatively linked with regulatory sequences, such as promoter regions, that are capable of effecting expression of such DNA fragments. Such additional segments can include promoter and terminator sequences, and optionally can include one or more origins of replication, one or more selectable markers, an enhancer, a polyadenylation signal, and the like. Expression vectors are generally derived from plasmid or viral DNA, or can contain elements of both. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well-known to those of skill in the art and include those that are replicable in eukaryotic cells and/or prokaryotic cells and those that remain episomal or those which integrate into the host cell genome.

As used herein, primary sequence refers to the sequence of amino acid residues in a polypeptide or the sequence of nucleotides in a nucleic acid molecule.

As used herein, sequence identity refers to the number of identical or similar amino acids or nucleotide bases in a comparison between a test and a reference polypeptide or polynucleotide. Sequence identity can be determined by sequence alignment of nucleic acid or protein sequences to identify regions of similarity or identity. For purposes herein, sequence identity is generally determined by alignment to identify identical residues. The alignment can be local or global. Matches, mismatches and gaps can be identified between compared sequences. Gaps are null amino acids or nucleotides inserted between the residues of aligned sequences so that identical or similar characters are aligned. Generally, there can be internal and terminal gaps. When using gap penalties, sequence identity can be determined with no penalty for end gaps (*e.g.,* terminal gaps are not penalized). Alternatively, sequence identity can be determined without taking into account gaps as the number of identical positions/length of the total aligned sequence x 100.

As used herein, a global alignment is an alignment that aligns two sequences from beginning to end, aligning each letter in each sequence only once. An alignment is produced, regardless of whether or not there is similarity or identity between the sequences. For example, 50% sequence identity based on global alignment means that in an alignment of the

full sequence of two compared sequences each of 100 nucleotides in length, 50% of the residues are the same. It is understood that global alignment also can be used in determining sequence identity even when the length of the aligned sequences is not the same. The differences in the terminal ends of the sequences will be taken into account in determining sequence identity, unless the no penalty for end gaps is selected. Generally, a global alignment is used on sequences that share significant similarity over most of their length. Exemplary algorithms for performing global alignment include the Needleman-Wunsch algorithm (Needleman et al. (1970) J. Mol. Biol. 48: 443). Exemplary programs for performing global alignment are publicly available and include the Global Sequence Alignment Tool available at the National Center for Biotechnology Information (NCBI) website (ncbi.nlm.nih.gov/), and the program available at deepc2.psi.iastate.edu/aat/align/align.html.

As used herein, a local alignment is an alignment that aligns two sequences, but only aligns those portions of the sequences that share similarity or identity. Hence, a local alignment determines if sub-segments of one sequence are present in another sequence. If there is no similarity, no alignment will be returned. Local alignment algorithms include BLAST or Smith-Waterman algorithm (Adv. Appl. Math. 2: 482 (1981)). For example, 50% sequence identity based on local alignment means that in an alignment of the full sequence of two compared sequences of any length, a region of similarity or identity of 100 nucleotides in length has 50% of the residues that are the same in the region of similarity or identity.

For purposes herein, sequence identity can be determined by standard alignment algorithm programs used with default gap penalties established by each supplier. Default parameters for the GAP program can include: (1) a unary comparison matrix (containing a value of 1 for identities and 0 for non-identities) and the weighted comparison matrix of Gribskov et al. (1986) Nucl. Acids Res. 14:6745, as described by Schwartz and Dayhoff, eds., Atlas of Protein Sequence and Structure, National Biomedical Research Foundation, pp. 353-358 (1979); (2) a penalty of 3.0 for each gap and an additional 0.10 penalty for each symbol in each gap; and (3) no penalty for end gaps. Whether any two nucleic acid molecules have nucleotide sequences or any two polypeptides have amino acid sequences that are at least 80%, 85%, 90%, 95%, 96%, 97%, 98% or 99% identical, or other similar variations reciting a percent identity, can be determined using known computer algorithms based on local or global alignment (*see e.g.,* wikipedia.org/wiki/Sequence_alignment_software, providing links to dozens of known and publicly available alignment databases and programs). Generally, for purposes herein sequence identity is determined using computer algorithms based on global alignment, such as the Needleman-Wunsch Global Sequence Alignment tool available from NCBI/BLAST (blast.ncbi.nlm.nih.gov/Blast.cgi?CMD=Web&Page_TYPE=BlastHome); LAlign (William Pearson implementing the Huang and Miller algorithm (Adv. Appl. Math. (1991) 12:337-357)); and program from Xiaoqui Huang available at deepc2.psi.iastate.edu/aat/align/align.html. Typically, the full-length sequence of each of the compared polypeptides or nucleotides is aligned across the full-length of each sequence in a global alignment. Local alignment also can be used when the sequences being compared are substantially the same length.

Therefore, as used herein, the term identity represents a comparison or alignment between a test and a reference polypeptide or polynucleotide. In one nonlimiting example, at least 90% identical to refers to percent identities from 90 to 100% relative to the reference polypeptide or polynucleotide. Identity at a level of 90% or more is indicative of the fact that, assuming for exemplification purposes a test and reference polypeptide or polynucleotide length of 100 amino acids or nucleotides are compared, no more than 10% (*i.e.,* 10 out of 100) of amino acids or nucleotides in the test polypeptide or polynucleotide differ from those of the reference polypeptide. Similar comparisons can be made between a test and reference polynucleotides. Such differences can be represented as point mutations randomly distributed over the entire length of an amino acid sequence or they can be clustered in one or more locations of varying length up to the maximum allowable, *e.g.*, 10/100 amino acid difference (approximately 90% identity). Differences also can be due to deletions or truncations of amino acid residues. Differences are defined as nucleic acid or amino acid substitutions, insertions or deletions. Depending on the length of the compared sequences, at the level of homologies or identities above about 85-90%, the result can be independent of the program and gap parameters set; such high levels of identity can be assessed readily, often without relying on software.

As used herein, a pharmaceutically effective agent includes any therapeutic agent or bioactive agents, including, but not limited to, for example, anesthetics, vasoconstrictors, dispersing agents, and conventional therapeutic drugs, including small molecule drugs and therapeutic proteins.

As used herein, a therapeutic effect means an effect resulting from treatment of a subject that alters, typically improves or ameliorates, the symptoms of a disease or condition or that cures a disease or condition.

As used herein, a therapeutically effective amount or a therapeutically effective dose refers to the quantity of an agent, compound, material, or composition containing a compound that is at least sufficient to produce a therapeutic effect following administration to a subject. Hence, it is the quantity necessary for preventing, curing, ameliorating, arresting or partially arresting a symptom of a disease or disorder.

As used herein, therapeutic efficacy refers to the ability of an agent, compound, material, or composition containing a compound to produce a therapeutic effect in a subject to whom the agent, compound, material, or composition containing a compound has been administered.

As used herein, a prophylactically effective amount or a prophylactically effective dose refers to the quantity of an

agent, compound, material, or composition containing a compound that when administered to a subject, will have the intended prophylactic effect, *e.g.,* preventing or delaying the onset, or reoccurrence, of disease or symptoms, reducing the likelihood of the onset, or reoccurrence, of disease or symptoms, or reducing the incidence of viral infection. The full prophylactic effect does not necessarily occur by administration of one dose, and can occur only after administration of a series of doses. Thus, a prophylactically effective amount can be administered in one or more administrations.

As used herein, amelioration of the symptoms of a particular disease or disorder by a treatment, such as by administration of a pharmaceutical composition or other therapeutic, refers to any lessening, whether permanent or temporary, lasting or transient, of the symptoms that can be attributed to or associated with administration of the composition or therapeutic.

As used herein, an anti-cancer agent refers to any agent that is destructive or toxic to malignant cells and tissues. For example, anti-cancer agents include agents that kill cancer cells or otherwise inhibit or impair the growth of tumors or cancer cells. Exemplary anti-cancer agents are chemotherapeutic agents.

As used herein therapeutic activity refers to the *in vivo* activity of a therapeutic polypeptide. Generally, the therapeutic activity is the activity that is associated with treatment of a disease or condition.

As used herein, the term subject refers to an animal, including a mammal, such as a human being.

As used herein, a patient refers to a human subject.

As used herein, animal includes any animal, such as, but not limited to, primates including humans, gorillas and monkeys; rodents, such as mice and rats; fowl, such as chickens; ruminants, such as goats, cows, deer, and sheep; and pigs and other animals. Non-human animals exclude humans as the contemplated animal.

As used herein, a composition refers to any mixture. It can be a solution, suspension, liquid, powder, paste, aqueous, non-aqueous, or any combination thereof.

As used herein, a combination refers to any association between or among two or more items. The combination can be two or more separate items, such as two compositions or two collections, a mixture thereof, such as a single mixture of the two or more items, or any variation thereof. The elements of a combination are generally functionally associated or related.

As used herein, combination therapy refers to administration of two or more different therapeutics. The different therapeutic agents can be provided and administered separately, sequentially, intermittently, or can be provided in a single composition.

As used herein, a kit is a packaged combination that optionally includes other elements, such as additional reagents and instructions for use of the combination or elements thereof, for a purpose including, but not limited to, activation, administration, diagnosis, and assessment of a biological activity or property.

As used herein, a unit dose form refers to physically discrete units suitable for human and animal subjects and packaged individually as is known in the art.

As used herein, a single dosage formulation refers to a formulation for direct administration.

As used herein, a multi-dose formulation refers to a formulation that contains multiple doses of a therapeutic agent and that can be directly administered to provide several single doses of the therapeutic agent. The doses can be administered over the course of minutes, hours, weeks, days or months. Multi-dose formulations can allow dose adjustment, dose-pooling and/or dose-splitting. Because multi-dose formulations are used over time, they generally contain one or more preservatives to prevent microbial growth.

As used herein, an article of manufacture is a product that is made and sold. As used throughout this application, the term is intended to encompass any of the compositions provided herein contained in articles of packaging.

As used herein, a fluid refers to any composition that can flow. Fluids thus encompass compositions that are in the form of semi-solids, pastes, solutions, aqueous mixtures, gels, lotions, creams and other such compositions.

As used herein, an isolated or purified polypeptide or protein (*e.g.,* an isolated antibody or antigen-binding fragment thereof) or biologically-active portion thereof (*e.g.,* an isolated antigen-binding fragment) is substantially free of cellular material or other contaminating proteins from the cell or tissue from which the protein is derived, or substantially free from chemical precursors or other chemicals when chemically synthesized. Preparations can be determined to be substantially free if they appear free of readily detectable impurities as determined by standard methods of analysis, such as thin layer chromatography (TLC), gel electrophoresis and high performance liquid chromatography (HPLC), used by those of skill in the art to assess such purity, or sufficiently pure such that further purification does not detectably alter the physical and chemical properties, such as enzymatic and biological activities, of the substance. Methods for purification of the compounds to produce substantially chemically pure compounds are known to those of skill in the art. A substantially chemically pure compound, however, can be a mixture of stereoisomers. In such instances, further purification might increase the specific activity of the compound.

As used herein, a cellular extract or lysate refers to a preparation or fraction which is made from a lysed or disrupted cell.

As used herein, a control refers to a sample that is substantially identical to the test sample, except that it is not treated with a test parameter, or, if it is a plasma sample, it can be from a normal volunteer not affected with the condition of

interest. A control also can be an internal control.

As used herein, psilocin is the active form of psilocybin. Psilocin is produced by oxidation of psilocybin in the liver. For purposes herein, in the context of MEV-mediated delivery, psilocybin and psilocin should have the same meaning: a mention to 'psilocybin' shall mean to 'psilocin' and vice versa.

As used herein, bregma is a unit that measures the distance between a location in the brain and the point of junction between the coronal and the sagittal sutures of the skull.

As used herein, a tropism of an MEV refers to cells, tissues, and/or organs in where the MEVs, upon administration, accumulate.

As used herein, natural tropism with reference to the MEVs provided herein, refers to the means: MEV are not modified to provide a specific tropism or targeting property.

As used herein, the singular forms "a," "an" and "the" include plural referents unless the context clearly dictates otherwise. Thus, for example, reference to a polypeptide, comprising an immunoglobulin domain includes polypeptides with one or a plurality of immunoglobulin domains.

As used herein, the term "or" is used to mean "and/or" unless explicitly indicated to refer to alternatives only or the alternatives are mutually exclusive.

As used herein, ranges and amounts can be expressed as about a particular value or range. "About" also includes the exact amount. Hence about 5 amino acids means about 5 amino acids and also 5 amino acids.

As used herein, "optional" or "optionally" means that the subsequently described event or circumstance does or does not occur and that the description includes instances where said event or circumstance occurs and instances where it does not. For example, an optionally variant portion means that the portion is variant or non-variant.

As used herein, the abbreviations for any protective groups, amino acids and other compounds, are, unless indicated otherwise, in accord with their common usage, recognized abbreviations, or the IUPAC-IUB Commission on Biochemical Nomenclature (see, Biochem. (1972) 11(9):1726-1732).

For clarity of disclosure, and not by way of limitation, the detailed description is divided into the subsections that follow.

## B. MICROALGAE AND OVERVIEW

Algae are a complex, polyphyletic collection of predominantly photosynthetic organisms. These organisms include micro- and macroscopic forms. Macroalgae (seaweed) are multicellular, large-size algae, visible with the naked eye. Microalgae are microscopic single cells and include prokaryotes (*e.g.*, cyanobacteria), and eukaryotes, such as green algae.

Compared to photosynthetic crops, microalgae have a higher growth rate and can be cultivated on non-arable land, and also in bioreactors. Many species of microalgae can be grown year-round in industrial scale photobioreactors under controlled cultivation conditions (Adamo et al. (2021) Journal of Extracellular Vesicles 10:e12081).

Algae generally are classified into eleven major phyla: Cyanophyta, Chlorophyta, Rhodophyta, Glaucophyta, Euglenophyta, Chlorarachniophyta, Charophyta, Cryptophyta, Haptophyta, Heterokontophyta, and Dinophyta (Barkia et al. (2019) Mar. Drugs 17(5):304). Different pigments occur in each algae group. Cyanobacteria (or Cyanophyta) contain chlorophyll-a, -d, and -f, in addition to the phycobiliproteins (proteins that capture light energy), phycocyanin, allophycocyanin, and phycoerythrin. Glaucophytes contain chlorophyll-a and harvest light via phycobiliproteins. Chlorophytes have chlorophyll-a and -b, as well as carotenoids, including β-carotene and various xanthophylls (*e.g.*, astaxanthin, canthaxanthin, lutein, and zeaxanthin). The primary pigments of Rhodophyta (red algae) are phycoerythrin and phycocyanin, which can mask chlorophyll-a; red algae also produce a broad spectrum of carotenes and xanthophyll light-harvesting pigments (Barkia et al. (2019) Mar. Drugs 17(5):304).

Provided herein are extracellular vesicles produced by algae, particularly unicellular green algae, such as species of *Chlorella,* for use for delivery of exogenously loaded cargo to animals and plants. The algae are unicellular eukaryotes that typically are haploid, but can have a diploid stage of the life cycle. The algae can be cultured in bioreactors and the extracellular vesicles isolated therefrom. The resulting extracellular vesicles can be loaded by methods such as electroporation, with cargo, generally a cargo of heterologous bioactive molecules to produce compositions that contain the extracellular vesicles for administration to animals and also to plants. The compositions can be formulated for any desired route of administration, including topical, local, systemic, parenteral, and oral. These routes include oral, intravenous, subcutaneous, inhalation, mucosal, rectal, vaginal, and other suitable routes. The cargo includes biomolecules, such as DNA, RNA, proteins, protein complexes, protein-nucleic acid complexes, plasmids, and also includes small molecules, such as small molecule drugs. The extracellular vesicles can be formulated as liquids, powders, including lyophilized powders, tablets, capsules, emulsions, particles, sprays, gels, ointments, creams, and other formulations. They can be used for therapeutic, diagnostic, theragnostic, cosmetic, and other uses. The extracellular vesicles can be used to treat diseases and conditions, that include cancers, inflammatory diseases and conditions in which the immune system plays a role in the etiology or symptoms, nervous system disorders, and pathogen infections, including viral and

bacterial and other pathogens. They can be used to treat dermatological diseases and conditions, lung diseases and conditions, and gastric diseases and conditions. The extracellular vesicles can be targeted to specific organs or tissues or can be locally administered.

As with extracellular vesicles (EVs) from other sources, such as mammalian EVs, microalgae EVs (MEVs) have evolved to efficiently pass genetic material and other kinds of molecules from cell to cell. They orchestrate intercellular and cross-kingdom communication between cells via exchange of biologically active molecules. MEV are natural nanoparticles. They are cell-derived, so, absent synthetic cargo, and genetic modifications, there are no synthetic components; they are safe, for example, there is no risk of endogenous viruses that are potentially dangerous to humans.

The MEVs provided herein include *Chlorella* MEVs, particularly *Chlorella vulgaris,* a freshwater microalgae. *Chlorella* is a unicellular haploid alga that is a natural and efficient producer of extracellular vesicles. *Chlorella vulgaris* has been consumed worldwide as a food supplement for decades; it is non-toxic and non-immunogenic, and can be cultured at large industrial scale at low cost. The MEVs provided herein can be directly used to protect, convey, and deliver a broad spectrum of innovative therapeutic molecules into target cells relevant to specific diseases.

As shown and described herein, the MEVs have a number of advantageous features including, for example, biodistribution patterns by route of administration, low toxicity, good pharmacokinetic profiles *in vivo.* They can be administered by a variety of routes including oral administration, administration to the respiratory tract, intranasally, intravenously, and among other routes. They traffic to specific organs, according to the route of administration, such as the intestine, the GALT, the spleen, the lungs, the liver, and the brain. Based on data herein and comparison with data for other EVs and drug delivery systems, the MEVs can have longer clearance rates so that they last longer in the targeted organs, tissues, and cells than reported for other delivery systems, including mammalian EVs.

As shown herein, the MEVs overcome natural body barriers (such as oral delivery, or specific lymphoid tissues delivery, or nose-to-brain delivery) that have not been attained with liquid nano-particles and EVs of mammalian origin.

The MEVs provided herein address unmet needs. These include the ability to convey and reliably deliver therapeutic molecules specifically to the site of treatment, while avoiding premature degradation or inactivation of the therapeutic agent by the immune system or by enzymes; for treatment of diseases for which a therapeutic agent already exists but cannot be properly delivered.

As shown herein, the purified or partially-purified MEVs can be loaded by physical methods (exogenous loading; exo-loading). Exo-loading is scalable and industrializable. The MEVs can be exo-loaded with a variety of molecules, varying in size, hydrophobicity, and nature, such as siRNA, mRNA, peptides, proteins, plasmids, oligonucleotides, and small molecules. The biological activity of the exo-loaded cargo is preserved, while at the same time it is protected from degradation by enzymes and other agents present *in vivo.* The MEVs can deliver their cargo to recipient cells of a myriad of origins, such as microalgae, bacteria, higher plant, mammal, and human. MEVs can also deliver the cargo to the proper cell compartments, ensuring the proper expression and biological activity of cargo molecules, including those having complex biological pathways such as siRNA, mRNA, receptor-binding peptides, among others.

## C. EXTRACELLULAR VESICLES

Extracellular vesicles (EVs) are biomolecular structures released from plant and animal cells that play a role in cell-to-cell communication. Structurally, EVs are negatively charged lipid bilayer vesicles with a density of 1.13 to 1.19 g/mL. EVs are able to cross barriers such as the plasma (or cytoplasmic) membrane and the blood/brain barrier, and enable the horizontal transfer of their functional contents (*i.e.,* proteins, lipids, RNA molecules, and circulating DNA) from a donor to a recipient cell (Kuruvinashetti et al. (2020) 20th International Conference on Nanotechnology 354-357). EVs also are naturally stable in various biological fluids, immunologically inert, and can exhibit organ-specific targeting abilities (Picciotto et al. (2021) Biomater. Sci. doi:10.1039/d0bm01696a).

EVs contain endogenous lipids, nucleic acids, and proteins. Although results differ due to variations in isolation techniques and methods of analyzing the data, EVs generally contain proteins associated with the plasma membrane, cytosol and those involved in lipid metabolism (see, *e.g.,* Doyle and Wang (2019) Cells 8(7):727). Proteins involved in the biogenesis of EVs (*e.g.*, components of the ESCRTs), EV formation and release (*e.g.,* RAB27A, RAB11B, and ARF6), signal transduction, and antigen presentation, as well as tetraspinins commonly occur in EVs (Abels and Breakefield (2016) Mol. Neurobiol. 36(3):301-312). EVs are enriched for cholesterol, sphingomyelin, glycosphingolipids, and phosphatidylserine (Kuruvinashetti et al. (2020) 20th International Conference on Nanotechnology 354-357). Although a small number of studies have identified genomic and mitochondrial DNA in EVs, EVs are primarily enriched with endogenous small RNAs. Studies have identified mRNAs, miRNAs, rRNAs, long and short non-coding RNA, tRNA fragments, piwi-interacting RNA, vault RNA, and Y RNA in EVs. Most of the RNA that naturally occurs in EVs is ~200 nucleotides long (with a small portion up to 4 kb) and thus it is fragmented, although circular RNAs also have been shown to be enriched and stable in EVs. RNA in EVs is protected from RNase digestion in the extracellular environment by the lipid bilayer (Abels and Breakefield (2016) Mol. Neurobiol. 36(3):301-312). The Exocarta, Vesiclepedia, and EVpedia databases are publicly available and provide data on the protein, nucleic acid, and lipid content of EVs (generally EVs

from mammalian origin, such as human origin), as well as the isolation and purification procedures used, from EV studies (Abels and Breakefield (2016) Mol. Neurobiol. 36(3):301-312).

EVs are used by cells to mediate several physiological processes or affect various pathological conditions associated with the activation of an immune response or the spread of disease or infection, and also constitute cross-species communication and are in all kingdoms of life. Sources of EVs include mammalian cells, bacteria, bovine milk and plants (Adamo et al. (2021) J. Extracell. Vesicles 10:e12081). Although plants and algae possess a cell wall outside their plasma membrane, which could be a physical barrier for the release of EVs, plants and algae release EVs (Picciotto et al. (2021) Biomater. Sci. doi:10.1039/d0bm01696a).

## 1. Types of Extracellular Vesicles (EVs)

### a. Exosomes

There are three primary subtypes of EVs; they are classified based on their biogenesis, mode of release, size, content, and function: microvesicles (MVs), exosomes, and apoptotic bodies (Doyle and Wang (2019) Cells 8(7):727). Exosomes, or intraluminal vesicles (ILVs) generally are 30-150 nm in diameter and are released through multivesicular bodies (MVBs) in the endosomal pathway. In the endosomal pathway, early endosomes form by inward budding of the plasma membrane and can transform into late endosomes, which accumulate ILVs by inward budding of the endosomal membrane. Late endosomes which contain a number of small vesicles are called MVBs. MVBs either fuse with the lysosome and are degraded, or the plasma membrane which releases the ILVs as exosomes into the extracellular space. The endosomal sorting complexes required for transport (ESCRT) pathway regulates MVB transportation and exosome formation and is reported to be the primary driver of exosome biogenesis, although other mechanisms of exosome biogenesis exist, including those mediated by the sphingolipid ceramide, which can facilitate membrane invagination, or proteins in the tetraspanin family. The ESCRT accessory proteins Alix, TSG101, HSC70 and HSP90β are often referred to as exosomal marker proteins (Doyle and Wang (2019) Cells 8(7):727).

Exosomes are released into the extracellular space by the fusion of the MVB limiting membrane with the plasma membrane. A number of proteins are involved in the release of exosomes, including Rab GTPases, diacylglycerol kinase α, and SNARE proteins (Abels and Breakefield (2016) Cell Mol. Neurobiol. 36(3):301-312).

Exosomes are candidates for drug delivery systems: they have a long circulating half-life; exosomes are tolerated by the human body and can penetrate cell membranes and target specific cell types; and they can be loaded with genetic material, a protein, or a small molecule (Doyle and Wang (2019) Cells 8(7):727).

### b. Microvesicles

Microvesicles (MVs, or ectosomes) form by outward budding, or pinching, of the cell's plasma membrane, and have a diameter of 100 nm to 1 μm. The formation of MVs involves cytoskeleton components, such as actin and microtubules, molecular motors such as kinesins and myosins, and fusion machinery such as SNAREs and tethering factors. The physiological state and microenvironment of the donor cell effects the number of MVs produced, and the physiological state and microenvironment of the recipient cell effects the number of MVs consumed. MVs also have a number of marker proteins, including cytosolic and plasma membrane associated proteins, as well as cytoskeletal proteins, heat shock proteins, integrins, and proteins containing post-translational modifications, although there are no known specific markers to distinguish MVs from exosomes. Like exosomes, MVs can be loaded with cargo (such as proteins, nucleic acids, and lipids) for delivery to another cell, thereby altering the recipient cell's functions (Doyle and Wang (2019) Cells 8(7):727).

### c. Apoptotic Bodies

Apoptotic bodies are released by dying cells into the extracellular space, and have a diameter from 50 nm to 5000 nm. Apoptotic bodies are formed when the cell's plasma membrane separates from the cytoskeleton due to increased hydrostatic pressure after the cell contracts. Unlike exosomes and MVs, apoptotic bodies contain intact organelles, chromatin, and small amounts of glycosylated proteins (Doyle and Wang (2019) Cells 8(7):727).

## 2. Uptake of EVs

Cells internalize EVs by fusion with the plasma membrane, or more commonly by endocytosis (Abels and Breakefield (2016) Cell Mol. Neurobiol. 36(3):301-312). Uptake via endocytosis can be through several types of endocytotic processes, and different processes have been described in different cell types: clathrin-dependent endocytosis and phagocytosis have been described in neurons, macropinocytosis in microglia, phagocytosis and receptor-mediated endocytosis in dendritic cells, caveolin-mediated endocytosis in epithelial cells, and cholesterol- and lipid raft-dependent endocytosis in tumor cells. Blocking heparin sulfate proteoglycans (HSPGs) on the plasma membrane with heparin reduces the uptake of EVs in cell culture, as does blocking the scavenger receptor type B-1 (SR-B1) with a synthetic nanoparticle mimic of HDL, which suggests a role for HSPGs and SR-B1 in EV uptake (Abels and Breakefield (2016) Cell Mol. Neurobiol. 36(3):301-312). Fusion of EVs with the plasma membrane also is a method of uptake, and requires low pH conditions; treatment of EVs with the combination of a pH-sensitive fusogenic peptide with cationic lipids resulted in increased cellular uptake of exosomes and the cytosolic release of cargo within the exosomes (Nakase and Futaki (2015) Sci. Rep. 5:10112). Low pH conditions occur in tumors (Abels and Breakefield (2016) Cell Mol. Neurobiol. 36(3):301-312), so that EVs for delivering therapeutic payloads to tumor cells can enter cells through fusion with the plasma membrane.

Like cells, EVs have extracellular receptors and ligands on the outside and cytoplasmic proteins and nucleic acid on the inside, and thus communicate with cells in different ways. EVs bind to the cell surface, undergo endocytosis, and/or fuse with the plasma membrane, and release their cargos in the extracellular space. If entering by endocytosis, the EV cargo must escape the degradative pathway; late endosomes can fuse with lysosomes or the plasma membrane, so cargo must exit before it is degraded in a lysosome or re-released through the fusion of MVBs with the plasma membrane. EVs containing cargo, including mRNAs and non-coding RNAs, can be transferred to recipient cells in culture and *in vivo* (Abels and Breakefield (2016) Cell Mol. Neurobiol. 36(3):301-312; Maas et al. (2017) Trends Cell Biol. 27(3):172-188).

### 3. General Methods for Isolating EVs

#### a. Ultracentrifugation

Ultracentrifugation methods are used to isolate exosomes; alternative methods also have been developed. Due to the complex nature of the biological fluids from which exosomes are isolated, the overlap in physiochemical and biochemical properties between exosomes and other types of EVs, and the heterogeneity among exosomes, isolation methods can result in complex mixtures of EVs and other components of the extracellular space. Differential ultracentrifugation depends on the initial sedimentation of larger and denser particles from the extracellular matrix, and results in an enrichment of exosomes, but not a complete separation of exosomes from other components in the extracellular space. Density gradient centrifugation is another ultracentrifugation method and is based on separation by size and density in the presence of a density gradient (typically made of sucrose or iodixanol) in the centrifuge tube. Density gradient centrifugation effectively separates EVs from protein aggregates and non-membranous particles but has low exosome recovery, although purity can be improved by coupling differential ultracentrifugation with types of density gradient centrifugation, such as rate-zonal centrifugation or isopycnic centrifugation (Doyle and Wang (2019) Cells 8(7):727).

#### b. Size-Based Techniques

There are a number of size-based techniques for isolating exosomes (Doyle and Wang (2019) Cells 8(7):727). Ultrafiltration separates particles based on the size and molecular weight cut off of the membrane, whereby particles larger than the molecular weight cut off of the membrane are retained, and particles smaller than the molecular weight cut off of the membrane are passed through into the filtrate; low isolation efficiency can occur however if the filter becomes clogged and vesicles become trapped. The ExoMir™ Kit (Bioo Scientific; Austin, TX) is a commercially available kit in which two membranes (200 nm and 20 nm) are placed into a syringe and a sample (typically pre-treated with centrifugation and proteinase K) is passed through the syringe; the larger vesicles remain above the first 200 nm filter, the smallest vesicles are passed through the syringe and discarded, and the vesicles between 20 and 200 nm remain between the two filters in the syringe. Sequential filtration also relies on a series of filtration steps to isolate exosomes (Doyle and Wang (2019) Cells 8(7):727).

Size Exclusion Chromatography (SEC), often used in parallel with ultracentrifugation methods (in which the exosome pellet obtained from ultracentrifugation is resuspended and further purified using SEC), of exosomes is similar to using SEC to separate proteins. In SEC, a column is packed with a porous stationary phase in which small particles can penetrate and thus elute after larger particles. Typically, SEC methods require several hours of run time; however, the qEV Exosome Isolation Kit (iZON Science, New Zealand) allows for rapid and precise exosome isolation by SEC within 15 minutes (Doyle and Wang (2019) Cells 8(7):727).

In Flow Field-Flow Fractionation (FFFF), a sample injected into a chamber is subjected to parabolic flow as it is pushed down the chamber, in addition to a flow perpendicular to the parabolic flow, a crossflow, to separate particles in the sample. Larger particles are more affected by the crossflow and are pushed toward the walls of the chamber, which have a slower parabolic flow, and smaller particles remain in the center. Smaller particles elute earlier, and larger particles later, in FFFF (Doyle and Wang (2019) Cells 8(7):727).

In Hydrostatic Filtration Dialysis (HFD), hydrostatic pressure forces a sample through a dialysis tube with a

membrane having a molecular weight cut off of 1000 kDa. The result is that small solutes are able to pass through the tube, but larger particles, including exosomes and EVs, remain in the tube and can then be further separated using, for example, ultracentrifugation (Doyle and Wang (2019) Cells 8(7):727).

**c. Immunoaffinity Capture-Based Techniques**

Immunoaffinity capture-based techniques can isolate exosomes based on expression of an antigen on the surface of the exosome, and allow for the isolation of exosomes derived from a particular source. In these methods, an antibody specific for a target antigen can be attached to a plate (*e.g.,* in Enzyme-Linked Immunosorbent Assay, ELISA), magnetic beads (*e.g.,* in magneto-immunoprecipitation), resins and microfluidic devices; these surfaces are then exposed to the exosome sample, resulting in the immobilization of the exosomes expressing the antigen. This assay requires that the protein/antigen for isolating the exosomes be expressed on the surface of the exosomes, and its specificity is limited by the specificity of the antibody that is used, often resulting in a lower yield but higher purity of isolated exosomes. These methods also can be used to separate exosomes within mixed populations of EVs. Immunoaffinity capture-based techniques often are used after ultracentrifugation or ultrafiltration (Doyle and Wang (2019) Cells 8(7):727).

**d. Exosome Precipitation**

Methods for precipitation of exomes include precipitation by polyethylene glycol (PEG) and lectin. In PEG precipitation, the PEG polymer ties-up the water molecules, allowing the other particles, including exosomes, to precipitate out of solution. PEG precipitation is quick and is not limited to the starting volume of solution, but lacks selectivity, as other EVs, extracellular proteins, and protein aggregates are precipitated with EVs. Sample pretreatment using filtration and/or ultracentrifugation can improve exosome yield. Commercially available kits for isolating exosomes using precipitation include, for example, ExoQuick® (System Biosciences, Palo Alto, CA) and Invitrogen® Total Exosome Isolation Kit (Thermo Fisher Scientific, Waltham, MA). Alternatively, lectin precipitation can be used, typically after ultracentrifugation, whereby lectins bind to carbohydrates on the surface of exosomes, altering their solubility and leading to their precipitation out of solution (Doyle and Wang (2019) Cells 8(7):727).

e. Microfluidic Based Isolation Techniques

Microfluidic based techniques isolate exosomes based on their physical and biochemical properties simultaneously, and are rapid, efficient, and require small starting volumes. In acoustic nanofilter, a matrix containing EVs and other cellular components is injected into a chamber and exposed to ultrasound waves. The particles respond differently to the radiation forces exerted by the waves, depending on their size and density; large particles experience stronger forces and migrate faster toward the pressure nodes. The immuno-based microfluidic isolation technique is similar to that of an ELISA, although, unlike ELISAs, it does not require prior ultrafiltration or ultracentrifugation of exosomes (Doyle and Wang (2019) Cells 8(7):727). The ExoChip (Kanwar et al. (2014) Lab Chip. 14(11):1891-1900) and ExoSearch Chip (Zhao et al. (2016) Lab Chip. 16(3):489-496) have been developed to isolate exosomes using microfluidic technology.

**4. Microalgae and Microalgae-Derived Extracellular Vesicles (MEVs)**

Taxonomy and classification of microalgae can vary. According to some schemes there are seven (7) divisions of microalgae: Euglenophyta (Euglenoids), Chrysophyta (Golden-brown algae and Diatoms), Pyrrophyta (Fire algae), Chlorophyta (Green algae), Rhodophyta (Red algae), Phaeophyta (Brown algae), and Xanthophyta (Yellow-green algae). Of interest herein are photosynthetic microalgae, such as the species *Chlorella* and *Chlamydomonas.* The methods and uses described herein use MEVs generally from green algae. Exemplary of such algae are *Chlamydomonas* and *Chlorella,* which belong to the classes Chlorophyceae and Trebouxiophyceae, respectively.

Microalgae are bioresources for the production of EVs for use in nanomedicine and other fields. The mechanism of secretion of EVs from microalgae is known in relation to primary and motile cilia/flagella (Picciotto et al. (2021) Biomater. Sci. doi:10.1039/d0bm01696a). *Chlamydomonas* flagella are devoid of MVBs, thus, ciliary EVs shed from *Chlamydomonas* are classified as ectosomes. Studies have shown the shedding of ectosomes from flagellar and ciliary tips of the chlorophyte *Chlamydomonas reinhardtii.* EVs also have been observed along the length of the cilium in *Chlamydomonas.* Membrane budding and ciliary EV formation are mediated by components of the endosomal sorting complex required for transport (ESCRT), which are found in isolated ciliary transition zones, ciliary membranes, and ciliary EVs in *Chlamydomonas* and can act as sensors of membrane curvature. The formation of ciliary EVs also can occur when ciliary membrane trafficking is disrupted or during ciliary resorption (Wang and Barr (2018) Essays Biochem. 62(2):205-213). Ciliary ectosomes from *Chlamydomonas* contain a lytic enzyme that digests the mother cell wall and is required for the release of daughter cells. *Ift88-null* mutants that do not have flagella were unable to be released from the mother cell, and

the addition of ciliary ectosomes from wild-type cells rescued the phenotype, suggesting a role for the flagella and intraflagellar transport (IFT) machinery in EV production (Wang and Barr (2016) Cell Mol. Neurobiol. 36(3):449-457).

EVs have been extracted from algal cells using ultra-centrifugation (Kuruvinashetti et al. (2020) 20th International Conference on Nanotechnology 354-357). In accord with this method, algal cells are cultured; the cultured algal cells are collected and centrifuged; the supernatant is collected (and further centrifuged); a sucrose solution is added to the supernatant; and the algal supernatant with the sucrose solution is ultra-centrifuged; because of the sucrose solution, the high-density EVs settle at the bottom of the ultra-centrifugation tube and can be collected using a pipette. Extracted algal EVs can be characterized in size and concentration using Nanoparticle Tracking Analysis (NTA). Studies using this method have isolated green algal EVs that range in size from 25-200 nm, with a concentration of 0.89E8 to 0.94E8 particles/mL (Kuruvinashetti et al. (2020) 20th International Conference on Nanotechnology 354-357).

An ultra-centrifugation protocol also can be used to isolate EVs from marine microalgae grown under various conditions; NTA showed that the nano-particles have a size distribution between 100 and 200 nm, and western blotting of proteins confirmed the presence of EV markers (VES4US, Extracellular vesicles from a natural source for tailor-made nanomaterials, 2020). Subsequent studies have identified microalgal small EVs (sEVs) isolated from the marine photosynthetic microalgal chlorophyte *Tetraselmis chuii,* termed nanoalgosomes. The production of nanoalgosomes is an evolutionarily conserved trait within microalgal strains as similar results were obtained using sEVs isolated from batch cultures of two other microalgae species, the chlorophyte *Dunaliella tertiolecta,* and the dinoflagellate *Amphidinium sp.* The nanoalgosomes were isolated using differential centrifugation (dUC) and tangential flow filtration (TFF), as well as gradient ultracentrifugation, which was used to further purify samples enriched for small EVs by TFF or dUC. The isolated nanoalgosomes were shown to share characteristics of EVs from other sources. The EV yield (measured by sEV protein content and sEV number) from dUC and TFF was consistent with reported numbers of isolated EVs, around $10^9$ EV particles/$\mu$g EV proteins. Biophysical analysis of particle size using multi-angle dynamic light scattering (DLS), nanoparticle tracking analysis (NTA), fluorescence nanoparticle tracking analysis (F-NTA), and fluorescence correlation spectroscopy (FCS) yielded consistent size distributions, with the size that appeared the most frequently from DLS (DLS mode) around 70 nm. Compared to exosomes derived from mammalian cells, which have a density of 1.15-1.19 g/mol, nanoalgosomes had a slightly lower density of 1.13 g/mol. Electron microscopy revealed that the nanoalgosomes are spherical, heterogeneous in size and shape, and possess a lipid-bilayer structure. Compared to the microvesicles (or large EVs, lEVs) and lysates, the sEVs were enriched for three of the four target protein biomarkers (Alix, enolase, HSP70 and $\beta$-actin). DLS measurements indicated that the nanoalgosomes were resistant to changes in pH and stable in human blood plasma. The tumorigenic MDA-MB-231 breast cancer cell line, the non-tumorigenic 1-7 HB2 cell line, and the human hepatocarcinoma Hep G2 cell line did not show cytotoxic or genotoxic effects after nanoalgosome treatment. Furthermore, the nanoalgosome were taken up by the MDA-MB-231 and 1-7 HB2 cell lines (Adamo et al. (2021) J. Extracell. Vesicles 10:e12081).

EVs have been isolated from at least eighteen microalgae strains (*Ankistrodesmus sp., Brachiomonas sp., Chlamydomonas reinhardtii, Dunaliella tertiolecta, Tetraselmis chuii, Chloromotias sp., Rhodella violacea, Kirchneriella sp., Pediastrum sp., Nannochloropsis sp., Cyanophora paradoxa, Cryptomonas pyrenoidifera, Phaeodactylum tricornutum, Phaeothamnion sp., Diacronema sp., Isochrysis galbana, Stauroneis sp.,* and *Amphidinium sp*.) from the main microalgal lineages that have been studied, including strains with a variety of features such as saltwater and freshwater inhabitants, small and large sized cells, colonial and single cells, and species with sequenced genomes.

MEVs can be isolated using a differential ultracentrifugation protocol and characterized following the International Society for Extracellular Vesicles (ISEV) guidelines. All strains tested showed the presence of MEVs in the culture medium. EV-producing microalgae strains were established based on the EV protein content, the expression of EV protein markers (*e.g.*, Alix, Hsp70, enolase, and $\beta$-actin), the total scattering signal (measured by dynamic light scattering, DLS) or total particle number (measured by NTA), and the sEV average size and size range. These EV-producing strains include *Cyanophora paradoxa, Tetraselmis chuii, Amphidinium sp., Rhodella violacea, Djacronerna sp., Dunaliella tertiolecta, Phaeodactylum tricornutum, Pediastrum sp.,* and *Phaeothamnion sp.* (Picciotto et al. (2021) Biomater. Sci. doi:10.1039/d0bm01696a). The data for *Cyanophora paradoxa* showed ~$2\times10^9$ sEV particles per mL of microalgal-conditioned media, with strong positive signals for EV markers, and a size distribution with a mode of 130 $\pm$ 5 nm, in agreement with data from plant-derived vesicles. Cytotoxicity and genotoxicity studies showed that sEVs isolated from *Cyanophora paradoxa,* a freshwater Glaucophyte, did not show toxicity on the tumorigenic MDA-MB-231 breast cancer or C2C12 myoblast cell lines, neither over time nor at different concentrations, nor did MDA-MB-231 cells treated with the sEVs show morphological nuclear changes associated with apoptotic events (Picciotto et al. (2021) Biomater. Sci. doi:10.1039/d0bm01696a).

EVs also have been isolated from *Synechocystis sp.* PCC6803 (a cyanobacterium), *Chlamydomonas reinhardtii* (a green microalgae), *Euglena gracilis* (an euglenophyte), and *Haematococcus pluvialis* (a chlorophyte) in work done by Zhao *et al.,* who also performed RNomic and proteomic analyses in EVs isolated from *C. reinhardtii* at different stages of cell growth and under different types of abiotic stress (Zhao *et al.* (2020) doi:10.21203/rs.3.rs-38027/v1). EVs were isolated using differential ultracentrifugation and filtration, and the resuspension was shown to contain membrane

structures with small clumps of particles 110-120 nm in diameter, in line with the reported diameter of exosomes and small MVs, although there were differences in diameters between the species of microalgae. Specifically, EVs from C. *reinhardtii* had diameters between 37-710 nm, with an average particle diameter of 120.1 nm. *Synechocystis*-derived EVs had diameters between 24-450 nm, with an average particle size of 94.68 nm. Despite the presence of a cell wall, *Chlamydomonas* cells were able to uptake EVs, as shown by the presence of EVs labeled with a fluorescent lipophilic dye inside microalgal cells. Thus, microalgal EVs can be absorbed by recipient cells. Non-coding RNAs were detected in microalgal EVs at different growth stages and treatment (biotic stress, nitrogen depletion, and nitrogen recovery), and proteomic analyses identified many flagellar-associated membrane proteins in microalgal EVs (Zhao *et al.* (2020) doi:10.21203/rs.3.rs-38027/v1).

These studies show that microalgae produce EVs that can be isolated using traditional or standard methods; microalgal-derived EVs are similar in size and concentration, and exhibit similar markers compared to EVs isolated from other species; EVs isolated from microalgae do not show cytotoxic or genotoxic effects *in vitro;* and that microalgal-derived EVs can be taken up by cells.

It has been shown that EVs from mammalian origin can deliver cargo to a target cell and thus have therapeutic use for delivery of a variety of cargos for use in treating a number of diseases or conditions; this has not been shown for in general for MEVs. Mammalian EVs, except for bovine milk EVs, however, cannot be administered orally because they do not survive the harsh conditions of the stomach. For example, small molecules such as hydrophobic and hydrophilic drugs can be injected into exosomes, or macromolecular proteins and nucleic acids can be embedded into the exosomes. The nucleic acids can include those encoding a gene of interest. Specific targeting ligands, imaging probes, and covalent linkage could be attached to the exosome surface and tracked using NTA, fluorescence, or by bioluminescence.

Besides a mention in a publication that microalgae EVs possibly can be used to deliver a drug of interest to a targeted cell, tissue, or organ (Kuruvinashetti et al. (2020) 20th International Conference on Nanotechnology 354-357), there is no published evidence nor technical descriptions for use of MEVs for delivery for treatment of mammalian disease, disorders, or conditions. There are no publications or technical descriptions describing how knowledge for application of EV technology to micro algae-derived extracellular vesicles, nor whether it is possible to do so, nor how to do so. Prior studies have not considered *Chlorella* species, nor have the prior studies assessed biodistribution and related properties of the MEVs in general. Hence methods, such as methods of oral delivery, exemplified herein with *Chlorella,* can employ MEVs from other microalgae.

As described and shown herein, however microalgal EVs have a number of advantages over the use of existing drug delivery systems, such as, exosomes derived from mesenchymal stem cells, gold nanoparticles, liposomes and other plant- and animal-derived EVs. Mesenchymal stem cells are a commonly used source of exosomes, and exosomes derived from mesenchymal stem cells are used in drug delivery, for example, anti-cancer vaccines, because they have enhanced passive targeting (a method of preparing a drug carrier system so that it remains circulating in the blood stream). Mesenchymal stem cell derived EVs possess the ability to passively target due to their small size, indigenous nature, and their ability to cross biological barriers. Mesenchymal stem cells, however, have limited secretion of exosomes, and scaling up production of exosomes is difficult due to the need to optimize purification, increase the homogeneity of exosomes, and establish efficient transfection strategies. Nanoparticles can lead to toxicity and current techniques for synthesizing nanoparticles limit their ability to scale for manufacturing purposes. Nanoparticle and liposome-based drug delivery methods also can lead to the formation of a teratoma (a tumor comprised of several different types of tissue). Liposome-based drug delivery methods have been further shown to be less efficient for internalization into a specific cell, tissue or organ, compared to exosomes. Plant-derived EVs, such as those from curcumin, ginger, grapefruit, and lemon, have been used for drug delivery, but their extraction process and use in treatment has not yet been optimized. The production of EVs from agricultural products, such as fruits and milk, is economically impractical and need 3-4 months to grow, compared to algal EVs, which can be grown anywhere and within a few days. Algal EVs avoid phagocytosis or degradation by macrophages and circulate for prolonged times *in vivo,* and have low immunogenicity. Algal EVs also have a lower risk of teratoma formation. Algae, thus, provide a source from which pure, well-characterized EVs of high quality can be obtained (Kuruvinashetti et al. (2020) 20th International Conference on Nanotechnology 354-357). Kuruvinashetti *et al.* does not describe the use of *Chlorella* species as a source of EVs, nor its advantages as a source. Prior art does not describe the biodistribution of MEVs per se, nor the implications thereof for administration of MEVs with drugs directed to particular organs, tissues, or systems.

### 5. Green algae - *Chlorella* species

Previous studies and consideration of EVs have not focused on nor assessed *Chlorella* species as sources of EVs. *Chlorella* and the resulting EVs have advantages for growth, manipulation, and administration of drugs that other species and EVs do not provide. Green algae belong to phylum Chlorophyta, and encompass a diverse group of photosynthetic eukaryotes. Green algae include unicellular and multicellular organisms. Algae originally included in the genus *Chlorella* are among the most widely distributed and frequently encountered algae in freshwater. These algae exist

in aqueous environments and on land. They are typically small (~2 to 10 μm in diameter), unicellular, spherical in shape, non-motile, and contain a single chloroplast, and some have a rigid cell wall (Blanc et al. (2010) Plant Cell 22(9):2943-2955).

Molecular analyses have separated *Chlorella* species into two classes of chlorophytes: the Trebouxiophyceae, which contains the true *Chlorella;* and the Chlorophyceae. For use herein, *Chlorella* species include any that can be or that are used as food complement or that can be consumed by humans or other animals, such as livestock. Exemplary species include, but are not limited to, the species: *Chlorella ellipsoidea, Chlorella pyrenoidosa, Chlorella sorokiniana, Chlorella vulgaris,* and *Chlorella variabilis.*

True *Chlorella* species are characterized by glucosamine as a major component of their rigid cell walls. Although most *Chlorella* species are naturally free-living, the Trebouxiophyceae include most of the known green algal endosymbionts, living in lichens, unicellular eukaryotes, plants, and animals (for example mussels and hydra). For example, *Chlorella variabilis* NC64A is a hereditary photosynthetic endosymbiont (or photobiont) of *Paramecium bursaria,* a unicellular protozoan, and NC64A also is a host for a family of large double-stranded DNA viruses that are occur in freshwater (Blanc et al. (2010) Plant Cell 22(9):2943-2955).

### a. Life Cycle

In unicellular organisms, such as microalgae, life cycle is the same as the cell cycle. *Chlorella* is a haploid organism that reproduces asexually by autosporulation. The cell cycle and proliferation of *Chlorella vulgaris* has been investigated using flow cytometric analysis of 5(6)carboxyfluorescein diacetate N-succinimidyl ester (CFSE)-stained algal cells by Rioboo *et al.* Their results indicate that, as generally described for microalgae, the growth of *C. vulgaris* mother cells takes place during light periods, whereas cytoplasmic division and liberation of daughter cells takes place during dark periods. *C. vulgaris* also shows a distinct light/dark cycle, marked by an increase in cell size, cell complexity, and autofluorescence during periods of light, measured over a 96-hour period. A monoparametric histogram of CFSE-stained *C. vulgaris* cells showing only one peak of daughter cells indicates that each mother cell undergoes only one division cycle in 96 hours; the cytoplasmic division was further shown to take place during periods of darkness. Thus, the strain of *C. vulgaris* used exhibits three life cycle phases: 1) growth of mother cells, 2) cell division, and 3) liberation of daughter cells. *C. vulgaris* cells grew during 2 light periods and began to divide during following dark period; cell division occurs once the mother cells are double the size of daughter cells. Furthermore, *C. vulgaris* cells exposed to the herbicide terbutryn need a longer growth period in order to reach a large enough cell size to divide. This suggests there is a critical threshold size needed for *C. vulgaris* to complete the growth phase and begin the division phase, and that this critical threshold can control the progression of the G1 phase of the *C. vulgaris* cell cycle. Finally, this study demonstrates that the intensity of the peak of CFSE-fluorescence of mother cells is four times greater than that of the daughter cells, indicating that 4 daughter cells are produced from each mother cell. Thus, *C. vulgaris* cells undergo a first mitosis followed by cytoplasmic division, and then two other simultaneous mitoses, which result in the liberation of 4 daughter cells (Rioboo *et al.* (2009) doi:10.1016/j.aquatox.2009.07.009).

### b. Genomic Analyses of *Chlorella* Species

Although species of *Chlorella* are reported to be non-motile and lack a sexual cycle, genomic analyses of *Chlorella variabilis* NC64A (NC64A) and *Chlorella vulgaris* 211/11P (211/11P) reveal the presence of genes involved in sexual reproduction and motility (Blanc et al. (2010) Plant Cell 22(9):2943-2955; Cecchin et al. (2019) Plant J. 100(6):1289-1305). The NC64A nuclear genome (GenBank® Accession No. ADIC00000000.1) is 46.2 Mb, and composed of 12 chromosomes. The meiosis-specific proteins dosage suppressor of MCk1 DMC1, homologous-pairing proteins HOP1 and HOP2, meiotic recombination protein MER3, meiotic nuclear division protein MND1, and mutS homolog protein MSH4 are encoded in NC64A; these genes also occur in most of the other sequenced chlorophyte algal species. Nineteen homologs of the *Chlamydomonas* gametolysin proteins, which promote disassembly of the gametic cell walls and allow gamete fusion, also were identified in NC64A. Additionally, an ortholog of the *Chlamydomonas* GCS1 protein, which is essential for cell fusion, occurs in NC64A (Blanc et al. (2010) Plant Cell 22(9):2943-2955). The primary genes involved in meiosis also occur in the *Chlorella vulgaris* 211/11P 40 Mb genome (GenBank® Accession No. SIDB00000000), in addition to the gene encoding gametolysin (g3347), and a gene encoding a protein that contains a domain with a putative GCS1/HAP2 function (Cecchin et al. (2019) Plant J. 100(6):1289-1305). Thus, although *Chlorella* species have been observed only in the haploid phase, the presence of meiosis genes indicates that the life cycle of *Chlorella* could include a diploid phase.

Similarly, while flagella have not been observed in NC64A, orthologs of the *Chlamydomonas* flagellar proteins were identified in the NC64A genome, including orthologs to the intraflagellar transport (IFT) proteins IFT52, IFT57, and IFT88, kinesin-2 motor protein FLA8, the kinesin-associated protein KAP, and proteins involved in the axonemal outer dynein arm (Blanc et al. (2010) Plant Cell 22(9):2943-2955).

Sequencing of three *Chlorella sorokiniana* strains, strain 1228, UTEX 1230, and DOE1412, reveals the presence of sex- and flagella-related genes (Hovde et al. (2018) Algal Research 35:449-461). The genome of several other *Chlorella* species has been sequenced: *Chlorella protothecoides* sp. 0710 (Gao et al. (2014) BMC Genomics 15(1):582; GenBank® Accession No. APJO00000000); *Chlorella sorokiniana* UTEX 1602 (GenBank® Accession No. LHPG00000000) and *Chlorella* sp. strain SAG 241.80 (*Micractinium conductrix;* GenBank® Accession No. LHPF00000000) (Arriola et al. (2018) Plant J. 93(3):566-586); and the *Chlorella vulgaris* strains UTEX 395 (Guarnieri et al. (2018) Front. Bioeng. Biotechnol. 6:37; GenBank® Accession No. LDKB00000000), UMT-M1 (Teh et al. (2019) Data Brief 27:104680; GenBank® Accession No. VJNP00000000), UTEX 259 (GenBank® Accession No. VATW00000000) and NJ-7 (Wang et al. (2020) Mol. Biol. Evol. 37(3):849-863; GenBank® Accession No. VATV00000000).

### c. Commercial and Biotechnological Uses of *Chlorella*

The commercial cultivation of microalgae for food purposes began with the production of *Chlorella vulgaris* in Japan and Taiwan in the 1960s. Dried biomass products from *Arthrospira* and *Chlorella* are included in dietary supplements due to reports of high protein content, nutritive value, and health benefits. For example, *Chlorella* extracts have been shown to lower cholesterol and have antioxidant, antibacterial, and antitumor activities. Production of high yields of *Chlorella* is routine, and, as detailed herein, MEVs can be isolated from the cell culture medium. For its use as a pharmaceutical, it is known that ingestion of *Chlorella* is non-toxic and non-immunogenic in humans.

*Chlorella* has been used in a variety of biotechnology applications, including biofuels, sequestering $CO_2$, producing molecules of high economic value, or removing heavy metals from wastewaters (Blanc et al. (2010) Plant Cell 22(9):2943-2955*). Chlorella* species show metabolic flexibility in response to environmental perturbations, and are capable of using nutrients, such as organic carbon and minerals, directly from wastewater for growth. Among microalgae, *Chlorella* species have higher photosynthetic efficiency over other photosynthetic organisms. Additionally, *Chlorella vulgaris* is able to grow either in autotrophic, heterotrophic or mixotrophic conditions (Zuñiga et al. (2016) Plant Physiol. 172(1):589-602).

*Chlorella* species also can be genetically modified by *Agrobacterium*-mediated transformation. A study by Cha *et al.* developed a method to genetically transform *Chlorella vulgaris* using the *Agrobacterium tumefaciens* strain LBA4404, and the presence of gene fragments in 30% of the transgenic lines, compared to the wild-type non-infected *Chlorella,* indicates the T-DNA was integrated into the *Chlorella* genome (Cha et al. (2012) World J. Microbiol. Biotechnol. 28:1771-1779).

### d. *Chlorella* MEVs

As described herein, *Chlorella* species, such as C. *vulgaris,* are advantageous species for the production of EVs, referred to herein as MEVs, for use for delivery of biomolecules and small molecules for many applications, including therapeutic, diagnostic, and cosmetic uses. Of particular interest herein are MEVs produced by *Chlorella* species. *Chlorella* EVs have not been exploited as sources of MEVs for exogenous loading of biomolecular products or small molecule drugs or diagnostic agents. *Chlorella,* as a source of EVs for such applications, provides numerous advantages. *Chlorella* is a haploid organism, which means that specific and targeted variants can be produced by genetic engineering; it readily can be genetically modified or loaded to produce or contain biologically active molecules and small molecules. Stable cell lines can be produced, including stable producers of encoded products. They are defined products, and, when exogenously loaded, the resulting compositions contain EVs that contain the same cargo.

Detailed genetic maps can be obtained, and correlations between genotype and phenotype can be established. *Chlorella* genomes have been fully sequenced, so the structure and function of various genes can be known. Phylogenetically, *Chlorella* is at the very crossroads between higher plants and microalgae. As such, *Chlorella* shares with higher plants a significant (and useful) number of molecular biological and metabolic features, but still is a unicellular haploid microalgae. Exemplary of molecular biological features shared with eukaryotes is the intracellular machinery that involves the dicer enzyme system for processing exogenous RNA into siRNA. *Chlorella* is autotrophic; unlike mammalian and other animal cells, it can therefore be cultured and reproduced without the need for nutrients or factors of animal origin.

With respect to use of its EVs as therapeutics, *Chlorella* species are not toxic. For example, tablets made from *Chlorella* vulgaris biomass (*i.e.,* compressed whole *Chlorella* cells) have been consumed regularly for years by the public worldwide as a dietary supplement, without constraints related to toxicity or immunogenicity. Japan is the world leader in the consumption of *Chlorella* biomass. It also is used, for example, in Japan, for medical treatments because it has shown to have immunomodulatory properties and purported anti-cancer activities, for use for anti-aging applications, such as for cardiovascular diseases, hypertension and cataracts; it reduces the risk of atherosclerosis and stimulates the synthesis of collagen for the skin.

*Chlorella* cells naturally produce extracellular vesicles (EVs) that respond to the 'standard specifications' of better known EVs (such as mammalian EVs). EVs from plant origin bear a number of features that make them more

promising/convenient than synthetic nanoparticles or semisynthetic EVs, for use as a drug delivery system in humans. These include, for example, higher stability, lower toxicity, and lower immunogenicity. Being as close as plants as it is, *Chlorella* provides a source of EVs with similar characteristics to plant EVs. At the same time, mass production of *Chlorella* in large scale is easier and cheaper than for higher plants. The glycosylation pattern of membrane proteins in *Chlorella* is similar/identical to the glycosylation pattern present in higher plants.

The size of the *Chlorella* MEVs ranges between about or between 50 nm and 200m, with an average size of about 130 nm. The morphology resembles plant and mammalian exosomes. For use for administration, the size distribution can be rendered more uniform by separating the MEVs by size and selecting those of a size of interest, which can vary depending upon the intended use and route of administration.

## D. EXOGENOUSLY LOADED MICROALGAE EXTRACELLULAR VESICLES (MEVS), CARGO, AND TARGETS

Targets and cargo (see discussions below) include any known to those of skill in the art. Sections F and G and examples below describe the biodistribution of MEVs following administration by various routes, and the implications, uses and methods for targeting or treating particular diseases, disorders, and conditions, and for formulating and administering the MEVs.

### 1. Isolation of MEVs

Methods for isolation are discussed in the sections above and detailed in the Examples.

### 2. MEV Loading and Cargos

The MEVs can be loaded with any desired cargo (also referred to as a payload), including, but not limited to, nucleic acid molecules, including, for example RNAi, plasmids, anti-sense nucleic acids, nucleic acids encoding the RNAi or anti-sense nucleic acid, detectable marker proteins and tags, small molecule drugs, gene editing systems, and others, and combinations thereof. The MEVs can deliver therapeutic molecules, can serve as vaccines, and can be used in human and other animal health, agricultural applications, gene therapy applications, including delivery genes, modification of genes with gene editing systems, and gene silencing nucleic acids, cosmetic applications, dermatological applications, diagnostic applications, industrial uses, and others. The MEVs can deliver nutrients, or regulators of gene pathways to produce a beneficial product, and can be used to deliver gene editing systems, such as CRISPR/Cas and to effect gene editing. The MEVs can be used to deliver gene therapy vectors, such as, but not limited to, adenoassociated (AAV) virus vectors, adenovirus vectors, vaccinia virus-derived vectors, and others, and products.

Diseases and conditions that can be treated include any known to those of skill in the art, including but not limited to, cardiovascular diseases, metabolic diseases, infections, including respiratory infections, bladder infections and other urinary tract infections, infectious diseases, including viral disease, such as hepatitis, HIV, corona viruses, including SARS-Cov-2, CNS diseases, ocular diseases, and liver diseases. As discussed, delivered cargo includes protein products, such as antibodies and antigen-binding forms thereof, RNA products, such as, but not limited to, siRNA, miRNA (micro RNA), lncRNA (long non-coding RNA), saRNA (small activating RNA), shRNA, and mRNA, nucleic acid encoding the products, such as plasmids, nucleic acid products such as DNA encoding anti-sense oligonucleotides and also the anti-sense oligonucleotides, and small molecule drugs.

The MEVs can carry cargos that include reporter genes and proteins and other detectable products, such as, for example, a fluorescent protein, such as, but not limited to an enhanced green fluorescent protein (EGFP; SEQ ID NO:10), a luciferase gene (SEQ ID NO:11), luxA (SEQ ID NO:8), luxB (SEQ ID NO:9), and the Lux operon (luxCDABE and luxABCDE; SEQ ID NO:12).

Other cargos can target genes or products involved in diseases, such as, but not limited to, Peptidyl-prolyl cis-trans isomerase FKBP4 or FKBP52 (SEQ ID NO:1); gamma-aminobutyric acid type B receptor subunit 1 (*GABBR1*; SEQ ID NO:3); oncogenes such as *MYCN* or *NMYC* (SEQ ID NO:38), RAS (*H-RAS, N-RAS,* and *K-RAS;* see SEQ ID NOs:39, 40, and 41, respectively), *BCL2* (SEQ ID NO:43), and *PLK1* (SEQ ID NO:44). Genes involved in diseases, such as oncogenes, and checkpoints, can be modulated by cargo that encodes a product that inhibits or agonizes expression of a gene, or inhibits or agonizes a gene product. Exemplary of such modulators, are RNAi-type modulators, such as for example, siRNAs, miRNAs, shRNAs, anti-sense oligonucleotides (ASOs), peptides and/or tetratricopeptides. For example, siRNAs and ASOs targeting EGFP (SEQ ID NOs:5 and 6), firefly luciferase (SEQ ID NO:7), *MYCN* (SEQ ID NOs:13-19), *RAS* (SEQ ID NOs:20-27), *BCL2* (SEQ ID NOs:29-31), and *PLK1* (SEQ ID NOs:32-35), and microRNA-34A, which targets *MYC* and *BCL2* (SEQ ID NO:28), are exemplified herein.

Gene silencing using RNA interference, including siRNAs and microRNAs, can be used to silence developmental genes, such as, for example, adhesion molecules, cyclin kinase inhibitors, Wnt family members, Pax family members, Winged helix family members, Hox family members, cytokines/lymphokines and their receptors, growth/differentiation factors and their receptors, and neurotransmitters and their receptors; oncogenes; tumor suppressor genes; enzymes; genes associated with a pathological condition; genes associated with autoimmune diseases; anti-angiogenic genes; angiogenic genes; immunomodulator genes; genes associated with alcohol metabolism and liver function; genes associated with neurological disease; genes associated with tumorigenesis or cell transformation; and genes associated with metabolic diseases and disorders (see, *e.g.,* WO 2009/082606, JP 2014-240428A, WO 2011/072292A2, WO 2010/141724, and WO 2020/097540). These types of products can be delivered in or encoded in MEVs to activate genes or pathways or to provide therapeutic effects. Certain cytokines can be used to treat diseases/disorders, such as certain cancers, in which immune suppression plays a role.

Extracellular vesicles and exosomes also can be used to transfer therapeutic agents such as nucleic acids, such as microRNA, mRNA, tRNA, rRNA, siRNA, regulatory RNA, non-coding and encoding RNA, DNA fragments, and DNA plasmids (see, *e.g.,* CN105821081A and CN110699382A); nucleotides or amino acids comprising a detectable moiety or a toxin or that disrupts transcription or translation, respectively; polypeptides (*e.g.*, enzymes); lipids; carbohydrates; and small molecules (*e.g.,* small molecule drugs and toxins) (see, U.S. Patent No. 10,195,290). Nonlimiting examples of proteins that may be encoded for by the nucleic acid cargo molecule include, but are not limited to: antibodies, intrabodies, single chain variable fragments, affibodies, enzymes, transporters, tumor suppressors, viral or bacterial inhibitors, cell component proteins, DNA and/or RNA binding proteins, DNA repair inhibitors, nucleases, proteinases, integrases, transcription factors, growth factors, apoptosis inhibitors and inducers, toxins, structural proteins, neurotrophic factors, membrane transporters, nucleotide binding proteins, heat shock proteins, CRISPR-associated proteins, cytokines, cytokine receptors, caspases and any combination and/or derivatives thereof (see, *e.g.*, AU2018365299).

For example, as summarized in the table below, a cocktail of three siRNA oligonucleotides targeting human MYCN with two thymidine residues (dTdT) at the 3'-end of the sequence (purchased from B-Bridge International Inc. (Sunnyvale, CA)) can be used. The anti-MYCN siRNA (siMYCN) and negative control siRNA (nontarget control pool) (siNeg) (both ON-TARGETplus siRNA, Dharmacon, Cambridge, UK) were used (see Ref 1). Exemplary target oncogenes and exemplary sequences of siRNA (see, also, SEQ ID NOs:13-35) are provided in the table below.

| Target oncogene | Type of sequence | Sequence(s) |
|---|---|---|
| [1]MYCN | siRNA cocktail | siMYCN-1: sense 5'-CGGAGATGCTGCTTGAGAA-3'<br>siMYCN-2: sense 5'-CGGAGTTGGTAAAGAATGA-3'<br>siMYCN-3: sense 5'-CAGCAGTTGCTAAAGAAAA-3' |
| [2]MYCN | siRNA | sense 5'-CAGCAGUUGCUAAAGAAAAUU-3'<br>antisense 5'-UUUUCUUUAGCAACUGCUGUU-3' |
| [3]MYCN | siRNA | 5'-UGAUCUGCAAGAACCCAGA-3' |
| [4]MYCN | ASO | 5'-AAC GTT GAG GGG CAT-3' |
| [5]RAS | synthetic miRNA | **Syn-miR-143s**<br>**No.** \| **Type** \| **Sequence**<br>#1 \| Wild \| S: 3'-GGUCUCUACGUCGUGACGUGGAGU-5' AS: 5'-UGAGAUGAAGCACUGUAGCUCAGG-3'<br>#12 \| F/Ome \| S: 3'-GGUCUCUACGUCGUGACGUGGAGU-5' AS: 5'-U^**G**^A**G**A**U**G**A**A**G**C**A**C**U**G**U**A**G**C**U**C**A**^Dt^Dt-3'<br>N:2'-F RNA<br>**N: 2'-Ome** RNA<br>N:RNA<br>^: PS<br>_: mismatch |
| [6]RAS | siRNA | siR-KRAS: 5'-AAUGCAUGA CAACACUGGAUGACCG-3'<br>siR-HRAS: 5'-CCAUCCAGCUGAUCCAGAACCAUUU-3' |
| [7]RAS | siRNA | GGACUCUGAAGAUGUACCUAGGUACAUCUUCAGAGUCC |
| [8]mutated K-RAS | ASO | 5'-CTACGCCACAAGCTCCA-3' |
| [9]MYC, BCL2, | miRNA | Homo sapiens microRNA 34a (MIR34A), microRNA |
| [10]BLC2 | 3p-siRNA | sense GCAUGCGGCCUCUGUUUGA<br>anti-sense CGUACGCCGGAGACAAACU |
| [11]BLC2 | ASO | TCT CCC AGC GTG CGC CAT |
| [12]PLK1 | modified siRNA | sense: 5'-AGA **U**CA CCC **U**CC U**U**A AA**U** AUU-3'<br>antisense: 5'-UAU UUA A**G**G AGG GUG A**U**C UUU-3'<br>*note: 2'-O-methylated modified nucleotides* ***in bold*** |
| [13]PLK1 | siRNA | 5'-AAGGGCGGCUUUGCCAAGUGC-3' |
| [14]PLK1 | ASO | P12: ACC AGT CCG GAG GGG AGG GC |

1 Nara *et al.* (2007) *Int. J. Oncol.* 30(5):1189-1196; Silencing of MYCN by RNA interference induces growth inhibition, apoptotic activity and cell differentiation in a neuroblastoma cell line with MYCN amplification

2 Maeshima *et al.* (2020) *Nucleic Acid Ther.* 30(4):237-248; MYCN Silencing by RNAi Induces Neurogenesis and Suppresses Proliferation in Models of Neuroblastoma with Resistance to Retinoic Acid

3 Veas-Perez de Tudela *et al.* (2010) *J. Neurochem.* 113(4):819-825; Human neuroblastoma cells with MYCN amplification are selectively resistant to oxidative stress by transcriptionally up-regulating glutamate cysteine ligase

4 Watson *et al.* (1991) *Cancer Res.* 51(15):3996-4000; Inhibition of c-myc expression by phosphorothioate antisense oligonucleotide identifies a critical role for c-myc in the growth of human breast cancer

5 Yoshikawa *et al.* (2019) *Mol. Ther. Methods Clin. Dev.* 13:290-302; Anti-cancer Effects of a Chemically Modified miR-143 on Bladder Cancer by Either Systemic or Intravesical Treatment

6 Tsujino *et al.* (2019) *Cancer Sci.* 110(7):2189-2199; MicroRNA-143/Musashi-2/KRAS cascade contributes positively to carcinogenesis in human bladder cancer

7 Tirella *et al.* (2019) *Int. J. Pharm.* 561:114-123; CD44 targeted delivery of siRNA by using HA-decorated nanotechnologies for KRAS silencing in cancer treatment

8 Nakada *et al.* (2001) *Pancreatology* 1(4):314-319; Antisense oligonucleotides specific to mutated K-ras genes inhibit invasiveness of human pancreatic cancer cell lines

9 Adams *et al.* (2015) *Expert Opin. Ther. Targets* 20(6):737-753; The Tumor-Suppressive and Potential Therapeutic Functions of miR-34a in Epithelial Carcinomas

10 Poeck *et al.* (2008) *Nat. Med.* 14(11):1256-1263; 5'-Triphosphate-siRNA: turning gene silencing and Rig-I activation against melanoma

11 Szegedi *et al.* (2008) *Pathol. Oncol. Res.* 14(3):275-279; Bcl-2 Antisense Oligonucleotide Inhibits the Proliferation of Childhood Leukemia/lymphoma Cells of the B-cell Lineage

12 Ripoll *et al.* (2018) *RSC Adv.* 8:20758-20763; Co-delivery of anti-PLK-1 siRNA and camptothecin by nanometric polydiacetylenic micelles results in a synergistic cell killing

13 Liu *et al.* (2012) *BMC Cancer* 12(1):519-529; MicroRNA-100 is a potential molecular marker of non-small cell lung cancer and functions as a tumor suppressor by targeting polo-like kinase 1

14 Spänkuch *et al.* (2008) *Neoplasia* 10(3):223-234; Downregulation of Plk1 expression by receptor-mediated uptake of antisense oligonucleotide-loaded nanoparticles

Cargo bioactive molecules can target central nervous system diseases, such as neurodegenerative diseases, such as Alzheimer's disease. Exemplary of such is FKBP52 and the teratricopeptide derivative therefrom. The full sequence of human peptidyl-prolyl cis-trans isomerase FKBP4 is (SEQ ID NO:1):

MTAEEMKATESGAQSAPLPMEGVDISPKQDEGVLKVIKREGTGTEMPMIGDRVFVHYTGW
LLDGTKFDSSLDRKDKFSFDLGKGEVIKAWDIAIATMKVGEVCHITCKPEYAYGSAGSPP
KIPPNATLVFEVELFEFKGEDLTEEEDGGIIRRIQTRGEGYAKPNEGAIVEVALEGYYKD
KLFDQRELRFEIGEGENLDLPYGLERAIQRMEKGEHSIVYLKPSYAFGSVGKEKFQIPPN
AELKYELHLKSFEKAKESWEMNSEEKLEQSTIVKERGTVYFKEGKYKQALLQYKKIVSWL
EYESSFSNEEAQKAQALRLASHLNLAMCHLKLQAFSAAIESCNKALELDSNNEKGLFRRG
EAHLAVNDFELARADFQKVLQLYPNNKAAKTQLAVCQQRIRRQLAREKKLYANMFERLAE
EENKAKAEASSGDHPTDTEMKEEQKSNTAGSQSQVETEA

The tetratricopeptide repeat (TPR) domain 260-400 is (SEQ ID NO:2):

MNSEEKLEQSTIVKERGTVYFKEGKYKQALLQYKKIVSWLEYESSFSNEEAQKAQALRLA
SHLNLAMCHLKLQAFSAAIESCNKALELDSNNEKGLFRRGEAHLAVNDFELARADFQKVL
QLYPNNKAAKTQLAVCQQRI

A relatively simple *in vitro* model was developed and described by a group from Institut National de la Santé et de la Recherche Médicale, Université Paris XI, (see, Chambraud et al. (2007) FASEB J. 21(11):2787-97; and Chambraud et al. (2010) Proc. Natl. Acad. Sci. U.S.A. 107(6):2658-63). The effect of depletion of FKBP52 in PC12 cultured cells was examined by introducing two different small, interfering RNA (siRNA) duplexes specific for rat FKBP52, named RNAi 1 and RNAi 2. The sense sequence of siRNAs and an oligonucleotide duplex with a scrambled sequence corresponding to RNAi 1 were used as negative control. In these experiments, the level of FKBP52 analyzed by Western blot was substantially reduced after 48 h and remained low 72 h post-transfection. Tubulin and FKBP52 stainings were performed 72 h post transfections. In cells transfected with RNAi 1 or 2, FKBP52 staining was significantly lower than that observed in control cells, and tubulin staining revealed a change in the PC12 cell phenotype-in particular, the loss of FKBP52 in PC12 cells results in these cells forming extensions. Therefore, these cells acquired a differentiated phenotype that could be compared with PC12 cells treated with NGF. No significant modification could be observed in cells transfected with

controls. In another study, Chambraud et al. (Proc. Natl. Acad. Sci. U.S.A., cited above) reports that FKBP52 prevents Tau accumulation and neurite outgrowth in PC12 Cells. The FKBP52-inducible expression system based on a tetracycline-responsive element was used. The system allows the generation of a stably transformed PC12 cell line to determine a cellular role for FKBP52. Among clones that were positively tested, one clone, so-called H7C2, was selected and used to study the effects of FKBP52 overexpression on PC12 cells and to further investigate the relationship between FKBP52 and Tau. Under basal conditions, H7C2 cells expressed endogenous FKBP52, and treatment with doxycycline (Dox) resulted in a marked increase of recombinant FKBP52 protein expression. FKBP52 induction in H7C2 cells was about four-fold after 5 days of Dox treatment. Next, the effect of FKBP52 on the accumulation of Tau was examined. The amount of Tau protein was determined by Western blotting of extracts from cultures of either PC12 cells or H7C2 cells, treated or not with nerve growth factor (NGF) (50 nM) for 5 days with or without Dox. In PC12 cells, FKBP52 expression was unchanged after treatment with NGF. As expected, in both PC12 and H7C2 cells, an increase in Tau was observed after NGF treatment. When H7C2 cells were exposed to Dox in addition to NGF, so that they overexpress FKBP52, no additional accumulation of Tau protein occurred. An increase in Tau protein was still observed in PC12 cells treated with NGF and Dox, ruling out the possibility that Dox was responsible for the lack of decrease in Tau. The report concludes that FKBP52 prevents the accumulation of Tau induced by NGF in PC12 cells.

Because one role of Tau is to stimulate neurite outgrowth, the consequence of FKBP52 overexpression on neurite length in PC12 and H7C2 cells also was investigated. In the absence of NGF, no neurite outgrowth was observed in H7C2 cells, whether or not they were treated with Dox for a week. In H7C2 cells treated with 50 nM NGF and Dox, a 40% ($\pm$7) decrease in neurite length, compared to control (H7C2 not treated with Dox) was observed. The same effect of Dox on neurite length was observed in H7C2 cells treated with 10 or 20 nM NGF. Dox by itself was not involved in the process of neurite outgrowth because there was no difference in neurite length between Dox-treated and untreated PC12 cells observed. The inhibition of neurite outgrowth resulting from FKBP52 overexpression is in agreement with the previous report from Chambraud *et al.* showing that the loss of FKBP52 in PC12 cells results in the formation of neurite extensions. The FKBP52 effect on neurite length could be explained by the binding of Tau to FKBP52, removing Tau from microtubules. The prevention of Tau accumulation by overexpression of FKBP52 is consistent with the decrease of neurite length and suggests a potential role of this immunophilin in Tau function. Hence, the above target and sequences can be delivered or encoded in MEVs for treatment of Alzheimer's disease by preventing accumulation of Tau.

**Reporter genes, reporter proteins, and/or modulators thereof can be delivered in the MEVs.**

**Reporter proteins**

Target sequences, in the form of siRNAs, miRNAs, anti-sense oligonucleotides (ASOs), peptides and/or tetratricopeptides, to modulate (inhibition or stimulation) of each of the marker genes, such as a GFP protein, a eukaryotic luciferase, or a prokaryotic Luciferase, such as: Lux operon (luxCDABE) and lux operon (luxABCDE), can be used, for example for diagnostics and gene expression assessments (SEQ ID NOs:5-6, 7, and 62-65, respectively):

| Target gene | Type of sequence | Sequence(s) |
|---|---|---|
| **EGFP** | siRNA | sense: 5'-GCAAGCUGACCCUGAAGUUCAUUU-3'<br>antisense: 5'-AUGAACUUCAGGGUCAGCUUGCCG-3' |
| **firefly luciferase** | shRNA | 5'-CTGACGCGGAATACTTCGA-3' |
| **luxA (Lux operon)** | siRNA | sense: 5'-CAAACAGAGGUAAUGAAAUGGUUG-3'<br>antisense: 3'-CAACCAUUUCAUUACCUCUGUUUG-5' |
| **luxB (Lux operon)** | siRNA | sense: 5' -AUGUUAAGUUGAAUAAGUUCUGCA-3'<br>antisense: 3'-UGCUCUUGAAUAAGUUGAAUUGAU-5' |

Other exemplary cargo can include chemotherapeutic agents, which include but are not limited to alkylating agents such as thiotepa and cyclophosphamide (available under the trademark CYTOXAN®); alkyl sulfonates such as busulfan, improsulfan and piposulfan; androgens such as calusterone, dromostanolone propionate, epitiostanol, mepitiostane, and testolactone; anti-adrenals such as aminoglutethimide, mitotane, and trilostane; anti-androgens such as flutamide, nilutamide, bicalutamide, leuprolide, and goserelin; antibiotics such as aclacinomycin, actinomycin, anthramycin, azaserine, bleomycin, cactinomycin, calicheamicin, carubicin, carminomycin, carzinophilin, chromomycin, dactinomycin, daunorubicin, detorubicin, 6-diazo-5-oxo-L-norleucine, doxorubicin, epirubicin, esorubicin, idarubicin, marcellomycin, mitomycin, mycophenolic acid, nogalamycin, olivomycin, peplomycin, porfiromycin, puromycin, quelamycin, rodorubicin, streptonigrin, streptozocin, tubercidin, ubenimex, zinostatin, and zorubicin; anti-estrogens including for

example tamoxifen, raloxifene, aromatase inhibiting 4(5)-imidazoles, 4-hydroxytamoxifen, trioxifene, keoxifene, LY 117018, onapristone, and toremifene (sold under the trademark Fareston®); anti-metabolites such as methotrexate and 5-fluorouracil (5-FU); folic acid analogs such as denopterin, methotrexate, pteropterin, and trimetrexate; aziridines such as benzodepa, carboquone, meturedepa, and uredepa; ethylenimines and methylmelamines including altretamine, triethylenemelamine, triethylenephosphoramide, triethylenethiophosphoramide and trimethylol melamine; folic acid replenisher such as folinic acid; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, and uracil mustard; nitrosoureas such as carmustine, chlorozotocin, fotemustine, lomustine, nimustine, and ranimustine; platinum analogs such as cisplatin and carboplatin; vinblastine; platinum; proteins such as arginine deiminase and asparaginase; purine analogs such as fludarabine, 6-mercaptopurine, thiamiprine, and thioguanine; pyrimidine analogs such as ancitabine, azacitidine, 6-azauridine, carmofur, cytarabine, dideoxyuridine, doxifluridine, enocitabine, floxuridine, 5-FU; taxanes, e.g., paclitaxel (such as paclitaxel sold under the trademark TAXOL®, Bristol-Myers Squibb Oncology, Princeton, N.J.) and docetaxel (TAXOTERE®, Rhone-Poulenc Rorer, Antony, France); topoisomerase inhibitor RFS 2000; thymidylate synthase inhibitor (such as Tomudex); additional chemotherapeutics including aceglatone; aldophosphamide glycoside; aminolevulinic acid; amsacrine; bestrabucil; bisantrene; edatrexate; defosfamide; demecolcine; diaziquone; difluoromethylomithine (DFMO); eflomithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2,2', 2"-trichlorotriethylamine; urethan; vindesine; dacarbazine; mannomustine; mitobronitol; mitolactol; pipobroman; gacytosine; arabinoside (Ara-C); cyclophosphamide; thiotepa; chlorambucil; gemcitabine; 6-thioguanine; mercaptopurine; methotrexate; etoposide (VP-16); ifosfamide; mitomycin C; mitoxantrone; vincristine; vinorelbine (such as vinorelbine tartrate sold under the trademark Navelbine®); Novantrone; teniposide; daunomycin; aminopterin; capecitabine (sold, for example, as Xeloda®); ibandronate; CPT-11; retinoic acid; esperamycins; capecitabine; and topoisomerase inhibitors such as irinotecan. Pharmaceutically acceptable salts, acids or derivatives of any of the above also can be used.

Chemotherapeutic agents include prodrugs, which include, but are not limited to, phosphate-containing prodrugs, thiophosphate-containing prodrugs, sulfate-containing prodrugs, peptide-containing prodrugs, D-amino acid-modified prodrugs, glycosylated prodrugs, beta-lactam-containing prodrugs, optionally substituted phenoxy acetamide-containing prodrugs or optionally substituted phenylacetamide-containing prodrugs, 5-fluorocytosine and other 5-fluorouridine prodrugs which can be converted into the more active cytotoxic free drug.

Other cargo includes, for example, anti-angiogenic agents. Anti-angiogenic agents can be a small molecule or protein, such as an antibody, Fc fusion, and cytokine, that binds to a growth factor or growth factor receptor involved in promoting angiogenesis. Examples of anti-angiogenic agents include but are not limited to antibodies that bind to Vascular Endothelial Growth Factor (VEGF) or that bind to VEGF-R, RNA-based therapeutics that reduce levels of VEGF or VEGF-R expression, VEGF-toxin fusions, Regeneron's VEGF-trap, angiostatin (plasminogen fragment), antithrombin III, angiozyme, ABT-627, Bay 12-9566, BeneFin, bevacizumab, bisphosphonates, BMS-275291, cartilage-derived inhibitor (CDI), CAI, CD59 complement fragment, CEP-7055, Col 3, Combretastatin A-4, endostatin (collagen XVIII fragment), farnesyl transferase inhibitors, fibronectin fragment, GRO-beta, halofuginone, heparinases, heparin hexasaccharide fragment, HMV833, human chorionic gonadotropin (hCG), IM-862, interferon alpha, interferon beta, interferon gamma, interferon inducible protein 10 (IP-10), interleukin-12, kringle 5 (plasminogen fragment), marimastat, metalloproteinase inhibitors (e.g., TIMPs), 2-methoxyestradiol, MMI 270 (CGS 27023A), plasminogen activator inhibitor (PAI), platelet factor-4 (PF4), prinomastat, prolactin 16 kDa fragment, proliferin-related protein (PRP), PTK 787/ZK 222594, retinoids, solimastat, squalamine, SS3304, SU5416, SU6668, SU11248, tetrahydrocortisol-S, tetrathiomolybdate, thalidomide, thrombospondin-1 (TSP-1), TNP470, transforming growth factor beta (TGF-$\beta$), vasculostatin, vasostatin (calreticulin fragment), ZS6126, and ZD6474.

Other cargo includes tyrosine kinase inhibitors, which include, but are not limited to quinazolines, such as PD 153035, 4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo(2,3-d) pyrimidines; curcumin (diferuloylmethane, 4,5-bis (4-fluoroanilino) phthalimide); tyrphostins containing nitrothiophene moieties; PD-0183805 (Warner-Lambert); antisense molecules (e.g., those that bind to ErbB-encoding nucleic acid); quinoxalines (U.S. Pat. No. 5,804,396); tyrphostins (U.S. Pat. No. 5,804,396); PTK-787 (Novartis/Schering A G); pan-ErbB inhibitors such as CI-1033 (Pfizer); Affinitac (ISIS 3521; Isis/Lilly); Imatinib mesylate (STI571, Gleevec®; Novartis); PKI 166 (Novartis); GW2016 (Glaxo SmithKline); CI-1033 (Pfizer); EKB-569 (Wyeth); Semaxinib (Sugen); ZD6474 (AstraZeneca); IMC-1C11 (ImClone); or as described in any of the following patent publications: U.S. Pat. No. 5,804,396; PCT WO 99/09016 (American Cyanamid); PCT WO 98/43960 (American Cyanamid); PCT WO 97/38983 (Warner-Lambert); PCT WO 99/06378 (Warner-Lambert); PCT WO 99/06396 (Warner-Lambert); PCT WO 96/30347 (Pfizer, Inc.); PCT WO 96/33978 (AstraZeneca); PCT WO 96/33979 (AstraZeneca); PCT WO 96/33980 (AstraZeneca), gefitinib (Iressa®, ZD1839, AstraZeneca), and OSI-774 (Tarceva®, OSI Pharmaceuticals/Genentech).

Other cargo includes immunomodulatory agents that increase or decrease production of one or more cytokines, up- or down-regulate self-antigen presentation, mask MHC antigens, or promote the proliferation, differentiation, migration, or activation state of one or more types of immune cells. Examples of immunomodulatory agents include but are not limited to non-steroidal antiinflammatory drugs (NSAIDs) such as aspirin, ibuprofen, celecoxib, diclofenac, etodolac, fenoprofen, indomethacin, ketorolac, oxaprozin, nabumetone, sulindac, tolmetin, rofecoxib, naproxen, ketoprofen, and nabumetone; steroids (e.g., glucocorticoids, dexamethasone, cortisone, hydroxycortisone, methylprednisolone, prednisone, prednisolone, triamcinolone, azulfidine eicosanoids such as prostaglandins, thromboxanes, and leukotrienes; as well as topical steroids such as anthralin, calcipotriene, clobetasol, and tazarotene); cytokines such as TGFβ, IFNα, IFNβ, IFNγ, IL-2, IL-4, IL-10; cytokine, chemokine, or receptor antagonists including antibodies, soluble receptors, and receptor-Fc fusions, B7, CCR2, CCR5, CD2, CD3, CD4, CD6, CD7, CD8, CD11, CD14, CD15, CD17, CD18, CD20, CD23, CD28, CD40, CD40L, CD44, CD45, CD52, CD64, CD80, CD86, CD147, CD152, complement factors (C5, D), CTLA4, eotaxin, Fas, ICAM, IFNα, IFNβ, IFNγ, IFNAR, IgE, IL-1, IL-2, IL-2R, IL-4, IL-5R, IL-6, IL-8, IL-9 IL-12, IL-13, IL-13R1, IL-15, IL-18R, IL-23, integrins, LFA-1, LFA-3, MHC, selectins, TGFβ, TNFα, TNFβ, TNF-R1, T-cell receptor, including Enbrel® (etanercept), Humira® (adalimumab), and Remicade® (infliximab); heterologous anti-lymphocyte globulin; other immunomodulatory molecules such as 2-amino-6-aryl-5 substituted pyrimidines, anti-idiotypic antibodies for MHC binding peptides and MHC fragments, azathioprine, brequinar, Bromocriptine, cyclophosphamide, cyclosporine A, D-penicillamine, deoxyspergualin, FK506, glutaraldehyde, gold, hydroxychloroquine, leflunomide, malononitriloamides (e.g., leflunomide), methotrexate, minocycline, mizoribine, mycophenolate mofetil, rapamycin, and sulfasalazine.

Other cargo includes cytokines which include, but are not limited to lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and -beta; Müllerian-inhibiting substance; mouse gonadotropin-associated peptide; inhibin; activin; vascular endothelial growth factor; integrin; thrombopoietin (TPO); nerve growth factors such as NGF-beta; platelet-growth factor; transforming growth factors (TGFs) such as TGF-alpha and TGF-beta; insulin-like growth factor-I and-II; erythropoietin (EPO); osteoinductive factors; interferons such as interferon-alpha, -beta, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-15, a tumor necrosis factor such as TNF-alpha or TNF-beta; and other polypeptide factors including LIF and kit ligand (KL).

Other exemplary cargo includes cytokines and other agents that stimulate cells of the immune system and enhance desired effector function. For example, agents that stimulate NK cells include IL-2; agents that stimulate macrophages include but are not limited to C5a, formyl peptides such as N-formyl-methionyl-leucyl-phenylalanine. Cargo include agents that stimulate neutrophils, such as, for example, G-CSF and GM-CSF. Additional agents include, but are not limited to, interferon gamma, IL-3 and IL-7.

Cargo includes antibiotics, for treatment of infections, particularly for hard-to-treat bacterial infections, including urinary tract infection, respiratory infections, particularly *Pseudomonas aeruginosa* or *Staphylococcus aureus* infections in subjects with cystic fibrosis, and sinus infections, which can be treated by local administration, such as by inhalation of aerosols containing the MEVs. The antibiotic treatments for pulmonary infections in subjects with cystic fibrosis can be combined with gene therapy using the same or different MEVs that comprise nucleic acid, DNA or RNA, encoding the cystic fibrosis transmembrane conductance regulator (CFTR) protein or providing a gene editing system to correct the defect in CFTR protein.

Antibiotics that can be loaded as cargo in the MEVs include but are not limited to: aminoglycoside antibiotics (*e.g.,* apramycin, arbekacin, bambermycins, butirosin, dibekacin, gentamicin, kanamycin, neomycin, netilmicin, paromomycin, ribostamycin, sisomicin, and spectinomycin), aminocyclitols (*e.g.,* spectinomycin), amphenicol antibiotics (*e.g.,* azidamfenicol, chloramphenicol, florfenicol, and thiamphenicol), ansamycin antibiotics (*e.g.,* rifamide and rifampin), carbapenems (*e.g.,* imipenem, meropenem, and panipenem); cephalosporins (*e.g.,* cefaclor, cefadroxil, cefamandole, cefatrizine, cefazedone, cefozopran, cefpimizole, cefpiramide, cefpirome, cefprozil, cefuroxime, cefixime, cephalexin, and cephradine), cephamycins (cefbuperazone, cefoxitin, cefminox, cefmetazole, and cefotetan); lincosamides *(e.g.,* clindamycin and lincomycin); macrolide *(e.g.,* azithromycin, brefeldin A, clarithromycin, erythromycin, roxithromycin, and tobramycin), monobactams (e.g., aztreonam, carumonam, and tigemonam); mupirocin; Oxacephems *(e.g.,* flomoxef, latamoxef, and moxalactam); penicillins (*e.g.,* amdinocillin, amdinocillin pivoxil, amoxicillin, bacampicillin, benzylpenicillinic acid, benzylpenicillin sodium, epicillin, fenbenicillin, floxacillin, penamecillin, penethamate hydriodide, penicillin o-benethamine, penicillin O, penicillin V, penicillin V benzoate, penicillin V hydrabamine, penimepicycline, and phenethicillin potassium); polypeptides (e.g., bacitracin, colistin, polymyxin B, teicoplanin, and vancomycin); quinolones (amifloxacin, cinoxacin, ciprofloxacin, enoxacin, enrofloxacin, fleroxacin, flumequine, gatifloxacin, gemifloxacin, grepafloxacin, lomefloxacin, moxifloxacin, nalidixic acid, norfloxacin, ofloxacin, oxolinic acid, pefloxacin, pipemidic acid, rosoxacin, rufloxacin,

sparfloxacin, temafloxacin, tosufloxacin, and trovafloxacin); rifampin; streptogramins (e.g., quinupristin, and dalfopristin); sulfonamides (sulfanilamide, and sulfamethoxazole); and tetracyclines (chlortetracycline, demeclocycline hydrochloride, demethylchlortetracycline, doxycycline, Duramycin®, minocycline, neomycin, oxytetracycline, streptomycin, tetracycline, and vancomycin).

Cargo also includes anti-fungal agents, which include, but are not limited to, amphotericin B, ciclopirox, clotrimazole, econazole, fluconazole, flucytosine, itraconazole, ketoconazole, miconazole, nystatin, terbinafine, terconazole, and tioconazole. In some examples, cargo-loaded MEVs described herein are administered with one or more antiviral agents, including but not limited to protease inhibitors, reverse transcriptase inhibitors, and others, including type I interferons, viral fusion inhibitors, neuraminidase inhibitors, acyclovir, adefovir, amantadine, amprenavir, clevudine, enfuvirtide, entecavir, foscarnet, ganciclovir, idoxuridine, indinavir, lopinavir, pleconaril, ribavirin, rimantadine, ritonavir, saquinavir, trifluridine, vidarabine, and zidovudine.

In all instances, the form of the cargo includes proteins, and also, nucleic acid encoding the proteins, such as the plasmids, and also, mRNA. The nucleic acids can be operably linked to regulatory elements that are recognized in the particular subject, such as a mammal, in which they are to be delivered.

| Target organ/tissue | Exemplary indication | Exemplary route of administration |
|---|---|---|
| Lung | Cystic Fibrosis | Inhalation |
| Lung | Idiopathic Pulmonary Fibrosis | Inhalation |
| Lung | Primary Ciliary Dyskinesia | Inhalation |
| Lung | Pulmonary Arterial Hypertension | Inhalation |
| Liver | Inborn error of metabolism | Intravenous or direct injection into the liver |
| Lung | Covid-19 (preventive or therapeutic) | Intranasal or Inhalation |
| Lymphatic | Covid-19 (vaccine) | Intravenous or intramuscular |
| Lung | Influenza (preventive or therapeutic) | Intranasal or Inhalation |
| Lymphatic | Influenza (vaccine) | Intravenous or intramuscular |
| Lymphatic | Viral pathogen | Intravenous or intramuscular |
| Lymphatic | Bacterial pathogen | Intravenous or intramuscular |

### 3. Generation of Payload-Loaded MEVs

As shown herein, the isolated *Chlorella* can be loaded with cargo for delivery to humans by any suitable route, including but not limited to intravenous, oral, topical, mucosal, inhalation, and any other routes known to those of skill in the art for delivery of vehicles, such as lipid nanoparticles, vectors, therapeutic bacteria, and therapeutic viruses. Upon administration, the MEVs are taken up by cells. Any cargo presently delivered in vectors, bacteria, exosomes, nanoparticles, and other such delivery vehicles can be loaded into the MEVs provided herein. The loaded cargo can be selected so that it only is expressed or produced in targeted cells, such as in instances in which the cargo is a plasmid encoding a therapeutic product. Transcription regulatory signals can be selected so that the encoded product is expressed in targeted cells. For example, for expression in the liver, the encoded product can be expressed under control of a liver-specific promoter, or the product can be targeted to a receptor or target expressed in targeted cells, such as in tumors or in the tumor microenvironment. Loading methods, described above, and in the Examples below, include, but are not limited to:

a. Electroporation
b. Sonication
c. Extrusion
d. Surfactants
e. Other Methods known to those of skill in the art for introducing exosomes into cells.

### 4. Exemplary Cargo and Exemplary Uses of the Exogenously Loaded MEVs

#### a. Cargo

As described above, the MEVs are loaded with cargo that can be used for any purpose of interest, including any

for which other delivery vehicles are used. These uses include delivery of mRNA, such as mRNA encoding corona virus spike proteins and modified spike proteins to improve the immune response to the viruses, RNAi, such as siRNA, and anti-sense RNA, or anti-sense DNA (ASO), to silence genes, such as bacterial and viral pathogen virulence genes, antibiotic resistance genes, antimicrobial resistance genes, genes that suppress the immune system, tumor genes, such as oncogenes, and host factors for viral infection, such as targeting angiotensin-converting enzyme-2 (ACE2), transmembrane protein serine 2 (TMPRSS2), and other such genes. The cargo also can include any therapeutic antibodies. Therapeutic antibodies, include, but are not limited to, anti-cancer antibodies, antibodies to treat autoimmune or inflammatory disease, antibodies to treat transplant rejection, antibodies to treat graft-versus-host-disease (GVHD), and antibodies to treat infectious diseases.

**1) RNA Cargo**

The mechanism of RNA interference or RNAi was originally described as a process of sequence-specific silencing of gene expression in the nematode *Caenorhabditis elegans* (Fire et al. (1998) Nature 391(6669):806-11; Fire and Mello, 2006 Nobel Prize in Medicine awarded to Andrew Fire and Craig Mello). The process of small RNAs targeting (and silencing) messenger RNAs involves a particular RNAi machinery (including silencing factors, such as DICER and ARGONAUTE).

In the plant kingdom, RNAi is involved in antiviral defense mechanisms, and in defense mechanisms against phytopathogenic fungi and oomycetes. Small regulatory RNAs can be active in silencing genes inside bacterial cells, which lack the said RNAi machinery. The silencing activity of siRNA has been demonstrated to be inter-kingdom (see, *e.g.*, PCT/EP2019/ 072169, published as International PCT Publication No,: WO2020/035619; PCT/ EP2019/072170, published as WO2020/035620; Singla et al. (2019c) bioRxiv, doi: doi.org/10.1101/863902).

RNAi-mediated regulation of gene expression has been exploited for several years in the field of biotechnology to confer resistance to viruses (Baulcombe (2015) Current Opinion in Plant Biology 26:141-146). The inter-kingdom RNAi has been used to characterize the function of genes of eukaryotic pathogens / parasites as well as to induce protection against these organisms.

In *Drosophila* and *Caenorhabditis,* RNAi plays a crucial role in antiviral defense by directly targeting viral RNAs via the small RNAs produced by the host in response to viruses. Recent work has shown that plant EVs naturally loaded (loaded by the plant cells producing the EVs) with small RNAs, from human edible plants, can modify the composition of the human gut microbiota and oral microbiota by silencing the expression of specific genes in certain commensal bacteria (Teng et al. (2018) Cell Host & Microbes 24:637-652; Sundaram et al. (2019) iScience 21:308-327).

Small interfering RNAs (siRNAs) and microRNAs (miRNAs) are noncoding RNAs with important roles in gene regulation. They have recently been investigated as novel classes of therapeutic agents for the treatment of a wide range of disorders including cancers and infections. Clinical trials of siRNA- and miRNA-based drugs have already been initiated. siRNAs and miRNAs share many similarities, both are short duplex RNA molecules that exert gene silencing effects at the post-transcriptional level by targeting messenger RNA (mRNA), yet their mechanisms of action and clinical applications are distinct. The major difference between siRNAs and miRNAs is that the former are highly specific with only one mRNA target, whereas the latter have multiple targets. The siRNAs and miRNAs have a role in gene regulation, and serve as targets for drug discovery and development. Compared with conventional small therapeutic molecules, siRNAs and miRNAs offer the potential to be highly potent and able to act on "non-druggable" targets (for example, proteins which lack an enzymatic function); moreover, RNAi can be designed to target and/or affect expression of any gene of interest.

**2) Antibody Cargo**

Examples of anti-cancer antibodies and other antibodies, include, but are not limited to, anti-17-1A cell surface antigen antibodies such as the antibody sold or provided under the trademark Panorex® (edrecolomab); anti-4-1BB antibodies; anti-4Dc antibodies; anti-A33 antibodies such as A33 and CDP-833; anti-$\alpha$1 integrin antibodies such as natalizumab; anti-$\alpha 4\beta 7$ integrin antibodies such as LDP-02; anti-$\alpha V\beta 1$ integrin antibodies such as F-200, M-200, and SJ-749; anti-$\alpha V\beta 3$ integrin antibodies such as abciximab, CNTO-95, Mab-17E6, and Vitaxin®; anti-complement factor 5 (C5) antibodies such as 5G1.1; anti-CA125 antibodies such as sold or provided under the trademark OvaRex® (oregovomab); anti-CD3 antibodies such as t hose sold or provided under the trademark Nuvion® (visilizumab) and Rexomab; anti-CD4 antibodies such as IDEC-151, MDX-CD4, OKT4A; anti-CD6 antibodies such as Oncolysin B and Oncolysin CD6; anti-CD7 antibodies such as HB2; anti-CD19 antibodies such as B43, MT-103, and Oncolysin B; anti-CD20 antibodies such as 2H7, 2H7.v16, 2H7.v114, 2H7.v115, the product sold or provided under the trademark Bexxar® (tositumomab), the antibody sold or provided under the trademark Rituxan® (rituximab), and the antibody sold or provided under the trademark Zevalin® (Ibritumomab tiuxetan); anti-CD22 antibodies such as the those sold or provided under the following generic names, tradenames, or trademarks: Lymphocide® (epratuzumab); anti-CD23 antibodies such as IDEC-152; anti-CD25 antibodies such as basiliximab and Zenapax® (daclizumab); anti-CD30 antibodies such as

AC10, MDX-060, and SGN-30; anti-CD33 antibodies such as gemtuzumab ozogamicin (sold under the trademark Mylotarg®), Oncolysin M, and Smart M195; anti-CD38 antibodies; anti-CD40 antibodies such as SGN-40 and toralizumab; anti-CD40L antibodies such as 5c8, Antova®, and IDEC-131; anti-CD44 antibodies such as bivatuzumab; anti-CD46 antibodies; anti-CD52 antibodies such as alemtuzumab (sold under the trademark Campath®); anti-CD55 antibodies such as SC-1; anti-CD56 antibodies such as huN901-DM1; anti-CD64 antibodies such as MDX-33; anti-CD66e antibodies such as XR-303; anti-CD74 antibodies such as IMMU-110; anti-CD80 antibodies such as galiximab and IDEC-114; anti-CD89 antibodies such as MDX-214; anti-CD123 antibodies; anti-CD138 antibodies such as B-B4-DM1; anti-CD146 antibodies such as AA-98; anti-CD148 antibodies; anti-CEA antibodies such as cT84.66, labetuzumab, and Pentacea™; anti-CTLA-4 antibodies such as MDX-101; anti-CXCR4 antibodies; anti-EGFR antibodies such as ABX-EGF, cetuximab (such as the product sold under the trademark Erbitux®), IMC-C225, and Merck Mab 425; anti-EpCAM antibodies such as Crucell's anti-EpCAM, ING-1, and KS-IL-2; anti-ephrin B2/EphB4 antibodies; anti-Her2 antibodies such as tratuzumab (trademark Herceptin®), MDX-210; anti-FAP (fibroblast activation protein) antibodies such as sibrotuzumab; anti-ferritin antibodies such as NXT-211; anti-FGF-1 antibodies; anti-FGF-3 antibodies; anti-FGF-8 antibodies; anti-FGFR antibodies, anti-fibrin antibodies; anti-G250 antibodies such as WX-G250 and Girentuximab (sold under the trademark Rencarex®); anti-GD2 ganglioside antibodies such as EMD-273063 and TriGem®; anti-GD3 ganglioside antibodies such as BEC2, KW-2871, and mitumomab; anti-gpIIb/IIIa antibodies such as ReoPro®; anti-heparinase antibodies; anti-Her2/ErbB2 antibodies such as trastuzumab, MDX-210, and pertuzumab; anti-HLA antibodies (such as the product sold under the trademark Oncolym®), Smart™ 1D10; anti-HM1.24 antibodies; anti-ICAM antibodies such as ICM3; anti-IgA receptor antibodies; anti-IGF-1 antibodies such as CP-751871 and EM-164; anti-IGF-1R antibodies such as IMC-A12; anti-IL-6 antibodies such as CNTO-328 and elsilimomab; anti-IL-15 antibodies (such as the product sold under the trademark HuMax®-IL15); anti-KDR antibodies; anti-laminin 5 antibodies; anti-Lewis Y antigen antibodies such as Hu3S193 and IGN-311; anti-MCAM antibodies; anti-Muc1 antibodies such as BravaRex and TriAb®; anti-NCAM antibodies such as ERIC-1 and ICRT; anti-PEM antigen antibodies such as Theragyn® and Therex®; anti-PSA antibodies; anti-PSCA antibodies such as IG8; anti-Ptk antibodies; anti-PTN antibodies; anti-RANKL antibodies such as AMG-162; anti-RLIP76 antibodies; anti-SK-1 antigen antibodies such as Monopharm C; anti-STEAP antibodies; anti-TAG72 antibodies such as CC49-SCA and MDX-220; anti-TGF-$\beta$ antibodies such as CAT-152; anti-TNF-$\alpha$ antibodies such as CDP571, CDP870, D2E7, adalimumab (such as the product sold under the trademark Humira®), and infliximab (such as the product sold under the trademark Remicade®); anti-TRAIL-R1 and TRAIL-R2 antibodies; anti-VE-cadherin-2 antibodies; and anti-VLA-4 antibodies (such as the product sold under the trademark Antegren®). Furthermore, anti-idiotype antibodies including but not limited to the GD3 epitope antibody BEC2 and the gp72 epitope antibody 105AD7, can be used. In addition, bispecific antibodies including but not limited to the anti-CD3/CD20 antibody Bi20 can be used.

Additional exemplary cargo, uses and treatments that can be effected with cargo-loaded MEVs are described, by way of example, as follows.

### b. Diseases and Methods of Treatment

As described above, the MEVs can be loaded with any desired cargo, including, but not limited to, nucleic acid molecules, detectable marker proteins and tags, small molecule drugs, gene editing systems, and others, and combinations thereof for delivering therapeutic molecules, serving as vaccines, and for use in human and other animal health, agricultural, cosmetic, dermatological and diagnostic applications, industrial uses, and other uses. The MEVs can deliver nutrients, or regulators of gene pathways to produce a beneficial product, gene editing systems, such as CRISPR/cas to effect gene editing, and gene therapy vectors and products.

MEVs can carry cargo, for example, for treating a disease characterized by a genetic defect that results in a deficiency of a functional protein, or for treating a disease characterized by overexpression of a polypeptide. Non-limiting examples of diseases that can be treated by silencing of a target gene, for example using siRNA or microRNA (see, *e.g.,* International Pub. No. WO 2013/048734) include cancer *(e.g.,* lung cancer, leukemia and lymphoma, pancreatic cancer, colon cancer, prostate cancer, glioblastoma, ovarian cancer, breast cancer, head and neck cancer, liver cancer, skin cancer, and uterine cancer), cardiovascular diseases, ocular diseases (e.g., age-related macular degeneration, herpes stromal keratitis, glaucoma, dry eye syndrome, diabetic retinopathy, and conditions associated with ocular angiogenesis and ocular hypertension), neurological diseases (e.g., amyotrophic lateral sclerosis, Alzheimer's disease, myasthenic disorders, Huntington's disease, spinocerebellar ataxia, frontotemporal dementia, Parkinson's disease, prion diseases, and Lafora disease, and those arising from ischemic or hypoxic conditions), kidney disorders, inflammatory or autoimmune diseases (*e.g.,* ischemia or reperfusion injury, restenosis, Rheumatoid arthritis, inflammatory bowel disease, *e.g.*, Crohn's Disease or ulcerative colitis, lupus, multiple sclerosis (MS), diabetes, *e.g.*, type II diabetes, and diabetic conditions, arthritis, *e.g.,* rheumatoid or psoriatic), respiratory diseases (*e.g.,* asthma, Chronic obstructive pulmonary diseases (COPD), cystic fibrosis, acute respiratory distress syndrome (ARDS), emphysema, and acute lung injury), hearing disorders, epilepsy, spinal cord injuries, oral mucositis, male infertility, uterine disorders, endometrial disorders or conditions, as well as conditions relating to metabolism (*e.g.,* obesity), ischemia, stroke, alcohol metabolism and liver

function (see, *e.g.,* International Pub. Nos. WO 2006/029161, WO 2007/022470, WO 2007/130604, WO 2008/021157, WO 2009/104051, WO 2009/142822, WO 2019/217459, WO 2020/123083; European Pub. No. EP 2504435; and U.S. Patent Pub. Nos. U.S. 2011/0223665, U.S. 2012/0116360, U.S. 2012/0071540, U.S. 2016/0257956, U.S. 2015/0196648, and U.S. 2017/0304459). The RNAi molecule may target a gene that encodes, for example, an oncogene, a transcription factor, a receptor, an enzyme, a structural protein, a cytokine, a cytokine receptor, a lectin, a selectin, an immunoglobulin, a kinase and a phosphatase.

Other cargos and uses are contemplated. For example, MEVs can carry cargo, for example, for treating conditions resulting from trauma, such as wounds, burns, skin cuts, broken bones, hair loss, dermis exposure, mucosal exposure, fibrosis, lacerations, and ulcerations. MEVs can carry cargo, for example, for treating conditions resulting from natural or induced aging, in particular on the skin, or of the vision.

MEVs can be used to deliver cargo to treat, *e.g.*, with gene silencing, or prevent, *e.g.*, through vaccination, infectious diseases. For example, MEVs derived from antigen-pulsed macrophages or dendritic cells were shown to elicit an immune response when introduced into naïve animals (György et al. (2015) Annu. Rev. Pharmacol. Toxicol. 55:439-464). Gene silencing also can be used to target a pathogen-associated protein, such as a viral protein involved in immunosuppression of the host, replication of the pathogen, transmission of the pathogen, or maintenance of the infection; or a host protein which facilitates entry of the pathogen into the host, drug metabolism by the pathogen or host, replication or integration of the pathogen's genome, establishment or spread of infection in the host, or assembly of the next generation of pathogen. Pathogens can include, for example, RNA and DNA viruses such as arenaviruses, coronaviruses, influenza viruses, paramyxoviruses, flaviviruses (*e.g.,* West Nile virus), picornaviruses (*e.g.,* Coxsackievirus, Poliovirus, and Rhinovirus), rhabdoviruses, filoviruses, retroviruses (*e.g.,* lentiviruses, and Rous sarcoma virus), adenoviruses, poxviruses, herpes viruses, human papilloma viruses, cytomegaloviruses, hepadnaviruses (*e.g.,* Hepatitis B and C), rotaviruses, respiratory syncytial viruses, polyomaviruses, and others; bacteria; fungi; helminths; schistosomes; trypanosomes; parasites including plasmodiums (*e.g., Plasmodium malariae* and others); and mammalian transposable elements (see, *e.g.,* International Pub. Nos. WO 2010/141724, WO 2011/071860, WO 2011/072292, WO 2013/126803, WO 2020/035620, and WO 2020/097540; Australian Pub. Nos. AU 2004257373 A1, AU 2013203219 B2, and AU 2016225873 A1; European Pub. Nos. EP 2395012, and EP 2888240; U.S. Patent Pub. Nos. U.S. 2011/0223665, U.S. 2014/0256785, and U.S. 2019/0032051; Japanese Pub. No. JP 2018-197239A; and Taiwanese Pub No. TW 201204351A).

MEVs also can be used to deliver DNA or mRNA sequences that encode therapeutically useful polypeptides. For example, in cases where subjects lack a specific gene product, the gene can be encoded in a nucleic acid molecule, such as a DNA or RNA molecule. The nucleic acid molecule encoding the gene product can be loaded into a MEV and delivered to a subject lacking the gene product. For example, diseases that occur due to the absence or deficiency of a gene product include but are not limited to, lysosomal storage disorders; metabolic disorders of the urea cycle; SMN1-related spinal muscular atrophy (SMA); amyotrophic lateral sclerosis (ALS); GALT-related galactosemia; cystic fibrosis (CF); SLC3A1-related disorders including cystinuria; COL4A5-related disorders including Alport syndrome; galactocerebrosidase deficiencies; X-linked adrenoleukodystrophy and adrenomyeloneuropathy; Friedreich's ataxia; Pelizaeus-Merzbacher disease; TSC1 and TSC2-related tuberous sclerosis; Sanfilippo B syndrome (MPS IIIB); CTNS-related cystinosis; the FMR1-related disorders which include Fragile X syndrome, Fragile X-Associated Tremor/Ataxia Syndrome and Fragile X Premature Ovarian Failure Syndrome; Prader-Willi syndrome; hereditary hemorrhagic telangiectasia; Niemann-Pick disease Type C1; the neuronal ceroid lipofuscinoses-related diseases including Juvenile Neuronal Ceroid Lipofuscinosis (JNCL), Juvenile Batten disease, Haltia-Santavuori disease, Jansky-Bielschowsky disease, and PTT-1 and TPP1 deficiencies; EIF2B1-, EIF2B2-, EIF2B3-, EIF2B4- and EIF2B5-related childhood ataxia with central nervous system hypomyelination/vanishing white matter; CACNA1A- and CACNB4-related Episodic Ataxia Type 2; the MECP2-related disorders including Classic Rett Syndrome, MECP2-related Severe Neonatal Encephalopathy and PPM-X Syndrome; CDKL5-related Atypical Rett Syndrome; Kennedy's disease (SBMA); Notch-3 related cerebral autosomal dominant arteriopathy with subcortical infarcts and leukoencephalopathy (CADASIL); SCN1A- and SCN1B-related seizure disorders; the Polymerase G-related disorders, including Alpers-Huttenlocher syndrome, POLG-related sensory ataxic neuropathy, dysarthria, and ophthalmoparesis, and autosomal dominant and recessive progressive external ophthalmoplegia with mitochondrial DNA deletions; X-Linked adrenal hypoplasia; X-linked agammaglobulinemia; and Wilson's disease (see, *e.g.*, International Pub. Nos. WO 2011/068810, WO 2019/243574, WO 2019/092287, and WO 2020/099682).

The MEVs may be loaded with a CRISPR/Cas system to effect gene editing. The clustered, regularly interspaced, short palindromic repeat (CRISPR) technology allows for the modification of the genome in a living organism, and is based on the bacterial CRISPR/Cas9 antiviral defense system. The system allows for DNA cleavage at a target site. The type II CRISPR system incorporates sequences from invading foreign nucleic acids, such as DNA from viruses or plasmids, between CRISPR repeat sequences encoded within the host genome. Transcripts from the CRISPR repeat sequences are processed into CRISPR RNAs (crRNAs). Each crRNAs harbors a variable sequence transcribed from the foreign DNA and a part of the CRISPR repeat. Each crRNA hybridizes with a second transactivating CRISPR RNA

(tracrRNA) and these two RNAs complex with and direct the Cas9 nuclease to cleave the target DNA sequence. By delivering a Cas nuclease complexed with a synthetic guide RNA (gRNA), which consists of a fusion of a crRNA and a tracrRNA, into a cell, the cell's genome can be cut at a desired location, allowing existing genes to be removed and/or new ones added *in vivo* (Sander and Joung (2014) Nat. Biotechnol. 32(4):347-355). The CRISPR technology can be used with the Cas polypeptide or the
single RNA guided endonuclease Cpf1 to effect genome modification, and can be delivered in lipid nanoparticles, EVs and other vesicles (see, *e.g.*, International Pub. Nos. WO 2017/161010, WO 2019/238626, and WO 2020/097540).

MEVs also can be used to treat diseases, including but not limited to those listed above, by introduction of a payload in the form of a therapeutic protein, polypeptide, or small organic molecule or compound to a target cell. Non-limiting examples of such therapeutically effective agents or drugs include oncology drugs (*e.g.,* chemotherapy drugs, hormonal therapeutic agents, immunotherapeutic agents, and radiotherapeutic agents), lipid-lowering agents for treating lipid diseases, anti-viral drugs, anti-fungal agents, anti-cholinergics, anti-inflammatory compounds, antidepressants, stimulants, analgesics, antibiotics, birth control medication, antipyretics, vasodilators, anti-angiogenics, cytovascular agents, anti-fibrotics, antihypertensives, aromatase or esterase inhibitors, signal transduction inhibitors, synthase inhibitors, cardiovascular drugs such as anti-arrhythmic agents, hormones or hormone antagonists, ion channel modifiers, anti-neoplastic agents, neuroactive agents, vasoconstrictors, cytotoxic agents, nucleolytic compounds, radioactive isotopes, pro-drug activating enzymes, and steroids (see, *e.g.*, International Pat. Pub. Nos. WO2015110957A2 and WO2019018349A1; and U.S. Patent Pub. Nos. U.S. 2019/0032051, U.S. 2012/0315324, U.S. 2019/0388347, and U.S. 2019/0175506). The therapeutic also can be a biologic therapeutic agent selected from an allergen, adjuvant, antigen, or immunogen, antibody (*e.g.*, whole antibodies, polyclonal, monoclonal and recombinant antibodies, fragments thereof, and further includes single-chain antibodies, humanized antibodies, murine antibodies, chimeric, mouse-human, mouse-primate, primate-human monoclonal antibodies, anti-idiotype antibodies, antibody fragments, such as, *e.g.*, scFv, (scFv)2, Fab, Fab', and F(ab')2, F(ab)2, Fv, dAb, and Fd fragments, diabodies, and antibody-related polypeptides), cytokine, hormone, factor, cofactor, cell component protein, metabolic enzyme, immunoregulatory enzyme, interferon, interleukin, gastrointestinal enzyme, an enzyme or factor implicated in hemostasis, growth regulatory enzyme, vaccine, antithrombolytic, toxin, antitoxin, or diagnostic or imaging biologic agent (see, *e.g.,* International Pub. Nos. WO 2017/203260, WO 2018/102397, WO 2019/081474, WO 2019/155060, WO 2020/041720; Australian Pub. No. AU 2018365299A1; Singapore Pub. No. SG 11201811149TA; and U.S. Patent Pub. No. U.S. 2019/0202892). For example, MEV therapies can be used to treat Crohn's disease, ulcerative colitis, ankylosing spondylitis, rheumatoid arthritis, multiple sclerosis, systemic lupus erythematosus, sarcoidosis, idiopathic pulmonary fibrosis, psoriasis, tumor necrosis factor (TNF) receptor-associated periodic syndrome (TRAPS), deficiency of the interleukin-1 receptor antagonist (DIRA), endometriosis, autoimmune hepatitis, scleroderma, myositis, stroke, acute spinal cord injury, vasculitis, Guillain-Barre syndrome, acute myocardial infarction, acute respiratory distress syndrome (ARDS), sepsis, meningitis, encephalitis, liver failure, non-alcoholic steatohepatitis (NASH), non-alcoholic fatty liver disease (NAFLD), kidney failure, heart failure or any acute or chronic organ failure and the associated underlying etiology, graft-vs-host disease, Duchenne muscular dystrophy and other muscular dystrophies, lysosomal storage diseases, neurodegenerative diseases, cancer-induced cachexia, anorexia, diabetes mellitus type 2, and cancers (*e.g.*, acute lymphoblastic leukemia (ALL), acute myeloid leukemia, adrenocortical carcinoma, AIDS-related cancers, AIDS-related lymphoma, anal cancer, appendix cancer, astrocytoma, cerebellar or cerebral, basal-cell carcinoma, bile duct cancer, bladder cancer, bone tumor, brainstem glioma, brain cancer, brain tumor (cerebellar astrocytoma, cerebral astrocytoma/malignant glioma, ependymoma, medulloblastoma, supratentorial primitive neuroectodermal tumors, visual pathway and hypothalamic glioma), breast cancer, bronchial adenomas/carcinoids, Burkitt's lymphoma, carcinoid tumor (childhood, gastrointestinal), carcinoma of unknown primary, central nervous system lymphoma, cerebellar astrocytoma/malignant glioma, cervical cancer, chronic lymphocytic leukemia, chronic myelogenous leukemia, chronic myeloproliferative disorders, colon cancer, cutaneous T-cell lymphoma, desmoplastic small round cell tumor, endometrial cancer, ependymoma, esophageal cancer, extracranial germ cell tumor, extragonadal germ cell tumor, extrahepatic bile duct cancer, eye cancer (intraocular melanoma, retinoblastoma), gallbladder cancer, gastric cancer, gastrointestinal carcinoid tumor, gastrointestinal stromal tumor (GIST), germ cell tumor (extracranial, extragonadal, or ovarian), gestational trophoblastic tumor, glioma (glioma of the brain stem, cerebral astrocytoma, visual pathway and hypothalamic glioma), gastric carcinoid, hairy cell leukemia, head and neck cancer, heart cancer, hepatocellular (liver) cancer, hypopharyngeal cancer, intraocular melanoma, islet cell carcinoma (endocrine pancreas), kidney cancer (renal cell cancer), laryngeal cancer, leukemias (acute lymphoblastic, acute myeloid, chronic lymphocytic, chronic myelogenous, hairy cell leukemia), lip and oral cancer, cavity cancer, liposarcoma, liver cancer (primary), lung cancer (non-small cell, small cell), lymphomas, AIDS-related lymphoma, Burkitt lymphoma, cutaneous T-cell lymphoma, Hodgkin lymphoma, non-Hodgkin, medulloblastoma, Merkel cell carcinoma, mesothelioma, metastatic squamous neck cancer with occult primary, mouth cancer, multiple endocrine neoplasia syndrome, multiple myeloma/-plasma cell neoplasm, mycosis fungoides, myelodysplastic/myeloproliferative diseases, myelogenous leukemia, chronic myeloid leukemia, myeloma, nasal cavity and paranasal sinus cancer, nasopharyngeal carcinoma, neuroblastoma, oral cancer, oropharyngeal cancer, osteosarcoma/malignant fibrous histiocytoma of bone, ovarian cancer, ovarian epithelial

cancer (surface epithelial-stromal tumor), ovarian germ cell tumor, ovarian low malignant potential tumor, pancreatic cancer, pancreatic islet cell cancer, parathyroid cancer, penile cancer, pharyngeal cancer, pheochromocytoma, pineal astrocytoma, pineal germinoma, pineoblastoma and supratentorial primitive neuroectodermal tumors, pituitary adenoma, pleuropulmonary blastoma, prostate cancer, rectal cancer, renal cell carcinoma (kidney cancer), retinoblastoma, rhabdomyosarcoma, salivary gland cancer, sarcoma (Ewing family of tumors sarcoma, Kaposi sarcoma, soft tissue sarcoma, uterine sarcoma), Sézary syndrome, skin cancer (nonmelanoma, melanoma), small intestine cancer, squamous cell, squamous neck cancer, stomach cancer, supratentorial primitive neuroectodermal tumor, testicular cancer, throat cancer, thymoma and thymic carcinoma, thyroid cancer, transitional cell cancer of the renal pelvis and ureter, urethral cancer, uterine cancer, uterine sarcoma, vaginal cancer, vulvar cancer, Waldenstrom macroglobulinemia, and/or Wilms' tumor) (see, *e.g.*, International Pub. Nos. WO 2017/203260 and WO 2019/155060A1: and U.S. Patent Pub. No. U.S. 2019/0388347).

**c. Agro-Veterinary Applications**

MEVs carrying cargos of biomolecules can be used for agro-veterinary applications. For example, immune ribonucleic acid can be used for the treatment and prevention of poultry diseases and resistance to virulent pathogens can be enhanced in plants and animals by selective modulation of the miRNA pathway (see, International Pub. No. WO 2008/087562). Cargo-loaded MEVs can thus be used to treat diseases in animals, including livestock.

Cargo-loaded MEVs can be used to treat plant diseases. As exemplified, the MEVs can be loaded with therapeutic molecules, such as siRNAs that target virulence genes in plant pathogens, including bacteria and viruses, and delivered to the plants, such as by application or spraying onto leaves and/or other surfaces, to target the genes to eliminate or to control the pathogen.

**d. Cosmetic and Dermatological Applications**

MEVs carrying payloads of pharmacological agents also can be used for cosmetic and dermatological applications. For example, skin care products such as creams, lotions, gels, emulsions, ointments, pastes, powders, liniments, sunscreens, and shampoos comprising EVs, particularly from stem cells, can be used to improve and/or alleviate symptoms and problems such as dry skin, elasticity, wrinkles, folds, ridges, and/or skin creases (see, *e.g.*, Singapore Pub. No. SG 11201811149TA). Stem cell EVs, which inherently carry cytokines, growth and transcription factors among their cargo, also have been shown to control inflammation, accelerate skin cell migration and proliferation, control wound scarring, improve angiogenesis, and ameliorate signs of skin aging. Although the exact mechanisms are being elucidated, the effect of stem cell EVs on wound healing may rely in the vertical transfer of microRNAs or proteins to skin cells. Angiogenesis, a part of wound healing, can be induced by stem cell EVs. Stem cell EVs also have beneficial effects for cellular matrix maintenance and collagen production, and have been shown to play a role in rejuvenating skin cells (da Fonseca Ferreira, A. and Gomes, D. (2019) Bioengineering (Basel) 6(1):4). MEVs loaded with a desired cargo can thus be used for cosmetic and dermatological applications.

**E. PHARMACEUTICAL COMPOSITIONS, FORMULATIONS, KITS, ARTICLES OF MANUFACTURE AND COMBINATIONS**

**1. Pharmaceutical Compositions and Formulations**

The compositions containing the MEVs and loaded MEVs provided herein can be formulated as pharmaceutical compositions provided for administration by a desired route, such as oral, mucosal, intravenous, and others. Pharmaceutically acceptable compositions are prepared in view of approvals for a regulatory agency or other agency prepared in accordance with generally recognized pharmacopeia for use in animals and in humans, and also, for agricultural applications, for plants. Typically, compounds are formulated into pharmaceutical compositions using techniques and procedures well-known in the art (see *e.g.,* Ansel Introduction to Pharmaceutical Dosage Forms, Fourth Edition, 1985, 126).

The pharmaceutical composition can be used for therapeutic, prophylactic, cosmetic, and/or diagnostic applications. The MEVs and cargo-loaded MEVs provided herein can be formulated with a pharmaceutically acceptable carrier or diluent. Generally, such pharmaceutical compositions include components that do not significantly impair the biological properties or other properties of the cargo. Each component is pharmaceutically and physiologically acceptable so that it is compatible with the other ingredients and not injurious to the subject to whom it is to be administered. The formulations can be provided in unit dosage form and can be prepared by methods well-known in the art of pharmacy, including but not limited to, tablets, pills, powders, liquid solutions or suspensions (*e.g.,* including injectable, ingestible and topical formulations, for example, eye drops, gels, pastes, creams, or ointments), aerosols (*e.g.,* nasal sprays and inhalers),

liposomes, suppositories, pessaries, injectable and infusible solutions and sustained release forms. See, *e.g.,* Gilman, et al. (eds. 1990) Goodman and Gilman's: The Pharmacological Bases of Therapeutics, 8th Ed., Pergamon Press; and Remington's Pharmaceutical Sciences, 17th ed. (1990), Mack Publishing Co., Easton, Pa.; Avis, et al. (eds. 1993) Pharmaceutical Dosage Forms: Parenteral Medications Dekker, NY; Lieberman, et al. (eds. 1990) Pharmaceutical Dosage Forms: Tablets Dekker, NY; and Lieberman, et al. (eds. 1990) Pharmaceutical Dosage Forms: Disperse Systems Dekker, NY. When administered systemically, the therapeutic composition is sterile, pyrogen-free, generally free of particulate matter, and in a parenterally acceptable solution having due regard for pH, isotonicity, and stability. These conditions are known to those skilled in the art. Methods for preparing parenterally administrable compositions are well-known or will be apparent to those skilled in the art and are described in more detail in, *e.g., "*Remington: The Science and Practice of Pharmacy (Formerly Remington's Pharmaceutical Sciences)", 19th ed., Mack Publishing Company, Easton, Pa. (1995).

Pharmaceutical compositions provided herein can be in various forms, *e.g.,* in solid, semi-solid, liquid, powder, aqueous, and lyophilized form. Examples of suitable pharmaceutical carriers are known in the art and include but are not limited to water, buffering agents, saline solutions, phosphate buffered saline solutions, various types of wetting agents, sterile solutions, alcohols, gum arabic, vegetable oils, benzyl alcohols, gelatin, glycerin, carbohydrates such as lactose, sucrose, amylose or starch, magnesium stearate, talc, silicic acid, viscous paraffin, perfume oil, fatty acid monoglycerides and diglycerides, pentaerythritol fatty acid esters, hydroxy methylcellulose, and powders, among others. Pharmaceutical compositions provided herein can contain other additives including, for example, antioxidants, preservatives, antimicrobial agents, analgesic agents, binders, disintegrants, coloring, diluents, excipients, extenders, glidants, solubilizers, stabilizers, tonicity agents, vehicles, viscosity agents, flavoring agents, emulsions, such as oil/water emulsions, emulsifying and suspending agents, such as acacia, agar, alginic acid, sodium alginate, bentonite, carbomer, carrageenan, carboxymethylcellulose, cellulose, cholesterol, gelatin, hydroxyethyl cellulose, hydroxypropyl cellulose, hydroxypropyl methylcellulose, methylcellulose, octoxynol-9, oleyl alcohol, povidone, propylene glycol monostearate, sodium lauryl sulfate, sorbitan esters, stearyl alcohol, tragacanth, xanthan gum, and derivatives thereof, solvents, and miscellaneous ingredients such as crystalline cellulose, microcrystalline cellulose, citric acid, dextrin, dextrose, liquid glucose, lactic acid, lactose, magnesium chloride, potassium metaphosphate, and starch, among others (see, generally, Alfonso R. Gennaro (2000) Remington: The Science and Practice of Pharmacy, 20th Edition. Baltimore, MD: Lippincott Williams & Wilkins). Such carriers and/or additives can be formulated by conventional methods and can be administered to the subject at a suitable dose. Stabilizing agents such as lipids, nuclease inhibitors, polymers, and chelating agents can preserve the compositions from degradation within the body.

The route of administration is in accord with known methods, *e.g.*, injection or infusion by intravenous, intraperitoneal, intracerebral, intramuscular, subcutaneous, intraocular, intraarterial, intrathecal, inhalation or intralesional routes, topical, rectal, mucosal, and by sustained release systems. The MEVs or cargo-loaded MEVs can be administered continuously by infusion or by bolus injection. One can administer the MEVs or cargo-loaded MEVs in a local or systemic manner.

The MEVs or cargo-loaded MEVs can be prepared in a mixture with a pharmaceutically acceptable carrier. Techniques for formulation and administration of the compounds are known to one of skill in the art (see *e.g., "*Remington's Pharmaceutical Sciences," Mack Publishing Co., Easton, Pa.). This therapeutic composition can be administered intravenously or through the nose or lung, such as a liquid or powder aerosol (lyophilized). The composition also can be administered parenterally or subcutaneously as desired. When administered systematically, the therapeutic composition should be sterile, pyrogen-free and in a parenterally acceptable solution having due regard for pH, isotonicity, and stability. These conditions are known to those skilled in the art.

Pharmaceutical compositions suitable for use include compositions wherein the MEVs or cargo-loaded MEVs are contained in an amount effective to achieve their intended purpose. Determination of a therapeutically effective amount is well within the capability of those skilled in the art. Therapeutically effective dosages can be determined by using *in vitro* and *in vivo* methods, and/or by a skilled person.

Therapeutic formulations can be administered in many conventional dosage formulations. Dosage formulations of MEVs and cargo-loaded MEVs provided herein are prepared for storage or administration by mixing the compound having the desired degree of purity with physiologically acceptable carriers, excipients, or stabilizers. Such materials are non-toxic to the recipients at the dosages and concentrations employed, and can include buffers such as Tris HCl, phosphate, citrate, acetate and other organic acid salts; antioxidants such as ascorbic acid; low molecular weight (less than about ten residues) peptides such as polyarginine, proteins, such as serum albumin, gelatin, or immunoglobulins; hydrophilic polymers such as polyvinylpyrrolidone; amino acids such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium, and/or nonionic surfactants such as polysorbates (TWEEN), pluronics, polyethylene glycol, and others.

In particular examples herein, provided are pharmaceutical compositions that contain a stabilizing agent. The stabilizing agent can be an amino acid, amino acid derivative, amine, sugar, polyol, salt or surfactant. In some examples,

the stable co-formulations contain a single stabilizing agent. In other examples, the stable co-formulations contain 2, 3, 4, 5 or 6 different stabilizing agents. For example, the stabilizing agent can be a sugar or polyol, such as a glycerol, sorbitol, mannitol, inositol, sucrose or trehalose. In particular examples, the stabilizing agent is sucrose. In other examples, the stabilizing agent is trehalose. The concentration of the sugar or polyol is from or from about 100 mM to 500 mM, 100 mM to 400 mM, 100 mM to 300 mM, 100 mM to 200 mM, 200 mM to 500 mM, 200 mM to 400 mM, 200 mM to 300 mM, 250 mM to 500 mM, 250 mM to 400 mM, 250 mM to 300 mM, 300 mM to 500 mM, 300 mM to 400 mM, or 400 mM to 500 mM, each inclusive.

In examples, the stabilizing agent can be a surfactant that is a polypropylene glycol, polyethylene glycol, glycerin, sorbitol, poloxamer and polysorbate. For example, the surfactant can be a polypropylene glycol, polyethylene glycol, glycerin, sorbitol, poloxamer and polysorbate, such as a poloxamer 188, polysorbate 20 and polysorbate 80. In particular examples, the stabilizing agent is polysorbate 80. The concentration of surfactant, as a % of mass concentration (w/v) in the formulation, is between or about between 0.005% to 1.0%, 0.01% to 0.5%, 0.01% to 0.1%, 0.01% to 0.05%, or 0.01% to 0.02%, each inclusive.

When used for *in vivo* administration, the formulation should be sterile and can be formulated according to conventional pharmaceutical practice. This is readily accomplished by filtration through sterile filtration membranes, prior to or following lyophilization and reconstitution. The MEVs or cargo-loaded MEVs can be stored in lyophilized form or in solution; they can be frozen or refrigerated. Other vehicles such as naturally occurring vegetable oil like sesame, peanut, or cottonseed oil or a synthetic fatty vehicle like ethyl oleate can be included. Buffers, preservatives, and antioxidants can be incorporated according to accepted pharmaceutical practice.

The MEVs or cargo-loaded MEVs provided herein, can be provided at a concentration in the composition of from or from about 0.1 to 10 mg/mL or higher or lower amounts, depending upon the application and the subject, such as, for example a concentration that is at least or at least about 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10 mg/mL or more. The volume of the solution can be at or about 1 to 100 mL, such as, for example, at least or about at least or 0.5, 1, 2, 3, 4, 5, 10, 15, 20, 25, 30, 35, 40, 45, 50, 55, 60, 65, 70, 75, 80, 85, 90, 95, 100 mL or more. In some examples, the MEVs or cargo-loaded MEVs are supplied in phosphate buffered saline.

The MEVs or cargo-loaded MEVs provided herein can be provided as a controlled release or sustained release composition. Polymeric materials are known in the art for the formulation of pills and capsules which can achieve controlled or sustained release of the MEVs and cargo-loaded MEVs provided herein (see, *e.g.,* Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Langer and Peppas (1983) J. Macromol. Sci. 23:61; see also Levy et al. (1985) Science 228:190; During et al. (1989) Ann. Neurol. 25:351; Howard et al. (1989) J. Neurosurg. 71:105; U.S. Pat. Nos. 5,679,377, 5,916,597, 5,912,015, 5,989,463, 5,128,326; and PCT Publication Nos. WO 99/15154 and WO 99/20253). Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. Generally, the polymer used in a sustained release formulation is inert, free of leachable impurities, stable on storage, sterile, and biodegradable. Any technique known in the art for the production of sustained release formulation can be used to produce a sustained release formulation containing the MEVs or cargo-loaded MEVs provided herein.

In some examples, the pharmaceutical composition contains the MEVs or cargo-loaded MEVs provided herein and one or more additional agents, such as an antibody or other therapeutic, for combination therapy.

### 2. Articles of Manufacture/Kits and Combinations

Pharmaceutical compositions of the MEVs or cargo-loaded MEVs can be packaged as articles of manufacture containing packaging material, a pharmaceutical composition which is effective for treating a disease or condition that can be treated by administration of the particular MEVs or cargo-loaded MEVs, such as the diseases and conditions described herein or known in the art, and a label that indicates that the cargo, such as an antibody or nucleic acid molecule, is to be used for treating the infection, disease or disorder. The pharmaceutical compositions can be packaged in unit dosage forms containing an amount of the pharmaceutical composition for a single dose or multiple doses. The packaged compositions can contain a lyophilized powder of the pharmaceutical compositions containing the cargo-loaded MEVs which can be reconstituted (*e.g.,* with water or saline) prior to administration.

The articles of manufacture provided herein contain packaging materials. Packaging materials for use in packaging pharmaceutical products are well-known to those of skill in the art (see, *e.g.,* U.S. Patent Nos. 5,323,907, 5,052,558 and 5,033,252). Examples of pharmaceutical packaging materials include, but are not limited to, blister packs, bottles, tubes, inhalers (*e.g.,* pressurized metered dose inhalers (MDI), dry powder inhalers (DPI), nebulizers (*e.g.,* jet or ultrasonic nebulizers) and other single breath liquid systems), pumps, bags, vials, containers, syringes, bottles, and any

packaging material suitable for a selected formulation and intended mode of administration and treatment.

The MEVs or cargo-loaded MEVs can be provided as combinations and as kits. Kits optionally can include one or more components such as instructions for use, devices and additional reagents (*e.g.,* sterilized water or saline solutions for dilution of the compositions and/or reconstitution of lyophilized protein), and components, such as tubes, containers and syringes for practice of the methods. Exemplary kits can include the MEVs or cargo-loaded MEVs provided herein, and can optionally include instructions for use, a device for administering the MEVs or cargo-loaded MEVs to a subject, a device for detecting MEVs or cargo-loaded MEVs in samples obtained from a subject, and a device for administering an additional therapeutic agent to a subject.

The kit can, optionally, include instructions. Instructions typically include a tangible expression describing the MEVs or cargo-loaded MEVs, and, optionally, other components included in the kit, and methods for administration, including methods for determining the proper state of the subject, the proper dosage amount, dosing regimens, and the proper administration method for administering the MEVs or cargo-loaded MEVs. Instructions also can include guidance for monitoring the subject over the duration of the treatment time.

Kits also can include a pharmaceutical composition described herein and an item for diagnosis. For example, such kits can include an item for measuring the concentration, amount or activity of the MEVs and cargo-loaded MEVs, in a subject.

In some examples, the MEVs or cargo-loaded MEVs are provided in a diagnostic kit for the detection of the MEVs or cargo-loaded MEVs or cargo in an isolated biological sample (*e.g.,* tumor cells, such as circulating tumor cells obtained from a subject or tumor cells excised from a subject).

Kits provided herein also can include a device for administering the MEVs to a subject. Any of a variety of devices known in the art for administering medications to a subject can be included in the kits provided herein. Exemplary devices include, but are not limited to, a hypodermic needle, an intravenous needle, a catheter, a nebulizer, and an inhaler. Typically, the device for administering the compositions is compatible with the desired method of administration of the composition.

### 3. Administration of Exogenously Loaded MEVs and Routes of Administration

The cargo-loaded MEVs provided herein can be administered to a subject by any method known in the art for the administration of polypeptides, including for example systemic or local administration. The cargo-loaded MEVs can be administered by routes, such as parenteral (*e.g.,* routes, such as intradermal, intramuscular, intraperitoneal, intravenous, subcutaneous, and intracavity), topical, epidural, or mucosal (*e.g.,* routes, such as topical, intranasal, oral, vaginally, vulvovaginal, esophageal, oroesophageal, bronchial, rectal, and pulmonary). The cargo-loaded MEVs can be administered externally to a subject, at the site of the disease for exertion of local or transdermal action. Compositions containing the cargo-loaded MEVs can be administered by any convenient route, for example by infusion, inhalation, by bolus injection, or by absorption through epithelial or mucocutaneous linings (*e.g.,* topical, oral, vaginal, rectal and intestinal mucosa). Compositions containing the cargo-loaded MEVs can be administered together with or sequentially with other biologically active agents. For example, the cargo-loaded MEVs are administered by infusion delivery, such as by infusion pump or syringe pump, and can be administered in combination with another therapeutic agent or as a monotherapy.

The method and/or route of administration can be altered to alleviate adverse side effects associated with administration provided herein. For example, if a patient experiences a mild or moderate (*i.e.,* Grade 1 or 2) infusion reaction, the infusion rate can be reduced (*e.g.,* reduced by 10%, 20%, 30%, 40%, 50%, 60%, 70%, 80%, 90% or more). If the patient experiences severe (*i.e.,* Grade 3 or 4) infusion reactions, the infusion can be temporarily or permanently discontinued.

In some examples, if the subject experiences an adverse side effect, such as severe skin toxicity, for example severe acneiform rash, treatment adjustments can be made. For example, after the occurrence of an adverse side effect, administration can be delayed, such as for 1 to 2 weeks or until the adverse side effect improves. In some examples, after additional occurrences of an adverse side effect, the dosage can be reduced. A particular regimen and treatment protocol can be established by the skilled physician or other practitioner.

Appropriate methods for delivery, can be selected by one of skill in the art based on the properties of the dosage amount of the cargo-loaded MEVs or the pharmaceutical composition containing the cargo-loaded MEVs. Such properties include, but are not limited to, solubility, hygroscopicity, crystallization properties, melting point, density, viscosity, flow, stability and degradation profile.

### 4. Combination Therapies

The cargo-loaded MEVs provided herein can be administered before, after, or concomitantly with one or more other therapeutic regimens or agents. The skilled medical practitioner can determine empirically, or by considering the pharmacokinetics and modes of action of the agents, the appropriate dose or doses of each therapeutic regimen or agent,

as well as the appropriate timings and methods of administration. The additional therapeutic regimens or agents can improve the efficacy or safety or other properties of the cargo-loaded MEVs. In some examples, the additional therapeutic regimens or agents can treat the same disease or a comorbidity. In some examples, the additional therapeutic regimens or agents can ameliorate, reduce or eliminate one or more side effects known in the art or described herein that are associated with administration of the cargo-loaded MEVs or the cargo.

For example, the cargo-loaded MEVs described herein can be administered with chemotherapy, radiation therapy, or both chemotherapy and radiation therapy, or for anti-viral or anti-bacterial or other pathogen therapy, the cargo-loaded MEVs can be administered with other anti-pathogen therapeutics and treatments. The cargo-loaded MEVs can be administered in combination with one or more other prophylactic or therapeutic agents, including but not limited to antibodies, cytotoxic agents, chemotherapeutic agents, cytokines, growth inhibitory agents, anti-hormonal agents, kinase inhibitors, anti-angiogenic agents, cardio-protectants, immunostimulatory agents, immunosuppressive agents, agents that promote proliferation of hematological cells, angiogenesis inhibitors, protein tyrosine kinase (PTK) inhibitors, FcγRIIb or other Fc receptor inhibitors, or other therapeutic agents.

The one or more additional agents can be administered simultaneously, sequentially or intermittently with the cargo-loaded MEVs. The agents can be co-administered, for example, as part of the same pharmaceutical composition or same method of delivery. In some examples, the agents can be co-administered at the same time as the cargo-loaded MEVs, but by a different means of delivery. The agents also can be administered at a different time than administration of the cargo-loaded MEVs, but close enough in time to have a combined prophylactic or therapeutic effect. In some examples, the one or more additional agents are administered subsequent to or prior to the administration of the cargo-loaded MEVs separated by a selected time period. In some examples, the time period is 1 day, 2 days, 3 days, 4 days, 5 days, 6 days, 1 week, 2 weeks, 3 weeks, 1 month, 2 months, or 3 months. In some examples, the one or more additional agents are administered multiple times and/or the cargo-loaded MEVs provided herein are administered multiple times.

## F. BIODISTRIBUTION OF MEVs FOLLOWING ADMINISTRATION VIA VARIOUS ROUTES

### 1. Biodistribution of mammalian EVs

Pharmacokinetics and biodistribution in organs and tissues of mammalian EVs have been extensively studied for their pharmacokinetics and distribution in organs and tissues (Vader et al. (2016) Advanced drug delivery reviews 106(Pt A):148-156, doi.org/10.1016/j.addr.2016.02.006; Morishita et al. (2017) Journal of pharmaceutical sciences 106(9):2265-2269, hdoi.org/10.1016/j.xphs.2017.02.030). Treatments with mammalian cell-derived EVs are generally based on intravenous or intraperitoneal routes of administration. Primary target organs upon systemic administration of mammalian EVs are the liver, spleen and lungs. A comprehensive study (see, Wiklander et al. (2015) J. Extracellular Vesicles 4:26316) of the tissue distribution of fluorescently-labelled mammalian EVs from various cell sources demonstrated that 24 hours after intravenous (i.v.) injection in mice, the highest fluorescence signal was in the liver, followed by spleen, gastrointestinal tract and lungs. Furthermore, cell source, EV dose, and route of administration was shown to affect EV distribution; for example, injection of higher EV doses resulted in relatively lower liver accumulation compared to lower doses, possibly caused by saturation of the mononuclear phagocyte system (MPS). Comparison between intraperitoneal (i.p.), subcutaneous (s.c.) and i.v. administrations showed that intraperitoneal and subcutaneous doses resulted in reduced EV accumulation in liver and spleen and enhanced pancreas and gastrointestinal tract accumulation compared to i.v. injections. Systemically administered EVs are reported to be rapidly taken up by the mononuclear phagocyte system (MPS), particularly in the liver and spleen. The mechanism of clearance resembles that described for synthetic nanoparticles, such as liposomes (Van der Meel et al. (2014) J. Control. Release 195:72-8). The majority of splenic accumulation is caused by EV storage in the spleen rather than uptake by the spleen (Lai C.P. et al. (2014) ACS Nano 8:483-494). Biodistribution of mammalian EVs following other routes of administration also has been investigated. For targeting of the central nervous system, intranasal administration of curcumin-loaded mammalian EVs resulted in EV localization in the brain. Drug levels peaked at 1 hour after administration, and a significant amount detected after 12 hours with no toxic effects observed (Zhuang et al. (2011) Mol. Ther. 19:1769-1779).

In general, mammalian EVs are not employed for oral delivery because of their low stability at various pH and temperatures, rapid degradation of biomolecules in the digestive tract, and the limitations of industrial scale production for oral dosing (Cheng et al. (2019) Protein Cell 10:295-299). The only exception so far are bovine milk-derived EVs, which upon oral delivery to mice have shown a pattern of distribution that, analyzed with whole-body in vivo imaging system (IVIS), included rapid accumulation in the intestine, where the EVs were detectable after 2 and 6 hours, followed by fluorescence signal observed in liver, spleen, lungs, kidney, heart, and the gastrointestinal tract at 24 hours. After 48 hours, the fluorescence signal subsided within most of the organs indicating the clearance of nanovesicles from the system (Samuel et al. (2021) Nat Commun 12:3950, doi.org/10.1038/s41467-021-24273-8). Thus, mammalian EVs (derived from sources other than milk) cannot be absorbed by the intestinal tract and from the intestines to become bioavailable in target organs (Zhong et al. (2021) Biomaterials. 277:121126. doi: 10.1016/j.biomaterials.2021.121126).

Treatments with mammalian cell-derived EVs generally employ intravenous or intraperitoneal routes of administration for systemic administration where the target organs are the liver, spleen and lungs. As noted, most mammalian EVs have not been employed for oral delivery due to their low stability at various pH and temperatures, rapid degradation of biomolecules in the digestive tract, and the limitations of industrial scale production for oral dosing (Cheng et al. (2019) Protein Cell 10(4):295-299). The only exception are bovine milk-derived EVs, which upon oral delivery to mice have shown a pattern of distribution that, analyzed with whole-body in vivo imaging system (IVIS), include rapid accumulation in the intestine, where the EVs were detectable after 2 and 6 hours, followed by fluorescence signal observed in liver, spleen, lungs, kidney, heart, and the gastrointestinal tract at 24-hour time point. After 48 hours, the fluorescence signal subsided within most of the organs indicating the clearance of nanovesicles from the system

As shown herein in the Examples, and discussed below, MEVs have different properties from mammalian EVs. For example, they are stable in the harsh environment of the gastrointestinal tract compared to mammalian cell-derived EVs. Thus, the microalgae EVs, as described herein, are particularly suitable for oral administration and drug delivery, as well as other routes of delivery as described herein.

**2. Microalgae EVs Biodistribution**

It is shown herein that MEVs, including those provided herein from *Chlorella,* have properties that are distinct from mammalian EVs, including bovine milk EVs. For example, a striking difference, discussed below, is that the MEVs can be administered orally, and that the primary target is the spleen, likely the white pulp of the spleen (white spleen).

The MEVs provided herein can deliver a variety of bioactive molecules, such as RNAs, such as mRNA, siRNA, and miRNA; proteins; peptides; and small molecules, which can be exogenously or endogenously loaded. These include products such as tissue-specific products and/or disease specific products. As discussed below, each route can be used to target particular organs and treat particular diseases. The MEVs can be formulated for administration by each route. Thus, provided are compositions containing MEVs that are for treating particular disease and for particular routes of administration.

It is shown herein, the route of administration determines the fate of the MEVs, and that the ultimate location of the MEVs is a function of the route of administration. Targets and endpoints of the MEVs include, but are not limited to, the liver, spleen, lungs, the intestines, and brain. Routes of administration include, but are not limited to, respiratory (nose, lungs), oral (digestive), intravenous, central nervous system (CNS), and topical. The selection of route depends upon the ultimate target and the payload. It is shown herein that intranasal administration goes to the lungs, intratracheal via a spray goes to the lung(s), intravenous accumulates in the spleen and liver, oral (per Os) goes to the digestive tract and spleen. In contrast, mammalian EVs cannot be taken orally.

MEVs are readily internalized by human cells. For example, *in vitro,* when administered to cells in culture, such as A549 cells, at a ratio of MEV/cell of 1000/1, 93% of the cells internalized the MEVs, and this occurred within 24 to 48 hours after contacting the cells with the MEVs.

DIR-labeled MEVs were administered to mice via four routes: intranasal (IN), intratracheal (IT), intravenous (IV), and oral, and, by full-body imaging as a function of time, the fate of the MEVs was visualized for 3 days, followed by sacrificing the mice to harvest organs for study. As shown in the examples, intravenous administration targets the liver at about 4-12 hours following administration, and the spleen, appearing to be in the red pulp of the spleen (red spleen), at 10-30 hours. Oral administration targets the intestine and spleen. It is shown herein that the MEVs are orally available; they resist passage through the stomach, and reach the intestine at 0.5 hour to 4 hours, and then the spleen at 0.5 hour to 10 hours. Of interest is the route to the spleen; there are two possible routes to the spleen, via the blood (to red spleen), and via lymphocytes (to white spleen), which has implications for targeting and delivering cargo to the immune system, accumulating from 4 hours to 28 hours. This can be effected by internalization by lymphocytes that are activated and end up in the spleen where they multiply, and/or by lymphocytes that phagocytose the MEVs, which are not activated, and go to the white pulp of the spleen (white spleen) from where they are disseminated through the immune system.

**a. Oral Administration**

Thus, orally ingested MEVs go into the intestine, then, as shown, end up in the spleen, likely the white spleen. The spleen is responsible for initiating immune reactions to blood-borne antigens, and for filtering foreign material and old or damaged red blood cells from the blood. These functions are performed by two different compartments in the spleen: the white spleen, and red spleen. The two compartments are vastly different in structure, vascular organization, and cellular composition (see, *e.g.,* Cesta (2006) Toxicologic Pathology 34:455-465 for a review of the structure, function and histology of the spleen).

White blood cells, which are plentiful in the intestine, migrate to the white spleen. When ingested orally the MEVs can be internalized by intestinal cells and, as discussed below, including by intestinal lymphocytes, which carry the MEVs to the spleen. This is in contrast to mammalian vesicles, which cannot be administered orally. Thus, MEVs provide a

delivery vehicle for agents for which the immune system is a target, such as for immune modulating cargo. As discussed above, the pathway to the white spleen can occur, for example, via activated lymphocytes and/or phagocytic lymphocytes. Lymphocytes can phagocytose the MEVs, and are homed to the spleen. The MEVs, unlike mammalian EVs, provide a way to orally deliver small molecule drugs and proteins and other therapeutics, such as nucleic acid therapeutics, that cannot be administered orally. In particular, orally administered MEVs provide a route for treatment of diseases, such as cancers and inflammatory diseases, in which the immune system is involved or in which the treatment can be effected by targeting the immune system. Such diseases include, but are not limited to, infectious disease, autoimmune diseases, cancers, prevention of organ transplant rejection. These diseases are treated by suppressing or augmenting the activity of immune cells.

**1) Components of the Lymphatic System**

The lymphatic system includes lymph, lymphatic vessels and lymphatic organs (see, discussion in Zgair et al., (2016) Targeting Immunomodulatory Agents to the Gut-Associated Lymphoid Tissue. In: Constantinescu C., Arsenescu R., Arsenescu V. (eds) Neuro-Immuno-Gastroenterology. Springer, Cham. (doi.org/10.1007/978-3-319-28609-9_14) and summarized below).

**Lymph**

Lymph is a generally clear and colorless fluid that drains from the interstitium, and contains recovered fluids and plasma proteins, and also can contain lipids, immune cells, hormones, bacteria, viruses, cellular debris, and cancer cells.

**Lymphatic Vessels**

The lymphatic system is the body's second circulatory system. The lymphatic system is a unidirectional, blind-ended and thin-walled system of capillary vessels where lymph is driven. Lymphatic capillaries drain in the afferent collecting vessels, which then pass through one or more gatherings of lymph nodes. Lymph fluid then passes through the efferent collecting vessels, larger trunks and then the lymphatic duct, which drain lymph to the systemic circulation. Primary lymphatic organs include the thymus gland and bone marrow, which produce mature lymphocytes, which identify and respond to antigens; secondary lymphatic organs include lymph nodes, spleen and mucosa-associated lymph tissues (MALT). Within the secondary lymphatic organs, lymphocytes initiate immune responses. MALT are distributed throughout mucous membranes and provide a defensive mechanism against a wide variety of inhaled or ingested antigens. MALT are categorized according to their anatomical location as: bronchus-associated lymphoid tissue (BALT), nasal-associated lymphoid tissue (NALT), salivary gland duct-associated lymphoid tissue (DALT), conjunctiva-associated lymphoid tissue (CALT), lacrimal duct-associated lymphoid tissue (LDALT) and gut-associated lymphoid tissue (GALT).

**Gut-Associated Lymphoid Tissue (GALT)**

GALT is composed of effector and immune induction sites. Effector sites include lymphocytes distributed throughout the lamina propria (LP) and intestinal epithelium; induction sites involve tissues, such as such as mesenteric lymph nodes (MLN), PP and smaller isolated lymphoid follicles (ILF). Mesenteric lymph nodes (MLN), which occur in the base of the mesentery, are the largest gatherings of lymph nodes in the body. The structure of MLN is divided into two regions: the medulla and cortex. The cortex primarily is composed of T-cell areas and B-cell follicles. Within the T-cell area, circulating lymphocytes enter the lymph node, and dendritic cells (DC) present antigens to T-cells. Lymph (containing cells, antigens and chylomicrons) is collected from the intestinal mucosa and reaches the MLN via the afferent lymphatics. Lymph fluid subsequently leaves the MLN through efferent lymphatics to reach the thoracic duct that drains to the blood.

Peyer's patches (PP) are a collection of lymphoid nodules distributed in the mucosa and submucosa of the intestine. They contain a sub-epithelial dome area and B-cell follicles dispersed in a T-cell area. A single layer of epithelial cells, called follicle-associated epithelium (FAE), separates lymphoid areas of PP from the intestinal lumen. FAE is permeated by specialized enterocytes called microfold (M) cells. These cells are a gate for the transport of luminal antigens to PP.

Isolated lymphoid follicles (ILF) are a combination of lymphoid cells in the intestinal LP. ILF are composed of germinal centers covered by FAE containing M-cells. ILF is a complementary system to PP for the induction of intestinal immunity.

GALT is the largest lymphatic organ in the human body and contains more than half of the body's lymphocytes. GALT is exposed to more antigens in the form of commensal bacteria and alimentary antigens, in addition to those from invasive pathogens, than any other part of the body. Intestinal lymphatic transport avoids hepatic first-pass metabolic loss by diverting the absorption of lipophilic drugs towards intestinal lymphatics rather than the portal vein. The intestinal

immune system must distinguish antigens that require a protective immune response and develop a state of immune hyporesponsiveness (oral tolerance) for harmless antigens. This is effected by sampling of luminal antigens in the intestinal epithelium by DC. Antigens can cross the epithelium through M-cells, which are specialized epithelial cells of the follicle-associated epithelium of the GI tract. The antigens interact with DC in the underlying sub-epithelial dome region. Antigens are presented to local T-cells in PP by DC.

DC also migrate to the draining MLN where they present antigens to local lymphocytes. Alternative pathways for antigen transport across the intestinal epithelial cells involve receptor-mediated transport, and direct sampling from the lumen by DC projections. Antigen-loaded DC then migrate to the MLN through afferent lymphatics where they present antigens to T-cells. Subsequently, differentiated lymphocytes migrate from MLN through the thoracic duct and blood stream and eventually accumulate in the mucosa for an appropriate immune response.

### 2) Targeting GALT

Orally administered MEVs can target gut-associated lymphoid tissue (GALT). Thus, GALT is a target (effective compartment) and/or a route through which MEVs and their therapeutic agent cargo can be used to deliver cargo to organs, tissues, and/or systemic circulation. GALT is an advantageous target for various pharmacological agents such as, for example, immunomodulators, chemotherapeutic agents, anti-infective agents. The lymphatic system is a main pathway for intestinal and other tumor metastases; therefore, targeting cytotoxic drugs to the intestinal lymphatics can be used to treat tumor metastases. GALT is a delivery target for antiviral agents, as some viruses, such as, for example, human immunodeficiency virus (HIV), morbillivirus, canine distemper virus, severe acute respiratory syndrome (SARS)-associated coronaviruses, hepatitis B and hepatitis C, spread and develop within the lymphatic system.

Thus, MEVs, including the *Chlorella* MEVs exemplified herein, can be used to target immune cells upon oral delivery. As described above, the microalgae MEVs show a distinct pattern of biodistribution when administered orally. This pattern includes initial intestine accumulation followed by targeting the spleen, where they are detectable up to 24 hours (see, *e.g.,* Fig. 7).

Since the microalgae MEVs are delivered to the spleen, the mechanism of this delivery can be based on cells of the immune system. Immune cells are abundant in the single-cell layer of intestinal epithelium and underlying lamina propria of the gut-associated lymphoid tissue (GALT). The immune cells include, T cells, plasma cells, mast cells, dendritic cells, and macrophages (Luongo et al. (2009) Current perspectives. International Reviews of Immunology 28(6) :446-464, doi.org/10.3109/08830180903236486). Macrophages, dendritic cells, neutrophils, and also B cells perform phagocytosis. The immune cells in the gut, thus, can phagocytose the MEVs to deliver them to the spleen. After phagocytosis, the fate of the MEV cargo can depend upon the type of cargo. For example, macrophage and dendritic cells participate in antigen presentation, and present proteins delivered in the MEVs, or the products in the MEVs can be secreted, or the products, such RNA, can be translated.

Immune cells present in the intestinal epithelium and lamina propria of the intestine migrate to the spleen and back to the intestine. This homing to the spleen can be involved in MEV transfer from the gut to secondary lymphatic organs, especially to the spleen. T cells exhibit a specific lymphocyte recirculation pathway (Mackay et al. (1990) J Exp Med 171:801-17) that can be part of MEV trafficking to the spleen upon oral delivery. Therefore, cells of the immune system are targeted by orally-administered MEVs, and this phenomenon contributes to MEV localization in the spleen within hours post-administration.

As shown herein, upon oral administration, the MEVs go to the intestine and then migrate to the spleen. The route to the spleen can be via absorption into the blood and/or by internalization by immune cells in the intestine. The blood route is an unlikely route, because the MEVs then would appear in the liver as shown for intravenous administration. When MEVs are administered intravenously they primarily reach the liver (massively) and to a much lesser extent the spleen. It is shown herein that clearance of the MEVs from the spleen follows different kinetics depending upon their origin (oral or IV). The migration to the spleen following oral administration therefore uses a different a pathway from the MEVs administered intravenously. When MEVs are administered by mouth, they reach the spleen after having passed through the intestine. These results indicate that the MEVs are located in "different compartments" inside the spleen, depending on the route of arrival: either from the intestine or from the blood. As discussed, upon oral administration, the likely route is that the MEVs in the intestine are internalized by lymphocytes present in the GALT, and that the subsequent migration of the MEVs from the intestine/GALT to the spleen occurs because the MEVs are transported by the lymphocytes. Coming from the intestine/GALT, the MEVs end up in the white spleen compartment. Thus, the MEVs provide a way to deliver cargo to different organs from mammalian EVs, which cannot be administered orally.

### 3. Diseases and conditions treated by MEVs

Based upon the targeted organs, a variety of diseases and disorders can be treated by MEVs. The MEVs can be loaded or produced to contain therapeutic agents for treating these diseases and conditions. The appropriate route of

administration for the targeted organ and disease is selected. For example, for targeting the spleen and intestines, oral administration is selected; and for targeting the lungs, inhalation or nasal administration is selected. Based on the biodistribution and pharmacokinetic data the following organs can be targeted to treat diseases exemplified as follows.

**liver:** cancer, cancer metastases, metabolic syndrome, genetic disorders (delivery of gene therapy), alpha-anti-trypsin (AAT) deficiency and other inborn errors of metabolism, hemophilia, hypercholesterolemia, liver inflammation, steatohepatitis, and other diseases and disorders that can be treated by delivery of a therapeutic to the liver;
**spleen:** diseases treated by immune modulation, including cancers, and immune cell disorders, and cancer, and other diseases that can be treated by administration to the spleen, particularly by immune cells that occur in or traffic to the white spleen;
**intestine:** diseases and disorders treated or prevented by vaccines, intestinal infections, microbiota modulation, Crohn's disease, cancer, ulcers, diseases treated by orally administered drugs, such as small molecules and proteins, and other such diseases, disorders, and conditions; and
**lungs:** infectious diseases, particularly respiratory diseases, chronic obstructive pulmonary disease (COPD), pulmonary hypertension, asthma, other inflammatory lung diseases, cystic fibrosis, ATT-deficiency, lung disease, cancer, cancer metastases, and other such diseases and disorders.

## G. FORMULATIONS, ROUTES OF ADMINISTRATION, AND DISEASE AND DISORDERS

Provided are compositions containing the MEVs in an amount suitable for effecting treatment for a particular disease or disorder. The amount can depend upon the therapeutic cargo, the disease, or disorder, and the subject treated. It is within the level of skill in the art to ascertain a particular dosage of MEVs. Formulations include any known to those of skill and include, for example:

injectables for intravenous administration, to reach the liver and the spleen;
oral, such as, for example tablets, capsules, films, and troches;
drops for per os administration, to reach the intestine, such as a vaccine, the immune system (immune cells), and the spleen;
compositions, such as emulsions (microemulsions and nanoemulsions) for inhalation, such for intratracheal, intrapulmonary administration; to reach the lungs;
drops for intranasal administration; and
formulations, such as creams, oils, gels, lotions, ointments for the skin and the mucosa.

Provided are pharmaceutical compositions containing, in a pharmaceutically acceptable vehicle microalgae extracellular vesicles (MEVs). The MEVs can contain an agent, generally a therapeutic or biologically active agent, such as nucleic acid, particularly an RNA, a protein, a small molecule, and other such agents. The compositions contain an amount of the MEV that can be diluted to deliver a therapeutically effective amount of the agent, or are formulated for direct administration without dilution. The particular concentration of MEVs depends upon a variety of parameters within the skill of a skilled artisan, including, for example, the treated indication; the active agent; the route of administration; the disease, disorder, or condition to be treated; and the regimen. Routes of administration include systemic and local routes, oral, rectal, intravenous, intramuscular, subcutaneous, mucosal, inhalation, nasal, eye, peritoneal, intratracheal, intravitreal, vaginal, and any suitable route known to the skilled person.

Pharmaceutical carriers or vehicles suitable for administration of the compounds provided herein include any such carriers known to those skilled in the art to be suitable for the particular mode of administration.

### Exemplary Formulations

Pharmaceutical compositions containing the MEVs can be formulated in any conventional manner, by mixing a selected amount of the active compound with one or more physiologically acceptable carriers or excipients. Selection of the carrier or excipient is within the skill of the administering professional, and can depend upon a number of parameters. These include, for example, the mode of administration (*i.e.,* systemic, oral, nasal, pulmonary, local, topical, or any other mode), and the disorder treated. The formulations also can be co-formulations with other active agents for combination therapy.

A selected amount of MEVs are formulated in a suitable vehicle for administration by a selected route. The pharmaceutical compositions can be formulated in any conventional manner, by mixing a selected amount of MEVs with one or more physiologically acceptable carriers or excipients or vehicles. The pharmaceutical composition can be used for therapeutic, prophylactic, cosmetic and/or diagnostic applications. The concentration of the MEVs in a composition, depends on a variety of factors, including those noted above, as well as the absorption, inactivation, and excretion rates of

the active agent cargo, the release of the cargo, the mechanism of release, the dosage schedule, and the amount administered, the age and size of the subject, as well as other factors known to those of skill in the art, and related to the properties of the MEVs.

The pharmaceutical compositions provided herein can be in various forms, such as, but not limited to, in solid, semi-solid, liquid, emulsions, powder, aqueous, and lyophilized forms. The pharmaceutical compositions provided herein can be formulated for single dosage (direct) administration, or for dilution, or other regimen. The concentrations of the compounds in the formulations are effective, either following dilution or mixing with another composition, or for direct administration, for delivery of an amount, upon administration, that is effective for the intended treatment. The compositions can be formulated in an amount for single or multiple dosage direct administration. The form of composition depends a variety of factors, including the intended mode of administration. The resulting mixtures are solutions, suspensions, emulsions and other such mixtures, and can be formulated as creams, gels, ointments, emulsions, solutions, lotions, suspensions, tinctures, pastes, foams, aerosols, irrigations, and sprays. For oral administration, the MEVs can be formulated as tablets, capsules, lozenges, liquids, and others.

For local internal administration, such as intramuscular, parenteral or intra-articular administration, the MEVs can be formulated in isotonically buffered saline. The effective concentration of the MEVs is sufficient to provide a sufficient amount of the cargo agent for the intended purpose, and can be empirically determined.

Generally, pharmaceutically acceptable compositions are prepared in view of approvals for a regulatory agency, or other agency, and/or are prepared in accordance with generally recognized pharmacopeia for use in animals and in humans. Pharmaceutical compositions can include a carrier, such as a diluent, adjuvant, excipient, or vehicle, with which a polypeptide is administered. Such pharmaceutical carriers can be sterile liquids, such as water and oils, including those of petroleum, animal, vegetable or synthetic origin, such as peanut oil, soybean oil, mineral oil, and sesame oil. Water is a typical carrier when the pharmaceutical composition is administered intravenously. Saline solutions and aqueous dextrose and glycerol solutions also can be employed as liquid carriers, particularly for injectable solutions. Compositions can contain, along with an active ingredient, a diluent, such as lactose, sucrose, dicalcium phosphate, and carboxymethylcellulose; a lubricant, such as magnesium stearate, calcium stearate and talc; and a binder, such as starch, natural gums, such as gum acacia, gelatin, glucose, molasses, polyvinylpyrrolidone, celluloses and derivatives thereof, povidone, crospovidone, and other such binders known to those of skill in the art. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, and ethanol. A composition, if desired, also can contain minor amounts of wetting or emulsifying agents, or pH buffering agents, for example, acetate, sodium citrate, cyclodextrin derivatives, sorbitan monolaurate, triethanolamine sodium acetate, triethanolamine oleate, and other such agents. These compositions can take the form of solutions, suspensions, emulsions, tablets, pills, capsules, powders, granules, and sustained release formulations. Capsules and cartridges of, *e.g.,* gelatin, for use in an inhaler or insufflator, can be formulated containing a powder mix of a therapeutic compound and a suitable powder base, such as lactose or starch. A composition can be formulated as a suppository, with traditional binders and carriers, such as triglycerides. Oral formulations can include standard carriers, such as pharmaceutical grades of mannitol, lactose, starch, magnesium stearate, sodium saccharine, cellulose, magnesium carbonate, and other such agents. Preparations for oral administration also can be suitably formulated with protease inhibitors, such as a Bowman-Birk inhibitor, a conjugated Bowman-Birk inhibitor, aprotinin and camostat. Examples of suitable pharmaceutical carriers are described in "Remington's Pharmaceutical Sciences" by E. W. Martin. Such compositions will contain a therapeutically effective amount of the compound, generally in purified form, together with a suitable amount of carrier, so as to provide the compound in a form for proper administration to a subject or patient.

The pharmaceutical compositions provided herein can contain other additives, including, for example, antioxidants, preservatives, antimicrobial agents, analgesic agents, binders, disintegrants, colorings, diluents, excipients, extenders, glidants, solubilizers, stabilizers, tonicity agents, vehicles, viscosity agents, flavoring agents, emulsions, such as oil-in-water or water-in-oil emulsions, emulsifying and suspending agents, such as acacia, agar, alginic acid, sodium alginate, bentonite, carbomer, carrageenan, carboxymethylcellulose, cellulose, cholesterol, gelatin, hydroxyethyl cellulose, hydroxypropyl cellulose, hydroxypropyl methylcellulose, methylcellulose, octoxynol-9, oleyl alcohol, povidone, propylene glycol monostearate, sodium lauryl sulfate, sorbitan esters, stearyl alcohol, tragacanth, xanthan gum, and derivatives thereof, solvents, and miscellaneous ingredients, such as crystalline cellulose, microcrystalline cellulose, citric acid, dextrin, dextrose, liquid glucose, lactic acid, lactose, magnesium chloride, potassium metaphosphate, and starch, among others (see, generally, Alfonso R. Gennaro (2000) Remington: The Science and Practice of Pharmacy, 20th Edition. Baltimore, MD: Lippincott Williams & Wilkins). Such carriers and/or additives can be formulated by conventional methods and can be administered to the subject at a suitable dose. Stabilizing agents, such as lipids, nuclease inhibitors, polymers, and chelating agents, can preserve the compositions from degradation within the body.

The formulation should suit the mode of administration. For example, the MEVs can be formulated for parenteral administration by injection (e.g., by bolus injection, or continuous infusion). The injectable compositions can take such forms as suspensions, solutions or emulsions in oily or aqueous vehicles. The sterile injectable preparation also can be a

sterile injectable solution, or a suspension in a non-toxic parenterally-acceptable diluent or solvent, for example, as a solution in 1,4-butanediol. Sterile, fixed oils are conventionally employed as a solvent or suspending medium. For this purpose, any bland fixed oil can be employed, including, but not limited to, synthetic mono- or diglycerides, fatty acids (including oleic acid), naturally occurring vegetable oils, such as sesame oil, coconut oil, peanut oil, cottonseed oil, and other oils, or synthetic fatty vehicles like ethyl oleate. Buffers, preservatives, antioxidants, and the suitable ingredients, can be incorporated as required, or, alternatively, can comprise the formulation. The MEVs provided herein, can be formulated as the sole pharmaceutically active ingredient in the composition, or can be combined with other active ingredients. Suspension of the MEVs can be suitable for administration. These can be prepared according to methods known to those skilled in the art.

Suitable compositions for intranasal administration include but are not limited to, powders, sprays, liquids, suspensions, emulsions, and any other form that can be administered directly to the nose and that can contain the MEVs. The concentration of MEVs can be empirically determined, and depends upon the cargo, the indication treated, or intended use.

The therapeutically concentration of the MEVs can be determined empirically by testing the compounds in known *in vitro* and *in vivo* systems Determination of a therapeutically effective amount is well within the capability of those skilled in the art.

## H. BIODISTRIBUTION AND DELIVERY OF MEVs TO THE BRAIN VIA INTRANASAL (IN) ADMINISTRATION FOR TREATING DISEASES, DISORDERS, AND CONDITIONS OF THE BRAIN AND CNS

As discussed throughout the disclosure herein, MEVs provide numerous advantages for delivery of bioactive molecules, including therapeutic and diagnostic or detectable molecules, compared to other vehicles, including EVs from other sources, including plant sources (see discussion in the section below, and throughout the disclosure). In particular, as described and shown herein, the MEVs can be administered intranasally, and traffic via unique pathways to areas of interest in the brain. The MEVs can be loaded with cargo that includes nucleic acids, such as plasmids, anti-sense oligonucleotides (ASO), mRNA, lncRNA, siRNA, miRNA, and other RNAs, peptides including proteins/peptides/polypeptides, small molecules, drugs, diagnostic agents, and other molecules. MEVs can serve as vectors to the brain of pharmacologically active compounds with poor stability in gastrointestinal fluids, poor intestinal absorption and/or extensive hepatic first-pass elimination, such as polar drugs.

Administration of MEVs by IN administration provides for transporting drugs that act in the brain, such as, for example, anti-depressants, antipsychotics, anxiolytics, memory enhancers, agents for treatment of dementia, and agents for treatment of cancers, to target cells where their effects are manifested, without general distribution in the body that can occur by systemic, such as intravenous, or by oral administration. MEVs provide a non-invasive solution for delivering drugs targeted for CNS and brain diseases, disorders, and conditions. MEVs can deliver molecules that are unable to cross the blood-brain barrier (BBB). For hydrophilic compounds, access to the brain is restricted by the BBB which does not allow the transfer from the vascular compartment to the brain tissue. IN administration also can deliver molecules that are not able to reach the brain after first-pass metabolism.

### 1. Brain structure

In view of the findings described and exemplified herein regarding biodistribution of MEVs in the brain (see, e.g., the working examples and accompanying figures) the following brain areas/nuclei are of interest in view of their recognized involvement in brain diseases, disorders, and conditions, including psychiatric and neurologic disorders, and major functions of the central nervous system.

#### a. Anterior Olfactory Nucleus

The anterior olfactory nucleus refers to the most rostral group of nerve cells that receive input from the olfactory bulb. In the human and the macaque they form small groups scattered within the olfactory tract from the olfactory bulb through the olfactory peduncle to a much larger group on the dorsal surface of the tract on the underside of the orbital gyri. It is composed of several subgroups, which are defined by topology.

The anterior olfactory nucleus is located posterior to the olfactory bulb in the olfactory peduncle. It is one of the major olfactory processing centers; the olfactory bulb is its major afferent input and also is the principal target of its axons. The anterior olfactory nucleus (AON) is the initial recipient of odor information from the olfactory bulb, and the target of dense innervation conveying spatiotemporal cues from the hippocampus. Episodic and contextually-relevant odor engrams are stored within the AON; its activity is necessary and sufficient for the behavioral expression of odor memory.

**b. Tenia Tecta**

The tenia tecta refers to a continuation ventrally of the supracallosal gyrus beyond the rostrum of the corpus callosum (see, *e.g.,* URL:/braininfo.rpre.washington.edu/centraldirectory.aspx?ID=1870). In the human and the macaque it lies on the rostral surface of the lamina terminalis and is considered identical to or part of the paraterminal gyrus. In the rat and the mouse it is located similarly in relation to the supracallosal gyrus; it is a more prominent layered structure that extends rostrally on the medial surface overlying the anterior olfactory nucleus. It is considered part of the olfactory areas (rodent) of the cerebral cortex. In rodents it consists of two parts, the dorsal tenia tecta and the ventral tenia tecta. The ventral tenia tecta (vTT) is a component of the olfactory cortex and receives both bottom-up odor signals and top-down signals. Tenia tecta (vTT), an area of the olfactory cortex located in the ventromedial aspect of the olfactory peduncle, transforms the perception of odor signals into reward-directed behaviors.

**c. Olfactory Tubercle**

The olfactory tubercle refers to a predominantly cellular structure defined on the basis of a Nissl stain. It is located on the ventral surface of the endbrain caudal to the anterior olfactory nucleus, medial to the olfactory tract, rostral to the piriform area and ventral to the nucleus accumbens and substantia innominata. It contains some of the islands of Calleja.

In humans the olfactory tubercle is not very developed; it is barely distinguishable from the overlying nucleus accumbens. In the macaque it is somewhat more prominent and bounded medially by the tenia tecta. In primates it does not protrude from surrounding areas and is penetrated by numerous small blood vessels. These give it the appearance on dissection that accounts for the name 'anterior perforated substance' in human neuroanatomy. The location in the rat and the mouse is the same as in the macaque. It protrudes on the rostroventral surface of the endbrain where it is more clearly stratified and much larger in proportion to the size of the brain than in primates. It is involved in the proper sense of smell.

**d. Piriform Cortex**

Primary olfactory cortex or Piriform Cortex is located in the temporal lobe. The piriform cortex (PC) is a key brain area involved in processing and coding of olfactory information. It is implicated in various brain disorders, such as epilepsy, Alzheimer's disease, and autism. The PC consists of the anterior (APC) and posterior (PPC) parts, which are different anatomically and functionally.

The piriform cortex (PC) is located in the ventrolateral region of the forebrain and extends broadly along the anterior to posterior (AP) axis in mammals. As one of the primary olfactory cortex, the PC is involved in encoding odor identification (Gottfried et al. (2006) Neuron 49:467-479; Howard et al.(2009) Nature Neuroscience 12:932-938; Wilson et al. (2011) Neuron 72: 506-519; Bekkers et al. (2013) Trends in Neuroscience 36:429-438; Courtiol et al.(2017) Perception 46(3-4):320-332, doi.org/10.1177/0301006616663216), odor associated values or contexts (Gottfried et al.(2003) Neuron 39:375-386; Calu et al. (2007) In: Cerebral cortex (New York, N.Y. : 1991) 17:1342-1349, Roesch et al. (2007) In: Cerebral cortex (New York, N.Y. : 1991) 17:643-652), and odor memory (Zelano et al. (2011) Neuron 72:178-187; Strauch et al. (2018) Cerebral Cortex 28:764-776).

The PC also is implicated in various neurological disorders, such as epilepsy (Loscher et al. (1996) Progress in Neurobiology 50:427-481; Vismer et al. (May, 2015) Front. Neural Circuits, 29, doi.org/10.3389/fncir.2015.00027; Young et al. (2019) Experimental Neurology 320:113013), Alzheimer's disease (Samudralwar et al. (1995) Journal of the Neurological Sciences 130:139-145; Saiz-Sanchez et al. (2015) Brain Struct Funct 220:2011-2025.,doi.org/10.1007/s00429-014-0771-3), autism spectrum disorder (Menassa et al. (2018) Neuroscience Letters 665:86-91; Koehler et al. (2018) Chemical Senses 43:627-634), and Parkinson's disease (Wu et al. (2011) Human Brain Mapping 32:1443-1457*).*

**e. Amygdala**

Social behaviors are disrupted in several psychiatric disorders. The amygdala is a key brain region involved in social behaviors, and amygdala pathology has been implicated in disease states ranging from social anxiety disorder to autism. Frequently implicated in psychotic spectrum disorders, the amygdala serves as a hub for elucidating the convergent and divergent neural substrates in schizophrenia and bipolar disorder, the two most studied groups of psychotic spectrum conditions.

**f. Entorhinal Cortex**

Entorhinal cortex (EC) relays object-related and spatial information from the perirhinal and parahippocampal cortices (PRC, PHC) to the hippocampus (HC). The entorhinal cortex projects weakly to the basal nucleus. Efferent fibers

from the entorhinal cortex pass through the lateral nucleus, but it is not clear if the fibers form synapses or terminal plexuses within the nucleus. The projection from the amygdala to the entorhinal cortex arises primarily from the lateral nucleus and is most robust passing to anterior portions of the cortex. Unlike its projections to other areas of cortex, the basal nucleus contributes only a weak projection to entorhinal cortex. Two-thirds of all cortical projections to the hippocampus are relayed through the entorhinal cortex. It is not known if the entorhinal cortex provides the same information to the amygdala as it does to the hippocampus.

The entorhinal cortex is located in the mesial temporal lobe and acts as the interface between the hippocampus and the neocortex. It has been considered part of the hippocampal formation. It occupies the middle portion of the medial temporal region and includes part of the parahippocampal gyrus and gyrus ambiens 2. It is increasingly defined by its connectivity to the hippocampus.

### g. Frontal Cortex

The frontal cortex (FC) is the cerebral cortex covering the front part of the frontal lobe. This brain region is implicated in planning complex cognitive behavior, personality expression, decision making, and moderating social behavior. The basic activity of this brain region is orchestration of thoughts and actions in accordance with internal goals. Functions carried out by the frontal cortex area are referred to as executive functions.

### h. **Striatum: caudate nucleus and putamen**

Two subcortical nuclei within the basal ganglia, the bilateral caudate nucleus and bilateral putamen, form the striatum. The caudate nucleus primarily is involved with emotion regulation, reward processing, decision making and executive functioning, while the putamen is primarily associated with the planning and production and purification; from the regulatory perspective, independent dossiers implementation of motor functions. Because of the strategic location and connectivity of the caudate nuclei and the putamen within frontostriatal circuits, morphological changes to these nuclei have been linked to the clinical functioning of patients with Parkinson's disease

### i. Nucleus accumbens

Nucleus accumbens is considered as a neural interface between motivation and action, having a key-role in food intake, sexual behavior, reward-motivated behavior, stress-related behavior and substance-dependence. It is involved in several cognitive, emotional and psychomotor functions, altered in some psychopathology. Moreover it is involved in some of the most common and most severe psychiatric disorders, such as depression, schizophrenia, obsessive-compulsive disorder and other anxiety disorders, as well as in addiction, including drugs abuse, alcoholism and smoking. Nucleus accumbens has also a role in other psychiatric disorders such as bipolar disorder, attention deficit/ hyperactivity disorder and post-traumatic stress disorder. Nucleus accumbens deep brain stimulation has been also associated with antidepressant and anxiolytic effect, as well as quality of life improvement in patients suffering from severe resistant depression. Finally, nucleus accumbens deep brain stimulation has been proved beneficial for all phenotypic components of the Tourette syndrome, with remarkable reduction of the syndrome's motor manifestations, including tics.

### j. Thalamus

The thalamus is concerned in the higher nervous functions such as language, cognition, memory and intelligence. Severe nerve cell loss with proliferation of hypertrophic astroglia is observed in the association nuclei and sensory relay nuclei in the thalami of patients suffering from Creutzfeldt-Jakob disease. In a brain imaging study, volume reduction of the thalamus, especially of dorsomedial nuclei, and degradation of glucose metabolism were observed in the thalami of patients with schizophrenia. Schizophrenia has been considered to be a subcortical neurotransmitter imbalance syndrome. Schizophrenia has been described as a misconnection syndrome or cognitive dysmetria induced by dysfunction of the cortico-cerebellar-thalamic-cortical circuit (CCTCC).

### k. Hypothalamus

The hypothalamus is responsible for the control of important and vital functions by the release of several hormones such as CRH (corticotropin-releasing hormone), TRH (tyrotropin-releasing hormone), GnRH (gonadotropin-releasing hormone or luteinizing-releasing hormone, oxytocin, vasopressin, somatostatin (growth hormone-inhibiting hormone, GHIH), GHRH (growth hormone-releasing hormone), responsible among other for the control of body temperature regulation, maintaining daily physiological cycles, controlling appetite, managing sexual behavior and regulating emotional responses.

**l. Substantia nigra pars compacta**

Pathologically, Parkinson's disease is characterized by the loss of dopaminergic neurons in the pars compacta of the substantia nigra.

**m. Hippocampus**

The hippocampus has a pivotal role in learning and in the formation and consolidation of memory and is critically involved in the regulation of emotion, fear, anxiety, and stress. Studies of the hippocampus have been central to the study of memory in humans. The hippocampus is a model for the study of neuroplasticity as many examples of synaptic plasticity such as long-term potentiation and depression have been identified and demonstrated in hippocampal circuits.

**n. Colliculus**

The extensive connections of the superior colliculus make it a major center for initiating eye movements and coordinating them with movements of the head and neck. The superficial layers of the superior colliculus contain a retinotopic map of the environment and the deeper layers contain premotor neurons with connections to networks that generate saccades and head movements. The auditory, somatosensory and visual signals that converge on the superior colliculus move the eyes, head and body to direct the line of sight towards objects of interest for orienting behavior.

**o. Pontine Raphe nuclei**

Raphe nuclei are characterized by high content in serotonin (5HT). They are responsible for the release of 5HT to other parts of the brain. Selective serotonin reuptake inhibitor (SSRI) drugs, for example, are thought to act on the raphe nucleus for achieving their antidepressant action.

**2. The Blood-Brain Barrier**

Drug development for central nervous system (CNS) diseases and psychiatric disorders is challenging due to the side effects of drugs, the complexity of the brain, and notably, the lack of efficient strategies to deliver drugs across the blood-brain barrier (BBB). The blood-brain barrier (BBB) restricts drug access to the brain, limiting the lipophilic drugs. In this context, development of molecules for delivery to the brain is challenging because of: (1) the design of active molecules, according to structure-activity rules discovered from 3D models, crystal structure of the target, in agreement with Lipinski's rule of five (Lipinski (2004) Drug Discovery Today: Technologies 1(4):337-34), which describes molecular properties important for a drug's pharmacokinetics in the human body; and (2) the specific constraints imposed by the BBB, which requires optimization of the permeation of molecules across the BBB, which in turn depends on molecular weight, lipophilicity, H bond donors and acceptors, charge, and polar surface area.

These problems can be solved by IN delivery of the MEVs, which bypass or are not restricted by the BBB. The blood-brain barrier (BBB) is formed by endothelial cells at the level of the cerebral capillaries. These endothelial cells interact with perivascular elements such as basal lamina and closely associated astrocytic end-feet processes, perivascular neurons (represented by an interneuron in Figure 47) and pericytes to form a functional BBB. Cerebral endothelial cells are unique in that they form complex tight junctions (TJ) produced by the interaction of several transmembrane proteins that effectively seal the paracellular pathway (Figure 47b). These complex molecular junctions make the brain practically inaccessible for polar molecules, unless they are transferred by transport pathways of the BBB that regulate the microenvironment of the brain. There also are adherens junctions (AJ), which stabilize cell-cell interactions in the junctional zone. In addition, the presence of intracellular and extracellular enzymes such as monoamine oxidase (MAO), γ-glutamyl transpeptidase (γ-GT), alkaline phosphatase, peptidases, nucleotidases and several cytochrome P450 enzymes endow this dynamic interface with metabolic activity. Large molecules such as antibodies, lipoproteins, proteins and peptides can be transferred to the central compartment by receptor-mediated transcytosis or nonspecific adsorptive-mediated transcytosis. Included are proteins, are receptors for insulin, low-density lipoprotein (LDL), iron transferrin (Tf), and leptin, that are involved in transcytosis. Others include, for example the multidrug resistance-associated protein family, such as P-glycoprotein.

Soluble molecules can cross the BBB via different mechanisms. Several lipid-soluble molecules can enter the brain by passive diffusion. In this mechanism, the molecule lipophilicity generally defines the penetration rate and extent into the brain. Many of these molecules are usually pumped back to the circulatory system by some efflux pumps expressed in the BBB. Small polar molecules, such as amino acids, glucose, nucleosides, and organic anions and cations, are transported by carrier-mediated transport. Another mechanism is receptor-mediated transcytosis, which transports large molecules, such as iron Tf, insulin, and leptin. Similar to Lipinski's rule of five, the permeation of a molecule across the

BBB depends on its molecular weight, lipophilicity, H bond donors and acceptors, charge, and polar surface area. Thus, only a small number of hydrophobic and low molecular weight molecules can cross the BBB, whereas others are restricted by the barrier characteristics of the BBB, which makes it difficult to develop drugs that target the brain.

**3. Brain and target cells**

The central nervous system (which includes the brain and spinal cord) is composed primarily of two cell types: neurons, and glial cells. Glial cells come in several types, and perform a number of critical functions, including structural support, metabolic support, insulation, and guidance of development. Both glial cells and neurons can be a target for MEVs, as delivery of a therapeutic cargo to either type of cells is of clinical relevance. Dysfunction in glial cells associates with a variety of brain diseases such as Alzheimer's disease, Parkinson's disease, multiple sclerosis, glioblastoma, autism and psychiatric disorders. Neuronal degeneration, on the other hand, is also involved in Alzheimer's disease and Parkinson's disease, as well as ischemic stroke and a number of genetic neurodegenerative conditions, including amyotrophic lateral sclerosis and Huntington's disease. Both cell types may undergo malignant transformation, resulting in brain tumors such as astrocytoma, glioblastoma and medulloblastoma.

**4. Differences between Biodistribution of MEVs and other delivery vehicles**

MEVs are different from any other kind EV and nanoparticle. Mammalian EVs are a very heterogenous group of EVs because they arise from different cells, tissues, and organs, such as from stem cells, dendritic cells, tumor cells, and other sources. There is no single mammalian EV; each has different properties and must be separately developed. They share some common phenotypic markers, but they are structurally different, carry different payloads *in vivo,* and, originate from different cells types. From an industrial/pharmaceutical perspective, each kind will require a more or less adapted process for must be constituted for each kind of mammalian EV, and they are not necessarily routine or easy to produce.

MEVs, as shown herein, are uniform in composition. For exogenously-loaded MEVs, a single and common process for production and purification can result in a myriad of products using the same MEVs, which when exogenously loaded, can carry different payloads, such as small molecules, RNA products, proteins, peptides, polypeptides, and others. The MEVs are uniform in structure and contents. From a regulatory perspective, as the outside of the MEV is the same (irrespective from the payload inside) the MEVs will share the same toxicities, if any, or lack thereof. Formulations can be developed for each route of delivery independent of cargo (payload). Endogenously-loaded MEVs will be similarly uniform in structure.

It is shown and described herein, that the MEVs traverse unique pathways, depending on the route of administration. For example, as discussed elsewhere herein, MEVs provide unique routes of delivery via oral administration compared to EVs from other sources, nanoparticles, and viruses. With respect to intranasal administration, prior art has established, for example that:

1) the IN route of administration is a suitable way to access the brain and to deliver drugs and biologicals;
2) Extracellular vesicles from mammalian origin have been reported to deliver payloads to the brain (including proteins, siRNA, miRNA, mRNA) by IN and other routes of administration. See, *e.g.*, ncbi.nlm.nih.gov/pmc/articles/PMC6202788/, ncbi.nlm.nih.gov/pmc/articles/PMC7409518/, ncbi.nlm.nih.gov/pmc/articles/PMC8363003/;
3) Synthetic nanoparticles (also referred to as nanovectors) made of synthetic molecules/lipids plus lipids extracted from grapefruit exosome-like nanoparticles, have been reported to deliver siRNA to the brain by IN administration. These synthetic nanovectors are reported to reach the olfactory bulb, the hippocampus, the thalamus, the cerebellum, the cerebral cortex, and the striatum.
4) Synthetic nanoparticles made of polycaprolactone (polycaprolactone nanoparticles (PCL NPs) and PEG-modified PCL NPs have been reported to deliver curcumin (small molecule) to the brain by IN administration. These particles are reported to enter the brain via the trigeminal nerve; they cannot get through the olfactory nerve.
5) Infectious viruses as well as endogenous intracellular vesicles have been reported to travel within the brain via axonal transportation.
6) The passage of viruses and intracellular vesicles over the synapses has been demonstrated.

The following Table provides a summary of prior art extracellular vesicles (EV) from other sources, including mammalian EVs, and other nanoparticles, and their pathways upon administration, to contrast with the results using MEVs as shown and described herein. It is apparent from the table that MEVs, when administered, behave differently from EVs from other sources, and differently from other vehicles, such as lipid nanoparticles. This is particularly apparent when administration is via intranasal administration (and as discussed elsewhere herein via oral administration). It is apparent from the table and description and results presented here that MEVs follow unique pathways upon administration. MEVs behave differently from EVs from other sources. Because of the pathways followed by MEVs they can provide for targeted

delivery to brain as well as other organs.

| # | Type of nanoparticle | Route of administration to the brain | Entry site as reported | Travel route | Pathways (brain regions targeted/ reached) | Reference |
|---|---|---|---|---|---|---|
| **1** | EVs from cultured mammalian cells | intranasal | Primary location in the olfactory bulb | Extraneuronal pathway | Rostral brain regions | Zhuang et al., Mol Ther. 19(10):1769 -1779 (2011). |
| **2** | EVs from GM mammalian cells | Intravenous, systemic | N/A | BBB crossing | Neurons, microglia, oligodendrocytes in the brain | Alvarez-Erviti et al., " Nat. Biotechnol. 29(4):341-5 (2011). |
| **3** | grapefruit-derived nanovectors (GNVs) | intranasal | Not specified | Non specified | *"the majority of the GNVs were located in the lung and brain"* | Wang et al., Nat. Commun. 4:1867 (2013). |
| **4** | enzyme-loaded mammalian EVs | intranasal | Transport along the olfactory nerve cells is indicated | Route not specified. Hypothesis: tetraspanins and integrins on exosomes are enable to attachment to the plasma membranes of target cells | Predominantly cerebral frontal cortex, central sulcus, and cerebellum | Haney et al., J. Control Release 207:18-30 (2015). |
| **5** | grapefruit-derived nanovectors (GNVs) | intranasal | by olfactory bulb | Route not specified, Hypothesis: extraneuronal pathway because rapid movement of GNVs into the brain within 1.5 hour of IN administratio n | Primary locations in the olfactory bulb, hippocampus, thalamus, and cerebellum | Zhuang et al., Mol. Ther. 24(1):96-105 (2016). |
| **6** | gold nanoparticle-labeled EVs from mammalian cells | intranasal, intravenous | by olfactory bulb | Route not specified. Hypothesis: extraneuronal pathway; transcytosis across blood brain barrier also indicated | Widespread biodistribution in normal brain; accumulation in ischemic-like damage region | Betzer et al., ACS Nano. 11(11):1088 3-10893 (2017). |

(continued)

| # | Type of nanoparticle | Route of administration to the brain | Entry site as reported | Travel route | Pathways (brain regions targeted/ reached) | Reference |
|---|---|---|---|---|---|---|
| 7 | solid lipid nanoparticles (SLN) | Intraperitoneal, intranasal | Not specified, Hypothesis: olfactory bulb for IN administration BBB after IP administration | IN route: SLN reaches CNS through 1) direct olfactory transport, along the olfactory and trigeminal nerves pathway or 2) systemic circulation pathway via the transmucosal absorption IP route: hepatic portal system and BBB | IN: nasal region, liver, spleen and kidneys; some in the brain | Esposito et al., Eur J Pharm Biopharm. 115:285-296 (2017). |
| 8 | modified EVs from mammalian cells | intravenous | N/A | BBB crossing | Ischemic lesion region, entering microglia, neurons and astrocytes | Tian et al., Biomaterials 150:137-149 (2018). |
| 9 | grapefruit-derived nanovectors (GNVs) | intranasal | by olfactory bulb | Route not specified Hypothesis: extraneuronal pathway because the authors found rapid movement of GNVs into the brain within 1.5 hour of IN administration | Primary locations in the olfactory bulb, hippocampus, thalamus, and cerebellum | US application publication 20180362974 |
| 10 | EVs from stimulated mammalian cells | intranasal | Not specified | EVs are taken up by microglial cells | Microglia at 4 hrs: primarily in the lungs, brain, gut, liver, and heart; at 24 hrs: in the brain and heart, but absent in the lungs and the gut | Hu et al., Mol. Ther. Nucleic Acids. 13:450-463 (2018). |

(continued)

| # | Type of nanoparticle | Route of administration to the brain | Entry site as reported | Travel route | Pathways (brain regions targeted/ reached) | Reference |
|---|---|---|---|---|---|---|
| **11** | drug-loaded artificial phospholipid -based vesicles | intranasal | Olfactory region | Nasal mucosa membrane indicated from *in vitro* experiments. Hypothesis: systemic absorption *in vivo* | High accumulation in the cerebrum and also in the olfactory bulb (within 10 minutes) | Natsheh et al., Drug Deliv. Transl. Res. 8(3):806-819 (2018). |
| **12** | gold nanoparticle-labeled EVs from human stems cells | intranasal | Migration of EVs to the spinal cord is by chemotaxis, but entry of EVs not specified | BBB crossing | in brain and olfactory bulbs in healthy animals, spinal cord lesions in a model of injury | Guo et al., ACS Nano. 13(9):10015 -10028 (2019). |
| **13** | gold nanoparticle-labeled EVs from human stems cells | intranasal | Migration of EVs to the spinal cord is by chemotaxis, but entry of EVs not specified | BBB crossing | brain and olfactory bulbs in healthy animals, spinal cord lesions in a model of injury | WO2019186 558A1 |
| **14** | enzyme-loaded mammalian EVs | intrathecal, intranasal, intravenous, intraperitoneal | Not specified | Not specified | hypothalamus, thalamus, pons, spinal cord | Haney et al., Cells. 9(5):1273 (2020). |
| | | | | | *little, if any, DiR signal after IN administration* (over 6-480 hrs) | |
| **15** | EVs from human stems cells | Intra-hippocampal transplantation, retro-orbital vein injection, intranasal | Not specified. Depends on route of administration : *i.h.*-Migrates through CA1 stratum radiatum, *r.o.*-Trigeminal nerve, *i.n.*- Olfactory bulb/ trigeminal nerve. | Extracellular space or circulation | *Various subregions of the brain at roughly equivalent levels* | Ioannides et al. J. Cancer Metastisis Treat. 6(15):10.205 17/2394-4722 (2020). |
| **16** | EVs from stimulated human stems cells | intranasal | Not specified, likely olfactory and trigeminal nerve pathways. | Not detailed, but rapid detection in brain indicates extraneuronal pathway; transcytosis across blood brain barrier | CA1 region of medial hippocampus (EVs *robustly incorporated into CA1 microglia and, to some extent, into neurons, but not in astrocytes*) | Losurdo et al. " Stem Cells Transl Med. 9(9):1068-1084 (2020). |

(continued)

| # | Type of nanoparticle | Route of administration to the brain | Entry site as reported | Travel route | Pathways (brain regions targeted/ reached) | Reference |
|---|---|---|---|---|---|---|
| **17** | EVs from human stems cells | intranasal | Not specified, likely olfactory and trigeminal nerve pathways. | Not specified, likely BBB crossing | EVs *did not accumulate in the brain over a 24 h* period in healthy mice; accumulation in the prefrontal cortex in a model of schizophrenia | Tsivion-Visbord et al. Transl. Psychiatry 10(1):305 doi:10.1038 (2020). |
| **18** | RNA-loaded EVs from cultured mammalian cells | intranasal | Not specified. olfactory and trigeminal nerve pathways. | Not detailed. The rapid detection in brain indicates extraneuronal pathway; transcytosis across blood brain barrier | Microglia; at 4 hrs labelled RNA cargo is detectable in the brain. | Chivero et al. Front Cell Dev Bio. 8:573 doi:10.3389 (2020). |
| **19** | drug-loaded artificial multilamellar vesicles | intranasal | Not specified. Hypothesis: nasal mucosa, likely olfactory and trigeminal pathways. | BBB crossing | Brain accumulation confirmed, by functional assays | Touitou et al. Int. J. Pharm. 580:119243 (2020). |
| **20** | enzyme-loaded mammalian EVs | intranasal | Transport along the olfactory nerve cells is indicated | BBB crossing | Predominantly cerebral frontal cortex, central sulcus, and cerebellum | US patent application pub 2020029763 1 A1 |
| **21** | EVs from cultured mammalian cells | intranasal | Primary location in the olfactory bulb | Extraneuronal pathway indicated; able to cross BBB | Intranasal delivery accumulates in microglia | US 10,799,457 B2 |
| **22** | EVs from human stems cells | intranasal | Not specified, likely olfactory and trigeminal nerve pathways. | Not specified, likely BBB crossing | Cortical damage region (traumatic brain injury model). | US 10,857,187 B2 |
| **23** | EVs from human stems cells | intranasal | Not specified, likely olfactory and trigeminal nerve pathways. | BBB crossing | Cortical damage region (traumatic brain injury model) | Moss et al. Neurochem Int. 150:105 173 (2021). |

(continued)

| # | Type of nanoparticle | Route of administration to the brain | Entry site as reported | Travel route | Pathways (brain regions targeted/ reached) | Reference |
|---|---|---|---|---|---|---|
| **24** | EVs from stimulated mammalian cells | intranasal | Olfactory and trigeminal nerve pathways | mechanism unclear. Hypothesis: nasal mucosa through paracellular or transcellular route; BBB crossing | Rostral portion of the brain and the cervical spinal cord | Pusic et al. PloS one 16(8):e0 255778 (2021). |
| **25** | nanoparticle-loaded natural grapefruit EVs | intravenous | Systemic circulatory system | BBB crossing | Experimental brain tumor uptake | Niu et al. Nano Lett. 21(3):1484-1492 (2021). |
| **26** | enzyme-loaded mammalian EVs | intranasal | olfactory and trigeminal tracts; blood via the nasal epithelium | BBB crossing | extensive signal in the prefrontal cortex, corpus callosum, and hippocampus | Hayes et al. Neurotox. Res. 39(5):1 418-1429 (2021). |
| **27** | EVs from GM mammalian cells | intranasal | Transport along the olfactory nerve cells is indicated | BBB crossing | Inflamed brain tissues via LFA1/ICAM1 interactions | Zhao et al. Cells 11(12) :1933 (2022). |
| **28** | magnetic nanoparticle-loaded EVs from mammalian cells | intranasal (multiple administrations) | Intranasal perfusion along olfactory and trigeminal neural pathways | BBB crossing | In the brain 1 day post-administration | Kutchy et al. Front. Pharmacol. 13, 819516:1-9 (2022). |
| **29** | drug-loaded artificial bilosomes (bile-salt-based nanovesicles) | intranasal (daily administrations over 21 days | Not specified | BBB crossing | Only functional assays showing improvement in a mouse model of Alzheimer's Disease | Elsheikh et al. Pharmaceuti cs vol. 14(3):576 (2022). |

It is shown and described herein that MEVs have properties, including biodistribution, that are distinct from other EVs. The pathways by which the MEVs enter the brain and their ultimate destination differ from those observed for other delivery vehicles. IN administration allows the penetration of MEVs into the brain. As discussed and demonstrated herein, the primary route of penetration of the brain and biodistribution upon IN administration of MEVs is via the *olfactory nerve* (ON), the *mitral/tufted neurons,* and the *lateral olfactory tract* (LOT) in contrast to biodistribution of prior art EVs and nanoparticles via the *trigeminal nerve.* The MEVs are internalized by the *olfactory sensory neurons* (OSNs) and travel intracellularly from the olfactory epithelium to the olfactory bulb, through the cribriform plate in the skull. This is in contrast to routes of other delivery vehicles and prior art that indicate or from which it can be inferred that prior art vehicles traffic from the olfactory epithelium to the brain by paracellular transport or transcellular transport. As shown and described herein, intranasally administered MEVs travel from one brain region to another via *axonal transport,* and more specifically, along the *axons of the mitral/tufted neurons* throughput the LOT circuit. As shown and described herein, MEVs pass through the synaptic space, in at least at three stages: (1) synapses between the olfactory sensory neurons (OSNs) and mitral/tufted neurons inside the glomeruli in the olfactory bulb; (2) synapses between mitral/tufted axons and neurons resident in brain regions primary colonized by the *lateral olfactory tract* (LOT), which includes the anterior olfactory nucleus, the tenia tecta, the piriform cortex, the amygdala, the entorhinal cortex; and (3) synapses between the axons afferent from those primary regions of the olfactory tract and the neurons in other brain regions, such as the cortex, the hippocampus, and the

hypothalamus connected to the olfactory tract.

As shown in the examples, the brain regions in which the MEVs are found after IN administration perfectly match: (1) the regions that are directly colonized by the mitral and tufted neurons in the LOT (the anterior olfactory nucleus, the tenia tecta, the piriform cortex, the amygdala, the entorhinal cortex); and (2) the regions in the brain that are secondarily connected with the primary target regions, such as the cortex, the hippocampus, the hypothalamus. It is these loci in the brain that can be targeted with therapeutic and diagnostic agents selected for treatment and/or detection of diseases, disorders, and conditions of the brain and the CNS, or diseases, disorders, and conditions that involve the brain and/or CNS.

## 5. Intranasal administration

With increasing knowledge of the pathways and functions inside the brain, and as the need for new therapeutics increases, there is a demand for new and more potent CNS drugs. One area is neurodegenerative disorders, where, not only is the pathogenesis not understood, but also drug molecules are not able to reach the target tissue in the brain at an appropriate concentration level. IN administration can provide a way for delivery of therapeutically effective drug concentrations in the brain parenchyma. The absorption of the drugs from the nasal cavity and how they are transported via extracellular (intercellular) or intracellular (transcellular) pathways is under study. Intracellular transport involves a first step of endocytosis into the olfactory sensory neurons. After the neuronal uptake, the molecules move away along the axons to the synapse where they are exocytosed (Figure 28) and transported further into the brain throughout various synapses.

However, there are limitations for IN delivery of naked drugs (unprotected, non-encapsulated, not loaded inside a nanoparticle, a vesicle or other delivery vehicle). IN delivery of naked drugs is limited to potent drugs delivered in small volumes (25-200 $\mu$L in humans), with active mucociliary clearance, short retention time, enzymatic degradation by nasal cytochrome P450/peptidases/proteases (pseudo first pass effect), low permeability for hydrophilic drugs, the need for absorption enhancers, low nasal epithelial pH, inter individual variability, low CNS delivery for proteins, and nasal secretion.

A few products have been approved for IN administration; a few more are in development; all of them are small molecules. Approved products are IMITREX® (GlaxoSmithKline) approved in 1997; MIGRANAL® (Bausch Health Companies) approved in 1997; ZOMIG® (Amneal Pharmaceuticals) approved in 2003, ONZETRA® X (Currax Pharmaceuticals) approved in 2016; and TOSYMRA® (Upsher-Smith Laboratories) approved in 2019. Although these products are administered by the IN route, they enter the blood through the highly vascularized respiratory epithelium (not the olfactory epithelium) and once in the blood they still need to go through the blood-brain barrier. The challenge of being able to bypass the blood-brain barrier by entering the olfactory epithelium has so far not been achieved, and the full potential of IN delivery is yet to be captured.

The olfactory route, trigeminal route, and vomeronasal route can provide direct access to certain regions of the brain, that will otherwise not be reachable. There is still need for optimization of this route(s) as well as full understanding of dosing and safety following nasal drug administration. IN delivery of therapeutic agents is a future perspective to treat neurological diseases.

Administration through the IN route has been recognized as a route for the administration of medicines to the brain, but it is not well-developed nor has it been effectively exploited. Most naked drugs are unstable in the nasal epithelium, and/or unable to cross the barriers from the olfactory epithelium to the olfactory bulb, and/or unable to efficiently move inside the neurons following the axonal networks to reach intimate and specific regions of the brain. Such endeavor has only been used for small chemical molecules; most (if not all) biological molecules (such as DNA, RNA (mRNA, siRNA), complex proteins (antibodies) cannot be administered via an IN route. Hence there is a need to develop vehicles to effectively exploit this route of delivery. As demonstrated herein, MEVs provide such a vehicle.

The olfactory region of the nose is the only part in the whole body where the CNS is in contact with the peripheral environment due to the presence of olfactory receptors neuronally linked to the olfactory bulb. Olfactory and trigeminal nerve pathways allow active agents to be absorbed in the olfactory region and transferred directly into the brain bypassing the BBB.

The nasal cavity is divided into the respiratory area (closer to the nostrils) and the olfactory area (situated high up in the cavity) (Sahin-Yilmaz et al. (2011) Proc. American Thoracic Society 8:31-39). The nasal epithelium is well vascularized (Sahin-Yilmaz (2011) Proc. American Thoracic Society 8:31-39), and, within the olfactory area, olfactory neurons are exposed providing the transport of naked drug compounds directly into the brain via the olfactory neurons. Absorption of naked molecules takes place at the olfactory and respiratory epithelia (Lochhead et al. (2012) Advanced Drug Delivery Reviews 64: 614-628). The routes of transportation of naked molecules from the nasal olfactory area to the olfactory bulb are transcellular through either the sustentacular cells or the exposed olfactory sensory neurons. The route of transfer of such compounds from the nasal respiratory epithelium to the brain is via the trigeminal nerves (Ying (Dec. 2007 online) Future Neurol.3 no. 1, doi.org/10.2217/14796708.3.1.1; Lochhead et al. (2012) Advanced Drug Delivery

Reviews 64: 614-628). Transport to other brain areas after entry to the brain (e.g., to the mid brain from the olfactory bulb or to the brain stem from the trigeminal nerve) is thought to be mainly by either extracellular convective bulk flow (Lochhead et al. (2012) Advanced Drug Delivery Reviews 64: 614-628) or via perivascular routes (Lochhead et al. (2015) J. Cerebral Blood Flow Metab. 35:371-381, doi.org/10.1038/jcbfm.2014.215).

In general, direct nose-to-brain delivery obtained demonstrated for rats or mice will overestimate direct nose-to-brain transport in humans if differences in the relative surface area are not adequately accounted for. In contrast, the nasal respiratory epithelium line approximately 50% of the nasal cavity in rats and 80-90% in humans. Therefore, it is possible that access to the brain through trigeminal pathway in primates is underestimated from experiments performed in rodents.

## 6. MEVs and delivery to the brain following intranasal administration

As discussed above and herein, the fate of MEVs upon administration via various routes, including intranasal administration, was not known, nor could it have been predicted from the prior art describing trafficking and biodistribution of administration, including IN administration of other delivery vehicles, such as nanoparticles and EVs from other sources. As discussed in more detail in the following section, it is shown and described herein that MEVs, as exemplified by IN administration of *Chlorella* MEVs, are delivered to the brain following IN administration. As discussed above, and shown in the Examples, MEVs are internalized by the dendrites of the *olfactory sensory neurons* (OSN) and subsequently are intracellularly passaged from the olfactory epithelium to the olfactory bulb, through the cribriform plate in the skull. As shown herein, the delivery to the brain of MEVs upon IN administration occurs via the *olfactory nerve* (ON), the *mitral tufted neurons,* and/or the *lateral olfactory tract* (LOT) in the biodistribution and penetration of the brain by MEV. Known EVs are reported to use the trigeminal nerve route and they do not enter the olfactory nerve, and some of the prior art, noted in the table above, that describe nanovectors do not mention the olfactory nerve, the mitral/tufted or the LOT. As noted, there is no suggestion in the prior art of the involvement of *axonal transport* in the movement of any delivery vehicle from one region to another region, inside the brain. Moreover, in the prior art it is systematically suggested that the entry of the particles to the brain and their movement inside the brain is paracellular or extracellular. Thus, those particles would be either reaching the interstitial liquid between brain cells, or the surrounding capillaries in which case they still need to cross the BBB. As shown herein, regions traversed by the MEVs match the regions that are directly colonized by the mitral and tufted neurons (i.e. the olfactory network), and also those regions in the brain cortex that are secondarily connected with the primary target regions.

## 7. Trafficking and biodistribution of MEVs following intranasal (IN) administration

Trafficking and biodistribution studies on the fate of MEVs following IN administration were performed and the distribution pathways and patterns were mapped. In the exemplified studies, MEVs were stained with DiR (DiIC18(7); 1,1'-dioctadecyl-3,3,3',3'-tetramethylindotricarbocyanine iodide), which is a lipophilic, near-infrared fluorescent cyanine dye. The dye is suitable for labeling lipid membranes detected with near-infrared *in vivo* imaging. The two long 18-carbon chains insert into the membrane, resulting in specific and stable vesicle staining with no or minimal dye transfer between vesicles. The studies were conducted in recognized rodent models.

The size of neural cells in mouse brain varies, but the typical nucleus diameter is approx. 5-8 microns. The size of MEVs is about 50-200 nm, with median diameter of 125 nm or 0.125 micron, as measured by Nanoparticle Tracker Analysis (NTA). Images from the positive control slides (Fig. 29) confirm the presence and detectability of the Dir-labelled MEV as well as demonstrate the size-homogeneity of the MEV material.

It is shown herein that from 1h to 8h after IN administration to mice, MEVs can be observed in the following regions of the brain: (1) the *olfactory bulb*, (2) *cortical regions* (*primary somatosensory, primary visual, primary motor, piriform, agranular insular, frontal, retrosplenial granular, temporal association, auditory, entorhinal),* (3) the *amygdaloid nuclei* (*basomedial and basolateral amygdaloid nuclei, amygdala-hippocampal area, amygdala-piriform transition area*), (4) the *arcuate hypothalamic nucleus,* (5) the *mammillary nucleus.* Upon 16h after IN administration, MEVs can be seen in the *thalamus,* the *cortex,* and in different levels of the *hippocampus* (*fimbria* and *dentate gyrus*). The olfactory bulb still contains fluorescent MEVs by 16h after administration, suggesting that MEVs may continue coming into the olfactory bulb/brain from the nasal cavity over a significant period of time.

Thus, after intra-nasal (IN) administration in an *in vivo* model, MEV are found in regions of the brain corresponding to projections of the olfactory bulb (OB) throughout the olfactory network as well as in other regions connected to the olfactory network. Such regions include the *piriform cortex* (which plays an important role in focal epileptogenesis, forms the major part of the primary olfactory cortex and has extensive connections with other parts of the olfactory network), as well as other cortical regions, such as the *temporal association cortex* (responsible for identification and integration of complex stimuli), the *ectorhinal cortex* (under control of OB during working memory performance), and the *auditory cortex* where auditory and olfactory codes are subjected to cross-modal modulation.

The highest density of vesicles was found in (1) the *arcuate nucleus* of the *hypothalamus* which has a central role

in homeostasis, (2) the *amygdala* at the center of behaviors related to panic, anxiety, stress, and addiction, and (3) the *mammillary bodies* which are very important in episodic memory processing within the *Papez circuit.* In accord with the trafficking data, following IN administration, MEVs are transported following the olfactory circuitry up to various brain regions that are connected to brain nuclei strongly involved in behavior, memory, emotions, and perception of the environment. Consequently, MEVs can be used for delivering directly to the brain cargos that are therapeutic tools for a large diversity of brain and CNS diseases, disorders, and conditions, including, for example, those related to epilepsy, food intake, appetite, sexual behavior, stress (PTSD), anxiety, depression, addiction, memory, and others. They also can be used for carrying and delivering detectable cargos for diagnostics and/or theranostics.

The data show that the IN administration of the MEVs leads to a progressive biodistribution of the vesicles in different but specific brain areas. This biodistribution is time-dependent and observed from the rostral to the caudal regions of the brain. One hour after MEV administration, MEVs are only detected in the olfactory nerve layer but not in other more caudal regions. At 2 hours post administration, MEVs reach cortical regions such as the *primary motor cortex,* the *piriform cortex,* the *frontal cortex,* the *agranular insular cortex,* the *primary somatosensory cortex,* the *auditory cortex,* the *retrosplenial granular cortex* and the *temporal association cortex.* MEVs also were found in the *basolateral amygdaloid nucleus,* and the *arcuate hypothalamic nucleus.* No fluorescence was observed in other regions at the same *bregma*; bregma is a unit that measures the distance between a location in the brain and the point of junction between the coronal and the sagittal sutures of the skull.

The *insula,* one of the regions of the brain that is reached rapidly by the MEVs upon IN administration, is a core region that is affected by or involved in many psychiatric and neurological disorders. Many of the anatomical and functional features of the insula are shared across rodents and men, so that rodent models are useful to demonstrate effects and uses for humans. The insula is a hub linking large-scale brain systems. The insular cortex is a true anatomical integration hub with heavy connectivity to an extensive network of cortical and subcortical brain regions serving sensory, emotional, motivational, and cognitive functions (see Figure 30).The insula receives heavy sensory inputs from all modalities. Direct thalamic and horizontal cortical afferents carry information to the insula from outside the body (auditory, somatosensory, olfactory, gustatory, and visual information) and from inside the body (interoceptive information). Several of these inputs project to topographically organized insular sensory regions, giving rise to the 'visceral insular cortex', the 'gustatory cortex' (the primary taste cortex), and the insular auditory and somatosensory fields. None of these sensory regions processes only its major sensory input; all regions of the insula receive heavy cross-modal afferents and can be considered to be multimodal integration sites. In addition to its sensory afferents, the insula makes reciprocal connections with the limbic system. For instance, the lateral and basolateral amygdala heavily project to the granular and dysgranular regions of the insula, which in turn send dense efferent signals to the basolateral, lateral and central amygdala nuclei. The insula also connects to the lateral part of the bed nucleus of the stria terminalis, the mediodorsal nucleus of the thalamus, the lateral hypothalamus, and parahippocampal regions, including the perirhinal and the lateral entorhinal cortices. The insula reciprocally connects with frontal brain regions such as the anterior cingulate, the orbitofrontal, and the medial prefrontal cortices, which are implicated in cognitive, emotional and executive functions, and projects to parts of the brain implicated in motivation and reward, such as the nucleus accumbens and the caudate putamen. The insular cortex receives strong neuromodulatory input from cholinergic, dopaminergic, serotonergic, and noradrenergic afferents.

At 4 hours post administration, MEVs migrate up to more caudal brain regions reaching the main body of the *amygdala,* the *left and right auditory cortex,* the *temporal association cortex* and the *ectorhinal cortex.*

Within 8h after administration, MEVs are not observed in the *caudate putamen,* the *thalamus,* the *hippocampus,* or the *substantia nigra,* nor in the most caudal sections examined corresponding to structures like the *trigeminal nucleus,* the *inferior colliculus,* the *tegmental nucleus* and the *parabrachial nucleus,* indicating that the MEVs do not reach the most caudal part of the brain within the 8h time window.

It is known that direct nose-to-brain transport of molecules or other structures may occur either via the *olfactory nerve* or via the *trigeminal nerve.* Projections of the olfactory nerve originating in the olfactory bulb (OB) penetrate the cribriform plate and terminate at the apical surface of the olfactory neuroepithelium; located at the roof of the nasal cavity. Filaments of the olfactory nerves are present both in the anterior and posterior parts at the middle turbinate. On the other side, the respiratory mucosa (located on the walls of the nasal cavity) is densely innervated by sensory and parasympathetic trigeminal nerves and is even more extensive than the olfactory nerve. Sensory maxillary branches innervate the deepest nasal segments, including the olfactory cleft. Unlike olfactory sensory neurons, the trigeminal nerve endings do not penetrate the mucosal surface. Access of molecules to the dense network of trigeminal nerve endings is thus limited by their ability to cross the mucosal layer.

The mechanisms involved in the transport of naked molecules from the nasal cavity to the brain are well known; they have been investigated by using [$^{125}$I]-proteins. Transport across the 'barriers' presented by the olfactory and/or respiratory epithelia can occur either by intracellular or extracellular pathways. Intracellular pathways across the olfactory epithelium include (i) endocytosis into olfactory sensory neurons (OSN) and subsequent intraneuronal transport, including intraneuronal axonal transport and also transport between neurons across synapses, to the olfactory bulb and (ii) transcytosis (i.e. transcellular transport) across sustentacular cells to the lamina propria as shown in Figures 28 and 32.

Figure 28 depicts routes of passage through the olfactory epithelium. Four different routes have been described for nose-to-brain drug delivery: (1) *OSN extracellular transport:* the compound enters directly to the CNS along the OSN (or trigeminal nerve which is not shown in the figure) via bulk flow processes through the tissular liquid surrounding the cells, (2) *OSN intracellular transport:* the compound is endocytosed and then shuttled to the CNS by a well-organized pathway of intracellular structures inside the OSN), (3) *Epithelial or supporting cells intracellular transport:* the compound is endocytosed by the supporting epithelial cells and then travel through the intracellular space, (4) *Epithelial or supporting cells extracellular pathway* : the compound has to pass through the tight junctions like zonula occludens (ZO), claudin (CL), and occludin (OC). Abbreviations: supporting cells (SUS); olfactory sensory neurons (OSN); olfactory ensheathing cells (OEC); globose basal cells (GOB); horizontal basal cells (HBC); Bowman's gland (BG); cribriform plate (CP); olfactory bulb (OB).

Figure 32, is a schematic showing the pathways and approximate average distances from the olfactory and respiratory epithelium to CNS targets. Figure reference: Perivascular and Perineural Pathways Involved in Brain Delivery and Distribution of Drugs after Intranasal Administration, Jeffrey J. Lochhead and Thomas P. Davis, Department of Pharmacology, University of Arizona. Pharmaceutics 2019, 11(11):598; doi.org/10.3390/pharmaceutics11110598

OSN have the ability to endocytose certain viruses, such as herpes, poliomyelitis, rhabdoviruses, coronaviruses, and also large molecules, such as horseradish peroxidase (HRP), wheat germ agglutinin-horseradish peroxidase (WGA-HRP), and albumin, from the nasal passages and then transport them intracellularly along the axon in the anterograde direction towards the olfactory bulb. HRP is taken up by OSN to a limited extent via fluid-phase endocytosis whereas WGA-HRP is internalized by OSN more avidly via adsorptive endocytosis. (Gänger et al. (2018) Pharmaceutics 10:116, doi:103390/10030116).

Olfactory sensory neurons (OSN) have several unique attributes: they are the only first order neurons possessing cell bodies located in a distal epithelium and the tips of their dendritic processes, which end as enlarged knobs with several non-motile cilia, extend far into the overlying mucus layer that is directly exposed to the external environment. (Figure 31; Son et al. (2021) BMB Reports 54(6):295-304).

Alternatively, intracellular pathways across the respiratory epithelium can include endocytosis into peripheral trigeminal nerve neurons located near the epithelial surface and subsequent intracellular transport to the brainstem or transcytosis across other cells of the respiratory epithelium to the lamina propria (see Figure 32). As observed in the OSN, intranasal WGA-HRP is internalized and transported intra-neuronally within the trigeminal nerve to the brainstem. Viruses and bacteria also can be transmitted to the CNS along trigeminal nerve components within the nasal passages (Lochhead et al. (2012) Advanced Drug Delivery Reviews 64:614-628).

Based on data shown and described herein, MEVs are transported through the olfactory nerve and the olfactory tract into the olfactory networks in the brain. While not observed because of the duration of the studies, the data do not exclude transportation of the MEVs through the trigeminal nerve and trigeminal network cannot be excluded, as the transport through the trigeminal nerves would take substantially longer before the entry point in the posterior part of the brain (the pons) is reached. The observed pathways, however, are unique to MEVs.

The respective distances from olfactory or respiratory epithelium to CNS have allowed the calculation of transportation time for proteins. Using published data (Buchner et al. (1987) Neuroscience 22:697-707) for fast (130 mm/day) and slow (36 mm/day) *axonal transport* of exogenous proteins in pike olfactory nerves (corrected to 37 °C), indicate that intracellular transport (via axonal transport) within olfactory neurons to the olfactory bulb for MEVs should take 0.74 h-2.7 h, and 3.7h-13h for intracellular transport (via axonal transport) within trigeminal ganglion cells to the brainstem, assuming similar transport rates in OSN and trigeminal cells. As discussed below, based on data herein, there is no evidence indicating involvement of the trigeminal nerve in the brain penetration of MEVs at least through the duration of study post-administration.

The data indicate that MEVs are transported through the axons projecting from mitral/tufted cells in the olfactory bulb. Axons of mitral/tufted cells are fasciculated and form the two *lateral olfactory tracts* (LOT), one on each side of the brain. They extend multiple collaterals that project to various areas of the *olfactory cortex,* including the *anterior olfactory nucleus,* the *olfactory tubercle,* the *piriform cortex,* the *lateral entorhinal cortex,* the *cortical amygdala,* among other (see, Figures 28, 31, and 33-35).

Figure 31 presents a schematic diagram of the brain neuronal pathway from the olfactory sensory neurons (OSN) through the olfactory bulb (OB) to the mitral and tufted neurons, to the olfactory tract (OT).

Figure 33 presents a cortical projection of mitral and tufted cells. Ventrolateral view of the brain is schematically shown (reproduced from Construction of functional neuronal circuitry in the olfactory bulb, November 2014. Seminars in Cell and Developmental Biology 35, DOI:10.1016/j.semcdb.2014.07.012, Takeshi Imai, Kyushu University).

Figure 34 shows possible pathways following IN administration. Based on the data herein, for the MEVs, after IN administration, the pathway is from the nose to the olfactory epithelium and then to the olfactory neurons, then the MEVs are transported by axonal transport to the olfactory bulb, then by mitral and tufted neurons to the primary olfactory regions that process the olfactory signal (Figure reproduced from Selvaraj et al. (2018) Artificial Cells, Nanomedicine, and Biotechnology An International Journal 46:2088-2095, doi.org/10.1080/21691401.2017.1420073).

Figure 35 (reproduced from "What-when-how in Depth tutorials and information, Olfaction and Taste, Sensory system, part 1" (what-when-how.com)) shows schematics of the pathways following IN administration after reaching the OB as described for the Figure. The olfactive pathway used by MEVs after IN administration is as follows. The olfactory bulb (OB) is the first region of the CNS where sensory signals from olfactory sensory neurons (OSNs) are processed. Axons of the OSN travel in olfactory nerves and spread over the surface OB, forming an olfactory nerve layer. Located near the surface of the OB is the glomerular layer. Each glomerulus contains clusters of nerve terminals from OSN, dendrites of the tufted cells, mitral cells, and y-aminobutyric acid (GABA)-ergic interneurons, called the periglomerular cells. The terminals of first order OSN form synapses with the dendrites of the tufted, mitral, and peri-glomerular neurons. The projections of the axons of the mitral and tufted cells are shown schematically in the Figure. Olfactory tracts (LOT), located on the ventral (inferior) surface of the frontal lobe, arise from the OB. The largest bundle of axons from the mitral and tufted cells exit from the OB in the LOT, and they project to the primary olfactory cortex, piriform cortex, amygdala, and entorhinal cortex. The entorhinal and piriform cortices, hippocampus, and amygdala are in the temporal lobe; the hippocampus lies in the medial temporal lobe. The neurons in the piriform cortex, amygdala, and entorhinal cortex project to the prefrontal cortex. Note that the olfactory projection system differs from other sensory systems in that the projection pathway can reach the prefrontal cortex without having to make a synapse in the thalamus first, which is typical of other sensory systems. Neurons in the entorhinal cortex project to the hippocampus (a major limbic structure) via a fiber bundle called the perforant fiber pathway. Therefore, olfactory inputs play an important role in modulating hippocampal functions in a manner like that for the amygdala. Although olfactory projections can reach the prefrontal cortex without making a synapse in the thalamus, there are direct tertiary inputs from the piriform cortex to the mediodorsal thalamic nucleus, which projects to wide areas of the frontal lobe, including the prefrontal cortex. Some fibers from the mitral and tufted cells exit the LOT via the medial olfactory tract. These axons project ipsilaterally to basal limbic forebrain structures, such as the substantia innominata, medial septal nucleus, and bed nucleus of the stria terminalis. Other fibers in the medial olfactory stria arise from the contralateral anterior olfactory nucleus. This nucleus, located in the posterior part of each OB, receives sensory signals from mitral and tufted cells and relays them to the contralateral OB via the anterior commissure.

**MEVs trafficking and regions of the brain that can be targeted or affected by delivery of MEV cargo.**

The *amygdala* has been implicated in aspects of emotional processing. The direct connection of the *olfactory bulb* with some subregions of the *amygdala* indicates a particular role in olfactory processing; the amygdala is involved in the generation of rapid responses to olfactory stimuli (including fight/flight) particularly in approach/avoid contexts, in olfactory-related reward processing, including learning and memory of approach/avoid responses (Noto et al. (2021) Front Syst Neurosci 15:752320, doi: 10.3389/fnsys.2021.752320). The amygdala is one of the regions targeted by psychedelics, including psilocin, to elicit the biological response. The amygdala also plays a role in posttraumatic stress disorder (PTSD) (Badura-Brack et al. (2018) Psychiatry Research: Neuroimaging 271:135-141; Badura-Brack et al. (2018) Biological Psychology 132:228-232). These findings indicate that MEVs can be used to transport drugs and other agents for treatment of diseases, disorders, and conditions involving such responses.

*Mammillary bodies* have unique connectivity to the *anterior olfactory nucleus* (Zhou et al. (2019) eLife 8:e47177, doi.org/10.7554/eLife.47177). *Mammillary bodies* have a central role in the *Papez circuit* involving *amygdala* and *thalamus* and play an active role in how recognitional memory is processed.

The presence of the MEVs in the *arcuate hypothalamic nuclei* is consistent with the fact that ghrelin containing neurons in the olfactory bulb send collateralized projections to this brain area (Russo et al.(2018) Algorithms 11:134, doi.org/10.3390/a11090134). Ghrelin is involved in eating behaviors and the *arcuate nucleus* is a major integration center for peripheral satiety signals and feeding behavior.

The data show that by 16h after IN administration of the MEVs, they are in the *corpus callosum,* the *dorsal fornix,* the *dorsal hippocampal commissure* and the *fimbria of the hippocampus* which is connected to the *fornix.* All these regions correspond to bundle of nerves forming white matter.

The olfactory bulb projects directly to a number of primary cortical brain structures, projections from each of these structures to the rest of the brain constitute a widespread olfactory network. Each of such primary brain structures of the olfactory network, which includes the *anterior olfactory nucleus,* the *olfactory tubercle,* and the *frontal and temporal piriform cortices,* subsequently form and connect to dissociable whole-brain networks. Such networks are characterized by unique functional connectivity profiles for each subregion, leading to higher profile, large-scale processing pathways of the olfactory system (Zhou et al. (2019) Nucleic Acids Research 47(Issue W1):W234-W241, doi.org/10.1093/nar/gkz240).

The data herein show effective IN delivery of MEVs, via the olfactory nerve and throughout the *lateral olfactory tracts* (LOT), to a number of brain regions. Upon IN administration, MEVs enter the brain via the *olfactory nerve.* MEVs are internalized by the dendrites of the *olfactory sensory neurons* (OSN) in the olfactory epithelium (at the roof of the nasal cavity) and then be transported to the *olfactory bulb* (OB), intracellularly, through the body of the OSN. The olfactory nerve starts at the nasal olfactory epithelium and ends at the olfactory bulb (OB). As noted, based on the data herein, there is no

evidence indicating involvement of the trigeminal nerve in the brain penetration of MEVs, in the times post-administration that have been evaluated.

Inside the olfactory bulb (OB) are the *glomeruli* where the incoming axons from the OSN synapse with dendrites of *mitral neurons* and of *tufted neurons.* The mitral/tufted neurons are the principal neurons in the OB. LOTs are composed of the long axons of mitral and tufted neurons that travel from the OB to the various anterior - posterior brain regions directly involved in the olfactory system, which is composed of the: *anterior olfactory nucleus, olfactory tubercle, tenia tecta, piriform cortex, amygdala,* and *entorhinal cortex.* Lateral ramifications of the main long axons of the mitral/tufted neurons enter and colonize each of the brain regions (*anterior olfactory nucleus, olfactory tubercle, tenia tecta, piriform cortex, amygdala,* and *entorhinal cortex).* Inside those regions, the mitral /tufted axons are connected (via synapses) with neurons from other regions (having a more secondary olfactory role), including the *frontal cortex,* the *hypothalamus,* and the *hippocampus.*

Data show that MEVs administered by IN reach all and each of the brain regions reached by the olfactory nerve and the lateral olfactory tracts (LOT) throughout the brain. MEVs arriving to the *glomeruli* from the OSN axons enter the *mitral neurons* and *tufted neurons* and travel following a clear pathway with a clear kinetics throughout the *lateral olfactory tract* (LOT) in both hemispheres: ventral, lateral and dorsal regions; external and internal regions; along the antero-posterior axis. The brain regions reached by the MEVs within 1 and 16 hours after IN administration are the anterior olfactory nucleus, the olfactory tubercle, the tenia tecta, the piriform cortex, the amygdala, the entorhinal cortex, the primary motor cortex, the frontal cortex, the agranular insular cortex, the primary somatosensory cortex, the auditory cortex, the retrosplenial granular cortex, the temporal association cortex, the basolateral amygdaloid nucleus, the arcuate hypothalamic nucleus, the corpus callosum, the internal capsule, the thalamus, and the hippocampus (fimbria, dentata gyrus). The biodistribution pattern of the MEVs, thus, perfectly matches the pathways and connections of the olfactory nerve, the mitral/tufted axons, and the LOT (*lateral olfactory tract*) throughout the entire brain.

The images in the figures (see, *e.g.,* Figures 36) show that, in the different regions they reach, MEVs are disposed in clusters rather than as individual spots, indicating that they are released in the extracellular space surrounding the axon terminals that transported them to those regions. Most of the brain areas where MEVs have been localized correspond to the olfactory bulb circuitry (see, *e.g.*, Imai (2014) Seminars in Cell & Developmental Biology 35: 180-188; Igarashi et al. (2012) Journal of Neuroscience 32:7970-7985; doi.org/10.1523/JNEUROSCI.0154-12.2012; and Nagayama et al. (23 September 2010) Front. Neural Circuits, 23; doi.org/10.3389/fncir.2010.00120).

Most brain regions reached by the MEVs are consistent with the conclusion of direct axonal connections from the olfactory bulb, such as (1) projections of ghrelin neurons from mitral cells into the *arcuate hypothalamic nucleus* (Russo et al. (2018) Experimental Brain Research 236:2223-2229); (2) the direct modulation of *entorhinal cortex* activity by the olfactory bulb (Salimi et al. (2021) The Journal of Physiological Sciences 71:Article number 21); (3) the reciprocal connections of the *agranular insular cortex* with the olfactory bulb (Gehrlach et al.(2020) eLife 9:e55585). The presence of the MEVs in other cortical regions is not due to direct axonal projections from the olfactory bulb but rather indirect projections from areas like the *amygdala* or the *mammillary bodies* (direct connections between *amygdala* and *motor cortex* (Grezes *et al.,* 2014), connections between the *retrosplenial granular cortex* and the *RGC* (Buckwalter et al. (2008) Experimental Brain Research 186:47-57), connections to the *somatosensory cortex* (Macdonald et al. (1998) J. Neurophysiol.79: 474-477), direct connections between *mammillary bodies* and *RGC* (Groen et al.(2003) J. Comparative Neurology 463: 249-263, doi.org/10.1002/cne.10757). On the other side, the presence of MEV in the *auditory cortex* and in the *primary visual cortex* appears to be due to diffusion from proximal *entorhinal cortex* or *RCG,* respectively, as no direct neuronal connections have been reported from any of the areas of distribution.

### 8. Primary and secondary circuitry of the olfactory system and regions reached by the MEVs upon IN administration

In this section, brain regions reached by the MEVs upon IN administration are indicated in bold and with an asterisk*. The **olfactory sensory neurons*** (OSN) are primary sensory neurons located within the olfactory epithelium in the upper nasal cavity. Axons of the OSN leave the olfactory epithelium and synapse in the **olfactory bulb*** (OB). These axons enter the anterior cranial fossa through the cribriform plate of the ethmoid bone. Neurons in the OB called mitral cells are secondary sensory neurons of the olfactory system. Their axons leave the OB and enter the **olfactory tract*** (two lateral olfactory tracts, or LOTs), which is not a peripheral nerve but part of the central nervous system.

MEVs reach cortical regions such as the **primary motor cortex*,** the **piriform cortex*,** the **frontal cortex*,** the **agranular insular cortex*,** the **primary somatosensory cortex*,** the **auditory cortex*,** the **retrosplenial granular cortex*,** the **temporal association cortex*,** the **basolateral amygdaloid nucleus*,** and the **arcuate hypothalamic nucleus*.**

Some fibers of the LOT turn laterally and project themselves to the **olfactory cortex*** (all gray matter areas that receive output from the OB). The main areas of the olfactory cortex are the **piriform cortex*** (the most posterior part of the orbitofrontal cortex), the **cortical amygdala*** (this is the superficial part of the amygdala). The olfactory cortex projects to

the **hypothalamus*.** The hypothalamus is described as using olfactory information to affect feeding, reproductive activity, and autonomic reflexes triggered by olfactory signals.

The olfactory cortex (and the gustatory cortex) project to the **orbital prefrontal cortex*** (the inferior surface of the frontal lobe), where information from both sensory modalities can be combined for the sensation of flavor. The limbic system contains the limbic lobe and other cortical regions that have connections with the limbic lobe. This group of structures is associated with learning, memory, emotion, and motivation. The limbic lobe is composed by the cingulate gyrus, the parahippocampal gyrus, and the hippocampus.

The **cingulate gyrus*** is the cortex adjacent to the **corpus callosum*.** The anterior half of the parahippocampal gyrus is called **entorhinal cortex*,** which is one of the four components of the hippocampus. The entorhinal cortex is part of both the parahippocampal gyrus and the **hippocampus*.**

There are four components of the hippocampus: the entorhinal cortex and the three components of the rolled-in part of the hippocampus - the dentate gyrus, the Ammon's horn, and the subiculum. The entorhinal axons synapse on the granule cells in the dentate gyrus, the granule cell axons synapse on the pyramidal cells of Ammon's horn, which synapse on the pyramidal cells of the subiculum. The hippocampus is necessary for storing recent memories of facts and events. It receives processed information from all sensory cortices which projects to the inferior temporal cortex, which in turn sends axons to the entorhinal cortex. Through these connections, the hippocampus is informed about sensory processing in all cortical areas.

A circle of connections from the hippocampus to the **mammillary body*,** to **anterior thalamus***, to cingulate cortex, and back to the hippocampus through the cingulum and parahippocampal gyrus is known as the Papez circuit. Axons of cells in the hippocampus form a layer of white matter known as the **fimbria*.** The fornix is the major projection from the **hippocampus*,** it dives into the **hypothalamus*** and terminates in the **mammillary body*.** The **mammillary body*** projects to the anterior nucleus of the **thalamus***, which at its turn sends axons to the cingulate gyrus. Axons from the cingulate gyrus go to the parahippocampal gyrus and the **entorhinal cortex*,** completing the circuit back to **hippocampus*.**

The observed nose-to-brain passage of the MEVs via the olfactory neurons in the olfactory epithelium (as opposed to via the trigeminal nerve) is advantageous for various reasons that include, for example: the turnover of the mucus in the epithelium of the upper nose cavity (where the olfactory epithelium is located) occurs in days, which allows and supports consistent drug absorption over time; while in the lower nose cavity (where the respiratory epithelium containing the terminals of the trigeminal nerve is located) turnover occurs in minutes.

The kinetic analysis of distribution from 1h to 8h, shows an increasing time-dependent density of MEV in all the regions analyzed from rostral to caudal parts of the brain (Figures 37-46). Kinetic studies show a strong directionality in the migration of the MEV inside the brain. There is clear movement of the MEVs from the site of entrance in the anterior or frontal part of the brain (the olfactory bulb) to the more posterior or distal parts, all along the axons of the mitral/tufted neurons, the regions they colonize and the secondary regions in their neural network.

It is known that neuron organelles and mitochondria, as well as endogenous intracellular vesicles (intracellular to the neurons) travel via a well-developed and structured mechanism of *axonal transport.* The mechanism and underlying structures for *axonal transport* also are used by viruses, such as, for example, herpes viruses, SARS-CoV-2, and others, when they infect the nerves, such as, for example, the olfactory nerve, to move to internal brain structures and expand the infection. Infecting viruses and intracellular neuronal vesicles travelling via *axon transport* can pass over the synaptic junctions and move from one neuron to another neuron.

As shown and described herein, MEVs travel intracellularly, via *axonal transport;* and can cross-over synapses, at least over (i) the synapses between the OSN and the mitral/tufted neurons, (ii) the synapses between the mitral/tufted neurons and the local neurons in the various brain regions colonized by the LOT, and (iii) the synapses between the neurons in the brain regions colonized by the LOT and neurons from the frontal cortex, the hippocampus, and the hypothalamus. Alternative ways of passage from the olfactory epithelium to the brain could have been paracellular transport or transcellular transport; but these kinds of transport are not compatible with the observed data.

In summary, upon IN administration, MEVs are internalized by the dendrites of the *olfactory sensory neurons* (OSN) and move intracellularly (inside the olfactory sensory neurons (OSN)) from the olfactory epithelium to the olfactory bulb, through the cribriform plate in the skull. The *olfactory nerve* (ON), the *mitral/tufted neurons,* and the *lateral olfactory tract* (LOT) are the main actors involved in the biodistribution and penetration of the brain by the MEVs. MEVs travel from one brain region to another via *axonal transport,* and more specifically, along the *axons of the mitral/tufted neurons* throughput the LOT circuit.

There is a strong correlation between the brain regions primarily reached by the MEVs upon IN administration, and the regions that are the target sites for some psychoactive drugs, such as psychedelics, such as psilocybin and/or psilocybin derivatives, and other drugs for treating the CNS. MEVs, as described herein, can be used for delivery of cargos, such as psilocybin derivatives, to the target sites in the brain where biological activity as an antidepressant or other activity is elicited.

**9. Delivery of MEVs via IN administration to the brain - exemplary bioactive cargo and uses thereof**

The cargo-loaded MEV's can be used and administered intranasally for treatment of brain disorders, psychiatric disorders, dementia, brain cancers, brain trauma, brain injury, and for treatment of any disease, disorder, or condition in which the brain is involved. The MEVs also can be used for diagnosis to detect brain disorders, such as cancers, and to monitor treatments. In general the MEVs are used for treatment of humans, but they can be used for treatment of animals, as they often suffer from similar or the same brain-associated disorders as humans. For brain delivery, cargo generally comprises molecules (small molecule drugs, proteins, nucleic acids, and others) that affect or treat or detect diseases, disorders, and conditions of the brain and/or CNS.

Diseases, disorders, and conditions involving the brain, include, for example, human psychiatric disorders, and also animal disorders, including anxiety disorders (panic disorders, social anxiety, phobia-related disorders, and generalized anxiety disorders); attention deficit hyperactivity disorders (inattentive type, hyperactive-impulsive type, combination type); autism spectrum disorders (Asperger's syndrome, Childhood Disintegrative Disorder (CDD), Kanner's syndrome, Pervasive Developmental Disorder (PDD-NOS)); bipolar disorders (Bipolar I disorder, Bipolar II disorder, bipolar with mixed features, bipolar with seasonal pattern major depression, Cyclothymia, rapid cycling bipolar); eating disorders (anorexia nervosa, bulimia nervosa, muscle dysmorphia, binge eating disorder, other specified eating or feeding disorder (OSFED), compulsive over eating, Prader Willi syndrome, Diabulimia, orthorexia nervosa, selective eating, drunkorexia, pregorexia); personality disorders (antisocial personality disorder, borderline personality disorder, histrionic personality disorder, narcissistic personality disorder, avoidant personality disorder, dependent personality disorder, obsessive-compulsive disorder (OCD)); posttraumatic stress disorders (PTSD)( acute stress disorder, uncomplicated PTSD, complex PTSD, comorbid PTSD); schizophrenia (catatonic schizophrenia, disorganized schizophrenia, paranoid schizophrenia, residual schizophrenia, and undifferentiated schizophrenia); other such psychiatric and brain-related conditions.

The MEVs provide for the delivery, via intranasal administration, and the other routes, as described herein, and expression of active small molecules, proteins, anti-sense oligonucleotide (ASOs), and RNA inside the brain. The MEV cargo for delivery to the brain includes psychoactive agents, enzymes, growth factors, detectable products, such as psilocybin derivatives, harmine, temozolomide, rivastigmine rhodamine (small molecules); catalase, GFP, nerve growth factors (NGFs), TrkA (tropomyosin kinase A), neurotrophic factors (NT-3, NT-4, BDNF (brain derived neurotrophic factor, CNTF (ciliary neurotrophic factor), EPO, IGF-1, bFGF (basic fibroblast growth factor), hGH, or luciferase (proteins); GABAB1A receptor, eGFP or Luciferase (mRNA); GABAB1A receptor (siRNA), PTEN (siRNA; SEQ ID NOs. 136-138); miR-17 (miRNA; SEQ ID NOs: 139-141); or MALAT1 (SEQ ID NO: 142; lncRNA) into the brain by the IN route. MALAT1 (SEQ ID NO: 142), a metastasis associated lung adenocarcinoma transcript 1, is a long noncoding RNA (lnc RNA). In the brain, over-expression of long noncoding RNA MALAT1 ameliorates traumatic brain injury induced brain edema by inhibiting AQP4 and the NF-κB/IL-6 pathway (see, e.g., Zhang et al., first published 19 June 2019, J. Cellular Biochemistry 120(10):17584-17592, doi.org/10.1002/jcb.29025).

Cargo for delivery in the MEVs can comprise any of the following single molecules, or in combination, for treatment of any of the above conditions, for example: 5-hydroxytryptamine-1A (5-HT1A) and 5-hydroxytryptamine-3 (5-HT3) receptor agonists, for example, Azapirones, Methylphenidate, Dexmethylphenidate, Ondansetron (Zofran®, GlaxoSmithKline); Acetylcholinesterase inhibitors, for example, Donepezil, Galantamine, Rivastigmine; Alpha-1-receptor antagonists, for example, Prazosin; Anticonvulsants, for example, Gabapentin, Pregabalin, Topiramate (sold under the trademark Topamax®, Ortho-McNeil Pharmaceutical), Carbamazepine, Eslicarbazepine, Levetiracetam, Licarbazepine, Oxcarbazepine, Valproic acid and derivatives, Lamotrigine; Anti-inflammatory drugs and supplements of various mechanisms, including non-steroidal anti-inflammatory drugs (NSAIDs) and selective and non-selective COX-2 inhibitors, for example, celecoxib, acetylsalicylic acid (Aspirin), ibuprofen, and naproxen, Infliximab, Omega-3 polyunsaturated fatty acids, Pioglitazone, TNF-alpha inhibitors, N-Acetyl-Cysteine, Dexamethasone, Minocycline; Antipsychotics, for example, Aripiprazole, Asenapine, Cariprazine, Chlorpromazine, Clozapine, Haloperidol, Lumateperone tosylate (Caplyta®, Intra-Cellular Therapies), Olanzapine, Paliperidone, Quetiapine, Risperidone, Ziprasidone; Beta blockers, for example, Azapirones, Propranolol; Drugs that modulate the cholinergic system, for example, Biperiden, scopolamine; Corticotropin Releasing Factor (CRF) antagonists; Drugs that modulate the GABAergic system, for example, Benzodiazepine, Brexanolone, Sage-217; Glucocorticoid receptor agonists, for example, Hydrocortisone; Drugs involved in glutamatergic modulation, for example, AGN-241751, AV-101, AVP-786, AVP-923, AXS-05, D-cycloserine, Dextromethorphan, Rapastinel; Glycine, and glycine reuptake inhibitors, for example, Sarcosine; Drugs that modulate the hypothalamic-pituitary-adrenal (HPA) axis, for example, Fludrocortisone, Metyrapone, Mifepristone, and probiotics; Drugs that modulate the Kynurenine Pathway (KP); Drugs that modulate the limbic and paralimbic brain areas, for example, Cannabidiol (CBD); Drugs that modulate the melatonergic system, for example, Agomelatine; Fatty acids, peptides, nucleic acids and other precursor molecules, for example, alpha-omega fatty acids, Coenzyme Q10, Myo-inositol, Methylfolate, S-adenosylmethionine, Cysteamine, and Oxytocin; Monoamine oxidase inhibitors (MAOIs), for example, Isocarboxazid (Marplan®, Validus Pharmaceuticals, Inc.), Phenelzine (Nardil®, Warner-Lambert Pharmaceutical Company), Selegiline (Emsam®, Somerset Pharmaceuticals, Inc.), and Tranylcypromine (Parnate®, GlaxoSmithKline ); Mood stabilizers, for example,

lithium salts, Valproate, Ebselen, and Divalproex; Multimodal antidepressants, for example Vilazodone and Vortioxetine; N-Nitrosodimethylamine (NDMA)-receptor antagonists, for example, Amantadine, Arketamine, Ketamine, Memantine, Riluzole, Esketamine; Neurokinin-1 (NK1) receptor antagonists; Neuropeptide Y (NPY) receptor agonists; Drugs with Neurotrophic effects, Cilostazol, Sildenafil, and Vildagliptin; Norepinephrine-dopamine reuptake inhibitors (NDRIs), Bupropion (Wellbutrin®, GlaxoSmithKline; Zyban®, Glaxo Group Ltd.; Aplenzin®, Biovail Laboratories International); Drugs that act on the opiate system, for example, ALKS-5461, AZD2327, BTRX-246040 (LY2940094), Buprenorphine, JNJ-67953964, Nalmefene, and Naltrexone; Protein Kinase C inhibitors or anti-estrogen drugs, for example, Endoxifen, Tamoxifen, and Verapamil; Psychedelic drugs, for example, 3,4-methylenedioxymethamphetamine (MDMA), Ayahuasca, Lysergic acid diethylamide (LSD), psilocybin and/or derivatives thereof; Selective serotonin reuptake inhibitors (SSRIs), for example, Citalopram (Celexa®, Forest Laboratories Inc.), Escitalopram (Lexapro®, Forest Laboratories Inc.), Fluvoxamine, Paroxetine (Paxil®; GlaxoSmithKline; Pexeva®, Synthon Pharmaceuticals Inc.), and Sertraline (Zoloft®, Pfizer); Selective norepinephrine transporter inhibitors, for example, Atomoxetine; Serotonin-norepinephrine reuptake inhibitors (SNRIs), for example, Desvenlafaxine (Pristiq®, Wyeth Corporation), Duloxetine (Cymbalta®, Eli Lily and Company), Levomilnacipran (Fetzima®, Forest Laboratories Inc.), and Venlafaxine; Stimulants, including adenosine receptor antagonists, and alpha-2-adrenergic receptor agonists, for example, Caffeine, Clonidine, Guanfacine, Extended Release amphetamines XR-OS, Dextroamphetamine sulfate, Lisdexamfetamine, Methamphetamine, Mixed amphetamine salts, Racemic amphetamine sulfate, and Triple-bead mixed amphetamine salts; Substance P-antagonists, for example, MK0869; Tricyclic serotonin-norepinephrine reuptake inhibitors, for example, Amitriptyline (Elavil®, Merck and Co.), Amoxapine, Buspirone (Buspar, Bristol Meyers Squibb), Clomipramine, Desipramine (Norpramin®, Lakeside Laboratories Inc.; Aventis, Aventis Corporation), Doxepin, Imipramine (Tofranil®, Mallinckrodt LLC; Ciba Geigy, Maria Sistro), Maprotiline, Nortriptyline (Pamelor, American Home Products of Delaware), Protriptyline, and Trimipramine; and Vasopressin 1B (V1B) receptor antagonists, for example, SSR149415.

Thus, the MEVs, via IN administration can deliver therapeutic and diagnostic molecules that include peptides, small peptides, proteins, polypeptides, nucleic acids, and small molecules. Such cargo includes, but is not limited to, anti-cancer therapeutics and diagnostics, psychoactive molecules, such as psilocybin/psilocin, other molecules including harmine, temozolomide, rivastigmine, rhodamine (small molecules); catalase, GFP, nerve growth factors (NGFs), TrkA (tropomyosin kinase A), neurotrophic factors (NT-3, NT-4, BDNF (brain derived neurotrophic factor, CNTF (ciliary neurotrophic factor), EPO, IGF-1, bFGF (basic fibroblast growth factor), hGH, and luciferase (proteins), to the brain by the IN route using any kind of EVs. The expression, in the brain, of an active mRNA, siRNA, or miRNA for GABAB1A receptor, eGFP and Luciferase (mRNA); nerve growth factors (NGFs), TrkA (tropomyosin kinase A), neurotrophic factors (NT-3, NT-4, BDNF (brain derived neurotrophic factor, CNTF (ciliary neurotrophic factor), EPO, IGF-1, bFGF (basic fibroblast growth factor), hGH, GABAB1A receptor (siRNA), PTEN (siRNA); miR-17 (miRNA); and MALAT1 (lncRNA) carried by the EVs as a payload. MALAT1 can be used to treat brain injury and trauma; it also is over-expressed in some conditions, such as cancer, and can be a target for inhibition.

Of interest is delivery of psychoactive drugs for treatment of psychiatric and other such disorders. Exemplary bioactive molecules and drugs that can be loaded in MEVs for such treatments are set forth in the following table.

| **Bioactive molecule or Drug** | **Drug Classification or Mechanism of Action** | **Condition(s)** |
|---|---|---|
| 3,4-methylenedioxymethamphetamine (MDMA; "ecstasy") | Psychedelic; induces short-term release of serotonin, dopamine, norepinephrine | Anxiety, Bipolar Disorder, Schizophrenia |
| 5-hydroxytryptamine (5-$HT_1$) receptor agonists | Agonizes 5-hydroxytryptamine receptors or serotonin receptors | Schizophrenia |
| AGN-241751 | Glutamatergic modulation | Depression |
| Agomclatinc | Modulates melatonergic system | Depression |
| ALKS-5461 | Opiate system | Depression |
| Amantadine | NDMA-receptor antagonist; | Autism |
| Amitriptyline (Elavil®, Merck and Co.) | Tricyclic: serotonin-norepinephrine reuptake inhibitors | Anxiety, Depression, Eating Disorders |
| Amoxapine | Tricyclic: serotonin-norepinephrine reuptake inhibitors | Anxiety, Depression |

(continued)

| Bioactive molecule or Drug | Drug Classification or Mechanism of Action | Condition(s) |
|---|---|---|
| Amphetamines: (Extended Release amphetamines XR-OS, EROS; Dextroamphetamine sulfate; Lisdexamfetamine; Methamphetamine; Mixed amphetamine salts; Racemic amphetamine sulfate; Triple-bead mixed amphetamine salts) | Stimulant; Increases dopamine and norepinephrine; inhibits monoamine oxidase; interacts with ACH, 5-HT, opioid, and glutamate pathways | ADHD |
| Aripiprazole | Anti-psychotic | Autism, Bipolar Disorder, Borderline |
| | | Personality Disorder, Schizophrenia |
| Arketamine | NDMA-receptor antagonist; Glutamatergic modulation | Bipolar Disorder |
| Asenapine | Anti-psychotic | Bipolar Disorder, Schizophrenia |
| Aspirin | Anti-inflammatory; COX-1 and COX-2 inhibitor | Bipolar Disorder, Depression |
| Atomoxetine | Selectively inhibits NE transporter; increases extracellular synaptic levels of NE and dopamine in prefrontal cortex | ADHD |
| AV-101 | Glutamatergic modulation | Depression |
| AVP-786 | Glutamatergic modulation | Depression |
| AVP-923 | Glutamatergic modulation | Depression |
| AXS-05 | Glutamatergic modulation | Depression |
| Ayahuasca | Psychedelic effects-Multiple mechanisms | Depression |
| Azapirones | 5-$HT_{1A}$ agonists | Anxiety |
| AZD2327 | Opiate system | Depression |
| Benzodiazepines | GABAergic system | Anxiety, Bipolar Disorder |
| Beta blockers/Azapirones | Beta blockers | Anxiety |
| Biperiden | Cholinergic system | Depression |
| Brexanolone | GABAergic system | Depression |
| BTRX-246040 (former LY2940094) | Opiate system | Depression |
| Buprenorphine | Opiate system | Depression |
| Bupropion (Wellbutrin®, Zyban®, Aplenzin®) | norepinephrine-dopamine reuptake inhibitors (NDRIs) | Anxiety, Depression |
| Buspirone (Buspar, Bristol Myers Squibb) | Tricyclic: serotonin-norepinephrine reuptake inhibitors | Depression, Eating disorders |
| Caffeine | Stimulant, antagonism of adenosine receptors | OCD |
| Cannabidiol (CBD) | Acts directly on limbic and paralimbic brain areas | Anxiety |
| Carbamazepine | Anticonvulsants | Bipolar Disorder |

(continued)

| Bioactive molecule or Drug | Drug Classification or Mechanism of Action | Condition(s) |
|---|---|---|
| Cariprazine | Anti-psychotic | Bipolar Disorder, Schizophrenia |
| Celecoxib | Anti-inflammatory; COX-2 inhibitor | Bipolar Disorder |
| Chlorpromazine | Anti-psychotic | Bipolar Disorder, Schizophrenia |
| Cilostazol | Neurotrophic effects | Depression |
| Citalopram (Celexa®) | Selective Serotonin Reuptake Inhibitor (SSRI) | Anxiety, Depression, OCD, PTSD |
| Clomipramine | Tricyclic: serotonin-norepinephrine reuptake inhibitors | OCD, Autism |
| Clonidine | Stimulant; α-2 receptor agonists | Autism |
| Clonidine-XR | Stimulates post-synaptic α-2-adrenergic receptors | ADHD |
| Clozapine | Anti-psychotic | Bipolar Disorder, Schizophrenia, Autism |
| Coenzyme Q10 | Miscellaneous; Anti-oxidant, aides in metabolism | Bipolar Disorder |
| Corticotropin Releasing Factor (CRF) antagonists | Corticotropin Releasing Factor (CRF) antagonists | Anxiety |
| Cysteamine | Miscellaneous; Precursor Molecule; Neurotrophic effects | Depression |
| D-cycloserine | Glutamatergic modulation | Depression |
| Desipramine (Norpramin®) | Tricyclic: serotonin-norepinephrine reuptake inhibitors | Anxiety, Depression |
| Desipramine (Norpramin®, Aventis) | Tricyclic: serotonin-norepinephrine reuptake inhibitors | Eating disorders |
| Desvenlafaxine (Pristiq®) | serotonin-norepinephrine reuptake inhibitors (SNRIs) | Anxiety, Depression |
| Dexamethasone | anti-inflammatory, immuosuppressive; antiemetic: | PTSD |
| Dextromethorphan | Glutamatergic modulation | Depression |
| Divalproex | Mood stabilizers | Borderline Personality Disorder |
| Donepezil | Acetylcholinesterase inhibitors | Autism |
| Doxepin | Tricyclic: serotonin-norepinephrine reuptake inhibitors | Anxiety, Depression |
| Duloxetine (Cymbalta®) | serotonin-norepinephrine reuptake inhibitors (SNRIs) | Anxiety, Depression, Borderline Personality Disorder |
| Ebselen | Mood stabilizer; Lithium mimetic | Bipolar Disorder |
| Endoxifen | Protein Kinase C inhibitor, anti-estrogen | Bipolar Disorder |

(continued)

| Bioactive molecule or Drug | Drug Classification or Mechanism of Action | Condition(s) |
|---|---|---|
| Escitalopram (Lexapro®) | Selective Serotonin Reuptake Inhibitor (SSRI) | Anxiety, Depression, OCD, PTSD, Autism |
| Esketamine | NDMA-receptor antagonist; Glutamatergic modulation; mu-opioid | Bipolar Disorder, Depression |
| Eslicarbazepine | Anticonvulsants | Bipolar Disorder |
| Fludrocortisone | Hypothalamic-Pituitary-Adrenal (HPA) axis | Depression |
| Fluoxetine (Prozac®) | Selective serotonin | Anxiety, Depression, Borderline Personality Disorder, OCD, PTSD, Autism, Eating Disorders |
| Fluvoxamine | Selective Serotonin Reuptake Inhibitor (SSRI) | Depression, OCD, Autism |
| Gabapentin | Alpha-delta calcium channel anticonvulsants | Anxiety, PTSD |
| Galantamine | Acetylcholinesterase inhibitors | Autism |
| Glycine | Miscellaneous; Amino acid, precursor molecule | OCD |
| Guanfacine-XR | Stimulates post-synaptic alpha 2A-adrenergic receptors | ADHD |
| Guanfacine | Stimulant; Alpha-2 receptor agonists | Autism |
| Haloperidol | Anti-psychotic | Bipolar Disorder, Borderline Personality Disorder |
| Hydrocortisone | Glucocorticoid receptor agonists | PTSD |
| ibuprofen | Anti-inflammatory; COX-1 and COX-2 inhibitor | Bipolar Disorder, Depression |
| Imipramine (Tofranil®, Ciba Geigy) | Tricyclic: serotonin-norepinephrine reuptake inhibitors | Anxiety, Depression, Eating disorders |
| Infliximab | Anti-inflammatory; TNF alpha inhibitor; Immune/inflammatory system | Depression |
| Isocarboxazid (Marplan®) | monoamine oxidase inhibitors (MAOIs) | Anxiety, Depression |
| JNJ-67953964 | Opiate system | Depression |
| Ketamine | NDMA-receptor antagonist; Glutamatergic modulation | Anxiety, Depression, OCD, PTSD, Bipolar Disorder |
| Lamotrigine | Anticonvulsants; modulates neuronal glutamate levels | Bipolar Disorder, OCD, Autism |
| Levetriracetam | Anticonvulsants | Autism |

(continued)

| Bioactive molecule or Drug | Drug Classification or Mechanism of Action | Condition(s) |
|---|---|---|
| Levomilnacipran (Fetzima®) | serotonin-norepinephrine reuptake inhibitors (SNRIs) | Anxiety, Depression |
| Licarbazepine | Anticonvulsants | Bipolar Disorder |
| Lithium salts | Mood stabilizer | Bipolar Disorder |
| lumateperone tosylate (Caplyta®) | Anti-psychotic | Bipolar Disorder, Schizophrenia |
| Lysergic acid diethylamide (LSD) | Psychedelic effects-Multiple mechanisms | Anxiety, Depression |
| Memantine | NDMA-receptor antagonist; Glutamatergic modulation | Bipolar Disorder, Autism, OCD |
| Methylfolate supplementation | Miscellaneous; Precursor molecule, nutritional supplement | Depression |
| Methylphenidate: Dexmethylphenidate | 5-HT1A receptor agonist; psycho-stimulant | ADHD, Autism |
| Metyrapone | Hypothalamic-Pituitary-Adrenal (HPA) axis | Depression |
| Mifepristone | Hypothalamic-Pituitary-Adrenal (HPA) axis | Depression |
| Minocycline | Anti-inflammatory; Immune Modulator, Alpha-2 receptor antagonist: Activation of immune system | Bipolar Disorder, Depression, OCD, Anxiety |
| MK-0869 | Substance P antagonism | Depression |
| Myo-inositol | Miscellaneous; Precursor molecule, can stimulate glucose uptake and decrease blood sugar levels. | OCD |
| N-Acetvl-Cvsteine | Anti-inflammatory, anti-oxidant with glutamate-modulating properties | Bipolar Disorder, OCD |
| Nalmefene | Opiate system | Borderline Personality Disorder |
| Naltrexone | Opiate system | Depression, Autism, Borderline Personality Disorder |
| Naproxen | Anti-inflammatory; COX-1 and COX-2 inhibitor | Bipolar Disorder, Depression |
| Neurokinin-1 ($NK_1$) antagonists | Neurokinin-1 NK(sub.1) antagonists | Anxiety, Depression |
| Neuropeptide-Y (NPY) receptor agonists | Neuropeptide Y (NPY) receptor agonists | Anxiety, Bipolar Disorder, Depression, PTSD |
| Nortriptyline (Pamelor) | Tricyclic: serotonin-norepinephrine reuptake inhibitors | Anxiety, Autism, Depression |

(continued)

| Bioactive molecule or Drug | Drug Classification or Mechanism of Action | Condition(s) |
|---|---|---|
| Olanzapine | Anti-psychotic | Bipolar Disorder, Borderline Personality Disorder, PTSD, Schizophrenia |
| Omega-3 Polyunsaturated Fatty Acids; Fish oil | Anti-inflammatory | Borderline Personality Disorder, Depression, PTSD |
| Ondansetron (Zofran®) | 5-HT3 receptor agonist; Anti-emetic | OCD, Eating Disorders |
| Oxcarbazepine | Anticonvulsants | Bipolar Disorder |
| Oxytocin | Miscellaneous; Prosocial Neuropeptide | Anxiety, Autism, Depression, PTSD, Schizophrenia |
| Paliperidone | Anti-psychotic | Bipolar Disorder, Borderline Personality Disorder, Schizophrenia |
| Paroxetine (Paxil®, Pexeva®) | Selective Serotonin Reuptake Inhibitor (SSRI) | Anxiety, Autism, Depression, OCD, PTSD |
| Phenelzine (Nardil®) | monoamine oxidase inhibitors (MAOIs) | Anxiety, Depression |
| Pioglitazone | Anti-inflammatory | Bipolar Disorder |
| Prazosin | Alphal-Adrenergic receptor antagonist | PTSD |
| Pregabalin | Anticonvulsant | Anxiety |
| Probiotics | Hypothalamic-Pituitary-Adrenal (HPA) axis | Anxiety, Bipolar Disorder, Depression |
| Propanolol | Blocks beta 1&2 adrenergic receptors | PTSD |
| Protriptyline | Tricyclic: serotonin-norepinephrine reuptake inhibitors | Anxiety, Depression |
| Psilocybin | Psychedelic effects-Multiple mechanisms | Anxiety, Depression |
| Quetiapine | Anti-psychotic | Anxiety, Bipolar Disorder, Borderline Personality Disorder |
| Rapastinel | Glutamatergic modulation | Depression |
| Riluzole | NDMA-receptor antagonist; Glutamatergic modulation | Anxiety, OCD |
| Risperidone | Anti-psychotic | Anxiety, Autism, Bipolar Disorder, OCD, PTSD, Schizophrenia |
| Rivastigmine | Acetylcholinesterase inhibitors | Autism |

(continued)

| Bioactive molecule or Drug | Drug Classification or Mechanism of Action | Condition(s) |
|---|---|---|
| S-adenosylmethionine (SAMe) | Miscellaneous; Precursor molecule: | Depression |
| SAGE-217 | GABAergic system | Depression |
| Sarcosine | Glycine reuptake inhibitor | OCD |
| Scopolamine | Cholinergic system | Depression |
| Selegiline (Emsam®) | monoamine oxidase inhibitors (MAOIs) | Anxiety, Depression |
| Sertraline (Zoloft®) | Selective Serotonin Reuptake Inhibitor (SSRI); Atypical Antipsychotic | Anxiety, Autism, Borderline Personality Disorder, OCD, PTSD |
| Sildenafil | Neurotrophic effects | Depression |
| SSR149415 | Vasopressin 1b receptor antagonist | Depression |
| Tamoxifen | Protein Kinase C inhibitor, antiestrogen | Bipolar Disorder |
| TNF-$\alpha$ inhibitor | Anti-inflammatory | Bipolar Disorder |
| Topiramate (Topamax®) | Anticonvulsant; modulates neuronal glutamate levels | Bipolar disorder, Eating Disorders, OCD, PTSD |
| Tranylcypromine (Parnate®) | monoamine oxidase inhibitors (MAOIs) | Anxiety, Depression |
| Trimipramine, Tetracyclic: Maprotiline | Tricyclic: serotonin-norepinephrine reuptake inhibitors | Anxiety |
| Valproate | Mood stabilizer | Bipolar Disorder |
| Valproic acid and derivatives | Anticonvulsants | Autism, Bipolar Disorder, Borderline Personality Disorder |
| Vasopressin (V1B) antagonists | Vasopressin (V1B) antagonists | Anxiety, Depression |
| Venlafaxine | serotonin-norepinephrine reuptake inhibitors (SNRIs) | Anxiety, Autism, Depression, OCD, PTSD |
| Verapamil | Protein Kinase C inhibitor, antiestrogen | Bipolar Disorder |
| Vilazodone | Multimodal antidepressant effect | Depression |
| Vildagliptin | Neurotrophic effects | Depression |
| Vortioxetine | Multimodal antidepressant effect | Depression |
| Ziprasidone | Anti-psychotic | Bipolar Disorder, Schizophrenia |

Thus provided herein are methods for using MEVs for the delivery of drugs, via intranasal administration, for the treatment of psychiatric conditions, brain diseases, disorders, and conditions of the CNS. As an example, the table below summarizes: (1) the brain regions reported to be sites of action for the biological activity of psilocybin (or derivatives) and other psychedelics; and (2) regions that are also targeted by MEV when administered by the IN route.

**Table Neuroanatomy of brain areas targeted by hallucinogens and MEVs**

| Brain area | MEV density | Psychedelic drug target | References |
|---|---|---|---|
| Olfactory bulb | +++ | Existence of binding sites with high affinity for [125I]LSD that correspond to 5-HT5A receptors and that are concentrated in the olfactory bulb, neocortex, and medial habenula | (Grailhe et al. (1999) Neuron 22(3):581-591; Increased Exploratory Activity and Altered Response to LSD in Mice Lacking the 5-HT5A Receptor) |
| Primary motor cortex | + | A single dose of psilocybin triggers dendritic remodeling, enhances excitatory neurotransmission in the medial prefrontal cortex | (Shao, Liao et al. (2021) Neuron 109(16):2535-2544; Psilocybin induces rapid and persistent growth of dendritic spines in frontal cortex in vivo) |
| Piriform cortex | ++ | SD and DOI act as highly potent partial agonists at cortical 5HT21 receptors in the piriform cortical cortex | (Marek and Aghajanian (1996) Eur. J. Pharmacol. 305(1-3):95-100; Alpha 1B-adrenoceptor-mediated excitation of piriform cortical interneurons) |
| Frontal cortex | + | We found that a single dose of psilocybin led to approximately 10% increases in spine size and density, driven by an elevated spine formation rate. The structural remodeling occurred quickly within 24 h and was persistent 1 month later.<br>Studies show that a single administration of a psychedelic produces rapid changes in plasticity mechanisms on a molecular, neuronal, synaptic, and dendritic level. | (Shao et al. (2021) Neuron 109(16):2535-2544; Psilocybin induces rapid and persistent growth of dendritic spines in frontal cortex *in vivo*)<br>(de Vos et al. (2021) Frontiers in Psychiatry 12:724606; Psychedelics and Neuroplasticity: A Systematic Review Unraveling the Biological Underpinnings of Psychedelics) |
| Agranular insular cortex | + | Psilocybin and ketamine produced acutely comparable elevations in c-Fos expression in numerous brain regions, including anterior cingulate cortex, locus coeruleus, primary visual cortex, central and basolateral amygdala, and claustrum | (Davoudian et al. (2022) bioRxviv 14(3):468-480, doi: doi.org/10.1101/2022.03.18.484437; Shared and distinct brain regions targeted for immediate early gene expression by ketamine and psilocybin) |
| Primary somatosensory cortex | + | The region containing the highest density of 5-$HT_{2A}$ binding sites was the neocortex, where the autoradiographic signal displayed a banded pattern corresponding predominantly to pyramidal neurons distributed according to the cytoarchitectural organization of different cortical areas | (Lopez-Gimenez and Gonzalez-Maeso (2018) Curr. Top Behav. Neurosci. 36:45-73; Hallucinogens and Serotonin 5-HT2A Receptor-Mediated Signaling Pathways) |

(continued)

| Brain area | MEV density | Psychedelic drug target | References |
|---|---|---|---|
| Auditory cortex | + | Psychedelic drugs also have notable effects on the perception of music. This is not surprising, as $5HT_{2A}$ signaling has been shown to alter neuronal responses to auditory stimuli from the cochlear nucleus along the precortical primary auditory sensory pathway through to primary auditory cortex | (Barrett et al. (2018) Intl. Rev. Psychiatry 30(4):350-362; Psychedelics and music: neuroscience and therapeutic implications) |
| | | LSD enhances the emotional response to music. | (Kaelen et al. (2015) Psychopharmacology (Berl) 232(19):3607-14; LSD enhances the emotional response to music) |
| | | Single oral administration of LSD (100µg) increased blood oxygen-level in the prefrontal cortex in response to music, suggesting an exaggerated activity in brain regions related to personal relevance processing | (Preller et al. (2017) Curr. Biol. 27(3):451-457; The Fabric of Meaning and Subjective Effects in LSD-Induced States Depend on Serotonin 2A Receptor Activation) |
| Retrosplenial granular cortex | + | Correlation of psilocybin's effects with gene expression highlighted potential roles of 5-HT2A and GluN2B subunit of NMDA receptors | (Davoudian et al. (2022) bioRxviv 14(3): 468-480, doi.org/10.1101/2022.03.18.484437; Shared and distinct brain regions targeted for immediate early gene expression by ketamine and psilocybin) |
| Temporal association cortex | + | | |
| Ectorhinal cortex | + | | |

(continued)

| Brain area | MEV density | Psychedelic drug target | References |
|---|---|---|---|
| Amygdaloid nucleus | +++ | Acute treatment with psilocybin decreased amygdala reactivity during emotion processing and that this was associated with an increase of positive mood in healthy volunteers | (Kraehenmann et al. (2015) Biol. Psychiatry 78(8):572-581; Psilocybin-Induced Decrease in Amygdala Reactivity Correlates with Enhanced Positive Mood in Healthy Volunteers) |
| | | The administration of LSD reduced reactivity of the left amygdala and the right medial prefrontal cortex relative to placebo during the presentation of fearful faces | (Mucllcr et al. (2017) Transl. Psychiatry 7(4):e1084; Acute effects of LSD on amygdala activity during processing of fearful stimuli in healthy subjects) |
| | | LSD administration produced a five- to eight-fold increase in Fos-like immunoreactivity in medial prefrontal cortex, anterior cingulate cortex, and central nucleus of amygdala. | (Gresch et al. (2002) Neuroscience 114(3):707-713; Lysergic acid diethylamide-induced Fos expression in rat brain: role of serotonin-2A Receptors) |
| | | Thus, we show psilocybin's effect as a modulator of major connectivity hubs of the amygdala | (Grimm et al. (2018) Eur. Neuropsychopharmacol. 28(6):691-700; Psilocybin modulates functional connectivity of the amygdala during emotional face discrimination) |
| | | Local infusion of the 5-HT2A receptor antagonist M100907 into the amygdala reversed the effect of systemic administration of DOI on fear expression while local administration of DOI into the amygdala was sufficient to suppress fear expression. | (Pedzich et al. (2022) Neuropsychopharmacology 47:1304-1314; Effects of a psychedelic 5-HT2A receptor agonist on anxiety-related behavior and fear processing in mice) |
| | | The correlation of fear perception and amygdala reactivity may be useful during psychotherapy. | (Dolder et al. (2016) Neuropsychopharmacology 41(11):2638-2646; LSD Acutely Impairs Fear Recognition and Enhances Emotional Empathy and Sociality) |
| Arcuate hypothalamic nucleus | +++ | | |
| Mammillary nucleus | +++ | rat brain exhibited a high level of specific binding of [125I]LSD in the tenia tecta and mammillary nuclei | (Watts et al. (1994) Neurochem. Intl. 24(6):565-574; Autoradiographic comparison of [125I]LSD-labeled 5-HT2A receptor distribution in rat and guinea pig brain) |
| | | High concentrations of [$^{125}$I]LSD sites were observed in layer IV of the cerebral cortex, caudate-putamen, claustrum, olfactory tubercle, nucleus accumbens, ependyma, mammillary nucleus and inferior olive | (Nakada et al. (1984) Neuroscience Letters 49(1-2):13-18; Localization and characterization by quantitative autoradiography of [125I]LSD binding sites in rat brain) |

Because the target regions for psychedelics and the target regions for MEV upon IN administration coincide, it is described and shown herein that this can be exploited for treatment of psychiatric and other diseases, disorders, and conditions that are or can be treated by administration of a psychedelic. Disorders responsive to treatment by psychedelics include, but are not limited to, PTSD and depression, among others. Advantages that are associated with the administration of therapeutical psychedelics by means of MEV-mediated nose-to-brain delivery, include, but are not limited to,

the following:

1) Direct entry to the brain avoids passage through the blood stream, which leads to systemic exposure of the entire body, degradation, metabolism, and clearance by filtering organs (liver, kidneys).

2) Protection of psilocybin and/or derivatives thereof against oxidative inactivation in the body (kidney, intestinal mucosa).

3) Avoidance of undesirable effects in the gastrointestinal tract (the gastrointestinal tract is among the tissues with the highest level of expression of the 5-HT2 receptor) and other organs (liver, kidneys, cardiovascular) expressing levels of the receptor that are significantly higher than those in the brain.

4) Better control of the timing involved in the process (from the administration of the drug to the end of the treatment), and lower variability from patient to patient due to the fluctuations introduced by the oral administration and the passage through the gastrointestinal tract, and blood.

As shown herein, MEV-mediated delivery provides entry to the brain via the olfactory nerve, resulting in the distribution of a psychoactive drug, such as psilocybin and/or derivatives thereof, via the mitral/tufted axons throughout the olfactory tract, and, accordingly, directly to the relevant brain regions. Thus provided herein are methods for using MEVs for the delivery of psychiatric drugs such as psychedelics and others, via intranasal administration, for the treatment of psychiatric conditions.

**I. MEV-MEDIATED INTRACELLULAR SIGNALING**

Other *in vivo* therapeutic targets, in general, not limited to the brain, include receptors that are involved in a disease, disorder, or condition. These include cell surface receptors, and internalized receptors, in which the MEVs deliver agonists, antagonists, ligands, or other modulators of activity. Targets of interest include, for example, modulation of toll-like receptors (TLRs) and other receptors, including receptors, such as the TLRs, that are internalized. Such receptors are internalized into vesicles and can interact with ligands delivered by MEVs. This is exemplified in the Examples in which the ligand flagellin is delivered into cells via MEVs; flagellin activates TLR-5, which in turn activates inflammatory cytokines.

Flagellin is a conserved protein, a component of the bacterial flagellum in mobile bacteria. The immune systems in plants and in animals that assure the defense against bacterial infections are set to recognize flagellin, or subrogate peptides thereof, and to react against the invading bacteria. Thus, the presence of bacteria, their flagella, or of fragments thereof triggers a signaling pathway, that leads to a reaction of the host to the putative infection agent. In plants, flagellin is recognized by the FLS2 receptor (Flagellin Sensing 2 receptor). For instance, the presence of flagellin, or of surrogate peptides of it, is detected by the leaf epithelium that surrounds the stomata (the respiratory pores on the surface of the leaves). Open stomata are entry doors into the leaf parenchyma for infective agents such as flagellin-bearing bacteria. When the presence of flagellin is detected in the leaf epithelium, the plant triggers an immune response, which includes the immediate closing of the stomata in order to physically prevent the entry of bacteria thereby. The signaling pathway, that starts by the detection of flagellin (or of subrogate peptides) and ends with the closing of the stomata, is triggered by the binding of flagellin (or of subrogate peptides) to the FLS2 receptor. The transmembrane protein receptor, FLS2, is the very first component in the signaling pathway. In *Arabidopsis thaliana,* a species commonly used as a plant model, the FLS2 receptor is found in the plasma membrane and in the membranes of endosomal vesicles inside the plant cell (see, Beck et al., (2012) The Plant cell 24(10):4205-4219 and Otegui et al. (2008) Traffic (Copenhagen, Denmark) 9(10):1589-1598). The flagellin binding domains are oriented either to the "extracellular space" (for the FLS2 molecules located in the plasma membrane); or to the "intra-endosomal space" (for the FLS2 molecules located in intracellular endosomal vesicles). The FLS2 domains in charge of the triggering of the signaling pathway are in both cases oriented towards the cytoplasmic side of the membranes. Thus, it can be expected that a signaling pathway and the subsequent biological immune response triggered by the binding of flagellin (or of a subrogate peptide) to FLS2 are indistinguishable whether the triggering FLS2 is located in the plasma membrane and detects flagellin in the cell surface or located in an endosomal vesicle and detects flagellin from within the same endosomal vesicle. Although, it is straightforward for FLS2 located in the plasma membrane to identify and bind to flagellin (or to subrogate peptides), it is less likely to expect that FLS2 will find flagellin (or a subrogate peptide) inside the endosomal vesicle where the intracellular FLS2 form is located. Experiments shown in the working Examples herein, demonstrate that MEVs that have been exoloaded with flp22, a subrogate peptide of flagellin, can trigger the expected biological immune reaction, i.e. the immediate closing of the stomata, presumably by the delivery of the bioactive peptide straight into the endosomal vesicles where FLS2 is located. As flp22-loaded MEVs are treated with proteases to destroy any trace of external flp22 that may remain from the exoloading reaction; the immune reaction cannot be explained by free flp22 binding to the FLS2 in the plasma membrane. Moreover, there is no evidence that MEVs (or

other EVs) spontaneously release their cargo in the extracellular or culture media which might trigger a signaling pathway from the FLS2 in the plant's membrane. Thus, the observed data are difficult to explain unless it is hypothesized that flp22-loaded MEVs are endocytosed by the epithelial plant cells, and that once inside the cells the endocytic vesicles carrying the loaded-MEVs may find and fuse with endosomal vesicles that carry the FLS2 protein.

The flg22 peptide, is a 22-amino acid synthetic peptide, which mimics a conserved N-terminal region of bacterial flagellin. It has been shown that flp22 binds to plasma membrane FLS2 triggering a defense response of the cell against bacteria. However, it has never been shown in the prior art that free flp22 can be delivered directly to endosomal vesicles or that from there it can trigger the same biological response that it triggers from the cell surface. Effective delivery of the peptide inside the endosomal vesicles, mediated by peptide loaded-MEVs, allows such phenomenon to take place and to be observed.

Multiple clathrin-independent mechanisms of endocytosis have been described and characterized; some of which play a role in membrane bulk flow and cell membrane turnover. It is also known that FLS2 can be internalized into vesicles, not only upon ligand binding, but also in a ligand-independent manner for constitutive recycling (see, Beck et al., (2012) The Plant cell 24(10):4205-4219). FLS2 signaling also occurs from endosomes after internalization (see, Otegui et al. (2008) Traffic (Copenhagen, Denmark) 9(10):1589-1598). Plant endosomes are highly dynamic organelles; hence a rendezvous of ligand-carrying MEVs and receptor-carrying vesicles is possible inside the cell. This intracellular interaction results in fusion of MEVs and endosomes, providing ligand-mediated activation of FLS2 and production of the effector signaling. Similar mechanisms of endosome turnover are present in yeast and mammals, where endosomes are also known to recycle vacuolar cargo receptors back to the trans Golgi network and sort membrane proteins for degradation in the vacuole/lysosome.

These results indicate that when a MEV loaded with a ligand (agonist or antagonist) for either an internalized receptor, or for an endosomal intracellular receptor, such as the FLS2 receptor, the TLRs or alike, are internalized, as in the Examples, they may happen to reach and deliver their payload inside the vesicles where the target receptors are located and thus elicit a biological response from "within the cell". Thus, MEVs can deliver agonists/antagonists or ligands to internalized or to intracellular receptors. No other alternative technologies have shown to be able of such an endeavor.

A significant relevance of the above observations comes from the fact that the plant FLS2 protein is a member of the large family of the so-called TLRs (or Toll-like Receptors), which play a pivotal role in innate immunity and in the modulation and triggering of immune responses in humans, mammals, and other animals.

There are similarities between how plants and animals perceive pathogens. Plants have highly sensitive perception systems that stimulate defense responses, and innate immunity in animals is based on the recognition of similar pathogen-associated molecular patterns. FLS2 in *Arabidopsis,* which is essential for flagellin perception, shares homology with the Toll-like receptor (TLR) family, which is a first line of defense against infectious diseases in animals (see, Gómez-Gómez et al. (2002) Trends in plant science 7(6):251-256).

TLR5 is responsible for flagellin perception in mammals, but other receptors from this family, including TLR3, TLR7, TLR8, TLR9, TLR13, (naturally located in cellular endocytic vesicles) also can be targets for intracellular MEV-mediated ligand delivery. This phenomenon paves the way for treatment of a number of therapeutic indications using specific ligand-loaded MEVs to trigger the immunomodulatory signaling pathways related to the different TLR family members; such as for infections (like sepsis and other), for inflammation (like rheumatoid arthritis and other), as vaccine adjuvants (including cancer vaccines) as well as for allergy treatment.

The following tables summarizes TLR members, their ligands and agonists/antagonists, and downstream effects:

| TLR Member | Ligand(s)/Agonists | Actions/Downstream effects |
|---|---|---|
| TLR1 | Triacyl lipopeptides (Pam3CSK4) | Myeloid differentiation primary response (MyD88)* and MAL/-TIRAP**-dependent activation of inflammatory cytokines, NF-KB and AP-1; Induces phagocytosis of ligand, or bound molecules |
| TLR2 | Zymosan, Porin, Modulin, Lipoproteins, Lipotechoic acid, Diacyl lipopeptides, Atypical LPS, Peptidoglycan, Triacyl lipopeptides | MyD88 and MAL/TIRAP-dependent activation of inflammatory cytokines, NF-KB and AP-1; Induces phagocytosis of ligand, or bound molecules |
| TLR3 | dsRNA | Trif dependent induction of Inflammatory cytokines and Type I IFN; Initiates adaptive immunity; Activates local cytokine burst and local inflammatory response; Production of TNF-alpha, IL-12, MCP1. |

(continued)

| TLR Member | Ligand(s)/Agonists | Actions/Downstream effects |
|---|---|---|
| TLR4 | Mannans, Taxol, LPS | Trif and TRAM dependent induction of inflammatory cytokines and Type -I IFN, TIRAP and MyD88 induction of inflammatory cytokines; Promotes addiction; Neuroinflammatory response |
| TLR5 | bacterial flagellin, profilin, HMGB1, Small molecule agonists (CBLB502) | MyD88-dependent induction of NF-kB and inflammatory cytokines, activation of innate and adaptive immunity |
| TLR6 | Zymosan, Porin, Modulin, Lipoproteins, Lipoteichoic acid, Diacyl lipopeptides (Pam2CSK4), Atypical LPS, Peptidoglycan | MyD88 and MAL/TIRAP-dependent activation of inflammatory cytokines, NF-KB and AP-1; Induces phagocytosis of ligand, or bound molecules |
| TLR7 | imidazoquinolinone, loxoribine, ssRNA, bropirimine, resiquimod | MyD88 dependent induction of inflammatory cytokines and Type I IFN |
| TLR8 | ssRNA, small synthetic compounds | MyD88 dependent induction of inflammatory cytokines and Type I IFN |
| TLR9 | CpG DNA | MyD88 dependent induction of inflammatory cytokines and Type I IFN |
| TLR10 | Diacyl and Triacyl lipopeptides | TRIF-dependent activation of IFN-B; TRADD dependent and/or MyD88 and TIRAP-dependent activation of pro-inflammatory cytokines; PI3K/Akt dependent activation of IL-1Ra; Direct inhibition of MyD88 or MAPK--Anti-inflammatory; |
| TLR11 | Profilin-like protein, non-pathogenic bacteria | MyD88 dependent induction of inflammatory cytokines |
| *TIR-containing adaptor protein (Tirap), also called MyD88 adaptor-like protein (MAL)<br>** toll/Interleukin-1 receptor domain containing adaptor protein | | |

| TLR Member | Ligand(s)/Antagonists | Action/Downstream Effects |
|---|---|---|
| TLR1 | Small molecule antagonists (CUT12-9, MMG-11) | Competitive inhibition of signaling cascade;<br>Inhibition heterodimerization of TLR1/2;<br>Induce M1 macrophages to M2 phenotype;<br>Inhibition of immune response;<br>Inhibition of inflammatory response |
| TLR2 | Small Molecule Antagonists(AT1-AT8, CU-CPT22, CU-T12-9, MMG-11, NPT1220-312), Phloretin, Sulfoglycolipids | Competitive inhibition of signaling cascade;<br>Inhibition of heterodimerization of TLR1/2;<br>Induction of M1 macrophages to M2 phenotype;<br>Reduction of immune response;<br>Reduction of inflammatory Response;<br>Reduction of TNF-alpha production, reduced nitric oxide (NO) formation; Reduced TLR2-induced NF-KB transcriptional activity;<br>Reduction of NLRP3 including IL-1B, and IL-18 |
| TLR3 | Small Molecule Antagonists (CU-CPT4a), Monoclonal antibodies (CNTO4685, CNTO5429) | Decreased production of IL-6, IL-8, MIP1-alpha, and TNF-alpha |
| TLR4 | Small Molecule Antagonists (Norbinaltorphimine, T4ICs, T5342126, Simvastatin | Decreased NF-kB activation and cytokine production, reduction of nitric oxide (NO) formation; Decreased addiction |

(continued)

| TLR Member | Ligand(s)/Antagonists | Action/Downstream Effects |
|---|---|---|
| TLR5 | Small Molecule Antagonist (TH1020) | Decreased NF-kB, AP-1 signaling and decreased induction of proinflammatory cytokines; Altered gut microbiome, tumor growth, metabolic syndrome, liver fibrosis |
| TLR6 | Simvastatin | Reduction in cytokine production; Prevention of macrophage activation |
| TLR7 | Chloroquine, hydroxychloroquine, quinacrine | Reduction of IFN-1 and cytokine signaling, reduction of inflammation |
| TLR8 | Small Molecule Antagonist (CU-CPT8m, CU-CPT9a) | Reduced induction of TNF, IL-6, and IL-10 |
| TLR9 | Small Molecule Antagonists (NPT1220-312), chloroquine, hydroxychloroquine, quinacrine; Suppressive or inhibitory oligonucleotides | Blocks release of inflammatory cytokines in monocyte/macrophage cells; decreased immunostimulatory response |
| TLR10 | - | - |

Thus, therapeutic targets for MEV-mediated delivery and expression, include receptors involved in diseases, disorders, or conditions, such that said receptors are cell surface receptors, or, most important, internalized receptors or internal (intracellular endosomal) receptors, like TLRs and others. MEVs may deliver agonists, antagonists, ligands, or other modulators of activity of such receptors. Targets of interest include, for example, modulation of toll-like receptors (TLRs). Such receptors are internalized into endosomal vesicles and can interact with ligands delivered by MEVs. This is exemplified in the Examples in which flagellin, surrogate thereof, and other known ligands for TLRs is delivered into cells via MEVs; activation of TLRs in turn activates inflammatory cytokines.

## J. EXAMPLES

The following examples are included for illustrative purposes only and are not intended to limit the scope of the invention.

### EXAMPLE 1

### Production of *Chlorella* cells and isolation of Microalgae Extracellular Vesicles (MEVs)

#### A. Batch production of the inoculum

*Chlorella vulgaris* of any strain can be used to produce MEVs. Exemplary strains include, but are not limited to, UTEX 265 strain, UTEX 395 strain, UTEX 26 strain, 15 UTEX 30 strain, UTEX 259 strain, UTEX 2219 strain, UTEX 2714 strain UTEX B 1811 (available from the UTEX Culture Collection), the strain designated CCAP 211/19, GEPEA, University of Nantes, France, and any other suitable strain, either transformed or not, can be used to produce the algal cell material. For exemplary purposes the UTEX 265 strain was used.

*Chlorella* was stored on nutrient agar slopes until flask/photobioreactor (PBR) inoculation. For different experiments, different scales of production, between 400 mL (flasks) to 170 L (several PBRs with different total volume) cultures, were used. This description relates to the highest volume of PBR used (170 L, HECTOR PBR ["Hector" photobioreactor designed by the Laboratory of Process Engineering - Environment - Agri-food (GEPEA) / CNRS for the culture of microalgae. Reference: 20160067_0017. Year of production: 2016. Maximum size: 56.43 x 37.66 cm/ 170 L / 300 dpi]).

A 5-Liter PBR was filled with 4 L of sterile BG11 medium (see, *e.g.,* utex.org/products/bg-11-medium for a description of its preparation and see table below (Table 1) for autotrophic growth), and inoculated directly from the stock algal slope on nutrient agar. Then, the *Chlorella* strain was grown as a batch culture in a bubble column using the following culture parameters: temperature of 23°C; medium pH 7.5-8.0; light intensity: 100 $\mu mol \cdot m^{-2} \cdot s^{-1}$; light cycle: continuous. Biomass concentration, specific growth rate and biomass productivity of *Chlorella* were estimated daily. Typically, after 6 days of continuous growth the cultures reached biomass concentrations of approx. 1.2 g/L. The total crop volume of 20 L

was collected for subsequent production scale-up to the HECTOR PBR.

**Table 1. Composition of the BG11 medium**

| | Conc | Ml |
|---|---|---|
| Salt Mix | 100x | 10 |
| Iron* | 1000x | 1 |
| Nitrate* | 100x | 10 |
| Trace metals | 1000x | 1 |
| agar | | 14 |
| $H_2O$ | | up to 1 L |
| pH 7 | | |
| * In case of precipitation, add after sterilization. | | |

**Stock solutions for BG11 medium:**

| Nitrate 100x | g/L | MW | µM |
|---|---|---|---|
| $NaNO_3$ | 150 | 85 | 1764.71 |
| | | | |
| **Iron 1000x** | | | |
| Fe-EDTA | 3 | 344.06 | 8.72 |
| | | | |
| **Salt Mix 100x** | | | |
| $K_2PO_4$ | 4 | 173.17 | 23.10 |
| $MgSO_4 \cdot 7H_2O$ | 7.5 | 246.48 | 30.43 |
| $CaCl_2 \cdot 2H_2O$ | 3.6 | 147.01 | 24.49 |
| $Na_2CO_3$ | 2 | 105.99 | 18.87 |
| add HCl if needed | | | |
| | | | |
| **Trace Metals 1000x** | g/L | MW | µM |
| $H_3BO_3$ | 2.9 | 61.84 | 46.25 |
| $MnCl_2 \cdot 4H_2O$ | 1.8 | 197.91 | 9.15 |
| $ZnSO_4 \cdot 7H_2O$ | 0.2 | 287.56 | 0.77 |
| $Na_2MoO_4 \cdot 2H_2O$ | 0.4 | 241.95 | 1.61 |
| $CuSO_4 \cdot 5H_2O$ | 0.1 | 249.68 | 0.32 |
| $CoCl_2 \cdot 6H_2O$ | 0.3 | 237.93 | 0.14 |

**B. Production scale-up in a semi-industrial Photobioreactor (PBR)**

The *Chlorella* cells were cultured further in a 170-Liter photobioreactor system (HECTOR). The inoculum was added to sterile BG11 medium (see Table 1) to the total volume of 150 L and the cells were grown autotrophically as a semi-batch culture with bubble column mixing. The following culture parameters were used: temperature of 18±4°C; medium pH 8.0±0.05; light intensity: 150-300 µmol·$m^{-2}$·$s^{-1}$; between 150 µmol·m-2·s-1 the three first days of each batch, 250

μmol·m-2·s-1 days four and five and 300 μmol·m-2·s-1 days six and seven before the harvesting as light cycle: continuous, with gradual increase in light intensity (see FIG. 1).

Biomass concentration, growth rate and biomass productivity of *Chlorella* were estimated daily. On the 6th day of cultivation, with a biomass concentration of approx. 1.5 g/L *Chlorella* harvesting was performed.

### C. Production of 3 consecutive batches of *Chlorella*

The *Chlorella* production was performed in 3 semi-batches of 130 L, from which about 80% of the culture volume was aseptically removed for downstream treatment and supplemented with sterile BG11 medium. Following the harvest, the light intensity was lowered to 140 $\mu mol \cdot m^{-2} \cdot s^{-1}$ to avoid excessive photon intake. A seeding line was set up to go from 100 mL of culture to 150 L of culture. Three consecutive batches lasting 6-7 days were carried out with the aim of extracting a vesicle concentrate devoid of microalgae.

### Culture parameters monitoring

### 1. Determination of the protein content

The protein content of cultures was determined by elemental analysis, resorting to Vario el III (Vario EL, Elementar Analyser systeme, GmbH, Hanau, Germany), according to the procedure provided by the manufacturer. The final protein content was calculated by multiplying the percentage of nitrogen given by the elemental analysis by 6.25.

### 2. Estimation of chlorophyll content

Culture samples were centrifuged at 2547 g for 15 min using a Hermle® centrifuge (HERMLE Labortechnik GmbH, Wehingen, Germany). Pigments were extracted from the resulting pellet by bead milling in acetone. The full absorbance spectrum of the extract was obtained with a Genesys™ 10S UV-VIS spectrophotometer (Thermo Fisher Scientific) and iteratively decomposed to the standard pigment spectra in order to obtain the total chlorophyll content.

### 3. Growth estimation

Dry weight was obtained by filtration of culture samples using pre-weighed 0.7 μm GF/C 698 filters (VWR, Pennsylvania, USA) and dried at 120°C until constant mass was obtained using a DBS 60-30 electronic moisture analyzer (KERN & SOHN GmbH, Balingen, Germany). All dry weight samples were washed with demineralized water to remove growth medium salts.

### D. Isolation of Microalgae Extracellular Vesicles: Production of concentrated MEV preparation (Down-Stream Processing: clarification and concentration step)

The culture harvested from the photobioreactor was centrifuged at 2,700 g for 5 minutes at room temperature for cell removal. The supernatant was transferred into fresh bottles and centrifuged again at 2,700 g for 5 minutes at room temperature. The clear MEV-containing solution was then subjected to membrane filtering using a 1.2 μm cut-off cartridge filter. The filtrate was concentrated with the use of a 100 kDa Molecular weight cut-off (MWCO) tangential filtration system. At each isolation step the material was analyzed spectrophotometrically for chlorophyll and particulate matter. Dry weight of the final product was <0.01 g/L and the concentration factor relative to the initial volume of the processed culture was approx. 20. The suspension of MEV thus obtained was stored at -50°C in 1-1.2 L pockets for further purification.

### E. Detailed Protocol for Purification of *Chlorella* Microalgae Extracellular Vesicles

1. Thaw in a cold room at 4°C, overnight, the previously clarified preparation of MEVs, concentrated and stored (1.0 -1.2 L) as described in section D.
2. When the preparation is thawed, harvest the biomass by centrifugation (set the temperature to 4°C): 2 x 10000g for 10 minutes at 4°C.
3. Collect the supernatant (MEVs) and filter by vacuum filter onto 0.65 μm filters to get rid of the remaining cells.
4. The MEVs are concentrated and purified by tangential flow filtration (TFF) using Sartorius VivaFlow systems.

    a. The membrane is washed by running water at ≈100 ml/minute, as described by the manufacturer. After that, the

circuit is washed with cell-free medium (BG-11 medium) at ≈200 ml/minute (pressure reading at 2/2,5 bars).

b. The MEV preparation (supernatant) is run in the circuit at ≈200 ml/minute (pressure reading at 2/2,5 bars). When the residual volume in the circuit plus the reservoir is about 200mL, the TFF is used to diafiltrate and change the medium from BG-11 to PBS using 1L of PBS.

c. When the residual volume in the circuit plus the reservoir is about 200 ml in PBS, slow the flow to ≈100 ml/minute (20 minutes, 1 bar).

d. From 30-60 ml of residual volume, slow the flow to a speed lower than 50 ml/minute and allow the MEVs in PBS to recirculate for 30 minutes in order to recover the particles trapped on the membrane surface.

e. MEVs are then filtered using 0.45 μm filters and purified by ultracentrifugation.

f. The filtered MEVs are loaded on the ultracentrifuge tubes and centrifuged for 1h at 4°C, at 100000g (27400rpm) (acceleration and deceleration at 10 max), for example in a SorVall™ WX ultra 80 TST 28.38. Pellets containing the MEVs are resuspended in 1-2 ml of PBS buffer and filter sterilized using a 0.2 μm filter and the particles are analyzed by *nanoparticle tracking analysis* (NTA; dilute up to 1:1000 before the NTA analysis).

### F. Protocol for Purification of *Chlorella* Microalgae Extracellular Vesicles by Size Exclusion Chromatography (SEC)

When higher purity of MEVs is needed, a last step of purification is added. The MEVs previously concentrated by TFF and purified by ultracentrifugation and formulated in PBS at concentration of $10^{11}$ to $10^{13}$ per mL are seeded in a pre-packed column qEV1 from IZON. The MEVs are eluted with PBS solution. The elution fractions of 0.5 mL are collected. MEVs are recovered in the first fractions as shown in FIG. 2 below.

The concentrations of MEVs in the initial sample and in the fractions collected throughout the elution are evaluated with the ZetaView® engine (Nanoparticle Tracking Analyzer from Particle Metrix) as the quantity of proteins by Bradford assay. The most concentrated fractions (4-5) are pooled and stored at 4°C before use.

## EXAMPLE 2

### MEV characterization

### A. Nanoparticle Tracking Analysis (NTA)

MEVs were analyzed for size and dispersity (size distribution) using a NanoSight NS500 system (Malvern Panalytical Instruments). The instrument was equipped with a 488 nm laser, a high sensitivity sCMOS camera and a syringe pump. The MEV samples were diluted in particle-free PBS (0.02 μm filtered) to obtain a concentration within the recommended measurement range ($1\text{-}10\times10^8$ particles/mL), corresponding to dilutions of from 1/1000 to 1/10000 depending on the initial sample concentration.

For each sample, 5 experiment videos of 60 second duration were analyzed using NTA 3.4 Build 3.4.003 (camera level 15-16) with syringe pump speed 30. A total of 1500 frames were examined per sample, which were captured and analyzed by applying instrument-optimized settings using a suitable detection threshold so that the observed particles are marked with a red cross and that no more than 5 blue crosses are seen. Further settings were set to "automatic" and viscosity to "water".

For the characterization of the MEVs, MEV samples are produced and purified as described in Example 1 as follows: MEV are concentrated by tangential flow filtration (TFF), diafiltration and ultracentrifugation, purified by SEC and sterilized by 0.22 μm filtration. After SEC purification and filtration, MEVs are diluted 10,000 times in PBS (1X) and measured in the ZetaView® analyzer (Particle Metrix GmbH, Ammersee, Germany).

Table 2, below, shows the results of 3 independent measurements.

| SAMPLES (MEVs after SEC) | Concentration (MEVs/mL) | MEVs Median size (nm) | MEVs Zeta potential (mV) |
|---|---|---|---|
| #1 | 2.9Exp12 | 159.9 | -20 |
| #2 | 2.9Exp12 | 163.6 | -22.58 |
| #3 | 2.9Exp12 | 161.7 | -22.3 |
| MEAN | 2.9Exp12 | 161.6 | -21.63 |
| SD | 5.7Exp8 | 2.6 | -2.3 |

### B. Transmission Electron Microscopy (TEM)

To verify the presence of intact MEVs, the preparations were analyzed using transmission electron microscopy (TEM). Fixed (4% formaldehyde, 0.2% glutaraldehyde) MEV samples were allowed to attach to Formvar/carbon-coated grids for 15-20 min, washed again with PBS followed by distilled water and finally stained with 0.4% uranyl acetate/1.8% methyl cellulose and then dried. The preparations were observed using a JEOL-JEM 1230 (JEOL Ltd., Tokyo, Japan) at 80 kV and images were acquired using a Morada digital camera and iTEM software (Olympus, Münster, Germany).

The TEM imaging, presented in the Figure 3, demonstrates that the MEVs are round shaped vesicles sized ~50-250 nm in diameter. MEVs are enveloped in a single lipid bilayer membrane, their lumen had slightly higher electron density, and the thickness of the membrane was estimated as ~5-10 nm, which matches the thickness of a plasma membrane. **Figure 3** shows exemplary images of MEVs obtained using Transmission Electron Microscopy (TEM).

### C. DiR fluorescent labelling

For uptake and internalization studies, and well as for further characterization in vivo, MEVs were labelled with DiR, a lipophilic carbocyanine derivative (1,1'-Dioctadecyl-3,3,3',3'-Tetramethylindotricarbocyanine Iodide; Thermo Fisher Scientific) that has low fluorescence in water, but becomes highly fluorescent upon membrane incorporation, and diffuses laterally within the plasma membrane. Fresh samples of the P40 fraction (prepared as above) were re-suspended in 1 ml of BG11 culture medium. 5 μl of 1 mg/ml DiR solution was added to the samples, following incubation at 37°C for 1 hour. Then, the samples were ultra-centrifuged at 100,000 g for 30 min using a Kontron TST 55.5 rotor at 28,100 rpm. The supernatant was removed, while the pellets were washed twice with 1 ml of PBS and centrifugation at 100,000g for 30 min. Finally, the pellet was re-suspended in 1 ml of PBS. The DiR-labelled MEVs were stored at 4°C and used promptly to ensure highest possible fluorescent intensity. DiR fluorescence of the labelled MEVs was measured using a SpectraMax® fluorescence microplate reader (Molecular Devices, USA) with excitation at 750 nm and emission at 780 nm.

### D. PKH26 fluorescent labelling

For uptake and internalization studies, and well as for further characterization in vivo, MEVs alternatively are labelled with PKH26 (Sigma-Aldrich), a fluorochrome in the red spectrum with peak excitation (551 nm) and emission (567 nm) that may also be excited by a 488 nm laser. Fresh samples of the P40 fraction (prepared as above) are re-suspended in 1 ml of Diluent C from the PKH26 kit. 6 μl of PKH26 dye is added to the samples, followed by continuous mixing for 30 seconds by gentle pipetting. After 5-minute incubation at room temperature, the samples are quenched by adding 2 ml of 10% BSA in 1× PBS. The volume is brought up to 8.5 ml in media and 1.5 ml of 0.971 M sucrose solution is added by pipetting slowly and carefully into the bottom of the tube, making sure not to create turbulence. The PKH26-labelled MEVs remain on top of a sucrose cushion. Then, the samples are ultra-centrifuged at 190,000 g for 2 hours at 2-8°C using a Kontron TST 55.5 rotor. The supernatant is removed, while the pellets are washed with 1× PBS by gentle pipetting and centrifuged again at 100,000g for 30 min. Finally, the pellet is re-suspended in 1 ml of 1× PBS. The PKH26-labelled MEVs are stored at 4°C and filtered with 0.45 μm filter before adding to cells.

### E. Flow cytometry experiments

Flow cytometry analyses were conducted using LSRII flow cytometer with CellQuest Pro software (BD Biosciences, San Jose, CA). Latex beads of 0.3 and 1. 1μm diameters were prepared and used according to the manufacturer's recommendation to define the MEV gate. Since latex beads typically have higher refractive index and thus lower limits of size detection by flow cytometry than MEVs, the thresholds for forward and side scatter were adjusted to avoid background noise during acquisition. The predefined MEV gate was applied to all samples during analysis.

### F. Identification of proteins present in *Chlorella* MEVs

#### 1. Proteomic analysis

In order to determine the identity and the sequence of proteins associated to *Chlorella* MEVs, MEV samples were subjected to SDS-PAGE separation and main protein bands were cut into small pieces and washed several times with a 1/1 (v/v) solution of acetonitrile / 100 mM ammonium bicarbonate pH 8. The proteins were then reduced with 100 mM dithiothreitol in ammonium bicarbonate pH 8 for 45 min at 56°C and then cysteine residues were alkylated with 55 mM iodoacetamide in 100 mM ammonium bicarbonate pH 8 for 30 min at room temperature and in the dark. The gel pieces were then digested by trypsin (PROMEGA, reference V511A in a ratio 1:20) in 100 mM ammonium bicarbonate pH 8 and placed at 4°C for 45 min prior to incubation overnight at 37°C. Tryptic peptides were analyzed by LC-MS/MS on a mass

spectrometer and protein were identified by comparison of peptide sequences deduced from MS/MS sequencing with the proteome of *Chlorella vulgaris.* Proteins exhibiting sequence coverage higher than 10% were identified.

**2. Sequence Homology (by BLAST) of proteins from the proteomic analysis**

The proteins below were individually "blasted" using NCBI-Blast Protein. The identified proteins either were previously characterized as occurring in *Chlorella vulgaris,* or had medium or strong homologies with proteins from other Chlorella species, such as *Chlorella variabilis, Chlorella desiccata* and *Chlorella sorokiniana:*

- Protein CHLNCDRAFT_26010: annotated as «Acetyl-coenzyme A synthetase », with acetate-CoA ligase, AMP binding and ATP binding activities (MF), involved in acetyl-CoA biosynthetic process from acetate (Glycolysis, Gluconeogenesis, Pyruvate, Glyoxylate, Dicarboxylate and Propanoate metabolisms) (BP).
- Protein CHLNCDRAFT_56016: annotated as « Clp R domain-containing protein », with ATP binding and ATP hydrolysis activities (MF).
- Protein atpA: annotated as «ATP synthase subunit alpha, chloroplastic », component of chloroplast thylakoid membrane (CC), with ATP binding, proton-transporting ATP synthase activity, proton-transporting ATPase and hydrolase activities (MF), possibly involved in Oxidative phosphorylation and Photosynthesis (BP).
- Protein atpB: annotated as «ATP synthase subunit beta, chloroplastic », component of chloroplast thylakoid membrane (CC), with ATP binding, proton-transporting ATP synthase activity, proton-transporting ATPase and hydrolase activities (MF), possibly involved in Oxidative phosphorylation and Photosynthesis (BP).
- Protein tufA: annotated as «Elongation factor Tu », component of chloroplast (CC), with GTP binding, GTPase and translation elongation factor activities (MF).
- Hypothetical protein D9Q98_001761 (coverage 48%). Highly homolog to hypothetical protein D9Q98_001760 from *Chlorella vulgaris* (85,6%) and to other proteins from different species of *Chlorella* (*variabilis, desiccata* and *sorokiniana*), 3 of these homologs being characterized in *Chlorella sorokiniana:* "chlorophyll a b-binding", "chlorophyll a b-binding of LHCII type 1 chloroplastic" and "MYST-like histone acetyltransferase 1", with respectively 73%, 84% and 89% of sequence homology.
- Hypothetical protein D9Q98_002614 (coverage 33%). Highly homolog to hypothetical protein D9Q98_001760 (72%) and D9Q98_001761 (70%) from Chlorella vulgaris and to other proteins from different species of *Chlorella* (*variabilis, desiccata* and *sorokiniana*), 2 of these homologs being characterized in *Chlorella sorokiniana* : "chlorophyll a b-binding", "chlorophyll a b-binding of LHCII type chloroplastic", with respectively 89% and 70% of sequence homology; and 1 of these homologs characterized in *Chlorella desiccata* : putative "chlorophyll a b-binding of LHCII type 1 chloroplastic", with 60% of sequence homology.
- Hypothetical protein D9Q98_006265 (coverage 26%). No homology in *Chlorella vulgaris.* Only one homology with "DNA replication licensing factor MCM3" from *Chlorella sorokiniana,* with 56% of sequence homology.
- Hypothetical protein D9Q98_000265 (coverage 19%). No homology in *Chlorella vulgaris.* Only two homologies with two proteins from *Chlorella desiccata* and *Chlorella sorokiniana,* with only one characterized: "flagellar associated protein", with 58% of sequence homology (*Chlorella sorokiniana*).
- Hypothetical protein D9Q98_007183 (coverage 19%). No homology in *Chlorella vulgaris.* Only 5 homologies with 5 proteins from *Chlorella variabilis, Chlorella desiccata,* and *Chlorella sorokiniana,* whose only one characterized: "Fe-assimilating1," with 47% of sequence homology, *Chlorella sorokiniana*).
- Hypothetical protein D9Q98_009617 (coverage 15%). Mostly homolog to hypothetical proteins D9Q98_000026 and D9Q98_009618 from *Chlorella vulgaris* (with respectively 55% and 33% of sequence homology), and with 11 other proteins from *Chlorella variabilis, Chlorella desiccata,* and *Chlorella sorokiniana,* whose 5 are characterized in *Chlorella sorokiniana*: 2 "lytic transglycosylases", 2 "peptidoglycan-binding" and 1 "spore coat assembly domain" with respectively 52%, 34%, 42%, 42% and 55% of sequence homology.
- Hypothetical protein D9Q98_005959 (coverage 12%). No homology in *Chlorella vulgaris.* Only 4 homologies with 4 proteins from *Chlorella variabilis, Chlorella desiccata,* and *Chlorella sorokiniana,* whose only 2 characterized: "elongation factor mitochondrial" (87% of sequence homology, *Chlorella sorokiniana*) and "putative elongation factor Tu mitochondrial" (86% of sequence homology, *Chlorella desiccata*).
- Hypothetical protein D9Q98_003812 (coverage 11%). No homology in *Chlorella vulgaris.* Only 3 homologies with 3 proteins from *Chlorella variabilis* and *Chlorella desiccata,* whose only one characterized: "putative iron uptake system component EfeO" (58% of sequence homology, Chlorella desiccata).
- Hypothetical protein D9Q98_010620, partial [*Chlorella vulgaris*] (coverage 11%). Mostly homolog with 9 proteins from *Chlorella vulgaris:* D9Q98_004806, D9Q98_004582, D9Q98_004143, D9Q98_002326, D9Q98_002347, D9Q98_008273, D9Q98_008254, D9Q98_008251 and D9Q98_008376 (with respectively 63%, 59%, 47%, 49%, 48%, 41%, 41%, 44% and 47% of sequence homology). Only 7 homologies with 7 proteins from *Chlorella variabilis* and *Chlorella sorokiniana,* whose only 3 are characterized: "cellulosome anchoring cohesin region," "nascent

polypeptide-associated complex subunit alpha isoform X1" and "Protein CBG24242" (with respectively 43%, 42% and 58% of sequence homology, *Chlorella sorokiniana*).

- Hypothetical protein D9Q98_009216 (coverage 11%). Highly homolog to hypothetical protein D9Q98_009214 and D9Q98_009215 from *Chlorella vulgaris* (with respectively 98% and 98% of sequence homology). Only 2 homologies with 2 proteins from *Chlorella sorokiniana,* both characterized: "mechanosensitive ion channel 10" and "beta-Ig-H3 fasciclin" (with respectively 52% and 53% of sequence homology).

Proteins containing sites susceptible to *N*-glycosylation are reported in Table 3, below.

**Table 3.** List of proteins identified by a proteomic approach. Sequence coverage and number of potential N-glycosylation sites are indicated.

| Protein | Sequence coverage | Potential Glycosite | Name of the putative protein relevant to the identified fragment |
|---|---|---|---|
| D9Q98_001761 | 48% | 1 | chlorophyll a/b-binding protein of LHCII type, chloroplastic |
| D9Q98_002614 | 33% | 1 | chlorophyll a/b-binding protein of LHCII type, chloroplastic |
| D9Q98_006265 | 26% | 1 | n/a |
| D9Q98_007183 | 19% | 1 | n/a |
| D9Q98_000265 | 19% | 3 | n/a |
| D9Q98_009617 | 15% | 0 | n/a |
| D9Q98_005959 | 12% | 2 | translation elongation factor Tu, mitochondrial |
| D9Q98_003812 | 11% | 4 | n/a |
| D9Q98_010620 | 11% | 1 | n/a |
| D9Q98_009216 | 11% | 4 | n/a |

**G. Characterization of RNAs present in Chlorella MEVs**

With a goal to inventory the RNAs present in MEVs of *Chlorella vulgaris,* the sequencing of total RNA (small RNAs and long RNAs) was performed. Two types of sequencing were performed in this study: total RNA sequencing (which is based on reverse transcription from short random primers) and small RNA sequencing (which is based on reverse transcription from RNA-ligated adapters.

**1. RNA extraction**

Total RNAs were extracted from the "MEVs" sample using the Direct-zol™ RNA Microprep kit (Ozyme, Ref.: ZR2061) from 300 μL. The concentration of the RNAs and their quality was determined by fluorometry on a Qubit™ fluorometer with the Qubit™ RNA Assay Kit (Thermo Fisher Scientific, Ref.: Q32852) and Qubit™ RNA IQ Kit (Fisher Scientific, Ref.: Q33221) kits, respectively. After isolation a concentration of about 80ng of total native RNA per $10^9$ MEV was quantified.

**2. Preparation of total RNA library**

Total RNA was produced using the NEBNext® Ultra™ II Directional RNA library prep kit (New England Biolabs, Ref.: E7760S) following the manufacturer's protocol which was adapted for this study. Thus, in step 1.1.1, the 10 μl volume of nuclease-free water was adapted for this study.

The PCR products obtained were analyzed by capillary electrophoresis on QIAxcel® (QIAGEN, QIAxcel DNA Screening Kit, Ref.: 929004). The migration profile showed an amplification of fragments whose size was between 81 and 200 base pairs (see FIG. 4).

The library then was purified using the Thermo Scientific™ GeneJET PCR pu rification kit (Thermo Fisher Scientific, Ref.: K0702). All the fragments whose size w as between 125 and 175 bp were purified by electrophoresis on a PippinHT instrument (Sage Science) using a 3% agarose cassette, marker 30G (Sage Sciences, Ref.: HTG3010).

Following purification, the size of the fragments constituting the purified pool was checked by capillary electrophoresis and its concentration was determined by fluorometric assay on a Qubit™ 4.0 fluorimeter before sequencing.

### 3. Sequencing on MiSeq® and on NovaSeq 6000® System (Illumina)

To validate the quality of the TotalRNA library, the sequencing of the PCR products was performed on a MiSeq® System (Illumina) in 2 X 250 bp using the MiSeq® Reagent Kit v2 (500 cycles) (Illumina; Ref.: MS-102 -2003). The mix of libraries was loaded at a concentration making it possible to obtain a theoretical density of clusters between 800 and 1000 K/$mm^2$. The quality control positive results allowed the follow-up with the sequencing on the NovaSeq 6000 Instrument. Sequencing of the Total RNA library was performed on NovaSeq 6000 system in paired end reads (2X150 bp). The total read count was 94,850,242.

The small RNA library was sequenced on MiSeq™ (Illumina) in 2 X 250 bp using the MiSeq™ Reagent Kit v2 (500 cycles) (Illumina; Ref.: MS-102-2003). The mix of libraries was loaded at a concentration making it possible to obtain a theoretical density of clusters between 800 and 1000 K/$mm^2$.

### H. Bioinformatics analysis

The quality of the reads was evaluated with the FastQC tool (TotalRNA.R1.fastqc.html and TotalRNA.R2.fastqc.html files). Poor quality adapter sequences and external bases were removed from reads using the cutadapt tool. Reads were then aligned to the *Chlorella vulgaris* reference genome (cvul assembly, GenBank® No: GCA_023343905.1) using Hisat2 (using default settings). 40 reads aligned with the *Chlorella vulgaris* genome. The counting of the different alignments was carried out with Stringtie (default parameters) from annotations of the *Chlorella vulgaris* genome contains only the genes (with corresponding annotations) to which the reads obtained was aligned with *Chlorella vulgaris* genome. The available annotations, coming from public data, do not allow assigning an RNA type to each sequence. The same alignment work was performed with the genome of the mitochondria and that of the chloroplast of *Chlorella vulgaris.* None of the reads aligned with these two genomes.

The bioinformatic analysis of the total library shows about 40 RNAs between 140 and 250 nucleotides that, using NCBI BLAST, align with the *Chlorella vulgaris* genome and encode proteins that were not identified. The quality of the reads was evaluated with the Fastseq tool (SmallRNA.R1.fastqc.html and SmallRNA.R2.fastqc.html files). Poor quality adapter sequences and external bases were removed from reads using the cutadapt tool. Reads were then aligned to the *Chlorella vulgaris* reference genome (cvul assembly, GenBank® No: GCA_023343905.1) using Hisat2 (using default settings). The counting of the different alignments was carried out with Stringtie (default parameters) from annotations of the *Chlorella vulgaris* genome. The SmallRNA_quantitative_measures_filtred.xlsx file which lists only the genes to which the reads obtained aligned contains only one gene.

As the MEVs used for the analysis contained a mixture of siRNAs against luciferase an alignment on the luciferase sequence was carried out using the bowtie2 tool from sequences of at least 18 bp. About 3.7% of the sequences align with the luciferase sequence.

### I. MEV Lipidomic Analysis

### 1. Sample processing

Samples were slowly thawed on ice; 1.5 mL of chloroform/methanol (2/1, v/v) solution was added, followed by 0.5 mL of pure water. Samples were vortexed for 1 min, sonicated at 4°C for 30 min and centrifuged at 3000 rpm for 10 min. The organic phase was removed to a clean test tube, dried with nitrogen and reconstituted with 200 μL isopropanol/methanol (1/1, v/v). After adding 5 μL of internal standard (125 μg/mL LPC (12:0)), samples were centrifuge again at 12000 rpm, 4°C for 10 min. The supernatant was transferred into the injection bottle.

### 2. LC-MS detection

Analysis platform: LC- MS (Waters, UPLC; Thermo, Q Exactive); column: (ACQUITY UPLC BEH C18 (2.1*100mm 1.7 μm)); chromatographic separation conditions:

- Column temperature: 40 °C; Flow rate: 0.3 mL/min;
- Mobile phase A: Acetonitrile /water (6:4, v/v, with 10mM Ammonium formate);
- Mobile phase B: Iso-Propyl alcohol / Acetonitrile (9:1, v/v, with 10 mM Ammonium formate);
- Injection volume: 3 μL (positive ion); 3 μL (negative ion); Automatic injector temperature: 4°C.

**Table 4.** The gradient of mobile phase used in LC-MS detection method.

| Time (min) | Flow rate (mL/min) | A (%) | B (%) |
|---|---|---|---|
| 0 | 0.3 | 70 | 30 |
| 10.50 | 0.3 | 0 | 100 |
| 12.50 | 0.3 | 0 | 100 |
| 12.51 | 0.3 | 70 | 30 |
| 16.00 | 0.3 | 70 | 30 |

**3. Mass spectrometry detection parameters:**

- ESI+:Heater Temp 300°C; Sheath Gas Flow rate, 45 arb; Aux Gas Flow Rate, 15 arb; Sweep Gas Flow Rate, 1arb; spray voltage, 3.0KV; Capillary Temp, 350°C; S-Lens RF Level, 30%.
- ESI-:Heater Temp 300°C, Sheath Gas Flow rate,45arb; Aux Gas Flow Rate, 15arb; Sweep Gas Flow Rate, 1arb; spray voltage, 3.2KV; Capillary Temp 350°C; S-Lens RF Level, 60%.
- Scanning mode: Full Scan (M/Z 70~1050) and data-dependent mass spectrometry (DD-MS2, TopN = 10); Resolution :70,000 (primary mass spectrometry) & AMP;
- Collision mode: High energy collision dissociation (HCD)

**4. Lipidomic analysis results**

**Table 5.** ESI+ lipidomic analysis results for MEVs.

| Lipid Ion | Lipid Group | Class | Rt | Ion Formula | Peak Area |
|---|---|---|---|---|---|
| LPC(12:0)+H | LPC(12:0)+H | LPC | 0.97 | C20 H43 O7 N1 P1 | 13265407632.00 |
| LPC(12:0)+Na | LPC(12:0)+Na | LPC | 0.93 | C20 H42 O7 N1 P1 Na1 | 2093451784.00 |
| MG(16:0)+H | MG(16:0)+H | MG | 2.72 | C19 H39 O4 | 627421076.70 |
| TG(36:3e)+H | TG(36:3e)+H | TG | 9.64 | C39 H71 O5 | 597367444.40 |
| DG(18:0_16:0)+NH4 | DG(34:0)+NH4 | DG | 9.76 | C37 H76 O5 N1 | 545916845.40 |
| Cer(d16:0_16:0)+H | Cer(d32:0)+H | Cer | 8.67 | C32 H66 O3 N1 | 434951801.90 |
| DG(16:0_16:0)+NH4 | DG(32:0)+NH4 | DG | 9.16 | C35 H72 O5 N1 | 430757024.40 |
| DG(18:0_16:0)+NH4 | DG(34:0)+NH4 | DG | 9.64 | C37 H76 O5 N1 | 408179029.10 |
| MG(18:0)+H | MG(18:0)+H | MG | 3.81 | C21 H43 O4 | 402213163.90 |

**Table 6.** ESI- lipidomic analysis results for MEVs.

| Lipid Ion | Lipid Group | Class | Rt[c-1] | Ion Formula | Peak Area |
|---|---|---|---|---|---|
| Cer(d35:1+O)-H | Cer(d35:1+O)-H | Cer | 7.28 | C35 H68 O4 N1 | 31988627.10 |
| Cer(d20:0_23:0)+HCOO | Cer(d43:0)+HCOO | Cer | 10.63 | C44 H88 O5 N1 | 12403541.83 |
| Cer(d18:0_18:1)-H | Cer(d36:1)-H | Cer | 8.41 | C36 H70 O3 N1 | 5523529.96 |
| Cer(m18:0_13:0)+HCOO | Cer(m31:0)+HCOO | Cer | 6.75 | C32 H64 O4 N1 | 4409871.47 |
| Cer(d18:0_18:1)-H | Cer(d36:1)-H | Cer | 8.27 | C36 H70 O3 N1 | 3956710.64 |
| Cer(d15:1_18:1)+HCOO | Cer(d33:2)+HCOO | Cer | 6.38 | C34 H64 O5 N1 | 2457056.42 |
| Cer(d15:1_18:1)+HCOO | Cer(d33:2)+HCOO | Cer | 6.51 | C34 H64 O5 N1 | 2373869.91 |
| Cer(d20:2_13:0)+HCOO | Cer(d33:2)+HCOO | Cer | 4.22 | C34 H64 O5 N1 | 2255771.34 |

(continued)

| Lipid Ion | Lipid Group | Class | Rt[c-1] | Ion Formula | Peak Area |
|---|---|---|---|---|---|
| BiotinylPE(31:1)-H | BiotinylPE(31:1)-H | Bioti nylPE | 5.43 | C46 H83 O10 N3 S1 P1 | 1767937.80 |
| Cer(d15:0_18:2)+HCOO | Cer(d33:2)+HCOO | Cer | 4.11 | C34 H64 O5 N1 | 1324275.01 |

The major lipids species extracted are: Lyso-phosphatidyl-choline (LPC), monoacylglycerol (MG), diacylglycerol (DG), triacylglycerol (TG), and ceramides (Cer). Total extract of *Chlorella* cells has a different lipid composition (phosphatidyl-choline (PC), phosphatidyl-ethanolamine (PE), phosphatidyl-glycerol (PG) and diacylglycerol (DG) (Cecchin et al., (2019) The Plant Journal 100:1289-1305).

With regard to fatty acid composition, MEVs from *Chlorella vulgaris* showed a different composition to *Chlorella* total cell extract. MEVs have longer fatty acids and are more unsaturated than cell extracts. These results are summarized in the tables herein.

**J. MEV Metabolomics Analysis**

**Table 7.** ESI+ metabolomics analysis results for MEVs.

| Name | Formula | Molecular Weight | m/z | RT [min ] | Peak Area |
|---|---|---|---|---|---|
| [Similar to: 5-chloro-8-hydroxy-6-methoxy-3-methyl-3,4-dihydro-1H-2-benzopyran-1-one; AMass: -130.0268 Da] | C6 H5 Cl | 112.01 | 113.02 | 2.79 | 2275517912.00 |
| Benzothiazole | C7 H5 N S | 135.01 | 136.02 | 5.58 | 884980308.30 |
| N,N-Dimethyldodecylamine | C14 H31 N | 213.25 | 214.25 | 11.70 | 834705736.40 |
| Palmitic Acid | C16 H32 O2 | 273.27 | 274.27 | 9.56 | 574595945.20 |
| Indole | C8 H7 N | 100.03 | 101.04 | 2.80 | 541857154.80 |
| n/a | n/a | 101.04 | 102.05 | 2.79 | 514881461.80 |
| 2-(Methylthio)benzothiazole | C8 H7 N S2 | 181.00 | 182.01 | 7.96 | 448808384.80 |
| 1-Lauroyl-2-hydroxy-sn-glycero-3-phosphocholine | C20 H42 N O7 P | 439.27 | 440.28 | 7.65 | 346845832.20 |
| [Similar to: 2-(Methylthio)benzothiazole; AMass: -15.0236 Da] | C3 H7 N2 P S2 | 165.98 | 166.99 | 7.96 | 321761664.00 |
| 2-Amino-1,3,4-octadecanetriol | C18 H39 N O3 | 317.29 | 318.30 | 9.69 | 286436435.80 |

**Table 8.** ESI- metabolomics analysis results for MEVs.

| Name | Formula | Molecular Weight | m/z | RT [min] | Peak Area |
|---|---|---|---|---|---|
| IS_2-chloro-phenylalanine | C9 H10 Cl N O2 | 199.04 | 198.0 3 | 2.76 | 4768994771.00 |
| LPE 14:0; [M-H]- | C19 H40 N O7 P | 425.25 | 424.25 | 7.65 | 1457941932.00 |
| 2-Mercaptobenzothiazole | C7 H5 N S2 | 166.99 | 165.98 | 5.83 | 613566495.90 |
| n/a | n/a | 93.93 | 92.93 | 0.72 | 567261536.20 |
| n/a | n/a | 179.98 | 178.98 | 14.66 | 480949549.40 |
| [Similar to: 3'-Dephosphocoenzyme A; ΔMass: -589.1732 Da] | n/a | 97.98 | 96.97 | 0.75 | 392534007.80 |
| n/a | C25 H32 O8 | 460.21 | 459.20 | 8.39 | 304244723.20 |

(continued)

| Name | Formula | Molecular Weight | m/z | RT [min] | Peak Area |
|---|---|---|---|---|---|
| Xanthine | C5 H4 N4 O2 | 152.03 | 151.03 | 0.98 | 269743132.80 |
| DL-β-Leucine | C6 H13 N O2 | 131.09 | 130.09 | 1.16 | 266337552.60 |
| n/a | C2 H4 N2 O4 S | 151.99 | 150.98 | 14.67 | 246714926.90 |

The results indicate that the MEVs naturally carry a number of small-molecule components, such as aromatic heterocyclic compounds (including purine base and organosulfur compounds), fatty acids, cationic detergents, amino acids and derivatives thereof.

**EXAMPLE 3**

**Loading of biomolecule cargo into the Microalgae Extracellular Vesicles (MEVs)**

**A. Passive and surfactant-assisted loading of biomolecules (*e.g.*, proteins, peptides, siRNA, mRNA, Antisense Oligonucleotides (ASOs), plasmids, complexes) using plasmid DNA (pDNA) and GFP (protein) as exemplary biomolecules**

Purified MEVs, as described in Example 1, were diluted in PBS to a specific concentration (10^8, 10^9 or 10^10). Next, different concentrations of cargos (0.2, 1 or 2 μg/ml for pDNA; 2 or 20 μg/ml for GFP) were added to the MEV suspension (total volume of 500-1000 μl), and incubated for 1 or 24 hours for pDNA and 0, 1 or 6 hours for GFP (passive loading). For surfactant-assisted loading, the mixture of MEVs and pDNA was supplemented with 0.2% or 0.5% of saponin and incubated at room temperature for 5 or 30 min, with or without an agitation at 700 rpm.

**Table 9.** Protocol for passive and surfactant-assisted loading of DNA (plasmids, ASOs); parameters and values used as variables for sensitivity analysis.

| MEV concentration (per ml) | DNA concentration (μg/ml) | saponin concentration (%) | incubation temperature | incubation time | agitation (rpm) |
|---|---|---|---|---|---|
| 4.00E+08 | 0.05<br>0.15 | 0 | RT 37°C | 5 min<br>30 min<br>1 h<br>24 h | 0<br>700 |
| 4.00E+09 | 0.25<br>0.35 | 0.2 | | | |
| 4.00E+10 | 0.50<br>0.70 | 0.5 | | | |

**Table 10.** Protocol optimization for passive and surfactant-assisted loading of proteins (oligopeptides, polypeptides); parameters and values used as variables for sensitivity analysis.

| MEV concentration (per ml) | protein concentration (μg/ml) | saponin concentration (%) | incubation temperature | incubation time | agitation (rpm) |
|---|---|---|---|---|---|
| 6.00E+08<br>6.00E+09<br>1.40E+10<br>6.00E+10 | 0.1<br>0.5<br>1.0<br>2.0 | 0<br>0.2<br>0.5 | RT 37°C | 5 min<br>30 min<br>1 h<br>24 h | 0<br>700 |

**B. Freeze-thaw cycles loading of biomolecules (*e.g.*, proteins, peptides, siRNA, mRNA, ASOs, plasmids, complexes) using plasmid DNA (pDNA), GFP (protein) and mRNA (encoding eGFP) as exemplary biomolecules**

Purified MEVs, as described in Example 1, were diluted in PBS to a specific concentration (10^8, 10^9, 10^10, 10^11 and 10^12). Next, various concentrations of cargos (0.2, 1, 2 or 40 μg/ml for pDNA; 0,2 and 2 μg/ml for mRNA) were

added to the MEV suspension (total volume of 500 μl). The mixture of MEVs and the cargo was frozen at -80°C or immersed into liquid nitrogen (-196°C). Then the sample was thawed at 37°C in a water bath. This freeze-thaw cycle was repeated 2 or 4 times for each sample.

**Table 11.** Protocol optimization for freeze-thaw cycles loading of DNA (plasmids, ASOs); parameters and values used as variables for sensitivity analysis.

| MEV concentration (per ml) | DNA concentration (μg/ml) | freezing temperature | thawing temperature | number of cycles |
|---|---|---|---|---|
| 8.00E+07<br>1.00E+08<br>8.00E+08<br>9.00E+08<br>1.00E+09<br>6.00E+09<br>8.00E+09<br>9.00E+09<br>6.00E+10<br>9.00E+10<br>9.00E+11 | 0.2<br>1.0<br>2.0<br>20 | -80°C<br>-196°C | 37°C | 2<br>4 |

**Table 12.** Protocol optimization for freeze-thaw cycles loading of RNA (mRNA, siRNA, miRNA); parameters and values used as variables for sensitivity analysis.

| MEV concentration (per ml) | RNA concentration (μg/ml) | freezing temperature | thawing temperature | number of cycles |
|---|---|---|---|---|
| 8.00E+07<br>1.00E+08<br>8.00E+08<br>9.00E+08<br>1.00E+09<br>6.00E+09<br>8.00E+09<br>9.00E+09<br>6.00E+10<br>8.00E+10<br>9.00E+010<br>9.00E+11 | 0.2<br>1.0<br>2.0<br>20 | -80°C<br>-196°C | 37°C | 2<br>4 |

**Table 13.** Protocol optimization for freeze-thaw cycles loading of proteins (oligopeptides, polypeptides); parameters and values used as variables for sensitivity analysis.

| MEV concentration (per mL) | protein concentration (μg/mL) | freezing temperature | thawing temperature | number of cycles |
|---|---|---|---|---|
| 8.00E+07<br>1.00E+08<br>8.00E+08<br>9.00E+08<br>1.00E+09<br>6.00E+09<br>8.00E+09<br>9.00E+09<br>6.00E+10 | 0.2<br>1.0<br>2.0<br>20 | -80°C<br>-196°C | 37°C | 2<br>4 |

(continued)

| MEV concentration (per mL) | protein concentration (μg/mL) | freezing temperature | thawing temperature | number of cycles |
|---|---|---|---|---|
| 8.00E+10<br>9.00E+10<br>9.00E+11 | | | | |

**C. Sonication loading of biomolecules (proteins, peptides, siRNA, mRNA, ASOs, plasmids, complexes) using plasmid DNA (pDNA), mRNA (encoding eGFP) and GFP (protein) as exemplary biomolecules**

Purified MEVs, as described in Example 1, were diluted in PBS to a predetermined concentration (10^8, 10^9, 10^10, 10^11 and 10^12 MEVs/mL). Next, various concentrations of cargos (0.2, 1, 2 or 20 μg/ml for pDNA; 2 and 20 μg/ml for mRNA; 2 or 20 μg/ml for GFP) were added to the MEV suspension (total volume of 500-1000 μl). The mixture of MEVs and the cargo was sonicated using Fisherbrand™ Model 50 Sonic Dismembrator (frequency: 20 kHz, wattage: 50 W). Before sonication, the signal amplitude was set to 20%, 40%, 50% or 60%. Each sample undergone a sonication for 30 seconds followed by a pause during 30 seconds on ice, or 4 seconds followed by a rest period of 120 seconds on ice. This cycle was repeated 2, 6 or 8 times for each sample.

**Table 14.** Protocol optimization for sonication loading of DNA (plasmids, ASOs); parameters and values used as variables for sensitivity analysis.

| MEV concentration (per ml) | DNA concentration (μg/ml) | amplitude (Pa) | cycle timing | number of cycles |
|---|---|---|---|---|
| 8.00E+08<br>8.00E+09<br>9.00E+10<br>1.50E+10<br>1.50E+11 | 0.2<br>1.0<br>2.0<br>20 | 20<br>40<br>50<br>60 | 4s ON, 2s OFF, 2 min on ice<br>30s ON, 30s OFF, 2 min on ice<br>30s ON, 30s OFF on ice | 2<br>6<br>8 |

**Table 15.** Protocol optimization for sonication loading of RNA (mRNA, siRNA, miRNA); parameters and values used as variables for sensitivity analysis.

| MEV concentration (per ml) | RNA concentration (μg/ml) | amplitude (Pa) | cycle timing | number of cycles |
|---|---|---|---|---|
| 8.00E+08<br>8.00E+09<br>9.00E+10<br>1.40E+10<br>1.50E+10 | 0.2<br>1.0<br>2.0<br>20 | 20<br>40<br>50<br>60 | 30s ON, 30s OFF, 2 min on ice<br>4s ON, 2s OFF, 2 min on ice<br>30s ON, 30s OFF on ice | 2<br>6<br>8 |

**Table 16.** Protocol optimization for sonication loading of proteins (oligopeptides, polypeptides); parameters and values used as variables for sensitivity analysis.

| MEV concentration (per mL) | protein concentration (μg/mL) | amplitude (Pa) | cycle timing | number of cycles |
|---|---|---|---|---|
| 1.40E+10<br>1.50E+10<br>1.50E+11<br>2.00E+11<br>6.00E+11<br>1.50E+12 | 2.0<br>20 | 20<br>50<br>70 | 30s ON, 30s OFF on ice | 4<br>6<br>8 |

**Table 17.** Protocol optimization for sonication loading of small molecules (hydrophilic molecules); parameters and values used as variables for sensitivity analysis.

| MEV concentration (per mL) | protein concentration ($\mu$g/mL) | amplitude (Pa) | cycle timing | number of cycles |
|---|---|---|---|---|
| 1.40E+10<br>1.50E+10<br>1.50E+11<br>2.00E+11<br>6.00E+11<br>1.50E+12 | 2.0<br>20 | 20<br>50<br>70 | 30s ON, 30s OFF on ice | 4<br>6<br>8 |

**E. Extrusion loading of biomolecules (*e.g.*, proteins, peptides, siRNA, mRNA, ASOs, plasmids, complexes) using plasmid DNA (pDNA), mRNA and GFP as exemplary biomolecules**

Purified MEVs, as described in Example 1, were diluted in PBS to a specific concentration (10^10). Next, different concentrations of cargos (2 or 20 $\mu$g/ml for pDNA; 0.1, 1, 2 and 10 $\mu$g/ml for mRNA; 2 or 20 $\mu$g/ml for GFP) were added to the MEV suspension (total volume of 500-1000 $\mu$l). The mixture of MEVs and the cargo was extruded through a syringe-based hand-held mini-extruder (SKU: 610023-1 EA, Avanti® Polar Lipids). The sample was extruded from 10 to 15 times across a membrane filter with 100 nm diameter pores, using two facing syringes. In some experiments, the sample was extruded sequentially through a 200 nm, a 100 nm and 50 nm diameter pore membranes. The extrusion was performed at room temperature or at 65°C.

**Table 18.** Protocol optimization for extrusion loading of DNA (plasmids, ASOs); parameters and values used as variables for sensitivity analysis.

| MEV concentration (per mL) | DNA concentration ($\mu$g/mL) | temperature | extrusion cycles | membrane pore diameter (nm) |
|---|---|---|---|---|
| 4.00E+08<br>9.00E+08<br>4.00E+09<br>4.00E+10<br>6.00E+10<br>8.00E+10<br>9.00E+10 | 0.2<br>1.0<br>2.0<br>20 | RT | 10<br>11<br>20 | 100<br>200 |

**Table 19.** Protocol optimization for extrusion loading of RNA (mRNA, siRNA, miRNA); parameters and values used as variables for sensitivity analysis.

| MEV concentration (per ml) | RNA concentration ($\mu$g/ml) | temperature | extrusion cycles | membrane pore diameter (nm) |
|---|---|---|---|---|
| 4.00E+08<br>9.00E+08<br>4.00E+09<br>7.00E+09<br>1.50E+10<br>4.00E+10<br>6.00E+10<br>8.00E+10<br>9.00E+10 | 0.2<br>1.0<br>2.0<br>20 | RT<br>65°C | 10<br>11<br>20 | 100<br>200 |

**Table 20.** Protocol optimization for extrusion loading of proteins (oligopeptides, polypeptides); parameters and values used as variables for sensitivity analysis.

| MEV concentration (per ml) | protein concentration (μg/ml) | temperature | extrusion cycles | membrane pore diameter (nm) |
|---|---|---|---|---|
| 6.00E+08<br>6.00E+09<br>1.50E+10<br>6.00E+10<br>9.00E+10 | 0.1<br>0.5<br>1.0<br>2.0<br>10<br>20 | RT<br>65°C | 10<br>11<br>20 | 50<br>100<br>200 |

**E. Sonication and extrusion-assisted active loading (SEAL) of biomolecules (*e.g.*, proteins, peptides, siRNA, mRNA, ASOs, plasmids, complexes) loading mRNA and GFP as exemplary biomolecules**

Purified MEVs, as described in Example 1, were diluted in PBS to a specific concentration (10^11). Next, different concentrations of cargos (2 or 20 μg/ml for mRNA; 2 or 20 μg/ml for GFP) were added to the MEV suspension (total volume of 500-600 μl). The mixture of MEVs and the cargo was sonicated for 8 min (30 sec on/30 sec off, on ice) at 20% amplitude by a Fisherbrand™ Model 50 Sonic Dismembrator (frequency: 20 kHz, wattage: 50 W). Then the sonicated MEVs were further extruded with the cargo through 100 nm polycarbonate (PC) membranes for ten cycles at room temperature.

**Table 21.** Protocol optimization for SEAL loading of RNA (mRNA, siRNA, miRNA); parameters and values used as variables for sensitivity analysis.

| MEV concentration (per ml) | RNA concentration (μg/ml) | amplitude (Pa) | cycle timing | number of cycles | temperature | extrusion cycles (per membrane) | membrane pore diameter (nm) |
|---|---|---|---|---|---|---|---|
| 1.50E+10 | 0.2<br>2.0<br>20 | 20 | 30s ON, 30s OFF on ice | 8 | RT | 9<br>10 | 200, followed by 100 |

**Table 22.** Protocol optimization for SEAL loading of proteins (oligopeptides, polypeptides); parameters and values used as variables for sensitivity analysis.

| MEV concentration (per ml) | protein concentration (μg/ml) | amplitude (Pa) | cycle timing | number of cycles | temperature | extrusion cycles (per membrane) | membrane pore diameter (nm) |
|---|---|---|---|---|---|---|---|
| 1.50E+10 | 2.0<br>20 | 20 | 30s ON, 30s OFF on ice | 8 | RT | 10 | 100 |

**F. Enzymatic activity of MEV-loaded catalase**

The loading efficiency for MEV formulations loaded as above was assessed by catalase activity assay using hydrogen peroxide decomposition. 1 mL of PBS (pH 7.4) was mixed into a quartz cuvette with 1-4 μL $H_2O_2$ (7.5-30% v/w) and 2 μL of catalase (0.06-0.5 mg/ml) or samples of cargo-loaded MEVs, respectively. Enzymatic activity was measured by monitoring the absorbance at 240 nm using a LAMBDA™ 25 UV VIS Spectrophotometer (PerkinElmer Instruments). Catalase stability in the loaded MEVs was evaluated by incubation with pronase (0.1-0.2 mg/mL) for 3 hours at 37°C. The samples were subsequently assayed for catalytic activity as described above. Stability of catalase was expressed as the residual activity vs. initial activity of catalase.

**G. Active dialysis of biomolecules (*e.g.*, proteins, peptides, siRNA, mRNA, ASOs, plasmids, complexes) using catalase or rhodamine B as exemplary biomolecules**

Hypotonic dialysis is performed by transferring MEVs and rhodamine B (acquired from Sigma-Aldrich) into dialysis membranes (cellulose ester, molecular weight cut-off 100-500 Da, acquired from Spectrum Labs) placed in 200 mL of 10 mM phosphate buffer (pH 7.4) and stirred at room temperature for 4 h. Samples are subsequently purified from free biomolecules (cargo) molecules using 300 kDa molecular weight cut-off centrifuge filters with washing with 500 μL of TE buffer (10 mM Tris, pH 7.5 and 1 mM EDTA, pH 8.0) and centrifuging at 5,000 g at 4°C for 5 min, repeated three times.

Hypotonic dialysis is performed by transferring MEVs and catalase into dialysis membranes (cellulose ester, molecular weight cut-off 100-500 Da, Spectrum Labs) placed in 200 mL of 10 mM phosphate buffer (pH 7.4) and stirred at room temperature for 4 h. Samples are subsequently purified from free cargo molecules using 300 kDa molecular weight cut-off centrifuge filters with washing with 500 μL of TE buffer (10 mM Tris, pH 7.5 and 1 mM EDTA, pH 8.0) and centrifuging at 5,000 g at 4°C for 5 min, repeated three times.

**H. Electroporation of biomolecules (*e.g.*, proteins, peptides, siRNA, mRNA, ASOs, plasmids, complexes) using plasmid DNA (pDNA) and mRNA as exemplary biomolecules**

Purified MEVs, as described in Example 1, were diluted in PBS to a specific concentration (10^8, 10^9 or 10^10). Next, different concentrations of cargos (1, 2, 5, 10 or 20 μg/ml for pDNA; 2 and 20 μg/ml for mRNA) were added to the MEV suspension (total volume of 110-150 μl). The mixture of MEVs and the cargo was transferred into a 100 μl electroporation cuvette and placed into a Super Electroporator NEPA 21 (NEPAGENE) device. The following parameters were set for each electroporation: the voltage (50 V, 100 V, or 200 V), the pulse length (5 ms, 10 ms, or 15 ms), the pulse interval (set to 50 ms), the decay rate (set to 10%), the polarity (set to positive), the number of pulse (1, 9 or 15), and the presence or absence of a transfer pulse (5 pulses of 20 V during 50 ms with 50 ms interval and 10 % decay rate). To assess aggregation, 20 mM of EDTA or 10 mM of citrate have been added before electroporation for each condition.

**Table 23.** Protocol optimization for electroporation loading of DNA (plasmids, ASOs); parameters and values used as variables for sensitivity analysis.

| MEV concentration (per ml) | DNA concentration (μg/ml) | voltage (V) | length (ms) | number of pulses | transfer pulse(s) |
|---|---|---|---|---|---|
| 8.00E+08 | 0.2 | 50 | 5 | 1 | 0 |
| 9.00E+08 | 1.0 | 100 | 10 | 9 | 1 |
| 1.50E+09 | 2.0 | 200 | 15 | | 6 |
| 8.00E+09 | 10 | | | | |
| 9.00E+09 | 20 | | | | |
| 1.50E+10 | | | | | |
| 6.00E+10 | | | | | |
| 8.00E+10 | | | | | |
| 9.00E+10 | | | | | |
| 8.00E+11 | | | | | |
| 9.00E+11 | | | | | |

**Table 24.** Protocol optimization for electroporation loading of RNA (mRNA, siRNA, miRNA); parameters and values used as variables for sensitivity analysis.

| MEV concentration (per ml) | RNA concentration (μg/ml) | voltage (V) | length (ms) | number of pulses | transfer pulse(s) |
|---|---|---|---|---|---|
| 8.00E+08 | | | | | |
| 9.00E+08 | | | | | |
| 1.50E+09 | | | | | |
| 8.00E+09 | 0.2 | | | | |
| 9.00E+09 | 0.1 | 50 | 5 | 1 | 0 |
| 1.50E+10 | 2.0 | 100 | 10 | 9 | 1 |
| 6.00E+10 | 10 | 200 | 15 | | 6 |

(continued)

| MEV concentration (per ml) | RNA concentration (μg/ml) | voltage (V) | length (ms) | number of pulses | transfer pulse(s) |
|---|---|---|---|---|---|
| 8.00E+10<br>9.00E+10<br>8.00E+11<br>9.00E+11 | 20 | | | | |

**Table 25.** Protocol optimization for electroporation loading of proteins (oligopeptides, polypeptides); parameters and values used as variables for sensitivity analysis.

| MEV concentration (per ml) | protein concentration (μg/ml) | voltage (V) | length (ms) | number of pulses | transfer pulse(s) |
|---|---|---|---|---|---|
| 6.00E+10 | 1.0<br>10 | 100 | 5<br>10<br>15 | 1<br>9 | 0<br>1<br>6 |

**I. DNase Treatment:**

After each loading method, a part of the samples undergone a treatment with a Deoxyribonuclease I (DNase) (10104159001, Roche) to eliminate all free nucleic acids (non-encapsulated by the loading). The DNase and a DNase buffer (Tris-HCl 10 mM, $MgCl_2$ 2.5 mM, $CaCl_2$ 0.5 mM, pH 7.6) were mixed with the sample and incubated for 40 minutes in an incubator at 37°C. Finally, ethylenediaminetetraacetic acid (EDTA) (20 mM) was added to inhibit DNase action. Samples were then stored at 4°C.

**J. RNase Treatment:**

After each loading method, a portion of the samples were treated with Ribonuclease I (RNase) (EN0601, Thermo Fisher Scientific) to remove any free (non-encapsulated by the loading) ribonucleic acids. The RNase was mixed with the sample and incubated for 15 minutes in a 37°C incubator. Finally, SUPERase·In™ (AM2694, Invitrogen, Waltham, MA), an RNAase inhibitor, was added to inhibit the action of RNase. The samples were then incubated for 4 hours in a 37°C incubator. Finally, the samples were stored at 4°C.

**EXAMPLE 4**

**MEV characterization after loading the biomolecule cargo**

**A. DNA extraction:**

QIAprep® Spin Miniprep Kit (acquired from QIAGEN) was used to extract nucleic acid encapsulated by the MEVs according to the manufacturer's recommendations. At the end of the protocol, nucleic acids were eluted in 50 μL of nuclease-free water.

**B. RNA extraction:**

The RNeasy® Micro Kit (50) (acquired from QIAGEN) was used to extract the ribonucleic acid encapsulated by the MEVs according to the manufacturer's recommendations. At the end of the protocol, the nucleic acids were eluted in 14 μL of nuclease-free water.

**C. Reverse Transcription:**

RNA was isolated from loading methods of MEVs with CleanCap® mRNA coding for eGFP (Tebu-bio) (to which 50 ng of an RNA (mCherry (Tebu-bio)) has been added) by the single-step purification method with RNeasy® Micro Kit (ref No. 74004, QIAGEN GmbH, Germany) described by the manufacturer's protocol (QIAGEN-RNeasy Micro Handbook). At the end of the protocol, the nucleic acids we re eluted in 14 μL of nuclease-free water.

Reverse transcription and real-time PCR: Quantitative RT-PCR (RT-Q-PCR) was performed as follows: 4μl of extracted total RNA was reverse transcribed using SuperScript™ II Reverse Transcriptase (Thermo Fisher Scientific cat. No. 18064-071, manufacturer's protocol: Part no. 18064.pps, MAN0001342) in a 16 μl reaction volume (constituted with hexamer random primer, dNTP Mix, Buffer, DTT, pure RNA, SuperScript™ II RT and RnaseOut). The reaction was mixed by very gentle pipetting to ensure that all reagents were thoroughly mixed. After mixing Hexamer Random primer, dNTP Mix, RNA and water, samples were spun down and incubated for 5 minutes at 65°C, put on ice and finally incubated 12 minutes at 25°C where buffer, DTT, RnaseOut and SuperScript™ II RT were added. Next, the reaction was incubated at 42°C for 50 min. To finish the mRNA reverse transcription protocol, the Reverse Transcriptase was heat-inactivated for 15 minutes at 70°C. The obtained volume of cDNA after RT was 16 μl.

After the RT reaction, 56μl of $H_2O$ were added to the sample to obtain in total a 72μl cDNA starting solution per sample. Before Real-time PCR measurements, the needed dilution factor of the 72μl cDNA starting solution was determined via prescreening experiments as X1000. Defining the right dilution factor ensures that the measured Cq output values will have values between 20 and 30. This Cq 20-30 range assures better linearity and avoids the signal's noise region which is expected to start at Cqs higher than 30.

**D. qPCR:**

To quantify the plasmid DNA extracted from the MEVs, quantitative Polymerase Chain Reaction (qPCR) was performed. DNA concentration was measured in the samples using a NanoDrop™ 2000 analyzer (acquired from Thermo Fisher Scientific) and the samples were diluted if necessary to be correctly amplified during the qPCR (volumes for each condition are listed in annexes). A plasmid range was realized from 0.4 x $10^{-6}$ ng to 5.1 x $10^{-6}$ ng of pDNA. A master mix was prepared with 5 μL of PowerUp™ SYBR™ Green per well (Cat. No. A25776, Fisher Scientific), 0.5 μL of forward primer per well (concentration = 10 μM), and 0.5 μL of reverse primer per well (concentration = 10 μM). Primers complementary to the resistance gene sequence of the pDNA have been previously designed to amplify only the plasmid of interest (primers sequences are in annexes). An Applied Biosystems™ MicroAmp™ Optical 384-Well reaction plate (Cat. No. 10411785, Thermo Fisher Scientific) was used, 6 μL of master mix were put in each well, followed by 4 μL of the sample. Two negative controls were made: nuclease-free water with SYBR® green dye, and nuclease-free water with SYBR® green dye and primers. All conditions were done in duplicates. The plate was sealed and briefly centrifuged. The thermocycler was a CFX384 Touch Real Time PCR detection system (from Bio-Rad). The three-step cycling, followed by the melt curve protocols are summarized in the tables below.

**Table 26.** Three-step cycling protocol for qPCR

| Step | Temperature (°C) | Time | Number of cycles |
|---|---|---|---|
| Dual lock DNA polymerase | 95 | 10 minutes | 1 |
| Denaturation | 95 | 15 seconds | 40 |
| Annealing | 60 | 1 minute | |

**Table 27.** Melt curve protocol for qPCR

| Step | Ramp rate | Temperature (°C) | Time |
|---|---|---|---|
| 1 | 1.6°C/second | 95 | 15 seconds |
| 2 | 1.6°C/second | 60 | 1 minute |
| 3 | 0.15°C/second | 95 | 15 seconds |

For the RNA detection after RT, real-time qPCR was performed in technical duplicates in qPCR plates with a rapid thermal cycler system (LightCycler® 480 II, 384-well). The protocol was the following: 3 μl cDNA (with defined dilution factor of X1000 were mixed with one forward and one reverse primer (added at optimized concentrations usually between 200 nM and 1.0 μM), mix Takyon® No ROX SYBR 2 X MasterMix blue dTTP (Eurogentec, UF-NSMT-B0701) with dNTPs, $MgCl_2$, Taq DNA polymerase and buffer, constituting a volume of 10 μl in total. Then, this mix was placed in LightCycler® plates.

The amplification protocol started with an initial incubation at 95°C for 10 min (serving for activation of Taq DNA polymerase), followed by 45 amplification cycles composed of two steps: step I) a 95°C denaturation for 10 seconds, step II) 60°C primer annealing and extension for 40 seconds, (the detection of the fluorescent product was performed at the end of this 60°C extension period with a single acquisition mode). The amplification protocol is followed by the step to perform

the melting curve with one cycle of 95°C denaturation for 5 seconds, 60°C annealing for 40 seconds, and a 95°C end point (achieved by a temperature raise with a ramp rate of 0.04°C/s from 60°C to 95°C and accompanied by continuous detection of the fluorescent product). The amplification protocol ended by cooling at 37°C for 10 seconds.

For plasmid quantification (dilution by 1000), the same protocol (mix and cycles) of the Lightcycler® instrument is used as with cDNA quantification, except the primers are modified.

The qPCR's primers and their concentrations are listed below:

RNA Primers (SEQ ID NOs: 66 and 67):

- eGfP(1-1)For3 AGCAAAGACCCCAACGAGA
- eGfP(1-1)Rev3 TCGTCCATGCCGAGAGTG
- eGfP(1-1) For3Rev3: 400 nM

RNA reference Primers (SEQ ID NOs:68 and 69):

- mCherry(1-1)F1: GACCACCTACAAGGCCAAGA
- mCherry(1-1)R1: CCGCTCGTACTGCTCCAC
- mCherry(1-1) FR1: 500 nM

Plasmid (pDNA) pcDNA3.1+ Primers (SEQ ID NOs:70 and 71):

- Left primer PCDNA3.1+1: GACCACCAAGCGAAACATGG
- Right primer PCDNA3.1+1: CCATGGGTCACGACGAGATC
- PCDNA3.1 LR1(Left primer PCDNA3.1+1, Right primer PCDNA3.1+1): 700 nM

**Controls for RT-qPCR assays**

To confirm the correct functioning of our RT-qPCR analysis, two different controls were included in the experiments: a negative control and an efficacy and quantification control. All two controls were applied on each qPCR plate.

One negative control, consisting of RNA free sample ($H_2O$ only), was added to each qPCR plate and mixed with the according primers on the qPCR plate. This negative $H_2O$ control allowed the estimation of the Cq background levels for each amplicon, that hybridizing primers create.

For the "RNA efficacy and quantification control" sample, a mix of CleanCap mRNA coding for eGFP was prepared. Subsequently, 9 different concentrations (to which 50 ng of an RNA (mCherry (Tebu-bio)) has been added) were prepared with RNAse/DNase free water (1000 ng, 500 ng, 100 ng, 50 ng, 10 ng, 5 ng, 1 ng, 0.5 ng, 0.1 ng), extracted, reverse transcribed, and then diluted (identically as the samples, dilution factor of X1000) and mixed with the corresponding primers used on a qPCR plate.

Efficacy and quantification controls were employed on each qPCR plate to control and quantify the primers' annealing efficacies at different DNA concentrations and quantified.

For the "DNA efficacy and quantification control" sample, a mix of Plasmid (pDNA) pcDNA3.1+ was prepared. Subsequently, 9 different concentrations were prepared with RNAse/Dnase-free water (1000 ng, 500 ng, 100 ng, 50 ng, 10 ng, 5 ng, 1 ng, 0.5 ng, 0.1ng), extracted and diluted (identically as the samples, dilution factor of X1000) and mixed with the according primers used on a qPCR plate.

**E. Fluorescence readout by ZetaView® nanoparticle tracking analysis (NTA) instrument.**

ZetaView® NTA instrument (engine) from Particle Metrix is a Nanoparticle Tracking Analysis engine for measuring hydrodynamic particle size, zeta potential, concentration and fluorescence. It was calibrated before the experiment according to the manufacturer's recommendations with polystyrene beads. The PBS used for the day's experiments was evaluated with the ZetaView® engine (normal average number of particles on screen in PBS: 0-5). Samples were diluted with PBS to be measured by ZetaView® within the manufacturer's recommended reading range (50-200 particles per frame). Dilutions were made in PBS, then the sample was vortexed and placed in a 1 mL syringe for the analysis.

The samples were analyzed with ZetaView® engine in scatter mode (laser 488 nm) to determine the number and

size distribution of the particles. The samples were then analyzed with the ZetaView® engine in fluorescence using a laser at 488 nm and a fluorescence filter at 500 nm at different percentages of sensitivity (95% or 90% ). The analog view of the ZetaView® engine was activated during the fluorescence analysis to visualize the background noise.

**F. Enzymatic activity of catalase-loaded MEVs**

The loading efficiency for MEV formulations loaded as above was assessed by catalase activity assay using hydrogen peroxide decomposition. Briefly, 1 mL of PBS (pH 7.4) was mixed into a quartz cuvette with 1-4 μL $H_2O_2$ (7.5-30% v/w) and 2 μL of catalase (0.06-0.5 mg/ml) or samples of cargo-loaded MEVs, respectively. Enzymatic activity was measured by monitoring the absorbance at 240 nm using Lambda 25 UV VIS Spectrophotometer (PerkinElmer® Instruments). Catalase stability in the loaded MEVs was evaluated by incubation with pronase (0.1-0.2 mg/mL) for 3 hours at 37°C. The samples were subsequently assayed for catalytic activity as described above. Stability of catalase was expressed in the residual activity vs. initial activity of catalase.

**G. Quantification of the rhodamine B cargo**

The cargo carrying MEVs loaded as above were lysed to release the cargo by adding 5 μL of 0.4% SDS into 95 μL of MEVs mixture, thorough pipetting, and incubation in a thermocycler for 15 min at 85°C. Next, the samples were transferred into black-walled clear bottom non-treated polystyrene 96-well plates. Control samples contained 0.1, 0.5, 1.0, 5.0 nmol of rhodamine B alone. The sample fluorescence was measured using a SpectraMax® fluorescence microplate reader (Molecular Devices, USA) with excitation at 540 nm and emission at 625 nm.

**H. Quantification of the siRNA cargo**

The cargo loaded MEVs were lysed as above. Next, the siRNA cargo was detected using Quant-iT® PicoGreen® Assay kit (from Life Technologies), able to quantify nucleic acids with picogram sensitivity. Briefly, 100 μL of dye reagent working solution was added to 100 μL of each sample to make final volume of 200 μl. A control sample prepared in the same buffer contained 10 pmol of siRNA alone. Samples were transferred into black-walled clear bottom non-treated polystyrene 96-well plates and incubated in the dark for 10 min at room temperature. The sample fluorescence was measured using a SpectraMax® fluorescence microplate reader (from Molecular Devices, USA) with excitation at 480 nm and emission at 520 nm.

**I. Quantification of total proteins**

Total proteins were quantified by BCA or Bradford test.

**J. Statistical analysis:**

Screening and optimized experimental designs were generated with NemrodW software. The results have been analyzed using RStudio® (1.4.1717 version) and GraphPad Prism® (8.3 version) software. Significance of the variables compared to the controls was evaluated with unpaired parametric t tests. "ns" stands for "not significant" meaning a p-value greater than 0.05, "*" stands for a p-value less than 0.05, "**" stands for a p-value less than 0.01, and "***" stands for a p-value less than 0.001. Loading efficiency was calculated based on the pDNA quantity obtained by qPCR compared to the initial pDNA quantity for each condition. Linear regression modeling was realized to evaluate the effect of tested variables on plasmid encapsulation.

**K. Exogenous-loading (Exo-Loading) Efficiency**

MEVs can be exogenously loaded with cargo following production and isolation the MEVs. Numerous methods for introducing cargo into the MEVs are known and described herein. Examples 1-4 describe and exemplify exo-loading of MEVs with various cargo (payloads). Preparation, purification, loading characterization, of MEVs. The efficiency of exo-loading using the different loading methods for a variety of different payloads is calculated using the parameters: (i) loading efficacy, and/or (ii) loading capacity, and (iii) the percentage of loaded MEV is shown in the following Table (Table 28).

**Table 28:**

| Payload | Method | | Tested Conditions | Loading Efficiency | Loading Capacity |
|---|---|---|---|---|---|
| **DNA (plasmids / ASOs)** | **A** | Incubation | 9 | (0) | (0) |
| | **B** | Saponification | 12 | (+) | (++) |
| | **C** | Freeze thaw cycle | 28 | (++) | (+++) |
| | **D** | Electroporation | 46 | (+++) | (+++) |
| | **E** | Sonication | 23 | (++) | (++) |
| | **F** | Extrusion | 25 | (+) | (+) |
| | **G** | Serial Extrusion | 12 | (+) | (+) |
| | **H** | SEAL | (-) | ND | ND |
| **RNA (mRNA / siRNA / miRNA)** | **A** | Incubation | (-) | ND | **ND** |
| | **B** | Saponification | (-) | ND | **ND** |
| | **C** | Freeze thaw cycle | 6 | (++) | (++) |
| | **D** | Electroporation | 12 | (++) | (++) |
| | **E** | Sonication | 6 | (++) | (++) |
| | **F** | Extrusion | 24 | (++) | (++) |
| | **G** | Serial Extrusion | 21 | (+++) | (+++) |
| | **H** | SEAL | 10 | (0) | (0) |

(continued)

| Payload | Method | Tested Conditions | Loading Efficiency | % of positive (fluorescent) MEV |
|---|---|---|---|---|
| **Proteins (peptides / GFP / other proteins)** | **A** Incubation | 9 | (+) | (+) |
| | **B** Saponification | 12 | (++) | (++) |
| | **C** Freeze thaw cycle | 12 | (++) | (++) |
| | **D** Electroporation | 12 | (++) | (++) |
| | **E** Sonication | 40 | (+++) | (+++) |
| | **F** Extrusion | 14 | (++) | (++) |
| | **G** Serial Extrusion | 14 | (++) | (++) |
| | **H** SEAL | 15 | (++) | (++) |

Key: Loading (0) very low; (+) low; (++) acceptable; (+++) best results; ND not determined, where the parameters are as follows:

• Internalized payload is the total amount of payload molecules (DNA, RNA, protein, or other molecules) measured inside the MEVs after the loading reaction. The internalized payload is determined by a specific quantification method (such as qPCR, RT-qPCR, determination of proteins, and other), after the elimination of the remaining payload molecules in the loading reaction medium at the end of reaction by specific enzymatic treatment (such as DNAse, RNAse, protease, other).

• Copy number of payloads: Number of copies of payload molecule (DNA or RNA) in the either initial amount or the internalized payload amount. The number of copies is obtained using the following calculation:

$$\text{Number of copies}=(\text{ng}\times[6.022\text{Exp}23])/(\text{length}\times[1\text{Exp}9]\times 650)$$

where: ng is the amount of nucleic acids (plasmid, RNA.), 6.022Exp23 = Avogadro's number, length is the length of the DNA fragment in base pairs multiplied by 1000 (kb), 1Exp9 is conversion factor to convert to units of nanograms.

• Loading efficiency is the percentage of the initial amount of payload molecule, in the loading reaction medium at the initial timepoint, internalized into extracellular vesicles from microalgae (MEVs).

• Loading capacity is the copy number of payload internalized per MEV. The loading capacity calculated as the ratio between the internalized payload copies and the number of MEVs in the reaction.

• As an example: if in a loading reaction there are initially 3.6Exp10 copies of DNA and 1Exp9 MEVs, and by qPCR is determined that 1.8Exp9 copies of DNA are internalized into the MEVs. For this loading reaction the loading parameters are the following:

- loading efficiency = (1.8Exp9 DNA copies/3.6Exp10 DNA copies) *100 = 5%
- loading capacity = 1.8Exp9 DNA copies/1Exp9 MEV = 1.8 DNA copies / MEV.

• Percentage of loading MEVs: is the number of fluorescent MEVs per total number of MEVs per 100. The fluorescence is determined using ZetaView® device at f90 and f95 are sensitivity values. Sensitivity values are expressed in arbitrary units ranges from 0 to 100. It represents the sensitivity of the camera sensor in the ZetaView® device. This sensitivity setting is comparable to the sensitivity setting of a camera used in digital photography. The number of detected particles in the field is associated to the sensitivity camera: increase of sensitivity -> increase of the number of particles detected in each sample. Sensitivity is calibrated before the measurement based on the negative control chose the highest sensitivity for which no event is detectable. Table 29, below, shows different payloads loaded into the MEV with sufficient/acceptable loading efficiency (SEQ ID NOs:111 - 127).

Table 29

| Payload | | SEQUENCE/SEQ ID or formula |
|---|---|---|
| Proteins & Peptides | GFP | MSKGEELFTGVVPILVELDGDVNGHKFSVSGEGEGDATYGKLT<br>LKFICTTGKLPVPWPTLVTTFSYGVQCFSRYPDHMKQHDFFKSA<br>MPEGYVQERTIFFKDDGNYKTRAEVKFEGDTLVNRIELKGIDFK<br>EDGNILGHKLEYNYNSHNVYIMADKQKNGIKVNFKIRHNIEDGS<br>VQLADHYQQNTPIGDGPVLLPDNHYLSTQSALSKDPNEKRDHM<br>VLLEFVTAAGITHGMDELYK (SEQ ID NO:111) |
| | luciferase | MEDAKNIKKGPAPFYPLEDGTAGEQLHKAMKRYALVPGTIAFT<br>DAHIEVNITYAEYFEMSVRLAEAMKRYGLNTNHRIVVCSENSLQ<br>FFMPVLGALFIGVAVAPANDIYNERELLNSMNISQPTVVFVSKK<br>GLQKILNVQKKLPIIQKIIIMDSKTDYQGFQSMYTFVTSHLPPGFN<br>EYDFVPESFDRDKTIALIMNSSGSTGLPKGVALPHRTACVRFSHA<br>RDPIFGNQIIPDTAILSVVPFHHGFGMFTTLGYLICGFRVVLMYRF<br>EEELFLRSLQDYKIQSALLVPTLFSFFAKSTLIDKYDLSNLHEIAS<br>GGAPLSKEVGEAVAKRFHLPGIRQGYGLTETTSAILITPEGDDKP<br>GAVGKVVPFFEAKVVDLDTGKTLGVNQRGELCVRGPMIMSGY<br>VNNPEATNALIDKDGWLHSGDIAYWDEDEHFFIVDRLKSLIKYK<br>GYQVAPAELESILLQHPNIFDAGVAGLPDDDAGELPAAVVVLEH<br>GKTMTEKEIVDYVASQVTTAKKLRGGVVFVDEVPKGLTGKLDA<br>RKIREILIKAKKGGKSKL (SEQ ID NO:112) |
| | catalase | MADSRDPASDQMQHWKEQRAAQKADVLTTGAGNPVGDKLNV<br>ITVGPRGPLLVQDVVFTDEMAHFDRERIPERVVHAKGAGAFGYF<br>EVTHDITKYSKAKVFEHIGKKTPIAVRFSTVAGESGSADTVRDPR<br>GFAVKFYTEDGNWDLVGNNTPIFFIRDPILFPSFIHSQKRNPQTHL<br>KDPDMVWDFWSLRPESLHQVSFLFSDRGIPDGHRHMNGYGSHT<br>FKLVNANGEAVYCKFHYKTDQGIKNLSVEDAARLSQEDPDYGI<br>RDLFNAIATGKYPSWTFYIQVMTFNQAETFPFNPFDLTKVWPHK<br>DYPLIPVGKLVLNRNPVNYFAEVEQIAFDPSNMPPGIEASPDKML<br>QGRLFAYPDTHRHRLGPNYLHIPVNCPYRARVANYQRDGPMC<br>MQDNQGGAPNYYPNSFGAPEQQPSALEHSIQYSGEVRRFNTAN<br>DDNVTQVRAFYVNVLNEEQRKRLCENIAGHLKDAQIFIQKKAV<br>KNFTEVHPDYGSHIQALLDKYNAEKPKNAIHTFVQSGSHLAARE<br>KANL (SEQ ID NO:113) |
| | ovalbumin | MGSIGAASMEFCFDVFKELKVHHANENIFYCPIAIMSALAMVYL<br>GAKDSTRTQINKVVRFDKLPGFGDSIEAQCGTSVNVHSSLRDILN<br>QITKPNDVYSFSLASRLYAEERYPILPEYLQCVKELYRGGLEPINF<br>QTAADQARELINSWVESQTNGIIRNVLQPSSVDSQTAMVLVNAI<br>VFKGLWEKAFKDEDTQAMPFRVTEQESKPVQMMYQIGLFRVAS<br>MASEKMKILELPFASGTMSMLVLLPDEVSGLEQLESIINFEKLTE<br>WTSSNVMEERKIKVYLPRMKMEEKYNLTSVLMAMGITDVFSSS<br>ANLSGISSAESLKISQAVHAAHAEINEAGREVVGSAEAGVDAAS<br>VSEEFRADHPFLFCIKHIATNAVLFFGRCVSP (SEQ ID NO: 114) |
| | Flagellin peptide (flg22) | QRLSTGSRINSAKDDAAGLQIA (SEQ ID NO:115) |
| | GUS | MARGSAVAWAALGPLLWGCALGLQGGMLYPQESPSRECKELD<br>GLWSFRADFSDNRRRGFEEQWYRRPLWESGPTVDMPVPSSFNDI<br>SQDWRLRHFVGWVWYEREVILPERWTQDLRTRVVLRIGSAHSY<br>AIVWVNGVDTLEHEGGYLPFEADISNLVQVGPLPSRLRITIAINN<br>TLTPTTLPPGTIQYLTDTSKYPKGYFVQNTYFDFFNYAGLQRSVL<br>LYTTPTTYIDDITVTTSVEQDSGLVNYQISVKGSNLFKLEVRLLD |

(continued)

| Payload | | SEQUENCE/SEQ ID or formula |
|---|---|---|
| | | AENKVVANGTGTQGQLKVPGVSLWWPYLMHERPAYLYSLEVQ LTAQTSLGPVSDFYTLPVGIRTVAVTKSQFLINGKPFYFHGVNKH EDADIRGKGFDWPLLVKDFNLLRWLGANAFRTSHYPYAEEVM QMCDRYGIVVIDECPGVGLALPQFFNNVSLHHHMQVMEEVVRR DKNHPAVVMWSVANEPASHLESAGYYLKMVIAHTKSLDPSRPV TFVSNSNYAADKGAPYVDVICLNSYYSWYHDYGHLELIQLQLA TQFENWYKKYQKPIIQSEYGAETIAGFHQDPPLMFTEEYQKSLLE QYHLGLDQKRRKYVVGELIWNFADFMTEQSPTRVLGNKKGIFT RQRQPKSAAFLLRERYWKIANETRYPHSVAKSQCLENSLFT (SEQ ID NO: 116) |
| DNA | Plasmid pcDNA3.1 | GACGGATCGGGAGATCTCCCGATCCCCTATGGTGCACTCTCA GTACAATCTGCTCTGATGCCGCATAGTTAAGCCAGTATCTGCT CCCTGCTTGTGTGTTGGAGGTCGCTGAGTAGTGCGCGAGCAA AATTTAAGCTACAACAAGGCAAGGCTTGACCGACAATTGCAT GAAGAATCTGCTTAGGGTTAGGCGTTTTGCGCTGCTTCGCGAT GTACGGGCCAGATATACGCGTTGACATTGATTATTGACTAGT TATTAATAGTAATCAATTACGGGGTCATTAGTTCATAGCCCAT ATATGGAGTTCCGCGTTACATAACTTACGGTAAATGGCCCGC CTGGCTGACCGCCCAACGACCCCCGCCCATTGACGTCAATAA TGACGTATGTTCCCATAGTAACGCCAATAGGGACTTTCCATTG ACGTCAATGGGTGGAGTATTTACGGTAAACTGCCCACTTGGC AGTACATCAAGTGTATCATATGCCAAGTACGCCCCCTATTGA CGTCAATGACGGTAAATGGCCCGCCTGGCATTATGCCCAGTA CATGACCTTATGGGACTTTCCTACTTGGCAGTACATCTACGTA TTAGTCATCGCTATTACCATGGTGATGCGGTTTTGGCAGTACA TCAATGGGCGTGGATAGCGGTTTGACTCACGGGGATTTCCAA GTCTCCACCCCATTGACGTCAATGGGAGTTTGTTTTGGCACCA AAATCAACGGGACTTTCCAAAATGTCGTAACAACTCCGCCCC ATTGACGCAAATGGGCGGTAGGCGTGTACGGTGGGAGGTCTA TATAAGCAGAGCTCTCTGGCTAACTAGAGAACCCACTGCTTA CTGGCTTATCGAAATTAATACGACTCACTATAGGGAGACCCA AGCTGGCTAGCGTTTAAACTTAAGCTTGGTACCGAGCTCGGA TCCACTAGTCCAGTGTGGTGGAATTCTGCAGATATCCAGCAC AGTGGCGGCCGCTCGAGTCTAGAGGGCCCGTTTAAACCCGCT GATCAGCCTCGACTGTGCCTTCTAGTTGCCAGCCATCTGTTGT TTGCCCCTCCCCCGTGCCTTCCTTGACCCTGGAAGGTGCCACT CCCACTGTCCTTTCCTAATAAAATGAGGAAATTGCATCGCATT GTCTGAGTAGGTGTCATTCTATTCTGGGGGGTGGGGTGGGGC AGGACAGCAAGGGGGAGGATTGGGAAGACAATAGCAGGCAT GCTGGGGATGCGGTGGGCTCTATGGCTTCTGAGGCGGAAAGA ACCAGCTGGGGCTCTAGGGGGTATCCCCACGCGCCCTGTAGC GGCGCATTAAGCGCGGCGGGTGTGGTGGTTACGCGCAGCGTG ACCGCTACACTTGCCAGCGCCCTAGCGCCCGCTCCTTTCGCTT TCTTCCCTTCCTTTCTCGCCACGTTCGCCGGCTTTCCCCGTCAA GCTCTAAATCGGGGGCTCCCTTTAGGGTTCCGATTTAGTGCTT TACGGCACCTCGACCCCAAAAAACTTGATTAGGGTGATGGTT CACGTAGTGGGCCATCGCCCTGATAGACGGTTTTTCGCCCTTT GACGTTGGAGTCCACGTTCTTTAATAGTGGACTCTTGTTCCAA ACTGGAACAACACTCAACCCTATCTCGGTCTATTCTTTTGATT TATAAGGGATTTTGCCGATTTCGGCCTATTGGTTAAAAAATG AGCTGATTTAACAAAAATTTAACGCGAATTAATTCTGTGGAA TGTGTGTCAGTTAGGGTGTGGAAAGTCCCCAGGCTCCCCAGC |

(continued)

| Payload | | SEQUENCE/SEQ ID or formula |
|---|---|---|
| | | AGGCAGAAGTATGCAAAGCATGCATCTCAATTAGTCAGCAAC CAGGTGTGGAAAGTCCCCAGGCTCCCCAGCAGGCAGAAGTAT GCAAAGCATGCATCTCAATTAGTCAGCAACCATAGTCCCGCC CCTAACTCCGCCCATCCCGCCCCTAACTCCGCCCAGTTCCGCC CATTCTCCGCCCCATGGCTGACTAATTTTTTTTATTTATGCAG AGGCCGAGGCCGCCTCTGCCTCTGAGCTATTCCAGAAGTAGT GAGGAGGCTTTTTTGGAGGCCTAGGCTTTTGCAAAAAGCTCC CGGGAGCTTGTATATCCATTTTCGGATCTGATCAAGAGACAG GATGAGGATCGTTTCGCATGATTGAACAAGATGGATTGCACG CAGGTTCTCCGGCCGCTTGGGTGGAGAGGCTATTCGGCTATG ACTGGGCACAACAGACAATCGGCTGCTCTGATGCCGCCGTGT TCCGGCTGTCAGCGCAGGGGCGCCCGGTTCTTTTTGTCAAGA CCGACCTGTCCGGTGCCCTGAATGAACTGCAGGACGAGGCAG CGCGGCTATCGTGGCTGGCCACGACGGGCGTTCCTTGCGCAG CTGTGCTCGACGTTGTCACTGAAGCGGGAAGGGACTGGCTGC TATTGGGCGAAGTGCCGGGGCAGGATCTCCTGTCATCTCACC TTGCTCCTGCCGAGAAAGTATCCATCATGGCTGATGCAATGC GGCGGCTGCATACGCTTGATCCGGCTACCTGCCCATTCGACC ACCAAGCGAAACATCGCATCGAGCGAGCACGTACTCGGATG GAAGCCGGTCTTGTCGATCAGGATGATCTGGACGAAGAGCAT CAGGGGCTCGCGCCAGCCGAACTGTTCGCCAGGCTCAAGGCG CGCATGCCCGACGGCGAGGATCTCGTCGTGACCCATGGCGAT GCCTGCTTGCCGAATATCATGGTGGAAAATGGCCGCTTTTCTG GATTCATCGACTGTGGCCGGCTGGGTGTGGCGGACCGCTATC AGGACATAGCGTTGGCTACCCGTGATATTGCTGAAGAGCTTG GCGGCGAATGGGCTGACCGCTTCCTCGTGCTTTACGGTATCG CCGCTCCCGATTCGCAGCGCATCGCCTTCTATCGCCTTCTTGA CGAGTTCTTCTGAGCGGGACTCTGGGGTTCGAAATGACCGAC CAAGCGACGCCCAACCTGCCATCACGAGATTTCGATTCCACC GCCGCCTTCTATGAAAGGTTGGGCTTCGGAATCGTTTTCCGGG ACGCCGGCTGGATGATCCTCCAGCGCGGGGATCTCATGCTGG AGTTCTTCGCCCACCCCAACTTGTTTATTGCAGCTTATAATGG TTACAAATAAAGCAATAGCATCACAAATTTCACAAATAAAGC ATTTTTTTCACTGCATTCTAGTTGTGGTTTGTCCAAACTCATCA ATGTATCTTATCATGTCTGTATACCGTCGACCTCTAGCTAGAG CTTGGCGTAATCATGGTCATAGCTGTTTCCTGTGTGAAATTGT TATCCGCTCACAATTCCACACAACATACGAGCCGGAAGCATA AAGTGTAAAGCCTGGGGTGCCTAATGAGTGAGCTAACTCACA TTAATTGCGTTGCGCTCACTGCCCGCTTTCCAGTCGGGAAACC TGTCGTGCCAGCTGCATTAATGAATCGGCCAACGCGCGGGGA GAGGCGGTTTGCGTATTGGGCGCTCTTCCGCTTCCTCGCTCAC TGACTCGCTGCGCTCGGTCGTTCGGCTGCGGCGAGCGGTATC AGCTCACTCAAAGGCGGTAATACGGTTATCCACAGAATCAGG GGATAACGCAGGAAAGAACATGTGAGCAAAAGGCCAGCAAA AGGCCAGGAACCGTAAAAAGGCCGCGTTGCTGGCGTTTTTCC ATAGGCTCCGCCCCCCTGACGAGCATCACAAAAATCGACGCT CAAGTCAGAGGTGGCGAAACCCGACAGGACTATAAAGATAC CAGGCGTTTCCCCCTGGAAGCTCCCTCGTGCGCTCTCCTGTTC CGACCCTGCCGCTTACCGGATACCTGTCCGCCTTTCTCCCTTC GGGAAGCGTGGCGCTTTCTCATAGCTCACGCTGTAGGTATCT CAGTTCGGTGTAGGTCGTTCGCTCCAAGCTGGGCTGTGTGCA CGAACCCCCCGTTCAGCCCGACCGCTGCGCCTTATCCGGTAA |

(continued)

| Payload | | SEQUENCE/SEQ ID or formula |
|---|---|---|
| | | CTATCGTCTTGAGTCCAACCCGGTAAGACACGACTTATCGCC ACTGGCAGCAGCCACTGGTAACAGGATTAGCAGAGCGAGGT ATGTAGGCGGTGCTACAGAGTTCTTGAAGTGGTGGCCTAACT ACGGCTACACTAGAAGAACAGTATTTGGTATCTGCGCTCTGC TGAAGCCAGTTACCTTCGGAAAAAGAGTTGGTAGCTCTTGAT CCGGCAAACAAACCACCGCTGGTAGCGGTTTTTTTGTTTGCA AGCAGCAGATTACGCGCAGAAAAAAAGGATCTCAAGAAGAT CCT TTGATCTTTTCTACGGGGTCTGACGCTCAGTGGAACGAAAAC TCACGTTAAGGGATTTTGGTCATGAGATTATCAAAAAGGATC TTCACCTAGATCCTTTTAAATTAAAAATGAAGTTTTAAATCAA TCTAAAGTATATATGAGTAAACTTGGTCTGACAGTTACCAAT GCTTAATCAGTGAGGCACCTATCTCAGCGATCTGTCTATTTCG TTCATCCATAGTTGCCTGACTCCCCGTCGTGTAGATAACTACG ATACGGGAGGGCTTACCATCTGGCCCCAGTGCTGCAATGATA CCGCGAGACCCACGCTCACCGGCTCCAGATTTATCAGCAATA AACCAGCCAGCCGGAAGGGCCGAGCGCAGAAGTGGTCCTGC AACTTTATCCGCCTCCATCCAGTCTATTAATTGTTGCCGGGAA GCTAGAGTAAGTAGTTCGCCAGTTAATAGTTTGCGCAACGTT GTTGCCATTGCTACAGGCATCGTGGTGTCACGCTCGTCGTTTG GTATGGCTTCATTCAGCTCCGGTTCCCAACGATCAAGGCGAG TTACATGATCCCCCATGTTGTGCAAAAAAGCGGTTAGCTCCTT CGGTCCTCCGATCGTTGTCAGAAGTAAGTTGGCCGCAGTGTT ATCACTCATGGTTATGGCAGCACTGCATAATTCTCTTACTGTC ATGCCATCCGTAAGATGCTTTTCTGTGACTGGTGAGTACTCAA CCAAGTCATTCTGAGAATAGTGTATGCGGCGACCGAGTTGCT CTTGCCCGGCGTCAATACGGGATAATACCGCGCCACATAGCA GAACTTTAAAAGTGCTCATCATTGGAAAACGTTCTTCGGGGC GAAAACTCTCAAGGATCTTACCGCTGTTGAGATCCAGTTCGA TGTAACCCACTCGTGCACCCAACTGATCTTCAGCATCTTTTAC TTTCACCAGCGTTTCTGGGTGAGCAAAAACAGGAAGGCAAAA TGCCGCAAAAAAGGGAATAAGGGCGACACGGAAATGTTGAA TACTCATACTCTTCCTTTTTCAATATTATTGAAGCATTTATCAG GGTTATTGTCTCATGAGCGGATACATATTTGAATGTATTTAGA AAAATAAACAAATAGGGGTTCCGCGCACATTTCCCCGAAAAG TGCCACCTGACGTC (SEQ ID NO: 117) |
| | Plasmid pEGFP-N1 | TAGTTATTAATAGTAATCAATTACGGGGTCATTAGTTCATAGC CCATATATGGAGTTCCGCGTTACATAACTTACGGTAAATGGC CCGCCTGGCTGACCGCCCAACGACCCCCGCCCATTGACGTCA ATAATGACGTATGTTCCCATAGTAACGCCAATAGGGACTTTC CATTGACGTCAATGGGTGGAGTATTTACGGTAAACTGCCCAC TTGGCAGTACATCAAGTGTATCATATGCCAAGTACGCCCCCT ATTGACGTCAATGACGGTAAATGGCCCGCCTGGCATTATGCC CAGTACATGACCTTATGGGACTTTCCTACTTGGCAGTACATCT ACGTATTAGTCATCGCTATTACCATGGTGATGCGGTTTTGGCA GTACATCAATGGGCGTGGATAGCGGTTTGACTCACGGGGATT TCCAAGTCTCCACCCCATTGACGTCAATGGGAGTTTGTTTTGG CACCAAAATCAACGGGACTTTCCAAAATGTCGTAACAACTCC GCCCCATTGACGCAAATGGGCGGTAGGCGTGTACGGTGGGAG GTCTATATAAGCAGAGCTGGTTTAGTGAACCGTCAGATCCGC TAGCGCTACCGGACTCAGATCTCGAGCTCAAGCTTCGAATTC TGCAGTCGACGGTACCGCGGGCCCGGGATCCACCGGTCGCCA |

(continued)

| Payload | | SEQUENCE/SEQ ID or formula |
|---|---|---|
| | | CCATGGTGAGCAAGGGCGAGGAGCTGTTCACCGGGGTGGTGC CCATCCTGGTCGAGCTGGACGGCGACGTAAACGGCCACAAGT TCAGCGTGTCCGGCGAGGGCGAGGGCGATGCCACCTACGGCA AGCTGACCCTGAAGTTCATCTGCACCACCGGCAAGCTGCCCG TGCCCTGGCCCACCCTCGTGACCACCCTGACCTACGGCGTGC AGTGCTTCAGCCGCTACCCCGACCACATGAAGCAGCACGACT TCTTCAAGTCCGCCATGCCCGAAGGCTACGTCCAGGAGCGCA CCATCTTCTTCAAGGACGACGGCAACTACAAGACCCGCGCCG AGGTGAAGTTCGAGGGCGACACCCTGGTGAACCGCATCGAGC TGAAGGGCATCGACTTCAAGGAGGACGGCAACATCCTGGGG CACAAGCTGGAGTACAACTACAACAGCCACAACGTCTATATC ATGGCCGACAAGCAGAAGAACGGCATCAAGGTGAACTTCAA GATCCGCCACAACATCGAGGACGGCAGCGTGCAGCTCGCCGA CCACTACCAGCAGAACACCCCCATCGGCGACGGCCCCGTGCT GCTGCCCGACAACCACTACCTGAGCACCCAGTCCGCCCTGAG CAAAGACCCCAACGAGAAGCGCGATCACATGGTCCTGCTGGA GTTCGTGACCGCCGCCGGGATCACTCTCGGCATGGACGAGCT GTACAAGTAAAGCGGCCGCGACTCTAGATCATAATCAGCCAT ACCACATTTGTAGAGGTTTTACTTGCTTTAAAAAACCTCCCAC ACCTCCCCCTGAACCTGAAACATAAAATGAATGCAATTGTTG TTGTTAACTTGTTTATTGCAGCTTATAATGGTTACAAATAAAG CAATAGCATCACAAATTTCACAAATAAAGCATTTTTTTCACTG CATTCTAGTTGTGGTTTGTCCAAACTCATCAATGTATCTTAAG GCGTAAATTGTAAGCGTTAATATTTTGTTAAAATTCGCGTTAA ATTTTTGTTAAATCAGCTCATTTTTTAACCAATAGGCCGAAAT CGGCAAAATCCCTTATAAATCAAAAGAATAGACCGAGATAG GGTTGAGTGTTGTTCCAGTTTGGAACAAGAGTCCACTATTAA AGAACGTGGACTCCAACGTCAAAGGGCGAAAAACCGTCTATC AGGGCGATGGCCCACTACGTGAACCATCACCCTAATCAAGTT TTTGTGCCGTAAAGCACTAAATCGGAACCCTAAAGGGAGCCC CCGATTTAGAGCTTGACGGGGAAAGCCGGCGAACGTGGCGA GAAAGGAAGGGAAGAAAGCGAAAGGAGCGGGCGCTAGGGC GCTGGCAAGTGTAGCGGTCACGCTGCGCGTAACCACCACACC CGCCGCGCTTAATGCGCCGCTACAGGGCGCGTCAGGTGGCAC TTTTCGGGGAAATGTGCGCGGAACCCCTATTTGTTTATTTTTC TAAATACATTCAAATATGTATCCGCTCATGAGACAATAACCC TGATAAATGCTTCAATAATATTGAAAAAGGAAGAGTCCTGAG GCGGAAAGAACCAGCTGTGGAATGTGTGTCAGTTAGGGTGTG GAAAGTCCCCAGGCTCCCCAGCAGGCAGAAGTATGCAAAGC ATGCATCTCAATTAGTCAGCAACCAGGTGTGGAAAGTCCCCA GGCTCCCCAGCAGGCAGAAGTATGCAAAGCATGCATCTCAAT TAGTCAGCAACCATAGTCCCGCCCCTAACTCCGCCCATCCCG CCCCTAACTCCGCCCAGTTCCGCCCATTCTCCGCCCCATGGCT GACTAATTTTTTTTATTTATGCAGAGGCCGAGGCCGCCTCGGC CTCTGAGCTATTCCAGAAGTAGTGAGGAGGCTTTTTTGGAGG CCTAGGCTTTTGCAAAGATCGATCAAGAGACAGGATGAGGAT CGTTTCGCATGATTGAACAAGATGGATTGCACGCAGGTTCTC CGGCCGCTTGGGTGGAGAGGCTATTCGGCTATGACTGGGCAC AACAGACAATCGGCTGCTCTGATGCCGCCGTGTTCCGGCTGT CAGCGCAGGGGCGCCCGGTTCTTTTTGTCAAGACCGACCTGT CCGGTGCCCTGAATGAACTGCAAGACGAGGCAGCGCGGCTAT CGTGGCTGGCCACGACGGGCGTTCCTTGCGCAGCTGTGCTCG |

(continued)

| Payload | | SEQUENCE/SEQ ID or formula |
|---|---|---|
| | | ACGTTGTCACTGAAGCGGGAAGGGACTGGCTGCTATTGGGCG AAGTGCCGGGGCAGGATCTCCTGTCATCTCACCTTGCTCCTGC CGAGAAAGTATCCATCATGGCTGATGCAATGCGGCGGCTGCA TACGCTTGATCCGGCTACCTGCCCATTCGACCACCAAGCGAA ACATCGCATCGAGCGAGCACGTACTCGGATGGAAGCCGGTCT TGTCGATCAGGATGATCTGGACGAAGAGCATCAGGGGCTCGC GCCAGCCGAACTGTTCGCCAGGCTCAAGGCGAGCATGCCCGA CGGCGAGGATCTCGTCGTGACCCATGGCGATGCCTGCTTGCC GAATATCATGGTGGAAAATGGCCGCTTTTCTGGATTCATCGA CTGTGGCCGGCTGGGTGTGGCGGACCGCTATCAGGACATAGC GTTGGCTACCCGTGATATTGCTGAAGAGCTTGGCGGCGAATG GGCTGACCGCTTCCTCGTGCTTTACGGTATCGCCGCTCCCGAT TCGCAGCGCATCGCCTTCTATCGCCTTCTTGACGAGTTCTTCT GAGCGGGACTCTGGGGTTCGAAATGACCGACCAAGCGACGC CCAACCTGCCATCACGAGATTTCGATTCCACCGCCGCCTTCTA TGAAAGGTTGGGCTTCGGAATCGTTTTCCGGGACGCCGGCTG GATGATCCTCCAGCGCGGGGATCTCATGCTGGAGTTCTTCGC CCACCCTAGGGTGAAACACGGAAGGAGACAATACCGGAAGG AACCCGCGCTTAAAAAGACAGAATAAAACGCACGGTGTTGG GTCGTTTGTTCATAAACGCGGGGTTCGGTCCCAGGGCTGGCA CTCTGTCGATACCCCACCGAGACCCCATTGGGGCCAATACGC CCGCGTTTCTTCCTTTTCCCCACCCCACCCCCCAAGTTCGGGT GAAGGCCCAGGGCTCGCAGCCAACGTCGGGGCGGCAGGCCC TGCCATAGCCTCAGGTTACTCATATATACTTTAGATTGATTTA AAACTTCATTTTTAATTTAAAAGGATCTAGGTGAAGATCCTTT TTGATAATCTCATGACCAAAATCCCTTAACGTGAGTTTTCGTT CCACTGAGCGTCAGACCCCGTAGAAAAGATCAAAGGATCTTC TTGAGATCCTTTTTTTCTGCGCGTAATCTGCTGCTTGCAAACA AAAAAACCACCGCTACCAGCGGTGGTTTGTTTGCCGGATCAA GAGCTACCAACTCTTTTTCCGAAGGTAACTGGCTTCAGCAGA GCGCAGATACCAAATACTGTCCTTCTAGTGTAGCCGTAGTTA GGCCACCACTTCAAGAACTCTGTAGCACCGCCTACATACCTC GCTCTGCTAATCCTGTTACCAGTGGCTGCTGCCAGTGGCGATA AGTCGTGTCTTACCGGGTTGGACTCAAGACGATAGTTACCGG ATAAGGCGCAGCGGTCGGGCTGAACGGGGGGTTCGTGCACA CAGCCCAGCTTGGAGCGAACGACCTACACCGAACTGAGATAC CTACAGCGTGAGCTATGAGAAAGCGCCACGCTTCCCGAAGGG AGAAAGGCGGACAGGTATCCGGTAAGCGGCAGGGTCGGAAC AGGAGAGCGCACGAGGGAGCTTCCAGGGGGAAACGCCTGGT ATCTTTATAGTCCTGTCGGGTTTCGCCACCTCTGACTTGAGCG TCGATTTTTGTGATGCTCGTCAGGGGGGCGGAGCCTATGGAA AAACGCCAGCAACGCGGCCTTTTTACGGTTCCTGGCCTTTTGC TGGCCTTTTGCTCACATGTTCTTTCCTGCGTTATCCCCTGATTC TGTGGATAACCGTATTACCGCCATGCAT (SEQ ID NO:118) |
| | ASO k-ras | GCTATTAGGAGTCTTT (SEQ ID NO:119) |
| | ASO c-myc | AACGTTGAGGGGCAT (SEQ ID NO:120) |

(continued)

| Payload | | SEQUENCE/SEQ ID or formula |
|---|---|---|
| RNA | mRNA (eGFP) | AUGGUGAGCA AGGGCGAGGA GCUGUUCACC GGGUGGUGC CCAUCCUGGU CGAGCUGGAC GGCGACGUAA ACGGCCACAA GUUCAGCGUG UCCGGCGAGG GCGAGGGCGA UGCCACCUAC GGCAAGCUGA CCCUGAAGUU CAUCUGCACC ACCGGCAAGC UGCCCGUGCC CUGGCCCACC CUCGUGACCA CCCUGACCUA CGGCGUGCAG UGCUUCAGCC GCUACCCCGA CCACAUGAAG CAGCACGACU UCUUCAAGUC CGCCAUGCCC GAAGGCUACG UCCAGGAGCG CACCAUCUUC UUCAAGGACG ACGGCAACUA CAAGACCCGC GCCGAGGUGA AGUUCGAGGG CGACACCCUG GUGAACCGCA UCGAGCUGAA GGGCAUCGACUUCAAGGAGG ACGGCAACAU CCUGGGGCAC AGCUGGAGU ACAACUACAA CAGCCACAAC GUCUAUAUCA UGGCCGACAA GCAGAAGAAC GGCAUCAAGGUGAACUUCAA GAUCCGCCAC AACAUCGAGG ACGGCAGCGU GCAGCUCGCC GACCACUACC AGCAGAACAC CCCCAUCGGC GACGGCCCCG UGCUGCUGCC CGACAACCAC UACCUGAGCA CCCAGUCCGC CCUGAGCAAA GACCCCAACG AGAAGCGCGA UCACAUGGUC CUGCUGGAGU UCGUGACCGC CGCCGGGAUC ACUCUCGGCA UGGACGAGCU GUACAAGUAA (SEQ ID NO:121) |

(continued)

| Payload | | SEQUENCE/SEQ ID or formula |
|---|---|---|
| | mRNA (luciferase) | ATGGAAGACGCCAAAAACATAAAGAAAGGCCCGGCGCCATT CTATCCTCTAGAGGATGGAACCGCTGGAGAGCAACTGCATAA GGCTATGAAGAGATACGCCCTGGTTCCTGGAACAATTGCTTT TACAGATGCACATATCGAGGTGAACATCACGTACGCGGAATA CTTCGAAATGTCCGTTCGGTTGGCAGAAGCTATGAAACGATA TGGGCTGAATACAAATCACAGAATCGTCGTATGCAGTGAAAA CTCTCTTCAATTCTTTATGCCGGTGTTGGGCGCGTTATTTATC GGAGTTGCAGTTGCGCCCGCGAACGACATTTATAATGAACGT GAATTGCTCAACAGTATGAACATTTCGCAGCCTACCGTAGTG TTTGTTTCCAAAAAGGGGTTGCAAAAAATTTTGAACGTGCAA AAAAAATTACCAATAATCCAGAAAATTATTATCATGGATTCT AAAACGGATTACCAGGGATTTCAGTCGATGTACACGTTCGTC ACATCTCATCTACCTCCCGGTTTTAATGAATACGATTTTGTAC CAGAGTCCTTTGATCGTGACAAAACAATTGCACTGATAATGA ATTCCTCTGGATCTACTGGGTTACCTAAGGGTGTGGCCCTTCC GCATAGAACTGCCTGCGTCAGATTCTCGCATGCCAGAGATCC TATTTTTGGCAATCAAATCATTCCGGATACTGCGATTTTAAGT GTTGTTCCATTCCATCACGGTTTTGGAATGTTTACTACACTCG GATATTTGATATGTGGATTTCGAGTCGTCTTAATGTATAGATT TGAAGAAGAGCTGTTTTTACGATCCCTTCAGGATTACAAAAT TCAAAGTGCGTTGCTAGTACCAACCCTATTTTCATTCTTCGCC AAAAGCACTCTGATTGACAAATACGATTTATCTAATTTACAC GAAATTGCTTCTGGGGGCGCACCTCTTTCGAAAGAAGTCGGG GAAGCGGTTGCAAAACGCTTCCATCTTCCAGGGATACGACAA GGATATGGGCTCACTGAGACTACATCAGCTATTCTGATTACA CCCGAGGGGGATGATAAACCGGGCGCGGTCGGTAAAGTTGTT CCATTTTTTGAAGCGAAGGTTGTGGATCTGGATACCGGGAAA ACGCTGGGCGTTAATCAGAGAGGCGAATTATGTGTCAGAGGA CCTATGATTATGTCCGGTTATGTAAACAATCCGGAAGCGACC AACGCCTTGATTGACAAGGATGGATGGCTACATTCTGGAGAC ATAGCTTACTGGGACGAAGACGAACACTTCTTCATAGTTGAC CGCTTGAAGTCTTTAATTAAATACAAAGGATATCAGGTGGCC CCCGCTGAATTGGAATCGATATTGTTACAACACCCCAACATC TTCGACGCGGGCGTGGCAGGTCTTCCCGACGATGACGCCGGT GAACTTCCCGCCGCCGTTGTTGTTTTGGAGCACGGAAAGACG ATGACGGAAAAAGAGATCGTGGATTACGTCGCCAGTCAAGTA ACAACCGCGAAAAAGTTGCGCGGAGGAGTTGTGTTTGTGGAC GAAGTACCGAAAGGTCTTACCGGAAAACTCGACGCAAGAAA AATCAGAGAGATCCTCATAAAGGCCAAGAAGGGCGGAAAGT CCAAATTGTAA (SEQ ID NO:122) |

(continued)

| Payload | | SEQUENCE/SEQ ID or formula |
|---|---|---|
| | mRNA (GUS) | AUGUUACGUCCUGUAGAAACCCCAACCCGUGAAAUCAAAAA<br>ACUCGACGGCCUGUGGGCAUUCAGUCUGGAUCGCGAAAACU<br>GUGGAAUUGAUCAGCGUUGGUGGGAAAGCGCGUUACAAGA<br>AAGCCGGGCAAUUGCUGUGCCAGGCAGUUUUAACGAUCAG<br>UUCGCCGAUGCAGAUAUUCGUAAUUAUGCGGGCAACGUCU<br>GGUAUCAGCGCGAAGUCUUUAUACCGAAAGGUUGGGCAGG<br>CCAGCGUAUCGUGCUGCGUUUCGAUGCGGUCACUCAUUACG<br>GCAAAGUGUGGGUCAAUAAUCAGGAAGUGAUGGAGCAUCA<br>GGGCGGCUAUACGCCAUUUGAAGCCGAUGUCACGCCGUAUG<br>UUAUUGCCGGGAAAAGUGUACGUAUCACCGUUUGUGUGAA<br>CAACGAACUGAACUGGCAGACUAUCCCGCCGGGAAUGGUGA<br>UUACCGACGAAAACGGCAAGAAAAAGCAGUCUUACUUCCAU<br>GAUUUCUUUAACUAUGCCGGAAUCCAUCGCAGCGUAAUGCU<br>CUACACCACGCCGAACACCUGGGUGGACGAUAUCACCGUGG<br>UGACGCAUGUCGCGCAAGACUGUAACCACGCGUCUGUUGAC<br>UGGCAGGUGGUGGCCAAUGGUGAUGUCAGCGUUGAACUGC<br>GUGAUGCGGAUCAACAGGUGGUUGCAACUGGACAAGGCAC<br>UAGCGGGACUUUGCAAGUGGUGAAUCCGCACCUCUGGCAAC<br>CGGGUGAAGGUUAUCUCUAUGAACUGUGCGUCACAGCCAA<br>AAGCCAGACAGAGUGUGAUAUCUACCCGCUUCGCGUCGGCA<br>UCCGGUCAGUGGCAGUGAAGGGCCAACAGUUCCUGAUUAAC<br>CACAAACCGUUCUACUUUACUGGCUUUGGUCGUCAUGAAGA<br>UGCGGACUUACGUGGCAAAGGAUUCGAUAACGUGCUGAUG<br>GUGCACGACCACGCAUUAAUGGACUGGAUUGGGGCCAACUC<br>CUACCGUACCUCGCAUUACCCUUACGCUGAAGAGAUGCUCG<br>ACUGGGCAGAUGAACAUGGCAUCGUGGUGAUUGAUGAAAC<br>UGCUGCUGUCGGCUUUAACCUCUCUUUAGGCAUUGGUUUCG<br>AAGCGGGCAACAAGCCGAAAGAACUGUACAGCGAAGAGGC<br>AGUCAACGGGGAAACUCAGCAAGCGCACUUACAGGCGAUUA<br>AAGAGCUGAUAGCGCGUGACAAAAACCACCCAAGCGUGGUG<br>AUGUGGAGUAUUGCCAACGAACCGGAUACCCGUCCGCAAGU<br>GCACGGGAAUAUUUCGCCACUGGCGGAAGCAACGCGUAAAC<br>UCGACCCGACGCGUCCGAUCACCUGCGUCAAUGUAAUGUUC<br>UGCGACGCUCACACCGAUACCAUCAGCGAUCUCUUUGAUGU<br>GCUGUGCCUGAACCGUUAUUACGGAUGGUAUGUCCAAAGC<br>GGCGAUUUGGAAACGGCAGAGAAGGUACUGGAAAAAGAAC<br>UUCUGGCCUGGCAGGAGAAACUGCAUCAGCCGAUUAUCAUC<br>ACCGAAUACGGCGUGGAUACGUUAGCCGGGCUGCACUCAAU<br>GUACACCGACAUGUGGAGUGAAGAGUAUCAGUGUGCAUGG<br>CUGGAUAUGUAUCACCGCGUCUUUGAUCGCGUCAGCGCCGU<br>CGUCGGUGAACAGGUAUGGAAUUUCGCCGAUUUUGCGACC<br>UCGCAAGGCAUAUUGCGCGUUGGCGGUAACAAGAAAGGGA<br>UCUUCACUCGCGACCGCAAACCGAAGUCGCGGCUUUUCUGC<br>UGCAAAAACGCUGGACUGGCAUGAACUUCGGUGAAAAACC<br>GCAGCAGGGAGGCAAACAA (SEQ ID NO:123) |
| | siRNA k-ras | GUCUCUUGGAUAUUCUCGA (Sense) (SEQ ID NO:124) UCGAGAAUACCCAAGAGAC (Antisense) (SEQ ID NO:125) |
| | miRNA c-myc | CAAAAACCGCAAUUACUUUUGCA (Sense) (SEQ ID NO:126) UGCAAAAGUAAUUGAGAUUUUUG (Antisense) (SEQ ID NO:127) |
| | siRNA mixed population | Mixture of synthetic siRNAs generated by T7 polymerase from *cfa6* gen and *hrpL* gen (see example 12) |

(continued)

| Payload | | SEQUENCE/SEQ ID or formula |
|---|---|---|
| Small molecule | Rhodamine | 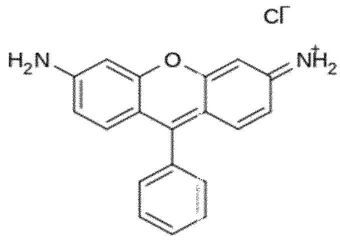 |

EXAMPLE 5

BIODISTRIBUTION STUDIES

A. *In Vivo* Biodistribution of MEVs

Experiments were performed to determine the pathway and fate of MEVs when administered via various routes.

1. MEV labelling using DiR fluorescent

As described in example 2, the fluorescent, lipophilic carbocyanine DiR (1,1-dioctadecyl-3,3,3,3-tetramethylindotricarbocyanine iodide) is weakly fluorescent in water but highly fluorescent and photostable when incorporated into membranes and can be tracked *in vivo* (see, Example 2 above; ThermoFisher Scientific). The DiR labelled (MEVs) are incubated with human cells. Their uptake by human cells is measured by fluorimetry analysis (fluorescence spectroscopy) on microplate readers. DiR has an excitation of 750 nm and an emission of 780 nm.

The methodology for the biodistribution studies is summarized as follows: *Chlorella* cells were grown in the photobioreactor as described above (120L per batch), were isolated and labeled with DiR, and labelling efficiency was assessed as described in Examples 1 and 2.

2. Biodistribution of MEVs in mice

The labeled MEVs were administered to the mouse model animals via one of four routes, via: intranasal, intratracheal, oral, and intravenous administration, to determine the pathways and fate of the MEVs by each route. Treated mice were subjected to full body imaging as a function of time, and at day 3 mice were sacrificed and the organs were imaged *ex vivo,* and by histology in several mice organs after different routes of application.

Animal model

MEVs' pharmacokinetics and biodistribution were studied in Specific Pathogen Free (SPF), 7-week old male Balb/cByJ mice (Charles River Laboratories). The animals were labelled with unique ear ID tags and acclimatized for at least 2 days. Background (control) mice were housed individually, while MEV-treated mice were housed collectively in disposable standard cages in ventilated racks at 21±3°C (temperature recorded and controlled) with a 12hr-12hr light/dark cycle. Filtered water and low fluorescence laboratory food for rodents were provided *ad libitum.* All mice were euthanized at the end of the *in vivo* experiments.

***In vivo* administration of DiR-labelled MEV for biodistribution studies**

The DiR-labelled MEVs (prepared as above in Example 2) were used to determine the biodistribution of the MEVs in the whole animal body or in several organs after different routes of application.

Prior to the imaging, the fur of each animal was clipped using an electric clipper in the following areas: abdomen, thorax, head, and whole back. Care was taken in order to clip the fur as homogeneously as possible. Next, DiR-labelled MEV preparations were re-suspended by vortexing before filling the syringes for injection.

## Routes of administration

The following routes were used to administer the MEVs to the animals:

- **Intravenous (*IV*):** 50 μL of MEV suspension containing $0.6x10^{12}$ MEV/mL were injected in a tail vein by disposable plastic syringe and appropriate needle. Dosage of MEV administered: $3x10^{10}$ MEV/mouse.
- **Intranasal *(IN):*** Animals were induced and maintained under anesthesia during IN administration by a mixture of isoflurane and oxygen as a carrier gas. A volume of 100 μL of MEV suspension containing $0.3\times10^{12}$ MEVs/mL was administered into the nostrils of the mouse using a thin pipette cone. Dosage of MEV administered: $3x10^{10}$ MEV/mouse.
- ***Per os (PO):*** 100 μL of MEV suspension containing $0.3\times10^{12}$ MEVs/mL was administered orally using a disposable plastic syringe and an appropriate feeding probe. Dosage of MEV administered: $3x10^{10}$ MEV/mouse.
- **Intratracheal (*IT*):** Animals were induced and maintained under anesthesia during IT administration by a mixture of isoflurane and oxygen as a carrier gas. Once adequate anesthesia was observed, animals were placed on a mouse intubation platform, suspended from the front incisors in the supine position, to maximize view of the trachea. Then, a cold light was placed on the skin near the trachea localization in order to backlight the trachea. If needed, a laryngoscope was inserted to guide the syringe of Microsprayer® sprayer into the trachea. An injection of 50 μL of MEV suspension containing $0.6\times10^{12}$ MEVs/mL was instilled into the trachea. Anesthesia was maintained throughout the procedure. After injection, the mice were maintained in the same position on the intubating platform for at least 30 seconds, before being replaced in their cage. Dosage of MEVs administered: $3x10^{10}$ MEV/mouse.

## *In vivo* imaging for biodistribution studies

Fluorescence acquisitions were performed with the IVIS® Spectrum optical imaging system (acquired from PerkinElmer). Bidimensional (2D) fluorescence imaging was performed by sensitive detection of light emitted by DiR-labelled MEVs. Mice were anesthetized and imaged 1 hour before MEV administration. Next, fluorescence of the material to be administered was confirmed with an *in vitro* acquisition performed on at least 1 drop of a minimum of 20 μL of MEV suspensions, deposited in 2 different Petri dishes. The fluorescence emission was measured and detected with the selected parameters (see below). Finally, *in vivo* fluorescence acquisitions were performed on mice administered MEVs. The animals were induced and maintained under anesthesia with a mixture of isoflurane and oxygen. Mice were positioned in dorsal recumbency to obtain ventral images and in ventral recumbency to obtain dorsal images. At least 2 acquisitions/mouse/timepoint were taken and mice were imaged in groups of maximum 3 mice/acquisition. Images were analyzed and fluorescence quantified on 6 organs (liver, spleen, lung, kidney, intestine, and brain).

The following timepoints were used (T0=MEV administration):

- Day 0 - 6 timepoints: T0 -1h ± 10 min; T0 +30 min ± 5 min; T0 +2 h ± 15 min; T0 +4 h ± 30 min; T0 +8 h ± 30 min; T0 +10 h ± 30 min
- Day 1 - 1 timepoint: T0 + 24 h ± 3 h
- Day 2 - 1 timepoint: T0 + 48 h ± 3 h
- Day 3 - 1 timepoint: T0 + 72 h ± 3 h

The data were analyzed with the IVIS® software (Living Image Software for IVIS®).

The following parameters were used for the in vivo and ex-vivo fluorescence acquisitions:

| | |
|---|---|
| Field of View (FOV) | 14 x 14 cm (FOV C) |
| Image number: | *In vivo:* For each mouse at each applicable timepoint, at least 2 acquisitions (ventral and dorsal)were performed; 3 mice (at maximum) were imaged in one acquisition. |

*Ex-vivo:* the organs of at least one mouse were imaged in one acquisition

| | |
|---|---|
| Image format | TIFF format |
| Fluorescent probe | DiR |
| Excitation wave-length | 745 nm |
| Emission wave-length | 800 nm |

(continued)

| | | |
|---|---|---|
| Exposition time | Automatic, depending on the fluorescence signal detected | |
| Minimum counts | 20000 | |
| Binning | Between 16 and 1 (depending on the fluorescence signal detected) | |
| F/STOP | 2 (in case of fluorescence signal saturation it was automatically increased to 4 or it was decreased to 1 in case of weak signal) | |
| Subject height | *In vivo:* | 1.5 cm |
| | *Ex-vivo:* | 0.5 cm |

### *Ex vivo* imaging for biodistribution studies

Production, purification, characterization, labelling and administration (by different routes) of MEVs was as described above. On Day 3 after administration, mice were euthanized and the organs of interest of each mouse were sampled and positioned in a Petri dish in order to perform *ex vivo* acquisitions. The following organs were sampled: liver, spleen, lungs, kidneys, brain, and intestine. The data were analyzed with the IVIS® software (Living Image Software for IVIS®). Figure 5 shows representative patterns of biodistribution according to the route of administration, for the Intravenous (IV), Intratracheal (IT) and Per os (PO) routes.

### Results, Discussion and Conclusions

### Pharmacokinetics

The pharmacokinetic curves demonstrating the accumulation as a percent of baseline over time (hours) after intravenous, Per os, intranasal, and intratracheal administration are set forth in Figures 10-13, respectively.

### Biodistribution - isolated organs- *ex vivo* imaging

Biodistribution - *in vivo* full body imaging of a representative animal is depicted in FIGs. 6-9.The biodistribution by *ex vivo* fluorescence analysis (total radiant efficiency) in liver; spleen; lungs; and brain after intravenous (IV), Per os (PO), intranasal (IN), and intratracheal (IT) administration is set forth in Figures 14A-D.

### 1. Summary of results by each route

### a. Intravenous administration

- Organs targeted by unmodified MEVs - **liver and spleen**
- PK parameters:
    - Peak/plateau time: 2-24 h (liver), 4-24 h (spleen)
    - % at t=day 3 compared to peak/plateau approximately 62% (liver), approx. 61% (spleen)
- Longer presence or duration compared to mammalian EVs (see, *e.g.,* Kang et al., Biodistribution of extracellular vesicles following administration into animals: A systematic review. J Extracell Vesicles. 2021;10:e12085. *(doi.org/10.1002/jev2.12085)*).
- No visible signs of toxicity

### b. Per os (oral) administration

- Organs targeted by following oral administration: **intestine and spleen**
- Oral availability:
    - MEVs are orally available; they resist the passage through the stomach, reach the intestine and subsequently appear in the spleen.

- Mammalian EVs are not orally available, with the exception of bovine milk EVs.

- PK parameters:
  - Peak/plateau time: 1-6 h (intestine), 1-10 h (spleen)
  - % at t=10h compared to peak/plateau: approx. 33% (intestine), approx. 44% (spleen)
  - % at t=48h compared to peak/plateau: 0% (intestine), 0% (spleen)
- No visible signs of toxicity

**c. Distribution in organs following intravenous and oral administration.**

Liver: Following intravenous administration, the liver is the primary target organ. Following oral administration, the liver is marginally targeted, if at all.

Spleen: The pharmacokinetics profile depends on whether administration is intravenous or oral (per os). Intravenous administration is longer lasting. At day 3, 50-70% of the peak is still remaining. Oral administration is shorter lasting; at 48 hours, 0% remains.

**d. Intranasal administration**

- Organs targeted by the unmodified MEVs are the **lungs**
- Preliminary PK parameters:
  - Peak/plateau time: 4-28 h
  - % at t=day 3 compared to peak/plateau: 70-80%
- No visible signs of toxicity

**e. Intratracheal administration**

- Organs targeted by the MEVs are the **lungs**
  - PK parameters:
    - Peak/plateau time: 2-72 h
    - % at t=day 3 compared to peak/plateau: approx. 80%
  - No visible signs of toxicity

**2. Biodistribution of MEVs labelled with PKH26 after per os (PO) administration in BALB/cByJ mouse.**

When MEVs are orally administrated to the mice, they follow through the digestive tract. MEVs first reach the stomach, where they resist the stringent conditions of the gastric juice. In about 30 minutes after administration, they reach the intestine, where they stay for several hours.

Once in the lumen of the intestine, as shown in Example 32, MEVs are internalized by the cells of the intestinal epithelium, or enterocytes. MEVs also pass through the epithelial layer into the GALT. The GALT is a lymphoid structure associated to the digestive tract (*gut-associated lymphoid tissues)* located beneath the intestinal epithelium. It is located at specific spots along the intestine. GALT is a dense tissue composed of germinal centers with B and T lymphocytes, plasmocytes and innate immune cells including dendritic cells and macrophages. In the intestine, fluorescence is observed mostly in the GALT. Fluorescence appears concentrated in discrete spots around nucleus meaning that MEVs are localized in the plasma of cells occupying all the space in the plasma. This allows revealing of the shape of cells. Cells with MEVs inside correspond to histocytes (resident macrophages) or dendritic cells respectively and are located mainly at the periphery of GALT and in the center of the GALT. Representative images are displayed in Figures 15A and 15B. No visible MEVs appear in the liver at all times evaluated (30 min to 24h). A few hours after administration, dendritic/macrophage cells with MEVs inside move from the GALT to the spleen and stay in the spleen for several hours. MEVs reach principally the red pulp (blood cells and some Th and B cells) are and in a lesser extent the white pulp of the spleen (lymphoid cells) (see FIG. 16).

In the spleen, fluorescence is visible is several areas corresponding to the white and to the red pulps. The intensity of the fluorescence decreases in a gradient from the red pulps to the white pulps. Fluorescence is detected in spots inside the cell's cytoplasm. Cells with MEVs inside are round or reticular and have similar size; they may respectively be histocytes and dendritic cells or macrophages.

Cells can move from the GALT into the bloodstream directly or they can join it after they have first passed through the lymph stream. GALT cells carrying MEVs inside eventually arrived at the liver by the portal vein, MEVs, however, are 'invisible' to the liver as they have been internalized by GALT cells that naturally transport them to the spleen (see Figure 17). These MEVS, therefore, are not captured by the liver, as are the MEVs that have been administered intravenously.

## EXAMPLE 6

### *In vitro* delivery of guest cargo by the Microalgae Extracellular Vesicles (MEVs)

### A. Uptake of loaded MEVs by neuronal cells - microplate assay

PC12 neuronal cells are used as a model for *in vitro* evaluation of neuroprotective effects. Briefly, PC12 cells are seeded into 96-well plate (50,000 cells/well) and cultured for three days. Next, the catalase carrying MEVs (loaded as described above) are stained with DiR (5 μg/mL) and added to the wells in serial dilutions for various times. Following the incubation, the cells are washed three times with ice-cold PBS and solubilized in 1% Triton X-100. The sample fluorescence is measured using a SpectraMax® fluorescence microplate reader (Molecular Devices, USA) with excitation at 750 nm and emission at 780 nm. The amount of MEVs accumulated in neuronal cells is normalized for total protein content and expressed as the number of MEVs per mg of protein. All MEV formulations are prepared at the same level of fluorescence, and a separate calibration curve is used for each MEV formulation.

### B. Uptake of loaded MEVs by neuronal cells - confocal microscopy

The catalase loaded MEVs (100 μg/mL total protein) are sonicated, stained with DiR (5 μg/mL) and incubated with PC12 cells grown on chamber slides ($1x10^5$ cells/chamber) for various time intervals. Following the incubation, the cells are washed with PBS, fixed in 4% paraformaldehyde and permeabilized with 0.1% Triton X-100. Fluorescent staining is performed using anti-actin antibody (Abcam ab179467, 1:100) with goat anti-rabbit IgG Alexa Fluor 488 (Abcam ab150077, 1:1000) as the secondary antibody. DAPI (4',6-diamidino-2-phenylindole) is used as nuclear counterstain prior to the imaging. Accumulation of fluorescently labelled MEVs is visualized by a LSM700 laser scanning confocal microscope (Zeiss) and images are processed with LSM Image Browser.

### C. Neuroprotective effects of the loaded MEVs

The protection of PC12 cells against 6-OHDA-induced cytotoxicity is assessed by an MTT assay. For this purpose, PC12 cells ($1x10^5$ cells/mL) are seeded into a 96-well plate and allowed to attach overnight. Then, the cells are exposed to 200 μM 6-hydroxydopamine (6-OHDA) and different catalase-loaded MEV formulations, or catalase alone, or empty MEVs for four hours. Following incubation, the cells are washed 3 times with ice-cold PBS, and incubated with the corresponding catalase loaded MEV formulations, or catalase alone, or empty MEVs for another 24 hours. Following the treatment, 20 μL of MTT tetrazolium dye solution (5 mg/mL) is added into each well. After 3 hours of incubation at 37°C, the MTT-containing medium is removed and 100 μL DMSO is added into each well to dissolve the purple formazan precipitate. Absorbance is measured at 570 nm using a SpectraMax® microplate reader (Molecular Devices, USA) and cell viability is expressed as a percentage of viable cells in the treated groups compared to the untreated control group.

### D. MEV internalization in human cancer cells

HeLa cell line of human cervix epithelioid carcinoma is used to study uptake of GFP-loaded MEVs. For flow cytometry analysis, the cells are seeded in 24-well plates (15,000 cells/well). After 24 h, cells are incubated with the cargo-loaded MEVs for 4 h and subsequently washed with PBS, washed again with acid wash buffer (0.5 M NaCl, 0.2 M acetic acid) to remove membrane-bound MEVs and once more with PBS. Cells are detached with trypsin, fixed in 0.2% paraformaldehyde in PBS and analyzed using LSRII flow cytometer with CellQuest Pro software (BD Biosciences). For confocal microscopy analysis, HeLa cells are seeded in 16-well chamber slides (Lab-Tek) at 4,000 cells/well. After 24 h, cells are incubated with the cargo-loaded MEVs for 4 h and subsequently washed with PBS, washed again with acid wash

buffer (0.5 M NaCl, 0.2 M acetic acid) to remove membrane-bound MEVs and once more with PBS. Cells are fixed with 4% paraformaldehyde in PBS at room temperature for 20 min. Slides are then washed with PBS and mounted using FluorSave™ reagent (Calbiochem, San Diego, CA). Confocal fluorescent imaging is performed using a LSM700 laser scanning confocal microscope (Zeiss®) and images are processed with LSM Image Browser.

**EXAMPLE 7**

**Evaluation of toxicity of MEVs in mice**

Experiments were performed to assess the signs of MEV toxicity *in vivo* using Balb/C mouse model after oral and intratracheal administration.

**A. Analysis of MEV toxicity *in vivo:***

Toxicity was evaluated in several ways: clinical signs, body weights, hematological analysis, biochemical analysis, histological analysis on main organs (liver, spleen, kidney, lung and brain). The MEV samples were stored at -80°C and thawed just prior to *in vivo* administrations. After thawing, each sample was mixed by vigorous vortexing for 1-2 minutes. Male Balb/C mice at 5 weeks of age and with a mass about 20 g each, were used. Animals were acclimatized. Animals were housed in polyethylene cages (<5 animals/cage), in a controlled environment with 12:12 light-dark at the temperature of 24±1°C (mean ± SD) and fed once daily with an adapted pelleted feed. Water was offered *ad libitum.* The animals were randomly assigned to experimental groups and acclimatized for at least 7 days before the initiation of the designed study. The experimental groups are described in Table 30, below.

The experiment was designed to determine the eventual toxicity of the test compound after its administration in mice through exemplary routes including oral, and respiratory tract (intratracheal) routes. As described above, male Balb/C mice, with a mass about 20 g each, were used. Animals were acclimatized. After a test item is administered, all mice are closely monitored for 10 days.

**Table 30.** Experimental groups in MEV toxicity study.

| Group no. | Group type | Route of administration | MEV dosage |
|---|---|---|---|
| 1 | Vehicle control (PBS) | PO (oral) | - |
| 2 | Experimental | PO (oral) | 4x10exp11 per animal |
| 3 | Experimental | PO (oral) | 1x10exp12 per animal |
| 4 | Vehicle control (PBS) | IT (intratracheal) | - |
| 5 | Experimental | IT (intratracheal) | 4x10exp11 per animal |

**Clinical examination**

Daily clinical examination of all animals included, observation of behavior and signs of suffering (cachexia, weakening, difficulty for moving or feeding, etc.); test item toxicity (hunching, convulsions), and other such parameters. Determination of body weight once a week for each animal, a body weight curve was designed (Mean + SD). Observation of acute reactions was done after administration of the tested compounds.

**Clinical Pathology Investigations**

After the end of the in-life phase, all animals were euthanized. Clinical pathology investigations were performed at experiment termination.

**Blood collection**

The blood samples were collected from mice by intracardiac puncture into different vials. Aliquots of blood were collected for various clinical pathology investigations into tubes containing anticoagulants: for hematology analysis with K2 EDTA and for biochemistry analysis with lithium heparin.

**Clinical chemistry**

Plasma was separated after centrifugation of whole blood samples, 45000 rpm for 15 minutes and analyzed for the following parameters at the end of treatment for all animals. Clinical chemistry analysis parameters are indicated as: Alanine Aminotransferase (ALT), Aspartate Aminotransferase (AST), and Gamma-glutamyl transferase (GGT) in units per liter (U/L); Urea in g/L and Creatinine in mg/L.

**Hematology**

The following hematological parameters were determined at the end of treatment of all animals: Hemoglobin (HGB) in g/L; Hematocrit (HCT) in L/L; Mean Corpuscular Volume (MCV) in fL; Eosinophils (EO) and MHCH (BASO) as $10^9$/L.

When animals are euthanatized, an autopsy is performed, and a careful macroscopic evaluation is performed. If any organs look abnormal, a detailed description and analysis is performed.

**Histological analysis**

Histological analysis is performed for the 5 following organs: Lung, Spleen, Liver, Kidney, and Brain. The organs are collected, weighed, macroscopically observed, fixed in 4% Paraformaldehyde (PFA) and paraffin-embedded, cut into 5-7 μM sections and then observed under fluorescence microscope.

**Histopathological scoring**

Each H&E (hematoxylin- and eosin-stained) section was thoroughly examined histologically, and lesions observed were recorded in an Excel spreadsheet, their severity graded (minimal, mild, moderate, or severe). Their distribution also was characterized (i.e., as focal, multifocal, focally extensive or diffuse), as well as, their localization.

**B. Toxicity study results: Clinical examinations**

The results of assessment of toxicity of MEVs in a mouse model after oral (PO) or intratracheal (IT) administration at different doses in 4 groups of mice for each parameter are shown in Figures 18A-I, which show clinical chemistry and hematology in mice after administration (PO, IT) of MEV.

**Clinical chemistry**

As shown below, two parameters Urea and Creatinine did not change in all treated groups compared to control groups. The enzymes Alanine Aminotransferase (ALT) and Aspartate Aminotransferase (AST) showed random non-significant deviations. Figure 18 presents these results. The parameter Gamma-glutamyl transferase value is detected at low level (<6) in all groups indicating a protocol error and are not presented.

The results of the clinical chemistry parameters do not indicate any toxicity at all tested doses in the mice either by oral or intratracheal administration.

**Hematology**

The measured parameters were not significantly changed compared to controls. Figures 18A-I present the results in which MEV toxicity was evaluated by (1) chemistry parameters: ALAT, ASAT, urea and creatine; and (2) by hematology parameters in four groups of mice: Group 1 mice were administered 100 μl of PBS by PO delivery; Group 2 mice were administered 100 μl of $4*10^{11}$ MEV/ mouse by PO delivery (bar with black and white tiles); Group 3 mice were administered 100 μl of $4*10^{12}$ MEV/ mouse by PO delivery (bars with vertical lines); Group 4 mice were administered 100 μl of $4*10^{11}$ MEV/ mouse by IT delivery

The results present blood parameters that can be altered when there is one or more of blood-, kidney-, and liver-related toxicity. Hematocrit, hemoglobin, eosinophil levels, RBC count, and volume (MCV) are hematologic parameters; urea and creatine are biochemical markers of kidney injury; ALAT (or ALT) and ASAT (or AST) are biochemical markers of liver injury. Alanine transaminase (ALT), also called alanine aminotransferase (ALAT), is a transaminase enzyme that occurs in plasma and in various body tissues, but most commonly in the liver. Significantly elevated levels of ALT can be related to liver-related problems, such as hepatitis and/or liver damage. Aspartate transaminase (AST), also known as aspartate aminotransferase (ASAT), is another transaminase enzyme important in amino acid metabolism. Beyond liver toxicity, AST can be elevated also in diseases affecting other organs. Serum ALT level, serum AST level, and their ratio

(AST/ALT ratio) are used clinically as biomarkers for liver health. In the instant study, no statistically-significant differences between experimental groups in ALT and AST levels were observed.

Eosinophils (eosinophiles) are a variety of white blood cells and one of the immune system components responsible for combating multicellular parasites and certain infections in vertebrates. Eosinophils usually account for less than 7% of the circulating leukocytes. Beyond causes related to infection or parasitic infestation, elevated eosinophil levels (also known as eosinophilia) can be also a sign of allergic or atopic reactions. In the instant study no statistically significant differences between experimental groups in eosinophil levels were observed.

**Histology**

The main organs were collected, fixed in 4% PFA, paraffin-embedded, cut into sections and stained with H&E.

*Lung*

Histopathological changes observed in lung sections are primarily limited to minimal to mild focal to multifocal alveolar hemorrhages in 8/10 lung sections examined in animals receiving intratracheal negative control material and test item. Changes were relatively subtle and limited in some of the alveolar spaces. This observation was observed at comparable incidence and severity in negative control and treated animals.

*Kidney*

Presence of a few basophilic tubules were observed in 1 animal treated PO with $4x10^{11}$ MEV dose. This finding was minimal in severity and often is observed spontaneously at low incidence and severity in laboratory rodent animals. It is therefore considered as incidental in origin in the present study.

*Spleen, Liver, and Brain*

No histopathological changes were observed in spleen, brain, or liver in all sections examined.

*Summary*

The pulmonary changes observed in most animals receiving the experimental material intra-tracheally were considered not treatment-related. There was no treatment-related observed in all organs examined, indicating that MEVs are not toxic to animals under the experimental conditions.

**C. Conclusion**

Regarding the clinical aspects, all the animals, at each time of the study, exhibited normal behaviors, body weight, water and food consumption. The clinical findings indicate general tolerance in the mouse model of the test MEV products. Under the experimental conditions, both negative controls (PBS) and MEV at concentration of $4*10^{11}$ MEV/ mouse or $4*10^{12}$ MEV/ mouse by oral administration and at concentration of $4*10^{11}$ MEV/ mouse by IT administration did not induce any abnormal, toxic effect on animals.

**EXAMPLE 8**

**Internalization of cargo loaded MEVs in normal human keratinocytes**

**A. Cell culture**

Human keratinocytes are isolated from skin of healthy volunteers and used for the experiments at the maximum passage of 4. The cells are grown as monolayers in 75 $cm^2$ flasks, incubated at 37°C with 100% humidity and 5% $CO_2$. RPMI1640 is used as culture medium, enriched with 20% fetal bovine serum (FBS), 1% penicillin/streptomycin, and 1% fungizone. The culture medium is changed every 2 days to ensure the growth of cells and to avoid contamination. The cells once reached sub-confluence, are detached from the bottom of the flask with trypsin (0.25%), centrifuged (1600 g, 4 min), re-suspended in fresh culture medium and seeded at $2\times10^5$ cells/plate.

### B. Cellular uptake of GFP-loaded MEVs

The internalization of MEVs by keratinocytes is studied by confocal microscopy. The experiments are performed on sub-confluent keratinocytes grown on glass slides. MEVs are loaded with GFP protein as described above, added to the cells at various concentrations, and incubated at 37°C for 2, 4, 8, and 24 h. The cells subsequently are washed twice with RPMI1640, fixed with 4% paraformaldehyde in PBS (pH 7.4), and treated with Triton X-100 (0.1%) to increase the permeability of the cell membrane. The fixed cells are washed with PBS and then stained with Hoechst blue to visualize the cell nucleus, and with phalloidin-fluorescein isothiocyanate conjugate for staining the actin cytoskeleton. After staining, the cells are washed again with PBS, and allowed to dry overnight. Microscopic images are obtained using an inverted confocal microscope FluoView FV1000 (Olympus) equipped with a 20× UPlanSApo objective. GFP and Hoechst are visualized with a wavelength of excitation/emission of 559/578 nm and 360/460 nm, respectively.

## EXAMPLE 9

### *In vitro* modulation of human cancer cells by the Microalgae Extracellular Vesicles (MEVs)

### A. Cell cultures

Human cell lines (ATCC®) are grown in DMEM (Dulbecco's modified Eagle's medium) supplemented with 10% fetal bovine serum (FBS), 50 units/ml penicillin, and 50 μg/ml streptomycin. The cells are kept in culture at 37°C and 5% $CO_2$, and the medium is changed every three days. To keep the cells at optimal proliferating conditions, they are passaged at 80% confluence and seeded at 20% confluence. The following cell lines have been used in the study:

1) *MYC* oncogene-expressing HT29 (colon cancer), PANC-1 (pancreatic cancer) and *MYC*-negative LN-18 (malignant glioma);
2) *RAS* oncogene-expressing SCLC-21H (small-cell lung cancer), MCF-7 (breast cancer) and *RAS*-negative PC3 (prostate cancer);
3) *BCL-2* oncogene-expressing AsPC-1 (pancreatic cancer), U-937 (pro-monocytic myeloid leukemia) and *BCL*-2-negative LM-MEL-53 (malignant melanoma);
4) *PLK-1* oncogene-expressing HepG2 (hepatoma), PC3 (prostate cancer) and *PLK-1*-negative AsPC-1 (pancreatic cancer).

### B. RT-PCR analysis and gel electrophoresis

The cells (1×$10^6$) are grown in culture plates for 24 h. Next, the siRNA carrying MEVs (loaded via electroporation as described above), or empty MEVs, or siRNA alone, are added to the wells in serial dilutions and incubated for 1-24 hours. Following the incubation, the medium is changed for the normal culture medium. 72 h after the MEV treatment, total RNA is extracted from the cultured cells using TRIzol® reagent. The RNA precipitate is centrifuged and dissolved in 20 μl of RNase-free water. UV spectrophotometer analysis at 260/280 nm and agarose gel electrophoresis are used to confirm the quality of purified RNA. 1 μg of total RNA is reverse transcribed to synthesize cDNA at 42°C for 1 h; this cDNA is then subjected to PCR amplification with specific primers in 25-μl reactions. Next, the PCR products are resolved on 2% agarose gel, stained with ethidium bromide and observed with a UV trans-illuminator. The digital images are quantified with ImageJ analysis software and the results are expressed as the relative expression level of relevant genes over GAPDH.

### C. Gene expression analysis with quantitative RT-PCR

The cells treated with the MEVs and cultured for a total of 72 h are trypsinized and washed with PBS. Total RNA is extracted from the cell pellets using the RNeasy® Minikit (QIAGEN) according to the manufacturer's instructions. Then, 500 ng of total RNA is reverse transcribed into cDNA using a reverse transcriptase (Promega) and real-time PCR is performed using the LightCycler® 480 SYBR Green I Master Kit on an LC480 device (both from Roche Diagnostics). The mRNA level is calculated by normalizing the threshold cycle (CT) of target genes to the CT of the 18S ribosomal RNA housekeeping gene. The primers are designed using primer software from Roche Diagnostics and are purchased from Eurogentec.

### D. Western blot analysis

The cells (2×$10^6$) treated with the MEVs and cultured for a total of 72 h are trypsinized, washed with PBS and lysed on ice in RIPA buffer supplemented with β-mercaptoethanol (Sigma-Aldrich). Then, 50 μg samples of total protein

are subjected to 10% standard sodium dodecyl sulfate-polyacrylamide gel electrophoresis (SDS-PAGE), with pre-stained molecular weight markers run in parallel. Subsequently, the resolved proteins are electro-transferred onto PVDF membranes. After extensive washing, the membranes are incubated with the relevant horseradish peroxidase-conjugated antibody (Sigma-Aldrich) for 1 h at room temperature, and developed with a chemiluminescence detection kit (Thermo Fisher Scientific). Membranes probed for the oncoproteins are re-probed for β-actin to normalize for loading and/or quantification errors and to allow comparisons of target protein expression. The protein expression is quantified with a gel analysis system (Bio-Rad).

**E. Cell proliferation assay**

The cells are seeded into 96-well plate (10,000 cell/well) and allowed to attach for 18 h. Next, the cells are treated with the MEVs as above; at 24, 48, and 72 h after the treatment, 10 μl 10 mg/ml MTT is added to the cells in each well. The cells are incubated for 4 h, after which 100 μl DMSO is added, and the cells are allowed to lyse for 15 min. All of the analyses are carried out in triplicate. Optical density at 492 nm is determined with a SpectraMax® microplate reader (Molecular Devices, USA). To determine the inhibitor rate, the absorbance values are then normalized to the values obtained from the blank control cells.

**1. Apoptotic cell morphology observation**

The cell morphology related to apoptotic cell death is established by staining the cell nuclei with the DNA-binding fluorochrome Hoechst 33258 and assessing the chromatin condensation by fluorescence microscopy. Briefly, the cells are seeded in 24-well plates with glass slides on the bottom of the wells, and treated with the MEVs as above. Afterwards, the slides are gently washed with cold PBS, fixed with 4% paraformaldehyde for 1 h and washed three times with PBS. The slides are stained with 0.5 ml Hoechst 33258 (10 μg/ml) at 37°C for 10 min in a dark room. In each group, five microscopic fields are randomly selected, and one hundred cells are counted. The apoptotic cell level is then calculated as the percentage of apoptotic cells over the total number of fluorescent cells.

**2. Fluorescent analysis of necrotic/apoptotic cell death**

For flow cytometry analysis, the cells are seeded in 24-well plates (15,000 cells/well). After 24 h, cells are treated with the MEVs as above. Subsequently, the cells are washed with PBS and detached with trypsin. Following fixing in 0.2% paraformaldehyde in PBS, the cells are labeled with Annexin V FITC/PI (Thermo Fisher Scientific) and analyzed using LSRII flow cytometer with CellQuest® Pro software (BD Biosciences).

**EXAMPLE 10**

***In vivo* delivery and expression of exogenously loaded siRNA**

This study assesses and demonstrates the efficacy of MEVs loaded with siRNA against genes of prokaryotic and eukaryotic microorganisms. The different bacterial strains (*E. coli, P. aeruginosa, S. aureus*) and yeast (*Candida albicans*) are genetically modified to carry the bacterial Luciferase gene Lux operon (luxCDABE) for Gram-negative species, (luxABCDE) for Gram-positive species, and the luciferase gene from the copepod *Gaussia* sp. for the yeast species. These microorganisms provide *in vivo* infection models as follows:

- *P. aeruginosa, S. aureus* / respiratory system,
- *P. aeruginosa, S. aureus, and Candida albicans* / skin,
- *E. coli* / digestive system,
- *E. coli* / urogenital, and

*Candida albicans* / urogenital system and skin.

Real-time monitoring of the interference activity of MEVs loaded with siRNA against the bacterial luciferase gene Lux operon or eukaryotic luciferase provides a way to follow, among other things: the capacity of the MEVs to penetrate infecting bacteria and yeast *in vivo;* to monitor the spread of MEVs in infection sites by different routes of applications and tissues; to monitor the capacity of MEVs to interfere with coding genes; and to assess the anti-microbial action of MEVs that deliver an anti-microbial therapeutic, such as an siRNA.

### *1. In vivo* imaging

The animals are the male Balb/C mice as described above. After infection, mice were closely monitored and imaged by IVIS bioluminescence to detect low levels of light emitted by luciferase *in vivo,* and bioluminescent bacteria that can be localized in specific tissues in living animals, thus allowing temporal monitoring of the infection process. The mice were treated after with $1x10^{10}$ particles/g of MEVs and closely monitored and imaged by IVIS bioluminescence to follow the effectiveness of interference antimicrobial treatment, as represented by the interference activity of different siRNA populations against luciferase mRNA. The data were analyzed with the IVIS software (Living Image Software for IVIS).

## EXAMPLE 11

### *In vivo* delivery and expression of exogenously loaded mRNA

### Ocular distribution following a single ocular topical administration in albino rabbits

### A. Material and Methods

A New Zealand White (albino) rabbit is one of the species most used for ocular biodistribution analysis. Eight 3-month males of weight between 2-2.5 kg were used in the ocular topical administration study.

All animals were ear-tagged at their arrival and the identification numbers were also marked in ears using indelible ink following the inclusion examination.

### B. Clinical examination and health status

Animals were daily observed for signs of illness and particular attention was paid to their eyes. Only healthy animals without visible ocular defect were used in the study.

### C. Housing

Animals were housed individually in standard cages. The temperature was held at 18+/-3°C and the relative humidity at 45-80%. Rooms were continuously ventilated (15-20 air volumes per hour). Animals were routinely exposed (in-cage) to 10-200 lx light in a 12-hour light (from 7:00 a.m. to 7:00 p.m.) and darkness-controlled cycle.

### D. Food and water

Animals had free access to food (90 g/day) and water available *ad libitum* from plastic bottles.

### E. Procedure

A 50-μL single instillation on both eyes was applicated to all animals, using an appropriate micropipette, at a dose of $2X10^8$ MEVs loaded with mRNA-eGFP. Fluorophotometry was performed as follows: at several time-points (0, 6, 24, 30, 48, 54, and 72-hours post-administration) animals were anesthetized and pupils were dilated; measurements of ocular fluorescence were performed with FM-2 Fluorotron™ Master ocular fluorophotometer in both eyes. A series of 148 scans with a step size of 0.25 mm were recorded from the cornea to the retina along the optical axis.

### F. Results:

The fluorescence signal in various ocular tissues (choroid/ retina, vitreous, lens, anterior chamber, cornea) was obtained at data points that will be 0.25 mm apart along an optical axis by the fluorophotometer. Any detectable fluorescence was measured in the vitreous or the anterior chamber. The results of all animals are summarized in Figure 19.

### A. Delivery of GFP (green fluorescent protein) to normal human hepatocytes

### 1. Isolation and culture of primary human hepatocytes (PHH)

Histologically normal liver tissue samples are obtained from subjects undergoing partial hepatectomy for the treatment of colorectal cancer metastases or primary liver cancer. All samples are seronegative for the hepatitis C virus

(HCV), the hepatitis B virus (HBV) and the human immunodeficiency virus (HIV). Briefly, the liver fragment is initially perfused with a pre-warmed (37°C) calcium-free buffer supplemented with 5 mmol/L ethylene glycol tetra-acetic acid (Sigma-Aldrich) followed by perfusion with a pre-warmed (37°C) buffer containing 6 mmol/L calcium ($CaCl_2$) and 0.05% collagenase (Sigma-Aldrich). The liver fragment is then gently shaken to disperse liver cells in Hepatocyte Wash Medium (Life Technologies). The resulting cellular suspension is filtered through a gauze-lined funnel. Cells are then centrifuged at low speed (50 g), the supernatant removed, and pelleted hepatocytes are re-suspended in Hepatocyte Wash Medium. The count of viable cells is determined using Trypan Blue exclusion. Freshly isolated hepatocytes are suspended in William's E medium (Life Technologies) containing 10% fetal calf serum (Eurobio), penicillin (200 U/ml)-streptomycin (200 μg/ml; Life Technologies), fungizone (2.5 μg/ml; Life Technologies) and insulin (0.1 U/ml; Sigma-Aldrich). The cells are then seeded in 12-, 24- and 96-well plates pre-coated with type I collagen at a density of $0.78\times10^6$, $0.4\times10^6$ and $0.5\times10^5$ viable cells/well, respectively, and incubated at 37°C in 5% $CO_2$ overnight. The medium is replaced with fresh complete hepatocyte medium supplemented with 1 μmol/L hydrocortisone hemisuccinate (SERB) and cells are maintained in this medium until incubation with cargo loaded MEVs.

**2. Effect of MEVs on PHH viability**

PHHs ($1x10^5$ cells/mL) are seeded into a 96-well plate and allowed to attach overnight. Then, the cells are exposed to different cargo loaded MEV formulations or empty MEVs for four hours. Following the incubation, 20 μL of MTT tetrazolium dye solution (5 mg/mL) is added into each well. After 3 hours of incubation at 37°C, the MTT-containing medium is removed and 100 μL DMSO is added into each well to dissolve the purple formazan precipitate. Absorbance is measured at 570 nm using a SpectraMax® microplate reader (Molecular Devices, USA) and cell viability is expressed as a percentage of viable cells in the treated groups compared to the untreated control group.

**3. MEV internalization in PHH**

The internalization in PHH of the GFP-protein-cargo-loaded MEVs was determined by flow cytometry. For flow cytometry analysis, PHHs are seeded in 24-well plates (15,000 cells/well). After 24 h, cells are incubated with the MEVs for 4 h and subsequently washed with PBS, washed again with acid wash buffer (0.5 M NaCl, 0.2 M acetic acid) to remove membrane bound MEVs and once more with PBS. Cells are detached with trypsin, fixed in 0.2% paraformaldehyde in PBS and analyzed using LSRII flow cytometer with CellQuest® Pro software (BD Biosciences). For confocal microscopy analysis, PHHs are seeded in 16-well chamber slides (Lab-Tek) at 4,000 cells/well. After 24 h, cells are incubated with the MEVs for 4 h and subsequently washed with PBS, washed again with acid wash buffer (0.5 M NaCl, 0.2 M acetic acid) to remove membrane bound MEVs and once more with PBS. Cells are fixed with 4% paraformaldehyde in PBS at room temperature for 20 min. Slides are then washed with PBS and mounted using FluorSave™ reagent (Calbiochem). Confocal fluorescent imaging is performed using a LSM700 laser scanning confocal microscope (Zeiss) and images are processed with LSM Image Browser.

**4. Gene expression studies**

The internalization and biological activity in PHH of the GFP-mRNA-cargo-loaded MEVs is determined by total RNA extracted from the PPHs using the RNeasy® Minikit (QIAGEN) according to the manufacturer's instructions. Then, 500 ng of total RNA is reverse transcribed into cDNA using a reverse transcriptase (Promega) and real-time PCR is performed using the LightCycler® 480 SYBR Green I Master Kit on an LC480 device (both from Roche Diagnostics). The mRNA level is calculated by normalizing the threshold cycle (CT) of target genes to the CT of the 28S ribosomal RNA housekeeping gene. The primers are designed using primer software from Roche Diagnostics and are purchased from Eurogentec.

**5. Protein expression studies**

Additionally, the targeted protein expression is verified using SDS-PAGE followed by Western blot analysis. Cells are lysed on ice in RIPA buffer supplemented with β-mercaptoethanol (Sigma-Aldrich). Then, 30 μg of proteins are boiled and subjected to SDS-PAGE and transferred to nitrocellulose membranes (Bio-Rad). The blots are blocked with Tris buffered saline (TBS), 0.1% Tween-20 containing 5% BSA and incubated overnight at 4°C with the following antibodies. Subsequently, the blots are incubated with the relevant HRP-conjugated secondary antibody for 1 h at room temperature. Signals are revealed by chemiluminescence (ThermoFisher Scientific).

**B. Delivery to human hepatocytes**

**1. Isolation of human hepatocytes**

PHH (Primary Human Hepatocytes) are isolated from normal liver tissue obtained from patients undergoing partial hepatectomy. Dissociation is based on a two-step collagenase perfusion method as previously described (L. Aoudjehane, DMM, 2020). The cells are then separated by centrifugation over gradient. Hepatocytes are isolated from the pellet, and non-parenchymal cells (NPCs) from the supernatant with different gradients. Cell viability is determined by Trypan blue dye exclusion, and freshly isolated PHH were then seeded in 12-, 96-well plates precoated with type I collagen at a density of $1x10^6$, $6x10^4$ viable cells/well, respectively, and incubated at 37°C in 5% $CO_2$, overnight.

**2. Cell culture conditions**

Cell lines Huh7 and HepG2 human hepatocytes cell lines were cultured in DMEM medium and incubated at 37°C, 5% $CO_2$ until the confluence. The medium of PHH isolated cells was replaced with fresh complete hepatocyte medium supplemented with hydrocortisone and cells were maintained in this medium until incubation between D3 and D5) with MEV with different conditions at 37°C in 5% CO2 for 24h and 48h. Huh7 and HepG2 ($2x10^6$ cells per well in 12-well plates) were seeded in triplicate on plastic in DMEM (10% FCS) and allowed to attach for 24 h. Then, in all experiments, the medium was replaced with free FCS-DMEM and the cells were cultured for 24h, 48h without MEV (for negative controls) or with the different MEV studied.

**3. Cell Viability Evaluation**

Cell viability was determined by 3-(4,5- dimethylthiazol-2-yl)-5-(3-carboxymethoxyphenyl)-2-(4-sulfophenyl)-2H-tetrazolium (MTT) colorimetric assay, which tests the ability of viable cells to convert a soluble tetrazolium salt, MTT, into a blue formazan end product by mitochondrial dehydrogenase enzymes. MTT (0.5 mg/mL) was added to each well and incubation at 37°C was prolonged for an additional 2 hours. The medium was then discarded and DMSO was added to each well to solubilize the colored formazan product. Absorbance was read at 550 nm on a scanning microtiter spectrophotometer plate reader.

**4. Fluorescence imaging and confocal imaging:**

The fluorescence images of PHHs and cell lines treated with MEV were viewed with an Olympus® IX83 microscope (Olympus) acquired with cellSens® V1.6 imaging software and analyzed with FIJI software.

**5. Reverse Transcriptase-Polymerase Chain Reaction (RT-PCR) for mRNA Detection**

Total RNA was prepared using the RNeasy® minikit (Qiagen SA, Courtaboeuf, France) according to the manufacturer's recommendations. cDNA synthesis was carried out for 90 min at 42°C in a reaction mixture containing two units of RNase inhibitor (Promega, Charbonnieres, France), three units of avian myeloblastosis virus reverse transcriptase (Promega), 120 ng of random hexamer primers (Sigma-Genosys Ltd, Saint-Quentin Fallavier, France) and 1mM dNTP (Promega), for 500 ng of total RNA.

**6. Real-Time Quantitative PCR for Collagens and α-SMA evaluation**

Collagen-1 and α-SMA gene expressions in HLMFs were analyzed by RT-PCR using the LightCycler® 480 SYBR Green I Master (2x con) Kit (Roche, Grenoble, France), with LC480 instruments and technology (Roche Diagnostics). The reaction was ensured using SYBR® Green I Master (2x con) Kit (Roche Diagnostics), with LC480 instruments and technology (Roche Diagnostics). PCR amplification was performed in a total volume of 10 μl, containing 5 μl of SYBR® green I Master Mix (2x), 10 ng of each primer (Sigma-Genosys Ltd; see Table) and 2 μl of cDNA (1/10eme). The PCR amplification protocol consisted in one step of initial denaturation for 10 min at 94° C, followed by 40 cycles: denaturation (95° C for 10 s), annealing for 10 s at 60° C and extension (72°C for 10 s).

**Table 31.** Primers used for quantitative real-time PCR.

| Gene | Sequence of forward (F) and reverse (R) primers | |
|---|---|---|
| eGFP | F:5'-AGCAAAGACCCCAACGAGA-3'<br>R:5'-TCGTCCATGCCGAGAGTG-3' | SEQ ID NO:66<br>SEQ ID NO:67 |
| Albumin (ALB) | F: 5'-TTTTATGCCCCGGAACTCCT-3' | SEQ ID NO:128 |

(continued)

| Gene | Sequence of forward (F) and reverse (R) primers | |
|---|---|---|
| | R: 5'-TTGGAGACTGGCACACTTGA-3' | SEQ ID NO:129 |
| Cytokeratin 18 (CK18) | F: 5'-CATAGACAAGGTACGGTTCCTG-3'<br>R: 5'-TGTTGTCCATGTTGCTTCGAGC-3' | SEQ ID NO:130<br>SEQ ID NO:131 |
| 28S (RN28S1) | F:5'- TTGAAAATCCGGGGGAGAG -3' | SEQ ID NO:132 |
| | R:5'- ACATTGTTCCAACATGCCAG-3 | SEQ ID NO:133 |

**7. Western blot analysis**

Cells and tissues were lysed on ice directly in RIPA buffer supplemented (Sigma Aldrich). 20 μg of proteins was boiled and subjected to SDS-PAGE and transferred to nitrocellulose membranes (Bio-Rad, Hercules, CA). The blots were blocked with TBS, 0.1% Tween-20 containing 5% non-fat dry milk, and incubated with the following antibodies: Anti-Albumin (Dako), anti-eGFP (Roche), overnight at 4°C and with secondary HRP-link antibody (1/2000, GE Healthcare Europe GmbH), for 1h at room temperature. Signals were revealed by chemiluminescence (Thermo Fisher Scientific).

**8. Results:**

Table 32 shows the results of mRNA expression by RT-qPCR analysis for the different experimental conditions.

**Table 32.** Results of gene expression analysis using quantitative real-time PCR in MEV internalization study with human hepatocytes.

| Experimental Conditions | eGFP | 28S | ALB | CK18 |
|---|---|---|---|---|
| After 24 hs | | | | |
| PHH (negative control) | (-) | (+++) | (+++) | (++) |
| PHH ( non-loaded MEV) | (-) | (+++) | (+++) | (++) |
| PHH ( GFP protein-loaded MEV) | (-) | (+++) | (+++) | (++) |
| PHH ( mRNA eGFP-loaded MEV) | (+++) | (+++) | (+++) | (+++) |
| Huh7 cells (negative control) | (-) | (+++) | (+++) | (+++) |
| Huh7 cells (non-loaded MEV) | (-) | (+++) | (+++) | (+++) |
| Huh7 cells ( GFP protein-loaded MEV) | (-) | (+++) | (+++) | (+++) |
| Huh7 cells ( mRNA eGFP-loaded MEV) | (++) | (+++) | (+++) | (++) |
| Hep-G2 cells (negative control) | (-) | (+++) | (+++) | (++) |
| Hep-G2 cells (non-loaded MEV) | (-) | (+++) | (+++) | (++) |
| Hep-G2 cells ( GFP protein-loaded MEV) | (-) | (+++) | (+++) | (++) |
| Hep-G2 cells ( mRNA eGFP-loaded MEV) | (++) | (+++) | (+++) | (++) |
| After 48 hs | | | | |
| PHH (negative control) | (-) | (+++) | (+++) | (++) |
| PHH ( non-loaded MEV) | (-) | (+++) | (+++) | (++) |
| PHH ( GFP protein-loaded MEV) | (-) | (+++) | (+++) | (++) |
| PHH ( mRNA eGFP-loaded MEV) | (++) | (+++) | (+++) | (++) |
| Huh7 cells (negative control) | (-) | (+++) | (+++) | (++) |
| Huh7 cells (non-loaded MEV) | (-) | (+++) | (+++) | (++) |
| Huh7 cells ( GFP protein-loaded MEV) | (-) | (+++) | (+++) | (++) |
| Huh7 cells ( mRNA eGFP-loaded MEV) | (+) | (+++) | (+++) | (++) |
| Hep-G2 cells (negative control) | (-) | (+++) | (+++) | (++) |
| Hep-G2 cells (non-loaded MEV) | (-) | (+++) | (+++) | (++) |
| Hep-G2 cells ( GFP protein-loaded MEV) | (-) | (+++) | (+++) | (++) |
| Hep-G2 cells ( mRNA eGFP-loaded MEV) | (+) | (+++) | (+++) | (++) |

(continued)

| | | | | |
|---|---|---|---|---|
| After 48 hs<br>mRNA eGFP-loaded MEV (positive control) | (++++) | (-) | (-) | (-) |
| *Key: (-) negative result (non mRNA present); (+) positive result (low ); (++) positive result (acceptable); (+++) positive result (best); (++++) positive result (mRNA eGFP-loaded MEV / positive control)* | | | | |

Table 33 shows the results of GFP expression by fluorescence expression after confocal analysis for the different experimental conditions. The results synthetize number of cells and fluorescence intensity. Further imaging results are presented in Figures 22 and 23.

**Table 33.** Results of protein expression analysis using confocal microscopy in MEV internalization study with human hepatocytes.

| Experimental Conditions | 100 loaded MEV per cell | 200 loaded MEV per cell | 400 loaded MEV per cell |
|---|---|---|---|
| Huh7 cells (negative control) | (-) | (-) | (-) |
| Huh7 cells ( non-loaded MEV) | (-) | (-) | (-) |
| Huh7 cells ( GFP protein-loaded MEV) | (+) | (+++) | (+++) |
| Huh7 cells ( mRNA eGFP-loaded MEV) | (++) | (+++) | (+++) |
| Hep-G2 cells (negative control) | (-) | (-) | (-) |
| Hep-G2 cells ( non-loaded MEV) | (-) | (-) | (-) |
| Hep-G2 cells ( GFP protein-loaded MEV) | (+) | (++) | (+++) |
| Hep-G2 cells ( mRNA eGFP-loaded MEV) | (++) | (++) | (+++) |
| *Key: (-) negative result (non GFP fluorescence detected); (+) positive result (GFP fluorescence detected); (++) positive result (acceptable); (+++) positive result* | | | |

Table 34, below, shows the viability results by MTT colorimetric assay. Nontoxicity was observed after the incubation with large quantities of MEVs (loaded or non-loaded MEVs).

**Table 34. Cell viability assay results in MEV internalization study with human hepatocytes.**

| Experimental Conditions | % of viability |
|---|---|
| Huh7 cells (negative control) | **100%** |
| Huh7 cells (10,000 MEV) | **80%** |
| Huh7 cells ( 20,000 MEV) | **80%** |
| Huh7 cells ( 40,000 MEV) | **80%** |
| Hep-G2 cells (negative control) | **100%** |
| Hep-G2 cells (10,000 MEV) | **100%** |
| Hep-G2 cells (20,000 MEV) | **100%** |
| Hep-G2 cells (20,000 MEV) | **100%** |

**C. Human monocytes and keratinocytes**

Human primary cells were used as an *in vitro* model to study payload delivery with MEVs loaded with a fluorescent probe (GFP) or GFP coding RNA. Cell penetration and fluorescence effect was assayed in human monocytes and/or Normal Human Epidermal Keratinocytes (NHEK).

**1. Primary Cells:**

Peripheral Blood Mononuclear Cells (PBMC) from healthy volunteers were obtained from l'Etablissement Français du Sang (EFS). Cell culture medium Macrophage-SFM (M-SFM) was purchased from Gibco. NHEK cells and culture medium (KGM-Gold Bullet kit) were purchased from Lonza.

**2. Monocyte isolation, cell cultures:**

PBMCs were obtained from healthy blood donor buffy coats by a standard Ficoll-Hypaque gradient method. Monocytes were isolated from PBMCs by adherence to plastic for 2 hours in serum-free medium (M-SFM) optimized for macrophage culture, at 37°C in a humidified atmosphere containing 5% $CO_2$. NHEK were cultured following manufacturer's (Lonza) recommendations and seeded in 96-wells plate the day before the assay.

**3. MEV penetration assay:**

MEVs either loaded with GFP protein (MEV-GFP) or mRNAeGFP (MEV-mRNA) were incubated with NHEK and monocytes in 96-well plates for 4h, 8h and 24h.
The doses used were:

| **For the GFP protein:** | **For the mRNA eGFP:** |
|---|---|
| D1= $1x10^7$ MEVs/well | D1= $1x10^7$ MEVs/well |
| D2= $5x10^6$ MEVs/well | D2= $5x10^6$ MEVs/well |
| D3= $1x10^6$ MEVs/well | D3= $2.5x10^6$ MEVs/well |

Then the cells were washed with PBS two times and fresh medium was added. The plates were then placed in the Incucyte® live cell analyzer apparatus and images were done at 0, 6, 12, 24 and 48h for Prot-GFP and 0, 12, 24, 48 and 72h for mRNA-GFP.

**4. Image analysis:**

Images were analyzed using the Incucyte® live cell analyzer software. The number of cells was quantified first, followed by the number of cells that contained green fluorescence by applying a threshold on the fluorescence level. The same threshold was applied on all the images. Then the percentage of cells exhibiting green fluorescence was calculated as a ratio = (cells containing green fluorescence / number of total cells) x 100. See Figure 51, which shows the timeline for analysis.

**5. Monocytes internal delivery:**

Green-fluorescent cells were observed at all times of incubation, for an interval of 48hrs fluorescence following treatment by Incucyte® Nuclight reagents. This observation demonstrates the penetration of MEVs to human monocytes and the delivery of green fluorescence protein (GFP) from MEVs into monocytes. Optimal incubation time was 8h based on the results at the first fluorescence measurement. It was observed a dose effect following different quantities of vesicles (between $1x10^6$ to $5x10^7$) incubated with the cells. FIGs. 20A and B show the results after 8h and 24h of incubation (in a kinetics of 48h after wash).

**6. Keratinocytes internal delivery:**

Green-fluorescent cells were observed at all times of incubation, for an interval of 48hrs fluorescence following treatment by Incucyte® reagent. This observation demonstrates the penetration of MEVs to human keratinocytes and the delivery of green fluorescence protein (GFP) from MEVs into keratinocytes. The optimal incubation times are between 8h to 24h based on the results at the first fluorescence measurement. It was observed a dose effect following different quantities of vesicles (between $1x10^6$ to $5x10^7$) incubated with the cells. Figure 21 shows the results after 8h and 24 h of incubation (in a kinetics of 48h after wash)

**D. Evaluation of the penetration of MEVs and delivering of payloads in different human cell models (fibroblasts)**

**1. Fluorescence Microscopy:**

Fibroblasts (human dermal CRL 2522 ATCC® batch: 70027155) were seeded at $2x10^4$ cells by well into a 96 well plate. Thermo Nunclon® Delta Surface Disposable plates (96 wells) provide optimal resolution for cell culture (Thermo Nunclon, Cat No. 167425). After 20 h cells were incubated overnight with the different conditions (ratio of MEVs/cells is between 100 to 1000) of MEVs loaded with green fluorescent protein (GFP) or mRNA coding for green fluorescent protein

(mRNA-eGFP) in non-complete medium. After 16h cells were rinsed two times and fresh complete medium added. Hoechst solution at a dilution of 1:2000 was added to the medium to stain nucleus. After that, around the wells of the plate 1 mL of water was put in the compartments made for this. The images were taken by a CellInsight® laser autofocus camera at t=20h, t=48h and t=72h. The culturing conditions used with the CellInsight® platform are the same as in an incubator at 37°C. Photos were taken with confocal module with 20X objective. Figure 24 show the results of 3 independent experiments at ratio MEVs/cells of 100.

**2. Flow Cytometry:**

Fibroblasts (human dermal CRL 2522 ATCC® batch: 70027155) were seeded at $2x10^6$ cells per well into a 6 well plate. After 20 hours, cells were incubated overnight with the different conditions (ratio of MEVs/cells is between 100 to 1000) of MEVs loaded with green fluorescent protein (GFP) or mRNA coding for green fluorescent protein (mRNA-eGFP) in non-complete medium. After 16 hours, cells were rinsed two times and fresh complete medium added. At different times, cells were rinsed, treated with trypsin, centrifuged at 1,200 g, washed one time with PBS and then resuspended in 1mL of PBS.

**3. Fibroblasts Fluorescence-activated cell sorting**

GFP expression in cultured fibroblast after incubation or not with MEVs loaded with GFP-protein or with MEVs loaded with mRNA-eGFP was assessed by FACS analysis. Fibroblasts cells were cultured in 96 wells plate at 2000 cells per well. After 24h fibroblasts were incubated with or without MEVs (different conditions). At 24 h after cells were detached from wells by trypsinization, washed two times with PBS and then living cells analyzed for GFP expression fluorescence cytometry. Figures 25A-D show green fluorescence positive cells. FIG. 25A depicts Fibroblasts not incubated with MEVs. FIGs. 25B and 25C depict fibroblast incubated with MEVs loaded with mRNA-eGFP by different methods (Freezing and Thawing or Electroporation, respectively). FIG. 25D shows fibroblasts incubated with MEVs loaded with GFP protein by sonication. The results show a percentage of positive cells over the control of 5% in B and D conditions and 8% in C.

**E. Studies on MEVs loaded with antibacterial peptide**

Isolated MEVs are loaded using sonication as above with antimicrobial peptide LL-37 (LLGDFFRKSKEKIGKEFKRIVQRIKDFLRNLVPRTES; SEQ ID NO:37), synthesized commercially by Proteogenix (Lille, France) with 90% purity. Release of the antimicrobial peptide is monitored through dialysis and fluorescamine assay (Thermo Fisher Scientific) according to manufacturer's protocol. 1 mL of the sample is placed in a dialysis device (Float-A-Lyzer® G2100 kDa MWCO device, Spectrum Laboratories Inc.) and allowed to dialyze in 20 mL of 20 mM sodium acetate buffer pH 5.5 at room temperature. Samples of 200 μL are withdrawn after 0, 1, 2, 4, 6 and 24 hours of dialysis. Peptide quantification is performed with fluorescamine assay using a microplate reader (Molecular Devices, USA) with a standard curve showing good linearity in the range 2.5-150 μg/mL of the peptide.

Proteolytic protection of peptide LL-37 is investigated after incubation of the MEV dispersions with *Pseudomonas aeruginosa* elastase (PE) or human neutrophil elastase (HNE). Peptide-loaded MEVs (total amount of 2 μg peptide) are incubated for 6 hours at 37°C in 10 mM TRIS buffer pH 7.4 together with 0.2 μg PE (BioCol Labs) or 0.4 μg HNE (Calbiochem) in a volume of 15 μL. For control samples, MEVs are lysed prior to incubation with enzymes to release the biomolecular cargo by adding 5 μL of 0.4% SDS into 95 μL of MEVs mixture, thorough pipetting and incubation in a thermocycler for 15 min at 85°C. Peptide degradation is visualized with Coomassie brilliant blue staining following SDS-PAGE. Band intensities are quantified by Molecular Imager Gel DOC with Image Lab Software (Bio-Rad).

Radial diffusion assays are performed on the MEV formulations before and after incubation with the enzymes, using *Escherichia coli* (ATCC® 25922) and *Staphylococcus aureus* (ATCC® 25923) to evaluate the bactericidal properties after proteolysis. $4\times10^6$ CFU is added to an underlay agarose gel cast in 85 mm Petri dishes and 4 mm diameter wells are punched after solidification. 6 μL of the samples are added to each well and the plates are incubated at 37°C for 3 hours. Lysed MEVs are used as control samples. The underlay gel is thereafter covered with molten overlay agarose gel and incubated at room temperature overnight. Antimicrobial activity of the samples is visualized as a clear zone around each well. The results are presented as mean diameters of the clear zones formed by different formulations.

**EXAMPLE 12**

**Targeting neuronal cells with Microalgae Extracellular Vesicles (MEVs)**

**Biological activity of siRNA- and mRNA-loaded Microalgae Extracellular Vesicles (MEVs) in neurons *in vitro***

**1. RNAi-induced knockdown in mouse neuronal cultures**

Murine pyramidal cells in DIV10- 14 hippocampal dissociated cultures are analyzed using whole-cell patch clamp technique, recording of spontaneous excitatory activity (sEPSCs). Cultures are either untreated or preincubated with siRNA loaded MEVs at different concentrations (from $10^6$ to $5x10^{10}$ particles) or preincubation periods (from 0.5 to 24 hours). For each cell, baseline signal is recorded for 3 mins and compared with post-application baclofen (20 μM) recording to assess knock-down efficacy. The analysis includes sEPSCs frequency, amplitude and membrane parameters (Rm, Rs, Cm).

**2. Overexpression/Rescue in KO neuronal mouse culture**

Pyramidal cells in DIV10- 14 hippocampal dissociated cultures from GABAB1a$^{-/-}$ mice are analyzed using whole-cell patch clamp technique, recording of spontaneous excitatory activity (sEPSCs). Cultures are either untreated or preincubated with MEVs at different concentrations (from $10^6$ to $5x10^{10}$ particles) or preincubation periods (from 0.5 to 24 hours). For each cell, baseline signal is recorded for 3 mins and compared with post-application baclofen (20 μM) recording to assess rescue efficacy. The analysis includes sEPSCs frequency, amplitude and membrane parameters (Rm, Rs, Cm).

**3. RNAi-induced knockdown in human neuronal cultures**

Human glutamatergic neuronal cells derived from iPSCs are analyzed using whole-cell patch clamp technique, recording of spontaneous excitatory activity (sEPSCs). Cultures are either untreated or preincubated with MEVs at different concentrations (from $10^6$ to $5x10^{10}$ particles) or preincubation periods (from 0.5 to 24 hours). For each cell, baseline signal is recorded for 3 mins and compared with post-application baclofen (20 μM) recording to assess knock-down efficacy. The analysis includes sEPSCs frequency, amplitude, and membrane parameters (Rm, Rs, Cm).

4. **Patch-Clamp analysis**

For voltage-clamp recordings, 8-10 days-aged cultured spinal neurons on their coverslip were submerged in normal Krebs solution containing 130.5 mM NaCl, 2.4 mM KCl, 2.4 mM $CaCl_2$, 19.5 mM $NaHCO_3$, 1.3 mM $MgSO_4$, 1.2 mM $KH_2PO_4$, 1.25 mM HEPES, 10.0 mM glucose, pH 7.4, equilibrated with 95% $O_2$ and 5% $CO_2$, at room temperature (20-23°C). The coverslip was placed in a chamber and visualized by means of infrared DIC video-microscopy using an upright microscope BX51WI (Olympus France, Rungis, France) equipped with a 40x water immersion lens (LUMPlan-FI/IR), and a CCD camera C2400-03 (Hamamatsu, Hamamatsu City, Japan). The culture was continuously superfused with Kreb solution at 1.6 $mL.mn^{-1.}$ Patch pipettes (6-8 M Ω) were filled with 120 mM potassium gluconate, 20 mM KCl, 0.1 mM $CaCl_2$, 1.3 mM $MgCl_2$, 1 mM EGTA, 10 mM HEPES, 0.1 mM GTP, 0.2 mM cAMP, 0.1 mM leupeptin, 3 mM $Na_2$-ATP and 77 mM D-mannitol, pH 7.3. Series resistance was negligible. Miniature EPSCs were recorded in voltage clamp mode (holding potential -60 mV) in the presence of 10-6 M Tetrodotoxin (TTX). In such experimental conditions, no action potential could be evoked following injection of positive current into the recorded neuron, demonstrating a complete blockade of action potential propagation. Signals were amplified using an Axoclamp 2B amplifier and digitized at 10 kHz. Individual events were extracted from raw data using mini analysis software over a duration of 2 min and visually filtered according to their general shape.

**EXAMPLE 13**

**Materials and methods for Examples 13 and 14**

**A. Total RNA extraction (*Chlorella*)**

200-800 ml of liquid *Chlorella* culture ($5 x10^6$ - $1 x 10^7$ cells/ml) were harvested by centrifugation (Beckman rotor JA18, 10000g, 10', 4°C), the pellet washed in 1X PBS and flash frozen in liquid nitrogen. The frozen pellet was ground to a fine powder in liquid nitrogen, using a mortar and pestle. Total RNA extraction was performed using a TRI Reagent® RNA isolation reagent (Sigma, St. Louis, MO) according to manufacturer's instructions, using about 100 mg of powder.

**B. Stomatal reopening assay**

To ensure full expansion of stomata, plants (4/5 weeks old, 8h/16h light/dark photoperiod) were kept under light (100 $\mu E/m^2/s$) for at least 3 hours before subjecting them to any treatment. Intact young leaf sections, at least 6 per

condition, were immersed in water or bacterial suspension (at a concentration of $10^8$ cfu/ml, $OD_{600}$ =0.2). One hour prior to the bacterial infection, the sections were treated with either the MEVs (from ≈10 pM) or total RNAs (20 μg). At 3 hours of infection with the bacteria, the leaf sections were labelled for 10 minutes with Propidium Iodide (10 ng/ml in $H_2O$), washed 5 minutes in $H_2O$ and observed under a SP5 laser scanning confocal microscope. For each condition, about 10-15 pictures were taken from different leaf surface regions. From the pictures, at least 60 stomata per condition were manually measured using ImageJ 1.53c to obtain their width and length. The width/length ratio was calculated using excel and statistical analysis performed using the Analysis of Variance (ANOVA) test.

**C. Stomatal closure assay**

Plants (4/5 weeks old, 8h/16h light/dark photoperiod) were kept under light (100 $\mu E/m^2/s$) for at least 3 hours before subjecting them to any treatment to ensure full expansion of stomata. Intact young leaf sections, at least 6 per condition, were immersed in water and treated with either the MEVs (from ≈10 pM) or flg22 1 μM (QRLSTGSRINSAKD-DAAGLQIA, SEQ ID NO:50, provided from GeneScript). At 1 hour post treatment, the sections were stained with Propidium Iodide, washed in water and observed under SP5 laser scanning confocal microscope. All these steps, including the data analysis were performed as described in the stomatal reopening assay protocol.

**D. Seedling growth-inhibition assay**

Seedlings were grown for 7-8 days on plates containing 13 Murashige and Skoog (MS) medium (Duchefa), 1% sucrose, and 1% agar under a 16h/8h photoperiod at 22°C. Seedlings then were transferred into MS liquid medium (one seedling per 500 μl of medium in wells of 24-well plates) containing MEVs loaded with the flg22 peptide (QRLSTGSRINSAKDDAAGLQIA, SEQ ID NO:50, provided from GeneScript), the flg22 peptide alone, water or control MEVs. The fresh weight of control *versus* treated seedlings from Col-0 genotypes was measured 7-8 days after.

**E. RT-qPCR quantification of Pattern Triggered Immunity (PTI) marker genes**

To quantify the expression of PTI markers in *Arabidopsis,* leaves of 4/5 week old plants were infiltrated with *Chlorella* MEVs loaded with flg22 1 μM, water or control MEVs. Following the infiltration, 3, 6 and 9 hours after the treatments the leaves were collected and frozen in liquid nitrogen. The frozen leaves were ground to powder with mortar and pestle before performing the RNA extraction with the NucleoSpin® RNA Plant kit (Macherey-Nagel, 740949.250), according to manufacturer's instructions and using about 100 mg of powder. Total RNA was quantified using a NanoDrop® system and 500 ng digested with the DNAse I (RQ1 RNase-free DNase, Promega, M6101) to eliminate all contaminating genomic DNA. The DNAse treatment was performed according to the manufacturer's protocol. The digested RNA was then retro-transcribed using the qScript® cDNA SuperMix (Quantabio, 733-1178) as described by the manufacturer. The resulting cDNA was diluted 1:3 in water and 1 μl used to set up a qRT-PCR reaction using the appropriate gene-specific oligonucleotides and the Takyon™ No ROX SYBR 2X MasterMix blue dTTP (Eurogentec, UF-NSMT-B0701) mix. The qRT-PCR was performed with a "two steps" protocol with a first denaturation step at 95°C for 1' followed by 45 cycles of: 95°C for 10s and 60°C for 40s. A final step of melting curve generation was performed at the end of the reaction. Expression was normalized to that of the *Arabidopsis Ubiquitin* gene (Pfaffl (2001) Nucleic Acids Res. 29(9):e45). The PTI-marker genes analyzed were: *WRKY29* (Fw: TATCCAACGGATCAAGAGCTGA; Rv: TTTTCTTGCCAAACACCCTTTT), *FRK1* (Fw: TATCTTGAGCTGGGAAGAGAGG; Rv: AGTCGAATAGTACTCGGGGTCA) and *WRKY53* (Fw: CAGACGGGGATGCTACGG; Rv: GGCGAGGCTAATGGTGGTG) (SEQ ID NOs: 51, 52, 53, 54, 55, and 56, respectively).

**F. Cell death assay by Trypan Blue staining**

About 16-20 leaves infiltrated with *Chlorella* MEVs containing GUS or AvrRpm1 proteins (SEQ ID NOs: 60 and 61, respectively) were transferred in the Lactophenol Trypan Blue Solution (10 ml lactic acid [DL Sigma L-1250], 10 g glycerol, 10 ml $H_2O$ and 10 mg trypan blue) diluted in ethanol 1:1 and boiled for 1-2 min. The leaves were then destained overnight in chloral hydrate (2.5g/ml) and washed several times with water (5-6 times, 10') before the observation under an Olympus Macro Zoom System microscope. Trypan blue staining was quantified using ImageJ 1.53c.

**G. Bacterial strains**

The *Pto* DC3000Δ*hrpL* strain was a gift from Dr. Cayo Ramos. The *Pto* DC3000-WT-HrpL and -mut-HrpL strain, the *Pto* DC3000ΔhrpL strain, express the $NPTII_{pro}$: WT-HrpL and $NPTII_{pro}$: mut-HrpL plasmids, respectively, and were previously characterized (International PCT application No PCT/EP2019/072169 and PCT/EP2019/072170, published as International PCT Publication Nos. WO2020035619 and WO2020035620, respectively).

### H. dsRNAs and sRNAs *in vitro* synthesis

The templates for the *in vitro* synthesis were amplified by PCR, introducing the T7 promoters at both the 5' and 3' ends of the oligonucleotide sequences. PCR products were gel-purified using the NucleoSpin® Gel and PCR Clean up kit (Macherey-Nagel) to eliminate any parasite amplification, and the purified PCR products were quantified and directly used for the *in vitro* RNA synthesis. The *in vitro* synthesis was performed using the MEGAscript® RNAi Kit (Life Technologies, Carlsbad, CA), following the manufacturer's instructions. 2 μg of PCR product were incubated for ON at 37°C with 2 μl of T7 Polymerase (T7 Enzyme Mix), 2 μl of 10X T7 Reaction Buffer and 2 μl of ribonucleotides (ATP, CTP; GTP and UTP, each at 75 mM) in a total reaction volume of 20 μl (adjusted with Nuclease-free water).

After the transcription reaction, the dsRNAs were treated with 2 μm of DNAse I, 2 μl of RNAse and 5 μl of Reaction Buffer to eliminate the DNA template and the ssRNAs. The dsRNAs were purified with filter cartridges provided with the kit and the long dsRNAs obtained after this step were quantified with the Nanodrop and directly used for the subsequent experiences. siRNAs were obtained by digesting the purified long dsRNAs with the ShortCut® RNAse III (NEB, Ipswich, MA). The digestion was performed for 30' at 37°C and the siRNAs purified using the mirVana™ miRNA Isolation kit (Life Technologies, Carlsbad, CA). The purified siRNAs were directly used for the subsequent experiences. At each step of the protocol, gel electrophoresis (TAE 1X, 1% agarose for DNA and 2% for RNAs) was performed to check the quality of the different nucleic acids.

### I. Infection Assays with *Fusarium graminearum* spores

For all *in vitro* infection assays, $5 \times 10^4$ conidia per $mL^{-1}$ were used for each of infection on leaves and on PDA (Potato Dextrose Agar) plates. Assays on PDA plates were performed by incubating for 2 hours at RT (about 25°C) the *F. graminearum* spores with different amount of *cyp51* dsRNAs-loaded *Chlorella* EVs. A total volume of 300 μl was deposited onto the PDA plate surface and dried in sterile conditions. To determine the efficacy of growth inhibition, the exogenously loaded vesicles were compared with total RNAs and *Chlorella* MEVs from a stable cell line, designated *Chlorella cyp51* IR transgenic lines (IT19), and a reference fungicide. Pictures of the plates at 24, 48 and 72 hours were taken, and the amount of developed mycelium was measured in order to determine the growth inhibition. Inoculation of *Arabidopsis* was done by wound inoculation on detached leaves with *F. graminearum.* About twenty rosette leaves of 15 different 4/5 weeks old Wt plants were detached and transferred in square Petri plates containing 1% agar. The exogenously loaded MEVs were deposed onto the surface of each leaf and let briefly dry. Inoculation of *F. graminearum* spores was carried out as previously described (Koch A et al., (2012) J Phytopathol 60:606-610). The progression of disease symptoms was determined by measuring the lesion size (in centimeters) at 3 dpi from the digital pictures of the petri dishes using the ImageJ 1.53c analyzer. These measures allowed to calculate the percentage of leaf area showing infection symptoms relative to the non-inoculated leaf.

### J. β-glucuronidase (GUS) staining assay

For this assay, about 20 leaves from 4/5 weeks old plants were infiltrated with the MEVs suspension at different concentrations. About 24/48 hours post infiltration, the leaves were detached, immersed in the GUS staining solution and vacuum infiltrated 2/3 times for 15' each. The plates containing the leaves are sealed with parafilm and incubated ON at 37°C. The following day several washes with 75% EtOH were performed until the leaves becomes completely transparent and only the blue GUS staining was visible. The GUS staining solution contains 1mM X-Gluc, 50 mM PBS pH 7.2, 10 mM EDTA pH 8, 0.5% Triton X-100. Pictures of the stained leaves are taken and the blue signal quantified using ImageJ 1.53c.

## EXAMPLE 14

### Determining the biological activity of Chlorella MEVs exogenously loaded with siRNAs targeting the Pto DC3000 hrpL virulence factor

The endogenous loading of MEVs was tested by generating transgenic *Chlorella* lines expressing an inverted repeat (IR) transgene carrying sequence homology with two major virulence factors of *Pto* DC3000. The first targeted *Pto* DC3000 virulence factor is the coronafacic acid polyketide synthase I (*cfa6*) gene, which encodes a major structural component of the phytotoxin coronatine (COR) (Brooks et al. (2004) Mol Plant Microbe Interact. 17(2):162-174). The second one is *hrpL,* which encodes an alternative sigma factor that is known to directly control the expression of type III-secretion system associated genes, and to indirectly regulate the expression of COR biosynthesis genes (Fouts et al. (2004) Proc. Natl. Acad. Sci. U.S.A. 99:2275-2280; Sreedharan et al. (2006) Molecular plant-microbe interactions MPMI 19:768-779).

To demonstrate that the exogenous loading of siRNAs in *Chlorella* MEVs can be effective, small RNA duplexes

directed against *Pto* DC30000 *hrpL* (SEQ ID NO:57) were synthesized *in vitro* to load exogenously into *Chlorella* MEVs, and to test their biological efficacy by monitoring their ability to suppress *Pto* DC3000-induced stomatal reopening. In this assay, the efficacy in inhibiting the stomatal reopening events by siRNA-loaded MEVs was compared with the events triggered by MEVs from stable *Chlorella* transgenic lines expressing siRNAs directed against *cfa6* and *hrpL* (described in International PCT application Nos. PCT/EP2019/072169 and PCT/EP2019/072170, published as International PCT Publication Nos. WO2020035619 and WO2020035620, respectively). Synthetic siRNA duplexes (1 μg) were loaded into *Chlorella* MEVs (sample 12). To exclude the possibility that the observed effects depend on siRNAs contamination, the samples were treated with Micrococcal nuclease (MNase), a broad range RNAse able to digest all unprotected RNAs that are either in a single-stranded or double-stranded forms. Empty MEVs were used as negative control and MNase-treated MEVs from the transgenic line IT20-3, expressing siRNAs targeting the *Pto* DC3000 *cfa6* and *hrpL* genes, were used as positive control. For the assays, all the vesicles were used at a concentration of about 20 pM (≈1,2E10 particles/ml). The results revealed that the siRNA-loaded MEVs are able to fully inhibit the stomatal reopening to the same extent as MEVs from the stable IT20-3 transgenic line (Figure 26). Furthermore, the sample maintained its biological activity upon the MNase treatment, indicating that the *Chlorella* MEVs protect siRNA from enzymatic degradation. These results further indicate that a sufficient quantity of exogenously-loaded antibacterial siRNAs are located inside the EVs to elicit the expected biological activity. Overall, the findings prove that *Chlorella* MEVs can be exogenously loaded with biologically-active siRNAs and that they can protect such small RNAs from enzymatic degradation, and that these siRNAs are delivered into the cell in sufficient quantity to trigger the specific biological activity.

Stomatal reopening assay at 3 hpi on leaf sections incubated with different MEVs one-hour prior infection. As controls, empty MEVs (Ctl-) were used as a negative control. MEVs from the *Chlorella* stable transgenic line IT20-3 (Ctl+) that encodes and expresses siRNAs targeting the *Pto* DC3000 virulence genes *cfa6* and *hrpL,* was used as positive control. The IT20-3 cell line encodes the siRNAs, and, thus, is endogenously loaded (see, International PCT Publication No. WO/2022/053687; see, also EP 3967746).

The siRNAs sample 12 corresponds to MEVs exogenously loaded with the synthetic siRNA duplexes targeting the *cfa6* and *hrpL* genes. The MNase treatment was performed for 15' at 37°C on the siRNAs sample 12 as well as on the Ctl+ vesicles. N, total number of analyzed stomata. Statistical analyses were performed using one-way ANOVA and comparing the mean of each condition with the means of all other conditions (p-value: ns >0.05; ***<0.001; ****<0.0001).

**EXAMPLE 15**

**Determining The Biological Activity Of Chlorella MEVs Exogenously Loaded With dsRNAs To Target The Cyp51 Genes In The Plant Pathogenic Fungi Fusarium Graminearum**

*F. graminearum* is a pathogenic ascomycete fungus that causes devastating diseases in cereal crops: the *Fusarium* head blight (FHB) and the root rot. It has been recently shown that Host Induced Gene Silencing (HIGS) strategies using dsRNAs targeting three paralog cytochrome genes, *cyp51A*, *B* and *C*, are effective in limiting the growth of this fungal pathogen in *in vitro* conditions (Koch et al. (2013) Proc. Natl. Acad. Sci. U.S.A. 110:19324-19329). In the study reported in Koch *et al.,* the authors demonstrated that the same fungal growth inhibition can be achieved by spraying the dsRNA onto the leaf surface prior to the fungal infection (Spray Induced gene Silencing; see, Koch et al. (2016) PLOS Pathogens 12(10): e100590). To demonstrate that antifungal dsRNAs can be exogenously loaded into *Chlorella* MEVs, *in vitro* synthesis of dsRNAs bearing sequence homologies against *cyp51A*, *B* and C was performed to load them into *Chlorella* MEVs, and to further test their antifungal activity *in vitro* and *in planta* assays. To perform this test, two *in vitro* approaches were employed: an analysis of *F. graminearum* growth onto PDA plates and on detached *Arabidopsis* leaves. In both cases, a MEV concentration carrying the equivalent of 800, 80 and 8 ng/ml of *cyp51* (SEQ ID NO:58) dsRNA were used and, as controls, naked *cyp51* dsRNAs, the total RNAs and MEVs from a transgenic *Chlorella* line (IT19) and a reference fungicide also were applied.

**EXAMPLE 16**

**Determining The Biological Activity Of Chlorella MEVs Exogenously Loaded with mRNA and Proteins**

Two different approaches to characterize the biological activity of the loaded *Chlorella* MEVs with mRNAs or proteins: 1) the GUS staining assay in *Arabidopsis* leaves using the GUS mRNA and protein (SEQ ID NOs: 59 and 60, respectively); and 2) the plant cell death assay, also known as the hypersensitive response (HR). This assay makes use of the protein encoded by the bacterial effector *AvrRpm1* (SEQ ID NO:61), which is known to be recognized by the intracellular host immune receptor RPM1 and to trigger a potent HR in *Arabidopsis* accessions expressing a functional copy of RPM1 (*e.g.,* the Col-0 reference accession). For the first approach, we syringe-infiltrated different concentrations of MEVs loaded with either the GUS mRNA or protein directly in detached *Arabidopsis* leaves. Single mRNA-embedded

MEVs with concentration, equivalent to 100 ng/µl of mRNA, and three different amounts of protein-loaded MEVs, equivalent to 1, 5 and 10 µg/ µl of GUS protein were used. After the infiltration of the MEVs, the leaves were vacuum infiltrated with the GUS staining solution and left incubating overnight for one night. The quantification of the blue signal resulting from the activity of the GUS enzyme allowed the direct comparison between the mRNA and protein delivery efficiency. For the second approach, the MEVs loaded with the effector protein AvrRpm1 were infiltrated in *Arabidopsis* leaves. The macro-HR was monitored by Trypan blue staining on at least 30 *Arabidopsis* Col-0 leaves treated with either MEVs carrying the GUS (negative control) or the AvrRpm1 proteins.

**EXAMPLE 17**

**Determining the biological activity of Chlorella MEVs exogenously loaded with peptides**

Small peptides ranging from 10 to 50 amino acids are potent effectors able to mediate a variety of responses in cell-to-cell communication, plant development, plant immune responses, and other processes and pathways. Such classes of molecules also can trigger the first layer of the immune defense through the Pathogen Associated Molecular Patterns (PAMPs) response, a mechanism that relies on the recognition of molecules released from the pathogen, including peptides, from the cell surface receptor of the host. In this context, a 22 amino acid synthetic peptide that mimic the conserved N-terminal region of the bacterial flagellin, which is sensed by the surface receptor Flagellin Sensing 2 (FLS2), thereby triggering a potent immune response in various plant species, is used. Different concentrations of the synthetic peptides flg22 (10 nM, 100 nM and 1µM; SEQ ID NO:50) are loaded in *Chlorella* MEVs and the ability of these MEVs to trigger hallmarks of PAMP-triggered immunity, such as flg22-induced stomatal closure, flg22-induced growth inhibition and/or flg22-triggered induction of PTI marker genes is assessed. As negative controls, empty MEVs and the flg22 peptide at 1 nM, for which no biological activity was expected, were used in the assay, whilst as positive controls, 3 different concentrations of the synthetic peptide flg22 (10 nM, 0.1 and 1 µM) were used. Two additional controls were also included in order to eliminate any possible flg22 peptide contamination still present after the MEVs preparation and to test whether *Chlorella* MEVs are indeed able to protect the peptide from the protease activity. To this aim, the flg22-loaded MEVs were treated either with proteinase K (ProK) alone (+ProK condition), or in combination with Triton™ X-100 (+ProK +Triton), which is a detergent proven to disrupt plant EVs (Rutter and Innes, 2017). The findings revealed that *Chlorella* MEVs exogenously loaded with flg22 peptides are able to induce stomata closure even after the proteinase K treatment. These data indicate that, when loaded, the flg22 peptide is bioactive, efficiently delivered to the host cells, and protected from enzymatic degradation by the EVs (Figure 27). The additional Triton treatment abolished such biological activity, likely by altering the integrity of *Chlorella* MEVs, thereby allowing ProK to degrade the flg22 peptide. This indicates that *Chlorella* MEVs protect the flg22 peptides located inside MEVs from ProK-triggered degradation. The results show that the biological effects on the stomata triggered by the flg22-loaded EVs are comparable to at least 10 nM of free flg22 peptide. Based on the concentration of MEVs used (about 16 pM (≈1E10 particles/ml)), it is estimated that each vesicle was loaded with more than $10^2$ peptide molecules in order to trigger the observed effect on stomata. Overall, the results show that *Chlorella* MEVs can be exogenously loaded with biologically active peptides, that the MEVs can protect the peptides from enzymatic degradation, and that these peptides are delivered into the cell in sufficient quantity to trigger the specific biological activity.

Stomatal closure assay at 1.5 hours post-treatment on *Arabidopsis thaliana* (accession Columbia-0 (Col-0)) leaf sections incubated with the flg22 peptides and MEVs loaded with the same molecule. As controls, empty EVs (Ctl-) and 1 nM of the flg22 peptide were used. Different concentrations of the flg22 peptide (10 nM, 0.1 and 1 µM) were used as positive control. The flg22-loaded EVs were either left untreated (-ProK) or incubated with the proteinase K alone (+ProK) or in combination with 1% of Triton X100 (+ProK +Triton), in order to remove possible flg22 contaminations (ProK alone) and/or affect the vesicle integrity. N, is the total number of analyzed stomata. Statistical analyses were performed using one-way ANOVA and comparing the mean of each condition with the means of all others conditions. A) and B) denote different statistical groups determined by analyzing the pairwise table from the ANOVA analysis (statistical visualization using the compact letter display format).

The results in these examples and description show that microalgal MEVs, particularly *Chlorella* MEVs, can be exogenously loaded with bioactive cargo that can be delivered to cells and tissues, and the cargo is active. Based on this result and the other data and results provided herein, *Chlorella* MEVs can be loaded exogenously bioactive cargo, delivered to cells and tissues, where the bioactive cargo is active.

**EXAMPLES 18-31 SHOW AND DESCRIBE BIODISTRIBUTION OF MEVs FOLLOWING INTRANASAL (IN) ADMINISTRATION**

**EXAMPLE 18**

**Pharmacokinetics and biodistribution of MEV to specific regions of the brain.**

MEVs produced, purified, characterized, and labelled with DiR as described in in Examples 1 and 2, above (see, also copending International PCT application No. PCT/EP2022/070371, which describes exogenous loading, and copending U.S. provisional application Serial No. 63/349,006, which describes endogenous loading) were administered to C57BL/6 mice by intranasal administration. Mice were euthanatized at several time points (1h, 2h, 4h and 8h) after single intranasal administration (20 mL in each nostril) per animal; the brains were carefully isolated; brains were sectioned, embedded in OCT at max. 30 min post sampling and sliced at different distance of the bregma and kinetics thereof (see, Figures 36-46; shown are at the end of this Example). Figure 29 shows a positive control DiR-MEV on DAPI-stained brain slice in which a drop of MEV suspension was deposited on top of a brain tissue slide; the red dots are DiR-labeled MEV.

**A. Intranasal administration**

Mice were housed in Makrolon® polycarbonate cages with filter hoods, in a room where the air is continuously filtered to avoid contamination. During experiments, paired animals were caged at a constant temperature with a day/night cycle of 12/12 hours.

Animals received water (control tap water) and nutrition *ad libitum*. Animal health was examined every day to ensure that only animals in good health enter to the testing procedures and follow up to the study. At the beginning of the study, animals were identified with a number on the tail and separated in 5 groups:

Group 1: PBS-treated animals and sampling at 1h
Group 2: MEV treated animals and sampling at 1h
Group 3: MEV treated animals and sampling at 2h
Group 4: MEV treated animals and sampling at 4h
Group 5: MEV treated animals and sampling at 8h

Intranasal compound administration was made using a dominant hand, the micropipette was loaded with 20 μl of MEV (for the first nostril). The tip of the filled pipette was placed near the mouse's left nostril, usually at a 45-degree angle. The droplet was placed close enough to the mouse's nostril so that the mouse could inhale the droplet. This procedure was reproduced for the second nostril. A total of 40 μl of MEV per animal was administrated. After full administration, the mouse was held in this position for 15 seconds.

**B. Organ sampling**

Mice were sacrificed by cervical dislocation. The temporal bone was opened, and the brain was sampled. Each sample rapidly was segmented into five parts (see, Figure 36), placed in single cryoblocks, and embedded with O.C.T. compound using isopentane and stored at -80 °C. Samples were cryosectioned, labeled with DAPI fluorescent staining and/or Cresyl violet staining.

**C. Imaging and Data Analysis**

The slide images were analyzed to determine the presence of MEV-labelled with DiR in DAPI and cresyl violet staining using an Akoya Phenochart™ whole slide viewer. Descriptive analysis by groups was expressed as mean ± SD for continuous variables. Each brain was analyzed independently. The cresyl violet staining was used to reference-estimate the DiR analyzed brain areas for each section.

**Quantification of MEV-DiR in the brain section 1 (+3.92 mm from the bregma).** For the section 1, the MEV-DiR was detected in left and right olfactory nerve layer from 1- hour post administration reaching a plateau at 4 hours post administration (Figures 37 and 38).

**Quantification MEV-DiR in the brain section 2 (+1.78 mm from bregma).** A progressive increase of MEV-DiR normalized intensity and number was observed in the primary motor cortex, piriform cortex, frontal cortex and agranular insular cortex from 2-hours post administration. Even if the number of animals per time-point was too small to profile a precise PK curve, the graphical representations suggest the MEVs reached a plateau in the primary motor cortex, frontal cortex and agranular insular cortex at 8 hours post administration (Figures 39 and 40). Data indicate a progressive diffusion of the MEVs through primary motor cortex, piriform cortex, frontal cortex and agranular insular cortex when administrated by the IN route in mice.

**Quantification of MEV-DiR in the brain section 3 (-1.82 mm from bregma).** A progressive increase of MEV-DiR normalized intensity and number was observed in the primary somatosensory cortex, auditory cortex, basolateral amygdaloid nucleus, retrosplenial granular cortex, temporal association cortex and the arcuate hypothalamic nucleus

from 2-hour post administration. Even if the number of animals per time-point was too small to profile a precise PK curve, the graphical representations suggest the MEVs reached a plateau in the primary somatosensory cortex, auditory cortex, basolateral amygdaloid nucleus and the retrosplenial granular cortex, but not in the temporal association cortex and the arcuate hypothalamic nucleus, at 8 hours post administration (Figures 41 and 42). These data indicate a progressive diffusion of the MEVs in the described brain regions when administrated by the IN route in mice.

Quantification of MEV-DiR in the brain section 4 (-2.70 mm from bregma). A progressive increase of MEV-DiR normalized intensity and number was observed in the amygdala, left and right auditory cortex, temporal association cortex and ectorhinal cortex, the left and right primary visual cortex and the mammillary nucleus from 4-hour posts administration (Figures 43 and 44). These data indicate a progressive diffusion of the MEVs in the described caudal brain regions when administrated by the IN route in mice.

Quantification of the MEV-DiR in the brain section 5 (-5.20 mm from bregma). No MEV-DiR was detected in the principal trigeminal nucleus, inferior colliculus and tegmental nucleus, parabrachial nucleus and cerebellar peduncle and any other caudal area at the analyzed time point. The lack of MEV-DiR can be explained by a lack of diffusion into these brain areas up to 8 hours post administration (Figure 45).

These results show that the intranasal administration of the MEVs leads to a progressive biodistribution of the vesicles in different but specific brain areas. This biodistribution is time-dependent and observed from the rostral to the caudal regions of the mouse brain. One hour after MEVs administration, the vesicles were specifically detected in the olfactory nerve layer but not in other more caudal regions. At two hours post MEV administration, the vesicles reached cortical regions and reached primary motor cortex, piriform cortex, frontal cortex, agranular insular cortex, primary somatosensory cortex, auditory cortex, retrosplenial granular cortex and temporal association cortex. MEVs also were found in basolateral amygdaloid nucleus, and the arcuate hypothalamic nucleus. No fluorescent signal was detected in the hippocampal regions, caudate putamen or in the nucleus accumbens. At four hours post administration, the MEV migrated up to the caudal brain regions reaching the amygdala, the left and right auditory cortex, the temporal association cortex and the ectorhinal cortex. No labelling was observed in hippocampus or substantia nigra. No vesicles were detected in the most caudal section examined where important structures, such as the trigeminal nucleus, inferior colliculus, tegmental nucleus, and parabrachial nucleus from 1 to 8 hours post administration indicating that the vesicles did not reach the most caudal part of the brain during the analyzed time points.

It is known that direct nose-to-brain transport of drugs and biologics (proteins, oligonucleotides, or viral vectors) is feasible via the olfactory or trigeminal nerve system. Olfactory nerve axons originating in the olfactory bulb (OB) penetrate the cribriform plate and terminate at the apical surface of the olfactory neuroepithelium; it is located at the roof of the nasal cavity. Filaments of the olfactory nerves are present in the anterior and in the posterior parts at the middle turbinate. The respiratory mucosa is densely innervated by sensory and parasympathetic trigeminal nerves and is more extensive than the olfactory nerve. Sensory maxillary branches innervate the deepest nasal segments, including the olfactory cleft. The pathways traversed by the MEVs, as shown herein, however are different.

**Figures 36(a) -(g) provide:** (i) a general overview of the experimental design of brain biodistribution studies; and (ii) the positions of the 5 brain sections studied; (c-g) the regions analyzed to determine the PK and biodistribution of MEVs in each of the 5 brain sections studied. The graphs depict and identify regions of the brain for reference with the figures 37, 39, 41, 43, and 45 that show MEVs in the brain following IN administration.

## EXAMPLE 19

### Distribution of MEVs in the bulbar region, in the hypothalamic region and in the cerebellar region of the brain following intranasal administration.

A. MEVs produced, purified, characterized, and labelled with DiR as described herein (see, also copending International PCT application No. PCT/EP2022/070371, published January 26, 2023, and copending U.S. provisional application Serial No. 63/349,006; see also U.S. provisional application Serial No. 63/224,656) were administered in C57BL/6 mice by intranasal administration. Mice were euthanized at 16 h after single intranasal administration (20 mL in each nostril) per animal; the brains were carefully isolated; brains were sectioned, embedded in OCT at max. 30 min post sampling and sliced at different distance of the bregma.

**B.** 8-week-old Swiss mice were treated by IN administration with up to 20 μl of a MEV suspension. 16 hours after, mice were rapidly anesthetized, perfused with 4% (weight/vol.) paraformaldehyde antigen fix solution (PFA). The brains were taken out from the skull with a special care not to damage the olfactory bulbs, post-fixed in 4% PFA and soaked in 30% sucrose.

**C.** Fluorescently labeled (DiR) MEVs were detected in the brain by histological examination. Briefly, the brains were cut using a cryostat into free-floating 20-μm coronal sections. Slices were selected in the bulbar region, in the

hypothalamic region and in the cerebellar region. The sections were mounted on microscopic slides are analyzed for fluorescence as follows:
DNA was stained with DAPI to visualize the brain tissue by labeling the nuclei of all the cells.

MEVs were labelled prior to the study with DiR as described previously and visualized accordingly example 1.
Positive control slides were prepared using MEV suspension alone see Figure 2b. The transport of MEVs into the brain was evaluated based on the level of fluorescently labeled MEVs in the analyzed areas of the brain: olfactory bulb, hypothalamus, and cerebellum.
D. Results obtained allowed to identify DiR staining inside the brain of mice after IN administration of DiR stained MEVs. The DiR staining can be detected in different brain regions all along the analyzed part of the brain from Bregma 4 mm to Bregma -1.5 mm, such as the olfactory bulbs, the corpus callosum, the cortex and the internal capsule, the thalamus and at different levels of the hippocampus (fimbria and close to dentate gyrus).

**EXAMPLE 20**

**Pharmacokinetics and biodistribution of MEV through the neuronal circuitry and networks in the brain.**

***3D biodistribution of MEVs in the brain following IN administration.***

The presence of fluorescent labelled MEVs was determined in the brain processed using CLARITY method by 3D imaging. Swiss mice (8 weeks old) were treated by intranasal administration of 40 μl (20 μl/nostril) of MEV suspension. 4, 8, 16 and 24 hours after the administration, whole brain was fixed with CLARITY and 3D imaging was performed. Briefly, hydrogel monomer solution and clearing solutions were prepared as published previously (Chung et al. (2013). Structural and molecular interrogation of intact biological systems. Nature 497, 332-337). Deeply anesthetized animals were perfused with ice-cold PBS and subsequently with the hydrogel monomer solution. Organs of interest were carefully harvested, placed immediately in ice-cold hydrogel monomer solution and kept in the dark for one day at 4°C. Then, samples were placed in tubes with fresh hydrogel monomer solution and transferred to a desiccation chamber for oxygen removal. Tubes were filled with nitrogen and allowed to incubate at 37°C for two hours with gentle shaking, followed by three washing steps with clearing solution at 37°C for 24 hours, again with gentle shaking. Next, the samples were subject to Electrophoretic Tissue-Clearing (ETC) for several days using an ETC chamber and the following conditions: voltage of 40 V, minimum pH of 7.3, maximum temperature of 37°C. Cleared samples were removed from the ETC system, washed twice with PBS for 24 hours each and completely covered with optical clearing solution for 2-day incubation, keeping the samples airtight and changing the solution daily. Finally, 3D imaging was performed on the fixed and cleared tissue samples.

**EXAMPLE 21**

***In vitro* - Internalization of MEVs by human iPSC-derived neural cells and expression of payload (mRNA, or protein or small molecules).**

MEVs can load a wide variety of cargo, such as siRNAs, mRNAs, small molecules, peptides, and proteins. To evaluate MEVs as a delivery system for therapeutic purposes to the central nervous system, the internalization of GFP-loaded MEVs by human brain cells derived from induced pluripotent stem cells was assessed. Human neural stem cells, astrocytes, glial cells and neurons, validated with cell type-specific markers, were used for the internalization assays.

**A.** MEVs were produced, loaded, and characterized as described in above.
**B.** Culture, differentiation, and characterization of derived cells from human induced pluripotent stem cells (hiPSCs).

Human induced pluripotent stem cells (hiPSCs) are cultured under a neural induction medium to generate neural stem cells (NSCs) according to the protocol described in Yan et al. (2013) NeuroImage 80:246-262. NSCs are expanded in NEM medium (Advanced DMEM/F12 and Neurobasal supplemented with Neural Induction Supplement). NSCs exhibit neural progenitor markers and are negative for pluripotency markers, indicating a commitment to the ectodermal lineage. During brain development, NSCs are capable to give rise to all neural cells (neurons, astrocytes, and oligodendrocytes) and have the capacity for self-renewal. In adulthood, NSCs persist in the hippocampus and in the subventricular zone (SVZ) of the lateral ventricles. These are the most studied regions capable to generate new neural cells and to contribute to neural plasticity. Human neural stem cells exhibited positive staining for Nestin and PAX6, both neural stem cell markers. No OCT4 positive cells were detected in NSCs, which is a pluripotency marker highly expressed in hiPSCs (Casas *et al,* 2018).

To generate astrocytes, NSCs are cultured under astrocyte differentiation medium (DMEM/F12 supplemented with N2 and 1% fetal bovine serum) for 21 days according to the protocol described in Yan Y *et al.,* 2013. After the differentiation stage, astrocytes are cultured for 5 weeks under astrocyte medium (DMEM/F12 10% fetal bovine serum) to ensure cell maturation. Mature astrocytes were previously characterized (Ledur *et al.,* 2020 and Trindade *et al.,* 2020) by the expression of main astrocytic markers (as ALDH1L1, GFAP, Vimentin, EAAT1, EAAT2, and S100b) and exhibit functional characteristics (as Human iPSC-derived astrocytes display impairment of [3H] D-aspartate uptake after TNF-$\alpha$ exposure. [3H] D-aspartate is a nonmetabolized analog of glutamate that is commonly used to measure the uptake activity of glutamate transporters and evaluate astrocyte functionality); Ledur *et al*., 2020 and Trindade *et al.,* 2020, respectively.

NSCs are differentiated in mixed neuronal culture on laminin-coated plates and under neuronal differentiation medium (Neurobasal supplemented with 1X B27, 1X Glutamax, 1X Non-Essential Amino Acids Solution, and 200nM ascorbic acid) (Yan Y *et al.,* 2013). On day 7 and 14 of the differentiation protocol, cultures are detached with Accutase and replated in laminin-coated coverslips in neuronal differentiation media containing 10 $\mu$M ROCK inhibitor to improve neuronal survival.

Neurons are cultured until day 45 to perform experiments and ensure proper maturation. The characterization uses neuronal markers, such as b-tubulin III (BTUBIII), microtubule-associated protein 2 (MAP2), and presynaptic marker synaptophysin (SYP). Neurons are negative to Nestin, a neural stem cell marker, indicating the mature profile of the culture.

**C.** Culture and characterization of glial cells.

Primary human glial cells, obtained from Lonza, were isolated. The human glial cells were seeded at a density of 36,000 cells/cm$^2$ and cultured in DMEM supplemented with 10% FBS, 2 mmol/l GlutaMAX, 1 mmol/l sodium pyruvate, 100 units/ml penicillin, and 100 $\mu$g/ml streptomycin for one week. Glial cells were characterized using specific markers as glial fibrillary acidic protein (GFPA) and S100b, Vimentin and Nestin.

**D.** Evaluation of the internalization of MEVs in human neuron stem cells, human neurons, human astrocytes and human glial cells.

Cells were incubated with MEVs-mRNA eGFP or GFP (protein) and monitored for up to 24h for vesicle internalization using a live-cell imaging platform. Hoechst dye is used to stain nuclei. After image acquisition, GFP intensity is measured to estimate the internalization efficiency and the percentage of GFP positive cells is calculated.

**E.** Internalization, delivery, and analysis of MEVs loaded with mRNA-eGFP or GFP protein to human neural stem cells.

Human NSCs were incubated with MEV-GFP for up to 24h. Cells were analyzed on a live-cell imaging platform after 30 min of Hoechst incubation for nuclei staining. After image acquisition, GFP intensity was measured and GFP positive cells were calculated. Cells were immuno-stained for PAX6 for representative imaging of each cell type. Calculations made: Mean intensity GFP values and percentage of GFP positive cells.

**F.** Internalization, delivery, and analysis of MEVs loaded with mRNA-eGFP or GFP protein to human astrocytes.

Human astrocytes were incubated with MEV-GFP for up to 24h. Cells were analyzed on a live-cell imaging platform after 30 min of Hoechst incubation for nuclei staining. After image acquisition, GFP intensity was measured and GFP positive cells were calculated. Cells were immuno-stained for GFAP for representative imaging of each cell type. Calculations made: Mean intensity GFP values and percentage of GFP positive cells.

**G.** Internalization, delivery, and analysis of MEVs loaded with mRNA-eGFP or GFP protein to human neurons.

Human neurons were incubated with MEV-GFP for up to 24h. Cells were analyzed on a live-cell imaging platform after 30 min of Hoechst incubation for nuclei staining. After image acquisition, GFP intensity was measured and GFP positive cells were calculated. Cells were immuno-stained for MAP2 for representative imaging of each cell type. Calculations made: Mean intensity GFP values and percentage of GFP positive cells.

**H.** Internalization, delivery, and analysis of MEVs loaded with mRNA-eGFP or GFP protein to human microglia cells: human glial cells were incubated with MEV-GFP for up to 24h. Cells were analyzed on a live-cell imaging platform after 30 min of Hoechst incubation for nuclei staining. After image acquisition, GFP intensity was measured and GFP positive cells were calculated. Cells were immuno-stained for GFAP for representative imaging of each cell type. Calculations made: Mean intensity GFP values and percentage of GFP positive cells.

**EXAMPLE 22**

**MEV characterization after loading of an enzyme (catalase)**

***A. Nanoparticle Tracking Analysis (NTA)***

MEVs were produced, purified, loaded and characterized as described herein (see, also copending International PCT application No. PCT/EP2022/070371, which describes exogenous loading, and copending U.S. provisional application Serial No. 63/349,006, which describes endogenous loading). Once loaded with a payload (e.g. small-molecule, protein, peptide, siRNA, ASO, or mRNA), the MEVs were analyzed for size and fluorescence (size distribution) using the NanoSight® NS500 system (Malvern Instruments). The loaded-MEVs samples were diluted in particle-free PBS (0.02 μm filtered) to obtain a concentration within the recommended measurement range ($1\text{-}10\times10^8$ particles/mL). For each sample, 5 experiment videos of 60 seconds duration were analyzed using NTA 3.4 Build 3.4.003 (camera level 15-16) with syringe pump speed 30. A total of 1500 frames were examined per sample; they were captured and analyzed by applying instrument-optimized settings using a suitable detection threshold so that the observed particles are marked with a red cross and that no more than 5 blue crosses are seen. Further settings were set to "automatic" and viscosity to "water."

***B. Enzymatic activity of ME V-loaded catalase***

The loading efficiency for MEV formulations loaded as above was assessed by catalase activity assay using hydrogen peroxide decomposition. 1 mL of PBS (pH 7.4) was mixed into a quartz cuvette with 1-4 μL $H_2O_2$ (7.5-30% v/w) and 2 μL of catalase (0.06-0.5 mg/ml) or samples of cargo loaded MEVs, respectively. Enzymatic activity was measured by monitoring the absorbance at 240 nm using a PerkinElmer® Lambda 25 UV VIS Spectrophotometer (PerkinElmer Instruments). Catalase stability in the loaded MEVs was evaluated by incubation with pronase (0.1-0.2 mg/mL) for 3 hours at 37°C. The samples were subsequently assayed for catalytic activity as described above. Stability of catalase was expressed in the residual activity vs. initial activity of catalase.

**EXAMPLE 23**

***In vitro* - MEV-mediated delivery of a therapeutic payload to neurons**

***A. Uptake of loaded MEVs by neuronal cells - microplate assay.***

PC12 neuronal cells are used as a model for *in vitro* evaluation of neuroprotective effects. Briefly, PC12 cells are seeded into 96-well plate (50,000 cells/well) and cultured for three days. Next, the catalase carrying MEVs (loaded as described above) are stained with DiR (5 μg/mL) and added to the wells in serial dilutions for various times. Following the incubation, the cells are washed three times with ice-cold PBS and solubilized in 1% Triton X-100. The sample fluorescence is measured using a SpectraMax® fluorescence microplate reader (Molecular Devices, USA) with excitation at 750 nm and emission at 780 nm. The amount of MEVs accumulated in neuronal cells is normalized for total protein content and expressed as the number of MEVs per mg of protein. All MEV formulations are prepared at the same level of fluorescence, and a separate calibration curve is used for each MEV formulation.

***B. Uptake of loaded MEVs by neuronal cells - confocal microscopy.***

The catalase loaded MEVs (100 μg/mL total protein) are sonicated, stained with DiR (5 μg/mL) and incubated with PC12 cells grown on chamber slides ($1x10^5$ cells/chamber) for various time intervals. Following the incubation, the cells are washed with PBS, fixed in 4% paraformaldehyde and permeabilized with 0.1% Triton™ X-100. Fluorescent staining is performed using anti-actin antibody (Abcam ab179467, 1:100) with goat anti-rabbit IgG Alexa Fluor 488 (Abcam ab150077, 1:1000) as the secondary antibody. DAPI (4',6-diamidino-2-phenylindole) is used as nuclear counterstain prior to the imaging. Accumulation of fluorescently labelled MEVs is visualized by a LSM700 laser scanning confocal microscope (Zeiss) and images are processed with LSM Image Browser.

***C. Neuroprotective effects of the loaded MEVs.***

The protection of PC12 cells against 6-OHDA-induced cytotoxicity is assessed by MTT assay. For this purpose, PC12 cells ($1x10^5$ cells/mL) are seeded into a 96-well plate and allowed to attach overnight. Then, the cells are exposed to 200 μM 6-hydroxydopamine (6-OHDA) and different catalase-loaded MEV formulations, or catalase alone, or empty MEVs for four hours. Following the incubation, the cells are washed 3 times with ice-cold PBS, and incubated with the

corresponding catalase loaded MEV formulations, or catalase alone, or empty MEVs for another 24 hours. Following the treatment, 20 μL of MTT tetrazolium dye solution (5 mg/mL) is added into each well. After 3 hours of incubation at 37°C, the MTT-containing medium is removed and 100 μL DMSO is added into each well to dissolve the purple formazan precipitate. Absorbance is measured at 570 nm using a SpectraMax® microplate reader (Molecular Devices, USA) and cell viability is expressed as a percentage of viable cells in the treated groups compared to the untreated control group.

***D. Uptake of loaded MEVs by glial cells - microplate assay.***

The ATCC® glioblastoma cell line GL26 is used as an *in vitro* model for therapeutic cargo delivery. Briefly, GL26 cells are cultured in Dulbecco's modified Eagle's medium (Invitrogen) supplemented with 2 mmol/L L-glutamate, 100 U/mL penicillin, 100 μg/mL streptomycin, and 10% fetal bovine serum. Cells are seeded onto 96-well plates and incubated for 1-24 hours with DiR-labeled MEVs (as described above). Following the incubation, the cells are washed three times with ice-cold PBS and solubilized in 1% Triton™ X-100. The sample fluorescence is measured using a SpectraMax® fluorescence microplate reader (Molecular Devices, USA) with excitation at 750 nm and emission at 780 nm. The amount of MEVs accumulated in neuronal cells is normalized for total protein content and expressed as the number of MEVs per mg of protein. All MEV formulations are prepared at the same level of fluorescence, and a separate calibration curve is used for each MEV formulation.

***E. Uptake of loaded MEVs by glial cells - confocal microscopy.***

Mixed glial cells were seeded at a density of 25,000 cells/$cm^2$ and 50,000 cells/$cm^2$, respectively, in chamber slides for overnight attachment before they were co-cultured with PKH26-labeled MEVs (as described above) for 6 h. Co-cultures were then fixed with 4% paraformaldehyde and blocked with 1% bovine serum albumin (BSA; Sigma-Aldrich) and 0.25% Triton™ X-100 (Sigma-Aldrich) in PBS for 1 h at room temperature. Cells were stained overnight with a rabbit primary antibody against β-tubulin (1:200, ab6046, Abcam, Cambridge, UK) at 4°C followed by the detection with an anti-rabbit IgG Alexa Fluor 488 secondary antibody (1:200, Thermo Fisher Scientific) at room temperature for 1 h. Nuclei were counterstained using 4',6-diamidino-2'-phenylindole-dihydrochloride (DAPI; Sigma-Aldrich). Confocal images were acquired on a laser scanning microscope (Carl Zeiss LSM 710) with a 63x magnification. Images were processed in Imaris® software.

***F - Protective effects of the loaded MEVs on the glial cells.***

Primary mixed glial cells were isolated from cortexes of 2-day-old Wistar rat pups according to the protocol published previously (Chen et al. (2007), Isolation and culture of rat and mouse oligodendrocyte precursor cells. Nat Protoc. 2:1044-1051). Mixed glial cells were seeded at a density of 36,000 cells/$cm^2$ and cultured in DMEM supplemented with 10% FBS, 2 mmol/l GlutaMAX, 1 mmol/l sodium pyruvate, 100 units/ml penicillin, and 100 μg/ml streptomycin on poly-d-lysine (100 μg/ml) coated dishes. Mixed glial cells were seeded at a density of 18,000 cells/$cm^2$ and 36,000 cells/$cm^2$, respectively, before being incubated with 100 ng/ml LPS (Sigma-Aldrich) for 6 h and 24 h for cytokine secretion analysis. Securinine-loaded MEVs were added simultaneously with LPS. As controls, cells were cultured either without the addition of LPS and exosomes or with empty MEVs only. Cytokine evaluation in supernatants from mixed glial cells was performed with enzyme-linked immunosorbent assay (ELISA) following the manufacturer's instructions. Rat IL-1β and TNFα DuoSet® ELISA development systems (Bio-Techne, Minneapolis, MN) were used to analyze supernatants at 6 and 24 h of co-culture with MEVs.

**EXAMPLE 24**

***In vivo* - Efficiency of catalase loaded MEVs in a mouse model of Parkinson's Disease**

***A- Biodistribution of MEVs in mouse brain with inflammation.***

8-week old C57BL/6 female mice are stereotactically injected with 6-OHDA solution (10 μg 6-OHDA in 0.9% NaCl with 0.02% ascorbic acid), flow rate of 0.1 μL/min into the striatum (AP: +0.5; L: -2.0 and DV: -3.0 mm). Twenty-one days later (at the peak of inflammation), mice are IN administered with fluorescently labeled MEVs. Four hours later, mice are sacrificed, perfused, and the brain slides are examined by confocal microscopy. 6-OHDA-intoxicated mice injected with PBS are used as controls. Nuclei are labeled with DAPI. To examine which type of cells accumulate MEVs in the brain, a co-localization study with cell-specific markers is carried out. For this purpose, the brain slides are co-stained with: (1) primary rabbit polyclonal antibodies to neurons, AntiNeuN (ab128886, Abcam, 1:500 dilution), or (2) anti-tyrosine hydroxylase (TH) rabbit antibodies to TH-neurons (Calbiochem, 1:1000 dilution), or (3) rabbit anti-CD146 to endothelial

cells (ab75769, Abcam, 1:250 dilution), or (4) rabbit anti-GFAP antibodies to astrocyte marker (ab7260, Abcam, 1:500 dilution), and secondary antibodies, donkey anti-rabbit IgG H&L Alexa 555 (abcam ab150074, Abcam, 1:500dilution). All slides are permeabilized for 60 min in 0.1 M citrate buffer pH 6.0 and 0.05% Tween 20, washed 3x 5 min with 0.05% Tween 20 in PBS, blocked for 30 minutes with PBS and 5% Normal Donkey Serum + 0.05% Tween 20, and stained with primary antibody at stated dilution overnight at 4°C. Following the incubation, slides are washed 3x 5 minutes/wash in PBS/Tween and stained with secondary antibodies for one hour at room temperature. Then, the slides are washed 3x PBS/Tween 5 min/wash ddH2O, and covered using Vectashield® Hardset™ mounting media with DAPI. The images are examined by a confocal fluorescence microscopic system ACAS-570 and corresponding filter set.

***B- Immunohistochemical and stereological analyses.***

6-OHDA-intoxicated mice are treated via IN administration with PBS, or catalase alone, or catalase-loaded MEVs with the same amount of catalase, or the same amount of empty MEVs 48 hours after the intoxication (10 times every other day). Two control groups of healthy non-intoxicated animals were i.c. injected with PBS, and 48 hours later were IN administered with PBS, or empty MEVs. Twenty-one days later, animals are sacrificed and perfused; brains are removed, washed, post-fixed, and immunohistochemical analysis is performed in 30 μm thick consecutive coronal brain sections as described by [Brynskikh et al. (2010). Macrophage delivery of therapeutic nanozymes in a murine model of Parkinson's disease. Nanomedicine (Lond) 5(3):379-396]. For the detection of microglia activation, tissue sections are incubated with primary monoclonal rat anti-mouse anti-CD11b antibodies (1:500 dilution), and secondary biotinylated goat anti-rat antibodies (Vector Laboratories, Burlingame, CA, 1:200 dilution). Thus, activated microglia within the SNpc exhibit a more amoeboid morphology with sent out branches, compared to ramified barely visible resting microglia. In addition, levels of astrocytosis are assessed in the ventral midbrain region by fluorescent analysis of glial fibrillary acidic protein (GFAP) expression. For the GFAP staining, tissue sections are permeabilized with 0.01% Triton™ X-100 in TBS for 30 minutes and blocked for 1 hour with 10% normal goat serum (NGS, Vector Laboratories Inc., Burlingame, CA), then incubated with rabbit anti-GFAP primary polyclonal antibodies ab7260 (AbCam, Cambridge, MA) 1:100 dilution for 16 hours at 4°C. Tissue slides are incubated with goat anti-rabbit Alexa Fluor 647 secondary antibodies (Invitrogen; 1:200 dilution) for 1 hour, and mounted on slides. Immunoreactivity is evaluated by fluorescent analysis using confocal microscope Zeiss® 510 Meta Confocal Laser Scanning Microscope (Jena, Germany), and ImageJ software (NIH, Bethesda, MA, USA). For the assessment of neuroprotective effects, a TH staining is used to quantitate numbers of DA neurons [Tieu et al. (2003) D-beta-hydroxybutyrate rescues mitochondrial respiration and mitigates features of Parkinson disease. J Clin Invest. 112(6):892-901]. The total number of TH-positive DA neurons is counted by using the optical fractionator module in Stereo Investigator® software (MicroBrightField, Inc., Williston, VT).

***C - Apomorphine Test.***

C57BL/6 mice are i.c. injected with 6-OHDA. Healthy mice i.c. injected with PBS are used as a control. Starting from 48 hours after intoxication, mice are treated IN with PBS, or catalase loaded MEVs every other day for two weeks. Twenty-one days later, the animals are injected with apomorphine (0.05 mg/kg, s.c.) and rotations are scored every 10 min for 90 min as described by (Papathanou et al. (2011) Induction and expression of abnormal involuntary movements is related to the duration of dopaminergic stimulation in 6-OHDA-lesioned rats. Eur J Neurosci. 33(12):2247-2254).

**EXAMPLE 25**

***In vivo*** **- Delivery to the brain in an animal model for Spinal Cord Injury**

MEVs (isolated and purified as previously) were loaded with 0.1 nmol of PTEN-siRNA (Advirna Company) for 2-3 h at 37°C, before being IN administered to the Spinal Cord Injury Model animals. Ketamine-xylazine-isoflurane anesthesia was induced in adult female Sprague-Dawley rats (200-250 g). Laminectomy at the level of the T9-T11 vertebral column was performed to expose the spinal cord. Complete spinal cord transection at the T10 level was made using a micro-scissor after lifting the spinal cord using a spinal cord hook. To verify lesion completeness, the severed stumps were lifted, and the hook was passed inside the gap to ensure no residual fibers on the bottom and lateral sides of the canal. After the suturing of muscle layers and skin, rats were allowed to recover in heated chambers. At 2-3 h post-operation rats were intranasally given 40 μL of saline, 40 μL of empty MEVs or 40 μL of MEVs loaded with PTEN-siRNA, and the administration was repeated every 24 h for 5 days. Bladders were manually expressed twice a day until urinary reflex was re-established. Rats received subcutaneous injections of antibiotics (cefazolin, 25 mg/kg, twice daily) for 1 week, cyclosporin (10 mg/kg/d) for 1 week, and buprenorphine (0.01-0.05 mg/kg) for 3 days.

***Behavioral analysis:*** Motor recovery was assessed and scored by blinded experimenters using the Basso, Beattie, and Bresnahan locomotor rating scale method. Measurements were made every 2-3 days as the baseline score,

following implantation, and then once every week. Sensory recovery was assessed using the von Frey filament test. Filaments (Bioseb) with a gradient of bending forces were applied on the paws to elicit nociceptive responses (quick paw withdrawals from the stimuli). The withdrawal threshold value is defined as the minimal force inducing positive responses in at least 2 out of 3 trials, with at least 30 s between trials.

***Western Blotting*:** Lesioned spinal cord segments and livers were harvested at week 8 and placed in RIPA solution (1% sodium deoxycholate (Sigma-Aldrich), 1% 100x-Triton (Biolab), 5 mM ethylene-diaminetetraacetic acid (Sigma-Aldrich), 150 mM NaCl (Sigma-Aldrich), 50 mM Tris-HCl (Sigma-Aldrich)) containing a protease inhibitor cocktail (100 mM phenylmethylsulfonyl fluoride, 2 mg/mL leupeptin, 50 mM Na-orthavanadate, 50 mM aprotinin, Sigma). Tissues were homogenized, and 20 μg total protein separation was done in 4-12% SDS-PAGE gels and transferred to a nitrocellulose membrane for 90 min. After blocking with 2% bovine serum albumin (BSA) in tris-buffered saline with 0.1% Tween 20 (TBST) for 3 h, membranes were incubated overnight with mouse-anti-PTEN, 1:1000 (cat. #9556, Cell Signaling), or rabbit-anti-β-actin, 1:1000 (cat. #8457, Cell Signaling) as a loading control, in 2% BSA in TBST. The next day, after three TBST rinses, membranes were incubated with anti-mouse IgG-HRP (1:5000, cat. NA931 V, GE Healthcare) or anti-rabbit IgG-HRP (1:5000, cat. NA934 V, GE Healthcare) for 2 h, at room temperature. The blot was exposed to enhanced chemiluminescent reagents (cat. #1705061, Bio-Rad) in a LAS-3000 imaging system (FujiFilm) to develop signals.

***Real-Time Quantitative PCR*:** At week 8, total RNA from the lesioned spinal cord tissues and livers was extracted using a QIAGEN RNeasy® mini kit; first-strand DNA was synthesized using the high-capacity cDNA reverse transcription kit (Applied Biosystems). Real-time quantitative PCR was conducted on the QuantStudio® 12K Flex real-time PCR system. Primers included PTEN (Thermo Fisher Scientific, assay ID: Rn00477208) or GAPDH (Thermo Fisher Scientific, assay ID: Rn01775763-g1). The amplification reaction conditions: 95 °C for 20 s, 40 cycles of 95 °C for 3 s, 60 °C for 30 s, in 10 μL reactions, in triplicates. The ΔΔCt method was used to determine relative expression levels, where the gene of interest was normalized to GAPDH expression.

***Immunofluorescence Analysis:*** Spinal cords were dissected, fixated in 4% PFA overnight, immersed in 30% sucrose solution overnight, embedded in optimal cutting temperature solution, and then cut longitudinally to 20 μm thickness. Sections were permeabilized in 0.5% Tween solution, blocked with 5% bovine serum, and then incubated with rabbit-anti-tubulin (1:500, Abcam, cat. ab18207), rabbit-anti-GFAP (1:1000, Millipore, cat. AB5804), mouse-anti-CD11b (1:500, Bio-Rad, cat. MCA275R), rabbit-anti-Tmem119 (1:150, NovusBio, cat. NBP2-3055), or mouse-anti-CD31 (1:50, BD science, cat. 550300) antibodies, overnight, at 4 °C. Sections were then incubated with goat-anti-rabbit-Alexa647 (Invitrogen, 1:800) and DAPI (1:1000) for 1 h and mounted with coverslips, and a confocal microscope (Zeiss® LSM700) was used to obtain images.

## EXAMPLE 26

### *In vivo* - Delivery to the brain in a model of brain inflammation

#### *A - Animal model and intranasal treatment.*

Mice (C57BL/6J strain) were administered IN with MEVs loaded with miR-17 mimic MIMAT0021803 (sequence: CAAAGUGCUUACAGUGCAGGUAG, SEQ ID NO:140) catalog number: 4464066, Life Technologies) as described in the literature. Bacterial LPS (2.5 mg/kg; Sigma-Aldrich) is injected intraperitoneally into C57BL/6J mice as a control for induction of brain inflammation.

#### *B - Evaluation of brain inflammation.*

After IN administration of MEVs, mice are transcardially perfused with PBS followed by a 2% paraformaldehyde solution at pH 7.4. Brain tissue is postfixed for 2 h in 2% paraformaldehyde and then cryopreserved in phosphate-buffered 30% sucrose. Brains are embedded in optimal cutting temperature compound (Tissue-Tek; Sakura, Torrance, CA) and kept at -20°C overnight. Brain tissue sections are cut with a cryostat (10 μm thick), and the tissue sections stored at -20°C. Immunofluorescent staining of microglial cells with rabbit anti-Iba1 antibody or F4/80 antibody is carried out according to previously described procedures. Tissues evaluated for the presence of Iba1 or F4/80 positive staining are assessed using a Nikon® A1R confocal microscope equipped with a digital image analysis system (Pixera, San Diego, CA).

## EXAMPLE 27

### *In vivo* - Delivery to the brain in an animal model for Alzheimer

***A - Analysis of neuroprotective effects in an animal model of Alzheimer's disease.***

Female triple-transgenic AD mice (3xTg-AD) expressing three mutant human transgenes -PS1M146V, APPSwe, and tauP301L - are purchased from The Jackson Laboratory (Sacramento, California). MEVs are loaded with rivastigmine as described previously. Seven-month-old 3xTg female mice are anesthetized with isoflurane before being carefully intranasally administered with PBS solution or MEVs in 5 μL spurts per nostril. Overall, each mouse received 100 μL of vehicle or EVs twice, each injection separated by 18 hours (50 μL/d). After 21 days, each mouse is anesthetized using the tribromoethanol drug (TBE; Sigma-Aldrich), and perfused transcardially with 0.1 M PBS followed by formaldehyde 10 vol%/vol%, buffered 4 wt%/vol% (Paraformaldehyde (PFA) 4%; Titolchimica, Rovigo, Italy), then processed for **microglia activation and dendritic spine density.**

***B - Immunofluorescence.***

Immunofluorescence (IF) is performed on 40 μm coronal sections of prefrontal cortex (from 2.40 to 2.80 mm Bregma), CA1 region of medial hippocampus (from -1.955 to -2.355 mm Bregma) and entorhinal cortex (from -2.80 to -3.30 mm Bregma). After masking the tissue specific binding sites by 30 minutes incubation with blocking solution (3% bovine serum albumin and 0.3% Triton™ X-100 [Sigma-Aldrich] in PBS), slices are processed O/N at 4°C with different anti-mouse primary antibodies: microglia - Rabbit anti-Ibal (1:1000, Wako); astrocyte - Rat anti-GFAP (1:1000, Invitrogen); neurons - Mouse anti-NeuN (1:500, Chemicon), all with secondary antibody: Alexa Fluor 488 conjugated anti-rabbit/anti-rat/anti-mouse (1:1000, Invitrogen); microglia - Rat anti-CD68 and CD206 (1:400, Bio-Rad) with secondary antibody: Alexa Fluor 594 conjugated anti-rat (1:1000, Invitrogen). Samples are acquired using a confocal laser scan microscope (Sp5, Leica). Fluorescent images are derived by z-stack projections (maximum intensity) of sections obtained with the open-source software for image processing ImageJ (NIH).

***C - Analysis of microglia activation.***

Microglia activation is investigated by evaluating the cell density and cell soma size. Microglia density is calculated as the total number of Iba-1+/40,6-diamidino-2-phenylindole (DAPI) positive cells, within the Z-projection acquired for each slice, by collecting stacks of 50 μm for a total volume of 0.0144 $mm^3$. Semiquantitative analysis, aiming at determining microglia cell soma size and expression of microglia markers, is performed using a specifically designed macro with Image**J** software.

***D - Golgi-Cox staining.***

After fixation, each half of the brain is stained with Golgi-Cox solution (1% mercury chloride, 1% potassium dichromate, and 1% potassium chromate in distilled water) and stored at room temperature in dark for 2 weeks. Then the brains are kept in 30% PBS sucrose solution for 24 hours in order to reduce the tissue fragility during the sectioning procedure. After collection of 100-μm-thick slices of hippocampus, prefrontal, and entorhinal cortices using vibratome (Leica® VT1200, Leica Biosystems), they are processed with Kodak® Developer and Fixer (GBX Carestream Dental, Congers) for 5 minutes and 15 minutes, respectively, and washed in distilled water for 5 minutes after each step. Finally, slices are dehydrated using increasing concentrations of ethanol (50%-60%-75%-85%) and mounted on slides with coverslips using Eukitt® mounting medium(Sigma-Aldrich).

***E - Dendritic spine analysis.***

Images are collected using an Olympus BX63 microscope (Olympus Corporation, Japan) and acquired by the Neurolucida® 64-Bit software (MBF Bioscience, Williston, North Dakota). Acquisition of dendritic spines in CA1 region of medial hippocampus, prefrontal, and entorhinal cortices occur at 100×. Images of 117 × 88 μm are collected and three slices per mice are analyzed at the Bregma points mentioned above, with each stack being acquired using a z-stack unit of 0.35 μm. Images are deconvolved through AutoQuant® software, converted in 8-bit images and, then, black signal is inverted for the analysis with Imaris® image processing software (Bitplane Software, UK). Dendritic length and the number of spines of neurons are reconstructed by using the Autopath system of the Imaris® software (FILAMENT COMMAND) with each single spine detected by the software being manually checked to avoid false positive signals. To reduce the bias related to different dendrite lengths, the medium spine density for each animal is calculated by dividing the total number of spines with the total length of every measured dendrite.

**EXAMPLE 28**

***In vivo* - Delivery to the brain in an animal model for Multiple Sclerosis**

***A - Experimental Autoimmune Encephalomyelitis (EAE) induction in a murine model***

In order to mimic the pathology of human Multiple Sclerosis, EAE is induced using a murine model as described in literature. Three-month old C57BL/6J mice (male and female) are immunized with myelin oligodendrocyte glycoprotein (MOG) peptide 35-55 to induce EAE (IACUC #19014). In brief, 300 μg of rodent MOG peptide (amino acids 35-55, New England Peptides) in Complete Freund's Adjuvant (CFA) containing 5 mg/mL killed *Mycobacterium tuberculosis* (Difco, Thermo Fischer Scientific) is administered into the subcutaneous flank of mice at day 0. At day 0, each mouse receives two subcutaneous injections of the MOG solution, as well as a 100 μl dose of 2 ng/μl pertussis toxins and virulence factors (List Biological Laboratories) diluted in sterile PBS. Pertussis toxins and virulence factors are administered again on day 2.

***B - Experimental treatment of EAE animals***

Following MOG immunization, disease symptom onset typically peaks at 15-20 days. In order to monitor disease progression, mice are weighed and scored daily. Neurological deficits are assessed on a five-point scale (limp tail or waddling gait = 1; limp tail and waddling gait = 2; single limb paresis and ataxia = 2.5; double limb paresis = 3; single limb paralysis and paresis of second limb = 3.5; full paralysis of 2 limbs = 4; moribund = 4.5; and death = 5). Mouse treatment groups are randomized in order to contain comparable numbers of males and females and an average score close to 3.5 to represent EAE onset. At peak motor deficiencies, intranasal treatment with MEVs loaded with TICAM-1 siRNA (SEQ ID NOs: 134 and 135, sense and antisense RNA, respectively: Santa Cruz Biotechnology, cat. no. sc-154266) is performed; MEVs loaded with non-specific controlled siRNA serve as a control. EAE mouse scoring is repeated up to day 40 post-MOG immunization.

***C - Tissue preparation***

Mice are euthanized by $CO_2$ asphyxiation and perfused with ice-cold PBS. Lumbar spinal cords are harvested, post-fixed in 4% paraformaldehyde (PFA) at room temperature for 2 h, cryopreserved in 30% sucrose overnight, and embedded in OCT. Frozen transverse sections of 14 μm are cut on a Leica® cryostat.

***D - Quantification of oligodendroglia survival***

Frozen sections are dried and blocked using PBS containing 0.1% Tween 20 and 10% donkey serum (Thermo Fisher Scientific) for 1 h at room temperature. Sections are incubated with primary antibodies directed against SOX-10 (clone EP268, Millipore-Sigma) at 4°C overnight, followed by 2 h incubation at room temperature with secondary antibody. TUNEL staining (Terminal deoxynucleotidyl transferase dUTP nick end labeling) is performed on samples to quantify DNA damaged within SOX10 populations. TUNEL is performed using the In-Situ Cell Death Detection Kit (TMR Red, Millipore-Sigma) according to the manufacturer's instructions. DAPI is used to label nuclei, and the samples are imaged using a Carl Zeiss® Axio Observer D1 inverted microscope and analyzed using NIH ImageJ software.

***E - Quantification of myelin loss***

In order to quantify the loss of myelin in treated EAE mice, Luxol Fast Blue (LFB) is used to stain frozen sections. LFB is a copper phthalocyanine dye that binds to lipoproteins found within the myelin sheath. Frozen sections are dried and rehydrated using 95% EtOH. LFB staining is performed according to the manufacturer's instructions (IHC World, USA). Samples were imaged using a Carl Zeiss® Axio Observer D1 inverted microscope and analyzed using NIH ImageJ software. Myelin stains blue using LFB, therefore, thresholds are standardized and lack of LFB staining is quantified to denote the percentage of myelin loss in each sample.

**EXAMPLE 29**

***In vivo* - Delivery to the brain in an animal model for brain injury**

***A. MEV-mediated treatment of Traumatic Brain Injury (TBI).***

*Animal model and surgical procedures*: C57BL/6 male mice are subjected to either TBI by controlled cortical impact or sham surgery of a craniectomy only. Mice are randomly distributed into the following groups: surgery with no TBI, TBI no treatment, TBI with RNA-loaded MEV and TBI with empty MEVs. Deep anesthesia is administered to mice in a

stereotaxic frame (David Kopf Instruments, Tujunga, CA) using 1-2% isoflurane in nitrous oxide/oxygen (60/30%). Animals are secured on a stereotaxic frame; eye ointment is administered, and the skull is exposed by a midline excision. From bregma, a center with the coordinates of -0.2 mm anteroposterior and - 0.2 mm mediolateral is identified and an approximate 4 mm diameter craniectomy performed using a handheld drill. The resultant bone flap is discarded. The brain is impacted with a 3 mm diameter impactor tip rotated 20° on the vertical axis to adjust for curvature of the brain at the cerebral cortex with a velocity of 4.0 m/s reaching a depth of 0.95 mm (mild TBI) and a dwell time of 300 ms. Body temperature of the animals is regulated during surgery with thermal heating pad. Following surgery, scalps are sutured closed, and mice are transferred to a recovery cage on top of a heating pad until fully ambulatory. All animals are closely monitored postoperatively. Mice are maintained on a regular rodent diet throughout experiment.

### *B. IN administration of MEVs*

MEVs are isolated and purified as described previously. MEVs are loaded with MALAT1, a long noncoding RNA (lncRNA). 48 h following TBI surgery, mice are lightly anesthetized with 1-2% isoflurane in nitrous oxide/oxygen (60/30%) and treated by IN administration with equal volumes delivered bilaterally between nostrils. Sham mice and TBI no treatment received PBS intranasally, whereas the other groups received MEVs with and without MALAT1 (SEQ ID NO:142), respectively.

Over-expression of long noncoding RNA MALAT1 ameliorates traumatic brain injury induced brain edema by inhibiting AQP4 and the NF-κB/IL-6 pathway (see, *e.g.*, Yamin et al.(2019) J. Cell. Biochem. 120: 7584-17592, doi.org/10.1002/jcb.29025). Administration of MEVs that deliver MALT1 as RNA (or in some embodiments DNA for transcription into RNA) is for treatment of injury and other trauma involving the brain. MALAT1 expression has been related to cancer but not involving the brain, it would be a target for inhibition for cancers and tumors.

### *C. Behavioral testing. Elevated body swing test (EBST).*

The elevated body swing test measures asymmetric motor behavior seen in unilateral brain injuries (Borlongan and Sanberg, 1995). Briefly, a mouse is placed into an empty cage and allowed to habituate for 2 min. To begin, the mouse is in a neutral position with all four paws on the ground and held about 1 inch from the base of its tail. The mouse is lifted from the surface and a swing is recorded when the mouse moved more than 10° from the vertical axis to either side. A total of 20 swings are recorded for each animal. An animal with a score of 10 exhibit no bias, whereas a score of 14 or higher indicates asymmetry and an associated motor deficit. The total number of swings made to the higher side are summed and divided by the total animals per group, computing an average number of biased swings per treatment group. Following traumatic brain injury, mice are tested 24 h post TBI and divided into 3 groups based on their EBST score in order to give an equal average score between groups before administration of the treatment.

### *D. Behavioral testing. Radial arm water maze.*

At 5 weeks post TBI injury, 8 arm radial warm water (RAWM) is performed to assess cognitive function. RAWM is a hippocampal-dependent memory task that assesses spatial learning using visual cues and a hidden platform. Briefly, an 8 arm RAWM (BIOSEB) is placed in 4-foot diameter water tank with water maintained at 20°C. Two platforms are utilized: a visible platform that sits 1 inch above the water level and has a colorful flag identified in it and a hidden platform that remains 1 inch under the water line. White paint is used to cloud the water and further hide the hidden platform. A total of 15 trials are run on Days 1 and 2 in the learning phase followed by 15 trials on Day 3 of reversal testing. Each trial begins with a mouse being placed in the start arm and the platform in the assigned goal arm. The goal arm remains the same for a mouse throughout the learning trials of Days 1 and 2, though the starting arm would change randomly for every trial. On Days 1 and 2, the platform alternates between hidden and visible for the first 6 trials, while the remaining trials only use the hidden platform. On Day 3, all trials only use the hidden platform and are placed in their "reversal" position that is directly across from their previous goal arm location. Mice are tested in two blocks of 6 trials followed by one block of 3 trials to allow for a proper resting period. On Day 3, all trials only use the hidden platform. Trials are run for a maximum of 60 seconds; if the mouse finds the platform within the 60 s, they are given 30 s to remain on the platform before being returned to their home cage however, if the mouse does not find the platform at the end of 60 s, they are guided to the platform and sat on platform for 30 s. Errors are counted when a mouse enters an arm that does not contain a platform or if no arm is chosen for 15 s. RAWM performance analysis is done by analyzing the number of errors in each of the 3 blocks per day, for a total of 9 blocks over the three days.

### *E. Immunohistochemical analysis.*

At 6 weeks post TBI, animals are anesthetized and transcardially perfused with 0.1M phosphate buffered saline

(PBS) at pH 7.2 followed by 4% paraformaldehyde (PFA) in PBS at pH 7.2. Brain is collected and stored in 4% PFA solution overnight followed by 30% sucrose in PBS until brains sink completely. Brains are sectioned coronally at a thickness of either 40 μm with a cryostat and stored in -20°C freezer in a cryoprotectant solution.

For lesion volume analysis, every sixth coronal section is used spanning the entire TBI insult area. Tissues are mounted onto glass slides and allowed to dry. Slides are placed in hematoxylin QS (Vector Laboratories, catalog #H-3404) and allowed to fully develop for 5-10 min, and then washed in tap water for 5 min. Slides are allowed to dry overnight before being dehydrated and cover-slipped with DPX mounting medium. To calculate lesion volume, slides are visualized under a light microscope (Nikon® E600) and the impact area is outlined in the ipsilateral hemisphere and measured using Cavalieri estimator (Stereo Investigator® software, Micro Brightfield Inc.).

## EXAMPLE 30

### *In vivo* - Delivery to the brain in an animal model of cancer

#### *A. Intranasal tumor treatment in a brain tumor-bearing mice model.*

GL26 cells are intracranially injected per mouse using a method described previously. In brief, 2 μl of PBS containing $5 \times 10^4$ tumor cells are injected unilaterally at the coronal suture, 1 mm lateral to the midline, and 3 mm deep into the frontal lobes, using a Hamilton syringe (Fisher Scientific). Tumor-bearing mice are treated every 3 days for 21 days beginning on day 5 post-tumor cells implantation with temozolomide-loaded MEVs, empty MEVs or temozolomide alone.

#### *B. Therapeutic endpoint evaluation.*

The mean survival time (MST) of animals is measured as the main factor for evaluating the therapeutic potential of the formulations. For this purpose, the animals are monitored daily in terms of body weight, alertness, gait disturbance, and responses to contact and are overdosed with anesthetic when they show the following symptoms together: a 20% body weight loss, incoordination, ophthalmic hemorrhage. These symptoms are indicative of impending death, and the animals are considered unlikely to recover. The MST was determined and analyzed using a Kaplan-Meier analysis.

#### *C. Toxicity studies.*

The toxicity effects of the formulations are evaluated by measuring the serum concentration of blood urea nitrogen (BUN), creatinine, alanine transaminase (ALT), aspartate transaminase (AST) and alkaline phosphatase (ALP) as the kidney and liver biochemical markers. Histopathological studies are performed to confirm the results of toxicity measurement. For this purpose, kidneys, and liver from all the animals in all groups are harvested. The organs are fixed in 10% formalin, paraffinized, sectioned (5 μm) and stained with H&E. Next, the toxicity effects are evaluated using a semiquantitative scoring system by a pathologist in blind analysis, in which organ toxicity is considered 0 when no toxicity symptoms are observed, 1 when a slight change is perceived, and 2 when medium organ changes are observed.

## EXAMPLE 31

### *In vivo* - Delivery of psychedelics and β-carbolines to the brain

Parkinson's disease (PD), the second most common neurodegenerative disease, affects over 1% of the population above 60 years old. Clinically, PD patients suffer low quality of life with motor disorders such as resting tremor, muscle rigidity, bradykinesia, and postural instability. The pathological characteristics of PD include progressive destruction of the nigrostriatal system, formulation of Lewy bodies (LBs), and sustaining inflammation. The experiment shown the activity of harmine loaded into MEVs after IN administration.

#### *A. Animals and reagents*

Male C57BL/6 mice (8 weeks, 22-25 g) were used for experiments. All mice were raised in an environment with a 12-h light/dark cycle at a temperature of 22±1 °C with available food and water. MPTP hydrochloride was obtained from Sigma Corporation (Sigma-Aldrich, St Louis, MO, USA).

#### *B. Experimental procedures*

To construct the model, mice were injected with MPTP (dissolved in 0.9% saline, 30 mg/kg, (i.p.)) for 5

consecutive days, while the control group was given an equal volume of normal saline (i.p.). All mice were adapted for one week and were trained for the behavioral tests. Three days before, during or three days after the MPTP treatment, mice were given the MEVs loaded with harmine by intranasal administration or an equal volume of PBS (controls groups) for 14 days.

Two days after the last injection, behavioral assessments were performed using the open field test, pole test and rotarod test.

***C. Open field test***

A white opaque plastic box (48 cm×32 cm×20 cm) was used for the open field. The floor of the box was divided into 24 grids of 8 cm×8 cm. Before estimation, all mice were pre-adapted to the box for 5 min. The next day, the mouse was placed in the center of the open field and video recorded for 5 min. The box was cleaned with 10% alcohol and water between trials. Then, the distance traveled and the amount of rearing in 5 min were manually scored.

***D. Rotarod test***

Mouse motor coordination was evaluated using a rotarod apparatus (IITC Life Science, CA, USA). Before administration, all mice were trained on the rotarod (5 to 10 r/min in 300 s linearly) for 300 s. This training process was performed for more than 3 rounds to train all mice to walk on the rotarod. After MPTP treatment, the rotarod test was conducted at a uniformly accelerating speed from 5 to 30 r/min in 300 s, and the latency to fall was recorded.

***E. Pole test***

The pole test was implemented as described in our previous work to evaluate the movement disorder of the mice. The apparatus consisted of a wooden pole (50 cm high, 0.5 cm in diameter, wrapped with gauze to prevent slipping) with a wooden ball at the top. The base of the pole was covered with bedding as a protection for mice from injury. After acclimatization, the mice were pre-trained with the pole three times to make sure that all animals would turn head down once they were put on the ball. During the pole test, the total time it took for the mouse to get from the top to the bottom was measured.

***F. Tissue preparation***

Mice were anesthetized with chloral hydrate (400 mg/kg, ip). The striatum was rapidly dissected, frozen in dry ice and stored at -80 °C for biochemical analysis. For histological analysis, mice were anesthetized and perfused with 0.1 mol/L phosphate-buffered saline (PBS), followed by 4% paraformaldehyde (PFA). Later, the mouse brains were collected and post-fixed in 4% PFA for 24 h. After fixation, the brains were dehydrated with different concentrations of sucrose (from 10%, 20%, finally to 30%) until the brain samples were at the bottom of the solution. Then, coronal sections (20 μm thick) containing the striatum or substantia nigra pars compacta (SNpc) were cut on a cryostat (CM3050S, Leica, Germany) and placed on coated slides for analysis.

***G. Immunohistochemistry***

The slices of striatum or SN were treated as follows: boiled in 0.01 mol/L citrate buffer solution for 10 min to retrieve antigen, steeped in 1% Triton™ X-100 for 10 min to increase the penetration of the antibody, incubated with 3% hydrogen peroxide ($H_2O_2$) for 10 min to eliminate endogenous peroxidase activity, and incubated in 5% bovine serum albumin (BSA) for 1 h to remove the non-specific binding. Additionally, these slices were washed with PBST (5 min once, 3 times) between every two steps. Then, they were incubated with primary antibodies, including anti-tyrosine hydroxylase (TH) (1:100, Santa Cruz, Dallas, TX, USA), anti-α-synuclein (1:100, Abcam, Cambridge, CB, UK), and anti-glial fibrillary acidic protein (GFAP) (1:500, Dako, Denmark), overnight at 4 °C. After washing, slices were incubated with the corresponding horseradish peroxidase-conjugated secondary antibodies (1:200, KPL, Gaithersburg, MD, USA) for 2 h and washed three times. Then, the slices were stained with 3,3'-diaminobenzidine (DAB) for 10-60 s and finally imaged with an Olympus BA51 photomicroscope. We ensured that the regions of interest among the different groups shared the same SN shape, for the analysis.

***H. Western blot analysis***

Brain tissue was diluted 20-fold (μL/mg) with ice-cold RIPA buffer (50 mmol/L Tris-HCl, pH 7.5, 150 mmol/L NaCl, 1 mmol/L EDTA, 0.5% sodium deoxycholate, 1% NP-40, 0.1% SDS, 0.1 mmol/L PMSF and 1% protease inhibitor cocktail)

and disrupted on ice by using an ultrasonication apparatus (VTX 130, Sonics, USA). After the crushed tissue was lysed on ice for 30 min, it was centrifuged (12 000×g, 4 °C) for 30 min. Then, the supernatants were collected, and the protein concentration was measured with a BCA kit. The extracted protein was denatured after it was boiled with loading buffer for 10 min. Protein samples of the same amount were separated by 15% SDS-PAGE gels and transferred to PVDF membranes (Millipore, USA). These membranes were blocked with 3% BSA (Sigma-Aldrich, St Louis, MO, USA) and incubated with primary antibodies such as anti-β-actin (1:5000, Sigma-Aldrich, St Louis, MO, USA), anti-TH (1:500, Santa Cruz, Dallas, TX, USA), anti-GFAP (1:500, Santa Cruz, Dallas, TX, USA), or anti-α-synuclein (1:500, Santa Cruz, Dallas, TX, USA), overnight at 4 °C. After the membranes were washed (10 min, 3 times), they were incubated with horseradish peroxidase-conjugated secondary antibody (1:5000, KPL, Gaithersburg, MD, USA) for 2 h. Protein bands were detected with an enhanced chemiluminescence (ECL) plus detection system.

**I. As an example, the MEVs can be used to deliver cargo to the striatum cargos.**

The MEVs can be used to deliver to the striatum cargo that is interest when directly injected or expressed in gene therapy vectors, such as AAV or lentiviral vectors, in the brain. Such cargo to be delivered via IN administration of MEVs include, for example:

i) Glial cell derived neurotrophic factor (GDNF) or neurturin (NTN) able to regulate and promote cell growth and survival
ii) Glutamic acid decarboxylase (GAD) able to enhance GABA concentrations in order the balance the DA/GABA pathways.
iii) Aromatic amino acid decarboxylase (AADC) converts levodopa to dopamine and is dramatically reduced in PD. AADC substitution could improve motor symptoms and reduce total exogenous levodopa requirements reducing side effects of the treatment.
iv) Tyrosine hydroxylase (TH) which converts L-tyrosine to levodopa
v) Guanosine triphosphate -cyclohydrolase-1 (GTPCH), facilitating the conversion by TH
vi) ProSavin is a lentivirus vector that delivers therapy for Parkinson's disease and encodes three dopamine biosynthetic enzymes: tyrosine hydrolase (TH), GTP-cyclohydrolase 1 (GTPCH), and aromatic L-amino acid decarboxylase (AADC)

**EXAMPLE 32**

**Internalization of fluorescently labelled MEVs in the mouse intestinal epithelium (enterocytes) after *per os* administration**

PKH26 is a lipophilic fluorescent dye used for general membrane labeling. It is used for *in vitro* studies, as well as long term *in vivo* experiments due to enhanced stability (*in vivo* half-life is over 100 days). MEVs were labeled with PKH26 as described above and administered orally to BALB/c mice at a dose of 4x10exp10 MEV particles per animal. Between 0.5 to 24 hours post-administration, the animals were sacrificed, and the intestine was harvested.

**A. Organ sampling and processing: Intestine**

The jejunum and ileum were gently rinsed with a cold PBS solution. Then, a piece of approximately 0.5 to 1 cm of jejunum and ileum were snap-frozen and lastly, each piece was longitudinally placed on bottom of the cryomold for OCT inclusion (as described below).

**B. Snap-frozen:**

A small stainless-steel bowl was placed in the bottom of a container containing dry ice in pellet form and liquid nitrogen. Then, isopentane/2-methylbutane was slowly added in the container. When, the dry ice pellets stop bubbling vigorously or/and the isopentane start to become opaque, the isopentane/2-methylbutane is at optimal temperature. Organs were gently immerged in isopentane/2-methylbutane and then placed in cassette (one cassette with each section of liver, the spleen and the piece of jejunum and one cassette with the piece of ileum only). Then, cassettes were stored at ≤-75°C until OCT inclusion.

**C. OCT inclusion protocol**

A small stainless-steel bowl was placed in the bottom of a container containing dry ice in pellet form and liquid

nitrogen. Some pellets of dry ice were also placed directly in the bowl. Then, isopentane/2-methylbutane was slowly added in the container. When, the dry ice pellets stop bubbling vigorously and/or the isopentane starts to become opaque, the isopentane/2-methylbutane is at optimal temperature. The frozen tissue samples were then placed and oriented as mentioned above for each organ in the cryomold. The tissues were gently pushed with forceps to ensure that the bottom of surface of the tissue is placed properly (touching the face of the bottom, center in the mold and properly oriented). The cryomold with frozen tissue samples were placed on the surface of the cold isopentane/2-methylbutane. Optimal cutting temperature compound (OCT compound) was carefully deposited onto the specimen until it is completely covered. After hardening of the OCT compound (between 30 seconds and 1 minute), the OCT embedded block was placed in a bag (such as zip freezer bag). The frozen blocks were temporarily stored in dry ice. Then, slices of 5 μm were performed with a cryostat then glued on untreated slides for the histology and on treated slides for the immunochemistry.

### D. HES Staining

The HES staining allowed the observation of the morphology and the structure of tissues. After fixation in acetone, sections were immersed successively in solutions of Harris hematoxylin, eosin and saffron. After dehydration, sections were mounted between slide and cover slip using Entellan® mounting medium. Cytoplasm appeared in pink and nuclei in violet blue. Extracellular matrix was stained in yellow to pink.

### E. PKH26 dye

Accumulation of fluorescent labeled MEVs was visualized by a LSM700 laser scanning confocal microscope (from Zeiss) and images were processed with LSM Image Browser. DAPI (4',6-diamidino-2-phenylindole) [Invitrogen, Ref. S36938] was used as nuclear counterstain prior to the imaging. Sections were mounted with aqueous medium with DAPI for fluorescent slide scanner observation (maximum 10 days after collection).

Fig 48 shows a microscopic image of mouse intestinal epithelium 8 hours after PKH26-labeled MEV administration. Fluorescent signal visible in the enterocytes confirms MEV internalization. Cell nuclei were stained with DAPI.

## EXAMPLE 33

## MEV-mediated *in vivo* delivery, expression and biologic activity of luciferase enzyme and luciferase-mRNA

### *A. Intra-tracheal administration of MEVs loaded with luciferase mRNA or luciferase protein*

*In vivo* bioluminescence was used to study the biodistribution and MEV-mediated delivery and expression of luciferase mRNA and luciferase protein. Eight (8) female BALB/cByJ mice were divided into two groups (4 animals per group) for intra-tracheal (IT) administration. Mice in each group were treated either with MEVs loaded with mRNA encoding luciferase (*MEV-luc mRNA*) or MEVs loaded with luciferase enzyme (*MEV-luc protein*). MEVs were loaded and characterized as described above. For the IT administration, the animals were maintained under isoflurane-induced anesthesia and placed on a mouse intubation platform. Then, the trachea was backlit with a cold light and 50 μL/mouse of MEV formulations were administered intra-tracheally using a Microsprayer® aerosolizer (Penn-Century). After administration, mice were maintained in the same position on the intubating platform for at least 30 s before being replaced in their cage. A background mouse also was included in the study; this mouse did not receive any MEV administration and served as a control to measure the background level of the bioluminescence acquisition.

### *B. Bioluminescence acquisition*

Prior to imaging, the fur of each animal was shaved on the abdomen and thoracic area using an electric clipper. Each mouse was intraperitoneally (IP) injected with 200 μL of luciferin solution at a concentration of 16.5 mg/mL. Bioluminescence acquisitions were performed 10 minutes after the luciferin injection, with mice anesthetized with a mix of isoflurane/oxygen. Animals were positioned in dorsal recumbency (ventral images) to visualize the mice abdomen and thorax and particularly the lungs, liver, intestine, and spleen. Bioluminescence acquisition *in vivo* was performed with the IVIS® LUMINA X5 optical imaging system (PerkinElmer). The bioluminescence was measured at 6 timepoints: 1 h before MEV administration and 6, 30, 48, 54 and 72 h post-administration. The bioluminescence signal was visualized and quantified in the lungs, liver, intestine, and spleen. Animals were euthanized after the last bioluminescence acquisition. At each timepoint, bioluminescence acquisitions were first performed on the background mouse to measure the background flux level corresponding to the auto-bioluminescence of mice and the noise emitted by the camera of the optical imaging system. Bioluminescence acquisitions then were performed on the experimental mice.

*C- Results: Delivery of luciferase mRNA, protein expression and luciferase activity in vivo*

Fig. 49 shows whole-body bioluminescence imaging of a representative animal treated with MEVs loaded with luciferase mRNA. MEV-mediated delivery of mRNA resulted in expression of enzymatically active luciferase in mouse tissues, as evidenced by the luminescence signal detected after the administration of luciferin. The target organs after intratracheal administration include the respiratory system (lungs and naso-buccal epithelium), as well as the gastrointestinal tract (intestinal epithelium) resulting from partial regurgitation of the MEV formulation. Luciferase activity after a single administration of MEVs was detectable starting from 6 hours post-administration and lasted for 2-3 days. The control was a background mouse with no MEV administration.

*D- Results: Luciferase delivery and activity in vivo*

Fig. 50 depicts whole-body bioluminescence imaging of a representative animal treated with MEVs loaded with luciferase enzyme. MEV-mediated delivery of the protein resulted in enzymatic activity of luciferase in mouse tissues, as evidenced by the luminescence signal detected after the administration of luciferin. The target organs after intratracheal administration include the respiratory system (lungs and naso-buccal epithelium), as well as the gastrointestinal tract (intestinal epithelium) resulting from partial regurgitation of the MEV formulation. Luciferase activity after a single administration of MEVs was detectable starting from 6 hours post-administration and lasted for 3-4 days. The control is a background mouse with no MEV administration.

**EXAMPLE 34**

**Inhibition or stimulation of Toll like receptors (TLR) using MEV-mediated delivery in macrophage cells *in vitro***

**A. Cell Cultures:**

*THP1 -Derived Macrophage Culture*

The ATCC® human monocyte THP-1 cell line is cultured in RPMI-1640 medium supplemented with 10% FBS, 2 mM L-glutamine, 100 U/mL penicillin, 100 μg/mL streptomycin, and 0.05 mM 2-mercaptoethanol. THP-1 monocytes are differentiated into M0 macrophages (M0-M). The cells are seeded at $1 \times 10^5$ cells/mL and incubated at 37 °C with 5% $CO_2$ for 48 h in the presence of 50 ng/mL of PMA; then, the conditioned medium with PMA are removed and replaced with fresh medium for 3 days for cell recovery. Macrophage maturations are assessed by flow cytometry. After five days of culture, the cells are harvested and stained for CD68, a marker of mature macrophages, using the antihuman monoclonal antibody (mAb) anti CD68 PE (clone Y1/82A) and its isotype control PE Mouse IgG2b, κ Isotype Ctrl Antibody (clone MPC-11). The expression of CD68 in THP-1-derived macrophages are assessed using a flow cytometer. At least 30,000 cells are acquired, and the twofold increase in mean fluorescence intensity (MFI) of CD68 compared with the isotype control are considered successful maturation.

*RAW 264.7 macrophage culture*

RAW 264.7 cells are obtained from ATCC®. Cells are maintained in D10 and passaged every three days when more than 80% confluent. In preparation for phagocytosis assays, cells are harvested and centrifuged at room temperature for 7 min at 1100 rpm using an IEC clinical benchtop centrifuge, re-suspended in R10, counted, and viability assessed using trypan blue dye exclusion (Sigma-Aldrich). Viability in all experiments is >90%.

**B. Reagents**

TLR ligands are dsRNA: (polyadenylic-polyuridilyc acid (polyA:U)); (polyinosinic:polycytidylic acid (Poly I:C)); antivirals (as R848, R837, CL075, and CL264); taxol; flagellin; the flagellin-mimetic peptide flp22 (peptide from flagellin); or unmethylated CpG motif oligonucleotides. For phagocytosis assays, macrophages are cultured in sterile tissue culture medium (R10), composed of RPMI 1640 medium (Life Technologies, Burlington, ON), supplemented with 10% heat-inactivated fetal calf serum (Life Technologies, Burlington, ON), 100 U/mL penicillin (Life Technologies, Burlington, ON), 100 μg/mL streptomycin (Life Technologies, Burlington, ON), 25 mM N-2-hydroxyethylpiperazine- NV-2-ethanesulphonic acid (HEPES, Life Technologies, Burlington, ON) and 10 mM L-glutamine (Life Technologies, Burlington, ON). Dulbecco's Modified Eagles Medium (DMEM) (Life Technologies, Burlington, ON), supplemented in the same way as the RPMI, to form D10, are used for growth of RAW 264.7 macrophages.

**C. Loading of MEVs**

MEVs are loaded with different ligands of TLRs as described herein (see, also, International PCT application No PCT/EP2022/07037). The TLR ligands included: dsRNA (polyadenylic-polyuridilyc acid (poly(A:U)); Polyinosinic:polycytidylic acid (Poly (I:C)); antivirals (as R848, R837, CL075, and CL264); taxol; flagellin; the flagellin-mimetic peptide flp22 (peptide from flagellin); or unmethylated CpG motif oligonucleotides, which are TLR3 (dsRNA (polyadenylic-polyuridilyc acid (poly(A:U)); Polyinosinic:polycytidylic acid (Poly (I:C)), TLR4 (taxol); TLR5 (flagellin; flagellin-mimetic peptide flp22 (peptide from flagellin)), TLR7 and TLR8 (antivirals such as R848, R837, CL075, and CL264), and TLR9 (unmethylated CpG motif oligonucleotides) agonists.

**D. MEVs incubation and internalization**

The THP-1 cells are treated at different time points with MEVs loaded with TLR ligands. As control condition TAK-242, an inhibitor of TLR, are resuspended in PBS, and stored at -80°C; before treatment with MEVs the THP-1 cells are treated with TAK-242 (5 μM) for 30 min.

Internalization of MEVs by M0 Macrophages: MEVs are labeled with PKH26 Red Fluorescent Cell Linker Kits following the methods described herein (see, also PCT/EP2022/070371 and U.S. provisional application Serial No. 63/349,006). The labeled MEVs are incubated with THP-1 cells for 3 and 6 h at 37°C with 5% CO2. After incubation, the cells are fixed with PFA 4%, permeabilized with 0.1% Triton™ X-100, and stained with Actin Green, USA, 1 drop/mL of PBS) that binds actin with high affinity, and the nuclei are stained with Hoechst, dilution 1:1000). The samples are analyzed by confocal microscopy.

**E. Macrophage phagocytosis assay**

For phagocytosis assays, $1\times10^5$ RAW 264.7 macrophages in R10 medium are plated onto sterile 4 well chambered Millicell® EZ SLIDES glass culture slides (Millipore Corporation, Bedford, MA), and macrophages allowed to adhere in a humidified 5% $CO_2$ incubator for 30 min at 37°C. Zymosan particles are added to the cells at a zymosan to macrophage ratio of 10:1. Phagocytosis is allowed to proceed for 1 h at 37°C in a humidified 5% $CO_2$ incubator. In experiments where the effects of TLR ligands are assessed, cells are incubated in the presence of these TLR receptor agonists for 16 h prior to addition of zymosan. For experiments assessing scavenger receptor blockade, inhibitors are added 15 min prior to addition of zymosan. Phagocytosis is terminated by vigorously washing off non-ingested particles with warm R10 three times. Glass slides are air-dried for 15 min, stained using Wright's stain (Sigma-Aldrich), and slides examined by light microscopy at X600 magnification using a Nikon® Eclipse 50i photomicroscope. Phagocytosis is determined from photomicrographs at X600 magnification by counting at least 200 macrophages in 16 different fields of view for each experimental treatment. Phagocytosis is assessed as the mean number of particles per cell (MNP). Phagocytic index (PI) is used to express overall phagocytosis and determined as percentage of phagocytic cells multiplied by MNP (PI = percentage phagocytosis X MNP). Photomicrographs of cells for illustrations are taken either unstained by phase contrast at X400 magnification using an inverted microscope (Nikon® Eclipse TE2000S), or stained cells captured at X600 magnification using a Nikon® Eclipse 50i photomicroscope.

**F. Macrophage cell surface receptor expression**

To assess the effect of LPS on macrophage cell surface receptor expression, $1 \times 10^5$ macrophages cells are cultured in R10 alone or pretreated overnight with LPS at 10 ng/mL. Wells are washed with $Ca^{2-}$ and $Mg^{2+}$ free PBS, and then the cells are detached using Accutase™ (BD Bioscience, Mississauga, ON). Cell surface expression of Class A scavenger receptors (SR-A) and the lectin receptor Dectin-1 is detected using FITC-labelled IgG antibodies against the respective surface proteins, and this is compared with fluorescence of appropriate isotype-matched control antibodies. Cells are stained using either anti SR-A IgG (1 μg/mL) (isotype control IgG2) or anti Dectin-1 IgG (1 μg/mL) (isotype control IgG2). All antibodies and controls are obtained from Bio-Rad (Raleigh, NC). Antibodies are added to 100 μl of cells re-suspended in staining buffer composed of PBS, 2% bovine serum albumin and 0.1% sodium azide, to a concentration of $10^6$ cells/mL. During staining, cells are incubated on ice and kept in the dark for 45 min. Thereafter, cells are washed, re-suspended in 200 μl of stain buffer, and analyzed using BD FACS-Jazz (BD Biosciences, San Jose, CA) and Flow-Jo software (Tree Star, Ashland, OR). Forward and side scatter are used to discriminate live macrophages prior to analysis of SR-A and Dectin-1 expression.

**G. Cytotoxicity determination**

*MTT (3-[4,5-Dimethylthiazol-2-yl]-2,5 Diphenyl Tetrazolium Bromide) Assay*

Cell viability is determined by an MTT assay as previously described. The THP-1 cells are seeded in triplicate in 48-well plates; 24 h post-seeding, the cells are treated with MEVs for 24 and 48 h. The absorbance is measured by an ELISA reader at 540 nm. Values are expressed as a percentage of cell growth versus that in the control (untreated cells).

**1. Western Blotting:**

Total proteins from the THP-1 cells treated with MEVs are isolated and analyzed by SDS-PAGE followed by Western Blotting. The primary antibodies used in the experiments are as follows: anti-PD-L1 antibody (Abcam, Cambridge, UK, ab213524, dilution 1:1000; antipSTAT3 (R&D System, AF4607-SP, dilution 1:500), anti-STAT3 (Novus Biologicals, Denver, CO, USA, NBP2-24463, dilution 1:1000), anti-HSP70 (Novus Biologicals, NB600-1469, dilution 1:1000), anti-HSC70 (Santa Cruz, Dallas, TX, USA, sc-7298, dilution 1:500), anti-Calnexin (Santa Cruz, sc-23954, dilution 1:500), anti-Tubulin (Santa Cruz, sc-398103, dilution 1:1000), anti-β Actin (Santa Cruz, sc-81178, dilution 1:1000), anti-GAPDH (Santa Cruz, sc-47724, dilution 1:1000), and anti-NF-kB (Novus, NB100-2176, dilution 1:500). After overnight incubation with the primary antibodies at 4 ◦C, the membranes are incubated with HRP-conjugated secondary antibody (Thermo Fisher Scientific) for 1 h, at 4°C; the chemiluminescent signal is detected using a ChemiDoc™ MP imaging system(Bio-Rad).

**2. Real-Time PCR**

The THP-1 cells are seeded in 12 well-plates at $1\times10^5$ cells/mL and differentiated in M0 macrophages, as described above; the cells then are treated with MEVs for 6 and 24 h. At the end of the treatments, total RNA is extracted using QIAGEN® kit. The RNA is reverse transcribed to cDNA using the High-Capacity cDNA Reverse Transcription kit. Then, the cDNA is subjected to quantitative real-time reverse transcriptase polymerase chain reaction (RT-PCR) analysis. The sequences of the primers used are indicated in the following Table.

**Table:** List of primers used for Real-Time PCR.

| Primers | Forward | SEQ ID NO. | Reverse | SEQ ID NO. |
|---|---|---|---|---|
| GAPDH | ATGGGGAAGGTGAAGGTCG | 150 | GGGTCATTGATGGCAACAAT AT | 151 |
| PD-L1 | TCACGGTTCCCAAGGACCTA | 152 | AGGTCTTCCTCTCCATGCAC | 153 |
| IL-6 | GGTACATCCTCGACGGCATC T | 154 | GTGCCTCTTTGCTGCTTTCA C | 155 |
| IL-1β | ACAGATGAAGTGCTCCTTCC A | 156 | GTCGGAGATTCGTAGCTGGA T | 157 |
| TNF-α | CCAGGCAGTCAGATCATCTT CTC | 158 | AGCTGGTTATCTCTCAGCTC CAC | 159 |

Real-time PCR is performed using Step One Real-time PCR System Thermal Cycling Block (Applied Biosystem) in a 20 μL reaction containing 300 nM of each primer, 2 μL of template cDNA, and 18 μL of 2X SYBR® Green I Master Mix. The PCR is run at 95°C for 20 s followed by 40 cycles of 95°C for 3 s and 60 ◦C for 30 s. GAPDH is used as the endogenous control. Relative changes in gene expression between control and treated samples are determined using the ΔCt method.

H. ELISA test

The amount of IL-6 in the culture medium of THP-1 cells treated with MEVs is determined using a human IL-6 ELISA kit (R&D Systems). The ELISA assay is performed according to the manufacturer's instructions.

## Claims

1. A composition for use in treatment of a disease, disorder or condition, the composition comprising microalgae extracellular vesicles (MEVs) for delivery of bioactive cargo to target tissue, wherein:

the composition is formulated for administration by oral delivery, intravenous delivery, intratracheal delivery, intramuscular delivery, intranasal delivery, instillation into the eye, or inhalation delivery into the lungs of a subject, for trafficking of MEVs to a target tissue;
the microalgae is a species of the family Chlorellaceae;
the MEVs comprise heterologous bioactive cargo for treatment of a disease, disorder, or condition involving the target tissue;
the formulation and route of administration of the MEVs effect delivery of the cargo to the target tissue.

**2.** The composition for use of claim 1, wherein the target tissue is selected from among tissues of the lungs, liver, spleen, intestine, brain, naso-buccal mucosa, heart, kidneys, retina and/or choroid of the eye.

**3.** The composition for use of claim 1 or claim 2, wherein the microalgae is a species of *Chlorella* selected from among *Chlorella ellipsoidea, Chlorella pyrenoidosa, Chlorella sorokiniana, Chlorella vulgaris,* and *Chlorella variabilis.*

**4.** The composition for use of claim 3, wherein the *Chlorella* is *Chlorella vulgaris.*

**5.** The composition for use of any of claims 1-4, wherein:

the MEVs are formulated for oral administration and the disease involves or treatment targets delivery to the intestine or spleen; or
the MEVs are formulated for oral administration and the disease involves or treatment targets the gastrointestinal tract or the immune system or the white spleen; or
the MEVs are formulated for intratracheal administration or inhalation and the disease involves or treatment targets the lungs or treats respiratory diseases; or
the MEVs are formulated for intranasal administration and the disease, disorder, or condition is a disease of or involving the brain; or
the MEVs are formulated for administration to the eye, and the disease, disorder, or condition is age-related macular degeneration, herpes stromal keratitis, glaucoma, dry eye syndrome, diabetic retinopathy, a condition associated with ocular angiogenesis, or ocular hypertension; or
the MEVs are formulated for intranasal administration, and the disease, disorder, or condition involves the central nervous system or the nervous system; or.
the MEVs are formulated for intravenous administration, and the disease involves, or the treatment targets the liver; or
the MEVs comprise a therapeutic agent or encode a therapeutic agent for treating a disease, disorder, or condition involving the liver, the composition is formulated for intravenous administration, and the disease involves, or the treatment targets the liver.

**6.** The composition for use of any of claims 1-5, comprising microalgae extracellular vesicles (MEVs) in a pharmaceutically acceptable vehicle, wherein:

the composition is formulated for oral administration, the target tissue is gut-associated lymphoid tissue (GALT), and the cargo is immunomodulatory or is an antigen for inducing an immune response; or
the composition is formulated for oral administration and the disease, disorder, or condition involves the gastrointestinal tract or the immune system, or the white spleen, and the cargo is for treatment of a disease, disorder, or condition by immune modulation or vaccination; or
the composition is formulated for intranasal administration, the target tissue is the brain, and the cargo comprises a bioactive molecule for treatment, detection, or diagnosis of a disease, disorder, or condition of the brain or involving the brain or for monitoring treatment of a disease, disorder, or condition of the brain or involving the brain; or
the composition is formulated for instillation into the eye as drops, the target tissue is choroid or retina, and cargo is for treatment of a disease, disorder, or condition of the eye; or
the composition is formulated for intramuscular or oral administration, the target is the immune system, and the cargo comprises an antigen for vaccination or an immunomodulatory protein or product; or.
the composition is formulated for oral administration and the disease, disorder, or condition involves or is of an organ or tissues accessible through the blood stream; or
the composition is formulated for intratracheal administration or inhalation or nebulization, the disease, disorder, or condition involves the respiratory tract or is of the respiratory tract, and the cargo is a product for treating the respiratory tract or for treating respiratory diseases or diseases involving the respiratory tract.

7. The composition for use of any of claims 1-6, wherein the disease is treated by immune modulation, and the MEVs comprise a vaccine.

8. The composition for use of any of claims 1-7, wherein the disease, disorder, or condition is a cancer and/or an immune cell disorder or a disease treated or prevented by a vaccine.

9. The composition for use of any of claims 1-8, wherein the cargo comprises a nucleic acid molecule, a polypeptide, a protein, a plasmid, an aptamer, an antisense oligonucleotide, or a small molecule drug.

10. The composition for use of claim 9, wherein the cargo is nucleic acid that is DNA or RNA.

11. The composition for use of claim 10, wherein the cargo comprises a plasmid.

12. The composition for use of any of claims 1-6, wherein the cargo comprises or encodes an immunomodulatory agent to increase or decrease production of one or more products that up or down-regulate self-antigen presentation; mask MHC antigens; or promote the proliferation, differentiation, migration, or activation state of one or more types of immune cells.

13. The composition for use of claim 12, wherein the cargo comprises or encodes a hormone or a cytokine or a chemokine.

14. The composition for use of any of claims 1-13, wherein the cargo comprises nucleic acid that encodes a product for gene therapy.

15. The composition for use of claim 14, wherein the gene therapy is for treating an inborn error of metabolism.

16. The composition for use of any of claims 1-13, wherein the cargo comprises or encodes a protein that is an antibody or antigen-binding fragment thereof.

17. The composition for use of claim 16, wherein the antibody is an scFv, a bi-specific antibody, or an antigen-binding fragment thereof.

18. The composition for use of any of claims 1-17, wherein the MEVs comprise a DNA vaccine, an RNA vaccine, or a protein vaccine.

19. The composition for use of any of claims 1-18, wherein the cargo comprises a nucleic acid or a protein or a nucleic acid encoding a protein that is a therapeutic product for treating cancer, or an infectious disease, or a neurodegenerative disease or other central nervous system (CNS) disorder, or for ameliorating effects of aging, or slowing or reversing aging, or treating an aging-associated disease, or an ophthalmic disorder, or an immunological disorder.

20. The composition for use of any of claims 1-19, wherein the cargo comprises an agonist or antagonist of an intracellular endosomal receptor.

21. The composition for use of claim 20, wherein the receptor is a toll-like receptor (TLR).

22. The composition for use of any of claims 1-21wherein:

the MEVs are formulated for oral administration and the disease involves or treatment targets delivery to the intestine or spleen; or
the MEVs are formulated for oral administration and the disease involves or treatment targets the gastrointestinal tract or the immune system or the white spleen; or
the MEVs are formulated for intratracheal administration or inhalation and the disease involves or treatment targets the lungs or treats respiratory diseases; or
the MEVs are formulated for intranasal administration and the disease, disorder, or condition is a disease of or involving the brain; or
the MEVs are formulated for administration to the eye and the disease, disorder, or condition is age-related macular degeneration, herpes stromal keratitis, glaucoma, dry eye syndrome, diabetic retinopathy, a condition associated with ocular angiogenesis, or ocular hypertension.

**23.** The composition for use of any of claims 20-22, wherein the disease, disorder, or condition is a disease of or involving the central nervous system or the nervous system, and the composition is administered intranasally.

**24.** The composition for use of any of claims 20-22, wherein the disease, disorder, or condition is treated by immune modulation, and the cargo comprises a vaccine.

**25.** The composition of any of claims 20-22, wherein the disease, disorder, or condition is a cancer and/or an immune cell disorder or a disease treated or prevented by a vaccine.

**26.** The composition for use of any of claims 20-22, wherein the composition is formulated for oral administration and the disease, disorder, or condition is an intestinal infection, Crohn's disease, or cancer.

**Patentansprüche**

**1.** Zusammensetzung zur Verwendung bei einer Behandlung einer Krankheit, Störung oder eines Zustands, wobei die Zusammensetzung extrazelluläre Vesikel von Mikroalgen (MEVs) zur Abgabe bioaktiver Fracht an Zielgewebe umfasst, wobei:

die Zusammensetzung zur Verabreichung durch orale Abgabe, intravenöse Abgabe, intratracheale Abgabe, intramuskuläre Abgabe, intranasale Abgabe, Eintröpfeln ins Auge oder Abgabe durch Inhalation in die Lunge eines Subjekts zum Transportieren von MEVs in ein Zielgewebe formuliert ist;
die Mikroalge eine Spezies aus der Familie der Chlorellaceae ist;
die MEVs heterologe bioaktive Fracht zur Behandlung einer Krankheit, Störung oder eines Zustands, der das Zielgewebe betrifft, umfassen;
die Formulierung und der Verabreichungsweg der MEVs die Abgabe der Fracht in das Zielgewebe beeinflussen.

**2.** Zusammensetzung zur Verwendung nach Anspruch 1, wobei das Zielgewebe aus Geweben der Lunge, Leber, Milz, Darms, Gehirns, Nasen-Mund-Schleimhaut, Herzens, Nieren, Netzhaut und/oder Aderhaut des Auges ausgewählt ist.

**3.** Zusammensetzung zur Verwendung nach Anspruch 1 oder Anspruch 2, wobei die Mikroalge eine Spezies von *Chlorella,* ausgewählt aus *Chlorella ellipsoidea, Chlorella pyrenoidosa, Chlorella sorokiniana, Chlorella vulgaris* und *Chlorella variabilis ist.*

**4.** Zusammensetzung zur Verwendung nach Anspruch 3, wobei die *Chlorella Chlorella vulgaris ist.*

**5.** Zusammensetzung zur Verwendung nach einem der Ansprüche 1-4, wobei:

die MEVs zur oralen Verabreichung formuliert sind, und die Krankheit die Abgabe in den Darm oder die Milz betrifft oder die Behandlung darauf abzielt; oder
die MEVs zur oralen Verabreichung formuliert sind, und die Krankheit den Magen-Darm-Trakt, das Immunsystem oder die weiße Milz betrifft oder die Behandlung darauf abzielt; oder
die MEVs zur intratrachealen Verabreichung oder Inhalation formuliert sind, und die Krankheit die Lunge betrifft oder die Behandlung darauf abzielt oder Atemwegskrankheiten behandelt; oder
die MEVs zur intranasalen Verabreichung formuliert sind und die Krankheit, Störung oder der Zustand eine Krankheit des Gehirns ist oder dieses betrifft; oder
die MEVs zur Verabreichung an das Auge formuliert sind, und die Krankheit, Störung oder der Zustand altersbedingte Makuladegeneration, Herpes Stromakeratitis, Glaukom, Syndrom des trockenen Auges, diabetische Retinopathie, ein Zustand im Zusammenhang mit okulärer Angiogenese oder Augenüberdruck ist; oder
die MEVs zur intranasalen Verabreichung formuliert sind, und die Krankheit, Störung oder der Zustand das zentrale Nervensystem oder das Nervensystem betrifft; oder.
die MEVs zur intravenösen Verabreichung formuliert sind, und die Krankheit die Leber betrifft oder die Behandlung darauf abzielt; oder
die MEVs einen therapeutischen Wirkstoff umfassen oder einen therapeutischen Wirkstoff zur Behandlung einer Krankheit, Störung oder eines Zustands, der die Leber betrifft codieren, die Zusammensetzung zur intravenösen Verabreichung formuliert ist, und die Krankheit die Leber betrifft oder die Behandlung darauf abzielt.

6. Zusammensetzung zur Verwendung nach einem der Ansprüche 1-5, umfassend extrazelluläre Vesikel von Mikroalgen (MEVs) in einem pharmazeutisch verträglichen Träger, wobei:

   die Zusammensetzung zur oralen Verabreichung formuliert ist, das Zielgewebe darmassoziiertes lymphatisches Gewebe (GALT) ist, und die Fracht immunmodulatorisch oder ein Antigen zur Auslösung einer Immunantwort ist; oder
   die Zusammensetzung zur oralen Verabreichung formuliert ist und die Krankheit, Störung oder der Zustand den Magen-Darm-Trakt, oder das Immunsystem oder die weiße Milz betrifft, und die Fracht der Behandlung einer Krankheit, Störung oder eines Zustands durch Immunmodulation oder Impfung dient; oder
   die Zusammensetzung zur intranasalen Verabreichung formuliert ist, das Zielgewebe das Gehirn ist, und die Fracht ein bioaktives Molekül zur Behandlung, zum Nachweis oder zur Diagnose einer Krankheit, Störung oder eines Zustands des Gehirns umfasst oder das Gehirn betrifft, oder zur Überwachung der Behandlung einer Krankheit, Störung oder eines Zustands des Gehirns oder betreffend das Gehirn; oder
   die Zusammensetzung zum Eintröpfeln ins Auge als Tropfen formuliert ist, das Zielgewebe Aderhaut oder Netzhaut ist, und die Fracht der Behandlung einer Krankheit, Störung oder eines Zustands des Auges dient; oder
   die Zusammensetzung zur intramuskulären oder oralen Verabreichung formuliert ist, das Ziel das Immunsystem ist, und die Fracht ein Antigen zur Impfung oder ein immunmodulatorisches Protein oder Produkt umfasst; oder.
   die Zusammensetzung zur oralen Verabreichung formuliert ist, und die Krankheit, Störung oder der Zustand ein Organ oder Gewebe betrifft oder davon ist, das über den Blutkreislauf erreichbar ist; oder
   die Zusammensetzung zur intratrachealen Verabreichung, Inhalation oder Zerstäubung formuliert ist, die Krankheit, Störung oder der Zustand die Atemwege betrifft oder von den Atemwegen betroffen ist, und die Fracht ein Produkt zur Behandlung der Atemwege oder zur Behandlung von Atemwegserkrankungen oder Erkrankungen ist, welche die Atemwege betreffen.

7. Zusammensetzung zur Verwendung nach einem der Ansprüche 1-6, wobei die Krankheit durch Immunmodulation behandelt wird und die MEVs einen Impfstoff umfassen.

8. Zusammensetzung zur Verwendung nach einem der Ansprüche 1-7, wobei die Krankheit, Störung oder der Zustand Krebs und/oder eine Störung der Immunzellen oder eine Krankheit ist, die durch einen Impfstoff behandelt oder verhindert wird.

9. Zusammensetzung zur Verwendung nach einem der Ansprüche 1-8, wobei die Fracht ein Nukleinsäuremolekül, ein Polypeptid, ein Protein, ein Plasmid, ein Aptamer, ein Antisense-Oligonukleotid oder ein niedermolekulares Arzneimittel umfasst.

10. Zusammensetzung zur Verwendung nach Anspruch 9, wobei die Fracht Nukleinsäure ist, die DNA oder RNA ist.

11. Zusammensetzung zur Verwendung nach Anspruch 10, wobei die Fracht ein Plasmid umfasst.

12. Zusammensetzung zur Verwendung nach einem der Ansprüche 1-6, wobei die Fracht ein immunmodulatorisches Mittel umfasst oder codiert, um die Produktion eines oder mehrerer Produkte zu erhöhen oder zu verringern, welche die Selbstantigenpräsentation hoch- oder herunterregulieren; MHC-Antigene maskieren; oder die Ausbreitung, Differenzierung, Migration oder den Aktivierungszustand einer oder mehrerer Arten von Immunzellen fördern.

13. Zusammensetzung zur Verwendung nach Anspruch 12, wobei die Fracht ein Hormon, ein Zytokin oder ein Chemokin umfasst oder codiert.

14. Zusammensetzung zur Verwendung nach einem der Ansprüche 1-13, wobei die Fracht Nukleinsäure umfasst, die ein Produkt zur Gentherapie codiert.

15. Zusammensetzung zur Verwendung nach Anspruch 14, wobei die Gentherapie zur Behandlung eines angeborenen Stoffwechseldefekts dient.

16. Zusammensetzung zur Verwendung nach einem der Ansprüche 1-13, wobei die Fracht ein Protein umfasst oder codiert, das ein Antikörper oder ein Antigen bindendes Fragment davon ist.

17. Zusammensetzung zur Verwendung nach Anspruch 16, wobei der Antikörper ein scFv, ein bispezifischer Antikörper oder ein Antigen bindendes Fragment davon ist.

**18.** Zusammensetzung zur Verwendung nach einem der Ansprüche 1-17, wobei die MEVs einen DNA-Impfstoff, einen RNA-Impfstoff oder einen Proteinimpfstoff umfassen.

**19.** Zusammensetzung zur Verwendung nach einem der Ansprüche 1-18, wobei die Fracht eine Nukleinsäure oder ein Protein oder eine Nukleinsäure, die ein Protein codiert, das ein therapeutisches Produkt zur Behandlung von Krebs oder einer Infektionskrankheit oder einer neurodegenerativen Erkrankung oder einer anderen Störung des zentralen Nervensystems (ZNS) oder zur Linderung der Auswirkungen des Alterns oder zur Verlangsamung oder Umkehrung des Alterns oder zur Behandlung einer altersbedingten Krankheit oder einer Augenerkrankung oder einer immunologischen Störung ist.

**20.** Zusammensetzung zur Verwendung nach einem der Ansprüche 1-19, wobei die Fracht einen Agonisten oder Antagonisten eines intrazellulären endosomalen Rezeptors umfasst.

**21.** Zusammensetzung zur Verwendung nach Anspruch 20, wobei der Rezeptor ein Toll-like-Rezeptor (TLR) ist.

**22.** Zusammensetzung zur Verwendung eines der Ansprüche 1-21, wobei:

die MEVs zur oralen Verabreichung formuliert sind, und die Krankheit die Abgabe in den Darm oder die Milz betrifft oder die Behandlung darauf abzielt; oder
die MEVs zur oralen Verabreichung formuliert sind, und die Krankheit den Magen-Darm-Trakt, das Immunsystem oder die weiße Milz betrifft oder die Behandlung darauf abzielt; oder
die MEVs zur intratrachealen Verabreichung oder Inhalation formuliert sind, und die Krankheit die Lunge betrifft oder die Behandlung darauf abzielt oder Atemwegskrankheiten behandelt; oder
die MEVs zur intranasalen Verabreichung formuliert sind und die Krankheit, Störung oder der Zustand eine Krankheit des Gehirns ist oder dieses betrifft; oder
die MEVs zur Verabreichung an das Auge formuliert sind, und die Krankheit, Störung oder der Zustand altersbedingte Makuladegeneration, Herpes-Stromakeratitis, Glaukom, Syndrom des trockenen Auges, diabetische Retinopathie, ein Zustand im Zusammenhang mit okulärer Angiogenese oder Augenüberdruck ist.

**23.** Zusammensetzung zur Verwendung nach einem der Ansprüche 20-22, wobei die Krankheit, Störung oder der Zustand eine Krankheit des zentralen Nervensystems oder des Nervensystems ist und die Zusammensetzung intranasal verabreicht wird.

**24.** Zusammensetzung zur Verwendung nach einem der Ansprüche 20-22, wobei die Krankheit, Störung oder der Zustand durch Immunmodulation behandelt wird und die Fracht einen Impfstoff umfasst.

**25.** Zusammensetzung nach einem der Ansprüche 20-22, wobei die Krankheit, Störung oder der Zustand Krebs und/oder eine Störung der Immunzellen oder eine Krankheit ist, die durch einen Impfstoff behandelt oder verhindert wird.

**26.** Zusammensetzung zur Verwendung nach einem der Ansprüche 20-22, wobei die Zusammensetzung zur oralen Verabreichung formuliert ist und die Krankheit, Störung oder der Zustand eine Darminfektion, Morbus Crohn oder Krebs ist.

## Revendications

**1.** Composition destinée à être utilisée dans le traitement d'une maladie, d'un trouble ou d'une affection, cette composition comprenant des vésicules extracellulaires de microalgues (MEV) pour l'administration d'une charge bioactive au tissu cible, dans laquelle :

la composition est formulée pour être administrée par voie orale, intraveineuse, intratrachéale, intramusculaire, intranasale, par instillation dans l'œil ou par inhalation dans les poumons d'un sujet, pour le transport des MEV vers un tissu cible ;
la microalgue est une espèce de la famille des Chlorellaceae ;
les MEV contiennent une charge bioactive hétérologue destinée au traitement d'une maladie, d'un trouble ou d'une affection touchant le tissu cible ;
la formulation et la voie d'administration des MEV influencent l'acheminement de la charge vers le tissu cible.

**2.** Composition selon la revendication 1, dans laquelle le tissu cible est choisi parmi les tissus des poumons, du foie, de la rate, de l'intestin, du cerveau, de la muqueuse naso-buccale, du cœur, des reins, de la rétine et/ou de la choroïde de l'œil.

**3.** Composition destinée à être utilisée selon la revendication 1 ou la revendication 2, dans laquelle la microalgue est une espèce de *Chlorella* sélectionnée parmi les *Chlorella ellipsoidea, Chlorella pyrenoidosa, Chlorella sorokiniana, Chlorella vulgaris* et *Chlorella variabilis.*

**4.** Composition à utiliser selon la revendication 3, dans laquelle la *Chlorella* est une *Chlorella vulgaris.*

**5.** Composition destinée à être utilisée selon l'une quelconque des revendications 1-4, dans laquelle :

les MEV sont formulées pour une administration orale et la maladie implique ou le traitement cible l'administration au niveau de l'intestin ou de la rate ; ou
les MEV sont formulées pour une administration orale et la maladie implique ou le traitement cible le tractus gastro-intestinal, le système immunitaire ou la rate blanche ; ou
les MEV sont formulées pour une administration intratrachéale ou par inhalation et la maladie implique ou le traitement cible les poumons ou traite les maladies respiratoires ; ou
les MEV sont formulées pour une administration intranasale et la maladie, le trouble ou l'affection est une maladie du cerveau ou l'impliquant ; ou
les MEV sont formulées pour une administration oculaire, et la maladie, le trouble ou l'affection est la dégénérescence maculaire liée à l'âge, la kératite stromale herpétique, le glaucome, le syndrome de l'œil sec, la rétinopathie diabétique, une affection associée à l'angiogenèse oculaire ou l'hypertension oculaire ; ou
les MEV sont formulées pour une administration intranasale, et la maladie, le trouble ou l'affection implique le système nerveux central ou le système nerveux ; ou.
les MEV sont formulées pour une administration intraveineuse, et la maladie concerne le foie, ou le traitement le cible ; ou
les MEV comprennent un agent thérapeutique ou codent pour un agent thérapeutique destiné au traitement d'une maladie, d'un trouble ou d'une affection touchant le foie, la composition est formulée pour une administration intraveineuse et la maladie touche le foie ou le traitement cible ce dernier.

**6.** Composition destinée à être utilisée selon l'une quelconque des revendications 1-5, comprenant des vésicules extracellulaires de microalgues (MEV) dans un excipient pharmaceutiquement acceptable, dans laquelle :

la composition est formulée pour une administration orale, le tissu cible est le tissu lymphoïde associé à l'intestin (GALT), et la charge est immunomodulatrice ou est un antigène destiné à induire une réponse immunitaire ; ou
la composition est formulée pour une administration orale et la maladie, le trouble ou l'affection concerne le tube digestif, le système immunitaire ou la rate blanche, et la charge est destinée au traitement d'une maladie, d'un trouble ou d'une affection par immunomodulation ou vaccination ; ou
la composition est formulée pour une administration intranasale, le tissu cible est le cerveau, et la charge comprend une molécule bioactive destinée au traitement, à la détection ou au diagnostic d'une maladie, d'un trouble ou d'une affection du cerveau ou impliquant le cerveau, ou au suivi du traitement d'une maladie, d'un trouble ou d'une affection du cerveau ou impliquant le cerveau ; ou
la composition est formulée pour être instillée dans l'œil sous forme de gouttes, le tissu cible est la choroïde ou la rétine, et la charge est destiné au traitement d'une maladie, d'un trouble ou d'une affection oculaire ; ou
la composition est formulée pour une administration intramusculaire ou orale, la cible est le système immunitaire et la charge comprend un antigène pour la vaccination ou une protéine ou un produit immunomodulateur ; ou.
la composition est formulée pour une administration orale et la maladie, le trouble ou l'affection concerne un organe ou des tissus accessibles par la circulation sanguine ; ou
la composition est formulée pour une administration intratrachéale, une inhalation ou une nébulisation, la maladie, le trouble ou l'affection concerne les voies respiratoires ou est liée aux voies respiratoires, et la charge est un produit destiné au traitement des voies respiratoires ou des maladies respiratoires ou des maladies impliquant les voies respiratoires.

**7.** Composition destinée à être utilisée selon l'une quelconque des revendications 1-6, dans laquelle la maladie est traitée par modulation immunitaire, et les MEV comprennent un vaccin.

**8.** Composition destinée à être utilisée selon l'une quelconque des revendications 1-7, dans laquelle la maladie, le

trouble ou l'affection est un cancer et/ou un trouble des cellules immunitaires ou une maladie traitée ou prévenue par un vaccin.

**9.** Composition destinée à être utilisée selon l'une quelconque des revendications 1-8, dans laquelle la charge comprend une molécule d'acide nucléique, un polypeptide, une protéine, un plasmide, un aptamère, un oligonucléotide antisens ou un médicament à petite molécule.

**10.** Composition à utiliser selon la revendication 9, dans laquelle la charge est un acide nucléique qui est de l'ADN ou de l'ARN.

**11.** Composition à utiliser selon la revendication 10, dans laquelle la charge comprend un plasmide.

**12.** Composition destinée à être utilisée selon l'une quelconque des revendications 1-6, dans laquelle la charge comprend ou code pour un agent immunomodulateur afin d'augmenter ou de diminuer la production d'un ou plusieurs produits qui régulent à la hausse ou à la baisse la présentation d'auto-antigènes ; masquent les antigènes MHC ; ou favorisent la prolifération, la différenciation, la migration ou l'état d'activation d'un ou plusieurs types de cellules immunitaires.

**13.** Composition destinée à être utilisée selon la revendication 12, dans laquelle la charge comprend ou code pour une hormone, une cytokine ou une chimiokine.

**14.** Composition destinée à être utilisée selon l'une quelconque des revendications 1-13, dans laquelle la charge comprend un acide nucléique qui code un produit pour la thérapie génique.

**15.** Composition destinée à être utilisée selon la revendication 14, dans laquelle la thérapie génique est destinée au traitement d'une erreur innée du métabolisme.

**16.** Composition destinée à être utilisée selon l'une quelconque des revendications 1-13, dans laquelle la charge comprend ou code pour une protéine qui est un anticorps ou un fragment de liaison à l'antigène de celui-ci.

**17.** Composition destinée à être utilisée selon la revendication 16, dans laquelle l'anticorps est un scFv, un anticorps bispécifique ou un fragment de liaison à l'antigène de celui-ci.

**18.** Composition destinée à être utilisée selon l'une quelconque des revendications 1-17, dans laquelle les MEV comprennent un vaccin à ADN, un vaccin à ARN ou un vaccin protéique.

**19.** Composition destinée à être utilisée selon l'une quelconque des revendications 1-18, dans laquelle la charge comprend un acide nucléique ou une protéine ou un acide nucléique codant pour une protéine qui est un produit thérapeutique pour traiter le cancer, ou une maladie infectieuse, ou une maladie neurodégénérative ou un autre trouble du système nerveux central (SNC), ou pour atténuer les effets du vieillissement, ou ralentir ou inverser le vieillissement, ou traiter une maladie associée au vieillissement, ou un trouble ophtalmique, ou un trouble immunologique.

**20.** Composition destinée à être utilisée selon l'une quelconque des revendications 1-19, dans laquelle la charge comprend un agoniste ou un antagoniste d'un récepteur endosomal intracellulaire.

**21.** Composition destinée à être utilisée selon la revendication 20, dans laquelle le récepteur est un récepteur de type Toll (TLR).

**22.** Composition destinée à être utilisée selon l'une quelconque des revendications 1-21 dans laquelle :

les MEV sont formulées pour une administration orale et la maladie implique ou le traitement cible l'administration au niveau de l'intestin ou de la rate ; ou
les MEV sont formulées pour une administration orale et la maladie implique ou le traitement cible le tractus gastro-intestinal, le système immunitaire ou la rate blanche ; ou
les MEV sont formulées pour une administration intratrachéale ou par inhalation et la maladie implique ou le traitement cible les poumons ou traite les maladies respiratoires ; ou
les MEV sont formulées pour une administration intranasale et la maladie, le trouble ou l'affection est une maladie

du cerveau ou l’impliquant ; ou
les MEV sont formulées pour être administrées dans l’œil et la maladie, le trouble ou l’affection est la dégénérescence maculaire liée à l’âge, la kératite stromale herpétique, le glaucome, le syndrome de l’œil sec, la rétinopathie diabétique, une affection associée à l’angiogenèse oculaire ou l’hypertension oculaire.

**23.** Composition destinée à être utilisée selon l’une quelconque des revendications 20-22, dans laquelle la maladie, le trouble ou l’affection est une maladie du système nerveux central ou du système nerveux, et la composition est administrée par voie intranasale.

**24.** Composition destinée à être utilisée selon l’une quelconque des revendications 20-22, dans laquelle la maladie, le trouble ou l’affection est traité par modulation immunitaire, et la charge comprend un vaccin.

**25.** Composition selon l’une quelconque des revendications 20-22, dans laquelle la maladie, le trouble ou l’affection est un cancer et/ou un trouble des cellules immunitaires ou une maladie traitée ou prévenue par un vaccin.

**26.** Composition destinée à être utilisée selon l’une quelconque des revendications 20-22, dans laquelle la composition est formulée pour une administration orale et la maladie, le trouble ou l’affection est une infection intestinale, la maladie de Crohn ou un cancer.

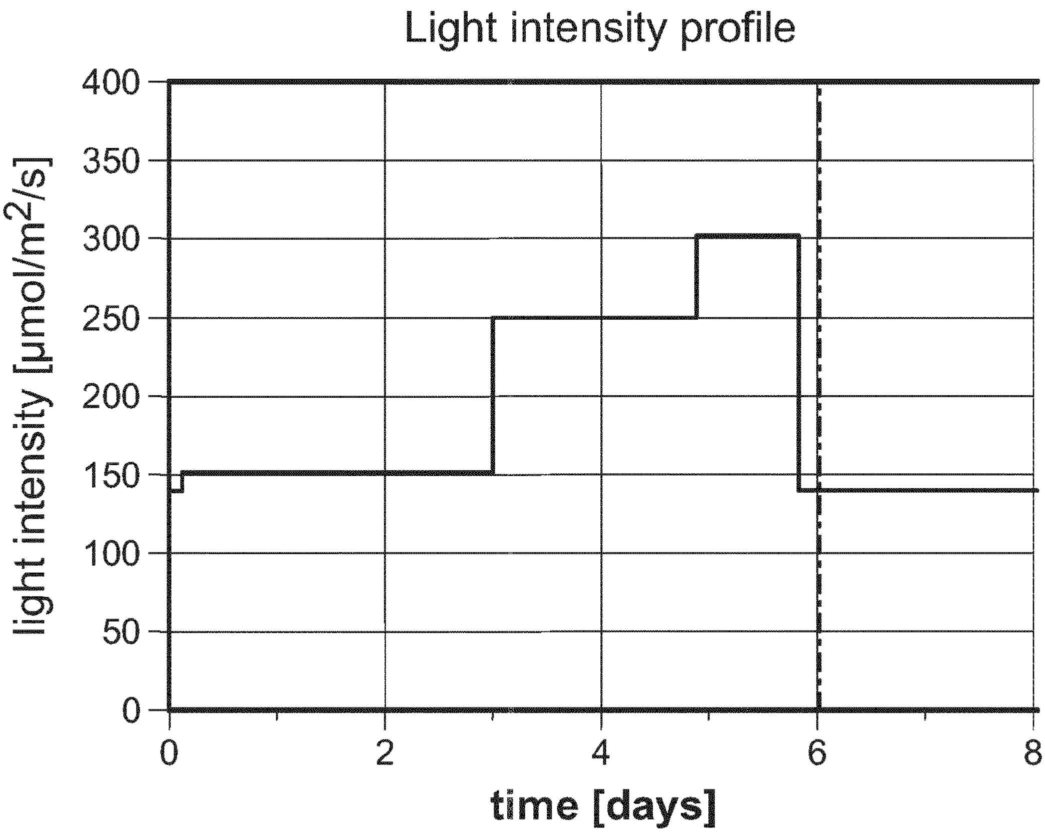
Light intensity profile
400
350
300
250
200
150
100
50
0
light intensity [µmol/m2/s]
0
2
4
6
8
time [days]

FIG. 1

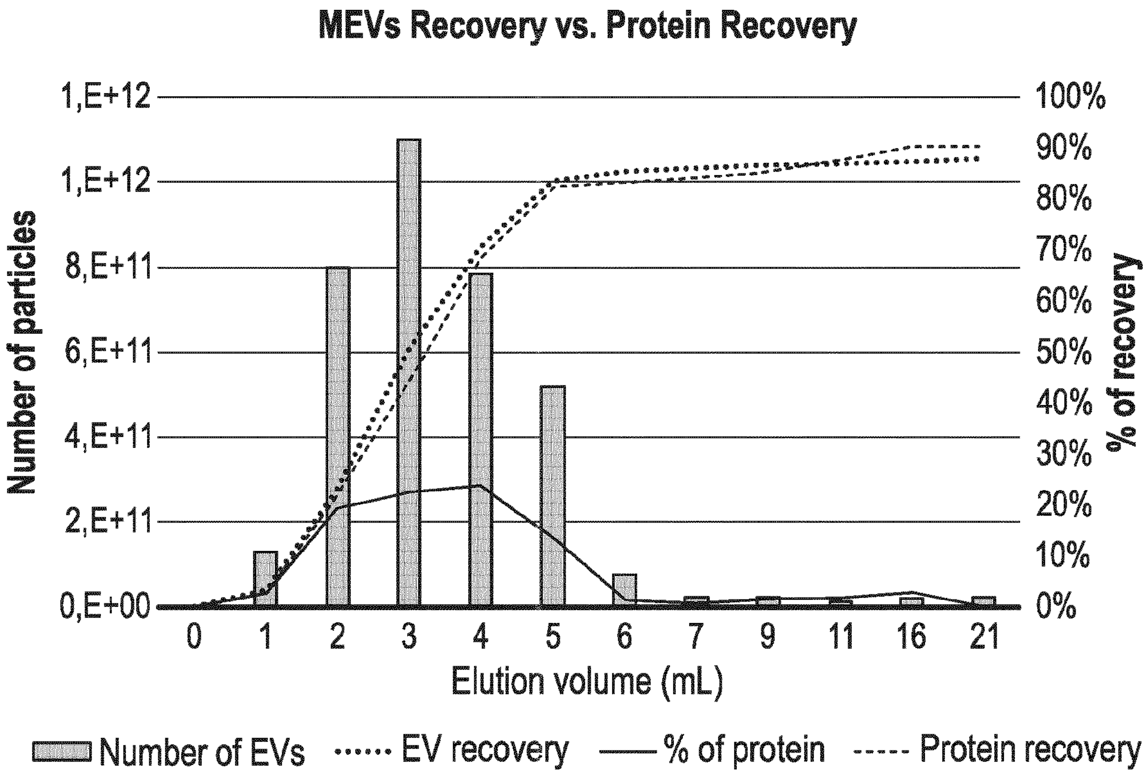
MEVs Recovery vs. Protein Recovery
1,E+12
1,E+12
8,E+11
6,E+11
4,E+11
2,E+11
0,E+00
Number of particles
100%
90%
80%
70%
60%
50%
40%
30%
20%
10%
0%
% of recovery
0
1
2
3
4
5
6
7
9
11
16
21
Elution volume (mL)
Number of EVs
EV recovery
% of protein
Protein recovery

FIG. 2

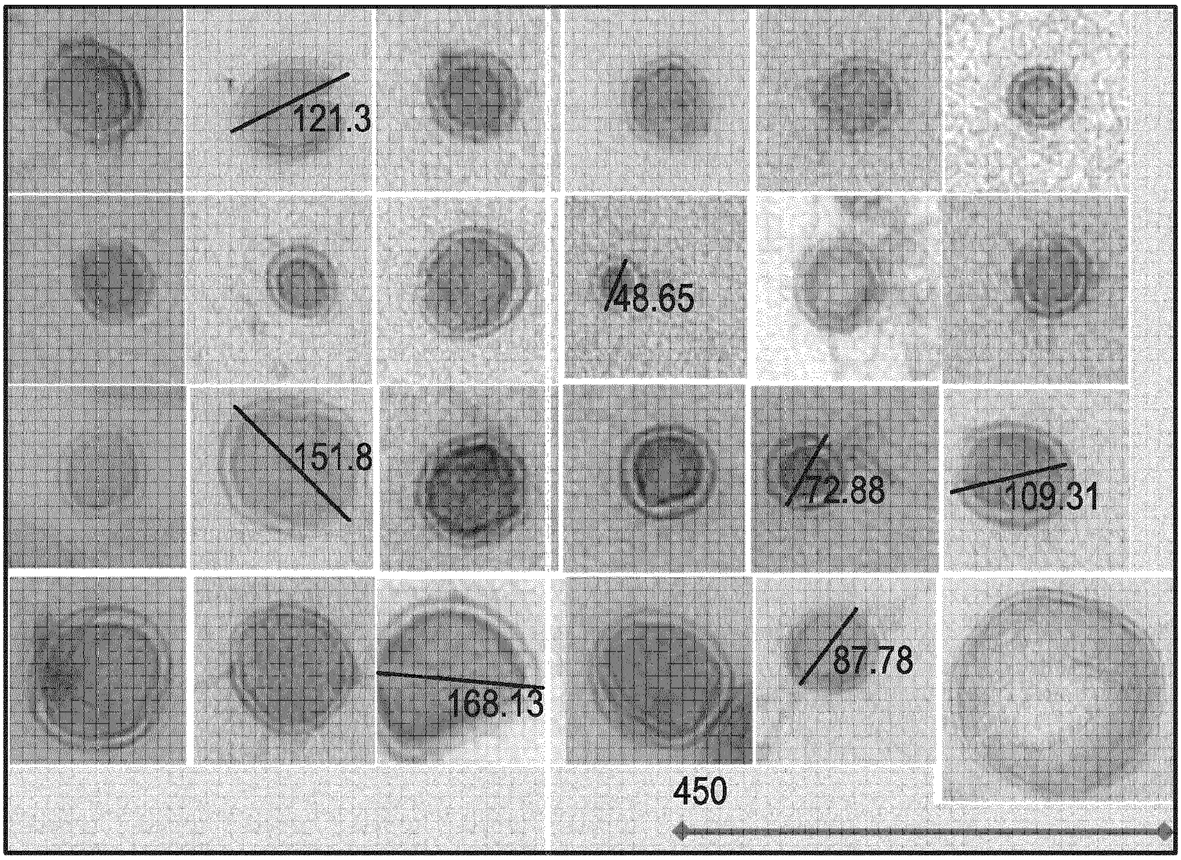


*all measurements in nm

FIG. 3

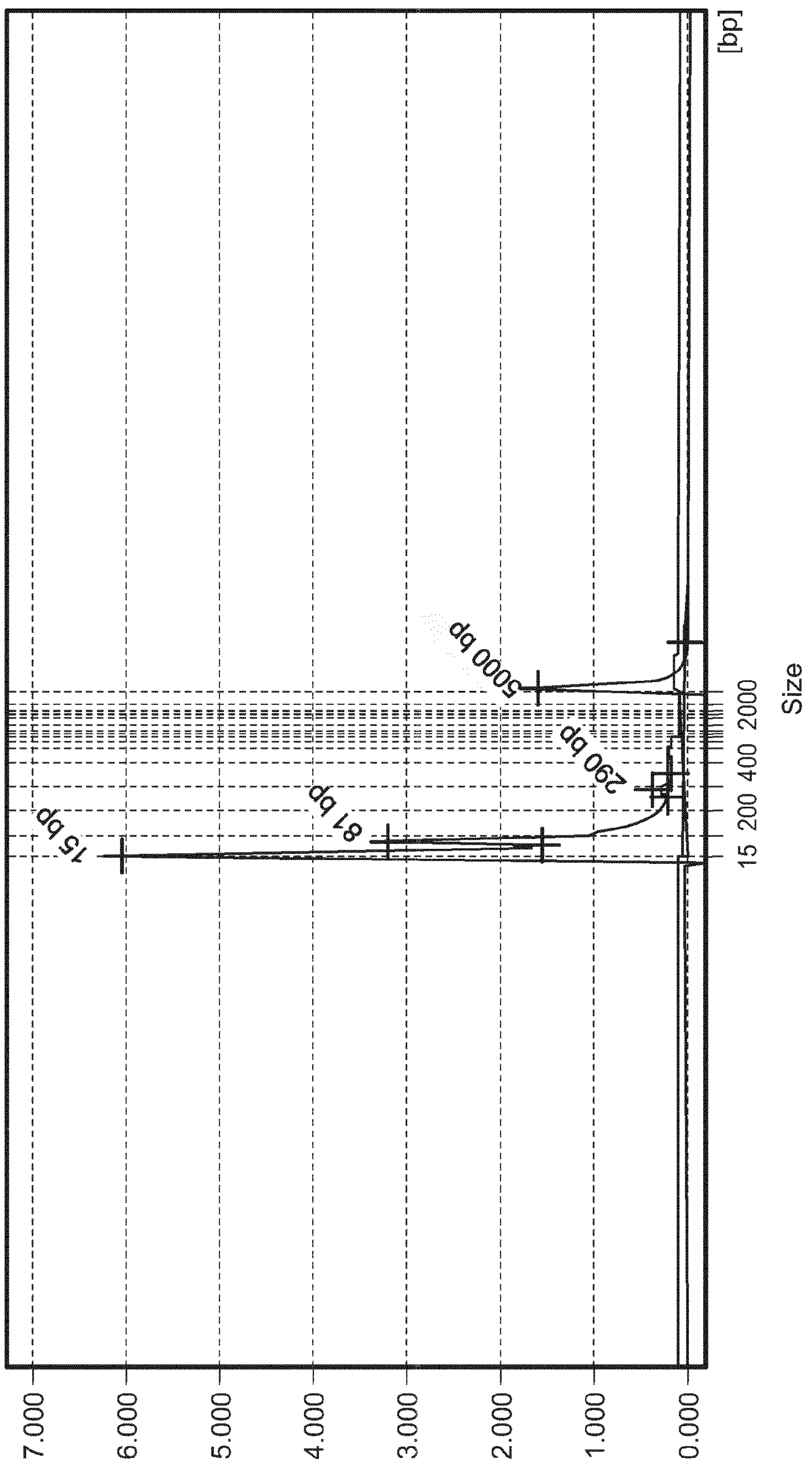
[RFU x 1E0]
7.000
6.000
5.000
4.000
3.000
2.000
1.000
0.000
15 bp
81 bp
290 bp
5000 bp
15
200
400
2000
Size
[bp]

FIG. 4

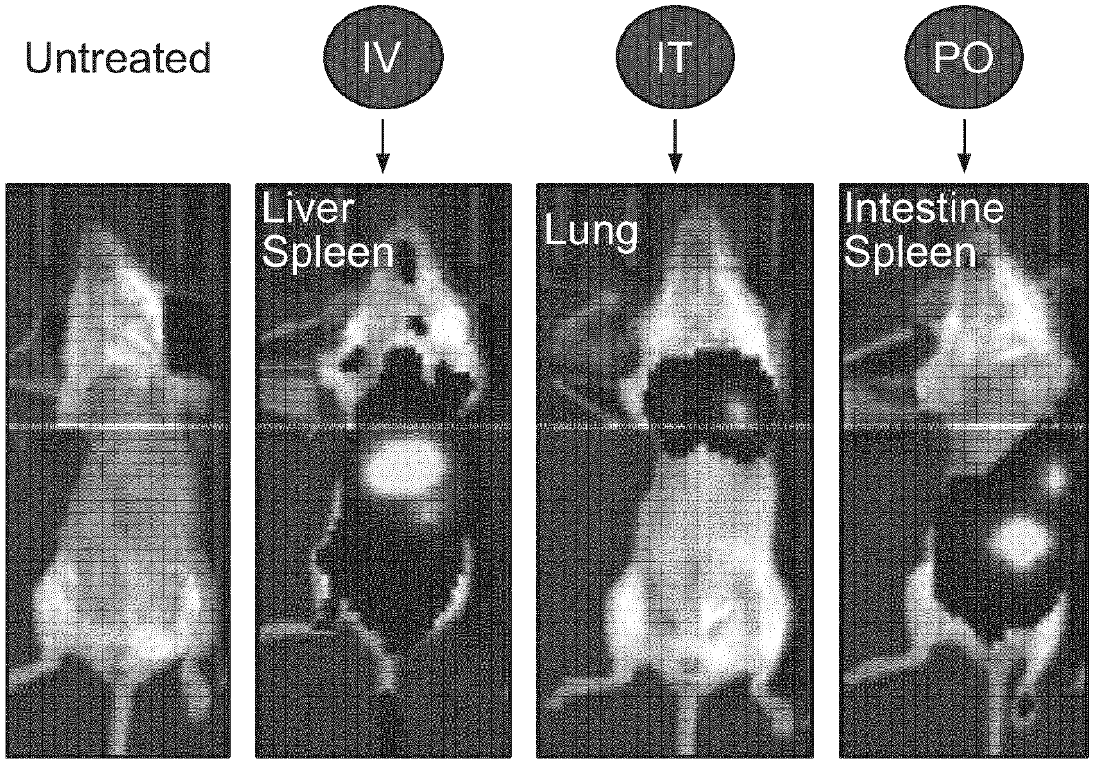


PO -PER OS
IT -INTRATHRACHEAL
IV -INTRAVENOUS

Each delivery route allows to target a specific set of organs

FIG. 5

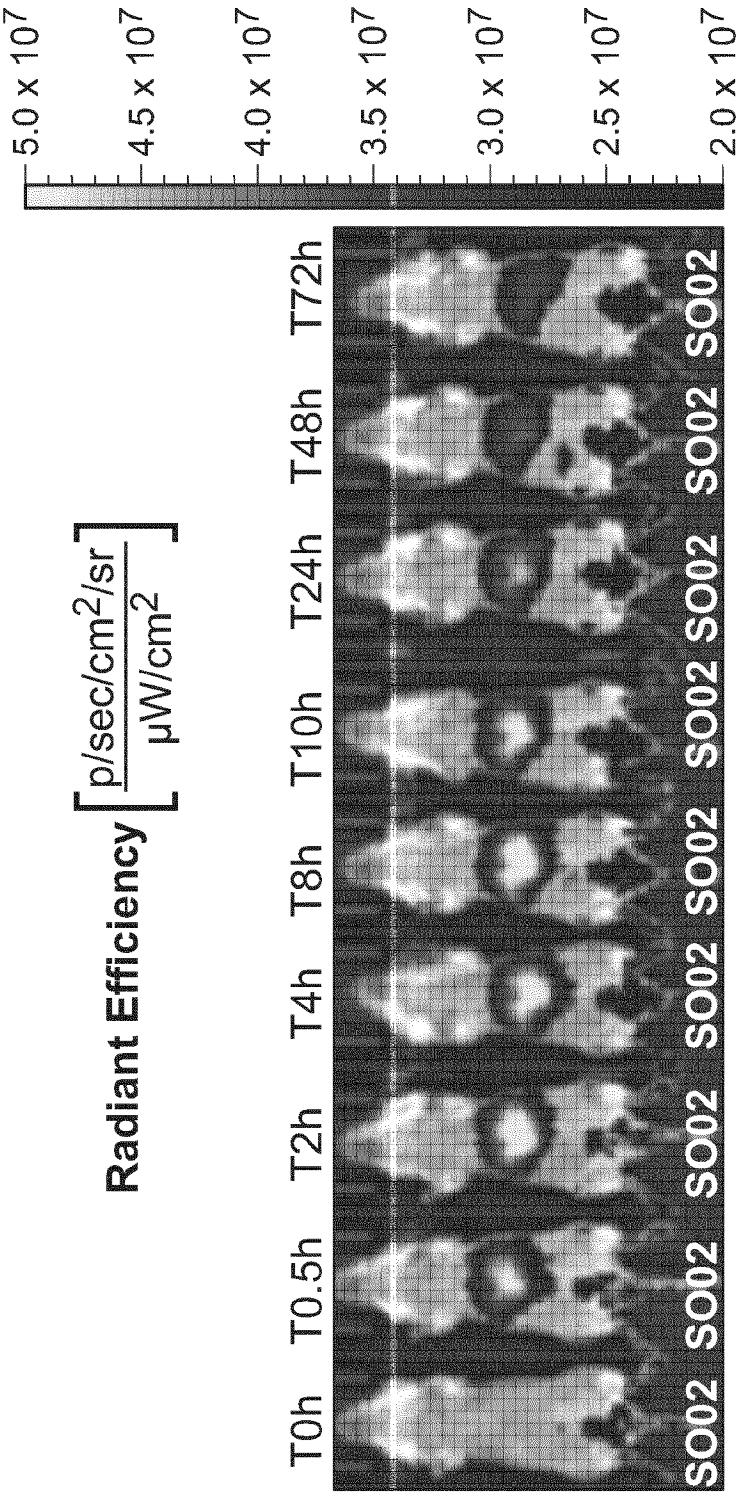
Radiant Efficiency [p/sec/cm²/sr / μW/cm²]
5.0 x 10^7
4.5 x 10^7
4.0 x 10^7
3.5 x 10^7
3.0 x 10^7
2.5 x 10^7
2.0 x 10^7
T0h
T0.5h
T2h
T4h
T8h
T10h
T24h
T48h
T72h
SO02
SO02
SO02
SO02
SO02
SO02
SO02
SO02
SO02

FIG. 6

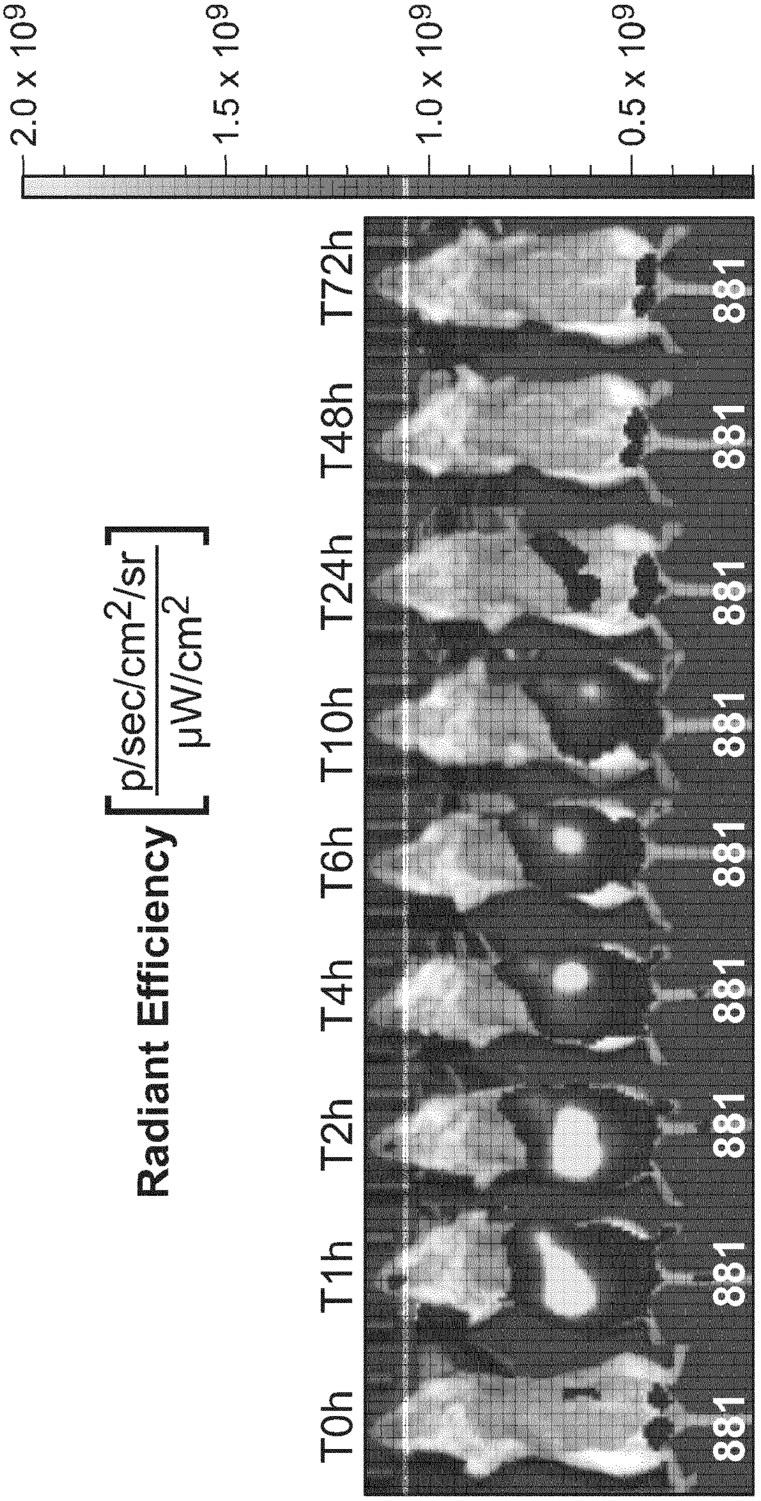

Radiant Efficiency [p/sec/cm²/sr / μW/cm²]
2.0 x 10⁹
1.5 x 10⁹
1.0 x 10⁹
0.5 x 10⁹
T0h
T1h
T2h
T4h
T6h
T10h
T24h
T48h
T72h
881
881
881
881
881
881
881
881
881


FIG. 7

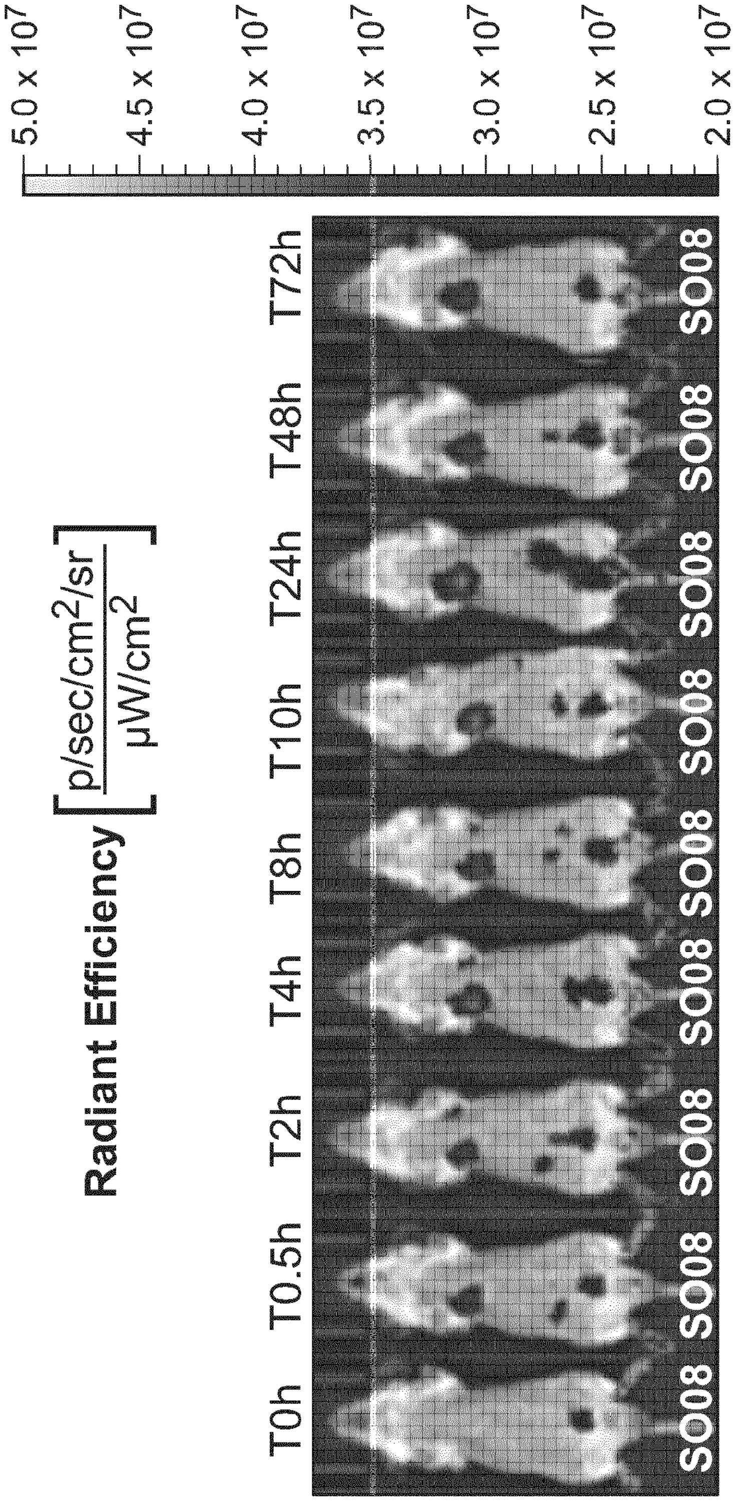
Radiant Efficiency [p/sec/cm²/sr / μW/cm²]
5.0 x 10^7
4.5 x 10^7
4.0 x 10^7
3.5 x 10^7
3.0 x 10^7
2.5 x 10^7
2.0 x 10^7
T0h
T0.5h
T2h
T4h
T8h
T10h
T24h
T48h
T72h
SO08
SO08
SO08
SO08
SO08
SO08
SO08
SO08
SO08

FIG. 8

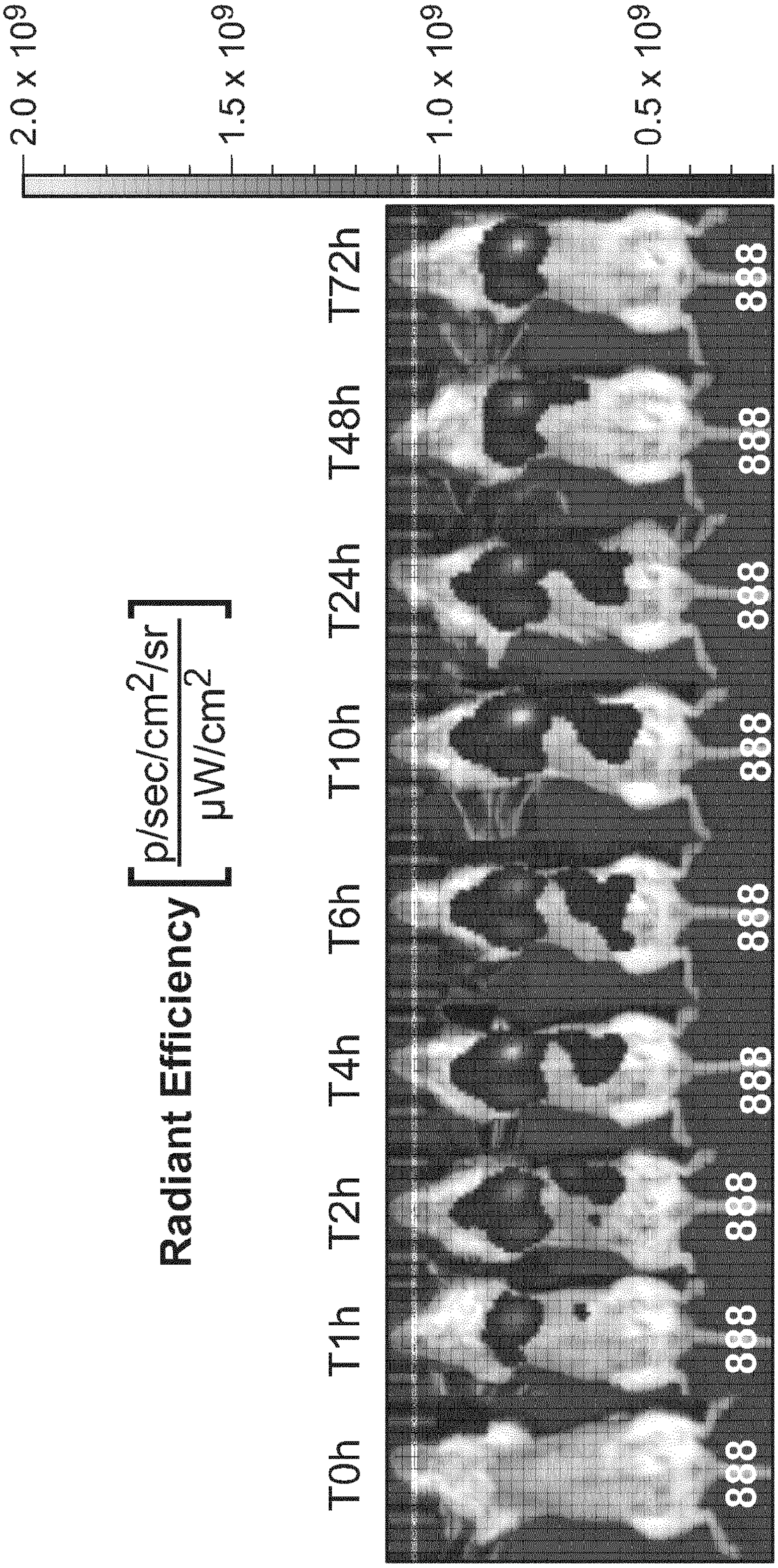
Radiant Efficiency [p/sec/cm²/sr / μW/cm²]
2.0 x 10⁹
1.5 x 10⁹
1.0 x 10⁹
0.5 x 10⁹
T0h
T1h
T2h
T4h
T6h
T10h
T24h
T48h
T72h

FIG. 9

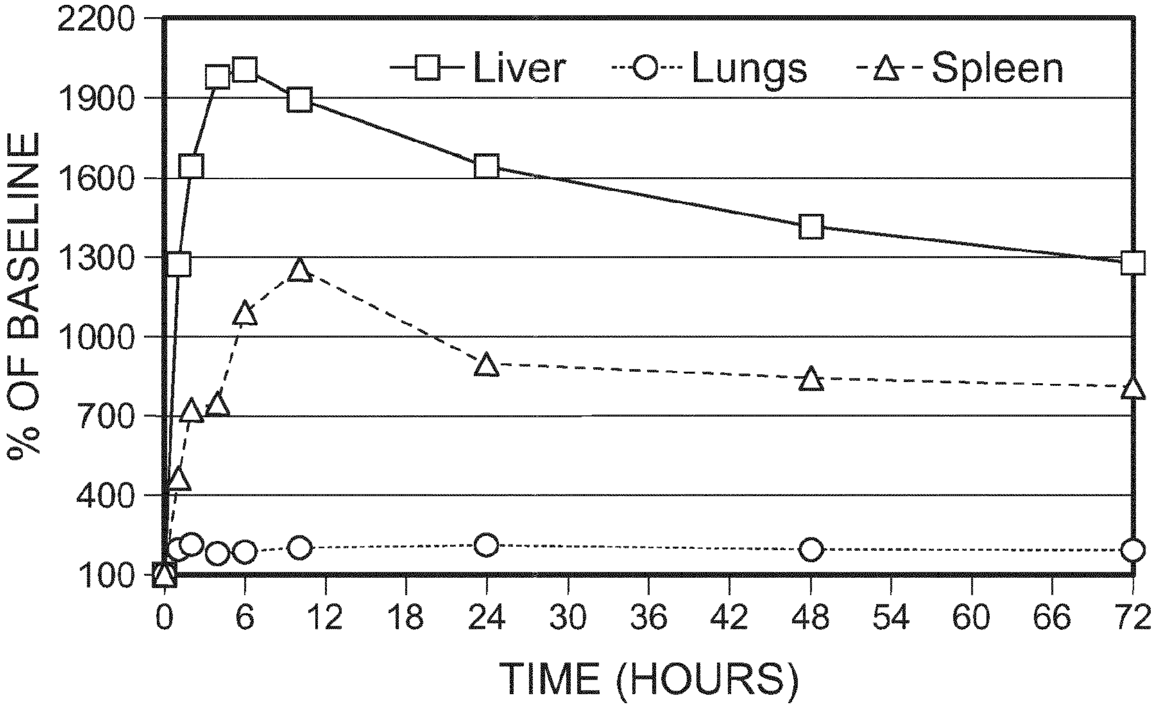
Liver
Lungs
Spleen
% OF BASELINE
2200
1900
1600
1300
1000
700
400
100
0
6
12
18
24
30
36
42
48
54
60
66
72
TIME (HOURS)

**FIG. 10**

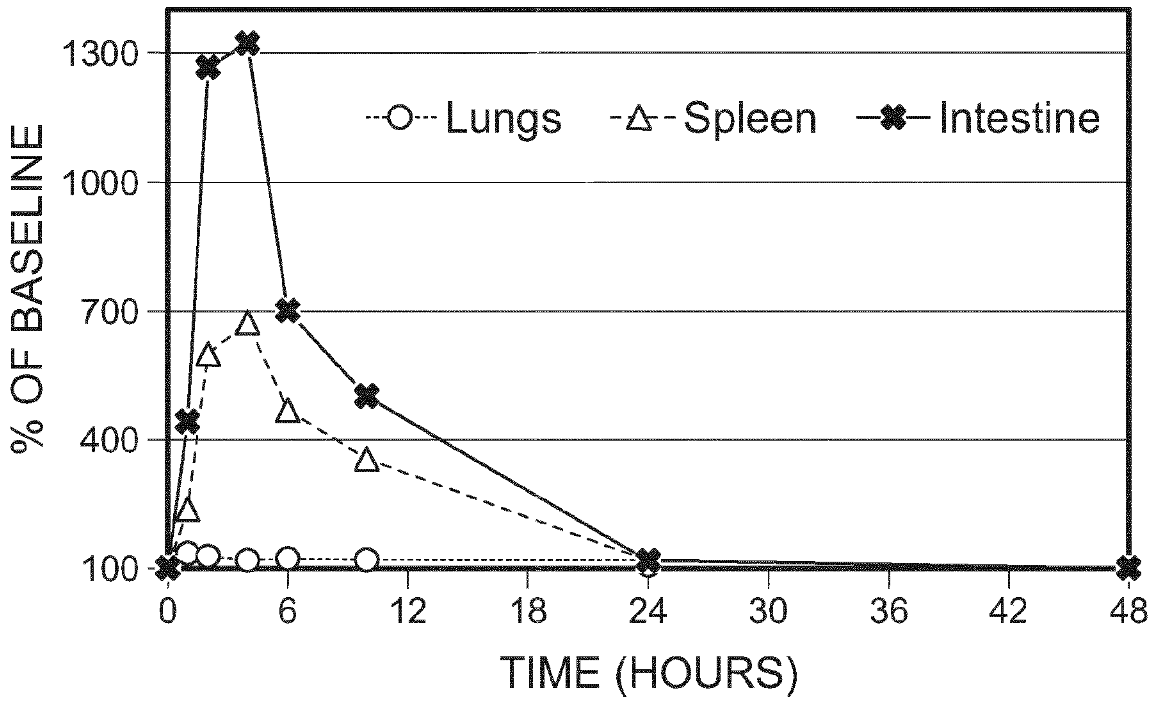
1300
1000
700
400
100
% OF BASELINE
0
6
12
18
24
30
36
42
48
TIME (HOURS)
Lungs
Spleen
Intestine

**FIG. 11**

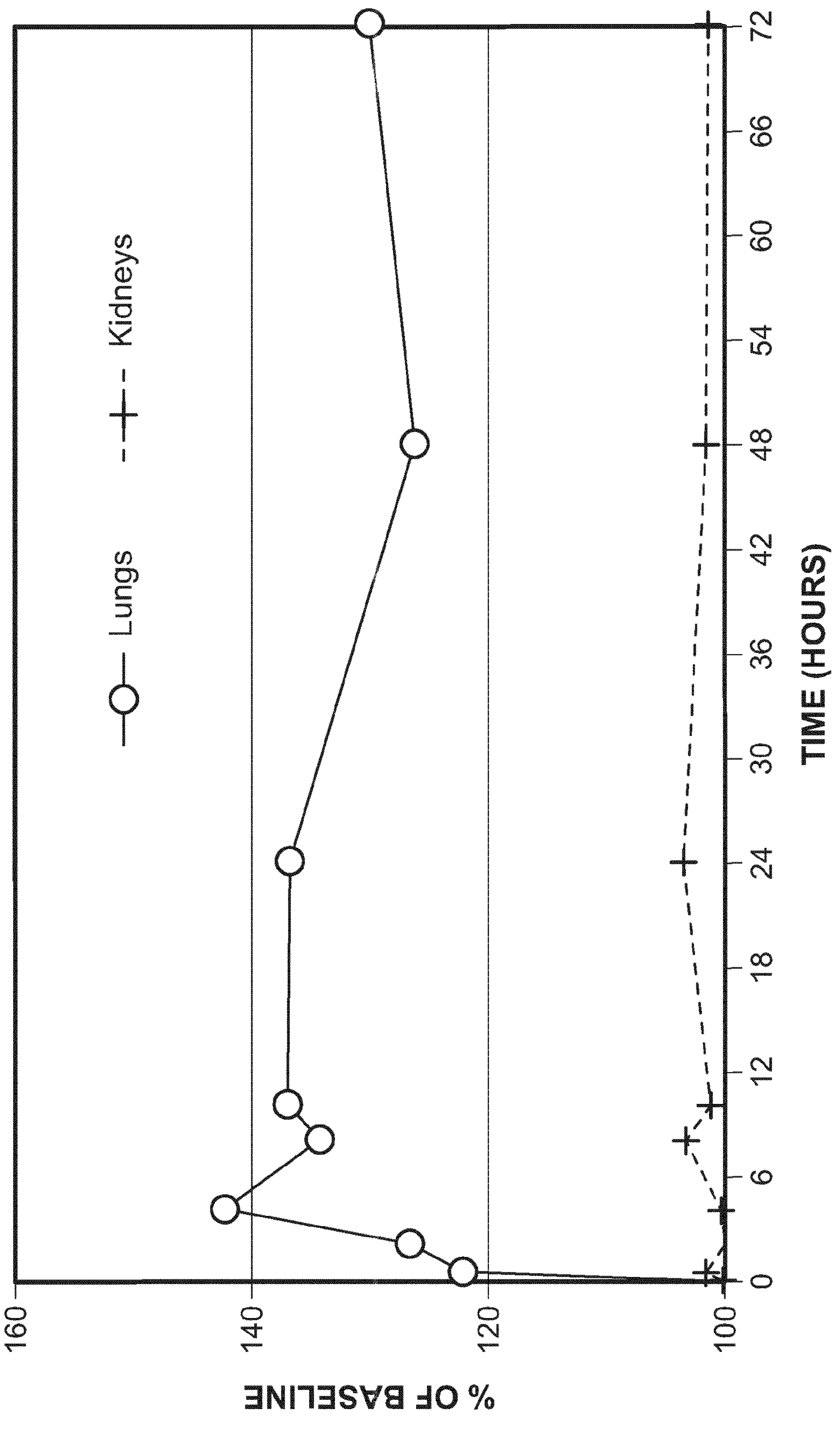
Lungs
Kidneys
TIME (HOURS)
% OF BASELINE
0
6
12
18
24
30
36
42
48
54
60
66
72
100
120
140
160

FIG. 12

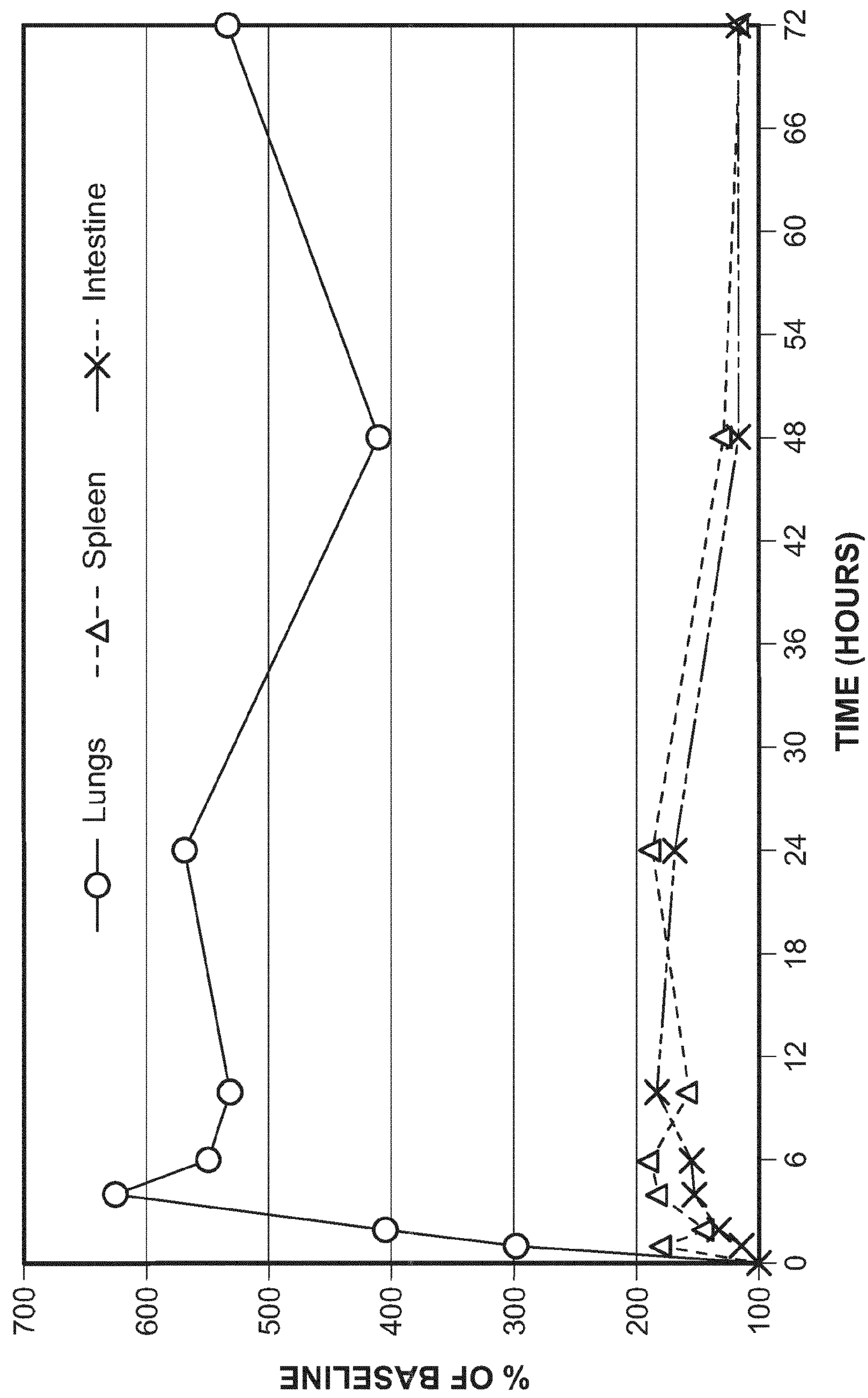
Lungs
Spleen
Intestine
700
600
500
400
300
200
100
% OF BASELINE
0
6
12
18
24
30
36
42
48
54
60
66
72
TIME (HOURS)

FIG. 13

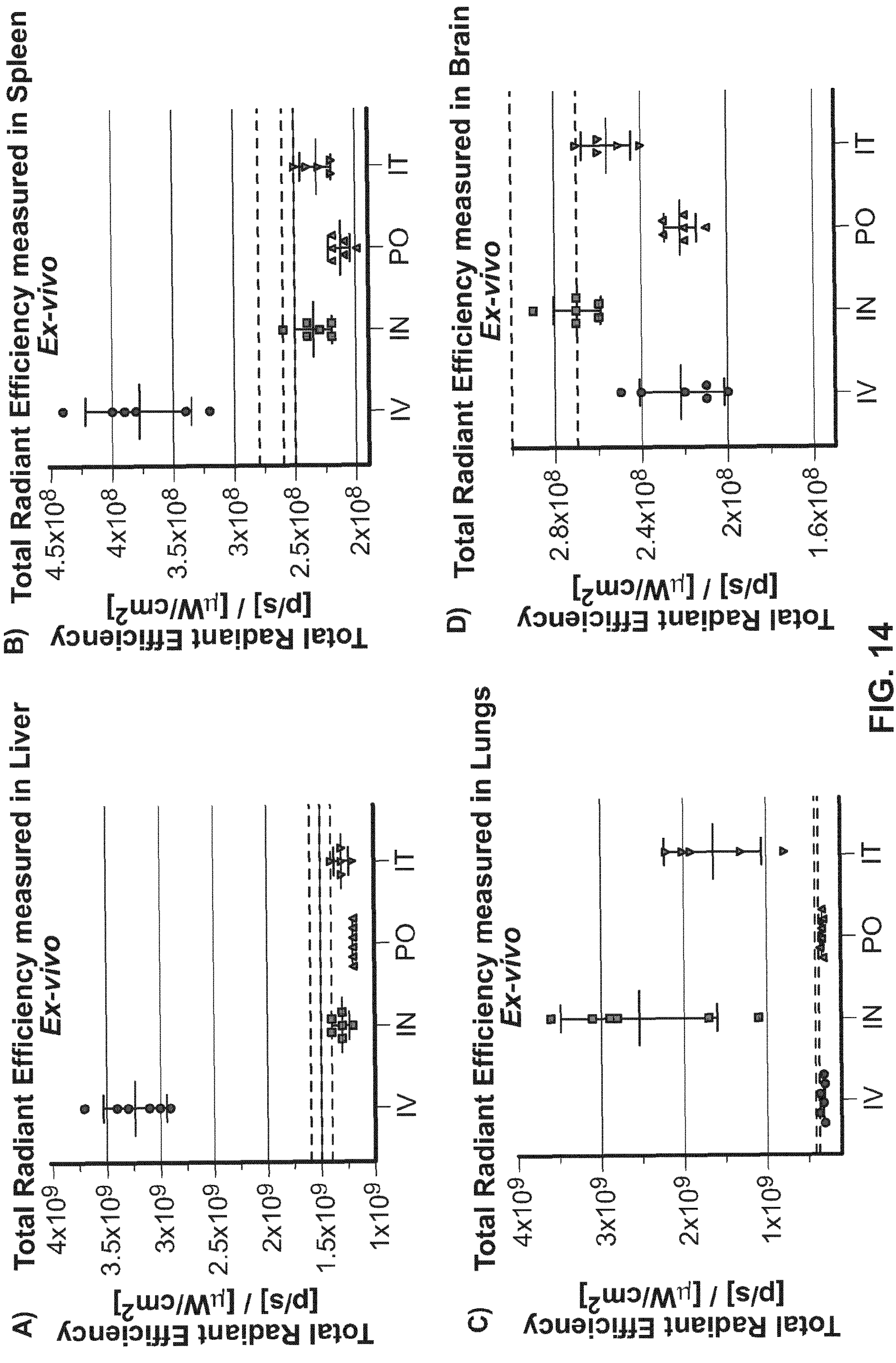
A) Total Radiant Efficiency measured in Liver
Ex-vivo
Total Radiant Efficiency [p/s] / [µW/cm²]
4x10⁹
3.5x10⁹
3x10⁹
2.5x10⁹
2x10⁹
1.5x10⁹
1x10⁹
IV
IN
PO
IT
B) Total Radiant Efficiency measured in Spleen
Ex-vivo
Total Radiant Efficiency [p/s] / [µW/cm²]
4.5x10⁸
4x10⁸
3.5x10⁸
3x10⁸
2.5x10⁸
2x10⁸
IV
IN
PO
IT
C) Total Radiant Efficiency measured in Lungs
Ex-vivo
Total Radiant Efficiency [p/s] / [µW/cm²]
4x10⁹
3x10⁹
2x10⁹
1x10⁹
IV
IN
PO
IT
D) Total Radiant Efficiency measured in Brain
Ex-vivo
Total Radiant Efficiency [p/s] / [µW/cm²]
2.8x10⁸
2.4x10⁸
2x10⁸
1.6x10⁸
IV
IN
PO
IT

FIG. 14

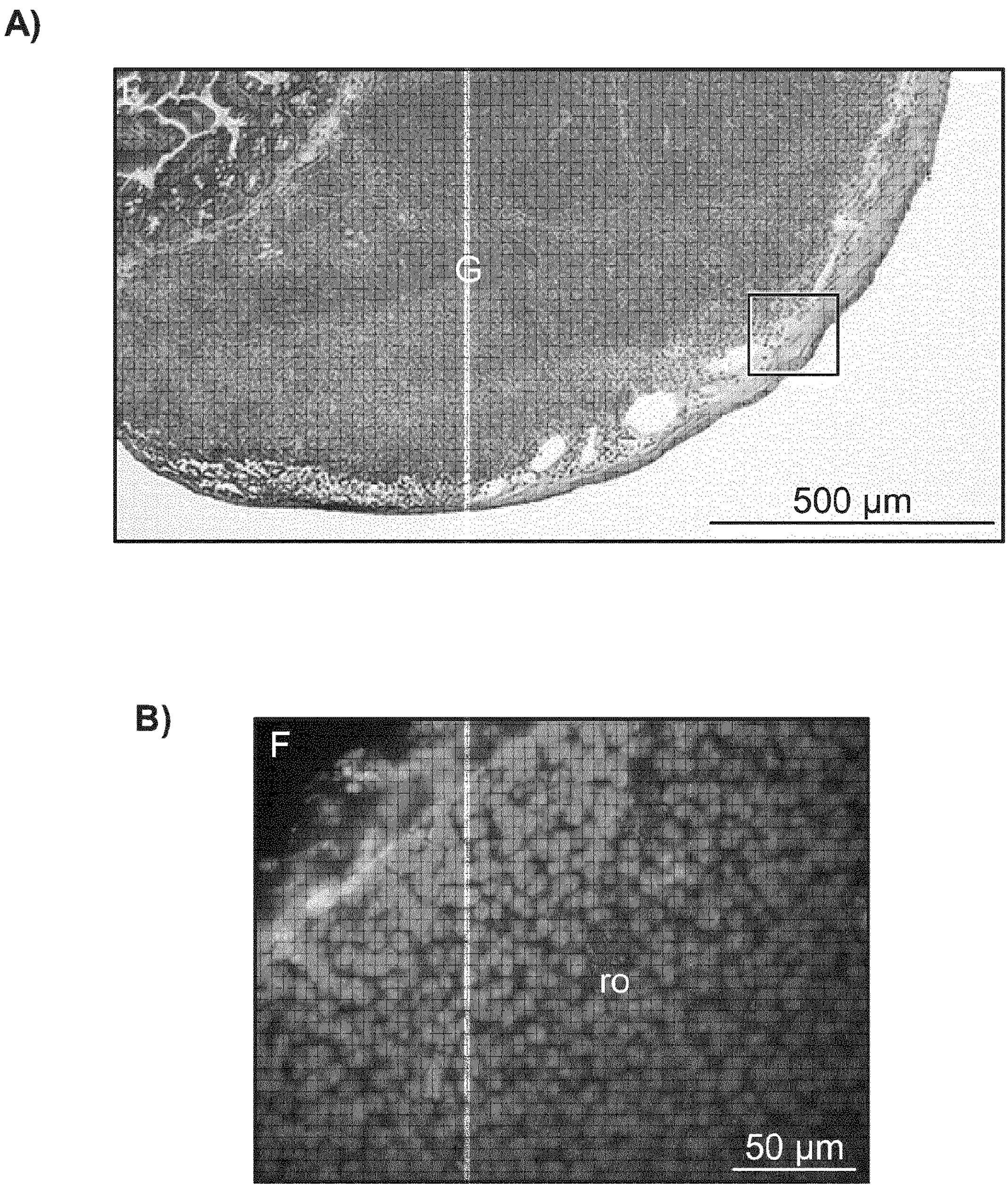


FIG. 15

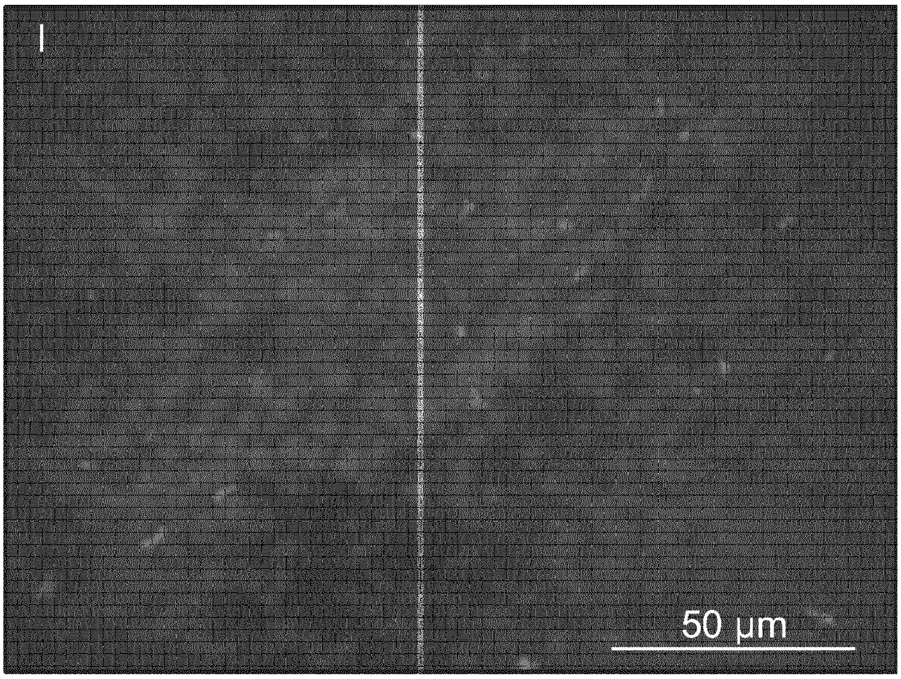


FIG. 16

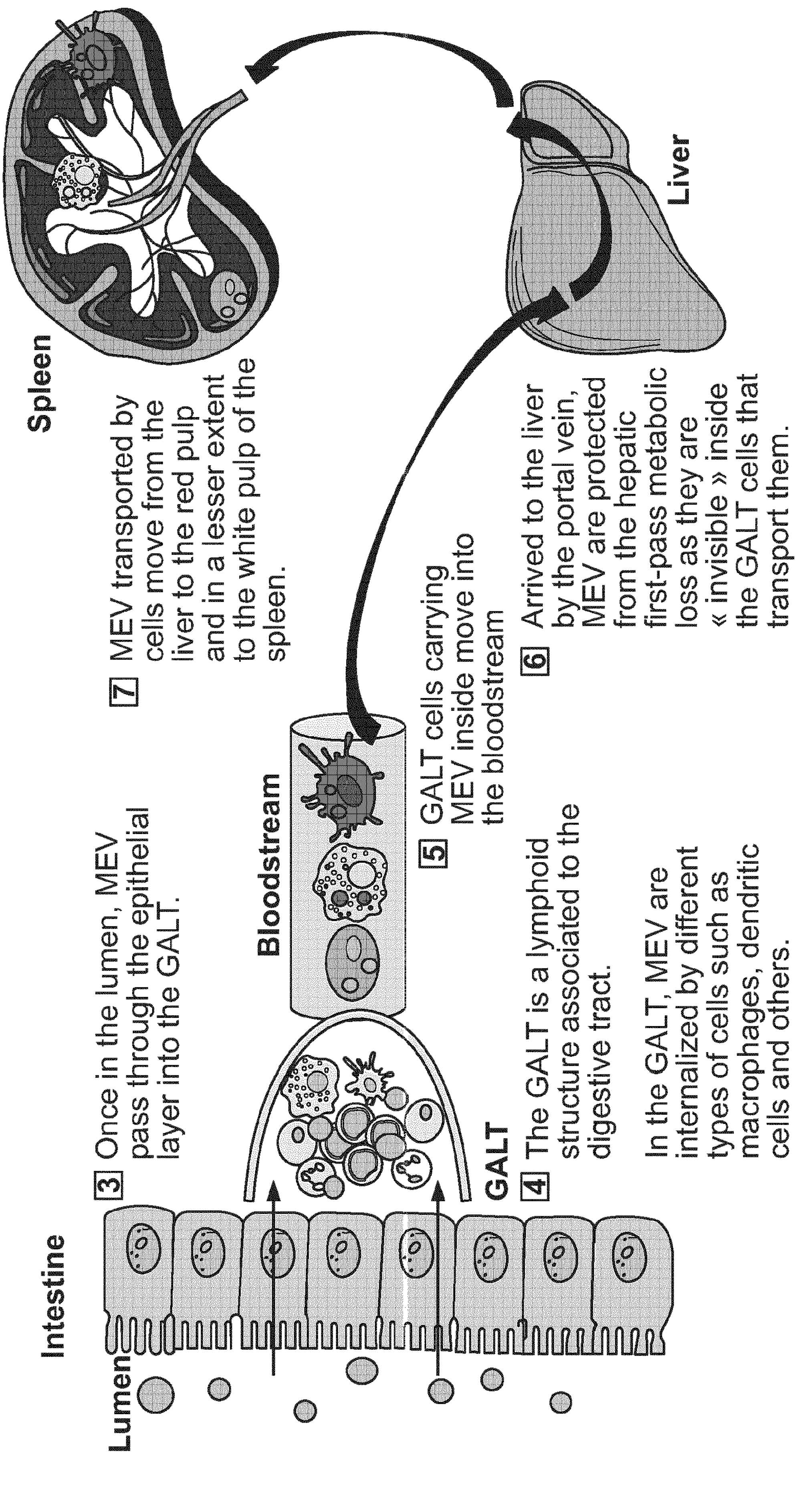
Intestine
Lumen
3 Once in the lumen, MEV pass through the epithelial layer into the GALT.
GALT
4 The GALT is a lymphoid structure associated to the digestive tract.
In the GALT, MEV are internalized by different types of cells such as macrophages, dendritic cells and others.
Bloodstream
5 GALT cells carrying MEV inside move into the bloodstream
Spleen
7 MEV transported by cells move from the liver to the red pulp and in a lesser extent to the white pulp of the spleen.
Liver
6 Arrived to the liver by the portal vein, MEV are protected from the hepatic first-pass metabolic loss as they are « invisible » inside the GALT cells that transport them.

FIG. 17

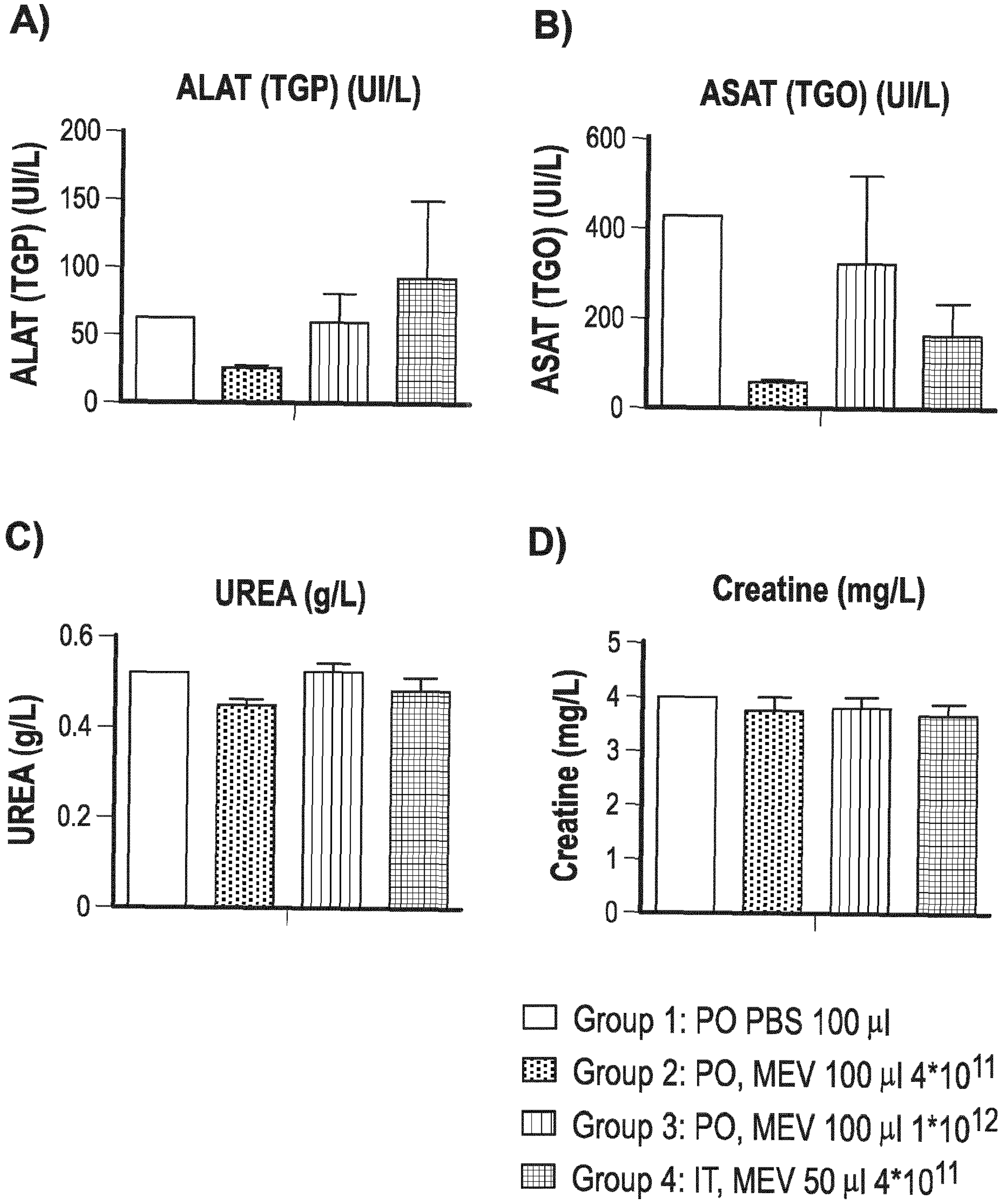
A)
ALAT (TGP) (UI/L)
ALAT (TGP) (UI/L)
200
150
100
50
0
B)
ASAT (TGO) (UI/L)
ASAT (TGO) (UI/L)
600
400
200
0
C)
UREA (g/L)
UREA (g/L)
0.6
0.4
0.2
0
D)
Creatine (mg/L)
Creatine (mg/L)
5
4
3
2
1
0
Group 1: PO PBS 100 µl
Group 2: PO, MEV 100 µl 4*10^11
Group 3: PO, MEV 100 µl 1*10^12
Group 4: IT, MEV 50 µl 4*10^11

FIG. 18

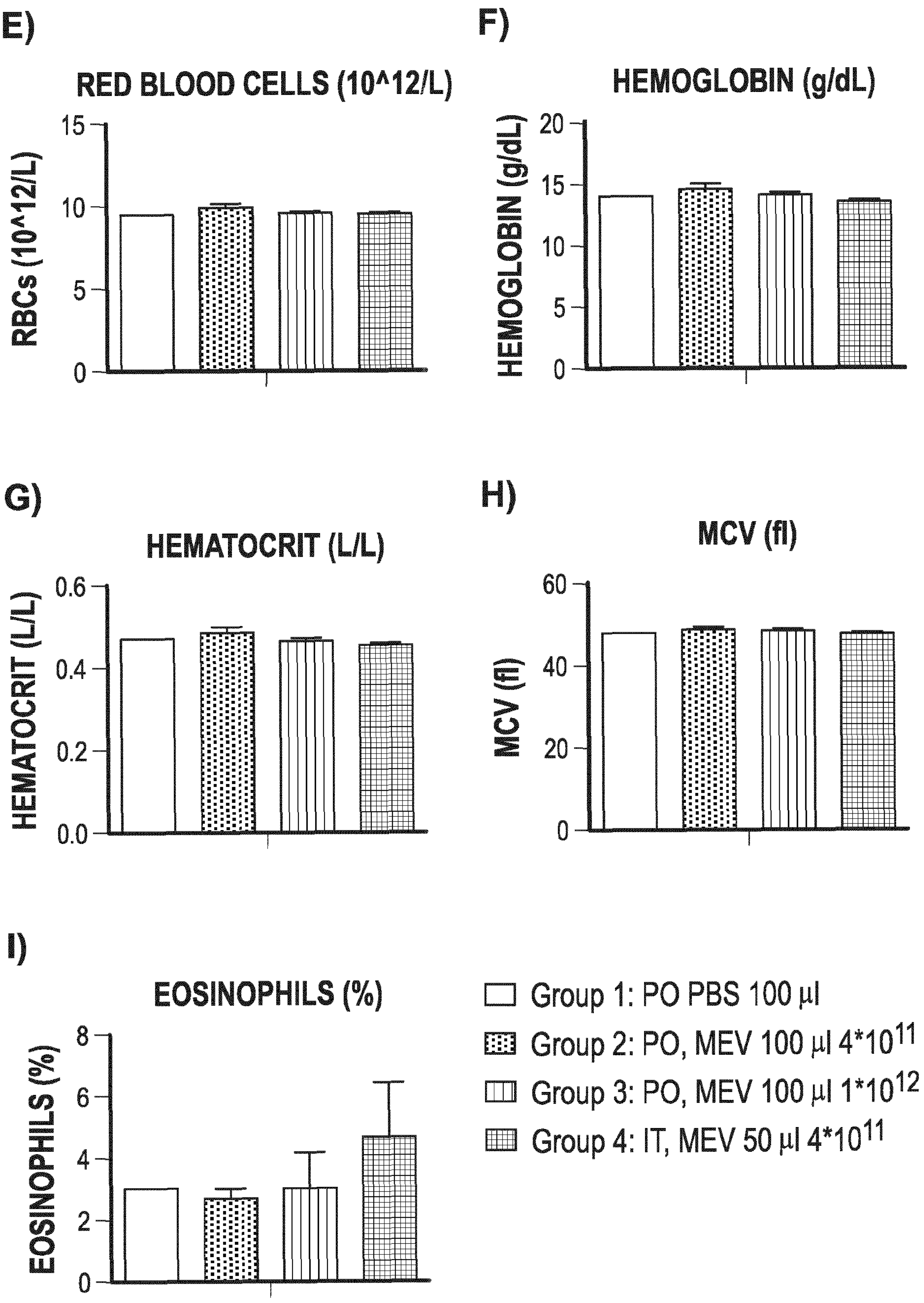
E)
RED BLOOD CELLS (10^12/L)
RBCs (10^12/L)
0
5
10
15
F)
HEMOGLOBIN (g/dL)
HEMOGLOBIN (g/dL)
0
5
10
15
20
G)
HEMATOCRIT (L/L)
HEMATOCRIT (L/L)
0.0
0.2
0.4
0.6
H)
MCV (fl)
MCV (fl)
0
20
40
60
I)
EOSINOPHILS (%)
EOSINOPHILS (%)
0
2
4
6
8
Group 1: PO PBS 100 µl
Group 2: PO, MEV 100 µl 4*10^11
Group 3: PO, MEV 100 µl 1*10^12
Group 4: IT, MEV 50 µl 4*10^11

FIG. 18 (Cont.)

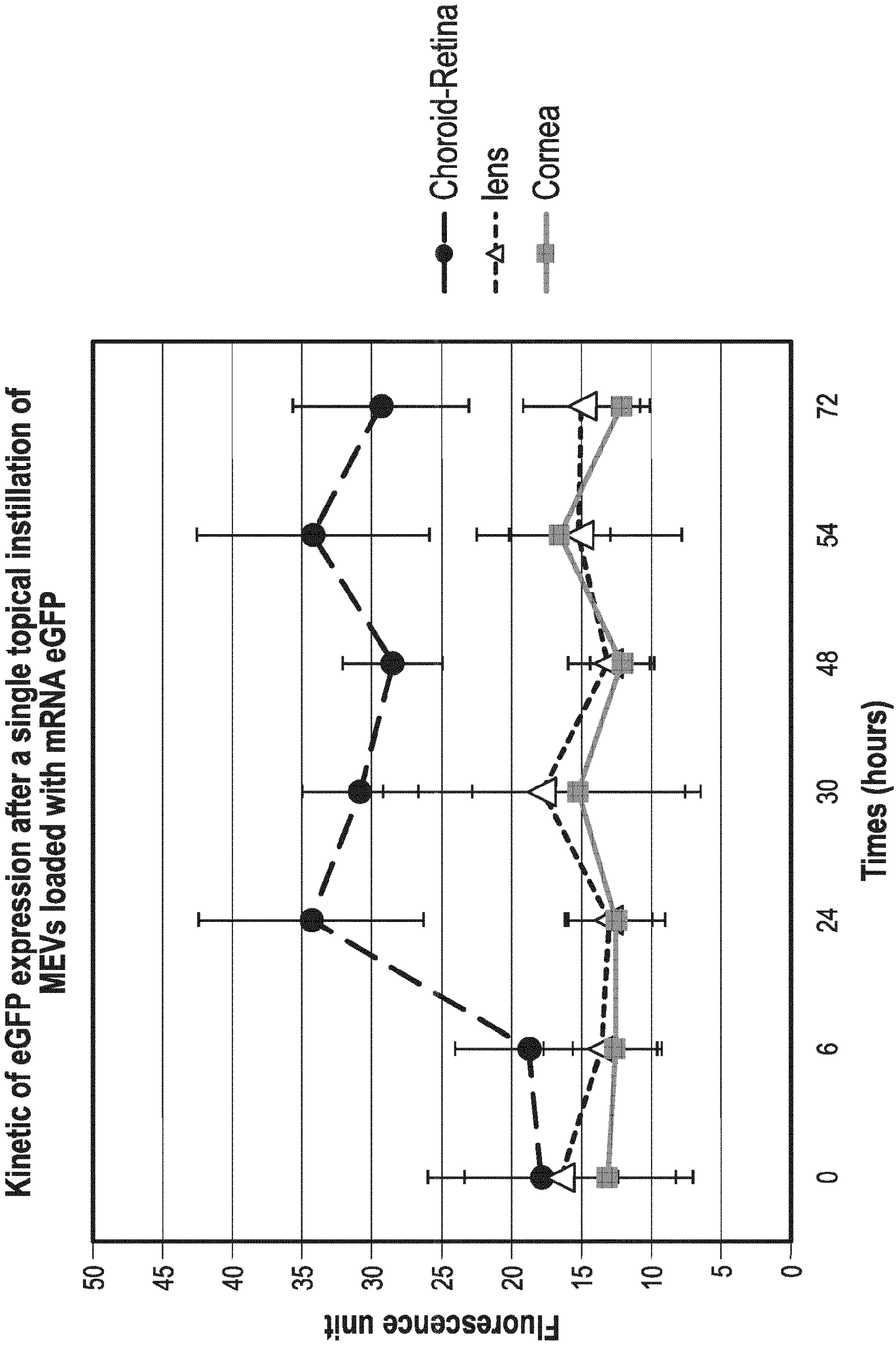
Kinetic of eGFP expression after a single topical instillation of MEVs loaded with mRNA eGFP
Fluorescence unit
0
5
10
15
20
25
30
35
40
45
50
0
6
24
30
48
54
72
Times (hours)
Choroid-Retina
lens
Cornea

FIG. 19

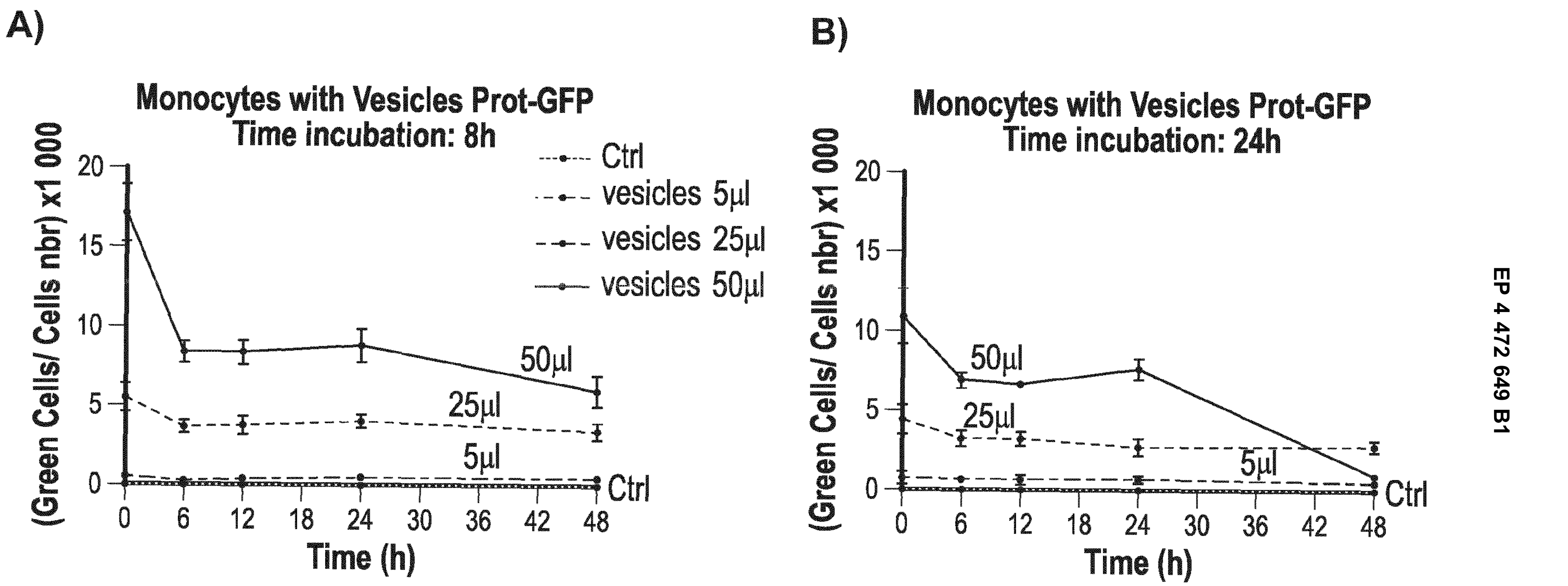
A)
Monocytes with Vesicles Prot-GFP
Time incubation: 8h
Ctrl
vesicles 5µl
vesicles 25µl
vesicles 50µl
50µl
25µl
5µl
Ctrl
(Green Cells/ Cells nbr) x1 000
20
15
10
5
0
0 6 12 18 24 30 36 42 48
Time (h)
B)
Monocytes with Vesicles Prot-GFP
Time incubation: 24h
50µl
25µl
5µl
Ctrl
(Green Cells/ Cells nbr) x1 000
20
15
10
5
0
0 6 12 18 24 30 36 42 48
Time (h)

FIG. 20

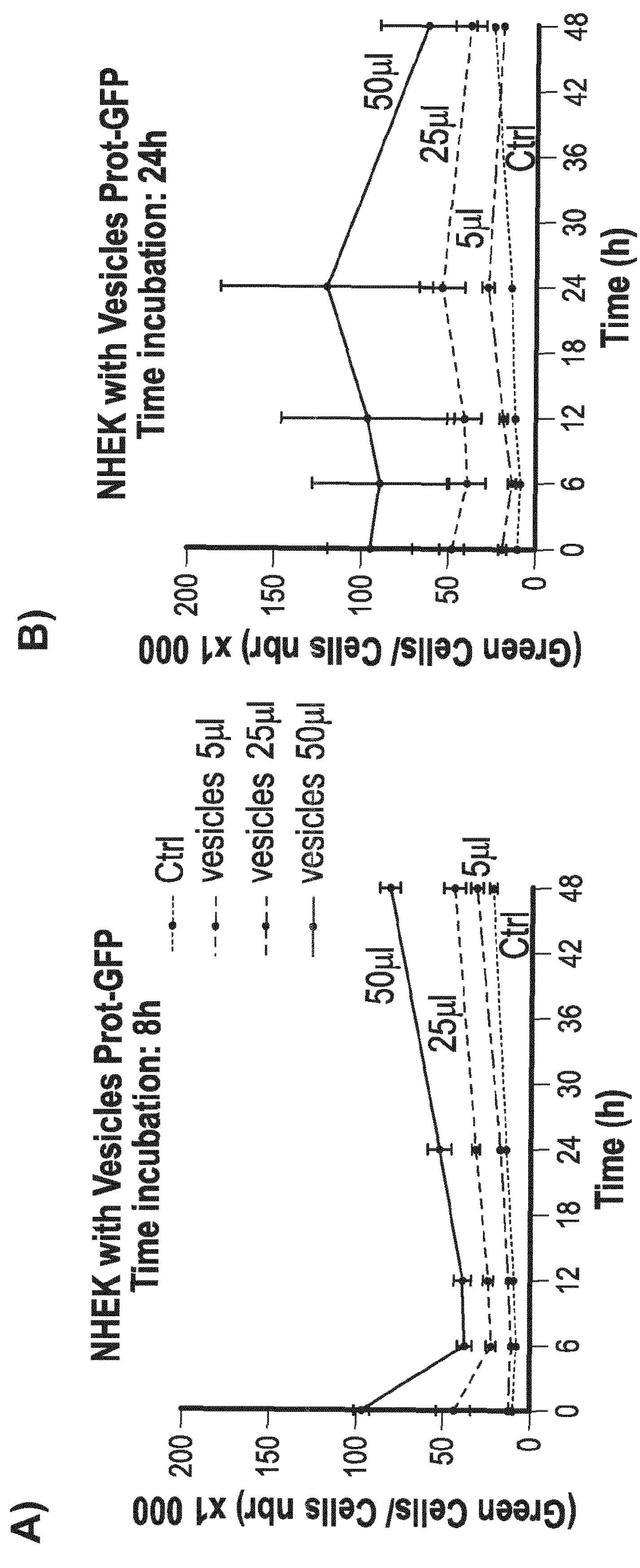
A)
NHEK with Vesicles Prot-GFP
Time incubation: 8h
Ctrl
vesicles 5µl
vesicles 25µl
vesicles 50µl
(Green Cells/ Cells nbr) x1 000
200
150
100
50
0
0
6
12
18
24
30
36
42
48
Time (h)
50µl
25µl
5µl
Ctrl
B)
NHEK with Vesicles Prot-GFP
Time incubation: 24h
(Green Cells/ Cells nbr) x1 000
200
150
100
50
0
0
6
12
18
24
30
36
42
48
Time (h)
50µl
25µl
5µl
Ctrl

FIG. 21

Hep-G2 cell line-24h

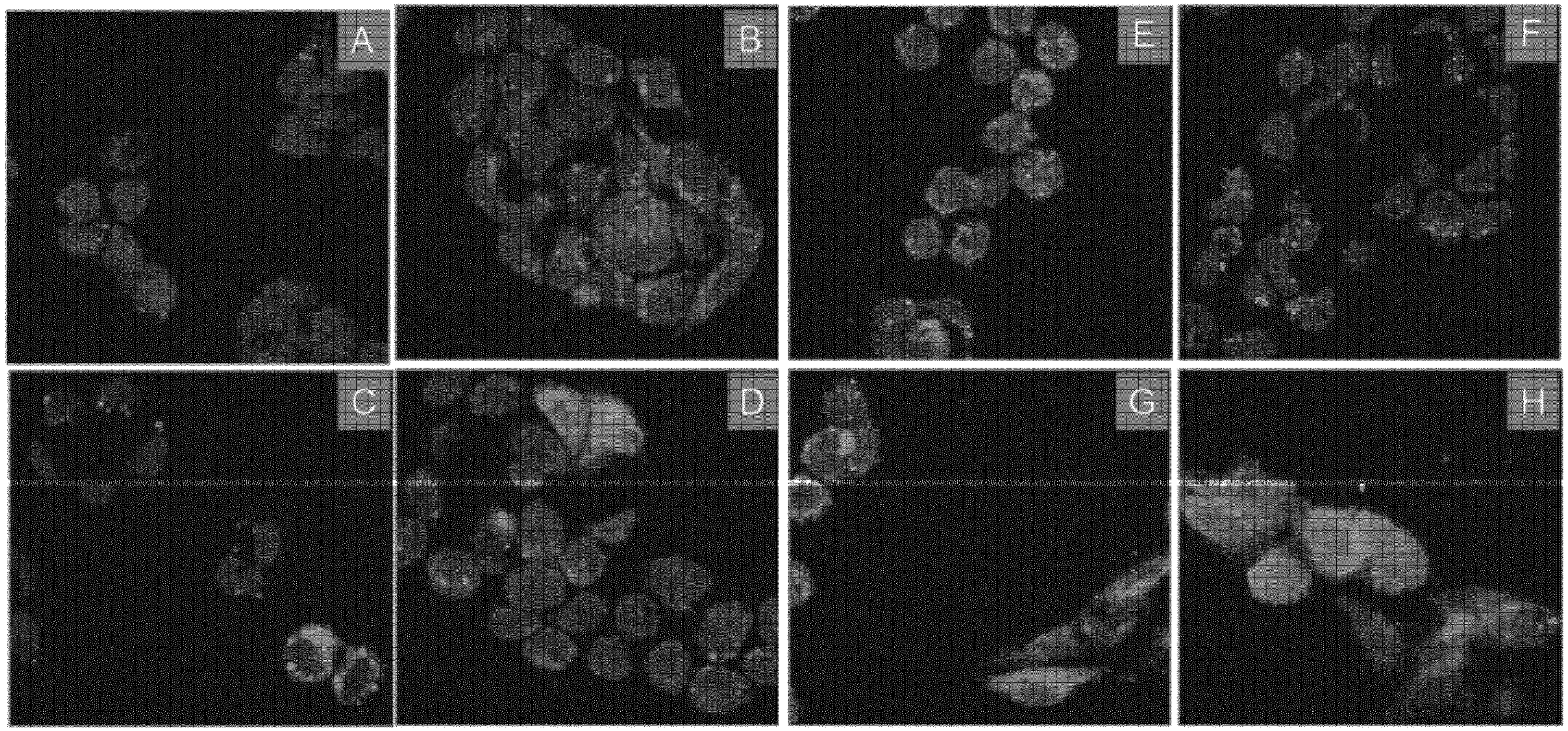


A- CTRL non loaded MEV

B- MEV-mRNA-eGFP
(200 loaded MEV per cell)

C- and D-MEV-mRNA-eGFP
(400 loaded MEV per cell)

E- MEV-GFP protein
(100 loaded MEV per cell)

F- MEV-GFP protein
(200 loaded MEV per cell)

G- and H-MEV-GFP protein
(400 loaded MEV per cell)

FIG. 22

Huh7 cell line-24h

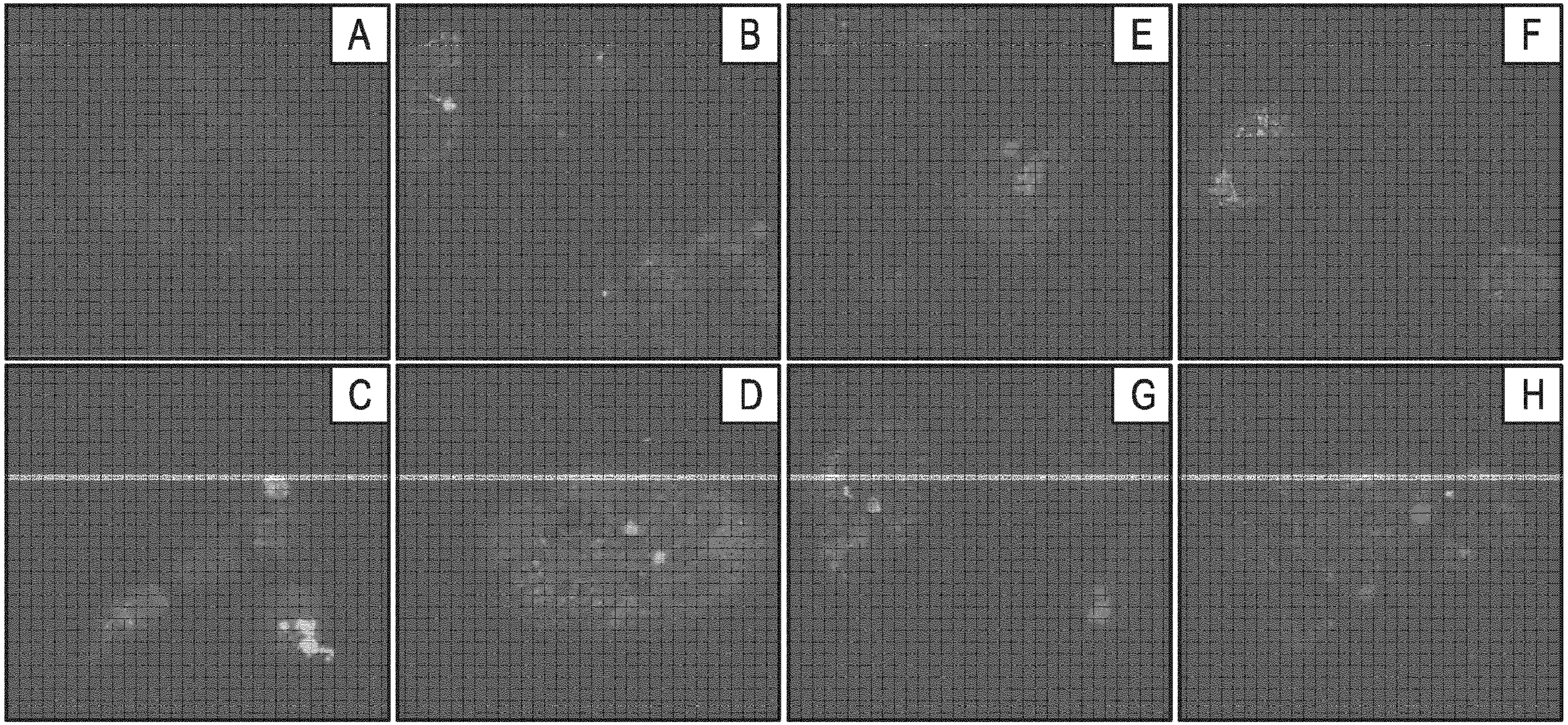


A- CTRL non loaded MEV

B- MEV-mRNA-eGFP
(200 loaded MEV per cell)

C- and D-MEV-mRNA-eGFP
(400 loaded MEV per cell)

E- MEV-GFP protein
(100 loaded MEV per cell)

F- MEV-GFP protein
(200 loaded MEV per cell)

G- and H-MEV-GFP protein
(400 loaded MEV per cell)

FIG. 23

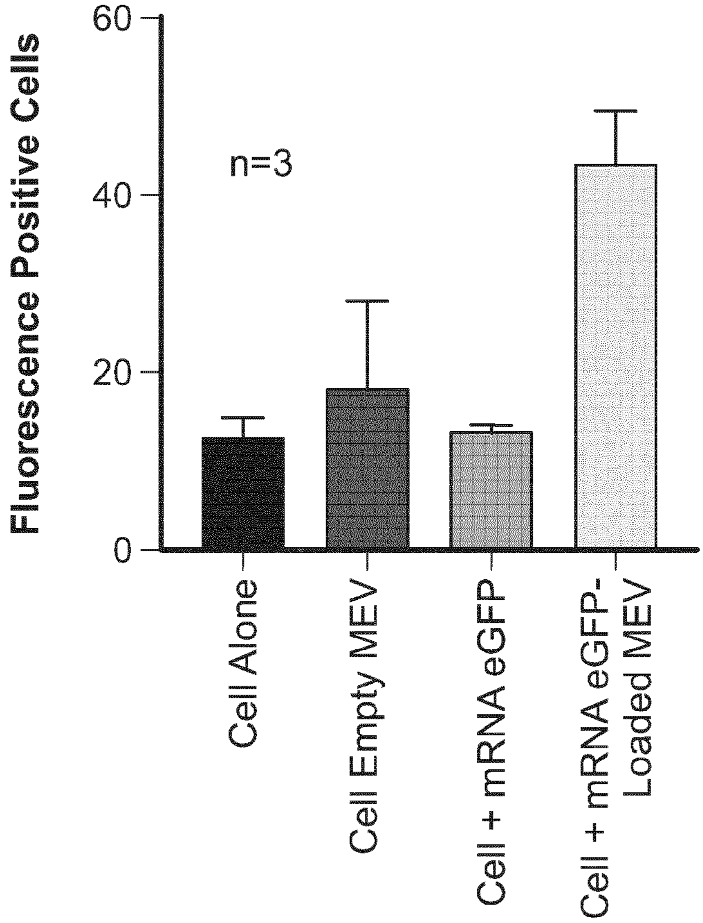
Fluorescence Positive Cells
60
40
20
0
n=3
Cell Alone
Cell Empty MEV
Cell + mRNA eGFP
Cell + mRNA eGFP-Loaded MEV

**FIG. 24**

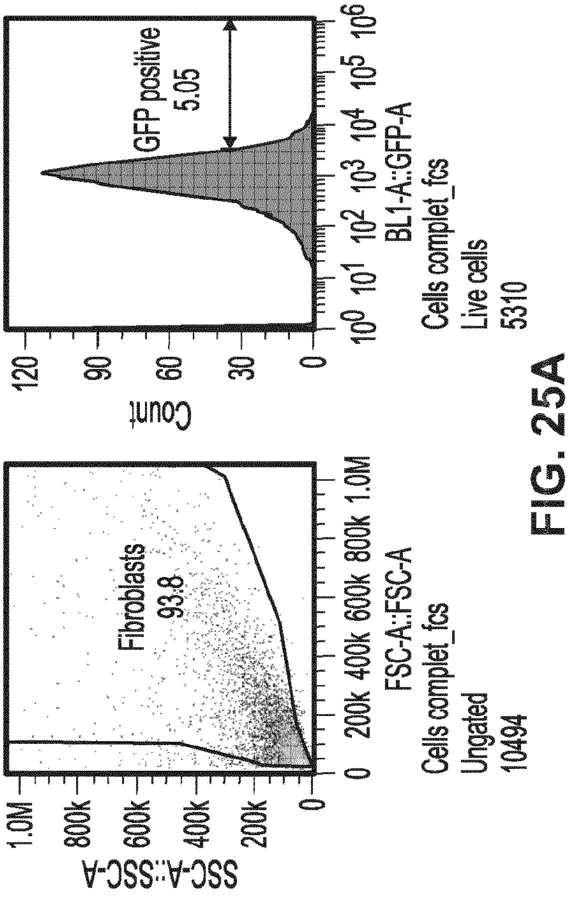


FIG. 25A

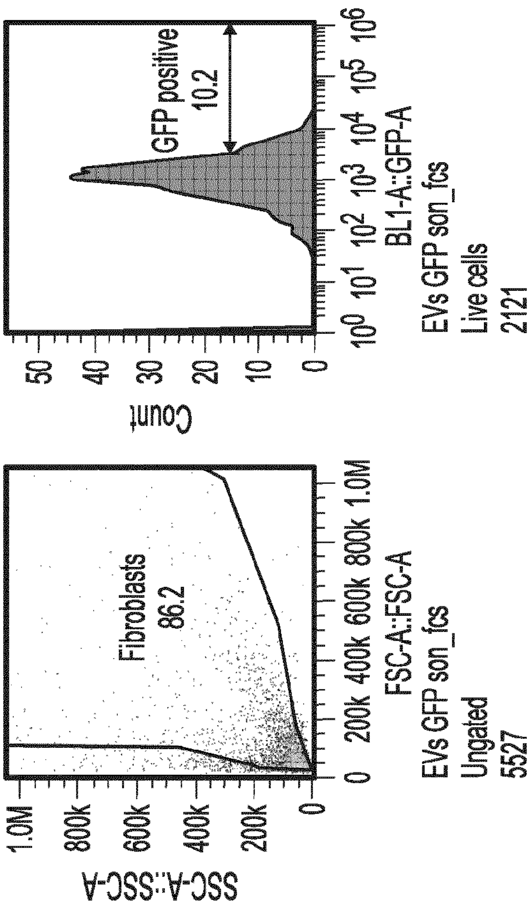


FIG. 25B

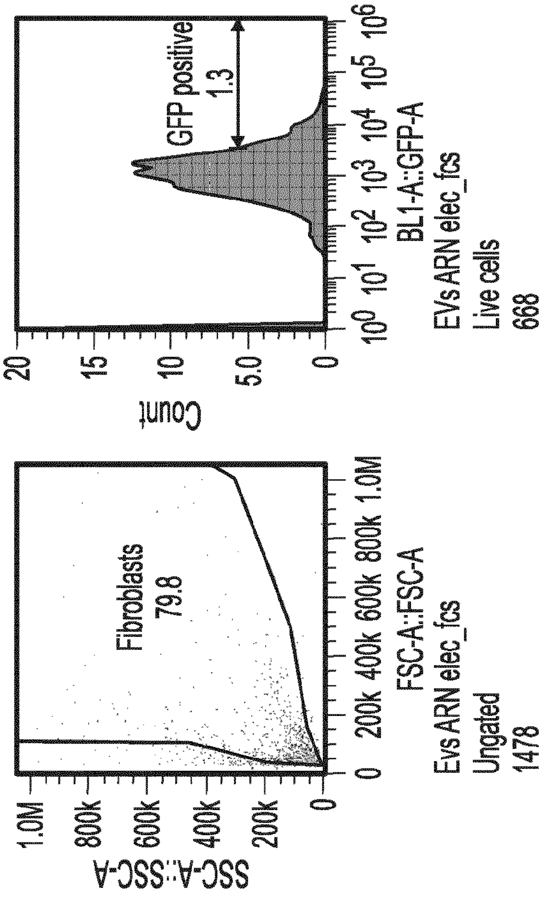


FIG. 25C

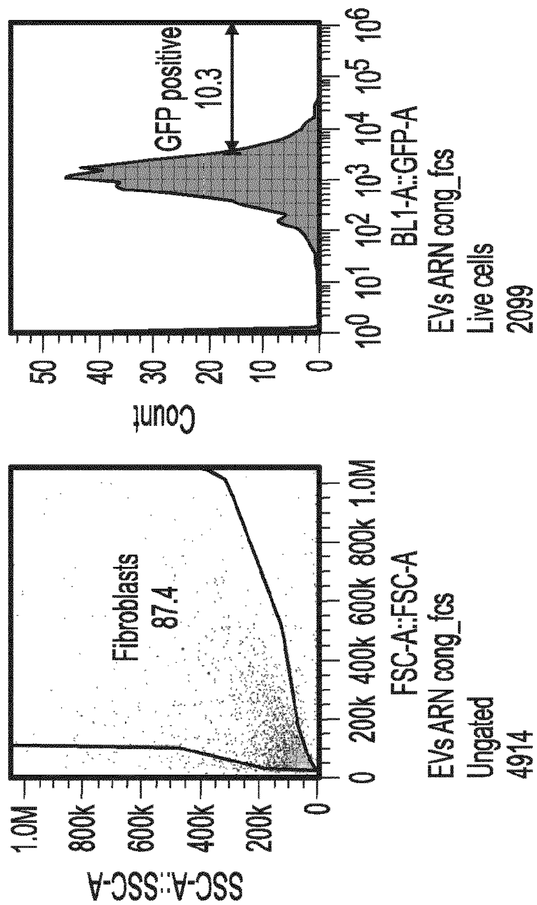


FIG. 25D

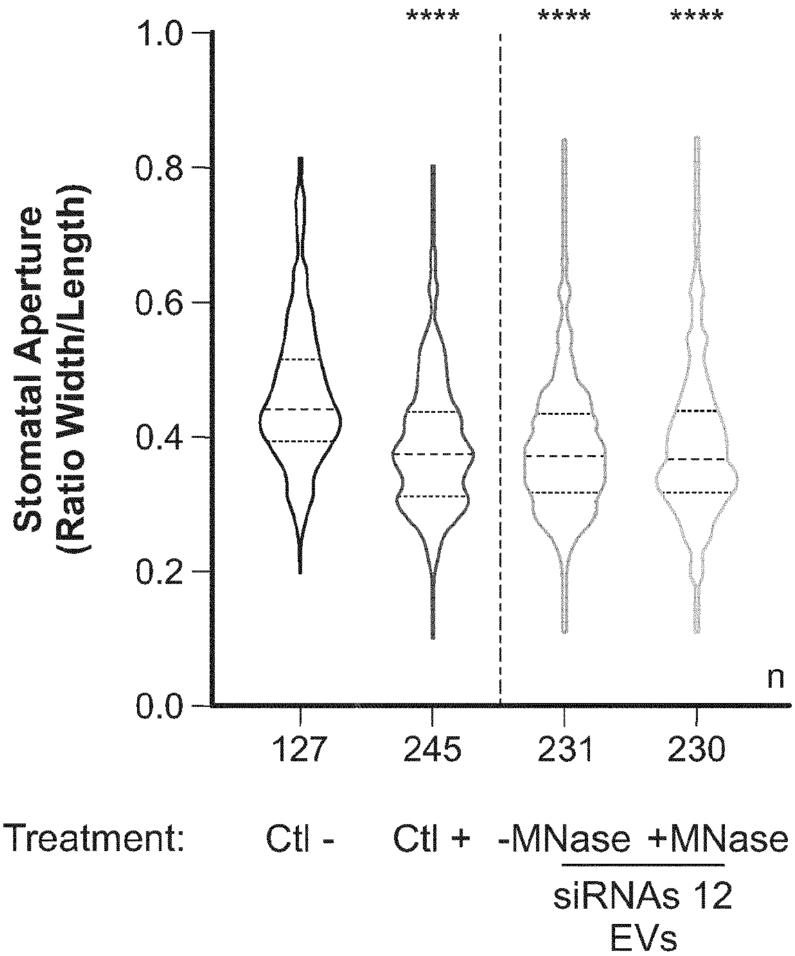
Stomatal Aperture (Ratio Width/Length)
1.0
0.8
0.6
0.4
0.2
0.0
****
****
****
n
127
245
231
230
Treatment:
Ctl -
Ctl +
-MNase
+MNase
siRNAs 12 EVs

FIG. 26

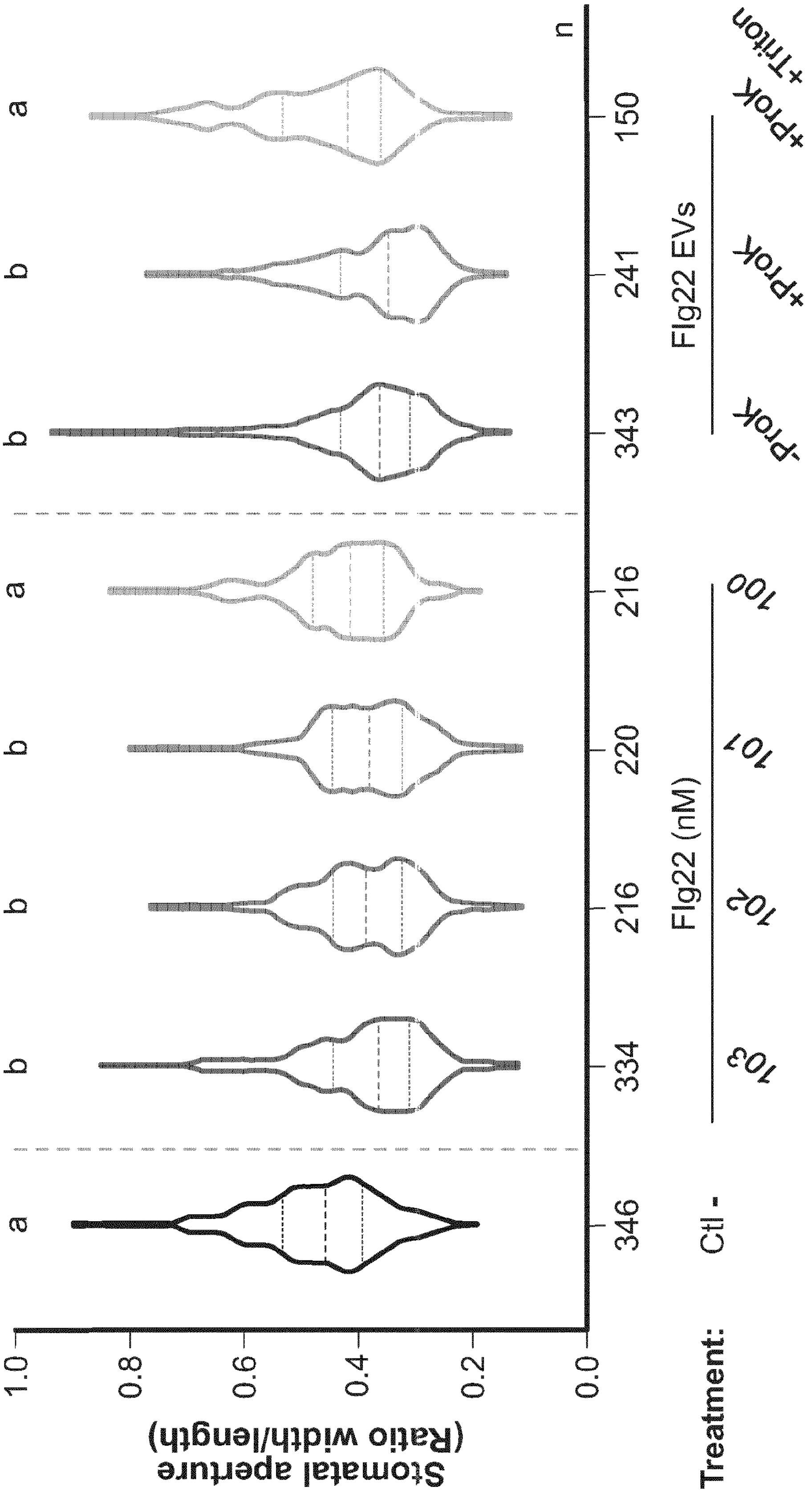
Stomatal aperture (Ratio width/length)
1.0
0.8
0.6
0.4
0.2
0.0
a
b
b
b
a
b
b
a
n
346
334
216
220
216
343
241
150
Treatment:
Ctl -
Flg22 (nM)
$10^3$
$10^2$
$10^1$
$10^0$
Flg22 EVs
-ProK
+ProK
+ProK +Triton

**FIG. 27**

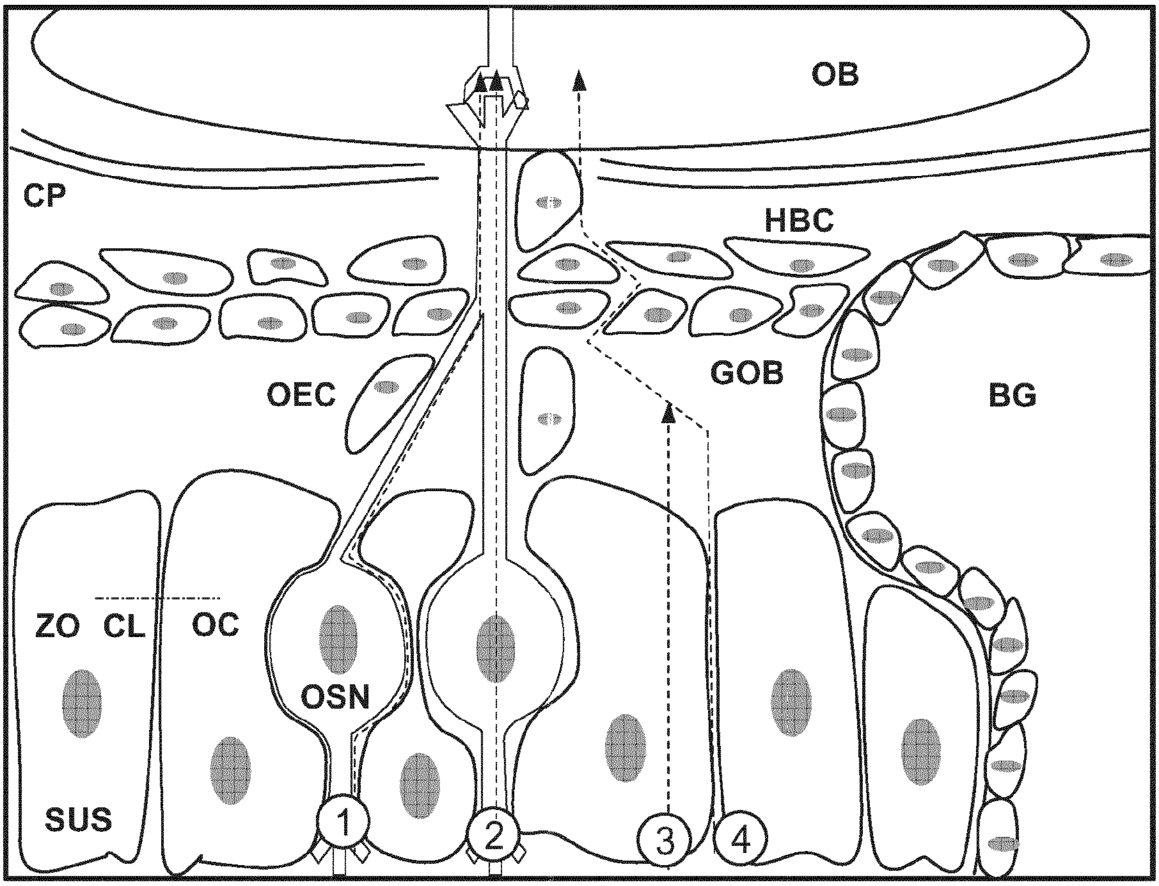


FIG. 28

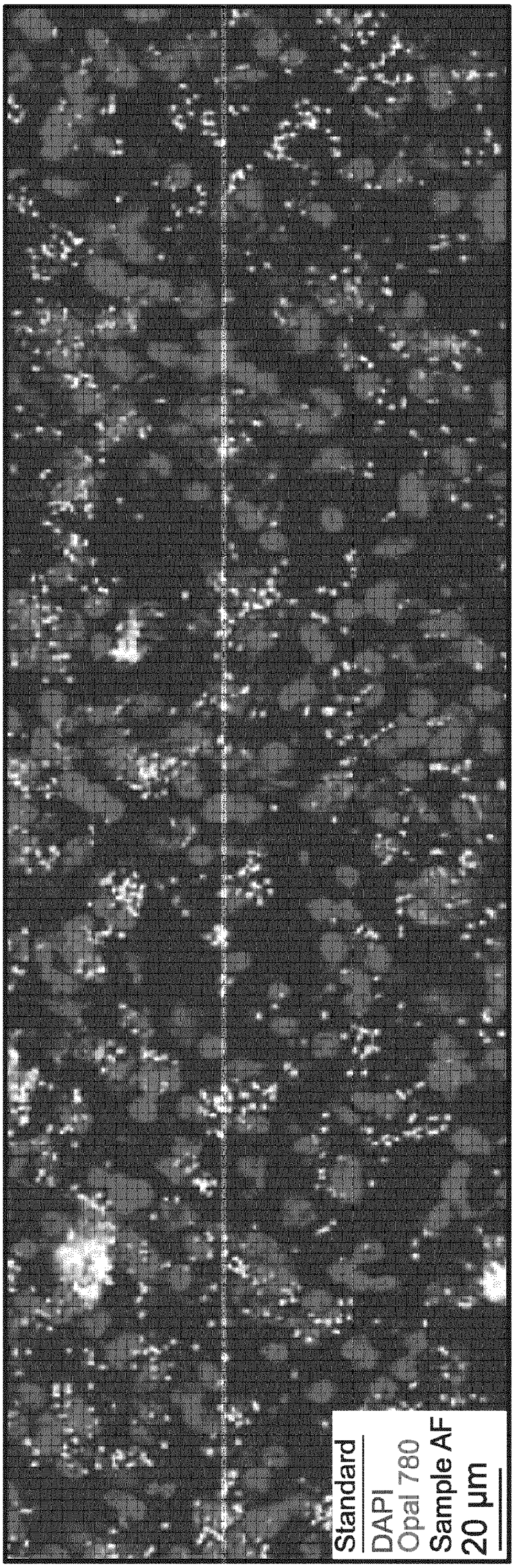


FIG. 29

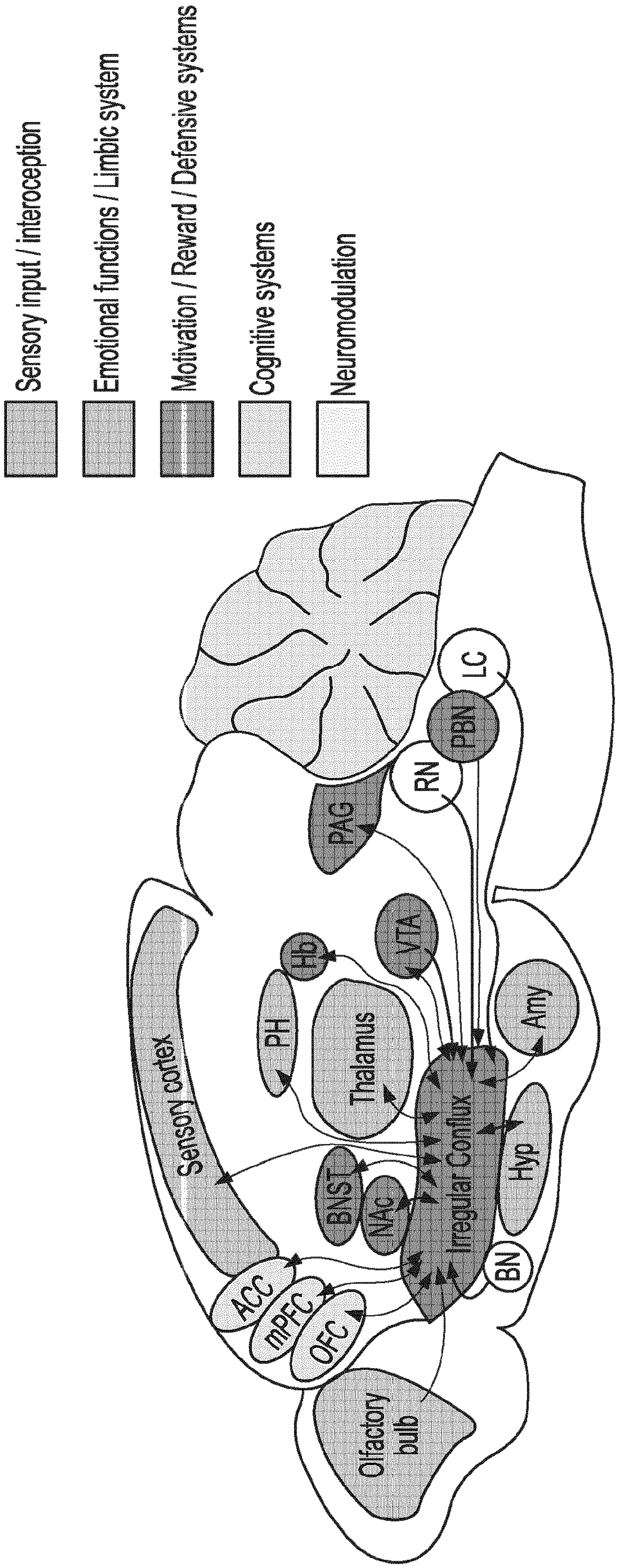
Sensory input / interoception
Emotional functions / Limbic system
Motivation / Reward / Defensive systems
Cognitive systems
Neuromodulation
Sensory cortex
PH
Hb
Thalamus
VTA
PAG
RN
PBN
LC
Amy
Irregular Conflux
Hyp
BNST
NAc
BN
ACC
mPFC
OFC
Olfactory bulb

FIG. 30

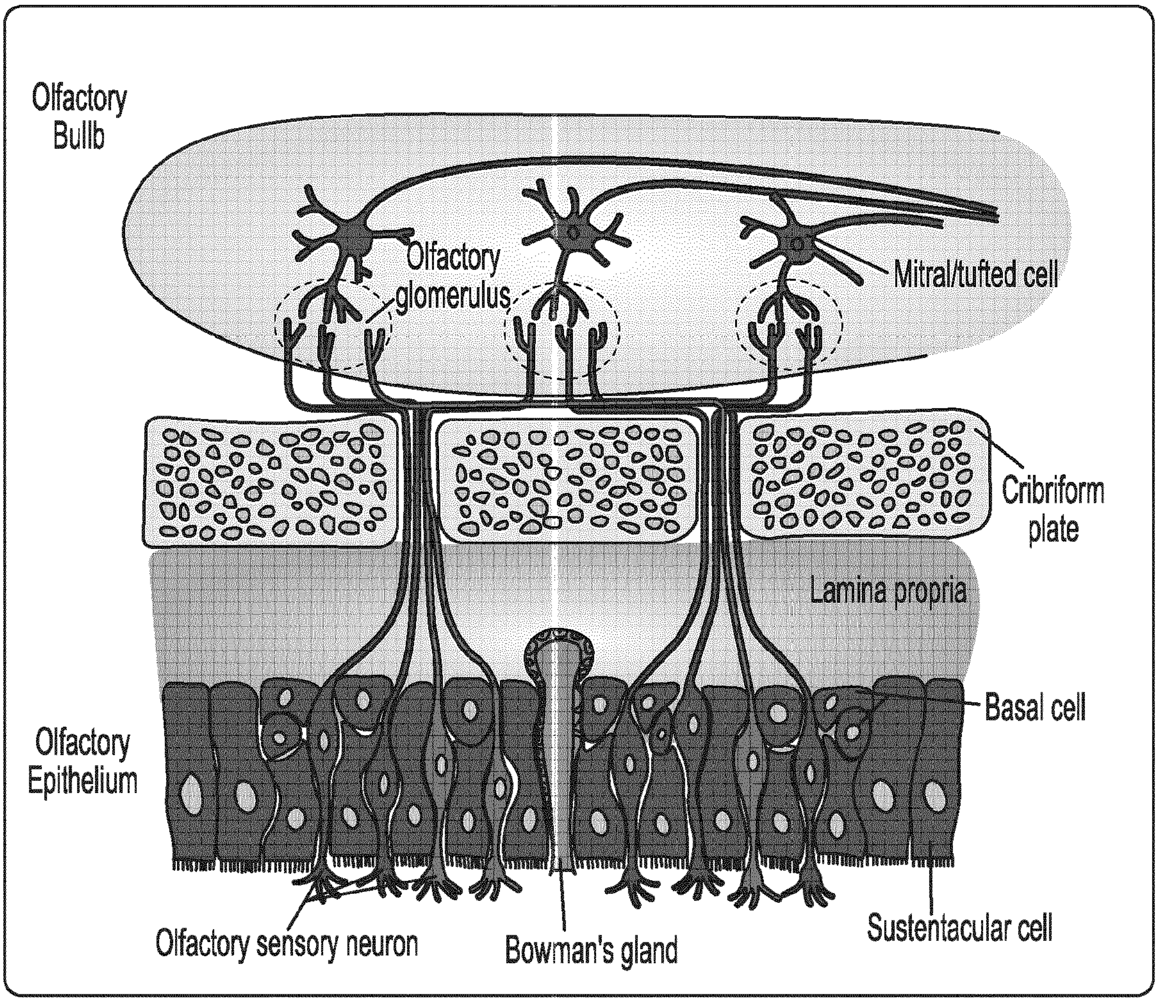
Olfactory
Bullb
Olfactory
glomerulus
Mitral/tufted cell
Cribriform
plate
Lamina propria
Basal cell
Olfactory
Epithelium
Olfactory sensory neuron
Bowman's gland
Sustentacular cell

FIG. 31

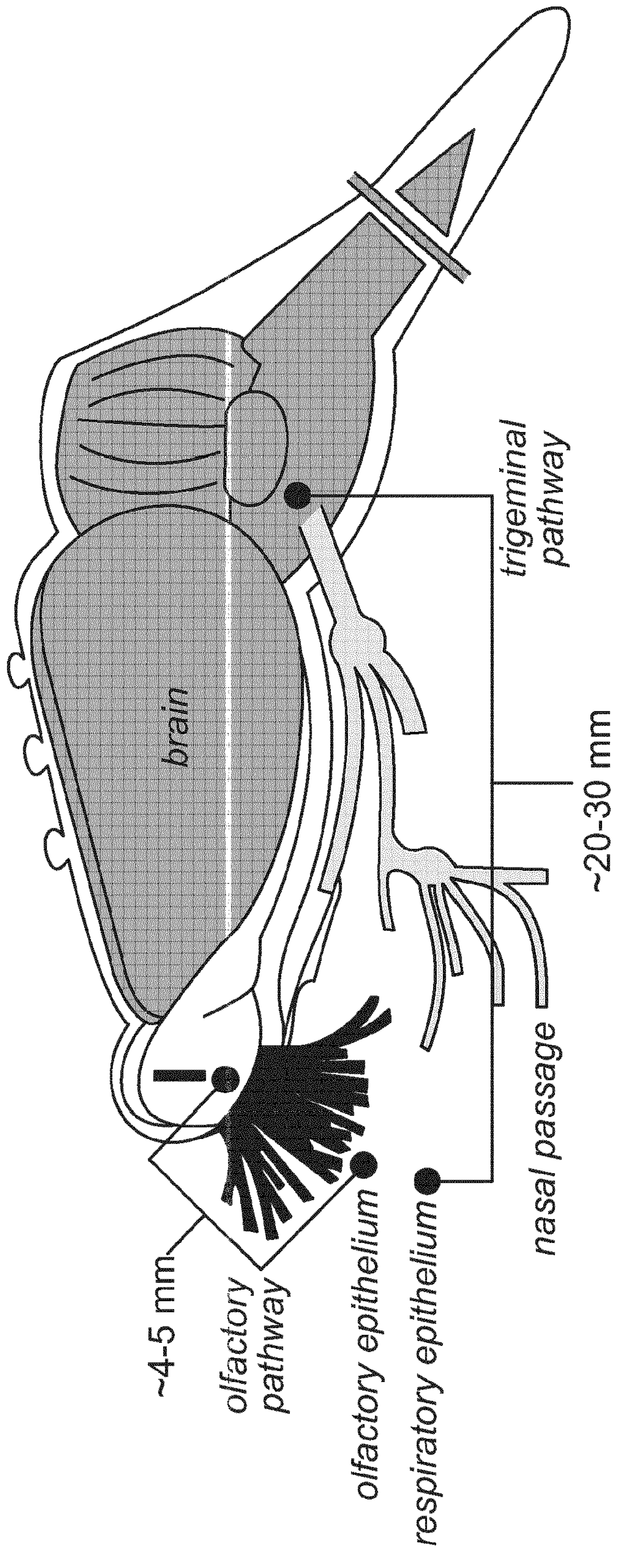
brain
trigeminal pathway
~20-30 mm
nasal passage
respiratory epithelium
olfactory epithelium
olfactory pathway
~4-5 mm

FIG. 32

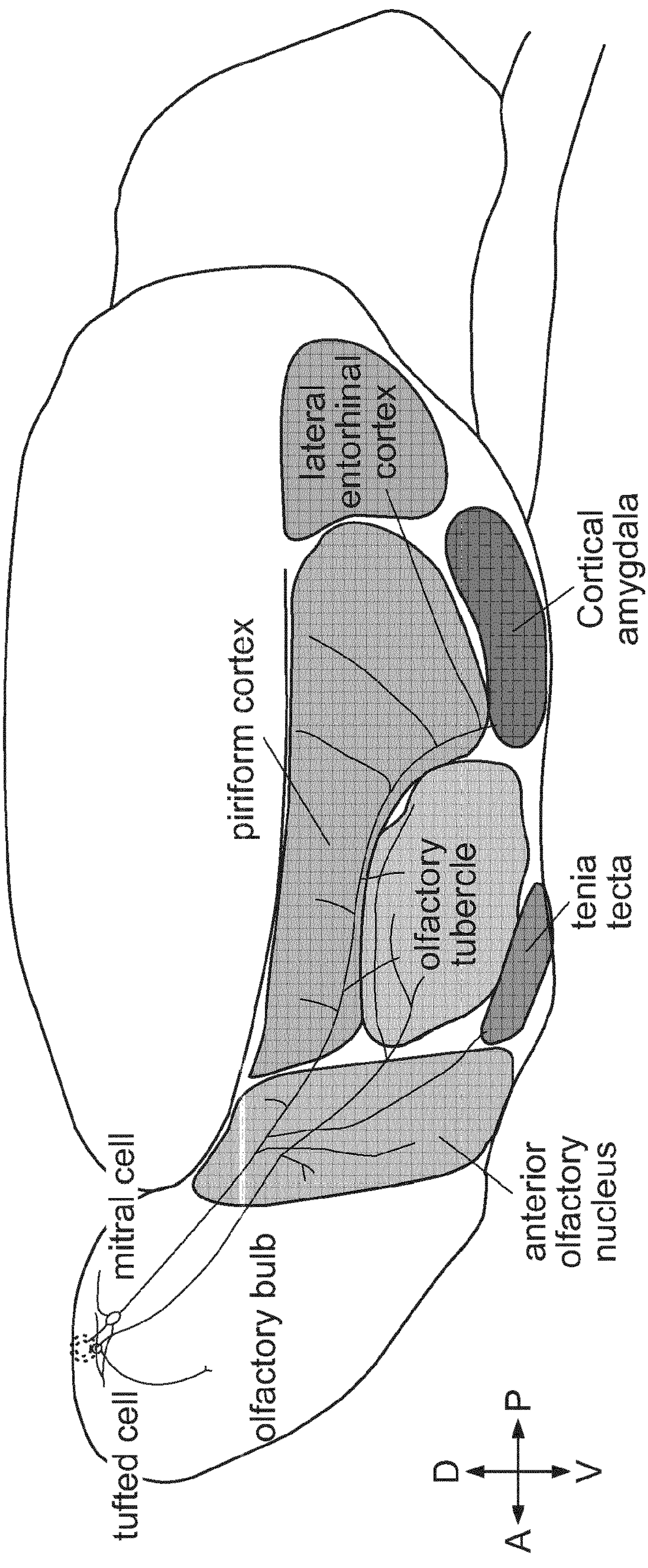
lateral entorhinal cortex
Cortical amygdala
piriform cortex
olfactory tubercle
tenia tecta
mitral cell
anterior olfactory nucleus
olfactory bulb
tufted cell
D
P
A
V

FIG. 33

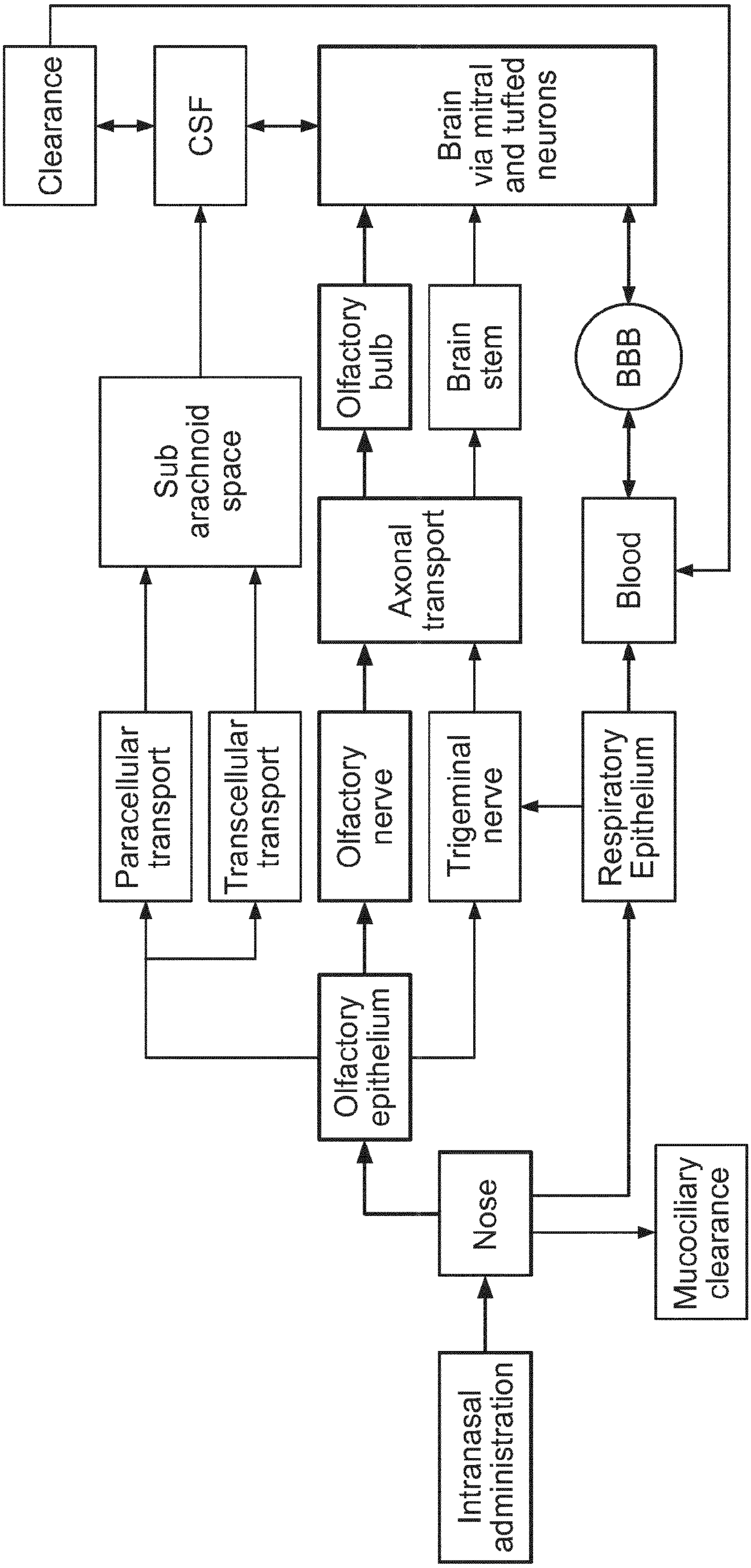
Intranasal administration
Nose
Mucociliary clearance
Olfactory epithelium
Paracellular transport
Transcellular transport
Olfactory nerve
Trigeminal nerve
Respiratory Epithelium
Sub arachnoid space
Axonal transport
Blood
Olfactory bulb
Brain stem
BBB
CSF
Brain via mitral and tufted neurons
Clearance

FIG. 34

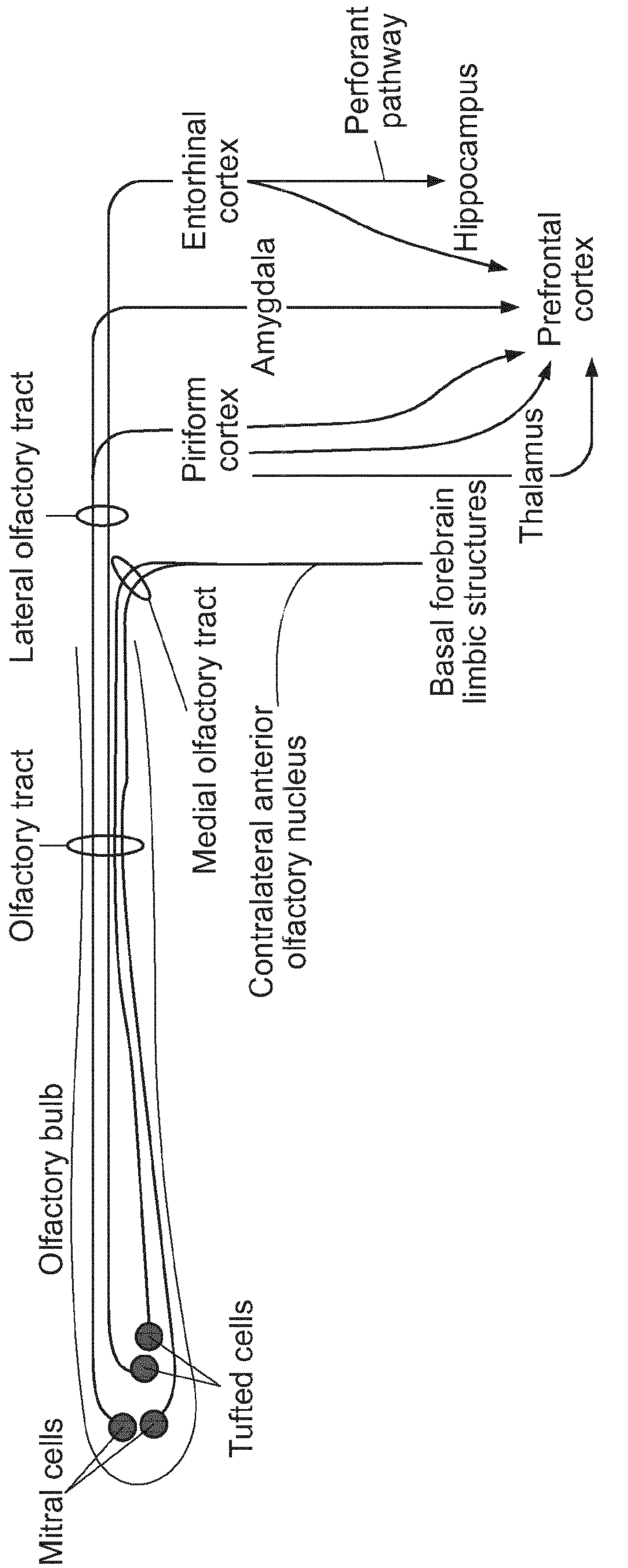
Perforant pathway
Hippocampus
Entorhinal cortex
Amygdala
Prefrontal cortex
Piriform cortex
Thalamus
Lateral olfactory tract
Basal forebrain limbic structures
Medial olfactory tract
Contralateral anterior olfactory nucleus
Olfactory tract
Olfactory bulb
Tufted cells
Mitral cells

FIG. 35

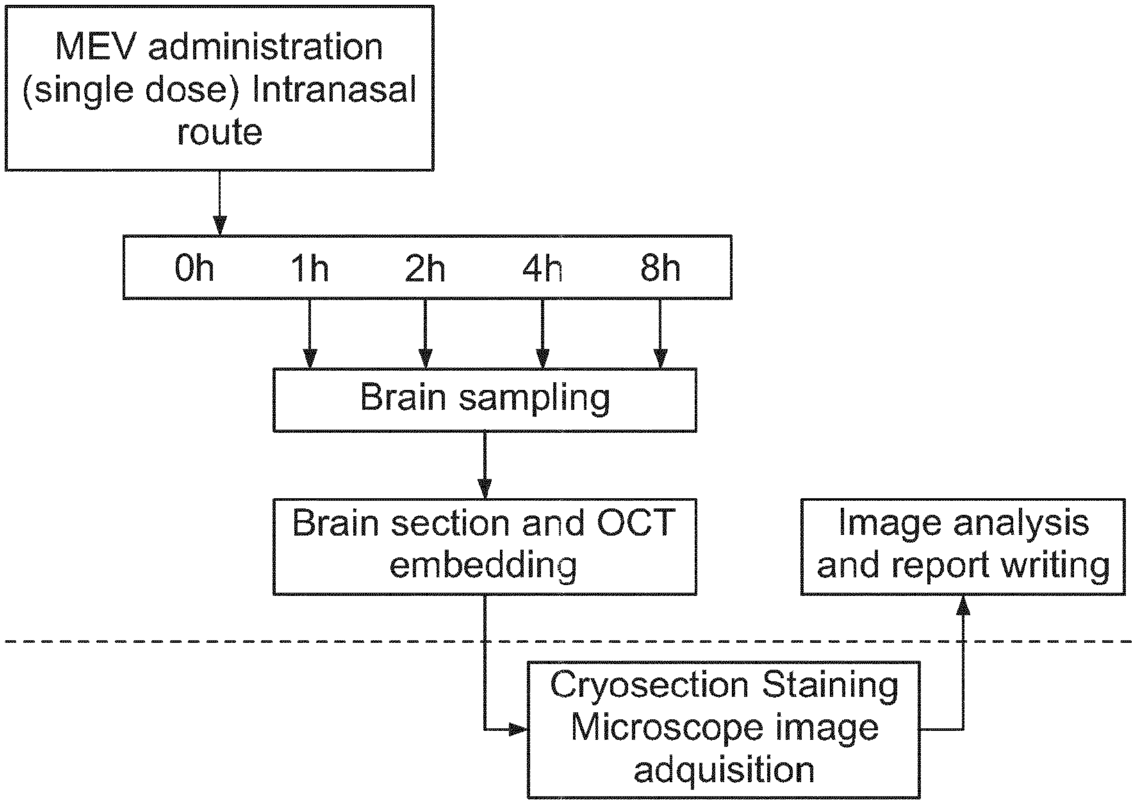


FIG. 36A

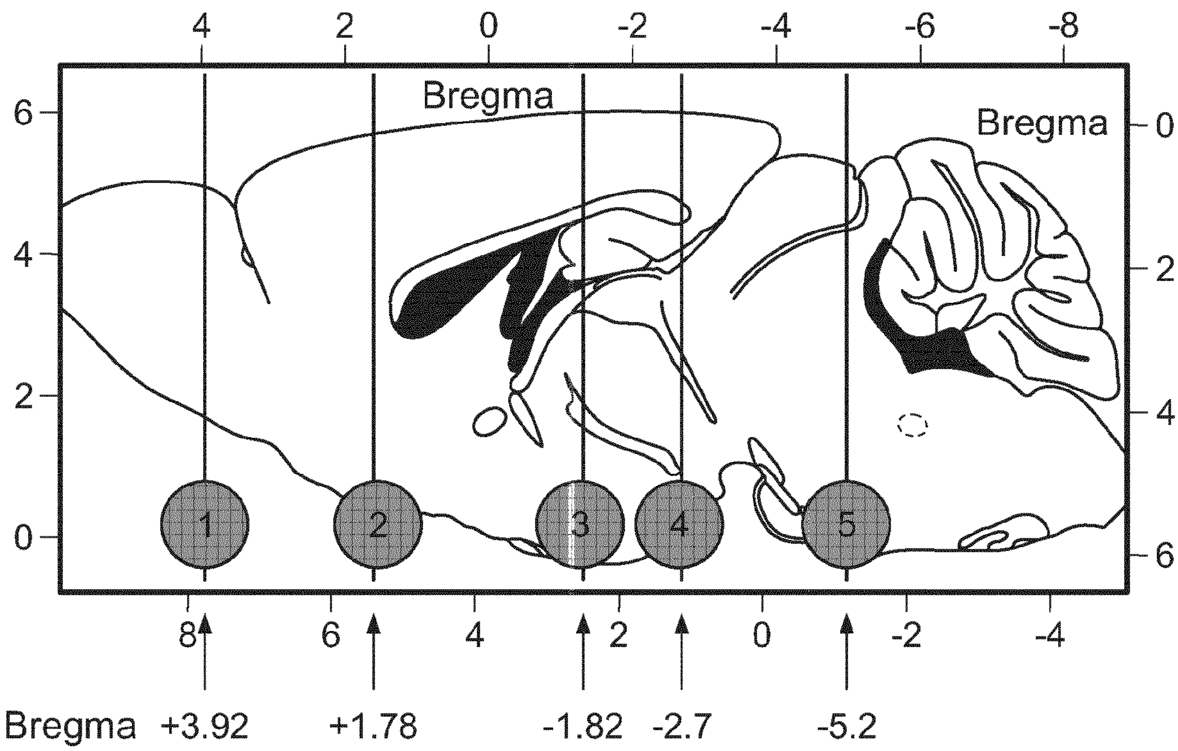


FIG. 36B

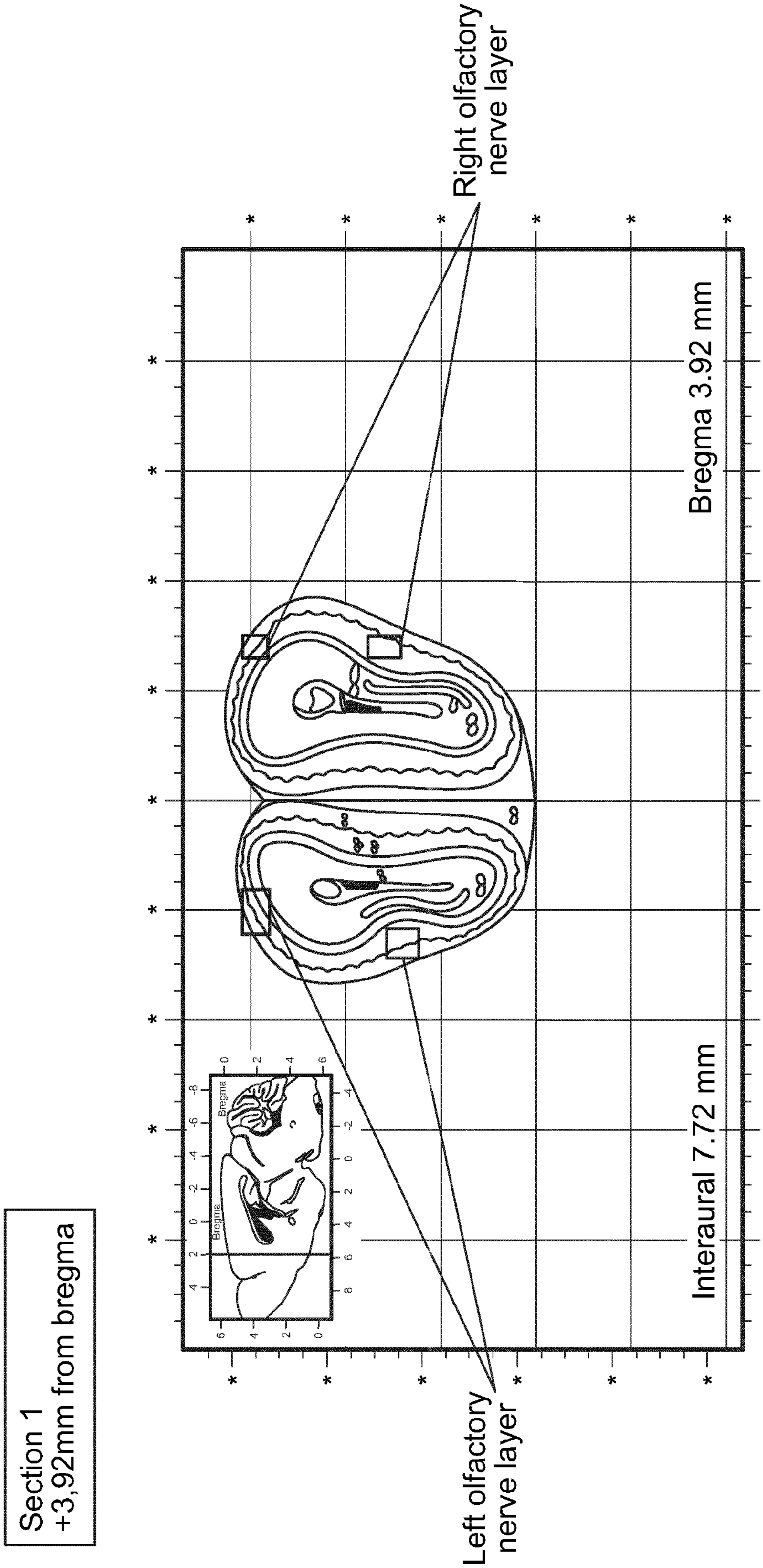
Section 1
+3,92mm from bregma
Right olfactory nerve layer
Left olfactory nerve layer
Bregma 3.92 mm
Interaural 7.72 mm
Bregma

FIG. 36C

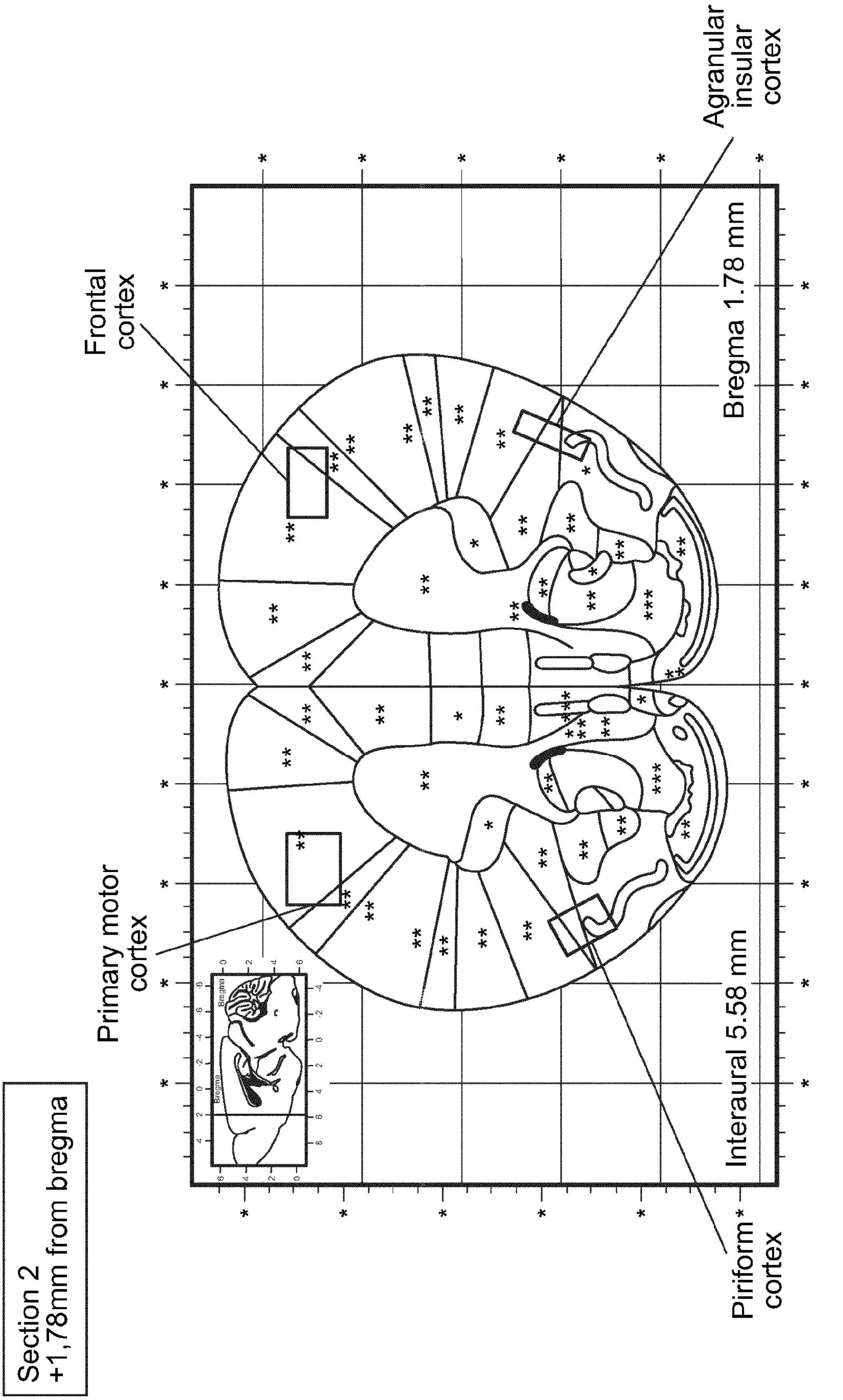
Section 2
+1,78mm from bregma
Frontal cortex
Agranular insular cortex
Primary motor cortex
Piriform cortex
Bregma 1.78 mm
Interaural 5.58 mm

FIG. 36D

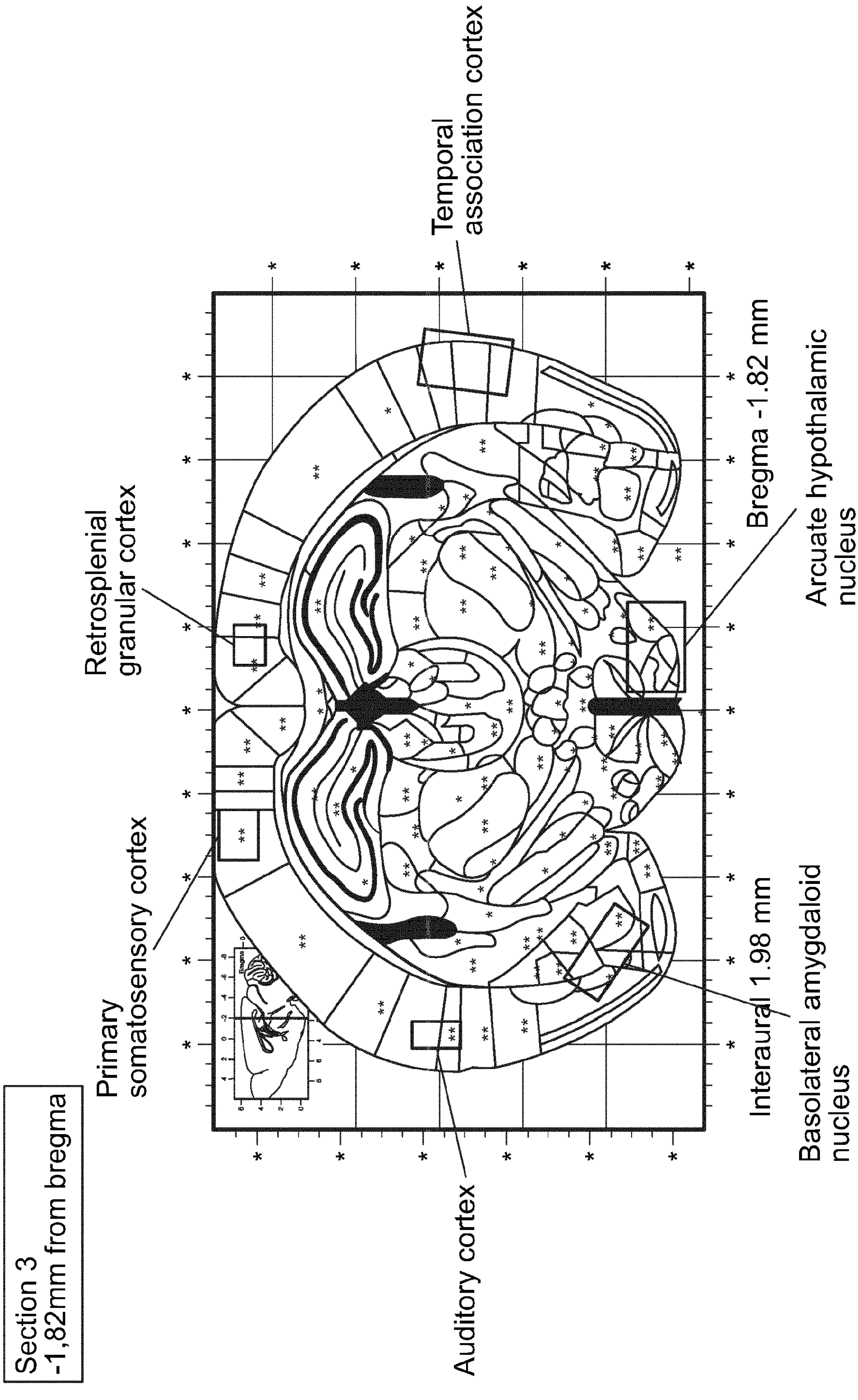
Section 3
-1,82mm from bregma
Primary
somatosensory cortex
Retrosplenial
granular cortex
Auditory cortex
Temporal
association cortex
Interaural 1.98 mm
Bregma -1.82 mm
Basolateral amygdaloid
nucleus
Arcuate hypothalamic
nucleus

FIG. 36E

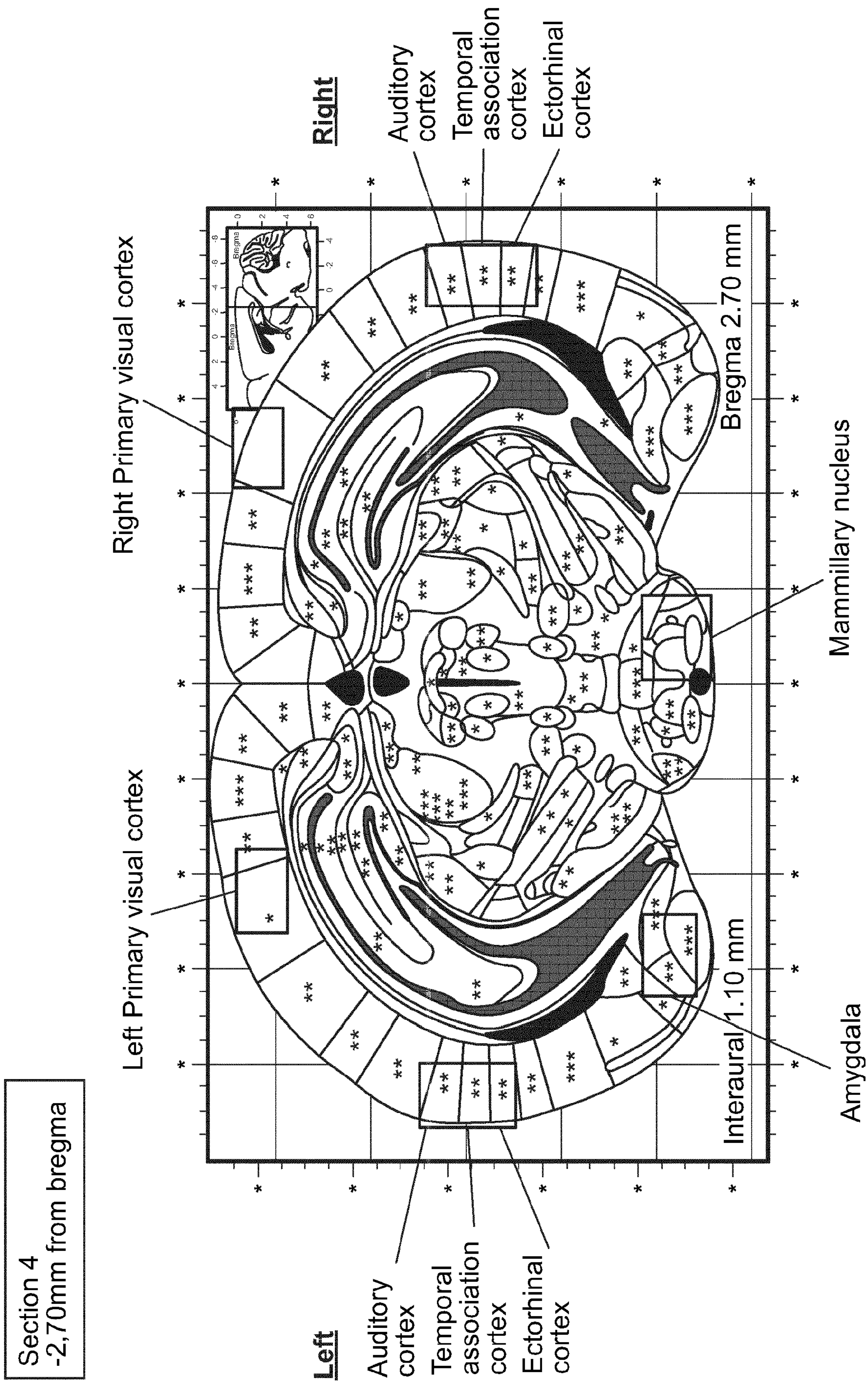
Section 4
-2,70mm from bregma
Left
Auditory cortex
Temporal association cortex
Ectorhinal cortex
Right
Auditory cortex
Temporal association cortex
Ectorhinal cortex
Right Primary visual cortex
Left Primary visual cortex
Mammillary nucleus
Amygdala
Bregma 2.70 mm
Interaural 1.10 mm

FIG. 36F

Section 5
-5,20mm from bregma

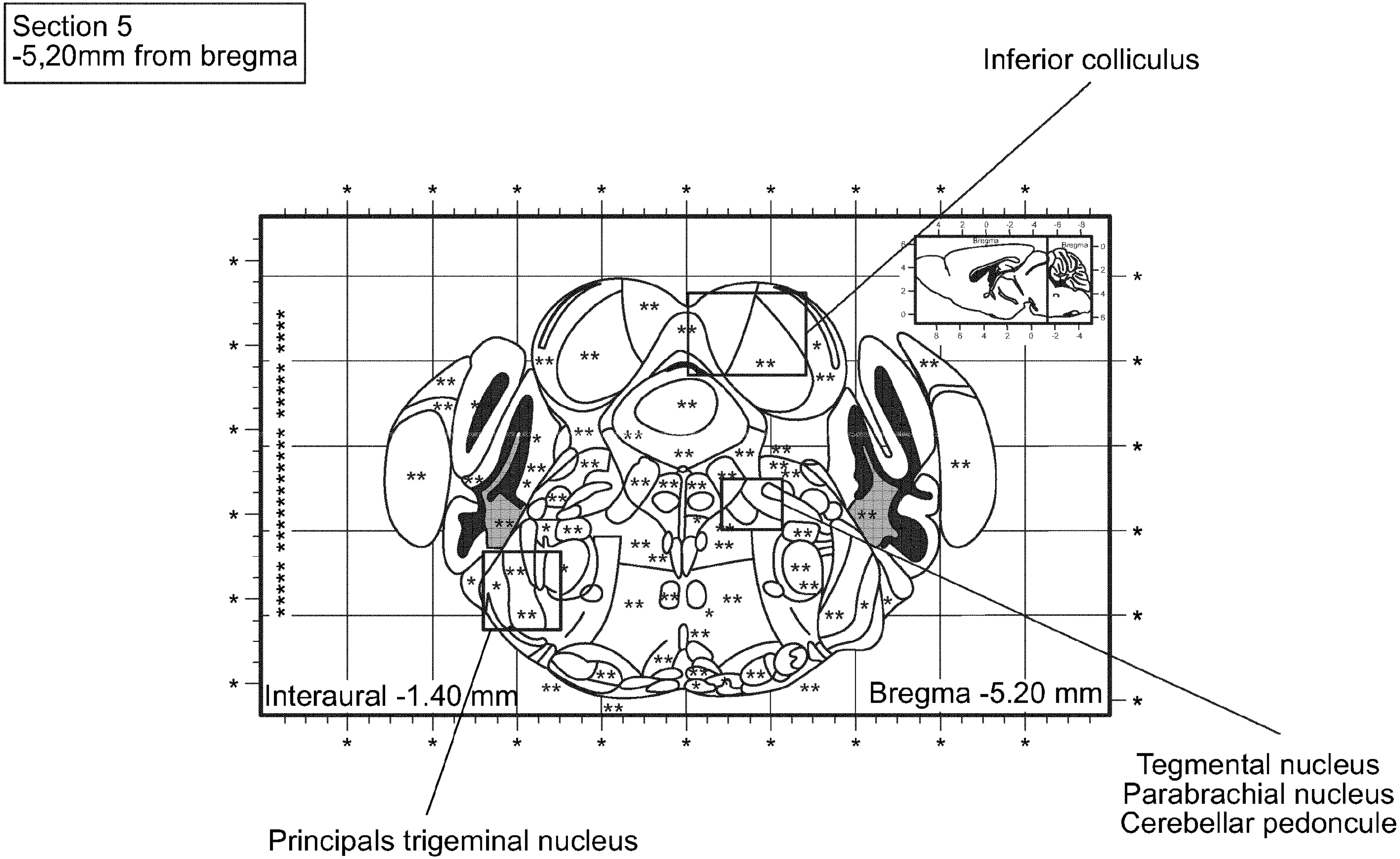


FIG. 36G

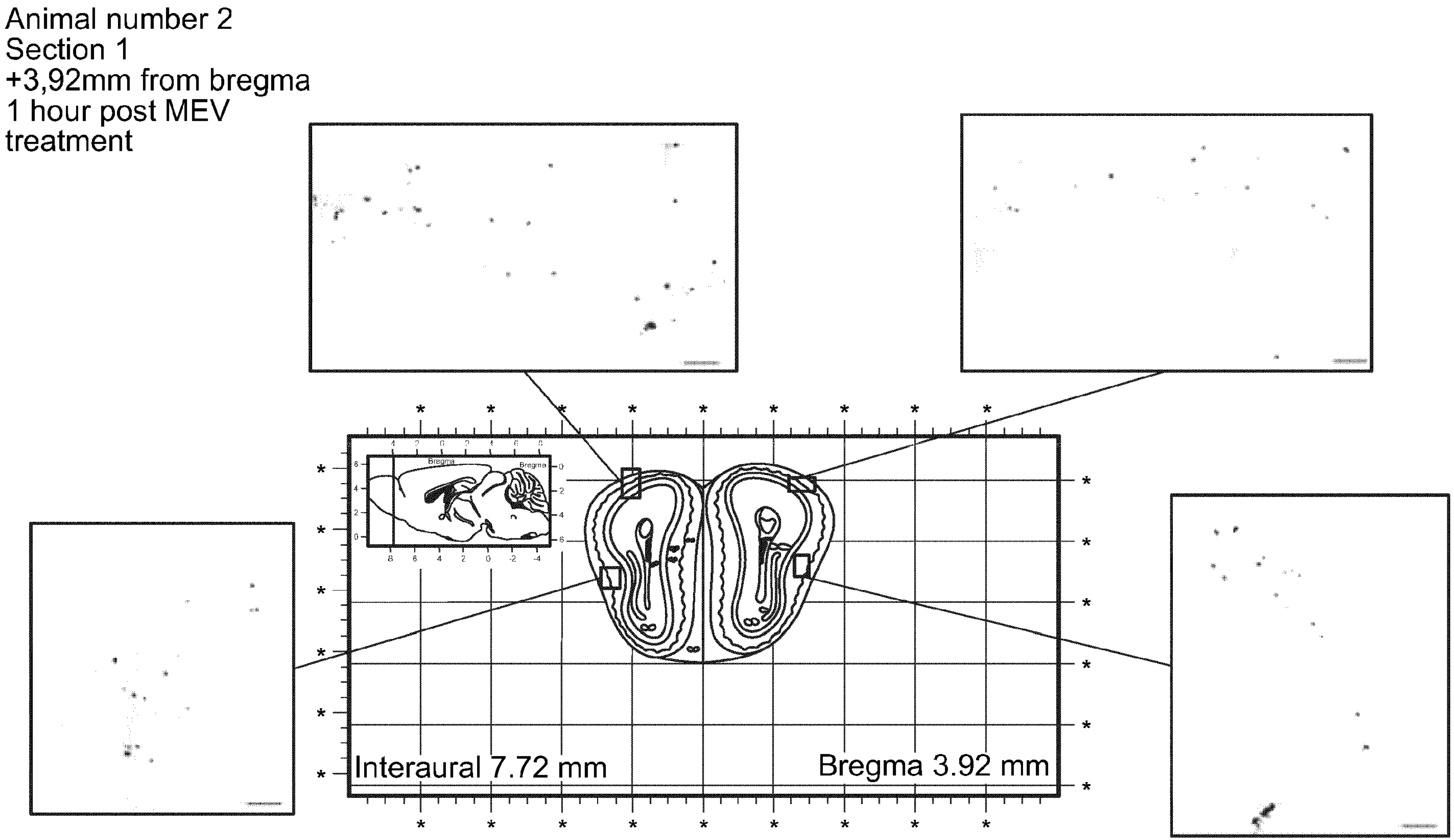
Animal number 2
Section 1
+3,92mm from bregma
1 hour post MEV
treatment
Interaural 7.72 mm
Bregma 3.92 mm

FIG. 37A

Animal number 4
Section 1
+3,92mm from bregma
2 hours post MEV
treatment

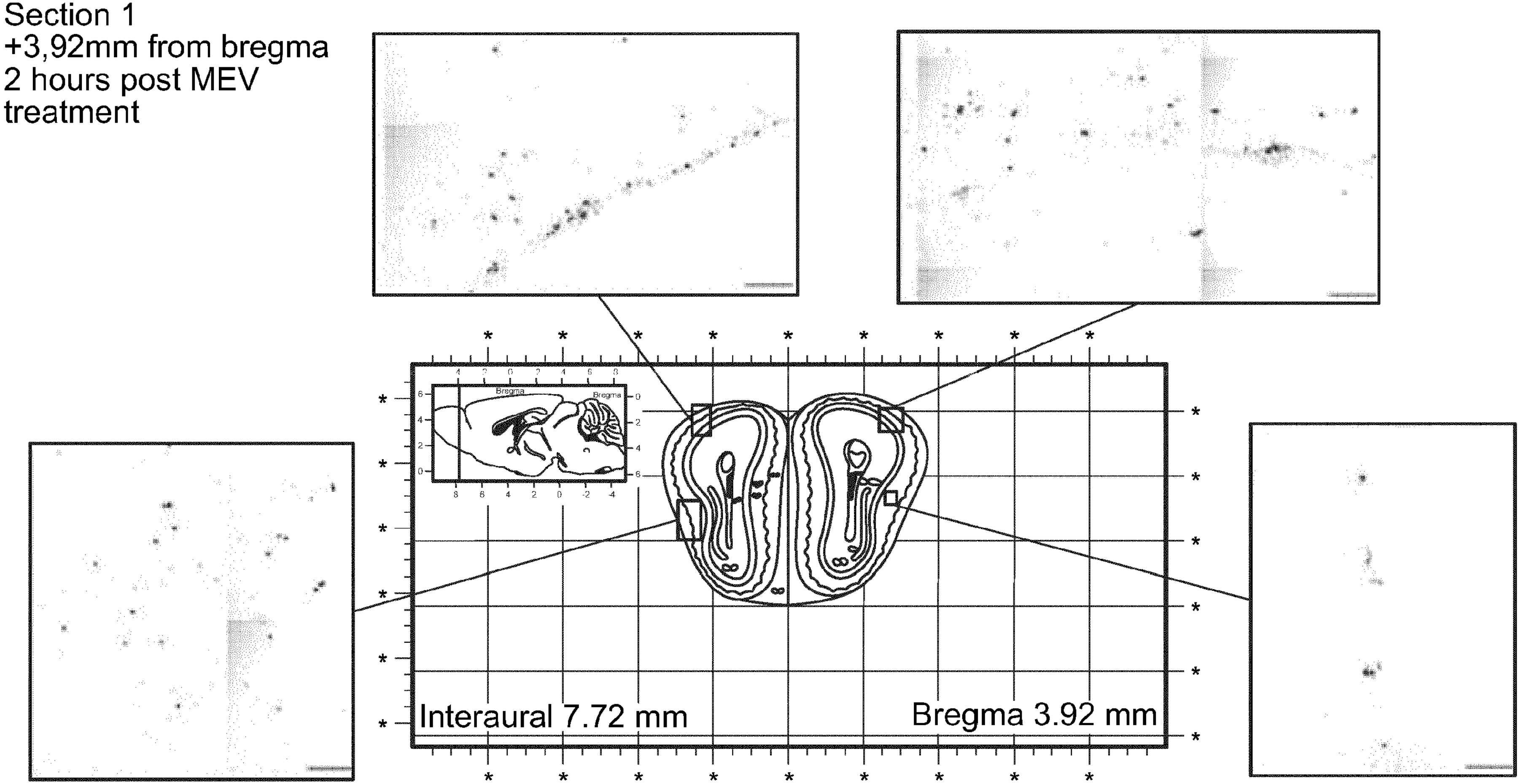


FIG. 37B

Animal number 7
Section 1
+3,92mm from bregma
4 hours post MEV treatment

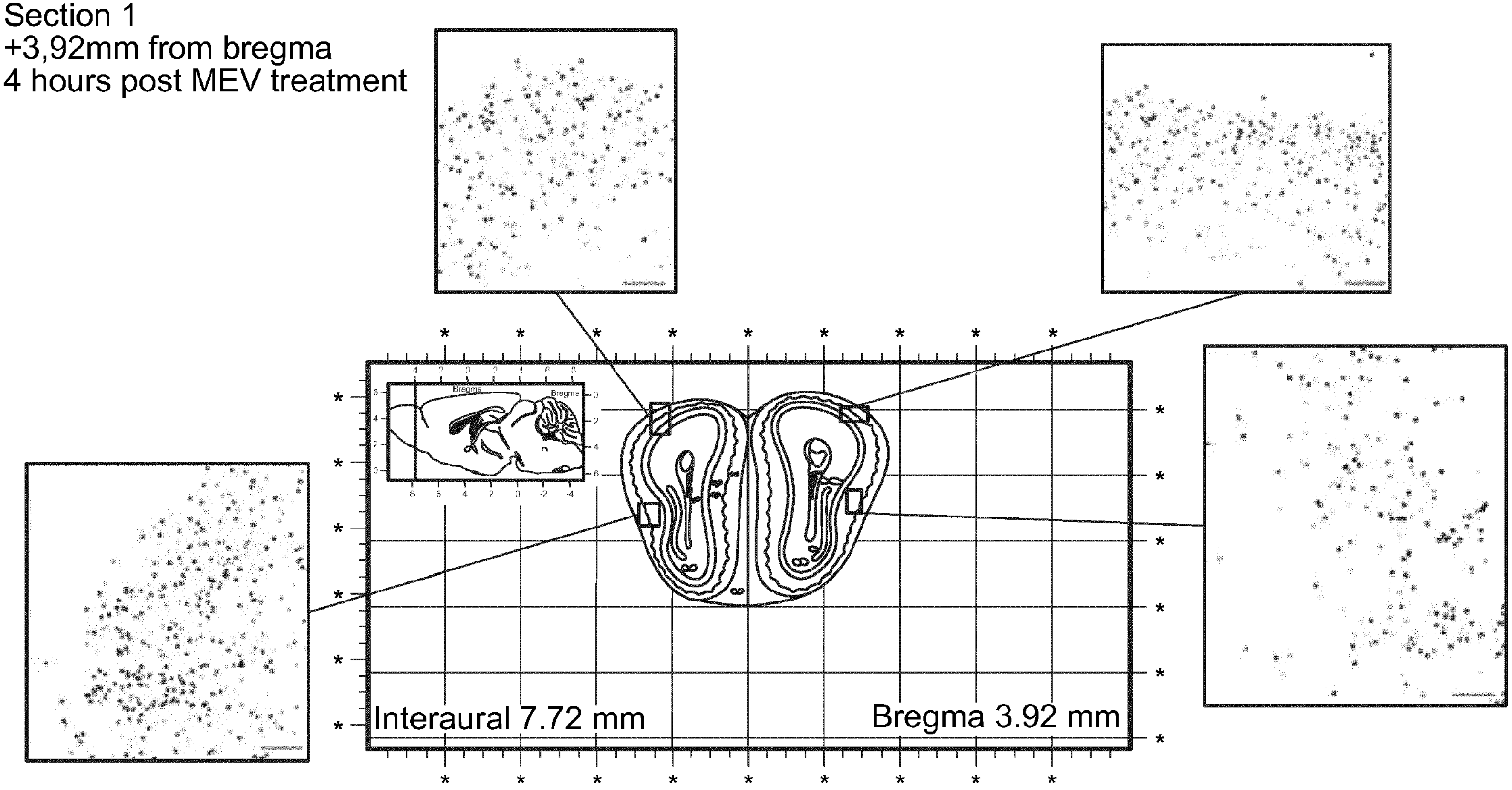


FIG. 37C

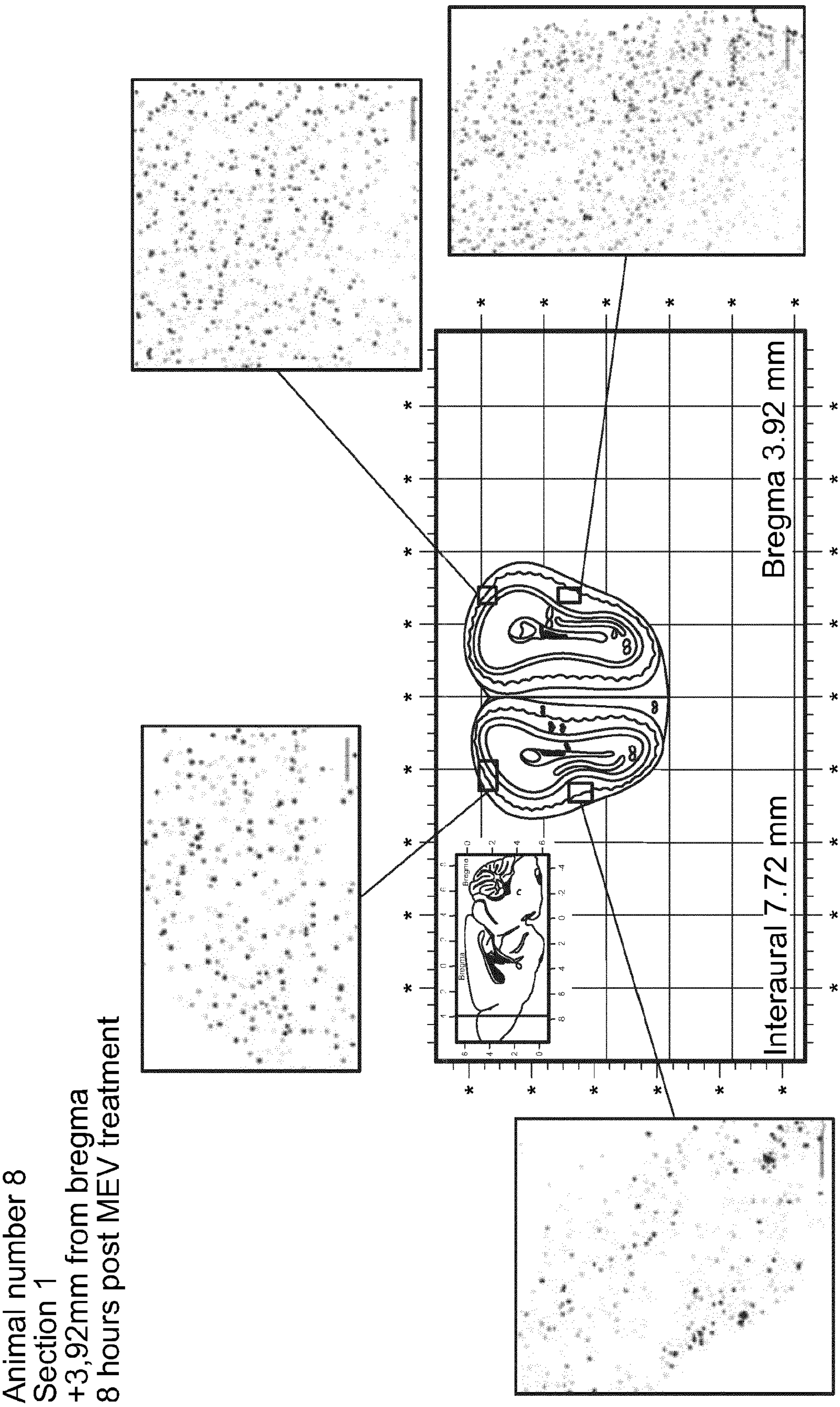
Animal number 8
Section 1
+3,92mm from bregma
8 hours post MEV treatment
Bregma 3.92 mm
Interaural 7.72 mm

FIG. 37D

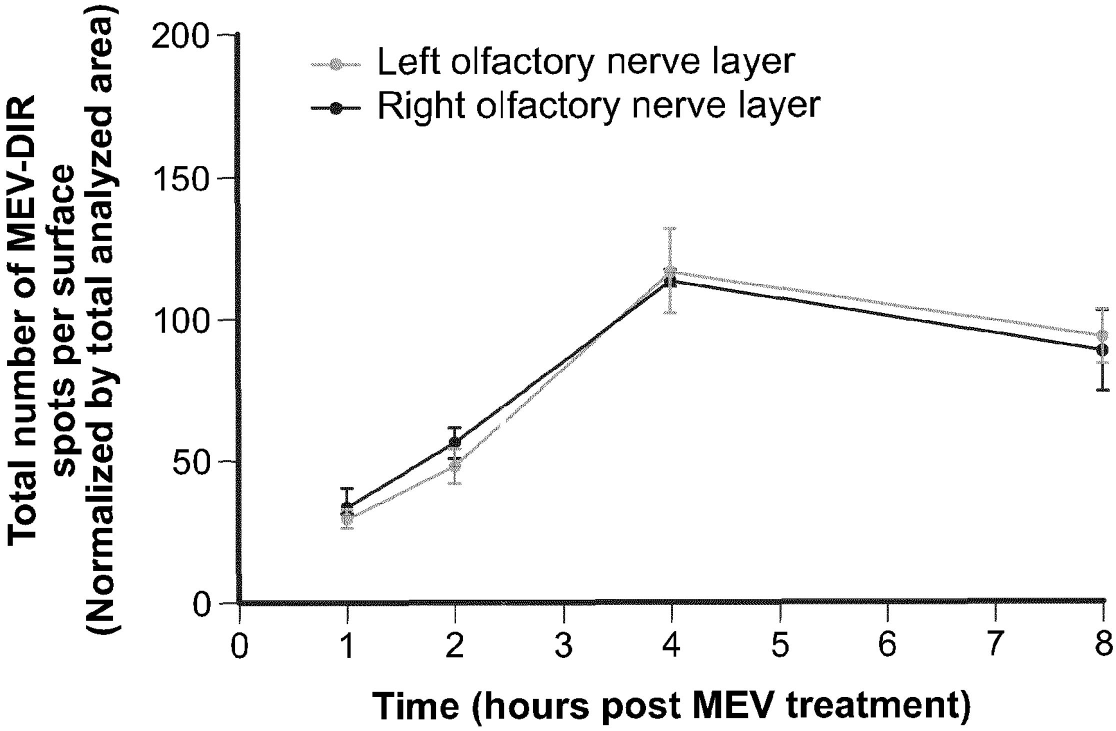
Total number of MEV-DIR spots per surface (Normalized by total analyzed area)
200
150
100
50
0
Left olfactory nerve layer
Right olfactory nerve layer
0
1
2
3
4
5
6
7
8
Time (hours post MEV treatment)

FIG. 38

Animal number 3
Section 2
+1,78mm from bregma
1 hour post MEV treatment

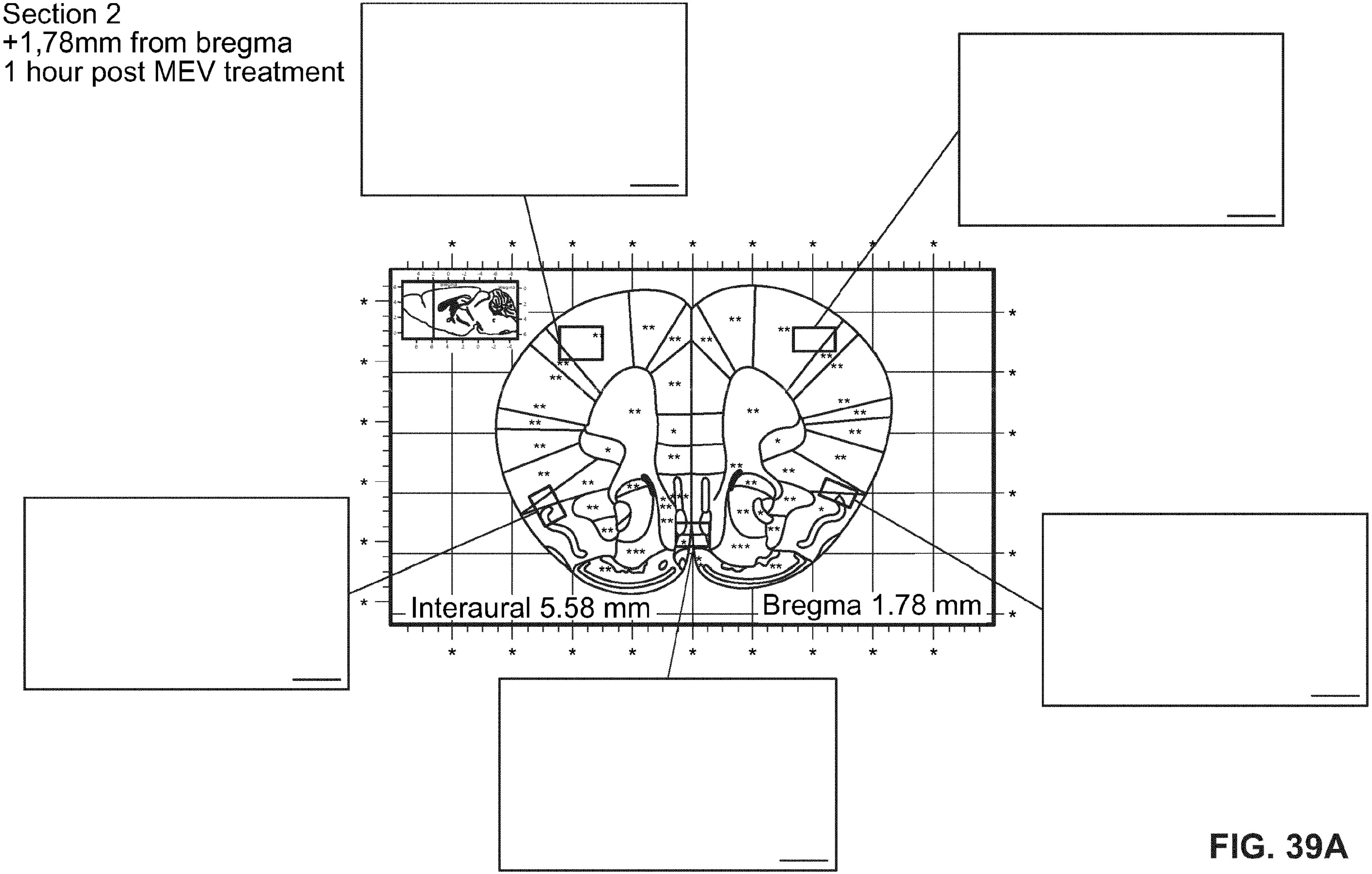


FIG. 39A

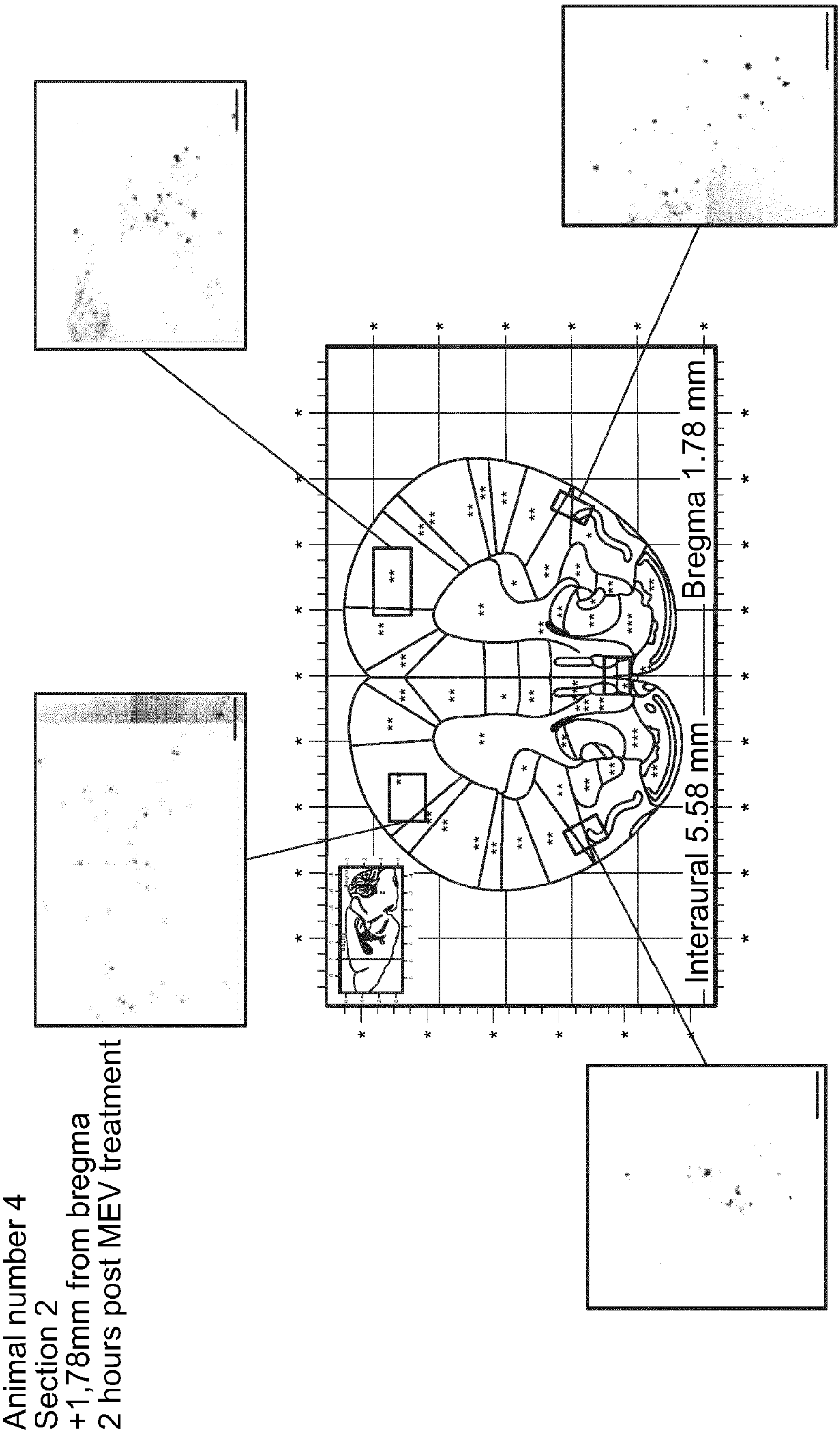
Animal number 4
Section 2
+1,78mm from bregma
2 hours post MEV treatment
Bregma 1.78 mm
Interaural 5.58 mm

FIG. 39B

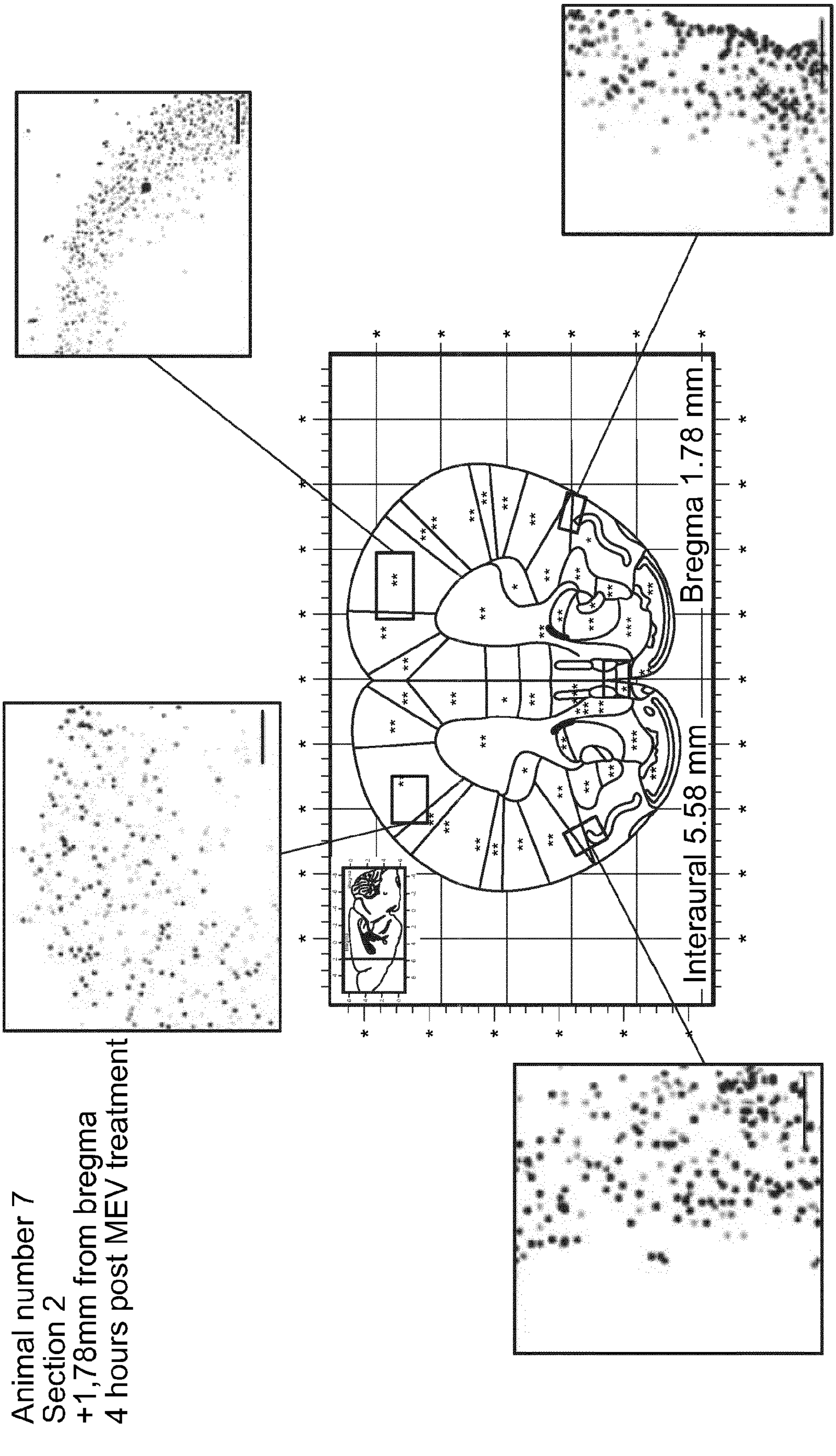
Animal number 7
Section 2
+1,78mm from bregma
4 hours post MEV treatment
Interaural 5.58 mm
Bregma 1.78 mm

FIG. 39C

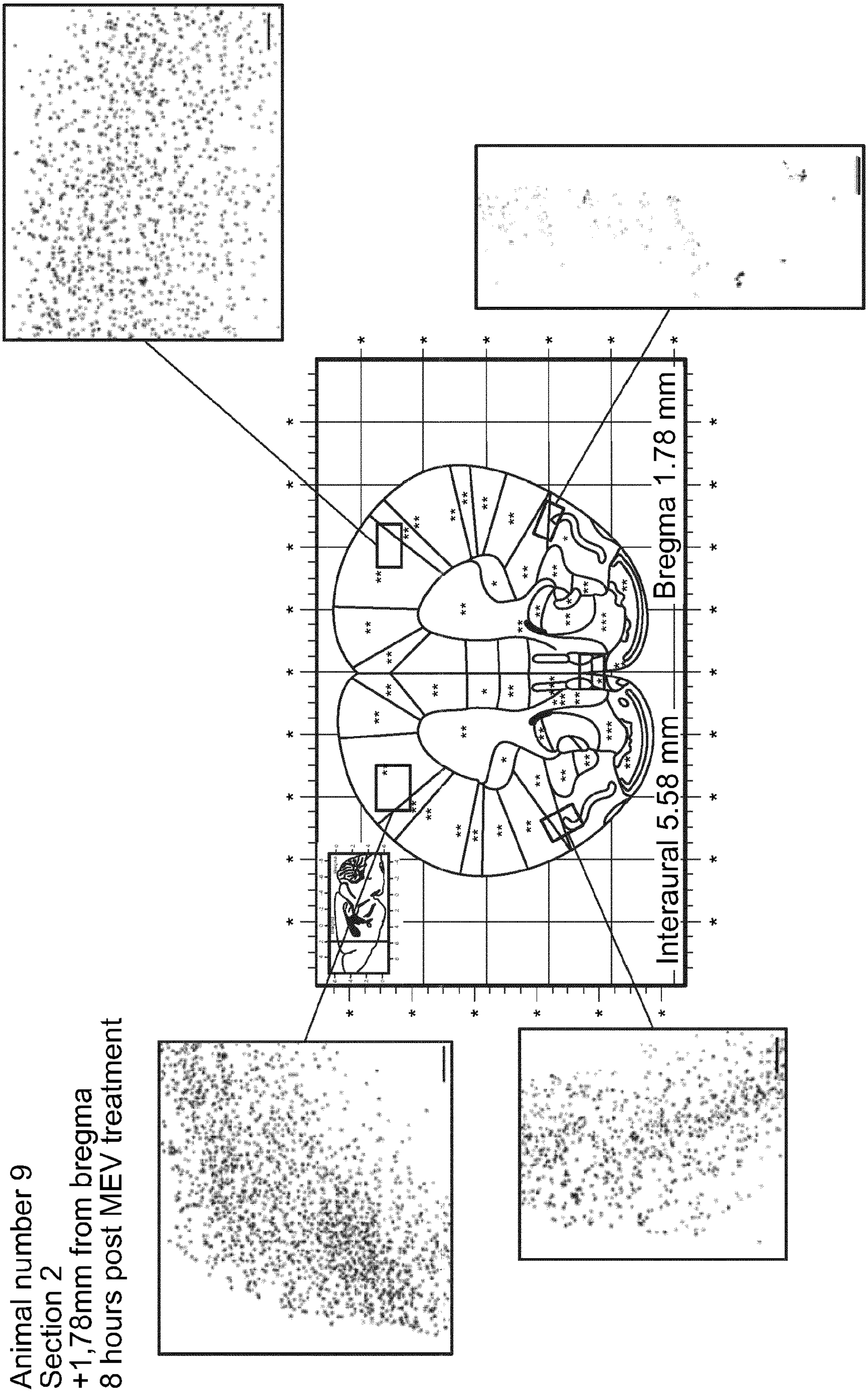
Animal number 9
Section 2
+1,78mm from bregma
8 hours post MEV treatment
Interaural 5.58 mm
Bregma 1.78 mm

FIG. 39D

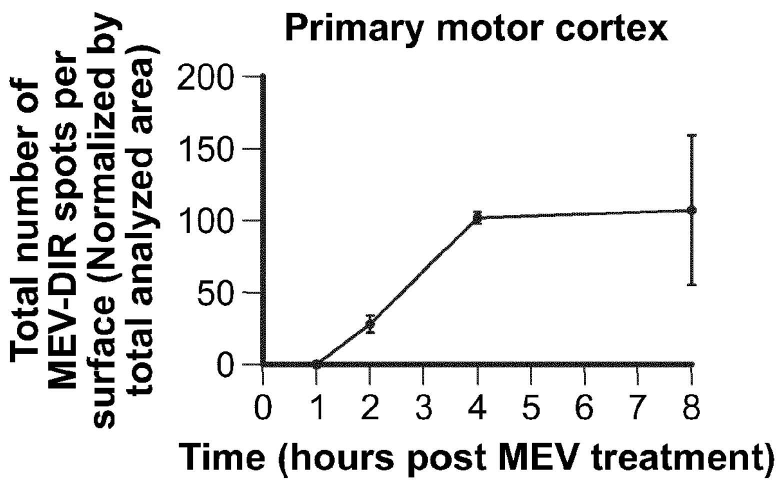


FIG. 40A

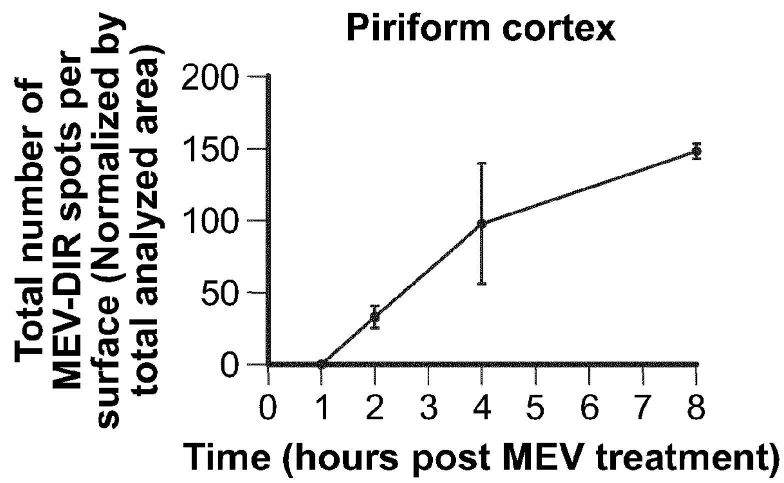


FIG. 40B

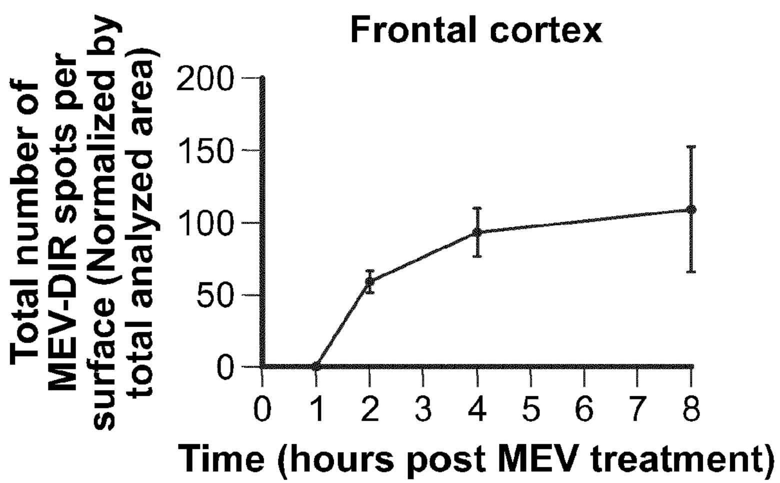


FIG. 40C

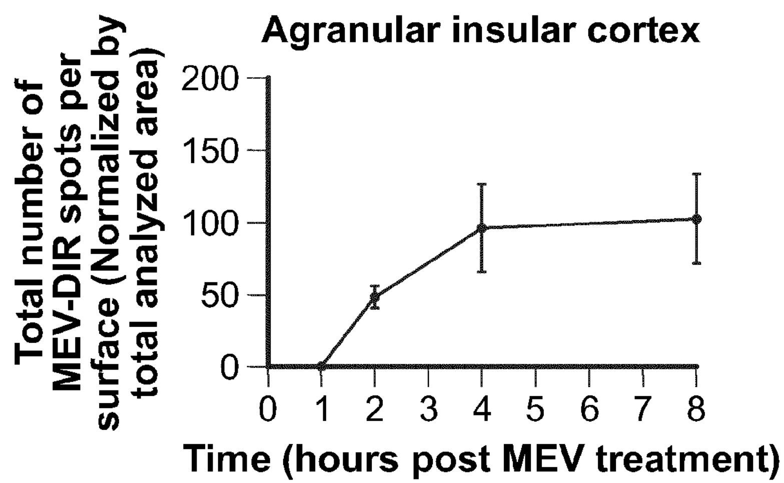


FIG. 40D

Animal number 1
Section 3
-1,82mm from bregma
1 hour post vehicle
treatment

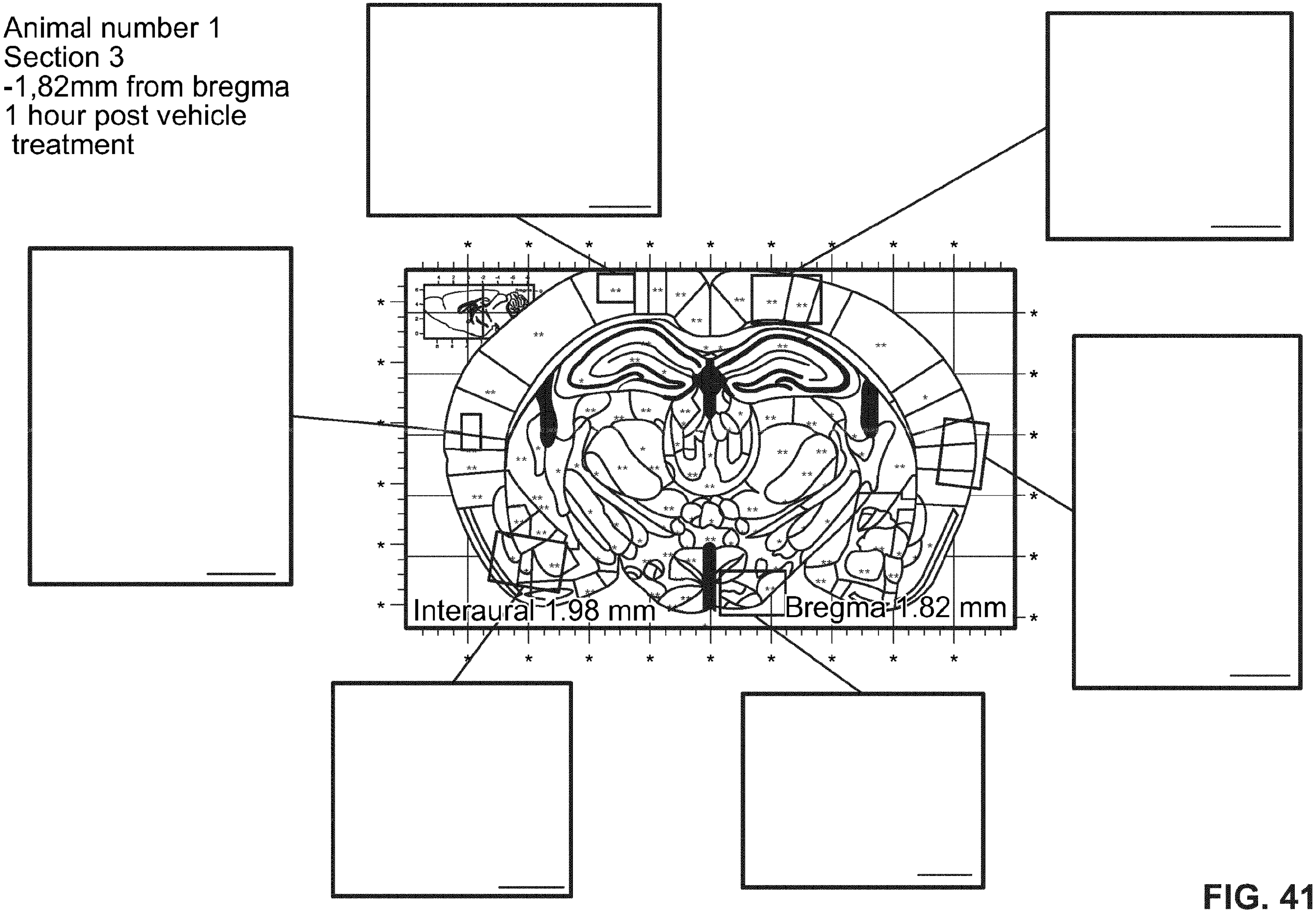


FIG. 41A

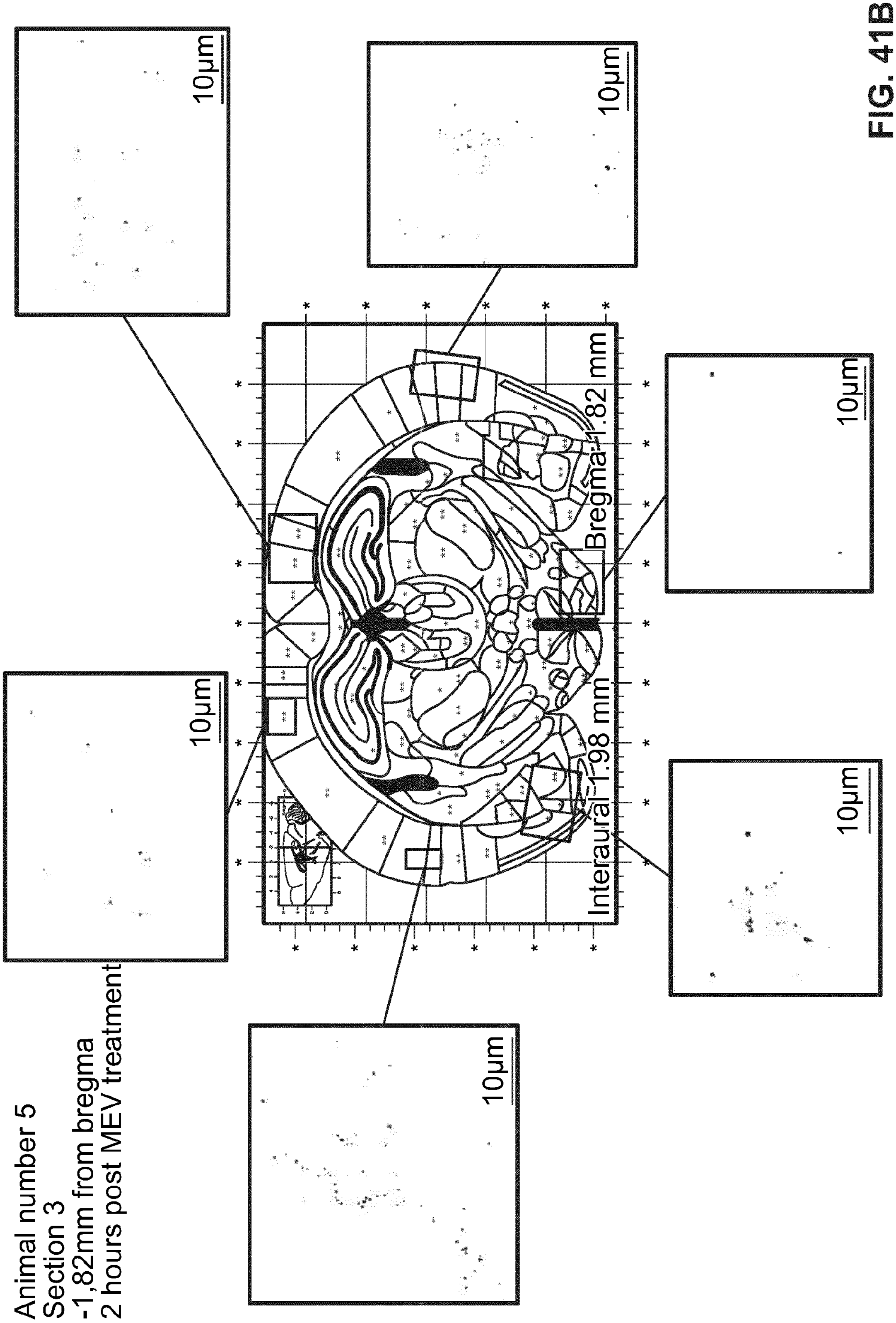
Animal number 5
Section 3
-1,82mm from bregma
2 hours post MEV treatment
Bregma -1.82 mm
Interaural 1.98 mm
10µm
10µm
10µm
10µm
10µm
10µm

FIG. 41B

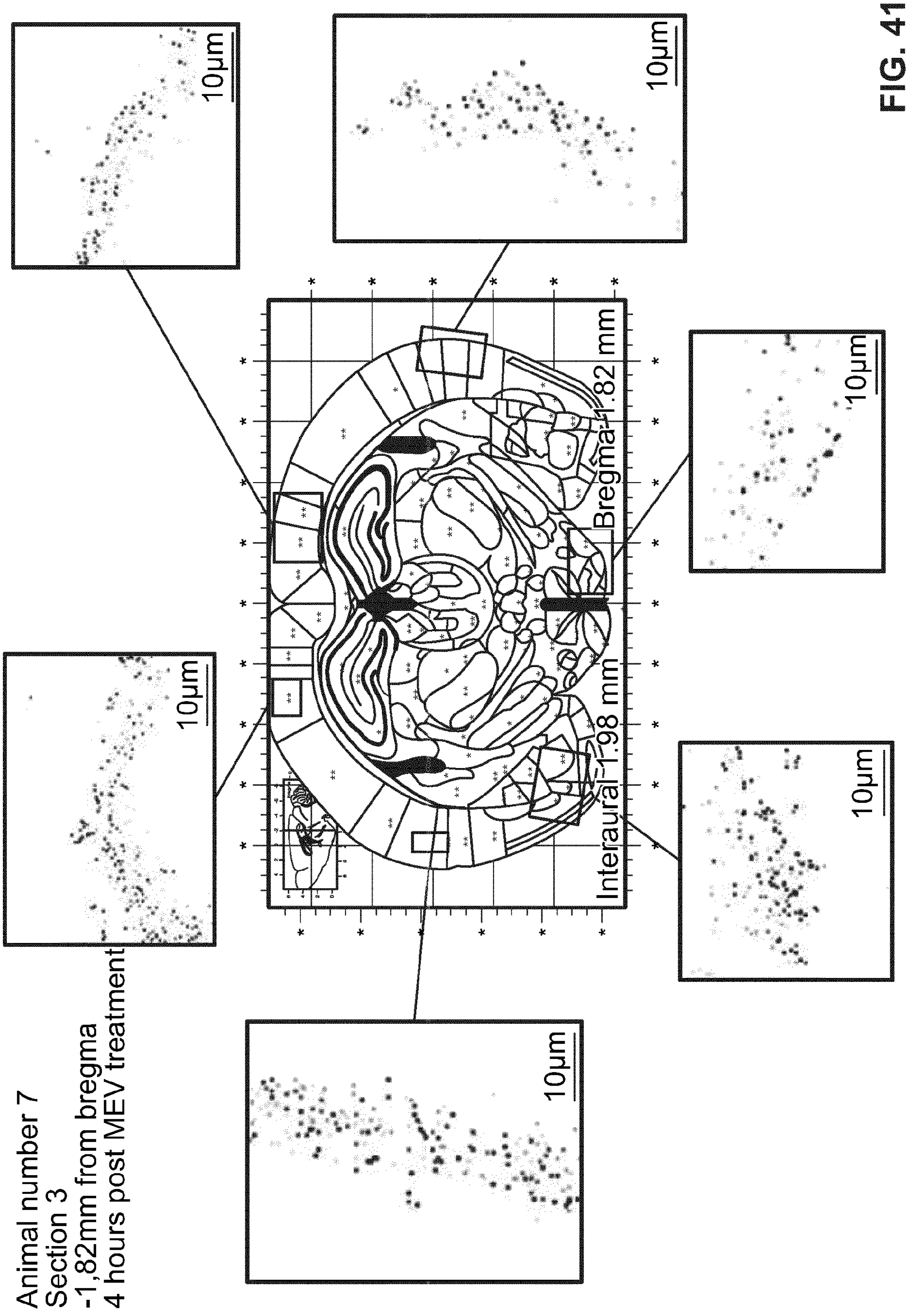
10µm
10µm
10µm
10µm
10µm
10µm
Bregma -1.82 mm
Interaural 1.98 mm
Animal number 7
Section 3
-1,82mm from bregma
4 hours post MEV treatment

FIG. 41C

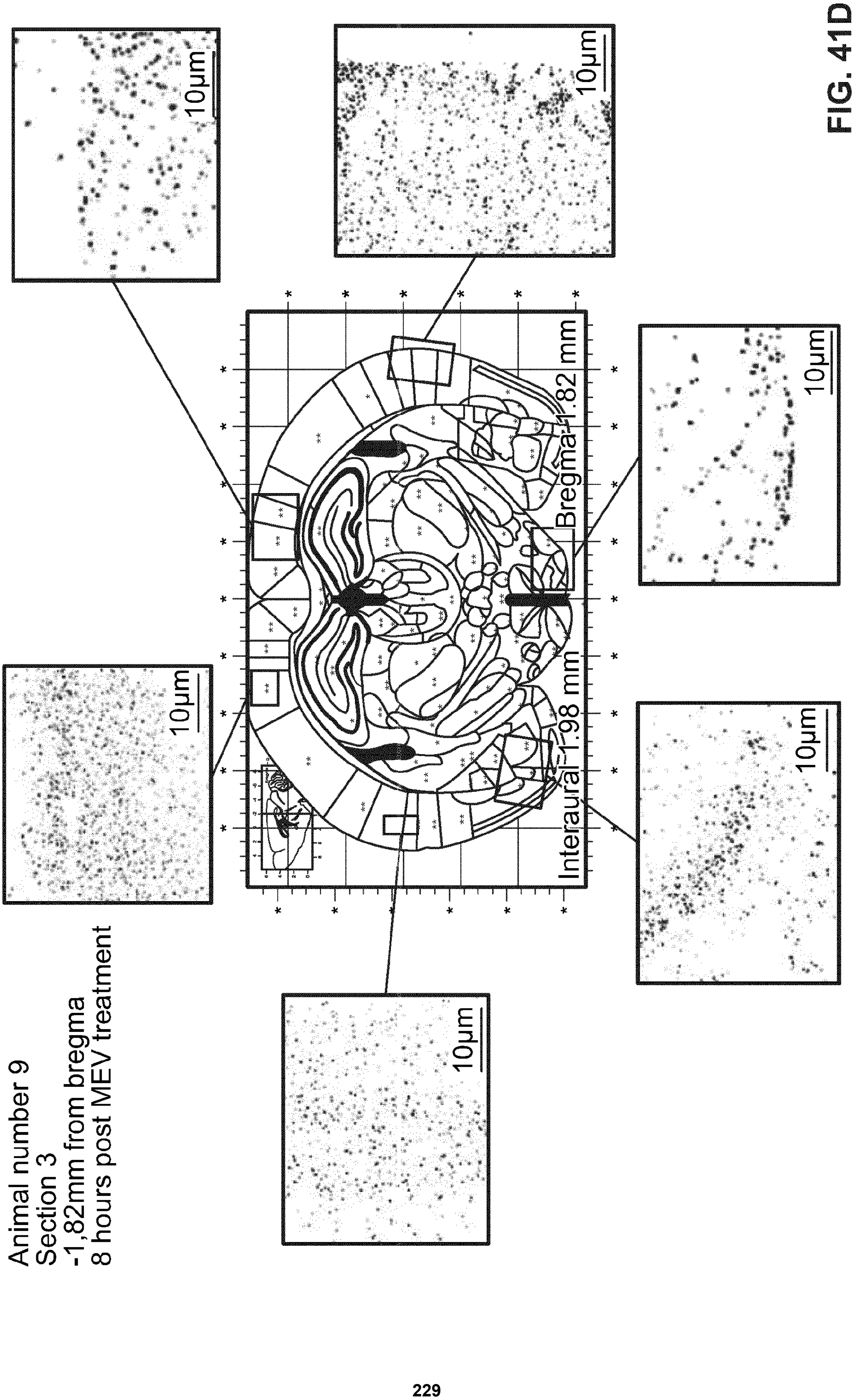
Animal number 9
Section 3
-1,82mm from bregma
8 hours post MEV treatment
Bregma -1.82 mm
Interaural 1.98 mm
10µm
10µm
10µm
10µm
10µm
10µm

FIG. 41D

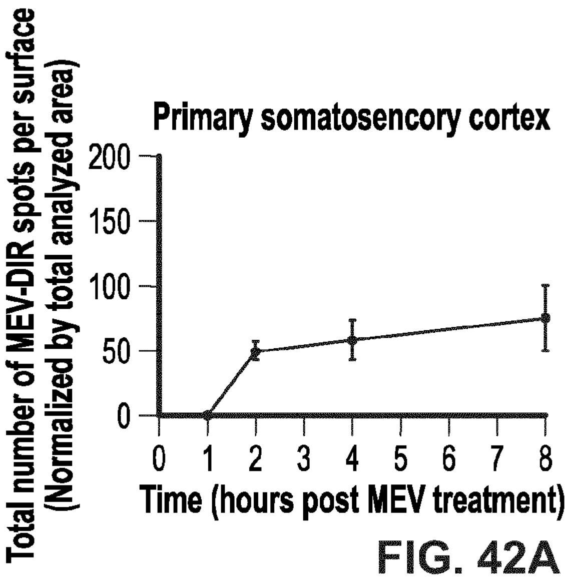


FIG. 42A

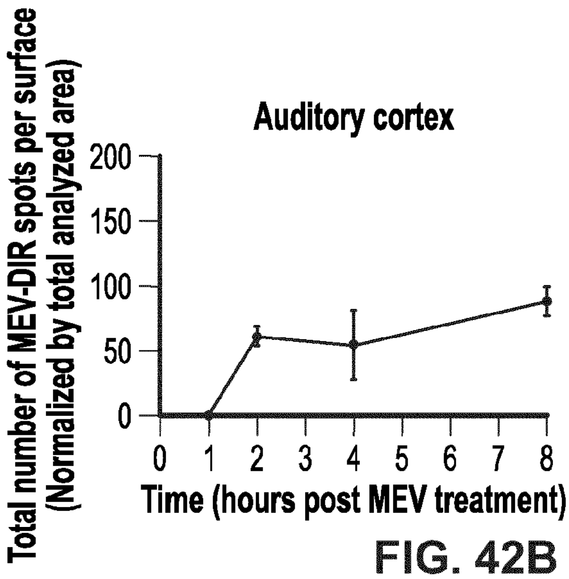


FIG. 42B

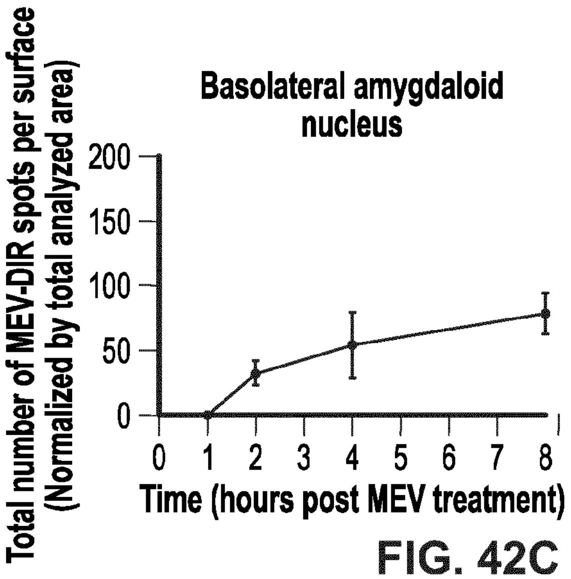


FIG. 42C

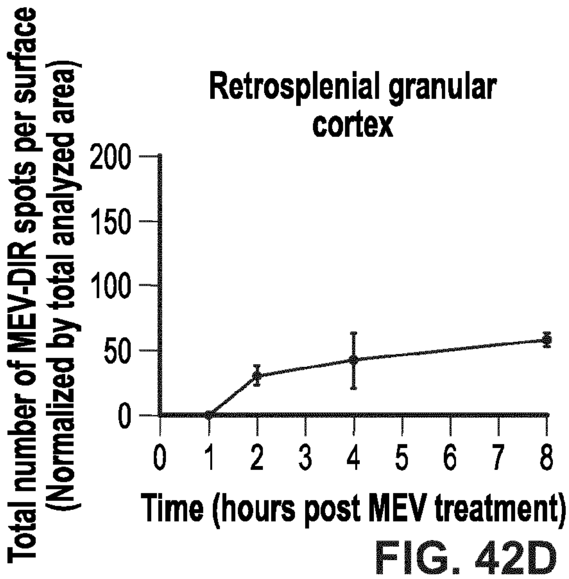


FIG. 42D

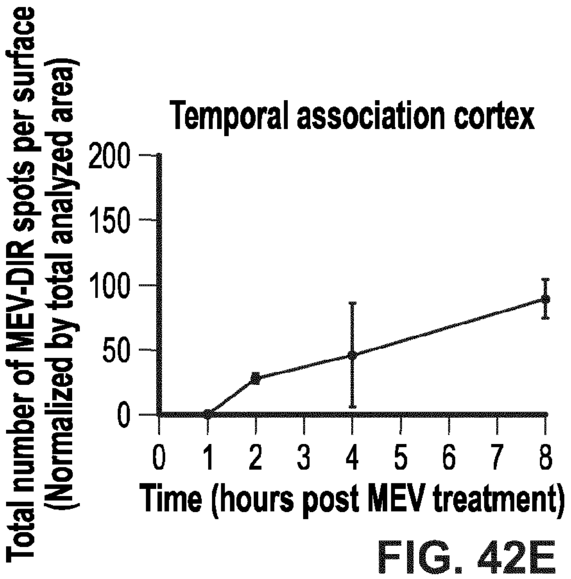


FIG. 42E

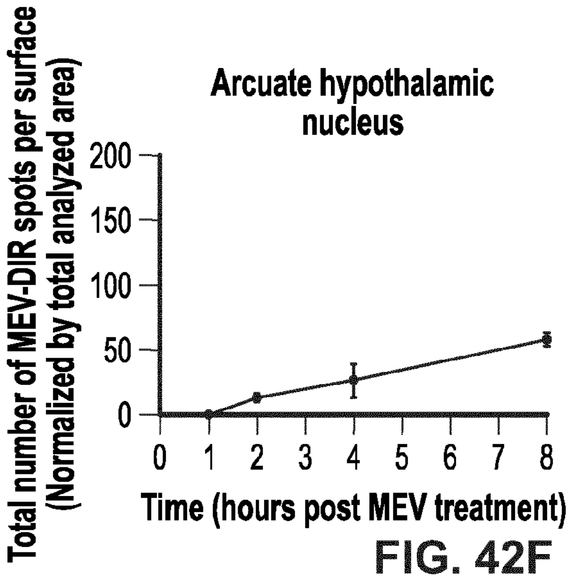


FIG. 42F

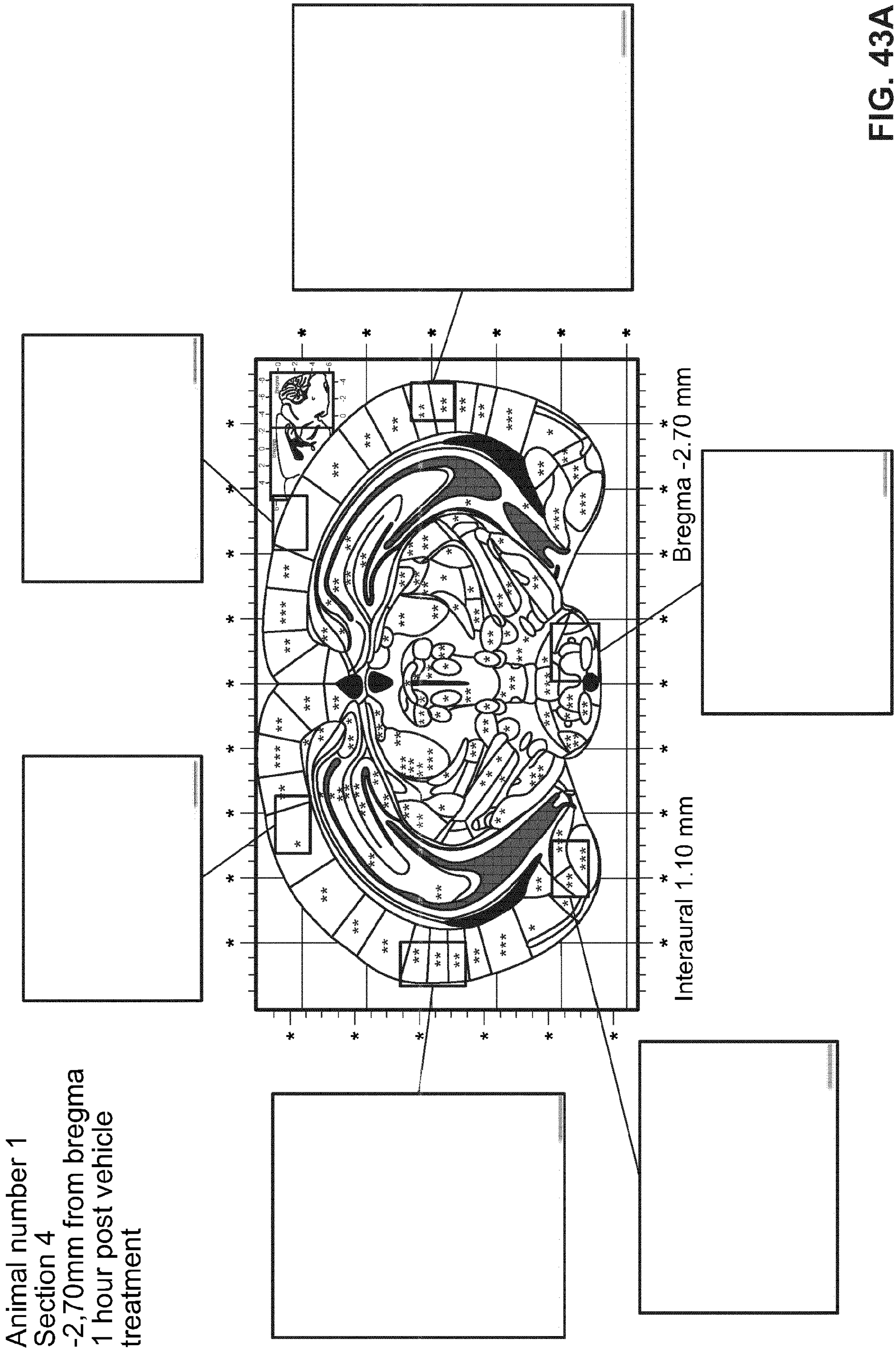
Animal number 1
Section 4
-2,70mm from bregma
1 hour post vehicle
treatment
Bregma -2.70 mm
Interaural 1.10 mm

FIG. 43A

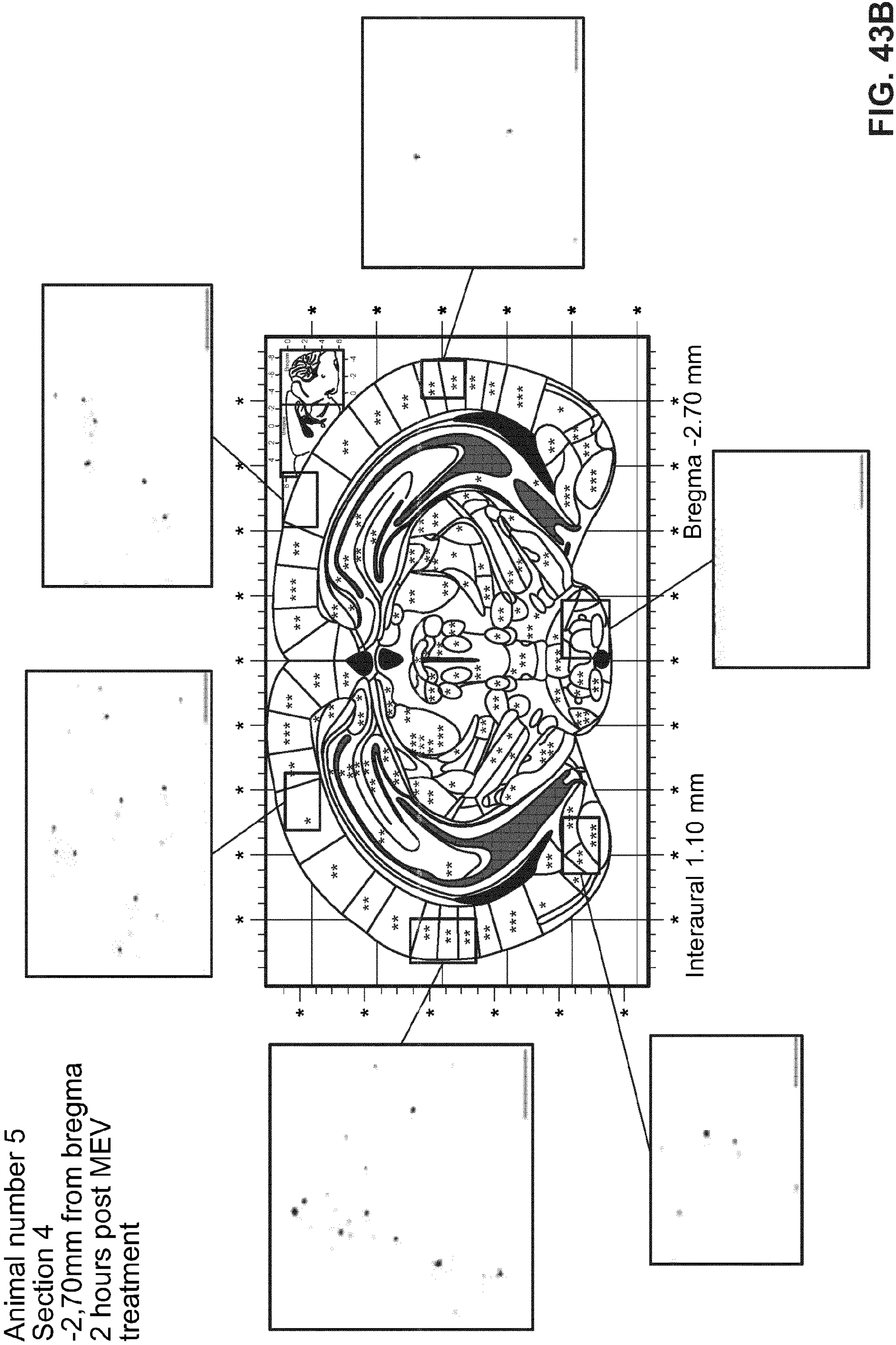
Animal number 5
Section 4
-2,70mm from bregma
2 hours post MEV
treatment
Bregma -2.70 mm
Interaural 1.10 mm

FIG. 43B

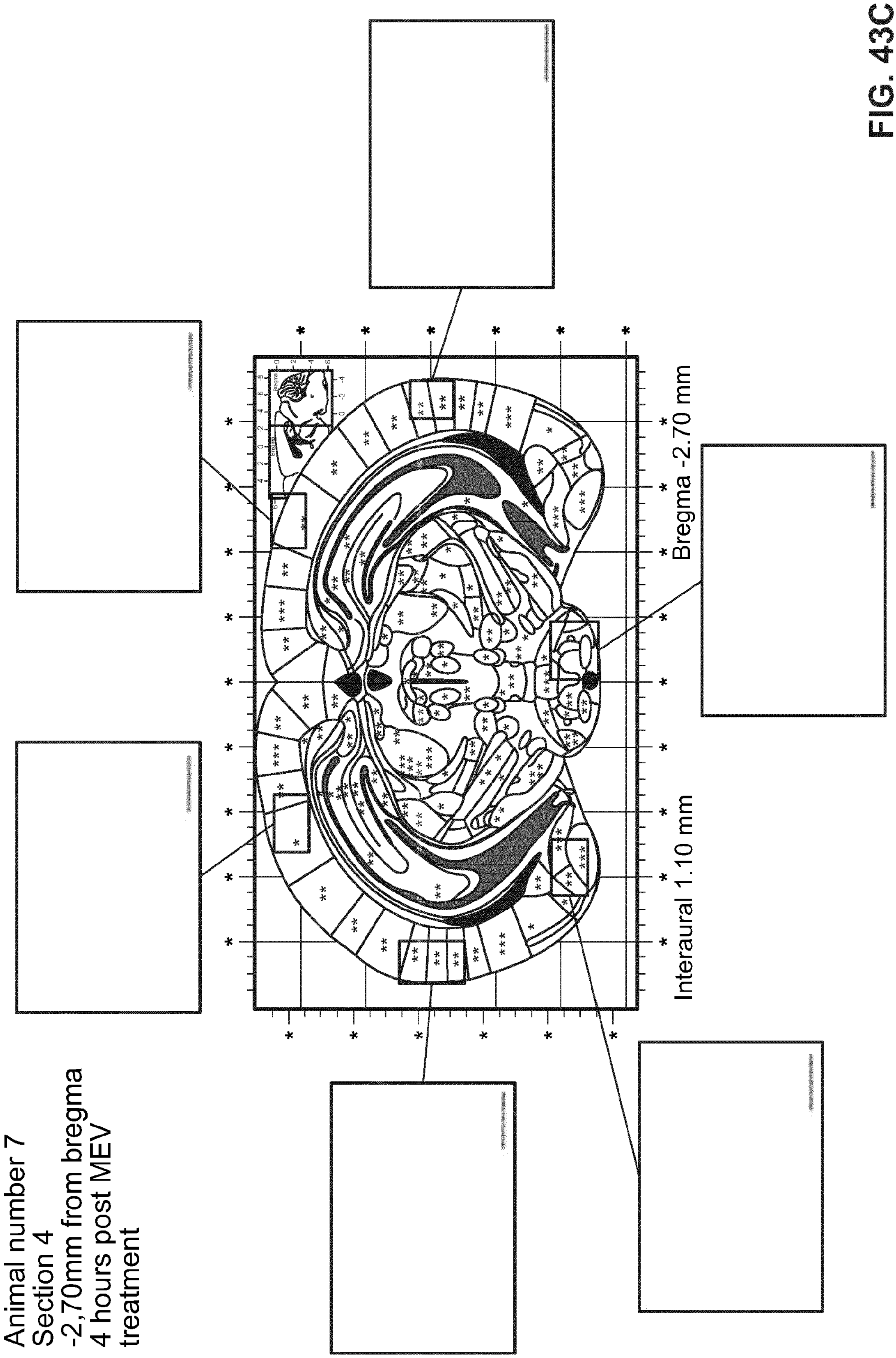
Animal number 7
Section 4
-2,70mm from bregma
4 hours post MEV
treatment
Bregma -2.70 mm
Interaural 1.10 mm

FIG. 43C

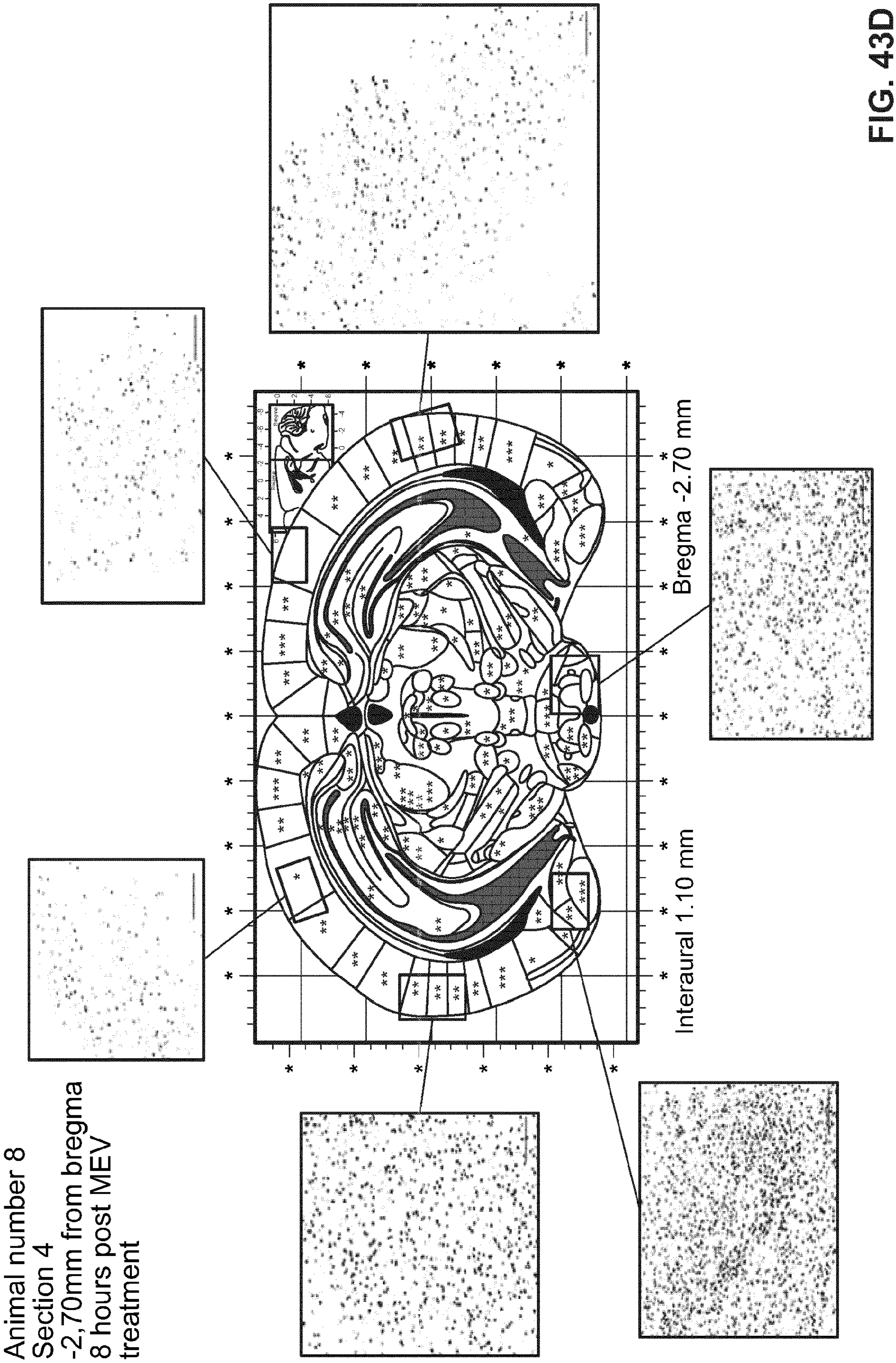
Animal number 8
Section 4
-2,70mm from bregma
8 hours post MEV
treatment
Bregma -2.70 mm
Interaural 1.10 mm

FIG. 43D

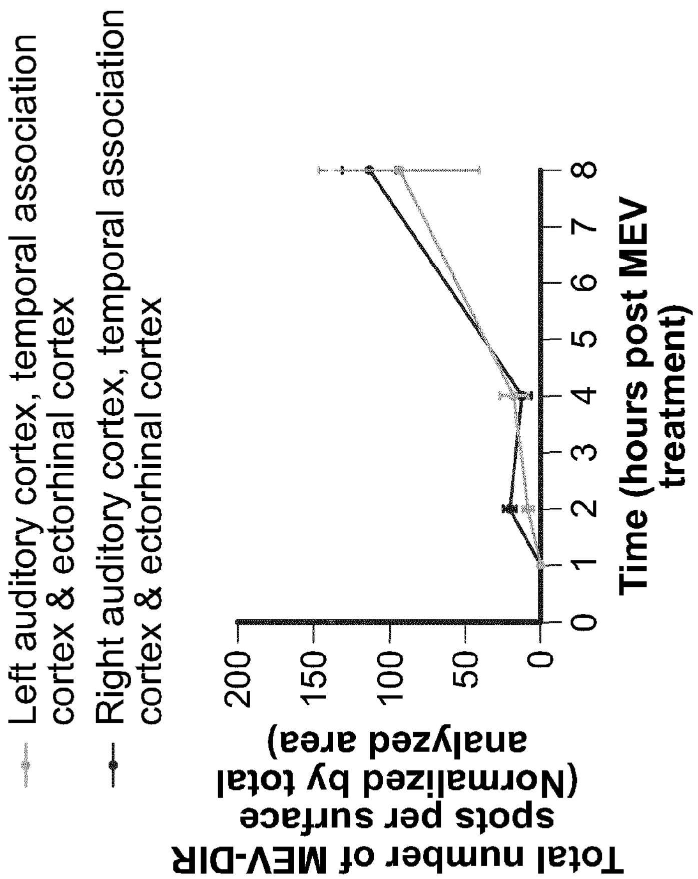


FIG. 44B

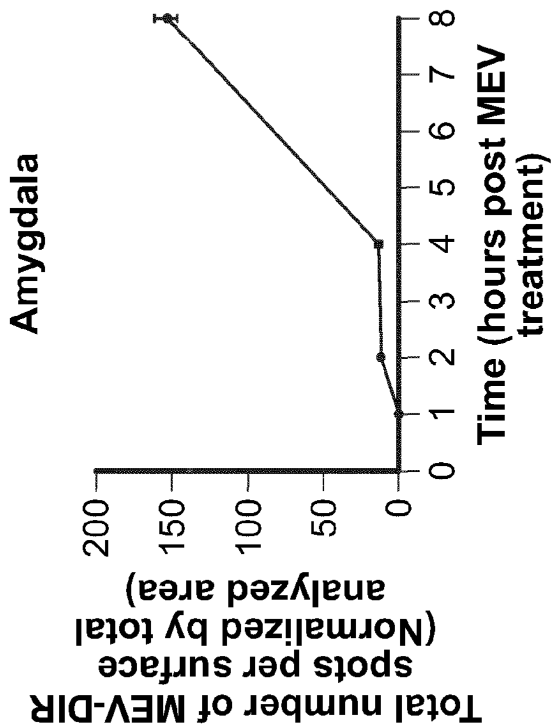


FIG. 44A

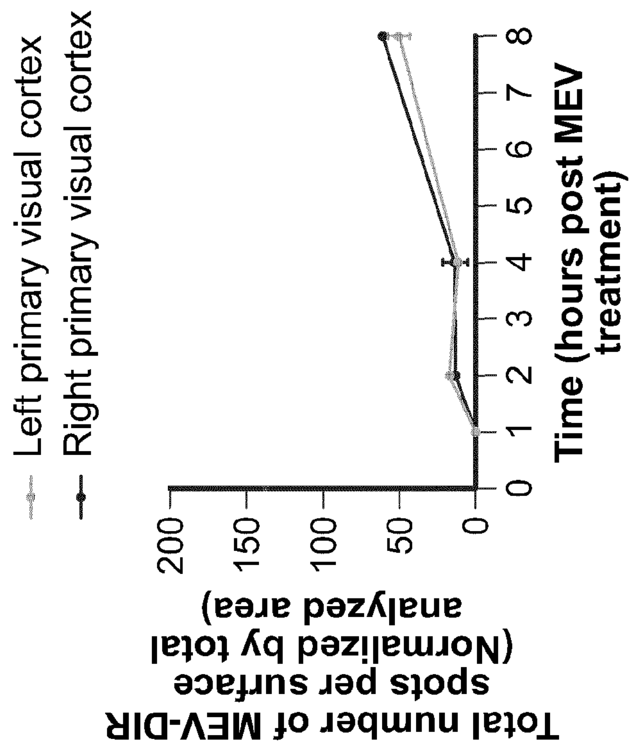


FIG. 44C

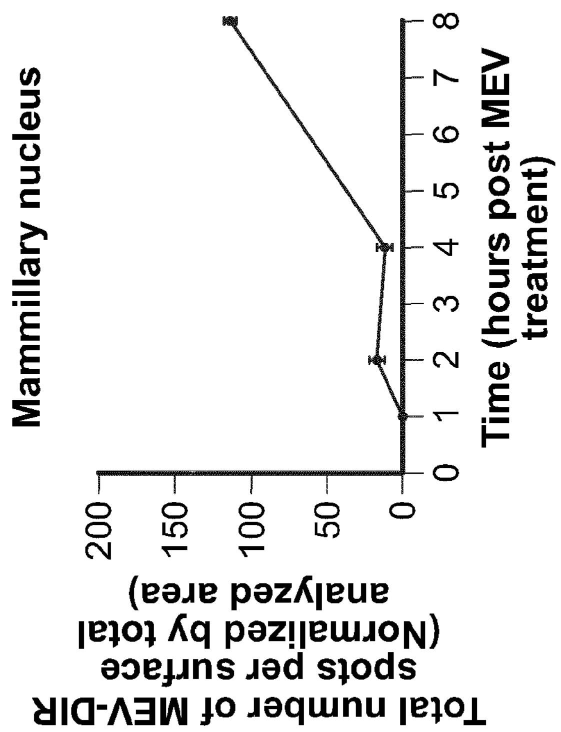


FIG. 44D

Animal number 1
Section 5
-5,20mm from bregma
1 hour post vehicle treatment

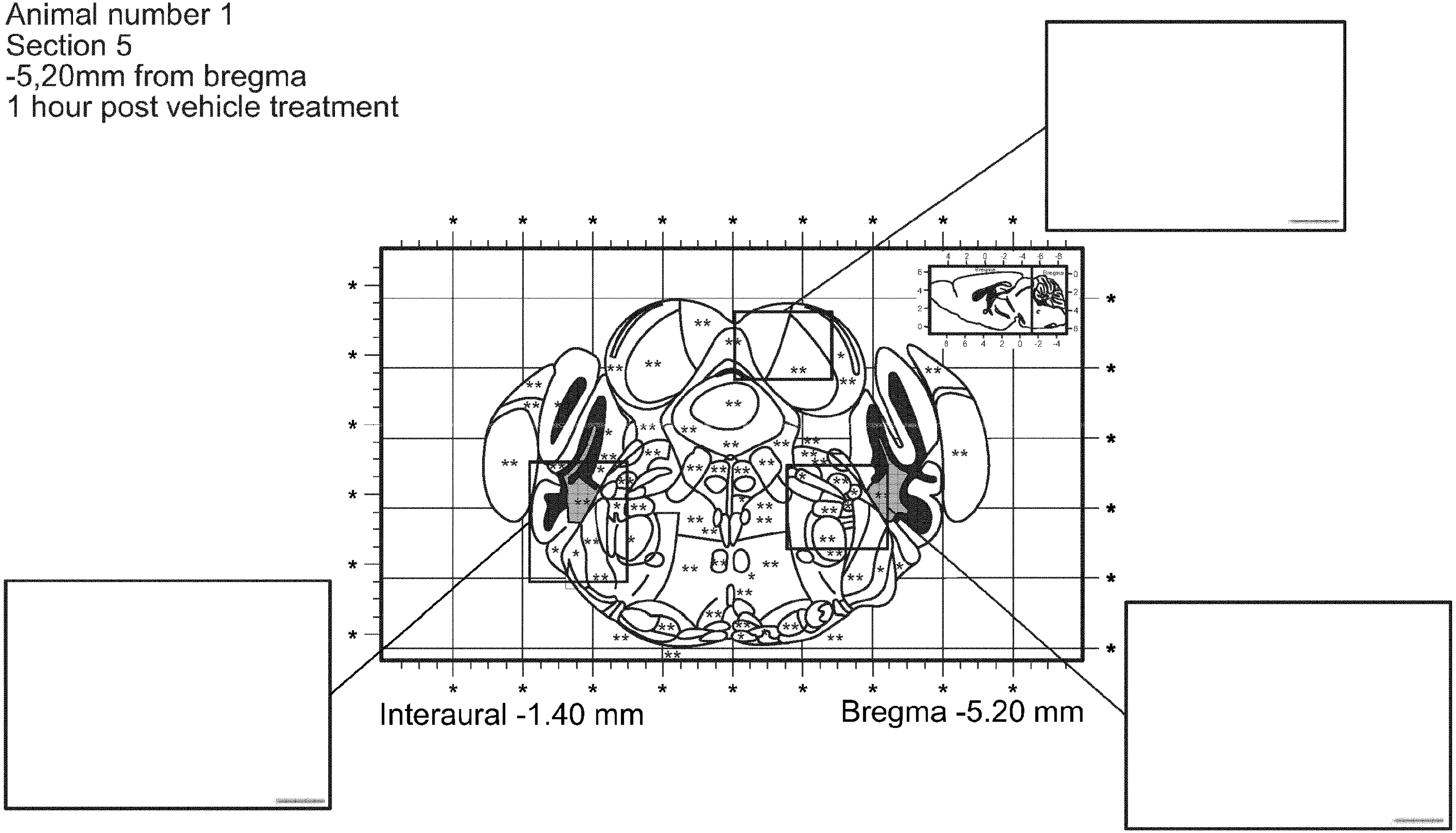


FIG. 45A

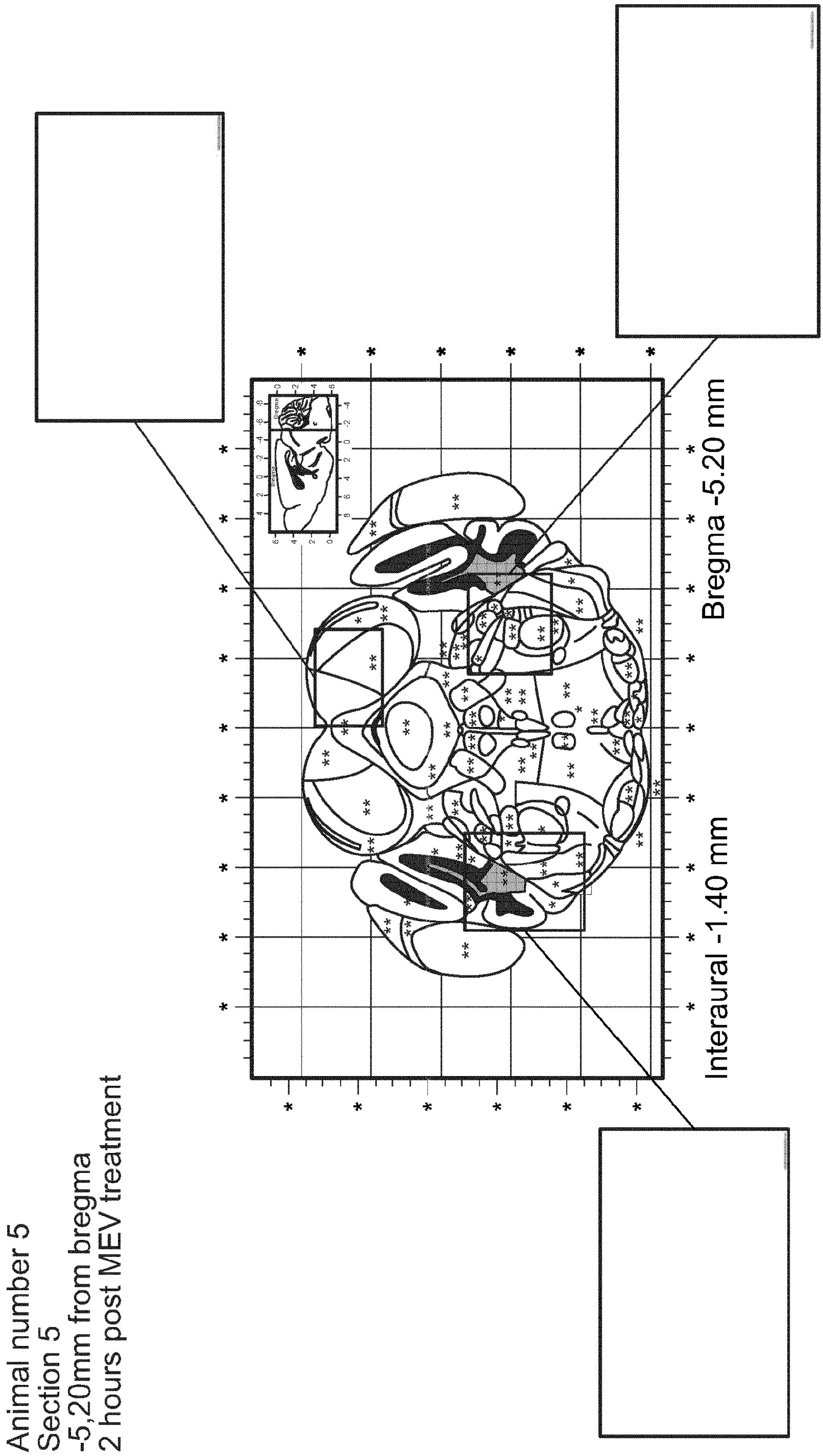
Animal number 5
Section 5
-5,20mm from bregma
2 hours post MEV treatment
Bregma -5.20 mm
Interaural -1.40 mm

FIG. 45B

Animal number 6
Section 5
-5,20mm from bregma
4 hours post MEV treatment

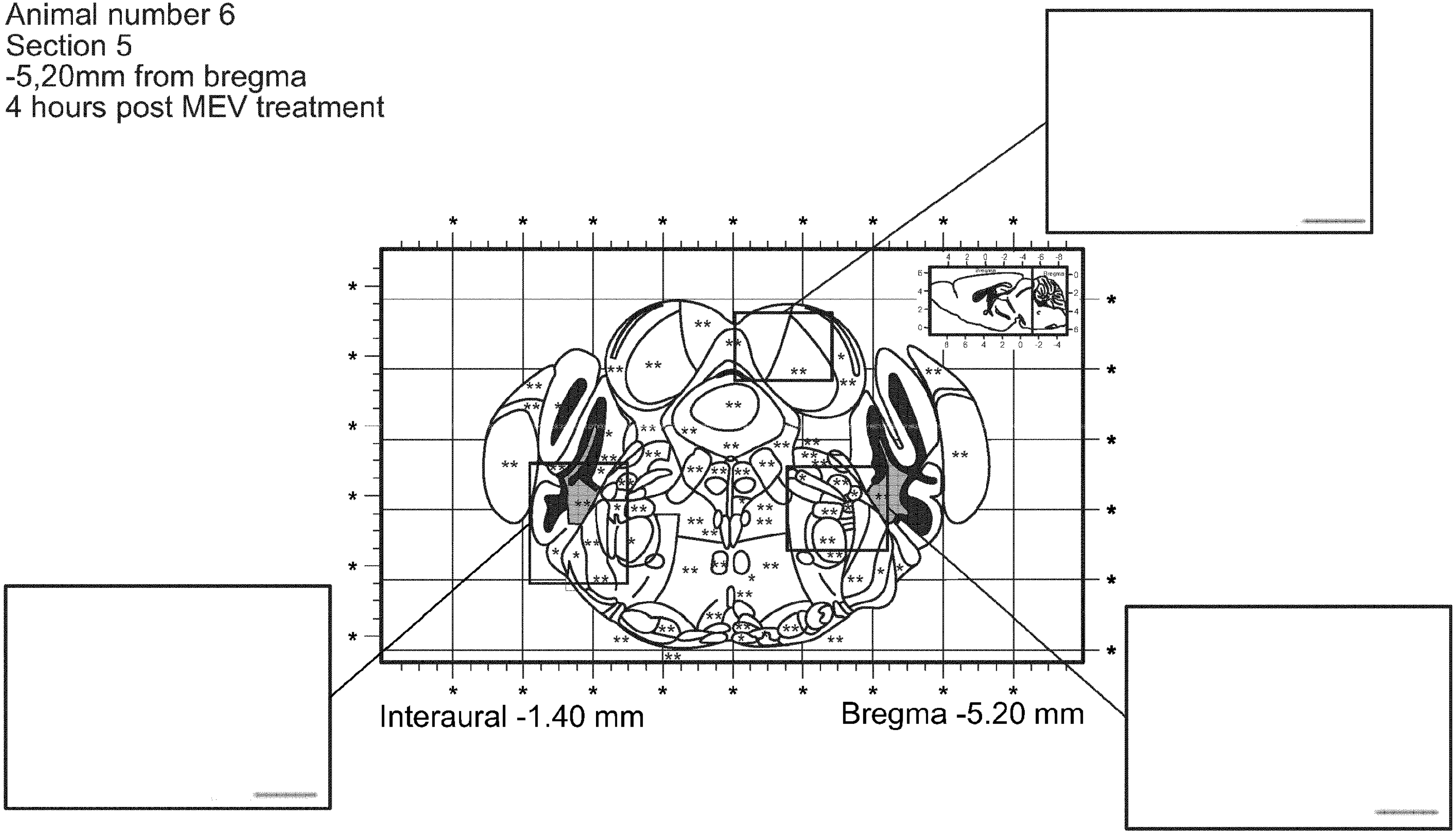


FIG. 45C

Animal number 9
Section 5
-5,20mm from bregma
8 hours post MEV treatment

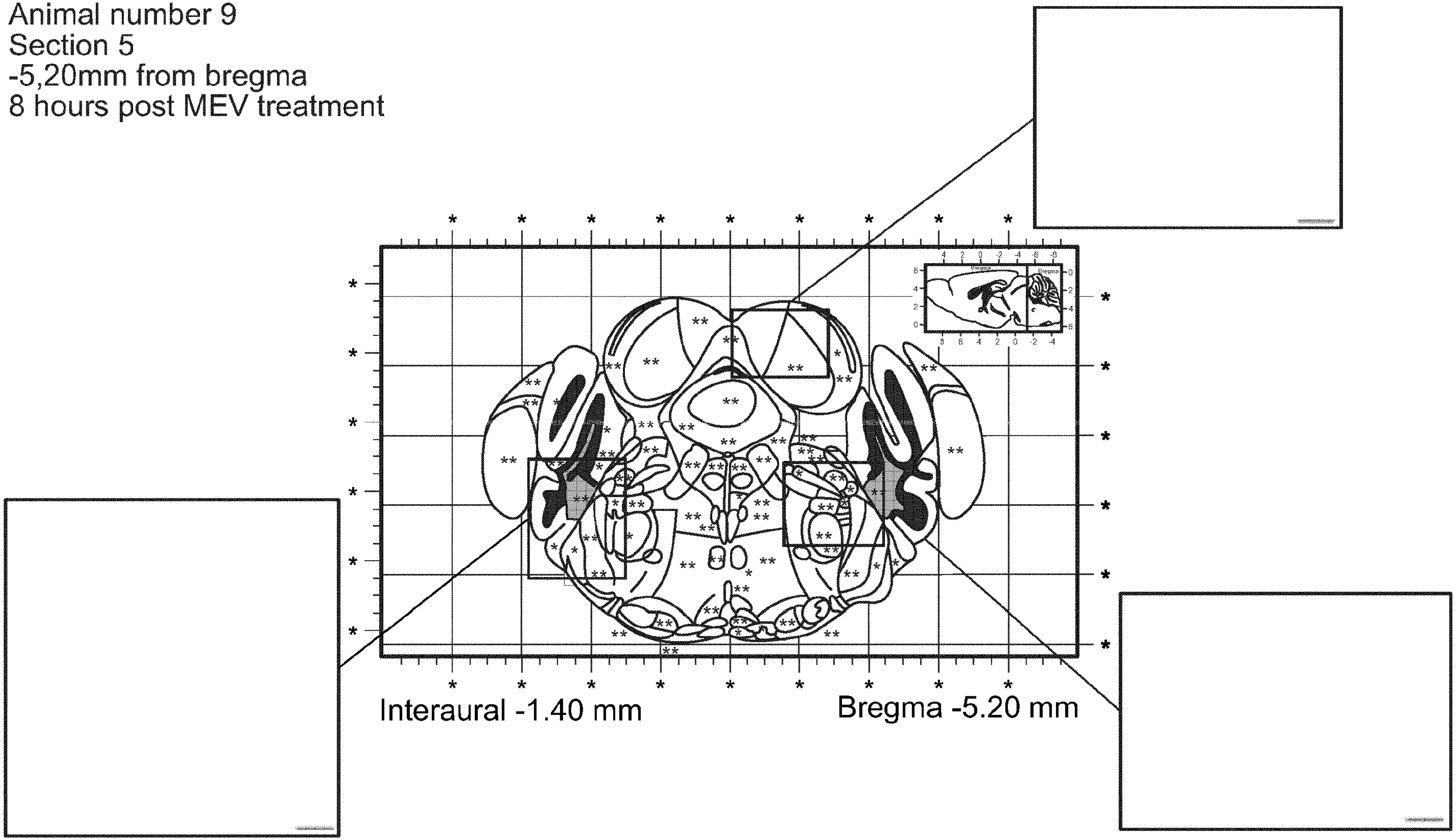


FIG. 45D

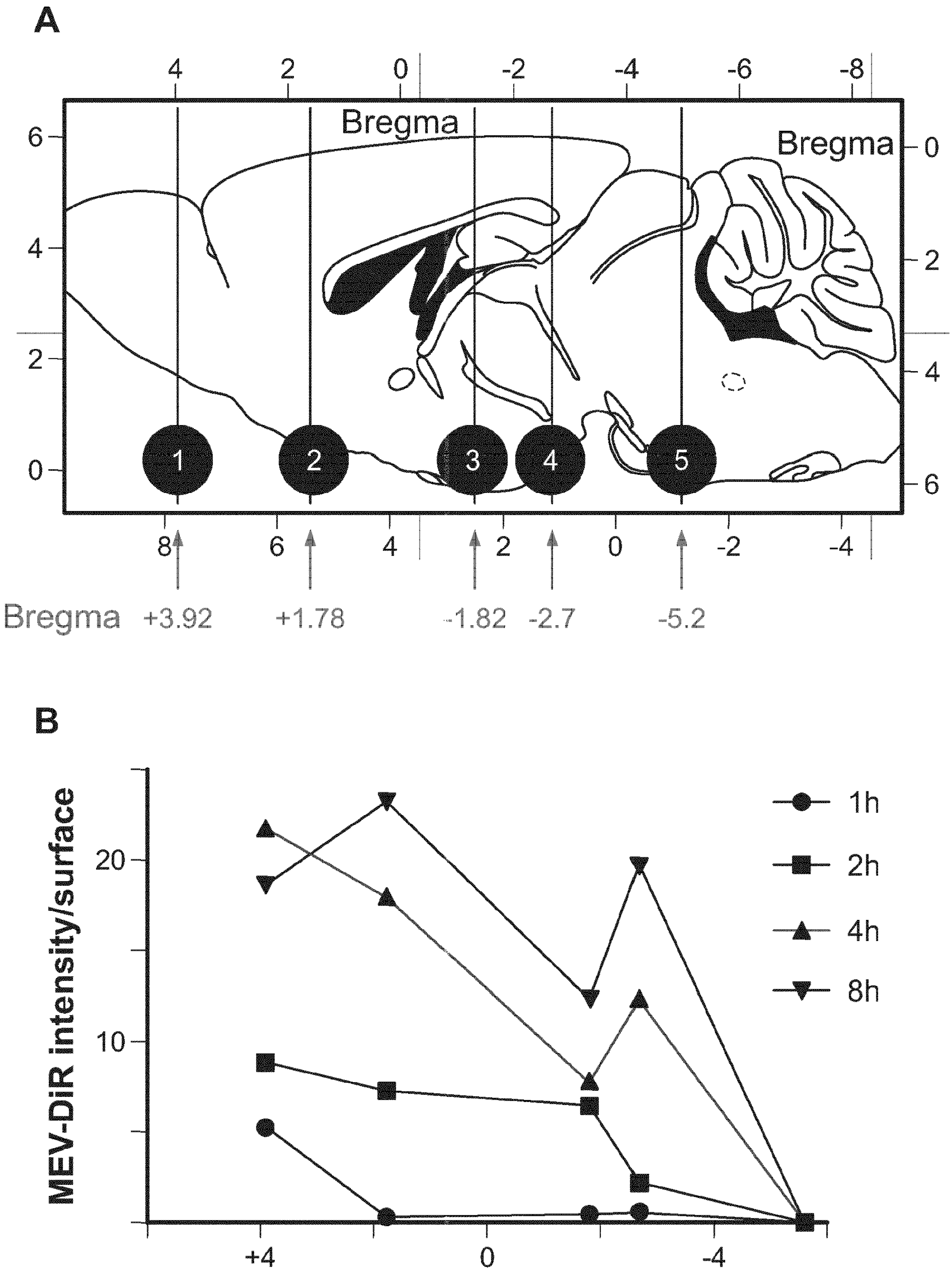
A
4
2
0
-2
-4
-6
-8
Bregma
Bregma
6
4
2
0
0
2
4
6
1
2
3
4
5
8
6
4
2
0
-2
-4
Bregma +3.92 +1.78 -1.82 -2.7 -5.2
B
MEV-DiR intensity/surface
20
10
+4
0
-4
1h
2h
4h
8h

FIG. 46

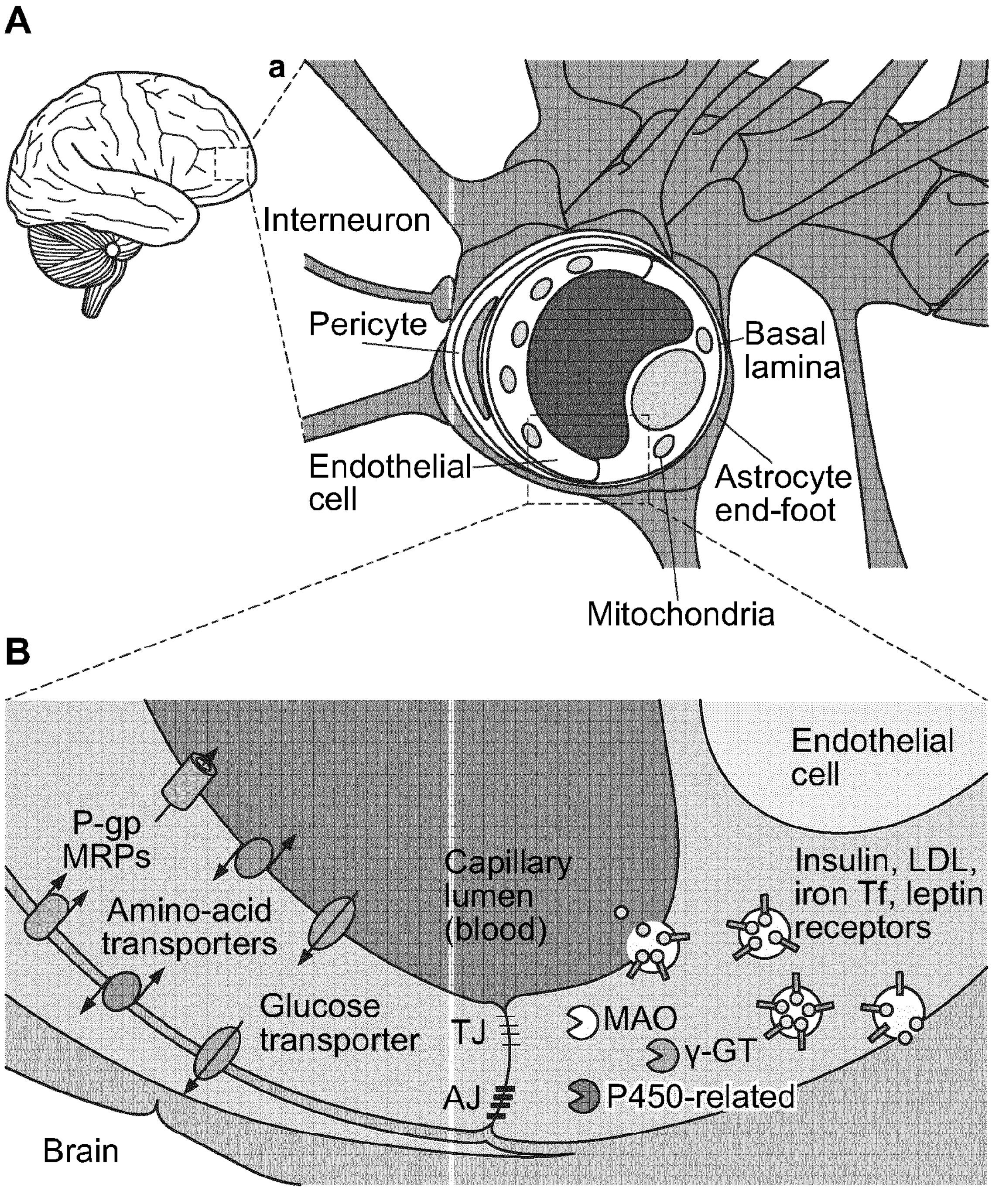
A
a
Interneuron
Pericyte
Endothelial cell
Basal lamina
Astrocyte end-foot
Mitochondria
B
P-gp
MRPs
Amino-acid transporters
Glucose transporter
Capillary lumen (blood)
Endothelial cell
Insulin, LDL, iron Tf, leptin receptors
TJ
AJ
MAO
γ-GT
P450-related
Brain

FIG. 47

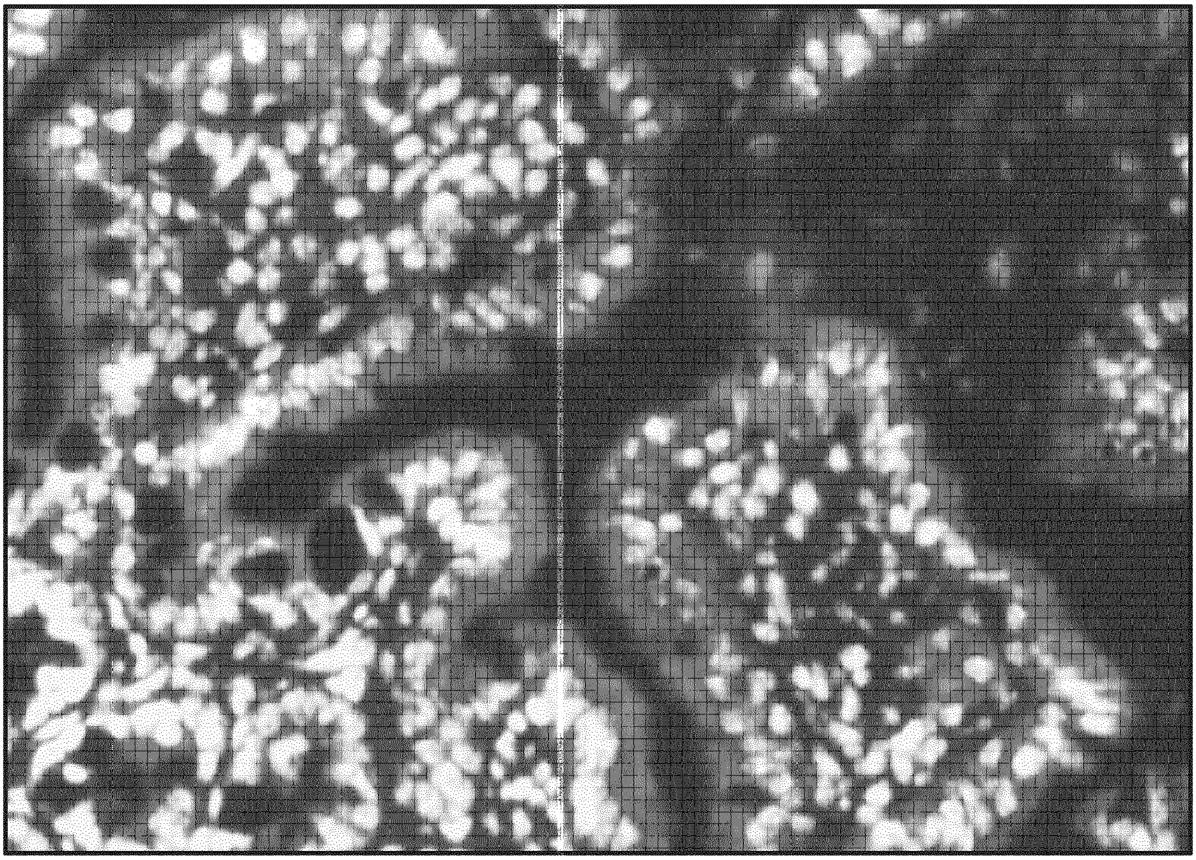

FIG. 48

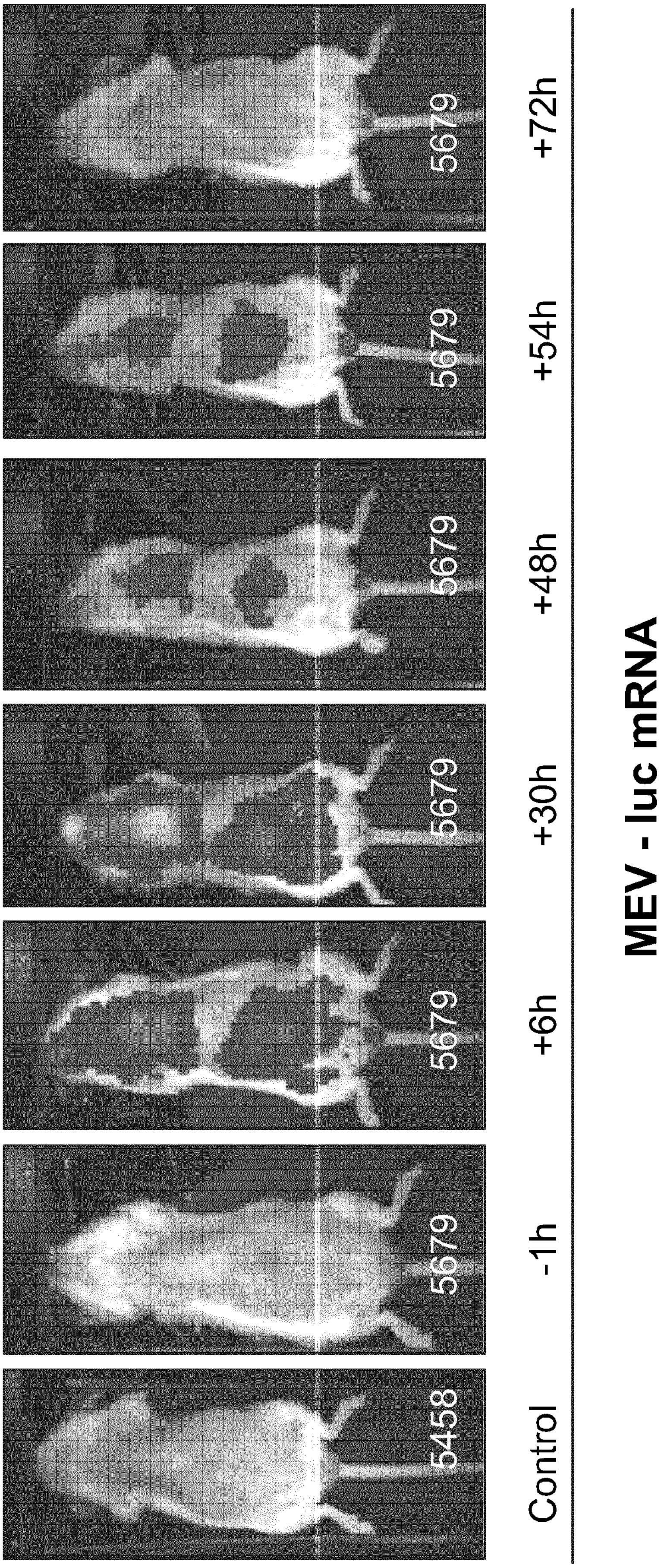
5458
5679
5679
5679
5679
5679
5679
Control
-1h
+6h
+30h
+48h
+54h
+72h
MEV - luc mRNA

FIG. 49

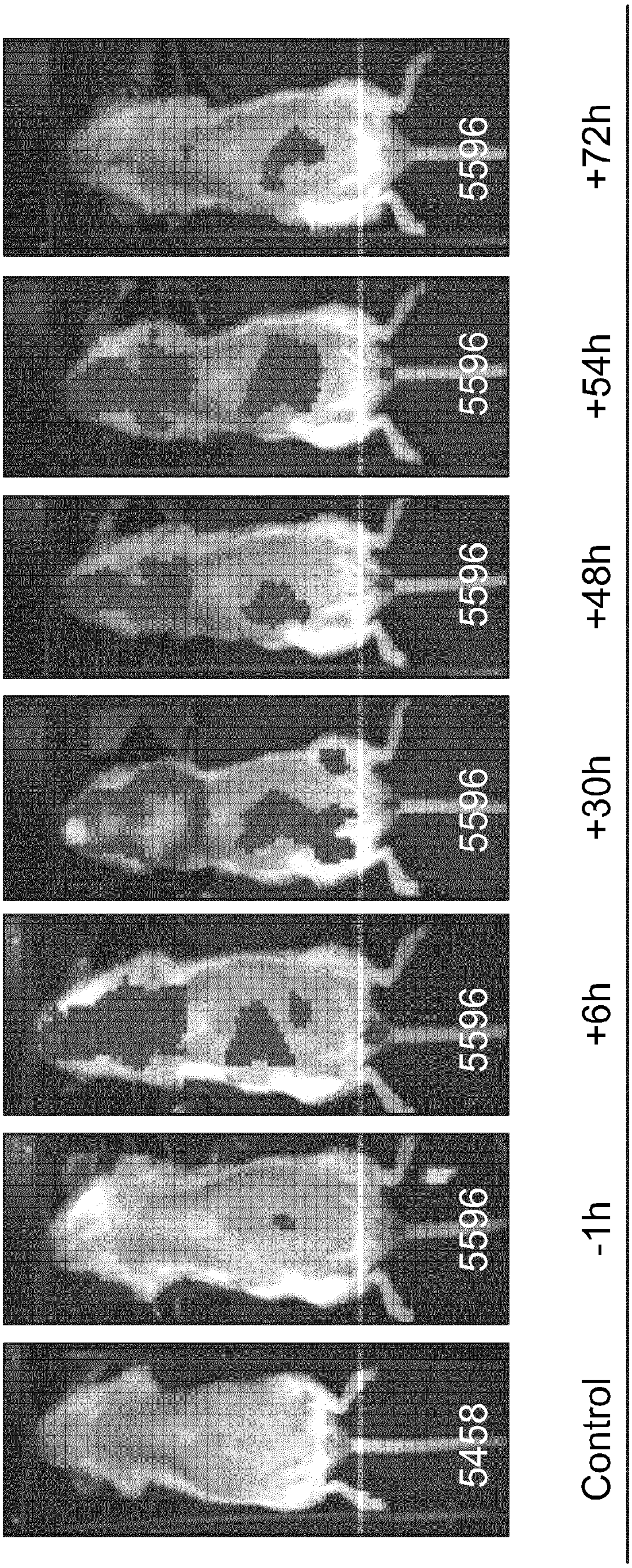


**MEV - luc protein**

FIG. 50

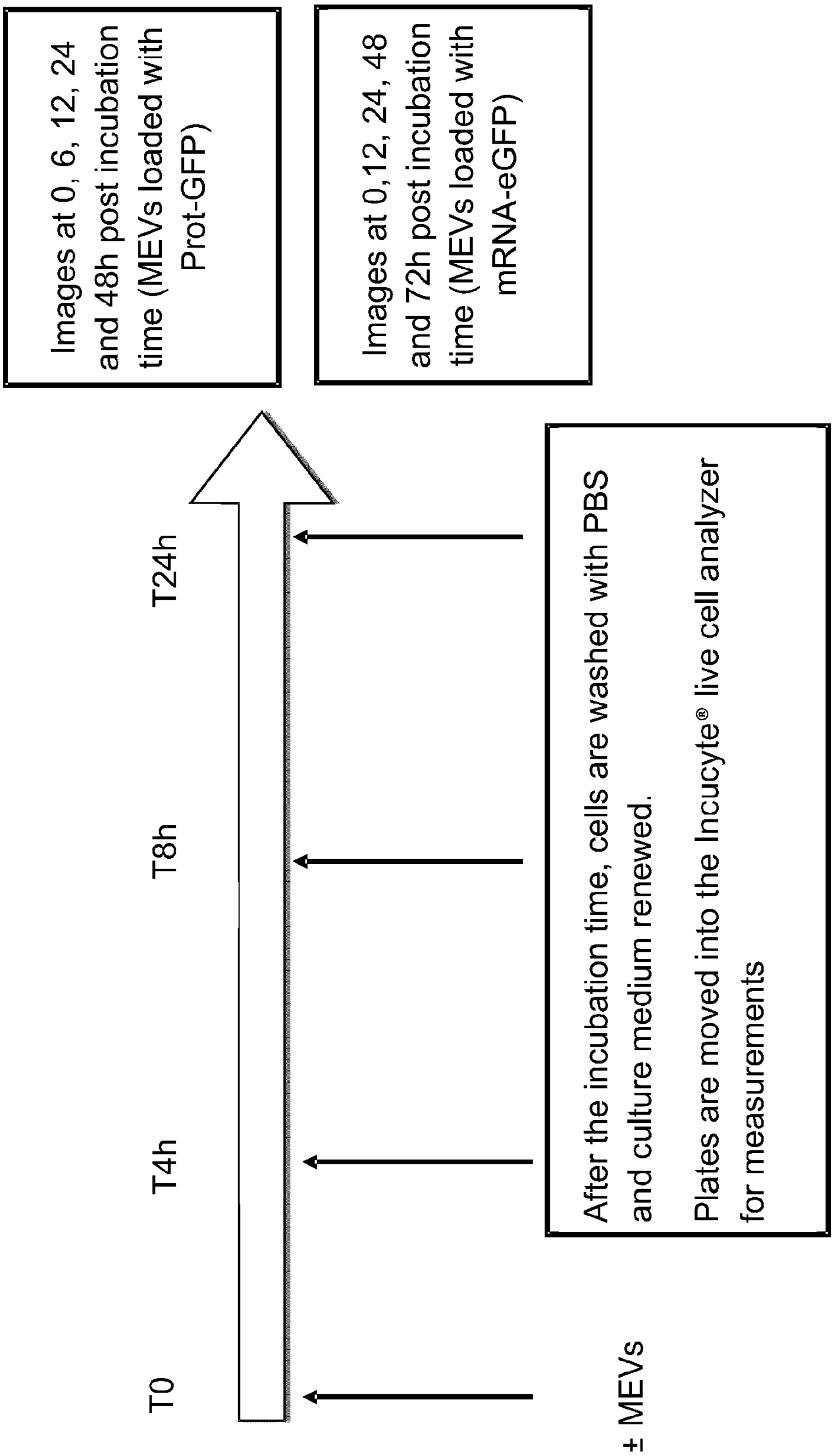
T0
T4h
T8h
T24h
± MEVs
After the incubation time, cells are washed with PBS and culture medium renewed.
Plates are moved into the Incucyte® live cell analyzer for measurements
Images at 0, 6, 12, 24 and 48h post incubation time (MEVs loaded with Prot-GFP)
Images at 0,12, 24, 48 and 72h post incubation time (MEVs loaded with mRNA-eGFP)

FIG. 51

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021122880 A
- US 63349006
- US 20200332273 A
- US 20200332274 A
- EP 2022070371 W
- WO 2009082606 A
- JP 2014240428 A
- WO 2011072292 A2
- WO 2010141724 A
- WO 2020097540 A
- CN 105821081 A
- CN 110699382 A
- US 10195290 B
- AU 2018365299
- US 5804396 A
- WO 9909016 A
- WO 9843960 A
- WO 9738983 A, Warner-Lambert
- WO 9906378 A, Warner-Lambert
- WO 9906396 A, Warner-Lambert
- WO 9630347 A
- WO 9633978 A
- WO 9633979 A
- WO 9633980 A
- EP 2019072169 W
- WO 2020035619 A
- EP 2019072170 W
- WO 2020035620 A
- WO 2013048734 A
- WO 2006029161 A
- WO 2007022470 A
- WO 2007130604 A
- WO 2008021157 A
- WO 2009104051 A
- WO 2009142822 A
- WO 2019217459 A
- WO 2020123083 A
- EP 2504435 A
- US 20110223665 A
- US 20120116360 A
- US 20120071540 A
- US 20160257956 A
- US 20150196648 A
- US 20170304459 A
- WO 2011071860 A
- WO 2011072292 A
- WO 2013126803 A
- AU 2004257373 A1
- AU 2013203219 B2
- AU 2016225873 A1
- EP 2395012 A
- EP 2888240 A
- US 20140256785 A
- US 20190032051 A
- JP 2018197239 A
- TW 201204351 A
- WO 2011068810 A
- WO 2019243574 A
- WO 2019092287 A
- WO 2020099682 A
- WO 2017161010 A
- WO 2019238626 A
- WO 2015110957 A2
- WO 2019018349 A1
- US 20120315324 A
- US 20190388347 A
- US 20190175506 A
- WO 2017203260 A
- WO 2018102397 A
- WO 2019081474 A
- WO 2019155060 A
- WO 2020041720 A
- AU 2018365299 A1
- SG 11201811149
- US 20190202892 A
- WO 2019155060 A1
- WO 2008087562 A
- US 5679377 A
- US 5916597 A
- US 5912015 A
- US 5989463 A
- US 5128326 A
- WO 9915154 A
- WO 9920253 A
- US 5323907 A
- US 5052558 A
- US 5033252 A
- US 20180362974
- WO 2019186558 A1
- US 20200297631 A1
- US 10799457 B2
- US 10857187 B2
- WO 2022053687 A
- EP 3967746 A
- US 63224656

**Non-patent literature cited in the description**


- **GOGOLLA**. The insular cortex. *Current Biology*, 2017, vol. 27 (12), R580-R586
- **LOCHHEAD et al.** Perivascular and Perineural Pathways Involved in Brain Delivery and Distribution of Drugs after Intranasal Administration. *Pharmaceutics*, 2019, vol. 11 (11), 598
- **IMAI**. Construction of functional neuronal circuitry in the olfactory bulb. *Seminars in Cell and Developmental Biology*, 2014, vol. 35
- **SELVARAJ et al.** Artificial Cells, Nanomedicine, and Biotechnology. *An International Journal*, 2018, vol. 46, 2088-2095
- **CECCHELLI et al.** *Nat Rev Drug Discov.*, 2007, vol. 6 (8), 650-661
- Computational Molecular Biology. Oxford University Press, 1988
- Biocomputing: Informatics and Genome Projects. Academic Press, 1993
- Computer Analysis of Sequence Data, Part I. Humana Press, 1994
- Sequence Analysis in Molecular Biology. Academic Press, 1987
- Sequence Analysis Primer. M Stockton Press, 1991
- **CARRILLO et al.** *SIAM J Applied Math*, 1988, vol. 48, 1073
- *J. Biol. Chem.*, 1968, vol. 243, 3557-59
- **WATSON et al.** Molecular Biology of the Gene. The Benjamin/Cummings Pub. Co., 1987, 224
- **NEEDLEMAN et al.** *J. Mol. Biol.*, 1970, vol. 48, 443
- *Adv. Appl. Math.*, 1981, vol. 2, 482
- **GRIBSKOV et al.** *Nucl. Acids Res.*, 1986, vol. 14, 6745
- Atlas of Protein Sequence and Structure. National Biomedical Research Foundation, 1979, 353-358
- *Adv. Appl. Math.*, 1991, vol. 12, 337-357
- *Biochem.*, 1972, vol. 11 (9), 1726-1732
- **ADAMO et al.** *Journal of Extracellular Vesicles*, 2021, vol. 10, e12081
- **BARKIA et al.** *Mar. Drugs*, 2019, vol. 17 (5), 304
- **KURUVINASHETTI et al.** *20th International Conference on Nanotechnology*, 2020, 354-357
- **PICCIOTTO et al.** *Biomater. Sci.*, 2021
- **DOYLE** ; **WANG**. *Cells*, 2019, vol. 8 (7), 727
- **ABELS** ; **BREAKEFIELD**. *Mol. Neurobiol.*, 2016, vol. 36 (3), 301-312
- **ADAMO et al.** *J. Extracell. Vesicles*, 2021, vol. 10, e12081
- **ABELS** ; **BREAKEFIELD**. *Cell Mol. Neurobiol.*, 2016, vol. 36 (3), 301-312
- **NAKASE** ; **FUTAKI**. *Sci. Rep.*, 2015, vol. 5, 10112
- **MAAS et al.** *Trends Cell Biol.*, 2017, vol. 27 (3), 172-188
- **KANWAR et al.** *Lab Chip.*, 2014, vol. 14 (11), 1891-1900
- **ZHAO et al.** *Lab Chip.*, 2016, vol. 16 (3), 489-496
- **WANG** ; **BARR**. *Essays Biochem.*, 2018, vol. 62 (2), 205-213
- **WANG** ; **BARR**. *Cell Mol. Neurobiol.*, 2016, vol. 36 (3), 449-457
- *VES4US, Extracellular vesicles from a natural source for tailor-made nanomaterials*, 2020
- **BLANC et al.** *Plant Cell*, 2010, vol. 22 (9), 2943-2955
- **CECCHIN et al.** *Plant J.*, 2019, vol. 100 (6), 1289-1305
- **HOVDE et al.** *Algal Research*, 2018, vol. 35, 449-461
- **GAO et al.** *BMC Genomics*, 2014, vol. 15 (1), 582
- **ARRIOLA et al.** *Plant J.*, 2018, vol. 93 (3), 566-586
- **GUARNIERI et al.** *Front. Bioeng. Biotechnol.*, 2018, vol. 6, 37
- **TEH et al.** *Data Brief*, 2019, vol. 27, 104680
- **WANG et al.** *Mol. Biol. Evol.*, 2020, vol. 37 (3), 849-863
- **ZUÑIGA et al.** *Plant Physiol.*, 2016, vol. 172 (1), 589-602
- **CHA et al.** *World J. Microbiol. Biotechnol.*, 2012, vol. 28, 1771-1779
- **CHAMBRAUD et al.** *FASEB J.*, 2007, vol. 21 (11), 2787-97
- **CHAMBRAUD et al.** *Proc. Natl. Acad. Sci. U.S.A.*, 2010, vol. 107 (6), 2658-63
- **CHAMBRAUD et al.** *Proc. Natl. Acad. Sci. U.S.A.*
- **FIRE et al.** *Nature*, 1998, vol. 391 (6669), 806-11
- **FIRE** ; **MELLO**. *Nobel Prize in Medicine awarded to Andrew Fire and Craig Mello*, 2006
- **SINGLA et al.** *bioRxiv*, 2019
- **BAULCOMBE**. *Current Opinion in Plant Biology*, 2015, vol. 26, 141-146
- **TENG et al.** *Cell Host & Microbes*, 2018, vol. 24, 637-652
- **SUNDARAM et al.** *iScience*, 2019, vol. 21, 308-327
- **GYÖRGY et al.** *Annu. Rev. Pharmacol. Toxicol.*, 2015, vol. 55, 439-464
- **SANDER** ; **JOUNG**. *Nat. Biotechnol.*, 2014, vol. 32 (4), 347-355

- **DA FONSECA FERREIRA, A.** ; **GOMES, D.** *Bioengineering (Basel)*, 2019, vol. 6 (1), 4
- **ANSEL**. Introduction to Pharmaceutical Dosage Forms. 1985, 126
- Goodman and Gilman's: The Pharmacological Bases of Therapeutics. Pergamon Press, 1990
- Remington's Pharmaceutical Sciences. Mack Publishing Co., 1990
- Pharmaceutical Dosage Forms: Parenteral Medications. Dekker, 1993
- Pharmaceutical Dosage Forms: Tablets. Dekker, 1990
- Pharmaceutical Dosage Forms: Disperse Systems. Dekker, 1990
- Remington: The Science and Practice of Pharmacy. Mack Publishing Company, 1995
- **ALFONSO R. GENNARO**. Remington: The Science and Practice of Pharmacy. Lippincott Williams & Wilkins, 2000
- Remington's Pharmaceutical Sciences. Mack Publishing Co.
- Medical Applications of Controlled Release. CRC Pres., 1974
- Controlled Drug Bioavailability, Drug Product Design and Performance. Wiley, 1984
- **LANGER** ; **PEPPAS**. *J. Macromol. Sci.*, 1983, vol. 23, 61
- **LEVY et al.** *Science*, 1985, vol. 228, 190
- **DURING et al.** *Ann. Neurol.*, 1989, vol. 25, 351
- **HOWARD et al.** *J. Neurosurg.*, 1989, vol. 71, 105
- **VADER et al.** *Advanced drug delivery reviews*, 2016, vol. 106 (A), 148-156
- **MORISHITA et al.** *Journal of pharmaceutical sciences*, 2017, vol. 106 (9), 2265-2269
- **WIKLANDER et al.** *J. Extracellular Vesicles*, 2015, vol. 4, 26316
- **VAN DER MEEL et al.** *J. Control. Release*, 2014, vol. 195, 72-8
- **LAI C.P. et al.** *ACS Nano*, 2014, vol. 8, 483-494
- **ZHUANG et al.** *Mol. Ther.*, 2011, vol. 19, 1769-1779
- **CHENG et al.** *Protein Cell*, 2019, vol. 10, 295-299
- **SAMUEL et al.** *Nat Commun*, 2021, vol. 12, 3950
- **ZHONG et al.** *Biomaterials*, 2021, vol. 277, 121126
- **CHENG et al.** *Protein Cell*, 2019, vol. 10 (4), 295-299
- **CESTA**. *Toxicologic Pathology*, 2006, vol. 34, 455-465
- Targeting Immunomodulatory Agents to the Gut-Associated Lymphoid Tissue. **ZGAIR et al.** Neuro-Immuno-Gastroenterology. Springer, 2016
- **LUONGO et al.** *Current perspectives. International Reviews of Immunology*, 2009, vol. 28 (6), 446-464
- **MACKAY et al.** *J Exp Med*, 1990, vol. 171, 801-17
- **GOTTFRIED et al.** *Neuron*, 2006, vol. 49, 467-479
- **HOWARD et al.** *Nature Neuroscience*, 2009, vol. 12, 932-938
- **WILSON et al.** *Neuron*, 2011, vol. 72, 506-519
- **BEKKERS et al.** *Trends in Neuroscience*, 2013, vol. 36, 429-438
- **COURTIOL et al.** *Perception*, 2017, vol. 46 (3-4), 320-332
- **GOTTFRIED et al.** *Neuron*, 2003, vol. 39, 375-386
- **CALU et al.** *Cerebral cortex (New York, N.Y.*, 1991, vol. 17, 1342-1349
- **ROESCH et al.** *Cerebral cortex (New York, N.Y.*, 1991, vol. 17, 643-652
- **ZELANO et al.** *Neuron*, 2011, vol. 72, 178-187
- **STRAUCH et al.** *Cerebral Cortex*, 2018, vol. 28, 764-776
- **LOSCHER et al.** *Progress in Neurobiology*, 1996, vol. 50, 427-481
- **VISMER et al.** *Front. Neural Circuits*, May 2015, 29
- **YOUNG et al.** *Experimental Neurology*, 2019, vol. 320, 113013
- **SAMUDRALWAR et al.** *Journal of the Neurological Sciences*, 1995, vol. 130, 139-145
- **SAIZ-SANCHEZ et al.** *Brain Struct Funct*, 2015, vol. 220, 2011-2025
- **MENASSA et al.** *Neuroscience Letters*, 2018, vol. 665, 86-91
- **KOEHLER et al.** *Chemical Senses*, 2018, vol. 43, 627-634
- **WU et al.** *Human Brain Mapping*, 2011, vol. 32, 1443-1457
- **LIPINSKI**. *Drug Discovery Today: Technologies*, 2004, vol. 1 (4), 337-34
- **ZHUANG et al.** *Mol Ther.*, 2011, vol. 19 (10), 1769-1779
- **ALVAREZ-ERVITI et al.** *Nat. Biotechnol.*, 2011, vol. 29 (4), 341-5
- **WANG et al.** *Nat. Commun.*, 2013, vol. 4, 1867
- **HANEY et al.** *J. Control Release*, 2015, vol. 207, 18-30
- **ZHUANG et al.** *Mol. Ther.*, 2016, vol. 24 (1), 96-105
- **BETZER et al.** *ACS Nano.*, 2017, vol. 11 (11), 1088 3-10893
- **ESPOSITO et al.** *Eur J Pharm Biopharm.*, 2017, vol. 115, 285-296
- **TIAN et al.** *Biomaterials*, 2018, vol. 150, 137-149
- **HU et al.** *Mol. Ther. Nucleic Acids.*, 2018, vol. 13, 450-463

- **NATSHEH et al.** *Drug Deliv. Transl. Res.*, 2018, vol. 8 (3), 806-819
- **GUO et al.** *ACS Nano.*, 2019, vol. 13 (9), 10015-10028
- **HANEY et al.** *Cells*, 2020, vol. 9 (5), 1273
- **IOANNIDES et al.** *J. Cancer Metastisis Treat.*, 2020, vol. 6 (15)
- **LOSURDO et al.** *Stem Cells Transl Med.*, 2020, vol. 9 (9), 1068-1084
- **TSIVION-VISBORD et al.** *Transl. Psychiatry*, 2020, vol. 10 (1), 305
- **CHIVERO et al.** *Front Cell Dev Bio.*, 2020, vol. 8, 573
- **TOUITOU et al.** *Int. J. Pharm.*, 2020, vol. 580, 119243
- **MOSS et al.** *Neurochem Int.*, 2021, vol. 150, 105-173
- **PUSIC et al.** *PloS one*, 2021, vol. 16 (8), e0 255778
- **NIU et al.** *Nano Lett.*, 2021, vol. 21 (3), 1484-1492
- **HAYES et al.** *Neurotox. Res.*, 2021, vol. 39 (5), 1 418-1429
- **ZHAO et al.** *Cells*, 2022, vol. 11 (12), 1933
- **KUTCHY et al.** *Front. Pharmacol.*, 2022, vol. 13 (819516), 1-9
- **ELSHEIKH et al.** *Pharmaceuti cs*, 2022, vol. 14 (3), 576
- **SAHIN-YILMAZ et al.** *Proc. American Thoracic Society*, 2011, vol. 8, 31-39
- **SAHIN-YILMAZ**. *Proc. American Thoracic Society*, 2011, vol. 8, 31-39
- **LOCHHEAD et al.** *Advanced Drug Delivery Reviews*, 2012, vol. 64, 614-628
- **YING**. *Future Neurol.*, December 2007, vol. 3 (1)
- **LOCHHEAD et al.** *J. Cerebral Blood Flow Metab.*, 2015, vol. 35, 371-381
- Perivascular and Perineural Pathways Involved in Brain Delivery and Distribution of Drugs after Intranasal Administration. **JEFFREY J. LOCHHEAD** ; **THOMAS P. DAVIS**. Pharmaceutics. Department of Pharmacology, University of Arizona, 2019, vol. 11, 598
- **GÄNGER et al.** *Pharmaceutics*, 2018, vol. 10, 116
- **SON et al.** *BMB Reports*, 2021, vol. 54 (6), 295-304
- **BUCHNER et al.** *Neuroscience*, 1987, vol. 22, 697-707
- Construction of functional neuronal circuitry in the olfactory bulb. **TAKESHI IMAI**. Seminars in Cell and Developmental Biology. Kyushu University, November 2014, vol. 35
- **NOTO et al.** *Front Syst Neurosci*, 2021, vol. 15, 752320
- **BADURA-BRACK et al.** *Psychiatry Research: Neuroimaging*, 2018, vol. 271, 135-141
- **BADURA-BRACK et al.** *Biological Psychology*, 2018, vol. 132, 228-232
- **ZHOU et al.** *eLife*, 2019, vol. 8, e47177
- **RUSSO et al.** *Algorithms*, 2018, vol. 11, 134
- **ZHOU et al.** *Nucleic Acids Research*, 2019, vol. 47 (W1), W234-W241
- **IMAI**. *Seminars in Cell & Developmental Biology*, 2014, vol. 35, 180-188
- **IGARASHI et al.** *Journal of Neuroscience*, 2012, vol. 32, 7970-7985
- **NAGAYAMA et al.** *Front. Neural Circuits*, 23 September 2010, vol. 23
- **RUSSO et al.** *Experimental Brain Research*, 2018, vol. 236, 2223-2229
- **SALIMI et al.** *The Journal of Physiological Sciences*, 2021, vol. 71
- **GEHRLACH et al.** *eLife*, 2020, vol. 9, e55585
- **BUCKWALTER et al.** *Experimental Brain Research*, 2008, vol. 186, 47-57
- **MACDONALD et al.** *J. Neurophysiol.*, 1998, vol. 79, 474-477
- **GROEN et al.** *J. Comparative Neurology*, 2003, vol. 463, 249-263
- **ZHANG et al.** *J. Cellular Biochemistry*, 19 June 2019, vol. 120 (10), 17584-17592
- **GRAILHE et al.** *Neuron*, 1999, vol. 22 (3), 581-591
- **SHAO, LIAO et al.** *Neuron*, 2021, vol. 109 (16), 2535-2544
- **MAREK** ; **AGHAJANIAN**. *Eur. J. Pharmacol.*, 1996, vol. 305 (1-3), 95-100
- **SHAO et al.** *Neuron*, 2021, vol. 109 (16), 2535-2544
- **DE VOS et al.** *Frontiers in Psychiatry*, 2021, vol. 12, 724606
- **DAVOUDIAN et al.** *bioRxviv*, 2022, vol. 14 (3), 468-480
- **LOPEZ-GIMENEZ** ; **GONZALEZ-MAESO**. *Curr. Top Behav. Neurosci.*, 2018, vol. 36, 45-73
- **BARRETT et al.** *Intl. Rev. Psychiatry*, 2018, vol. 30 (4), 350-362
- **KAELEN et al.** *Psychopharmacology (Berl)*, 2015, vol. 232 (19), 3607-14
- **PRELLER et al.** *Curr. Biol.*, 2017, vol. 27 (3), 451-457
- **KRAEHENMANN et al.** *Biol. Psychiatry*, 2015, vol. 78 (8), 572-581
- **MUCLLCR et al.** *Transl. Psychiatry*, 2017, vol. 7 (4), e1084
- **GRESCH et al.** *Neuroscience*, 2002, vol. 114 (3), 707-713
- **GRIMM et al.** *Eur. Neuro-psychopharmacol.*, 2018, vol. 28 (6), 691-700
- **PEDZICH et al.** *Neuropsychopharmacology*, 2022, vol. 47, 1304-1314
- **DOLDER et al.** *Neuropsychopharmacology*, 2016, vol. 41 (11), 2638-2646

- **WATTS et al.** *Neurochem. Intl.*, 1994, vol. 24 (6), 565-574
- **NAKADA et al.** *Neuroscience Letters*, 1984, vol. 49 (1-2), 13-18
- **BECK et al.** *The Plant cell*, 2012, vol. 24 (10), 4205-4219
- **OTEGUI et al.** *Traffic (Copenhagen, Denmark)*, 2008, vol. 9 (10), 1589-1598
- **GÓMEZ-GÓMEZ et al.** *Trends in plant science*, 2002, vol. 7 (6), 251-256
- **CECCHIN et al.** *The Plant Journal*, 2019, vol. 100, 1289-1305
- **KANG et al.** Biodistribution of extracellular vesicles following administration into animals: A systematic review. *J Extracell Vesicles.*, 2021, vol. 10, e12085
- **PFAFFL**. *Nucleic Acids Res.*, 2001, vol. 29 (9), e45
- **KOCH A et al.** *J Phytopathol*, 2012, vol. 60, 606-610
- **BROOKS et al.** *Mol Plant Microbe Interact.*, 2004, vol. 17 (2), 162-174
- **FOUTS et al.** *Proc. Natl. Acad. Sci. U.S.A.*, 2004, vol. 99, 2275-2280
- **SREEDHARAN et al.** *Molecular plant-microbe interactions MPMI*, 2006, vol. 19, 768-779
- **KOCH et al.** *Proc. Natl. Acad. Sci. U.S.A.*, 2013, vol. 110, 19324-19329
- **KOCH et al.** *PLOS Pathogens*, 2016, vol. 12 (10), e100590
- **CHUNG et al.** Structural and molecular interrogation of intact biological systems.. *Nature*, 2013, vol. 497, 332-337
- **YAN et al.** *NeuroImage*, 2013, vol. 80, 246-262
- **CHEN et al.** Isolation and culture of rat and mouse oligodendrocyte precursor cells.. *Nat Protoc.*, 2007, vol. 2, 1044-1051
- **BRYNSKIKH et al.** Macrophage delivery of therapeutic nanozymes in a murine model of Parkinson's disease. *Nanomedicine (Lond)*, 2010, vol. 5 (3), 379-396
- **TIEU et al.** D-beta-hydroxybutyrate rescues mitochondrial respiration and mitigates features of Parkinson disease.. *J Clin Invest.*, 2003, vol. 112 (6), 892-901
- **PAPATHANOU et al.** Induction and expression of abnormal involuntary movements is related to the duration of dopaminergic stimulation in 6-OHDA-lesioned rats.. *Eur J Neurosci.*, 2011, vol. 33 (12), 2247-2254
- **YAMIN et al.** *J. Cell. Biochem.*, 2019, vol. 120, 7584-17592